# EUROPEAN PATENT SPECIFICATION

(11) **EP 2 473 491 B1**
(45) Date of publication and mention of the grant of the patent: **17.07.2013**
(21) Application number: 10722268.9
(22) Date of filing: 01.06.2010
(51) Int. Cl.: C07D 265/32, C07D 413/04, C07D 413/12, C07D 413/14, A61K 31/5377, A61P 7/02

(54) **MORPHOLINONE COMPOUNDS AS FACTOR IXA INHIBITORS**
MORPHOLINONVERBINDUNGEN ALS FAKTOR-IXA-HEMMER
COMPOSÉS MORPHOLINONES COMME INHIBITEURS DU FACTEUR IXA

(30) Priority: 31.08.2009 US 238455 P; 03.12.2009 WO PCT/US9066/548
(43) Date of publication of application: 11.07.2012
(73) Proprietor: Mochida Pharmaceutical Co., Ltd., Tokyo 160-8515 (JP); Merck Sharp & Dohme Corp., Rahway, NJ 07065-0907 (US)
(72) Inventor: NISHIDA, Hidemitsu, Tokyo 160-8515 (JP); SAITOH, Fumihiko, Tokyo 160-8515 (JP); HIRABAYASHI, Tomokazu, Tokyo 160-8515 (JP); CHACKALAMANNIL, Samuel, Califon, New Jersey 07830 (US); CHAN, Tin-Yau, Edison, New Jersey 08817 (US); CHELLIAH, Mariappan, V., Edison, New Jersey 08820 (US); CLASBY, Martin, C., Plainsboro, New Jersey 08536 (US); DWYER, Michael, P., Scotch Plains, New Jersey 07076 (US); GREENLEE, William, J., Teaneck, New Jersey 07666 (US); XIA, Yan, Edison, New Jersey 08820 (US); NEELAMKAVIL, Santhosh, Scotch Plains, New Jersey 07076 (US); SHAH, Unmesh, G., Green Brook, New Jersey 08812 (US)
(74) Representative: Hussain, Deeba
(86) International application number: PCT/US2010/036853
(87) International publication number: WO 2011/025565

(56) References cited:
- WO-A1-2005/073201
- WO-A1-2010/065717
- DE-A1- 10 315 377
- SMALLHEER J M ET AL: "SAR and factor IXa crystal structure of a dual inhibitor of factors IXa and Xa" BIOORGANIC & MEDICINAL CHEMISTRY LETTERS, PERGAMON, ELSEVIER SCIENCE, GB LNKD- DOI:10.1016/J.BMCL.2004.08.034, vol. 14, no. 21, 1 November 2004 (2004-11-01), pages 5263-5267, XP004580511 ISSN: 0960-894X

## Description

### FIELD OF THE INVENTION

The invention relates to novel compounds of the Formula (I) having antithrombotic activity which, in particular, inhibit blood clotting factor IXa, to processes for their preparation and to use thereof as medicaments.

### BACKGROUND OF THE INVENTION

Factor IXa is a plasma serine protease involved in the regulation of blood coagulation. While blood coagulation is a necessary and important part of the regulation of an organism's homeostasis, abnormal blood coagulation can also have deleterious effects. For instance, thrombosis is the formation or presence of a blood clot inside a blood vessel or cavity of the heart. Such a blood clot can lodge in a blood vessel blocking circulation and inducing a heart attack or stroke. Thromboembolic disorders are the largest cause of mortality and disability in the industrialized world.

Blood clotting is a process of control of the blood stream essential for the survival of mammals. The process of clotting, as shown in the Patent Document 1, and the subsequent dissolution of the clot after wound healing has taken place commences after vascular damage and can be divided into four phases:
1. The phase of vasoconstriction or vasocontraction: By means of this the blood loss in the damaged area is decreased.
2. The next phase is platelet activation by thrombin. The platelets attach to the site of the vessel wall damage and form a platelet aggregate. The protein fibrinogen is responsible here for the crosslinkage of the platelets by means of appropriate surface receptors. Platelets also bind to exposed collagen of the extracellular matrix of the damaged vessel wall and are activated by this means. After activation of the platelets, a number of messenger substances are secreted, which induce the activation of further platelets. At the same time, a membrane lipid, phosphatidylserine, is transported from the inside of the membrane of the platelets to the outside, on which complexes of clotting factors can accumulate. The platelets accelerate blood clotting by means of this mechanism.
3. The formation of these clotting complexes leads to the massive formation of thrombin, which converts soluble fibrinogen to fibrin by cleavage of two small peptides. Fibrin monomers spontaneously form threadlike strands, from which, after crosslinkage by clotting factor XIII, a stable protein network forms. The initially even looser platelet aggregate is stabilized by this fibrin network; platelet aggregates and fibrin network are the two essential constituents of a thrombus.
4. After wound healing, the thrombus is dissolved by the action of the key enzyme of the endogenous fibrinolysis system, plasmin.

Two alternative pathways can lead to the formation of a fibrin clot, the intrinsic and the extrinsic pathway. These pathways are initiated by different mechanisms, but in the later phase they converge to give a common final path of the clotting cascade. In this final path of clotting, clotting factor X is activated. The activated factor X is responsible for the formation of thrombin from the inactive precursor prothrombin circulating in the blood. The formation of a thrombus on the bottom of a vessel wall abnormality without a wound is the result of the intrinsic pathway. Fibrin clot formation as a response to tissue damage or an injury is the result of the extrinsic pathway. Both pathways comprise a relatively large number of proteins, which are known as clotting factors.

The intrinsic pathway requires the clotting factors V, VIII, IX, X, XI and XII and also prekallikrein, high molecular weight kininogen, calcium ions and phospholipids from platelets.

The intrinsic pathway is initiated when prekallikrein, high molecular weight kininogen factor XI and XII bind to a negatively charged surface. This point in time is designated as the contact phase. Exposure to vessel wall collagen is the primary stimulus of the contact phase. The result of the processes of the contact phase is the conversion of prekallikrein to kallikrein, which in turn activates factor XII. Factor XIIa hydrolyzes further prekallikrein to kallikrein, such that activation is the result. With increasing activation of factor XII, activation of factor XI occurs, which leads to a release of bradykinin, a vasodilator. As a result, the ending of the initial phase of vasoconstriction occurs. Bradykinin is formed from high molecular weight kininogen. In the presence of Ca²⁺ ions, factor XIa activates factor IX. Factor IX is a proenzyme, which contains vitamin K-dependent, γ-carboxyglutamic acid (GLA) residues. The serine protease activity becomes noticeable after binding of Ca²⁺ to these GLA residues. A number of the serine proteases of the blood clotting cascade (factors II, VII, IX and X) contain such vitamin K-dependent GLA residues. Factor IXa cleaves factor X and leads to activation to factor Xa. The prerequisite for the formation of factor IXa is the formation of a tenase complex from Ca²⁺ and the factors VIIIa, IXa and X on the surface of activated platelets. One of the reactions of activated platelets is the presentation of phosphatidylserine and phosphatidylinositol along the surfaces. The exposure of these phospholipids first makes the formation of the tenase complex possible. Factor VIII in this process has the function of a receptor for the factors IXa and X. Factor VIII is therefore a cofactor in the clotting cascade. The activation of factor VIII with formation of factor VIIIa, the actual receptor, needs only a minimal amount of thrombin. With increase in the concentration of thrombin, factor VIIIa is finally cleaved further and inactivated by thrombin. This dual activity of thrombin in relation to factor VIII leads to a self-restriction of tenase complex formation and thus to a limitation of blood clotting.

The extrinsic pathway requires a tissue factor (TF) and clotting factors V, VII, VIII, IX and X. In the case of a vessel injury, the tissue factor (TF) accumulates with the clotting factor VII and the latter is activated. The complex of TF and clotting factor VII has two substrates, clotting factors X and IX.

Clotting factor IX can be activated by means of the intrinsic pathway and the extrinsic pathway. The activation of factor IXa is thus a central point of intersection between the two pathways of activation of clotting.

Factor IXa has an important role in blood clotting. Defects in factor IXa lead to hemophilia B, while increased concentrations of factor IXa in the blood lead to a significantly increased risk of thrombosis formation (Weltermann A, et al., J Thromb Haemost. 2003; 1: 28-32). The regulation of factor IXa activity can reduce thrombus formation in animal models (Feuerstein GZ, et al., Thromb Haemost. 1999; 82: 1443-1445).

Recently, compounds having a Factor IXa antagonism are being studied. Known compounds each having an amide bond are disclosed in, for example, PCT Publication No. 08/031508, and PCT Publication No. 08/031509. However, these patent documents do not disclose cyclic morpholinone derivatives.

In the development of pharmaceuticals, it is required to satisfy strict criteria for not only target pharmacological activity but also absorption, distribution, metabolism, excretion, and the like. With respect to drug interactions, desensitization or tolerance, digestive absorption in oral administration, the rate of transfer to a small intestine, the rate of absorption and first-pass effect, an organ barrier, protein binding, induction of a drug-metabolizing enzyme, an excretion pathway and body clearance, a method of administration (an application site, a method, and purpose), and the like, various agenda are required. However, a drug that satisfies these requirements is seldom discovered.

These comprehensive problems in drug development might also exist for Factor IXa antagonists, and Factor IXa antagonists have not yet been released onto the market. More specifically, known compounds having a Factor IXa antagonism may also include problems in terms of usefulness and safety. For example, these compounds may have low absorption, and oral administration of these compounds may be difficult; these compounds also may exhibit inhibitory activity of the human ether-a-go-go related gene (hERG) channel, which may cause arrhythmia, and pharmacokinetics of these compounds might not satisfactory.

Accordingly, a compound in which these problems are solved and which has high activity has been desired.

### SUMMARY OF THE INVENTION

In its many embodiments, the present invention provides a novel class of cyclic morpholine compounds or its analogue, pharmaceutical compositions comprising one or more said compounds, and methods for using said compounds for treating or preventing a thromboses, embolisms, hypercoagulability or fibrotic changes.

The compounds of the Formula (I) according to the invention are suitable for prophylactic and for therapeutic administration to humans who suffer from diseases which accompany thromboses, embolisms, hypercoagulability or fibrotic changes. They can be employed for secondary prevention and are suitable both for acute and for long-term therapy.

The invention therefore relates to a compound of Formula (I)

In another aspect, compounds of the Formula (I), or a pharmaceutical acceptable salt or a solvate thereof can be useful for treating or preventing a disorder or disease mediated by factor IXa, or a thromboembolic disorder (each disorder being a "Condition").

In another aspect, the present invention provides pharmaceutical compositions comprising at least one compound of the Formula (I) or a pharmaceutically acceptable carrier. The composition can be useful for treating or preventing a Condition.

In another aspect, the present invention discloses a method for treating a Condition, the method comprising administering to a patient an effective amount of at least one compound of Formula (I) or a pharmaceutically acceptable salt or a solvate thereof.

### DETAILED DESCRIPTION OF THE INVENTION

In an embodiment, the present invention provides compounds of Formula (I) and/or pharmaceutically acceptable salts, solvates and prodrugs thereof. The compounds of Formula (I) can be useful for treating or preventing a Condition in a patient.

The invention provides compounds of Formula (I) 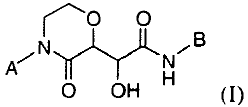 or a pharmaceutically acceptable salt or a solvate thereof, wherein
A is 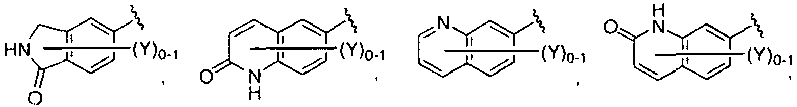 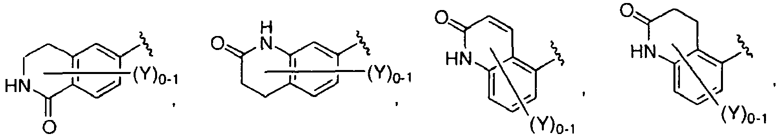 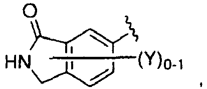 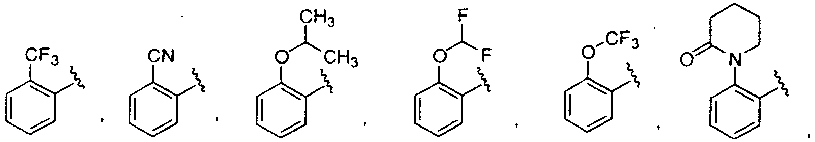 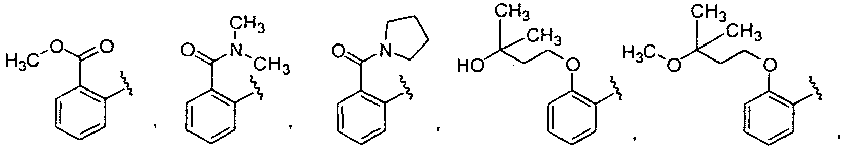 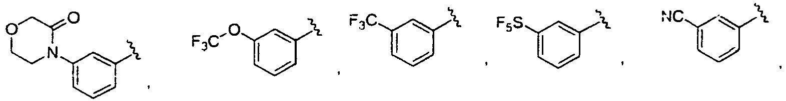 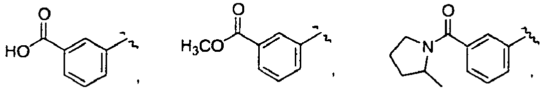 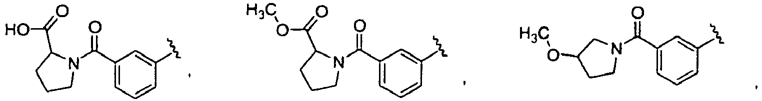 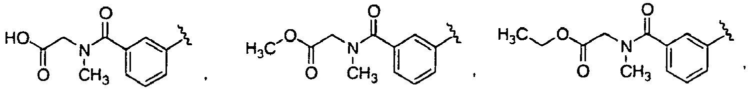 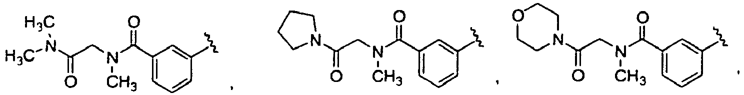 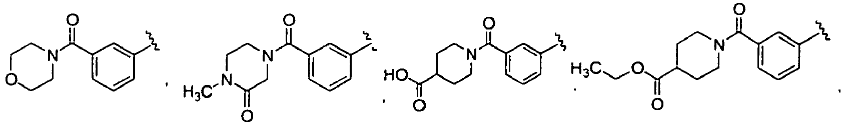 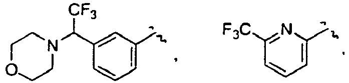 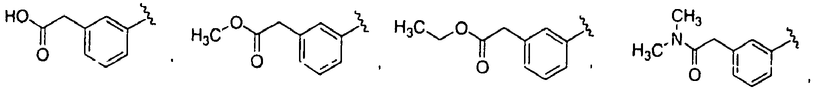 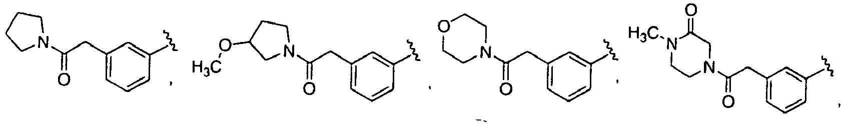 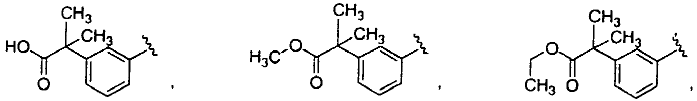 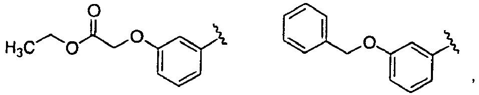 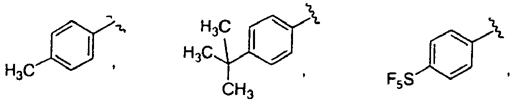 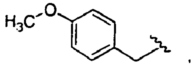 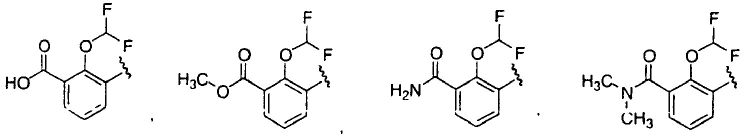 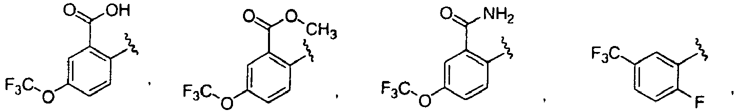 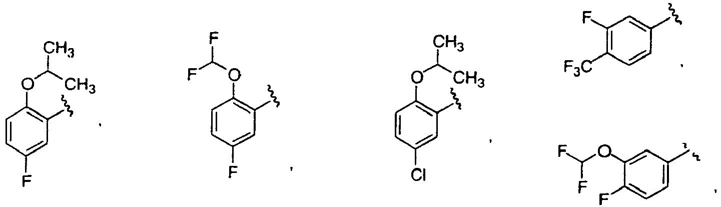 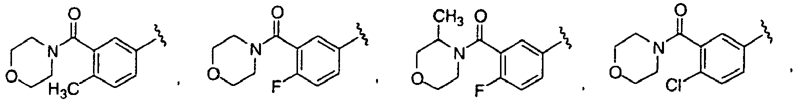 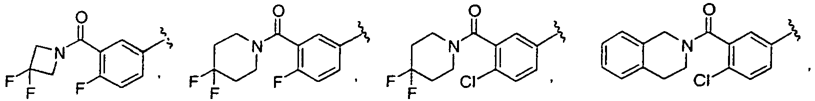 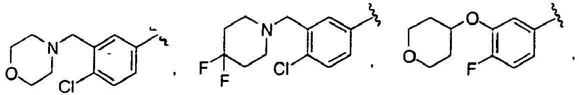 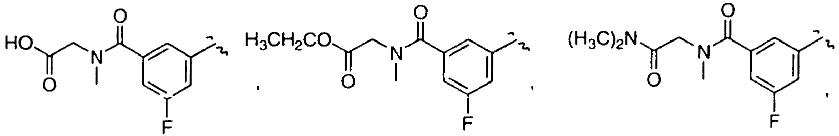 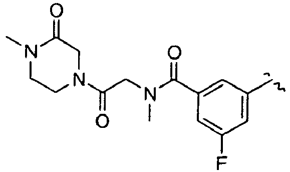 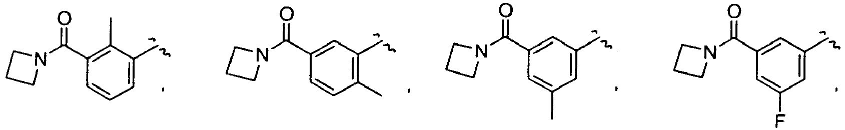 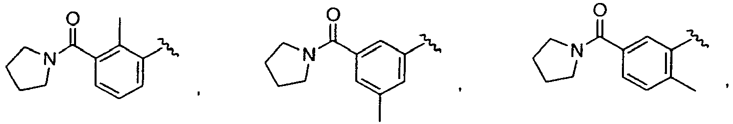 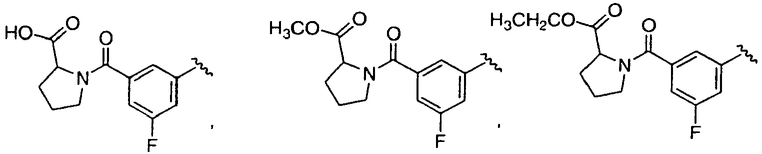 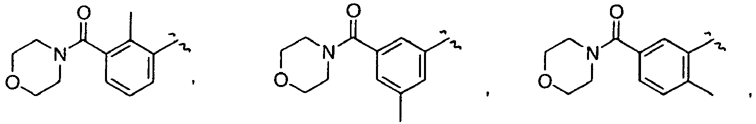 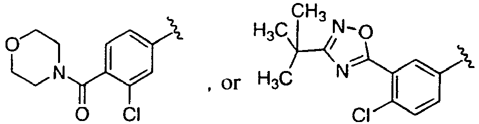 Y, attached to a carbon or nitrogen ring atom, is
1) halogen,
2) -(C₁-C₆)-alkyl,
3) -(C₁-C₃)-haloalkyl,
4) -(C₃-C₈)-cycloalkyl,
5) -OH,
6) -O-(C₁-C₆)-alkyl,
7) -O-(C₁-C₃)-haloalkyl,
8) =O (oxo),
wherein said -(C₁-C₆)-alkyl part of 2) and 6) of said Y is unsubstituted or substituted independently with the substituents selected from the group consisting of -(C₃-C₈)-cycloalkyl, -C(O)OH, and -C(O)-(C₁-C₆)-alkyl,
B is 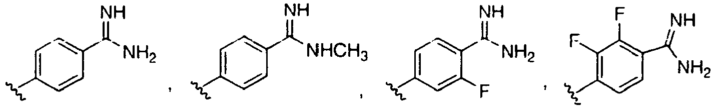 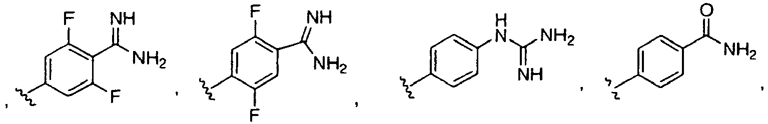 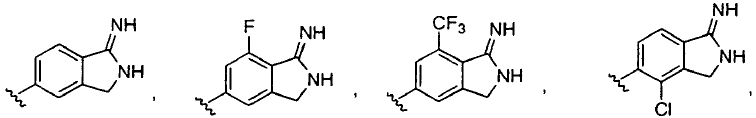 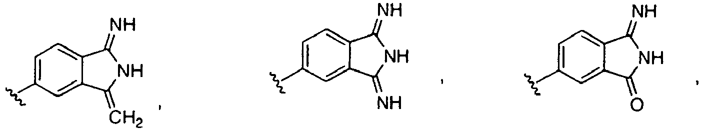 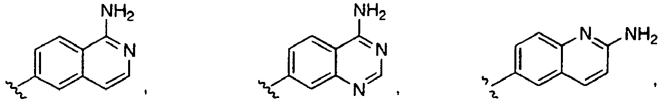 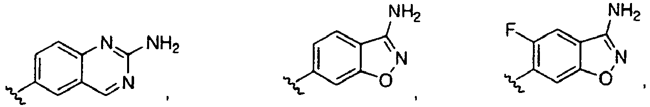 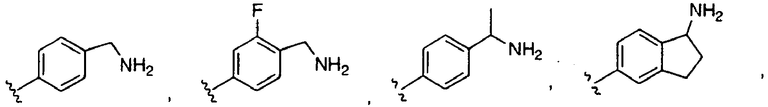 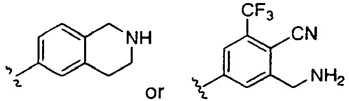 provided that
when A is 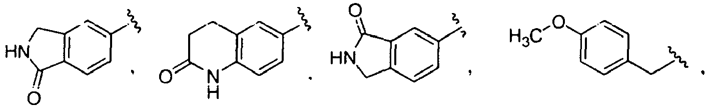 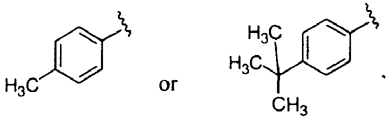 B is not 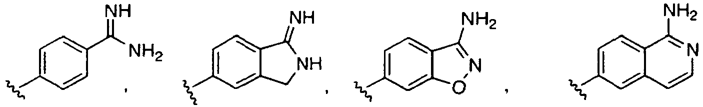 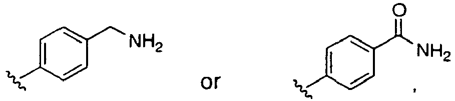 and
when A is 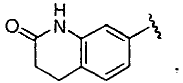 B is not

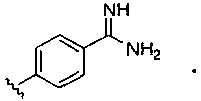

In one aspect of the invention, the compounds are of the formula 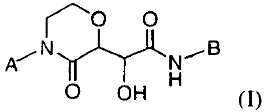 or a pharmaceutically acceptable salt or a solvate thereof, wherein
A is 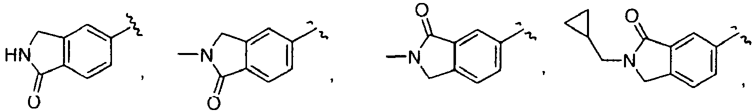 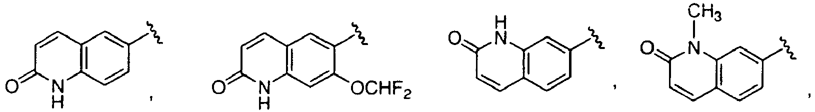 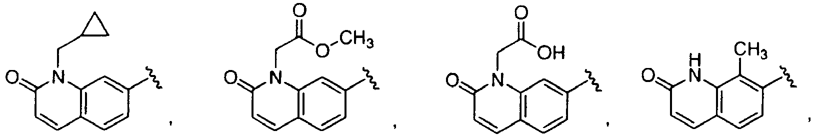 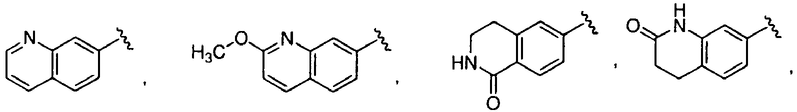 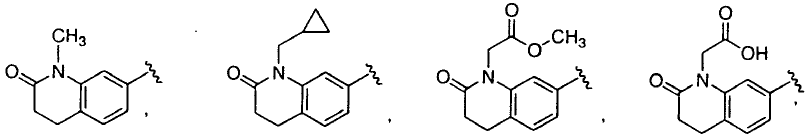 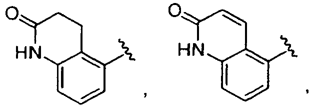 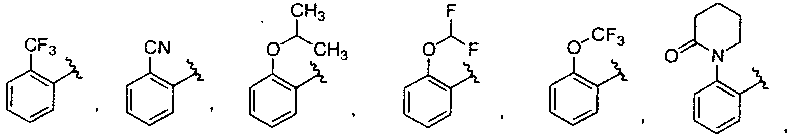 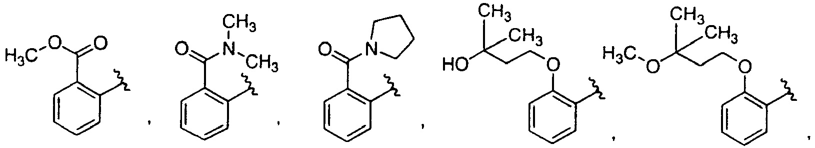 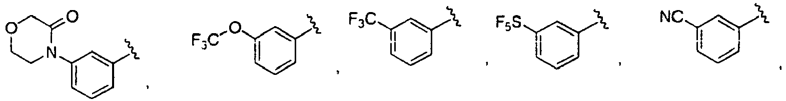 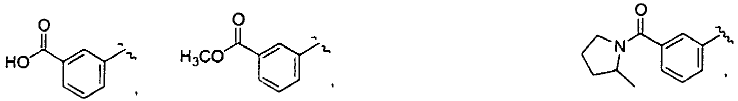 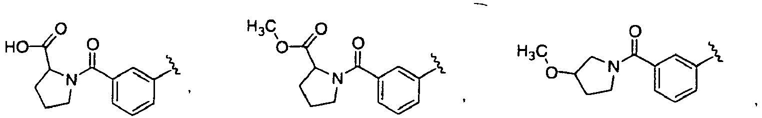 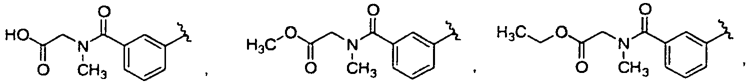 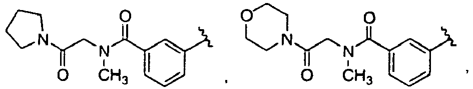 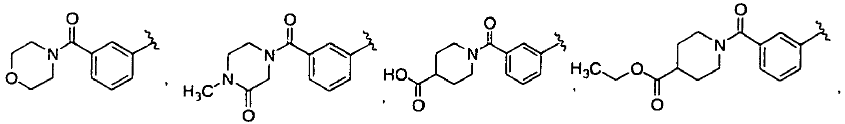 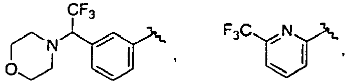 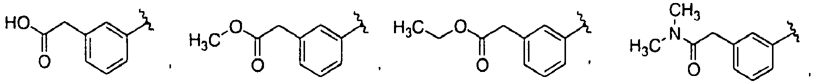 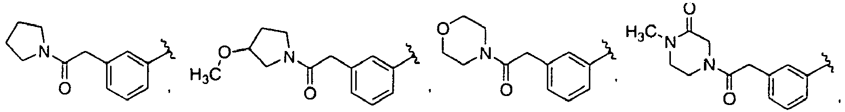 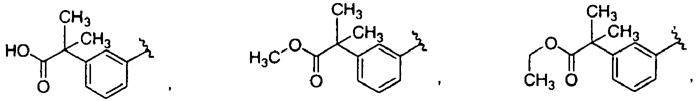 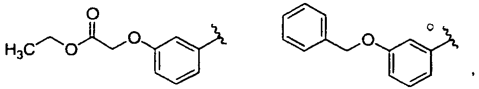 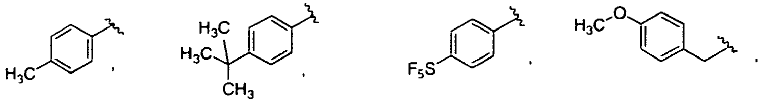 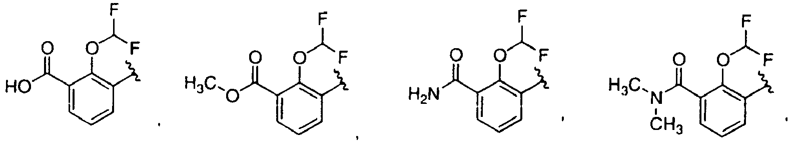 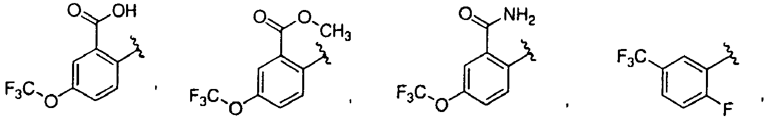 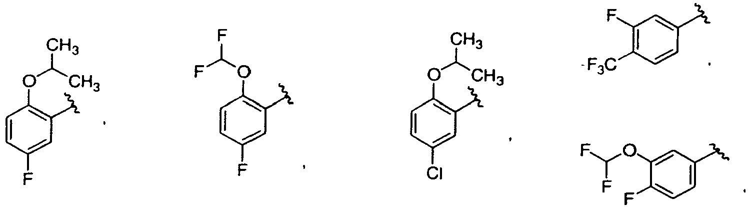 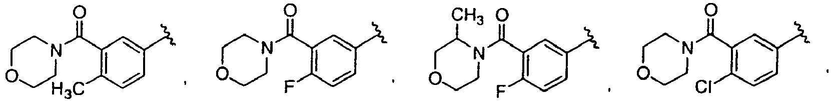 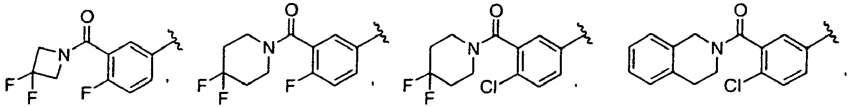 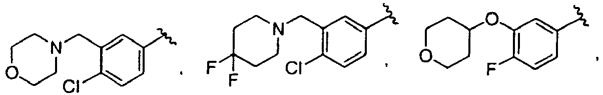 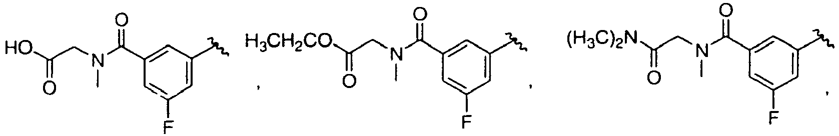 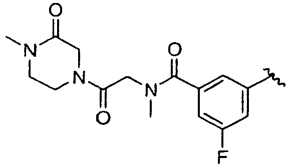 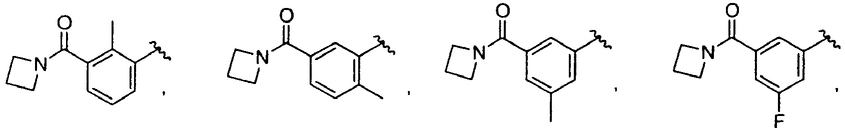 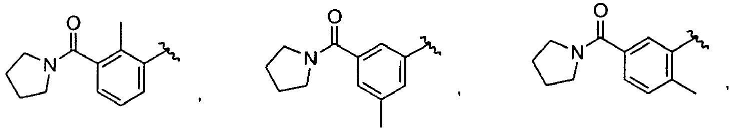 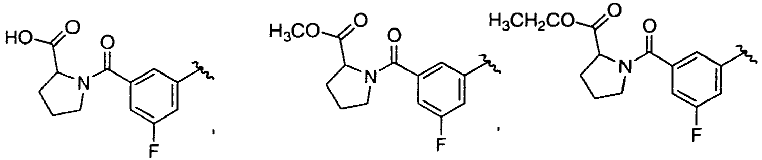 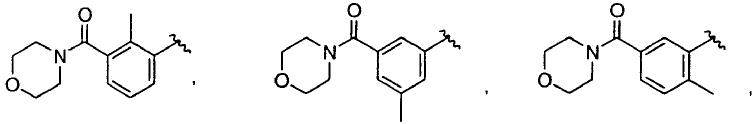 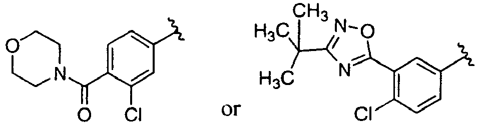 B is 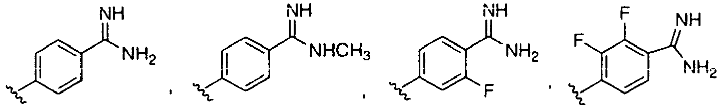 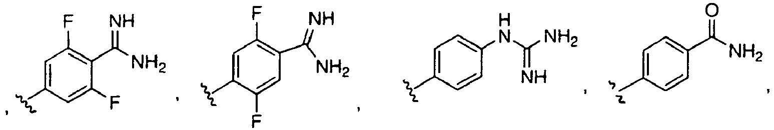 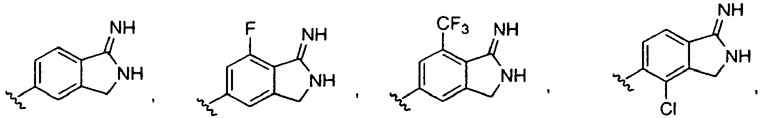 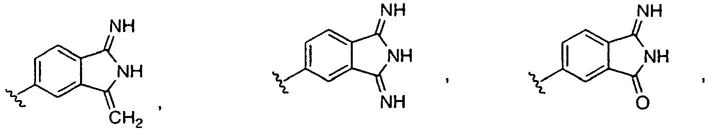 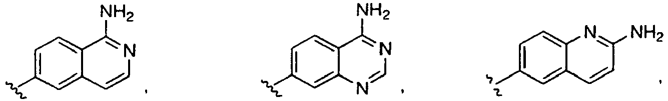 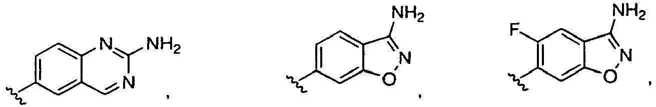 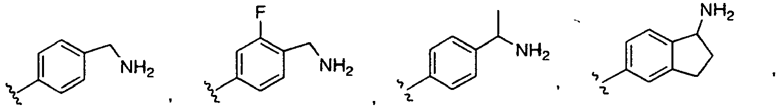 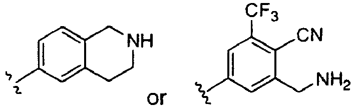 provided that
when A is 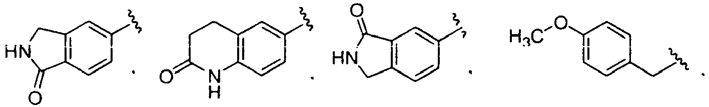 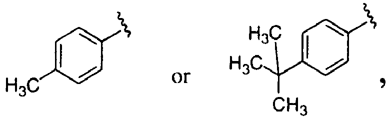 B is not 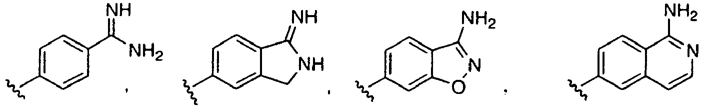 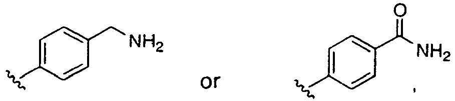 and
when A is 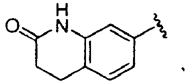 B is not

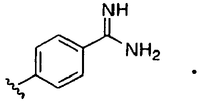

In another aspect of the invention, the compounds are of the absolute configuration of Formula (I) is

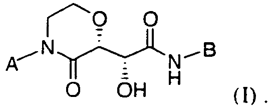

In another aspect of the invention, the compound is
(2R)-2-[(2R)-4-(4-tert-butylphenyl)-3-oxomorpholin-2-yl]-2-hydroxy-N-[4-(N-methylcarbamimidoyl)phenyl]acetamide,
(2R)-2-hydroxy-N-(1-imino-2,3-dihydroisoindol-5-yl)-2-[(2R)-3-oxo-4-(1-oxo-2,3-dihydroisoindol-5-yl)morpholin-2-yl]acetamide,
(2R)-2-hydroxy-N-(1-imino-2,3-dihydroisoindol-5-yl)-2-[(2R)-3-oxo-4-(2-oxo-1H-quinolin-6-yl)morpholin-2-yl]acetamide,
(2R)-2-[(2R)-4-[7-(difluoromethoxy)-2-oxo-1H-quinolin-6-yl]-3-oxomorpholin-2-yl]-2 -hydroxy-N-(1-imino-2,3-dihydroisoindol-5-yl)acetamide,
(2R)-N-(4-carbamimidoylphenyl)-2-hydroxy-2-[(2R)-3-oxo-4-(2-oxo-1H-quinolin-6-yl )morpholin-2-yl]acetamide,
(2R)-N-(4-carbamimidoyl-3-fluorophenyl)-2-hydroxy-2-[(2R)-3-oxo-4-(2-oxo-1H-quinolin-6-yl)morpholin-2-yl]acetamide,
(2R)-2-hydroxy-N-(1-imino-2,3-dihydroisoindol-5-yl)-2-[(2R)-3-oxo-4-(2-oxo-1H-quinolin-7-yl)morpholin-2-yl]acetamide,
(2R)-2-hydroxy-N-(1-imino-2,3-dihydroisoindol-5-yl)-2-[(2R)-4-(1-methyl-2-oxoquinolin-7-yl)-3-oxomorpholin-2-yl]acetamide,
(2R)-2-[(2R)-4-[1-(cyclopropylmethyl)-2-oxoquinolin-7-yl]-3-oxomorpholin-2-yl]-2-hydroxy-N-(1-imino-2,3-dihydroisoindol-5-yl)acetamide,
2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetate,
2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetic acid,
(2R)-2-hydroxy-N-(1-imino-2,3-dihydroisoindol-5-yl)-2-[(2R)-4-(8-methyl-2-oxo-1H-quinolin-7-yl)-3-oxomorpholin-2-yl]acetamide,
(2R)-2-hydroxy-N-(1-imino-2,3-dihydroisoindol-5-yl)-2-[(2R)-4-(2-methoxyquinolin-7 -yl)-3-oxomorpholin-2-yl]acetamide,
(2R)-2-hydroxy-N-(1-imino-2,3-dihydroisoindol-5-yl)-2-[(2R)-3-oxo-4-quinolin-7-ylmorpholin-2-yl]acetamide,
(2R)-N-(3-amino-1,2-benzisoxazol-6-yl)-2-hydroxy-2-[(2R)-3-oxo-4-quinolin-7-ylmorpholin-2-yl]acetamide,
(2R)-2-hydroxy-N-(1-imino-2,3-dihydroisoindol-5-yl)-2-[(2R)-3-oxo-4-(2-oxo-1H-quinolin-5-yl)morpholin-2-yl]acetamide,
(2R)-2-hydroxy-N-(1-imino-2,3-dihydroisoindol-5-yl)-2-[(2R)-3-oxo-4-(1-oxo-3,4-dihydro-2H-isoquinolin-6-yl)morpholin-2-yl]acetamide,
(2R)-2-hydroxy-N-(1-imino-2,3-dihydroisoindol-5-yl)-2-[(2R)-3-oxo-4-(2-oxo-3,4-dihydro-1H-quinolin-7-yl)morpholin-2-yl]acetamide,
(2R)-2-hydroxy-N-(1-imino-2,3-dihydroisoindol-5-yl)-2-[(2R)-4-(1-methyl-2-oxo-3,4-dihydroquinolin-7-yl)-3-oxomorpholin-2-yl]acetamide,
(2R)-2-[(2R)-4-[1-(cyclopropylmethyl)-2-oxo-3,4-dihydroquinolin-7-yl]-3-oxomorpholin-2-yl]-2-hydroxy-N-(1-imino-2,3-dihydroisoindol-5-yl)acetamide,
methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquinolin-1-yl]acetate,
2-[7-[(2R)-2-[(1R)-1-hydroxy-2-[(1-imino-2,3-dihydroisoindol-5-yl)amino]-2-oxoethyl ]-3-oxomorpholin-4-yl]-2-oxo-3,4-dihydroquinolin-1-yl]acetic acid,
(2R)-N-(4-carbamimidoyl-3-fluorophenyl)-2-hydroxy-2-[(2R)-3-oxo-4-(2-oxo-3,4-dihydro-1H-quinolin-7-yl)morpholin-2-yl]acetamide,
(2R)-N-(3-amino-1,2-benzisoxazol-6-yl)-2-hydroxy-2-[(2R)-3-oxo-4-(2-oxo-3,4-dihydro-1H-quinolin-7-yl)morpholin-2-yl]acetamide
(2R)-N-(1-aminoisoquinolin-6-yl)-2-hydroxy-2-[(2R)-3-oxo-4-(2-oxo-3,4-dihydro-1H-quinolin-7-yl)morpholin-2-yl]acetamide,
(2R)-N-(4-aminoquinazolin-7-yl)-2-hydroxy-2-[(2R)-3-oxo-4-(2-oxo-3,4-dihydro-1H-quinolin-7-yl)morpholin-2-yl]acetamide,
(2R)-2-hydroxy-N-(1-imino-2,3-dihydroisoindol-5-yl)-2-[(2R)-3-oxo-4-(2-oxo-3,4-dihydro-1H-quinolin-5-yl)morpholin-2-yl]acetamide,
(2R)-2-hydroxy-2-[(2R)-4-[2-(3-hydroxy-3-methylbutoxy)phenyl]-3-oxomorpholin-2-yl]-N-(1-imino-2,3-dihydroisoindol-5-yl)acetamide,
(2R)-2-hydroxy-N-(1-imino-2,3-dihydroisoindol-5-yl)-2-[(2R)-4-[2-(3-methoxy-3-methylbutoxy)phenyl]-3-oxomorpholin-2-yl]acetamide,
(2R)-2-hydroxy-N-(1-imino-2,3-dihydroisoindol-5-yl)-2-[(2R)-3-oxo-4-[2-(2-oxopiperidin-1-yl)phenyl]morpholin-2-yl]acetamide,
(2R)-2-[(2R)-4-[2-(difluoromethoxy)-5-fluorophenyl]-3-oxomorpholin-2-yl]-2-hydroxy -N-(1-imino-2,3-dihydroisoindol-5-yl)acetamide,
methyl 2-(diftuoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoate,
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]benzoic acid,
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]-N,N-dimethylbenzamide,
2-(difluoromethoxy)-3-[2-[(1-hydroxy-2-[(1-imino-2,3-dihydroisoindol-5-yl)amino]-2-oxoethyl]-3-oxomorpholin4-yl]benzamide,
methyl 2-[(2R)-2-[(1R)-1-hydroxy-2-[(1-imino-2,3-dihydroisoindol-5-yl)amino]-2-oxoethyl]-3-oxomorpholin-4-yl]-5-(trifluoromethoxy)benzoate,
2-[(2R)-2-[(1R)-1-hydroxy-2-[(-imino-2,3-dihydroisoindol-5-yl)amino]-2-oxoethyl-3 -oxomorpholin-4-yl]-5-(trifluoromethoxy)benzoic acid,
2-[(2R)-2-[(1R)-1-hydroxy-2-[(1-imino-2,3-dihydroisoindol-5-yl)amino]-2-oxoethyl]-3 -oxomorpholin-4-yl]-5-(trifluoromethoxy)benzamide,
(2R)-N-(3-amino-1,2-benzisoxazol-6-yl)-2-hydroxy-2-[(2R)-3-oxo-4-(2-oxo-1H-quinol in-6-yl)morpholin-2-yl]acetamide,
(2R)-N-(3-amino-1,2-benzisoxazol-6-yl)-2-hydroxy-2-[(2R)-3-oxo-4-(2-oxo-1H-quinol in-7-yl)morpholin-2-yl]acetamide, or
(2R)-N-(3-amino-1,2-benzisoxazol-6-yl)-2-hydroxy-2-[(2R)-3-oxo-4-(2-oxo-1H-quinol in-5-yl)morpholin-2-yl]acetamide,
or a pharmaceutically acceptable salt or a solvate thereof.

In another embodiment, the compound is
(2R)-2-[(2R)-(4-tert-butylphenyl)-3-oxomorpholin-2-yl]-2-hydroxy-N-[4-(N-methyle arbamimidoyl)phenyl]acetamide hydrochloride (Example aa1),
(2R)-2-hydroxy-N-(1-imino-2,3-dihydroisoindol-5-yl)-2-[(2R)-3-oxo-4-(1-oxo-2,3-dih ydroisoindol-5-yl)morpholin-2-yl]acetamide hydrochloride (Example aa2),
(2R)-2-hydroxy-N-(1-imino-2,3-dihydroisoindol-5-yl)-2-[(2R)-3-oxo-4-(2-oxo-1H-qui nolin-6-yl)morpholin-2-yl]acetamide hydrochloride (Example aa3),
(2R)-2-[(2R)-4-[7-(difluoromethoxy)-2-oxo-1H-quinolin-6-yl]-3-oxomorpholin-2-yl]-2 -hydroxy-N-(1-imino-2,3-dihydroisoindol-5-yl)acetamide hydrochloride (Example aa4),
(2R)-N-(4-carbamimidoylphenyl)-2-hydroxy-2-[(2R)-3-oxo-4-(2-oxo-1H-quinolin-6-yl )morpholin-2-yl]acetamide hydrochloride (Example aa5).
(2R)-N-(4-carbamimidoyl-3-fluorophenyl)-2-hydroxy-2-[(2R)-3-oxo-4-(2-oxo-1H-quin olin-6-yl)morpholin-2-yl]acetamide hydrochloride (Example aa6),
(2R)-2-hydroxy-N-(1-imino-2,3-dihydroisoindol-5-yl)-2-[(2R)-3-oxo-4-(2-oxo-1H-qui nolin-7-yl)morpholin-2-yl]acetamide hydrochloride (Example aa7),
(2R)-2-hydroxy-N-(-imino-2,3-dihydroisoindol-5-yl)-2-[(2R)-4-(1-methyl-2-oxoquino lin-7-yl)-3-oxomorpholin-2-yl]acetamide hydrochloride (Example aa8),
(2R)-2-[(2R)-4-[1-(cyclopropylmethyl)-2-oxoquinolin-7-yl]-3-oxomorpholin-2-yl]-2-h ydroxy-N-(-imino-2,3-dihydroisoindol-5-yl)acetamide hydrochloride (Example aa9),
2-[7-[(2R)-2-[(1R)-1-hydroxy-2-[(1-imino-2,3-dihydroisoindol-5-yl)amino]-2-oxoethyl [-3-oxomorpholin-4-yl]-2-oxoquinolin-1-yl]acetate hydrochloride (Example aa10),
2-[7-[(2R)-2-[(1R)-1-hydroxy-2-[(1-imino-2,3-dihydrolsoindol-5-yl)amino]-2-oxoethyl ]-3-oxomorpholin-4-yl]-2-oxoquinolin-1-yl]acetic acid hydrochloride (Example aa11),
(2R)-2-hydroxy-N-(-imino-2,3-dihydroisoindol-5-yl)-2-[(2R)-4-(8-methyl-2-oxo-1H-quinolin-7-yl)-3-oxomorpholin-2-yl]acetamide hydrochloride (Example aa12),
(2R)-2-hydroxy-N-(1-imino-2,3-dihydroisoindol-5-yl)-2-[(2R)-4-(2-methoxyquinolin-7 -yl)-3-oxomorpholin-2-yl]acetamide hydrochloride (Example aa13),
(2R)-2-hydroxy-N-(1-imino-2,3-dihydroisoindol-5-yl)-2-[(2R)-3-oxo-4-quinolin-7-ylm orpholin-2-yl]acetamide dihydrochloride (Example aa14),
(2R)-N-(3-amino-1,2-benzisoxazo)-6-yl)-2-hydroxy-2-[(2R)-3-oxo-4-quinolin-7-ylmorpholin-2-yl]acetamide hydrochloride (Example aa15),
(2R)-2-hydroxy-N-(1-imino-2,3-dihydroisoindol-5-yl)-2-[(2R)-3-oxo-4-(2-oxo-1H-qui nolin-5-yl)morpholin-2-yl]acetamide hydrochloride (Example aa16),
(2R)-2-hydroxy-N-(1-imino-2,3-dihydroisoindol-5-yl)-2-[(2R)-3-oxo-4-(1-oxo-3,4-dih ydro-2H-isoquinolin-6-yl)morpholin-2-yl]acetamide hydrochloride (Example aa17),
(2R)-2-hydroxy-N-(1-imino-2,3-dihydroisoindol-5-yl)-2-[(2R)3-oxo-4-(2-oxo-3,4-dih ydro-1H-quinolin-7-yl)morpholin-2-yl]acetamide hydrochloride (Example aa18),
(2R)-2-hydroxy-N-(1-imino-2,3-dihydroisoindol-5-yl)-2-[(2R)-4-(1-methyl-2-oxo-3,4-dihydroquinolin-7-yl)-3-oxomorpholin-2-yl]acetamide hydrochloride (Example aa19),
(2R)-2-[(2R)-4-[1-(cyclopropylmethyl)-2-oxo-3,4-dihydroquinolin-7-yl]-3-oxomorphol in-2-yl]-2-hydroxy-N-(1-imino-2,3-dihydroisoindol-5-yl)acetamide hydrochloride (Example aa20),
methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquinolin-1-yl]acetate hydrochloride (Example aa21),
2-[7-[(2R)-2-[(1R)-1-hydroxy-2-[(1-imino-2,3-dihydroisoindol-5-yl)amino]-2-oxoethyl ]-3-oxomorpholin-4-yl]-2-oxo-3,4-dihydroquinolin-1-yl]acetic acid hydrochloride (Example aa22),
(2R)-N-(4-carbamimidoyl-3-fluorophenyl)-2-hydroxy-2-[(2R)-3-oxo-4-(2-oxo-3,4-dihy dro-1H-quinolin-7-yl)morpholin-2-yl]acetamide hydrochloride (Example aa23),
(2R)-N-(3-amino-1,2-benzoxazol-6-yl)-2-hydroxy-2-[(2R)-3-oxo-4-(2-oxo-3,4-dihydro -1H-quinolin-7-yl)morpholin-2-yl]acetamide (Example aa24),
(2R)-N-(1-aminoisoquinolin-6-yl)-2-hydroxy-2-[(2R)-3-oxo-4-(2-oxo-3,4-dihydro-1H-quinolin-7-yl)morpholin-2-yl]acetamide hydrochloride (Example aa25),
(2R)-N-(4-aminoquinazolin-7-yl)-2-hydroxy-2-[(2R)-3-oxo-4-(2-oxo-3,4-dihydro-1H-q uinolin-7-yl)morpholin-2-yl]acetamide (Example aa26),
(2R)-2-hydroxy-N-(1-imino-2,3-dihydroisoindol-5-yl)-2-[(2R)-3-oxo-4-(2-oxo-3,4-dih ydro-1H-quinolin-5-yl)morpholin-2-yl]acetamide hydrochloride (Example aa27),
(2R)-2-hydroxy-2-[(2R)-4-[2-(3-hydroxy-3-methylbutoxy)phenyl]-3-oxomorpholin-2-y l]-N-(1-imino-2,3-dihydroisoindol-5-yl)acetamide hydrochloride (Example aa28),
(2R)-2-hydroxy-N-(1-imino-2,3-dihydroisoindol-5-yl)-2-[(2R)-4-[2-(3-methoxy-3-met hylbutoxy)phenyl]-3-oxomorpholin-2-yl]acetamide hydrochloride (Example aa29),
(2R)-2-hydroxy-N-(1-imino-2,3-dihydroisoindol-5-yl)-2-[(2R)-3-oxo-4-[2-(2-oxopiperi din-1-yl)phenyl]morpholin-2-yl]acetamide hydrochloride (Example aa30),
(2R)-2-[(2R)-4-[2-(difluoromethoxy)-5-fluorophenyl]-3-oxomorpholin-2-yl]-2-hydroxy -N-(1-imino-2,3-dihydroisoindol-5-yl)acetamide hydrochloride (Example aa31),
methyl 2-(difluoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoate hydrochloride (Example aa32),
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]benzoic acid hydrochloride (Example aa33),
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]-N,N-dimethylbenzamide hydrochloride (Example aa34),
2-(difluoromethoxy)-3-[2-[(1-hydroxy-2-[(1-imino-2,3-dihydroisoindol-5-yl)amino]-2-oxoethy]-3-oxomorpholin-4-yl]benzamide hydrochloride (Example aa35),
methyl 2-[(2R)-2-[(1R)-1-hydroxy-2-[(1-imino-2,3-dihydroisoindol-5-yl)amino]-2-oxoethyl]-3-oxomorpholin-4-yl]-5-(trifluoromethoxy)benzoate hydrochloride (Example aa36),
2-[(2R)-2-[(1R)-1-hydroxy-2-[(1-imino-2,3-dihydroisoindol-5-yl)amino]-2-oxoethyl]-3 -oxomorpholin-4-yl]-5-(trifluoromethoxy)benzoic acid hydrochloride (Example aa37), or
2-[(2R)-2-[(1R)-1-hydroxy-2-[(1-imino-2,3-dihydroisoindol-5-yl)amino]-2-oxoethyl]-3 -oxomorpholin-4-yl]-5-(trifluoromethoxy)benzamide hydrochloride (Example aa38), or a solvate thereof.

In another embodiment, the compound is
2-(3-((R)-2-((R)-2-(4-carbamimidoylphenylamino)-1-hydroxy-2-oxoethyl)-3-oxomorp holino)phenyl)acetate (Example aa40),
(R)-N-(4-carbamimidoylphenyl)-2-hydroxy-2-((R)-4-(3-methyl-5-(pyrrolidine-1-carbo nyl)phenyl)-3-oxomorpholin-2-yl)acetamide (Example aa53),
(R)-2-hydroxy-N-(1-iminoisoindolin-5-yl)-2-((R)-4-(3-methyl-5-(pyrrolidine-1-carbon yl)phenyl)-3-oxomorpholin-2-yl)acetamide (Example aa54),
2-(3-((R)-2-((R)-1-hydroxy-2-(1-iminoisoindolin-5-ylamino)-2-oxoethyl)-3-oxomorph olino)-N-methylbenzamido)acetic acid (Example aa50),
2-(3-((R)-2-((R)-1-hydroxy-2-(1-iminoisoindolin-5-ylamino)-2-oxoethyl)-3-oxomorph olino)-N-methylbenzamido)acetate (Example aa51),
(S)-1-(3-((R)-2-((R)-2-(4-carbamimidoylphenylamino)-1-hydroxy-2-oxoethyl)-3-oxom orpholino)benzoyl)pyrrolidine-2-carboxylic acid (Example aa58),
1-(3-((R)-2-((R)-2-(4-carbamimidoylphenylamino)-1-hydroxy-2-oxoethyl)-3-oxomorp holino)benzoyl)pyrrolidine-2-carboxylate (Example aa52),
3-((R)-2-((R)-2-(4-carbamimidoylphenylamino)-1-hydroxy-2-oxoethyl)-3-oxomorpholi no)-N-methyl-N-(2-oxo-2-(pyrrolidin-1-yl)ethyl)benzamide (Example aa56),
3-((R)-2-((R)-1-hydroxy-2-(1-iminoisoindolin-5-ylamino)-2-oxoethyl)-3-oxomorpholin o)-N-methyl-N-(2-oxo-2-(pyrrolidin-1-yl)ethyl)benzamide (Example aa59),
(R)-N-(4-carbamimidoylphenyl)-2-hydroxy-2-((R)-4-(3-methyl-5-(morpholine-4-carbo nyl)phenyl)-3-oxomorpholin-2-yl)acetamide (Example aa60),
(R)-2-hydroxy-N-(1-iminoisoindolin-5-yl)-2-((R)-4-(3-methyl-5-(morpholine-4-carbon yl)phenyl)-3-oxomorpholin-2-yl)acetamide (Example aa61),
(R)-2-hydroxy-N-(1--iminoisoindolin-5-yl)-2-((R)-4-(3-((R)-3-methoxypyrrolidine-1-ca rbonyl)phenyl)-3-oxomorpholin-2-yl)acetamide (Example aa63),
(R)-2-hydroxy-N-(1-iminoisoindolin-5-yl)-2-((R)-4-(3-((S)-3-methoxypyrrolidine-1-ca rbonyl)phenyl)-3-oxomorpholin-2-yl)acetamide (Example aa64),
(R)-2-((R)-4-(3-chloro-4-(morpholine-4-carbonyl)phenyl)-3-oxomorpholin-2-yl)-2-hyd roxy-N-(1-iminoisoindolin-5-yl)acetamide (Example aa66),
(R)-2-hydroxy-N-(1-iminoisoindolin-5-yl)-2-((R)-4-(3-(2-morpholino-2-oxoethyl)phen yl)-3-oxomorpholin-2-yl)acetamide (Example aa68),
(S)-1-(3-((R)-2-((R)-1-hydroxy-2-(1-iminoisoindolin-5-ylamino)-2-oxoethyl)-3-oxomo rpholino)benzoyl)pyrrolidine-2-carboxylic acid (Example aa55),
1-(3-((R)-2-((R)-1-hydroxy-2-(1-iminoisoindolin-5-ylamino)-2-oxoethyl)-3-oxomorph olino)benzoyl)pyrrolidine-2-carboxylate (Example aa57),
2-(3-((R)-2-((R)-1-hydroxy-2-(1-iminoisoindolin-5-ylamino)-2-oxoethyl)-3-oxomorph olino)phenyl)acetate (Example aa65),
2-(3-((R)-2-((R)-1-hydroxy-3-(1-iminoisoindolin-5-ylamino)-2-oxoethyl)-3-oxomorph olino)phenyl)acetic acid (Example aa67),
2-(3-((R)-2-((R)-1-hydroxy-2-(1-iminoisoindolin-5-ylamino)-2-oxoethyl)-3-oxomorph olino)-N-methylbenzamido)acetate (Example aa62),
2-(3-((R)-2-((R)-1-hydroxy-2-(1-iminoisoindolin-5-ylamino)-2-oxoethyl)-3-oxomorph olino)phenyl)-2-methylpropanoate (Example aa70),
(R)-2-hydroxy-N-(1-iminoisoindolin-5-yl)-2-((R)-4-(3-(morpholine-4-carbonyl)phenyl) -3-oxomorpholin-2-yl)acetamide (Example aa69),
3-((R)-2-((R)-1-hydroxy-2-(1-iminoisoindolin-5-ylamino)-2-oxoethyl)-3-oxomorpholin o)-N-methyl-N-(2-morpholino-2-oxoethyl)benzamide (Example aa71),
(R)-2-hydroxy-N-(1-iminoisoindolin-5-yl)-2-((R)-4-(3-(2-((R)-3-methoxypyrrolidin-1-yl)-2-oxoethyl)phenyl)-3-oxomorpholin-2-yl)acetamide (Example aa72),
(R)-2-hydroxy-N-(1-iminoisoindolin-5-yl)-2-((R)-4-(3-(2-((S)-3-methoxypyrrolidin-1-yl)-2-oxoethyl)phenyl)-3-oxomorpholin-2-yl)acetamide (Example aa73),
2-(3-((R)-2-((R)-1-hydroxy-2-(1-iminoisoindolin-5-ylamino)-2-oxoethyl)-3-oxomorph olino)phenyl)-2-methylpropanoate (Example aa74),
2-(3-((R)-2-((R)-1-hydroxy-2-(1-iminoisoindolin-5-ylamino)-2-oxoethyl)-3-oxomorph olino)phenyl)acetate (Example aa75),
(R)-2-hydroxy-N-(1-iminoisoindolin-5-yl)-2-((R)-4-(3-(4-methyl-3-oxopiperazine-1-ca rbonyl)phenyl)-3-oxomorpholin-2-yl)acetamide (Example aa76),
2-(3-((R)-2-((R)-1-hydroxy-2-(1-iminoisoindolin-5-ylamino)-2-oxoethyl)-3-oxomorph olino)phenyl)-2-methylpropanoate (Example aa77),
(R)-2-hydroxy-N-(1-iminoisoindolin-5-yl)-2-((R)-4-(3-(2-(4-methyl-3-oxopiperazin-1-yl)-2-oxoethyl)phenyl)-3-oxomorpholin-2-yl)acetamide (Example aa79),
(R)-2-((R)-4-(3-(2-(dimethylamino)-2-oxoethyl)phenyl)-3-oxomorpholin-2-yl)-2-hydro xy-N-(1-iminoisoindolin-5-yl)acetamide (Example aa82),
1-(3-((R)-2-((R)-1-hydroxy-2-(1-iminoisoindolin-5-ylamino)-2-oxoethyl)-3-oxomorph olino)benzoyl)piperidine-4-carboxylic acid (Example aa83),
1-(3-((R)-2-((R)-1-hydroxy-2-(1-iminoisoindolin-5-ylamino)-2-oxoethyl)-3-oxomorph olino)benzoyl)piperidine-4-carboxylate (Example aa84),
2-(3-((R)-2-((R)-1-acetoxy-2-(1-imino-3-oxoisoindolin-5-ylamino)-2-oxoethyl)-3-oxo morpholino)-N-methylbenzamido)acetic acid (Example aa85),
(R)-N-(1,3-diiminoisoindolin-5-yl)-2-((R)-4-(3-(2-(dimethylamino)-2-oxoethyl)phenyl) -3-oxomorpholin-2-y))-2-hydroxyacetamide (Example aa86),
(R)-2-(1-imino-3-oxoisoindolin-5-ylamino)-1-((R)-4-(3-(methyl(2-oxo-2-(pyrrolidin-1-yl)ethyl)carbamoyl)phenyl)-3-oxomorpholin-2-yl)-2-oxoethyl acetate (Example aa87),
(R)-N-(3-aminobenzo[d]isoxazol-6-yl)-2-((R)-4-(4-fluoro-3-(morpholine-4-carbonyl)p henyl)-3-oxomorpholin-2-yl)-2-hydroxyacetamide ((Example aa95),
(R)-N-(-aminobenzo[d]isoxazol-6-yl)-2-((R)-4-(4-chloro-3-((4,4-difluoropiperidin-1-y 1)methyl)phenyl)-3-oxomorpholin-2-yl)-2-hydroxyacetamide (Example aa97),
(R)-N-(3-aminobenzo[d]isoxazol-6-yl)-2-hydroxy-2-((R)-3-oxo-4-(3-(3-oxomorpholin o)phenyl)morpholin-2-yl)acetamide (Example aa98),
(R)-N-(3-aminobenzo[d]isoxazol-6-yl)-2-((R)-4-(2-(difluoromethoxy)phenyl)-3-oxomo rpholin-2-yl)-2-hydroxyacetamide (Example aa99),
(R)-N-(3-aminobenzo[d]isoxazol-6-yl)-2-hydroxy-2-((R)-4-(2-isopropoxyphenyl)-3-ox omorpholin-2-yl)acetamide (Example aa100),
(R)-N-(3-aminobenzo[d]isoxazol-6-yl)-2-((R)-4-(3-cyanophenyl)-3-oxomorpholin-2-yl )-2-hydroxyacetamide (Example aa101),
(R)-N-(3-aminobenzo[d]isoxazol-6-yl)-2-((R)-4-(4-chloro-3-(morpholine-4-carbonyl)p henyl)-3-oxomorpholin-2-yl)-2-hydroxyacetamide (Example aa103),
(R)-N-(3-aminobenzo[d]isoxazol-6-yl)-2-hydroxy-2-((R)-4-(4-methyl-3-(morpholine-4 -carbonyl)phenyl)-3-oxomorpholin-2-yl)acetamide (Example aa104),
(R)-N-(3-aminobenzo[d]isoxazol-6-yl)-2-hydroxy-2-((R)-3-oxo-4-(3-(trifluoromethoxy )phenyl)morpholin-2-yl)acetamide (Example aa105),
(R)-N-(3-aminobenzo[d]isoxazol-6-yl)-2-hydroxy-2-((R)-3-oxo-4-(3-(trifluoromethyl)p henyl)morpholin-2-yl)acetamide (Example aa106),
(R)-N-(3-aminobenzo[d]isoxazol-6-yl)-2-((R)-4-(4-chloro-3-(1,2,3,4-tetrahydroisoquin oline-2-carbonyl)phenyl)-3-oxomorpholin-2-yl)-2-hydroxyacetamide (Example aa107),
(R)-N-(3-aminobenzo[d]isoxazol-6-yl)-2-((R)-4-(4-chloro-3-(4,4-difluoropiperidine-1-carbonyl)phenyl)-3-oxomorpholin-2-yl)-2-hydroxyacetamide (Example aa108),
(R)-N-(3-aminobenzo[d]isoxazol-6-yl)-2-((R)-4-(3-fluoro-4-(trifluoromethyl)phenyl)-3 -oxomorpholin-2-yl)-2-hydroxyacetamide (Example aa109),
(R)-N-(4-carbamimidoyl-3-fluorophenyl)-2-hydroxy-2-((R)-3-oxo-4-(3-(3-oxomorphol ino)phenyl)morpholin-2-yl)acetamide (Example aa114),
(R)-2-hydroxy-N-(1-imino-7-(trifluoromethyl)isoindolin-5-yl)-2-((R)-3-oxo-4-(3-(3-ox omorpholino)phenyl)morpholin-2-yl)acetamide (Example aa116),
(R)-2-Hydroxy-N-(1-iminoisoindolin-5-yl)-2-((R)-3-oxo-4-(3-(trifluoromethoxy)pheny 1)morpholin-2-yl)acetamide (Example aa117),
Ethyl 2-(3-((R)-2-((R)-1-hydroxy-2-(1-iminoisoindolin-5-ylamino)-2-oxoethyl)-3-oxomorph olino)phenoxy)acetate (Example aa120),
(R)-2-Hydroxy-N-(1-iminoisoindolin-5-yl)-2-((R)-4-(4-methoxybenzyl)-3-oxomorphol in-2-yl)acetamide (Example aa121),
(R)-2-((R)-4-(3-(Benzyloxy)phenyl)-3-oxomorpholin-2-yl)-2-hydroxy-N-(1-iminoisoin dolin-5-yl)acetamide (Example aa123),
(R)-N-(1-Aminoisoquinolin-6-yl)-2-((R)-4-(4-fluoro-3-(morpholine-4-carbonyl)phenyl )-3-oxomorpholin-2-yl)-2-hydroxyacetamide (Example aa132),
N-(2,3-Dihydro-1-imino-1h-isoindol-5-yl)-4-[2-[(dimethylamino)carbonyl]phenyl]-alp ha(R)-hydroxy-3-oxo-2(R)-morpholineacetamide ((Example aa140),
N-(2,3-Dihydro-1-imino-1H-isoindol-5-y)-alpha(R)-hydroxy-3-oxo-4-[2-(1-pyrrolidin ylcarbonyl)phenyl]-2(R)-morpholineacetamide ((Example aa141),
4-(2-Cyanophenyl)-N-(2,3-dihydro-1-imino-1h-isoindol-5-yl)-alpha(R)-hydroxy-3-oxo -2(R)-morpholineacetamide ((Example aa142),
N-(2,3-Dihydro-1-imino-1h-isoindol-5-yl)-4-[4-fluoro-3-(4-morpholinylcarbonyl)phen yl]-alpha(R)-hydroxy-3-oxo-2(R)-morpholineacetamide (Example aa144),
N-(2,3-Dihydro-1-imino-1h-isoindol-5-yl)-4-[4-fluoro-3-[(3(R)-methyl-4-morpholinyl) carbonyl]phenyl]-alpha(R)-hydroxy-3-oxo-2(R)-morpholineacetamide(Example aa145),
N-(2,3-Dihydro-1-imino-1h-isoindol-5-yl)-4-[4-fluoro-3-[(3(S)-methyl-4-morpholinyl) carbonyl]phenyl]-alpha(R)-hydroxy-3-oxo-2(R)-morpholineacetamide (Example aa146),
N-[4-(Aminoiminomethyl)phenyl]-4-(2-cyanophenyl)-alpha(R)-hydroxy-3-oxo-2(R)-m orpholineacetamide (Example aa147),
N-(4-Amino-7-quinazolinyl)-4-[4-fluoro-3-(4-morpholinylcarbonyl)phenyl]-alpha(R)-h ydroxy-3-oxo-2(R)-morpholineacetamide (Example aa148),
[3-[2(R)-[2-[(3-Amino-1,2-benzisoxazol-6-yl)amino]-1(R)-hydroxy-2-oxoethyl]-3-oxo-4-morpholinyl]phenyl]pentafluorosulfur (Example aa150),
[4-[2(R)-[2-[(3-Amino-1,2-benzisoxazol-6-yl)amino]-1(R)-hydroxy-2-oxoethyl]-3-oxo-4-morpholinyl]phenyl]pentafluorosulfur (Example aa151),
N-(3-Amino-5-fluoro-1,2-benzisoxazol-6-yl)-4-[4-fluoro-3-(4-morpholinylcarbonyl)ph enyl]-alpha(R)-hydroxy-3-oxo-2(R)-morpholineacetamide (Example aa152),
N-(2-Amino-6-quinazolinyl)-4-[4-fluoro-3-(4-morpholinylcarbonyl)phenyl]-alpha(R)-h ydroxy-3-oxo-2(R)-morpholineacetamide ((Example aa153),
(R)-2-((R)-4-(5-fluoro-2-isopropoxyphenyl)-3-oxomorpholin-2-yl)-2-hydroxy-N-(1-imi noisoindolin-5-yl)acetamide (Example aa154),
(R)-2-hydroxy-N-(1-iminoisoindolin-5-yl)-2-((R)-3-oxo-4-(2-(trifluoromethyl)phenyl) morpholin-2-yl)acetamide (Example aa155),
(R)-2-((R)-4-(2-(difluoromethoxy)phenyl)-3-oxomorpholin-2-yl)-2-hydroxy-N-(1-imin oisoindolin-5-yl)acetamide (Example aa156),
(R)-2-hydroxy-N-(1-iminoisoindolin-5-yl)-2-((R)-4-(2-isopropoxyphenyl)-3-oxomorph olin-2-yl)acetamide (Example aa157),
(R)-2-hydroxy-N-(1-iminoisoindolin-5-yl)-2-((R)-3-oxo-4-(2-(trifluoromethoxy)phenyl )morpholin-2-yl)acetamide ((Example aa159),
N-(3-Amino-1,2-benzisoxazol-6-yl)-4-[4-fluoro-3-[(tetrahydro-2H-pyran-4-yl)oxy]phe nyl]-alpha(R)-hydroxy-3-oxo-2(R)-morpholineacetamide ((Example aa160),
N-(3-Amino-1,2-benzisoxazol-6-yl)-4-[3-[(3,3-difluoro-1-azetidinyl)carbonyl]-4-fluoro phenyl[-alpha(R)-hydroxy-3-oxo-2(R)-morpholineacetamide ((Example aa162),
N-(3-Amino-1,2-benzisoxazol-6-yl)-4-[3-[(4,4-difluoro-1-piperidinyl)carbonyl]-4-fluor ophenyl]-alpha(R)-hydroxy-3-oxo-2(R)-morpholineacetamide (Example aa163),
N-(3-amino-1,2-benzisoxazol-6-yl)-4-[4-chloro-3-[3-(1,1-dimethylethyl)-1,2,4-oxadiaz ol-5-yl]phenyl]-alpha(R)-hydroxy-3-oxo-2(R)-morpholineacetamide (Example aa164),
N-(3-amino-1,2-benzisoxazol-6-yl)-4-[2-fluoro-5-(trifluoromethyl)phenyl]-alpha(R)-hy droxy-3-oxo-2(R)-morpholineacetamide ((Example aa165),
or a solvate thereof.

In another embodiment, the compound is
(R)-N-(4-carbamimidoylphenyl)-2-hydroxy-2-((R)-4-(3-((S)-2-methylpyrrolidine-1-car bonyl)phenyl)-3-oxomorpholin-2-yl)acetamide (Example aa39),
(R)-N-(4-carbamimidoylphenyl)-2-hydroxy-2-((R)-4-(2-methyl-3-(morpholine-4-carbo nyl)phenyl)-3-oxomorpholin-2-yl)acetamide (Example aa41),
(R)-N-(4-carbamimidoylphenyl)-2-hydroxy-2-((R)-4-(2-methyl-3-(pyrrolidine-1-carbo nyl)phenyl)-3-oxomorpholin-2-yl)acetamide (Example aa42),
(R)-2-((R)-4-(3-(azetidine-1-carbonyl)-5-fluorophenyl)-3-oxomorpholin-2-yl)-N-(4-car bamimidoylphenyl)-2-hydroxyacetamide (Example aa43),
(R)-2-((R)-4-(3-(azetidine-1-carbonyl)-2-methylphenyl)-3-oxomorpholin-2-yl)-N-(4-ca rbamimidoylphenyl)-2-hydroxyacetamide (Example aa44),
(R)-2-((R)-4-(5-(azetidine-1-carbonyl)-2-methylphenyl)-3-oxomorphol in-2-yl)-N-(4-ca rbamimidoylphenyl)-2-hydroxyacetamide (Example a45),
(R)-N-(4-carbamimidoylphenyl)-2-hydroxy-2-((R)-3-oxo-4-(6-(trifluoromethyl)pyridin -2-yl)morpholin-2-yl)acetamide (Example aa46),
(R)-N-(4-carbamimidoylpheny1)-2-hydroxy-2-((R)-4-(2-methyl-5-(pyrrolidine-1-carbo nyl)phenyl)-3-oxomorpholin-2-yl)acetamide (Example aa47),
(R)-2-((R)-4-(3-(azetidine-1-carbonyl)-5-methylphenyl)-3-oxomorphol in-2-yl)-N-(4-ca rbamimidoylphenyl)-2-hydroxyacetamide (Example aa48),
(R)-N-(4-carbamimidoylphenyl)-2-hydroxy-2-((R)-4-(2-methyl-5-(morpholine-4-carbo nyl)phenyl)-3-oxomorpholin-2-yl)acetamide (Example aa49),
(S)-1-(3-((R)-2-((R)-2-(4-carbamimidoylphenylamino)-1-hydroxy-2-oxoethyl)-3-oxom orpholino)-5-fluorobenzoyl)pyrrolidine-2-carboxylic acid (Example aa78),
1-(3-((R)-2-((R)-2-(4-carbamimidoylphenylamino)-1-hydroxy-2-oxoethyl)-3-oxomorp holino)-5-fluorobenzoyl)pyrrolidine-2-carboxylate (Example aa80),
1-(3-((R)-2-((R)-2-(4-carbamimidoylphenylamino)-1-hydroxy-2-oxoethyl)-3-oxomorp holino)-5-fluorobenzoyl)pyrrolidine-2-carboxylate (Example aa81),
2-(3-((R)-2-((R)-2-(4-carbamimidoylphenylamino)-1-hydroxy-2-oxoethyl)-3-oxomorp holino)-5-fluoro-N-methylbenzamido)acetic acid (Example aa88),
2-(3-((R)-2-((R)-2-(4-carbamimidoylphenylamino)-1-hydroxy-2-oxoethyl)-3-oxomorp holino)-5-fluoro-N-methylbenzamido)acetate (Example aa89),
3-((R)-2-((R)-2-(4-carbamimidoylphenylamino)-1-hydroxy-2-oxoethyl)-3-oxomorpholi no)-N-(2-(dimethylamino)-2-oxoethyl)-5-fluoro-N-methylbenzamide (Example aa90),
(R)-N-(1,3-diiminoisoindolin-5-yl)-2-hydroxy-2-((R)-4-(3-(2-(4-methyl-3-oxopiperazin -1-yl)-2-oxoethyl)phenyl)-3-oxomorpholin-2-yl)acetamide (Example aa91),
N-(2-(dimethylamino)-2-oxoethyl)-3-((R)-2-((R)-1-hydroxy-2-(1-iminoisoindolin-5-yla mino)-2-oxoethyl)-3-oxomorpholino)-N-methylbenzamide (Example aa92),
(R)-N-(3-aminobenzo[d]isoxazol-6-yl)-2-((R)-4-(4-chloro-3-(morpholinomethyl)pheny 1)-3-oxomorpholin-2-yl)-2-hydroxyacetamide (Example aa96),
(R)-N-(3-aminobenzo[d]isoxazol-6-yl)-2-hydroxy-2-((R)-4-(3-(morpholine-4-carbonyl) phenyl)-3-oxomorpholin-2-yl)acetamide (Example aa102),
3-((R)-2-((R)-2-(4-carbamimidoyl-3-fluorophenylamino)-1-hydroxy-2-oxoethyl)-3-oxo morpholino)benzoic acid (Example aa110),
(R)-N-(4-carbamimidoyl-3,5-difluorophenyl)-2-hydroxy-2-((R)-3-oxo-4-(3-(3-oxomor pholino)phenyl)morpholin-2-yl)acetamide (Example aa111),
(R)-N-(4-carbamimidoyl-2,3-difluorophenyl)-2-hydroxy-2-((R)-3-oxo-4-(3-(3-oxomor pholino)phenyl)morpholin-2-yl)acetamide (Example aa112),
(R)-N-(4-carbamimidoyl-2,5-difluorophenyl)-2-hydroxy-2-((R)-3-oxo-4-(3-(3-oxomor pholino)phenyl)morpholin-2-yl)acetamide (Example aa113),
(R)-N-(3-(aminomethyl)-4-cyano-5-(trifluoromethyl)phenyl)-2-hydroxy-2-((R)-3-oxo-4-(3-(3-oxomorpholino)pheny)morpholin-2-yl)acetamide (Example aa115),
(R)-2-Hydroxy-N-(1-iminoisoindolin-5-yl)-2-((R)-4-(2-methyl-1-oxoisoindolin-5-yl)-3 -oxomorpholin-2-yl)acetamide (Example aa118),
(R)-2-Hydroxy-N-(1-iminoisoindolin-5-yl)-2-((R)-4-(2-methyl-3-oxoisoindolin-5-yl)-3 -oxomorpholin-2-yl)acetamide hydrochloride (Example aa119),
(R)-2-((R)-4-(2-(Cyclopropylmethyl)-3-oxoisoindolin-5-yl)-3-oxomorpholin-3-yl)-3-hy droxy-N-(1-iminoisoindolin-5-yl)acetamide (Example aa124),
(R)-N-(4-(Aminomethyl)-3-fluorophenyl)-2-hydroxy-2-((R)-3-oxo-4-(3-(3-oxomorphol ino)phenyl)morpholin-2-yl)acetamide (Example aa125),
(R)-N-(4-Guanidinophenyl)-2-hydroxy-2-((R)-3-oxo-4-(3-(3-oxomorpholino)phenyl)m orpholin-2-yl)acetamide (Example aa126),
(R)-N-(4-(Aminomethyl)phenyl)-2-((R)-4-(4-fluoro-3-(morpholine-4-carbonyl)phenyl) -3-oxomorpholin-2-yl)-2-hydroxyacetamide (Example aa127),
(R)-2-(4-Carbamoylphenylamino)-1-((R)-4-(4-fluoro-3-(morpholine-4-carbonyl)phenyl )-3-oxomorpholin-2-yl)-2-oxoethyl acetate (Example aa128),
(R)-2-((R)-4-(4-Fluoro-3-(morpholine-4-carbonyl)phenyl)-3-oxomorpholin-2-yl)-2-hyd roxy-N-(1.2,3,4-tetrahydroisoquinolin-6-yl)acetamide (Example aa129),
(R)-N-(4-(Aminomethyl)-3-fluorophenyl)-2-((R)-4-(4-fluoro-3-(morpholine-4-carbonyl )phenyl)-3-oxomorpholin-2-yl)-2-hydroxyacetamide (Example aa130),
(R)-N-(4-((R)-1-Aminoethyl)phenyl)-2-((R)-4-(4-fluoro-3-(morpholine-4-carbonyl)phe nyl)-3-oxomorpholin-2-yl)-2-hydroxyacetamide (Example aa131),
(R)-N-((R)-1-amino-2,3-dihydro-1H-inden-5-yl)-2-((R)-4-(4-fluoro-3-(morpholine-4-c arbonyl)phenyl)-3-oxomorpholin-2-yl)-2-hydroxyacetamide (Example aa133),
(R)-N-((S)-1-Amino-2,3-dihydro-1H-inden-5-yl)-2-((R)-4-(4-fluoro-3-(morpholine-4-c arbonyl)phenyl)-3-oxomorpholin-2-yl)-2-hydroxyacetamide (Example aa134),
(R)-N-(2-Aminoquinolin-6-yl)-2-((R)-4-(4-fluoro-3-(morpholine-4-carbonyl)phenyl)-3 -oxomorpholin-2-yl)-2-hydroxyacetamide (Example aa135),
N-(7-fluoro-2,3-dihydro-1-imino-1H-isoindol-5-yl)-alpha(R)-hydroxy-3-oxo-4-[3-(3-o xo-4-morpholinyl)phenyl]-2(R)-morpholineacetamide (Example aa136),
N-(4-chloro-2,3-dihydro-1-imino-1H-isoindol-5-yl)-alpha(R)-hydroxy-3-oxo-4-[3-(3-o xo-4-morpholinyl)phenyl]-2(R)-morpholineacetamide (Example aa137),
3-[2(R)-[2-[(2,3-Dihydro-1-imino-1h-isoindol-5-yl)amino]-1(R)-hydroxy-2-oxoethyl]-3-oxo-4-morpholinyl]benzoic acid (Example aa138),
Methyl 3-[2(R)-[2-[(2,3-dihydro-1-imino-1H-isoindol-5-yl)amino]-1(R)-hydroxy-2-oxoethyl]-3-oxo-4-morpholinyl]benzoate (Example aa139),
Methyl 2-[2(R)-[2-[(2,3-dihydro-1-imino-1h-isoindol-5-yl)amino]-1(R)-hydroxy-2-oxoethyl]-3-oxo-4-morpholinyl]benzoate (Example aa143),
N-(3-Amino-1,2-benzisoxazol-6-yl)-4-[4-fluoro-3-[2,2,2-trifluoro-1-(4-morpholinyl)eth yl]phenyl]-alpha(R)-hydroxy-3-oxo-2(R)-morpholineacetamide (Example aa149),
(R)-2-((R)-4-(5-chloro-2-isopropoxyphenyl)-3-oxomorpholin-2-yl)-2-hydroxy-N-(1-im inoisoindolin-5-yl)acetamide (Example aa158),
N-(3-Amino-1,2-benzisoxazol-6-yl)-4-[3-(difluoromethoxy)-4-fluorophenyl]-alpha(R)-hydroxy-3-oxo-2(R)-morpholineacetamide (Example aa161),
(2R)-N-(3-amino-1,2-benzoxazol-6-yl)-2-hydroxy-2-[(2R)-3-oxo-4-(2-oxo-1H-quinolin -6-yl)morpholin-2-yl]acetamide.
(2R)-N-(3-amino-1,2-benzoxazol-6-yl)-2-hydroxy-2-[(2R)-3-oxo-4-(2-oxo-1H-quinolin -7-yl)morpholin-2-yl]acetamide,
(2R)-N-(3-amino-1,2-benzoxazol-6-yl)-2-hydroxy-2-[(2R)-3-oxo-4-(2-oxo-1H-quinolin -5-yl)morpholin-2-yl]acetamide,
or a solvate thereof.

The invention is also the compound
2-((S)-hydroxy((R)-3-oxo-4-p-tolylmorpholin-2-yl)methyl)-4-oxo-1,4-dihydroquinazoline-6-carboximidamide (Example aa93), or
1,1-dioxo-3-((S)-hydroxy((R)-3-oxo-4-p-tolylmorpholin-2-yl)methyl)-4H-benzole][1,2, 4]thiadiazine-7-carboximidamide (Example aa94) or a pharmaceutically acceptable salt or a solvate thereof.

As used above, and throughout this disclosure, the following terms, unless otherwise indicated, shall be understood to have the following meanings:
The term "(Cₐ-C_{b})-alkyl", in which a and b is each independently integers representing 1 to 6, is understood as meaning hydrocarbon radicals whose carbon chain are each straight-chain or branched and contains a to b carbon atoms, for example methyl, ethyl, propyl, isopropyl, butyl, isobutyl, tertiary-butyl, pentyl, isopentyl, neopentyl, hexyl, 2,3-dimethylbutyl or neohexyl.
The term "-(C₀-C₄)-alkylene" is understood as meaning a bond or hydrocarbon radicals whose carbon chain is straight-chain or branched and contains I to 4 carbon atoms, for example methylene, ethylene, propylene, isopropylene, isobutylene, butylene or tertiary-butylene. "-C₀-alkylene" is a covalent bond. The term "-(C₁-C₄)-alkylene" is understood as meaning hydrocarbon radicals whose carbon chain is straight-chain or branched and contains 1 to 4 carbon atoms. for example methylene (-CH₂-), ethylene (-CH₂-CH₂-), (-CH₂(CH₃)-), propylene (-CH₂-CH₂-CH₂-), isopropylene, isobutylene, butylene or tertiary-butylene.
The term "-(C₃-C₁₂)-cycloalkyl" is understood as meaning rings of 3 to 12 carbon atoms such as compounds which partially have monocycles having 3 to 8 carbon atoms in the ring such as cyclopropane, cyclobutane, cyclopentane, cyclohexane, cycloheptane or cyclooctane, which are derived from the bicycles bicyclo[4,2,0]octane, octahydroindene, decahydronaphthalene, decahydroazulene, decahydrobenzocycloheptene or dodecahydroheptalene or from the bridged cycles such as spiro[2,5]octane, spiro[3,4]octane, spiro[3,5]nonane, bicyclo[3,1,1]heptane, bicyclo[2,2,1]heptane or bicyclo[2,2,2]octane.
The term "-(C₃-C₈)-cycloalkyl" is understood as meaning radicals which are derived from monocycles having 3 to 8 carbon atoms in the ring such as cyclopropane, cyclobutane, cyclopentane, cyclohexane, cyclo-heptane or cyclooctane.
The term "-(C₆-C₁₄)-aryl" is understood as meaning aromatic hydrocarbon radicals having 6 to 14 carbon atoms in the ring. -(C₆-C₁₄)-aryl radicals are, for example, phenyl, 1-naphthyl, 2-naphthyl, anthryl or fluorenyl. Naphthyl radicals and in particular phenyl radicals are preferred aryl radicals.
The term "three- to fifteen-membered heterocyclic ring" is understood as meaning ring systems having 3 to 15 carbon atoms, which are present in one, two or three ring systems connected to one another and in which one, two, three or four identical or different heteroatoms from the group consisting of oxygen, nitrogen or sulfur can replace the respective carbon atoms.

One of the examples of "three- to fifteen-membered heterocyclic ring" is a bicyclic ring system represented by Formula (a). In the bicyclic ring system represented by Formula (a); 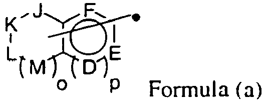 wherein Formula (a) is unsubstituted or substituted independently with one to four Y; and wherein:
o and p are independently selected from 0 or 1;
J, K, L and M are independently selected from the group consisting of CH2, C(O), NH, O and S(O)q, wherein q is 0, 1 or 2;
D. E and F are independently selected from the group consisting of carbon atom, nitrogen atom, oxygen atom and sulfur atom.

Examples of three- to fifteen-membered heterocyclic ring are the radicals acridinyl, azepinyl, azetidinyl, aziridinyl, benzimidazolinyl, benzimidazolyl, benzisoxazole, benzofuranyl, benzothiofuranyl, benzothiophenyl, benzoxazolyl, benzothiazolyl, benzotriazolyl, benzotetrazolyl, benzisoxazolyl, benzisothiazolyl, carbazolyl, 4H-carbazolyl, carbolinyl, beta-carbolinyl, chromanyl, chromenyl, cinnolinyl, deca-hydroquinolinyl, dibenzofuranyl, dibenzothiophenyl, dihydrofuran [2,3-b]-tetrahydrofuranyl,dihydrofuranyl, 1,1-dioxido-2H-1,2,4-benzothiadiazinyl, dioxolyl, dioxanyl, dioxolenyl, 2H, 6H-1,5,2-dithiazinyl, furanyl, furazanyl, imidazolidinyl, imidazolinyl, imidazolyl, 1H-indazolyl, indolinyl, indolizinyl, indolyl, isobenzofuranyl, isoquinolinyl, isochromanyl, isoindazolyl, isoindolinyl, isoindolyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxiranyl, oxothiolanyl, phenanthridinyl, phenanthrenyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridothiophenyl, pyridyl, pyrimidinyl, pyrrolidinyl, pyrrolinyl, pyrrolyl, 2H-pyrrolyl, quinazolinyl, quinolinyl, quinolizinyl, 4H-quinolizinyl, quinoxalinyl, quinuclidinyl, tetrahydrofuranyl, tetrahydroisoquinolinyl, tetrahydroquinolinyl, tetrahydropyridinyl, tetrazolyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazinyl, thiazolyl, thienyl, thienoimidazolyl, thienooxazolyl, thienopyridinyl, thienopyrrolyl, thienothiazolyl, thienothiophenyl, thiomorpholinyl, thiopyranyl, triazinyl, 1,2,3-triazolyl, 1,2,4-triazolyl, 1,2,5-triazolyl, 1,3,4-triazolyl, xanthenyl, oxindolyl, benzimidazolinyl, benzoxalonyl, 1,3-dihydro-benzisothiazolyl, 3,4-dihydro-2,3-benzothiazinyl, 2,3-dihydro-isoindolyl, 1,4-dihydro-isoquinolinonyl, 3,4-dihydro-quinolinonyl or 3,4-dihydro-benzothiadiazinyl.

The term "-(C₁-C₃)-haloalkyl" is understood as meaning a partially or completely fluorinated or chlorinated alkyl radical which is selected, for example, from the following radical -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂ or -CF₂-CF₂-CH₂F.

The term or "halogen" is understood as meaning fluorine, chlorine, bromine or iodine; fluorine, chlorine or bromine is preferred, in particular fluorine or chlorine is preferred.

Functional groups of the intermediates used, for example amino or carboxyl groups, can be masked here by suitable protective groups. Suitable protective groups for amino functions are, for example, para methoxy benzyl, benzyl, t-butoxycarbonyl, benzyloxycarbonyl, phthalolyl, trityl or tosyl protective group. Suitable protective groups for the carboxyl function are, for example, alkyl, aryl or arylalkyl esters. Protective groups can be introduced and removed by techniques which are well-known or described here (see Greene, T.W., et. al., Protective Groups in Organic Synthesis (2007), 4th Ed., Wiley, New York. or Kocienski, P., Protecting Groups (1994), Thieme). The term protective group can also include polymer-bound protective groups. Such masked compounds according to Formula (I), in which the functional groups can optionally also be masked, can, although optionally themselves not pharmacologically active, optionally be converted after administration to mammals by metabolization to the pharmacologically active compounds according to the invention.

When any variable occurs more than one time in any constituent or in Formula (I), its definition on each occurrence is independent of its definition at every other occurrence.

As used herein, the term "composition" is intended to encompass a product comprising the specified ingredients in the specified amounts, as well as any product which results, directly or indirectly, from combination of the specified ingredients in the specified amounts.

Compounds having Factor IXa antagonistic activity (determined by, for example, pharmacological examples described below: a measurement of fluorescence value using microtiter plate reader, ARVO 1420 Multilabel Counter) of 30 µM or less, preferably 1 µM or less, more preferably 100 nM or less, and the most preferably 50 nM or less in terms of an IC50 value are preferably used.

In the embodiments described in this description, "agent" or "drug" means a material which is used for improvement of disease or symptom, not only for treatment of disease or symptom.

In all the above embodiments, when the term "compound" is used, the term also refers to pharmaceutically acceptable salts thereof. The compounds of the present invention have asymmetric carbon atoms. Accordingly, the compounds of the present invention include mixtures of various stereoisomers, such as geometrical isomers, tautomers, such as keto- and enol- tautomers, or amidino- and imidino- tautomers, and optical isomers, and isolated isomers, for example, *(R, R), (S, S), (R, S)* and *(S. R)* isomers. (*R, R)* isomer is preferred. The isolation and the purification of such stereoisomers can be performed by those skilled in the art with a known technique such as optical resolution using preferential crystallization or column chromatography, or asymmetric synthesis.

The compounds represented by Formula (I) of the present invention may form acid addition salts. Alternatively, these compounds may form salts with a base according to the type of substituent. These salts are not particularly limited as long as the salts are pharmaceutically acceptable salts. Specific examples of the salts include acid addition salts with a mineral acid such as hydrochloric acid, hydrobromic acid, hydroiodic acid, sulfuric acid, nitric acid, or phosphoric acid; an organic carboxylic acid such as an aliphatic monocarboxylic acid, e.g., formic acid, acetic acid, trifluoroacetic acid (TFA), propionic acid, butyric acid, valeric acid, enanthic acid, capric acid, myristic acid, palmitic acid, stearic acid, lactic acid, sorbic acid, or mandelic acid, an aromatic monocarboxylic acid, e.g., benzoic acid or salicylic acid, an aliphatic dicarboxylic acid, e.g., oxalic acid, malonic acid, succinic acid, fumaric acid, maleic acid, malic acid, or tartaric acid, and an aliphatic tricarboxylic acid e.g., citric acid; an organic sulfonic acid such as an aliphatic sulfonic acid, e.g., methanesulfonic acid, ethanesulfonic acid, trifluoromethanesulfonic acid, or 2-hydroxyethanesulfonic acid, or an aromatic sulfonic acid, e.g., benzenesulfonic acid or p-toluenesulfonic acid; or an acidic amino acid. e.g., aspartic acid or glutamic acid; salts with a metal such as an alkali metal, e.g., sodium or potassium, or an alkaline earth metal, e.g., magnesium or calcium; salts with an organic base such as methylamine, ethylamine, ethanolamine, pyridine, lysine, arginine, or ornithine; and ammonium salts.

These salts can be obtained by a known method, for example, by mixing a compound of the present invention with an equivalent amount and a solution containing a desired acid, base, or the like, and then collecting the desired salt by filtering the salt or distilling off the solvent.

The compounds of the present invention and salts thereof can form solvates with a solvent such as water, ethanol, or glycerol.

The salts of a compound of the present invention include monosalts and di-salts. The compounds of the present invention can form an acid addition salt and a salt with a base at the same time according to the type of substituent of the side chain.

Furthermore, the present invention includes hydrates, pharmaceutically acceptable various solvates, and crystal polymorphism of the compounds represented by Formula (I) of the present invention. The present invention is not limited to the compounds described in examples below and includes all compounds represented by Formula (I) of the present invention and pharmaceutically acceptable salts thereof.

Prodrugs and solvates of the compounds of the invention are also contemplated herein. A discussion of prodrugs is provided in T. Higuchi and V. Stella, Pro-drugs as Novel Delivery Systems, 14, 1987, of the A.C.S. Symposium Series, and in Bioreversible Carriers in Drug Design, 1987, Edward B. Roche, ed., American Pharmaceutical Assosiation and Pergamon Press. The term "prodrug" means a compound (e.g, a drug precursor) that is transformed in vivo to yield a compound of Formula (I) or a pharmaceutically acceptable salt, hydrate or a solvate of the compound. The transformation may occur by various mechanisms (e.g., by metabolic or chemical processes), such as, for example, through hydrolysis in blood. A discussion of the use of prodrugs is provided by T. Higuchi and W. Stella, "Pro-drugs as Nobel Delivery Systems," Vol. 14 of the A.C.S. Symposium Series, and in Bioreversible Carriers in Drug Design, ed. Edward B. Roche, American Pharmaceutical Associatiion and Pergamon Press, 1987.

For example, if a compound of Formula (I) or a pharmaceutically acceptable salt, hydrate or a solvate of the compound contains an alcohol functional group, a prodrug can he formed by the replacement of the hydrogen atom of the alcohol group with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanyl, arylacyl and α-aminoacyl, or α-aminoacyl-α-aminoacyl, where each α-aminoacyl group is independently selected from the naturally occurring L-anino acids, -P(O)(OH)₂, -P(O)(O(C₁-C₆)alkyl)₂ or glycosyl (the radical resulting from the removal of a hydroxyl group of the hemiacetal from of a carbohydrate), and the like.

### Methods for Making Morpholinone Compounds of the Invention

Compounds of the invention can be prepared by procedures described below. Variables used in the described procedures correspond to compounds of the invention included within the scope of Formula (I), 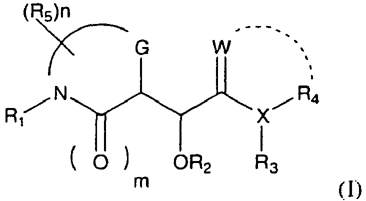 and have the following meanings:
R1 is selected from the group consisting of:
   1) -(C₆-C₁₄)-aryl, which is unsubstituted or substituted independently with one to four Y:
   2) -(three- to fifteen-membered heterocyclic ring), which is unsubstituted or substituted independently with one to four Y;
   3) -(C₆-C₁₄)-aryl-U-(C₆-C₁₄)-aryl, wherein each of said -(C₆-C₁₄)-aryl-independently is unsubstituted or substituted independently with one to four Y;
   4) -(C₆-C₁₄)-aryl-U-(C₃-C₁₂)-cycloalkyl, wherein said -(C₆-C₁₄)-aryl and -(C₃-C₁₂)-cycloalkyl independently are unsubstituted or substituted independently with one to four Y;
   5) -(C₆-C₁₄)-aryl-U-(three- to fifteen-membered heterocyclic ring), wherein said -(C₆-C₁₄)-aryl and said (three- to fifteen-membered heterocyclic ring) are independently unsubstituted or substituted independently with one to four Y;
   6) -(three- to fifteen-membered heterocyclic ring)-U-(C₆-C₁₄)-aryl, wherein said -(C₆-C₁₄)-aryl and said (three- to fifteen-membered heterocyclic ring) are independently unsubstituted or substituted independently with one to four Y;
   7) -(three- to fifteen-membered heterocyclic ring)-U-(three- to fifteen-membered heterocyclic ring), wherein each of said -(three- to fifteen-membered heterocyclic ring)- is independently unsubstituted or substituted independently with one to four Y;
   8) -(three- to fifteen-membered heterocyclic ring)-U-(C₃-C₁₂)-cycloalkyl, wherein said -(C₃-C₁₂)-cycloalkyl, and said -(three- to fifteen-membered heterocyclic ring)- are independently unsubstituted or substituted independently with one to four Y;
   9) -V-(C₆-C₁₄)-aryl, which is unsubstituted or substituted independently with one to four Y; and
   10) -V-(three- to fifteen-membered heterocyclic ring), which is unsubstituted or substituted independently with one to four Y;
R2 is selected from the group consisting of hydrogen atom, -C(O)-R6, -C(O)-O-R6, -C(O)-NH-R6 , -C(O)-N(R6)₂, -P(O)(OR6)₂ and
   -(C₁-C₆)-alkyl, wherein
each R6 independently is selected from the group consisting of hydrogen atom, -(C₁-C₆)-alkyl, -(C₃-C₈)-cycloalkyl, -(C₆-C₁₄)-aryl and a three- to fifteen-membered heterocyclic ring;
R3 is absent, or selected from the group consisting of hydrogen atom and -(C₁-C₄)-alkyl,
R4 is selected from the group consisting of:
   1) -(C₆-C₁₄)-aryl-Z, wherein said Z is a basic nitrogen-containing group and wherein said -(C₆-C₁₄)-aryl is unsubstituted or substituted independently with one to four Y:
   2) -(C₃-C₁₂)-cycloalkyl-Z, wherein said Z is a basic nitrogen-containing group and wherein said -(C₃-C₁₂)-cycloalkyl is unsubstituted or substituted independently with one to four Y;
   3) -(three- to fifteen-membered heterocyclic ring)-Z, wherein said Z is a basic nitrogen-containing group and wherein said (three- to fifteen-membered heterocyclic ring) is unsubstituted or additionally substituted independently with one to four Y:
   4) -(three- to fifteen-membered heterocyclic ring)-U-(three- to fifteen-membered heterocyclic ring) -Z, wherein said Z is a basic nitrogen-containing group and wherein each of said (three- to fifteen-membered heterocyclic ring) is independently unsubstituted or additionally substituted independently with one to four Y;
   5) -(C₆-C₁₄)-aryl, which is unsubstituted or substituted independently with one to four Y;
   6) -(C₃-C₁₂)-cycloalkyl, which is unsubstituted or substituted independently with one to four Y;
   7) -(three- to fifteen-membered heterocyclic ring), which is unsubstituted or substituted independently with one to four Y;,
   8) -(three- to fifteen-membered heterocyclic ring)-U-(three- to fifteen-membered heterocyclic ring), wherein each of said (three- to fifteen-membered heterocyclic ring) is independently unsubstituted or substituted independently with one to four Y;
each R5 independently is selected from the group consisting of:
   1) halogen;
   2) -(C₁-C₆)-alkyl, which is unsubstituted or substituted independently with one to four -(C₁-C₃)-haloalkyl, -N-C(O)-OH, -N-C(O)-(C₁-C₄)-alkyl, or -C(O)OR7;
   3) -(C₁-C₃)-haloalkyl;
   4) -(C₃-C₈)-cycloalkyl;
   5) -OH;
   6) -O-(C₁-C₄)-alkyl;
   7) -O-(C₁-C₃)-haloalkyl;,
   8) -NO,;
   9) -CN;
   10) -N(R7)(R8);
   11) -C(O)-N(R7)(R8);,
   12) -N(R8)-C(O)-R7;
   13) -N(R8)-SO₂-R7;
   14) -SO₂*-*(C₁-C₄)-alkyl;
   15) -SO₂-N(R7)(R8);
   16) -SO₂-(C₁-C₃)-haloalkyl;
   17) -S-(C₁-C₄)-alkyl;
   18) -S-(C₁-C₃)-haloalkyl;
   19) =O (oxo);
   20) -C(O)OR7; and
   21) -C(O)R7
   wherein each of R7 and R8 is independently selected from the group consisting of hydrogen atom, -(C₆-C₁₄)-aryl, -(C₃-C₈)-cycloalkyl, and -(C₁-C₆)-alkyl, wherein said -(C₁-C₆)-alkyl is unsubstituted or substituted with at least one substituent selected from the group consisting of OH, -O-(C₁-C₄)-alkyl, -(C₁-C₃)-fluoroalky, -O-(C₁-C₃)-haloalkyl, C(O)OH, and C(O)O-(C₁-C₆)-alkyl;
each U independently is selected from the group consisting of a covalent bond, -(C₁-C₄)-alkylene, -NH-, -N((C₁-C₄)-alkyl)-, -O-, -SO₂- or -S-,
   wherein said -(C₁-C₄)-alkylene or -(C₁-C₄)-alkyl is unsubstituted or substituted independently with one to four T,
   or wherein geminal hydrogens in said -(C₁-C₄)-alkylene or -(C₁-C₄)-alkyl can be replaced by a (C₃-C₈)-cycloalkyl to form a spiro cyclic ring;
each V independently is selected from the group consisting of -(C₁-C₄)-alkylene, -SO₂-, -C(O)-, -C(O)-NH- and -SO₂-NH-,
   wherein the carbon atom of said -C(O)-NH- or the sulfur atom of said -SO₂-NH- is connected to a nitrogen atom of the morpholinone ring,
   and wherein said -(C₁-C₄)-alkylene is unsubstituted or substituted independently with one to four T,
   or wherein geminal hydrogens in said -(C₁-C₄)-alkylene can be replaced by a (C₃-C₈)-cycloalkyl to form a spiro cyclic ring;
each T independently is selected from the group consisting of:
   1) halogen;
   2) -(C₁-C₆)-alkyl, which is unsubstituted or substituted independently with one to four substituents selected from the group consisting of OH, -O-(C₁-C₄)-alkyl, -(C₁-C₃)-haloalkyl, -O-(C₁-C₃)-haloalkyl, -N-C(O)-OH and -N-C(O)-(C₁-C₄)-alkyl;
   3) -(C₁-C₃)-haloalkyl;
   4) -(C₃-C₈)-cycloalkyl;
   5) -OH;
   6) -O-(C₁-C₄)-alkyl, which is unsubstituted or mono substituted with OH. -O-(C₁-C₄)-alkyl, -(C₁-C₃)-haloalkyl, -O-(C₁-C₃)-haloalkyl, -N-C(O)-OH or -N-C(O)-(C₁-C₄)-alkyl;
   7) -O-(C₁-C₃)-haloalkyl;
   8) -NO₂;
   9) -CN;
   10) -N(R7)(R8);
   11) -C(O)- N(R7)(R8);
   12) -N(R8)-C(O)-R7;
   13) -N(R8)-SO₂-R7;
   14) -SO₂-(C₁-C₄)-alkyl;
   15) -SO₂-N(R7)(R8);
   16) -SO₂-(C₁-C₃)-haloalkyl;
   17) -S-(C₁-C₄)-alkyl;
   18) -S-(C₁-C₃)-haloalkyl;
   19) -(C₁-C₆)-alkyl-N(R7)(R8);
   20) -NH-C(O)-N(R7)(R8);
   21) =O (oxo); and
   22) -C(O)OR7;
   23) -C(O)OR7;
   24) -N-C(O)-OR7
   wherein each of R7 and R8 independently is selected from the group consisting of a hydrogen atom, -(C₃-C₈)-cycloalkyl, halogen and -(C₁-C₆)-alkyl, wherein said -(C₁-C₆)-alkyl is optionally substituted with at least one substituent selected from the group consisting of OH, -O-(C₁-C₄)-alkyl, -(C₁-C₃)-fluoroalky, and -O-(C₁-C₃)-haloalkyl;
G is selected from the group consisting of oxygen atom, imino, sulfur atom, sulfoxide, sulfone and methylene;
W is selected from the group consisting of oxygen atom, nitrogen atom and carbon atom;
X is selected from the group consisting of nitrogen atm, carbon atom and oxygen atom;
Y is selected from the group consisting of:
   1) halogen;
   2) -(C₁-C₆)-alkyl;
   3) -(C₁-C₃)-haloalkyl;
   4) -(C₃-C₈)-cycloalkyl;
   5) -OH;
   6) -O-(C₁-C₆)-alkyl;
   7) -O-(C₁-C₃)-haloalkyl;
   8) -NO,;
   9) -CN;
   10) -N(R7)(R8);
   11) -C(O)-N(R7)(R8);
   12) -N(R8)-C(O)-R7;
   13) -N(R8)-SO₂-R7;
   14) -SO₂-(C₁-C₄)-alkyl;
   15) -SO,-N(R7)(R8);
   16) -SO₂-(C₁-C₃)-haloalkyl;
   17) -S-(C₁-C₄)-alkyl;
   18) -S-(C₁-C₃)-haloalkyl;
   19) -(C₁-C₆)-alkyl-N(R7)(R8);
   20) -N(R8)-C(O)-N(R7)(R8);
   21) =O (oxo);
   22) -SF₅;
   23) -C(O)OR7:
   24) -N-C(O)-OR7
   25) -N(R8)-C(O)-(C₁-C₄)-alkyl-O-(C₁-C₄)-alkyl-(C₆-C₁₄)-aryl, wherein said -(C₆-C₁₄)-aryl is unsubstituted or substituted independently with one to four Y selected from (1) to (24) as set forth above;
   26) -N(R8)-C(O)-(C₁-C₄)-alkyl-O-(C₁-C₄)-alkyl-(three- to fifteen-membered heterocyclic ring), wherein said -(three- to fifteen-membered heterocyclic ring) is unsubstituted or substituted independently with one to four Yselected from (1) to (24) as set forth above;
   wherein said -(C₁-C₄)-alkyl part or -(C₁-C₆)-alkyl part of 2), 6), 14), 17), 19), 25) or 26) of said Y is unsubstituted or substituted independently with one to four T;
   wherein each of R7 and R8 of 10), 11), 12), 13), 15), 19), 20), 23), 24), 25) or 26) of said Y independently is selected from the group consisting of hydrogen atom, -(C₃-C₈)-cycloalkyl, and -(C₁-C₆)-alkyl, wherein said -(C₁-C₆)-alkyl is optionally substituted with OH, -O-(C₁-C₄)-alkyl, -(C₁-C₃)-fluoroalky, -O-(C₁-C₃)-haloalkyl, -C(O)OH, or C(O)O-(C₁-C₆)-alkyl;
m is 0 or 1,
n is 0, 1, 3 or 4,
the linkage between G atom and Nitrogen atom of the substructure (II) 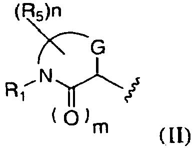
in Formula (I) comprises one to four carbon atoms to form alkylene chain, wherein said alkylene chain or G (imino or methylene) is unsubstituted or substituted independently with one to four R5:
   the dotted linkage between W and R4 of the substructure (III) 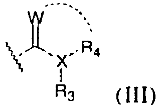
   in Formula (I) is
      1) absent,
      2) present such that substructure (III) is a (three- to fifteen-membered heterocyclic ring)-Z, wherein said Z is a basic nitrogen-containing group and wherein said (three- to fifteen-membered heterocyclic ring) is unsubstituted or additionally substituted independently with one to four Y;
      3) present such that substructure (III) is a (three- to fifteen-membered heterocyclic ring)-U-(three- to fifteen-membered heterocyclic ring) -Z, wherein said Z is a basic nitrogen-containing group and wherein each of said (three- to fifteen-membered heterocyclic ring) is independently unsubstituted or additionally substituted independently with one to four Y;
      4) present such that substructure (III) is a (three- to fifteen-membered heterocyclic ring), wherein said (three- to fifteen-membered heterocyclic ring) is unsubstituted or substituted independently with one to four Y;
      5) present such that substructure (III) is a (three- to fifteen-membered heterocyclic ring)-U-(three- to fifteen-membered heterocyclic ring), wherein each of said (three- to fifteen-membered heterocyclic ring) is independently unsubstituted or substituted independently with one to four Y.

### General Methods

The compounds represented by Formula (I) and salts thereof, which are the compounds of the present invention can be readily produced from known compounds or commercially available compounds by, for example, known processes described in published documents, and produced by production processes described below. The present invention is not limited to the production processes described below.

Unless otherwise noted, R1, R2, R3, R4, R5, G, W, X, m, and n in the formulae shown in the description of the production method are as defined above for the Formula (I). The alkylene group in the side chain or ring of the compound may be substituted with the substituents defined for the Formula (I). R4' and R in the formulae shown in the description of the production method are defined in the corresponding part.

Unless otherwise noted, each of P¹, P², P³, P⁴ or P⁵ in the production method independently designate protecting group, and exemplary appropriate protecting groups include typical an arylmethyl group such as benzyl group, para methoxy benzyl group or triphenylmethyl group; acyl protecting groups, namely, an alkanoyl group such as acetyl group; an alkoxycarbonyl group such as methoxycarbonyl group, ethoxycarbonyl group, or t-butoxycarbonyl group; an arylmethoxycarbonyl group such as benzyloxycarbonyl group, paramethoxybenzyloxycarbonyl group, or para(ortho)nitrobenzyloxycarbonyl group; or an aroyl group such as benzoyl group. The method used for deprotecting such protecting group differs depending on the chemical nature of the protecting group employed, and in the case of an arylmethyl group such as para methoxy benzyl group or benzyl group, the deprotection can be accomplished by hydrogenation using a palladium-carbon catalyst for conversion into nitrogen-hydrogen bond, or alternatively, by Birch reduction using metal sodium in liquid ammonia, or by oxidative condition using such as CAN (ceric ammonium nitrate) or DDQ (2,3-dichloro-5,6-dicyano-p-benzoquinone). The triphenylmethyl group can be removed by using an appropriate acid such as acetic acid, hydrochloric acid, hydrobromic acid, sulfuric acid, phosphoric acid, trifluoroacetic acid, trifluoromethanesulfonic acid, or a combination thereof, or alternatively, or by Birch reduction using metal sodium in liquid ammonia.

In the case of an acyl protecting group such as an alkanoyl group, an alkoxycarbonyl group, or aroyl group, the deprotection can be accomplished by the hydrolysis using an appropriate base such as an alkaline metal hydroxide such as lithium hydroxide, sodium hydroxide, or potassium hydroxide.

The substituted methoxycarbonyl protecting group such as t-butoxycarbonyl group or paramethoxybenzyloxycarbonyl group can be removed by an appropriate acid such as acetic acid, hydrochloric acid, hydrobromic acid, sulfuric acid, phosphoric acid, trifluoroacetic acid, trifluoromethanesulfonic acid, or a combination thereof.

The arylmethoxycarbonyl group such as benzyloxycarbonyl group, paramethoxybenzyloxycarbonyl group, or para(ortho)nitrobenzyloxycarbonyl group can be removed by the hydrolysis using a palladium-carbon catalyst. The protecting groups P¹, P², P³, P⁴ or P⁵ of the imino group (-NH-) can be independently or simultaneously deprotected by adequately selecting the type of the protecting group and deprotection conditions, and if desired, the protecting group can be re-introduced.

Unless otherwise noted, each of L₁, L₂, L₃, L₄, L₅, L₆, L₇ or L₈ in the production method designates a leaving group such as a halogen atom (for example, fluorine, chlorine, bromine, or iodine), methanesulfonyloxy group, or p-toluenesulfonyloxy group, or a replaceable substituent such as hydroxy group or an alkoxy group.

It should be noted that, when the derivative of the Formula (I) of the present invention synthesized has a reactive group such as hydroxy group, amino group, carboxyl group, or thiol group as its substituent, such group may be adequately protected with a protective group in each reaction step and the protective group may be removed at an adequate stage. The process of such introduction and removal of the protective group may he adequately determined depending on the group to be protected and the type of the protective group, and such introduction and removal are conducted, for example, by the process described in the review section of Greene, T.W., et. al., "Protective Groups in Organic Synthesis", 2007, 4th Ed., Wiley, New York, or Kocienski, P., "Protecting Groups" 1994, Thieme. The required starting materials, such as (i-a), (i-b), (i-c), (i-d), (i-e), (i-f), (i-g), (ii-a), (iii-a), (iii-b), (v-b), (viii-a), (viii-b), (viii-c), (viii-d), (viii-e), (ix-a), (xvi-a), or (xvii-a) are either commercially available, or capable of being readily synthesized by the method commonly used in the organic chemistry from commercially available products. Unless otherwise noted, the reaction conditions employed in the production method are as described below:

Reaction temperature is in the range of -78°C to the solvent-reflux temperature, and reaction time is the time sufficient for required progress of the reaction. Solvent which is not involved in the reaction may be any of the aromatic hydrocarbon solvents such as toluene and benzene: polar solvents such as water, methanol, DMF, and DMSO; basic solvents such as triethylamine and pyridine; halogen solvents such as chloroform, methylene chloride, and 1,2-dichloroethane; ethereal solvent such as diethylether, tetrahydrofuran, and dioxane; and mixed solvents thereof; and the solvent used may be adequately selected depending on the reaction conditions. Base may be any of inorganic bases such as potassium carbonate, cesium carbonate, sodium hydroxide, potassium hydroxide, and sodium hydride; and organic bases such as triethylamine, pyridine, N,N-dialkylaniline, lithium diisopropylamide and lithium hexamethyldisilazide; and acid may be any of mineral acids such as hydrochloric acid, and sulfuric acid: and organic acids such as methanesulfonic acid, trifluoroacetic acid and p-toluenesulfonic acid. The base and the acid are not necessarily limited to those mentioned above. The production processes will now be described in detail.

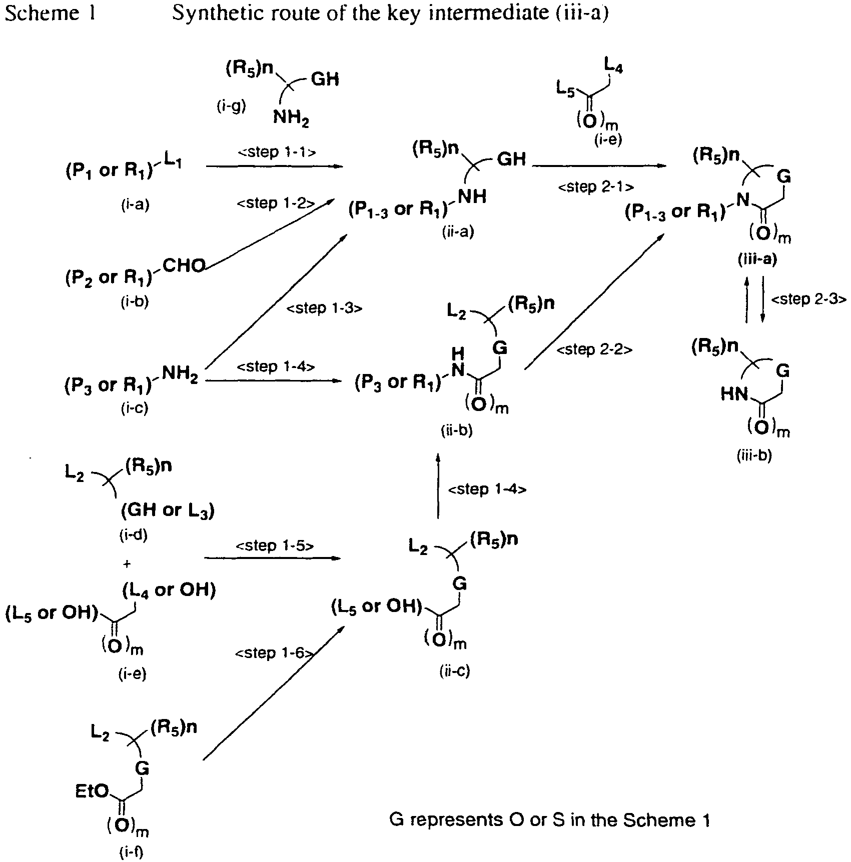

### (Reaction scheme 1)

### <step 1-1>

A compound represented by formula (ii-a) can be produced by allowing a compound represented by formula (i-a) to react with a commercially available aminoalcohol or aminothiol (i-g), readily prepared aminoalcohol or aminothiol from known compounds by a process similar to that described in published documents, for example, Organic Synthesis. Collective Vol.5. pp.88 1973*,* in the presence of a base such as potassium *tert*-buthoxide, sodium hydride, lithium hydroxide, sodium hydroxide, potassium hydroxide, lithium carbonate, sodium carbonate, or potassium carbonate using a solvent which is inactive to the reaction, such as methanol, ethanol, acetone, N,N-dimethylformamide, dioxane or tetrahydrofuran, or a mixed solvent thereof at a temperature in the range of room temperature to the solvent-reflux temperature.

### <step 1-2>

Alternatively, a compound represented by formula (ii-a) can be produced by conducting a reaction using a compound represented by formula (i-b) by a process of reductive amination. After a compound represented by formula (i-b) is converted to an imine with a suitable aminoalcohol (i-g), an aminothiol (i-g) using a solvent which is inactive to the reaction, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0°C to the solvent-reflux temperature, a compound represented by formula (ii-a) can be produced by a process similar to that described in published documents, for example, Journal of Medical Chemistry, 23(12), pp. 1405-1410, 1980 in the presence of a reductive reagent such as sodium borohydride using a solvent which is inactive to the reaction, such as an alcoholic solvent, e.g., methanol, ethanol, 2-propanol, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0°C to the solvent-reflux temperature.

Alternatively, hydrogen gas can be used to an imine with a suitable process similar to that described in published documents, for example, Jikken Kagaku Koza (Experimental Chemistry Series), 4th edition, 26, Organic synthesis VIII, Asymmetric synthesis, reduction, sugar, and labeled compound, pp. 251-266, 1992, Maruzen Co., Ltd., in the presence of a catalyst such as palladium-carbon (Pd-C), Raney-Ni, or platinum oxide (PtO₂) in a solvent which is inactive to the reaction, such as an alcoholic solvent. e.g., methanol, ethanol, or 2-propanol, a halogenated solvent, e.g., dichloromethane or chloroform, an ethereal solvent, e.g., diethyl ether or tetrahydrofuran, a polar solvent, e.g., ethyl acetate or acetonitrile, an aromatic hydrocarbon solvent, e.g., toluene or benzene, or an acid solvent, e.g., acetic acid at a temperature in the range of room temperature to the solvent-retlux temperature, thereby producing a compound represented by formula (ii-a).

### <step 1-3>

Alternatively, a compound represented by formula (ii-a) can be produced by conducting a reaction using a compound represented by formula (i-c) by a process similar to that of <step 1-1> with a suitable alcohol or thiol in the presence of copper iodide and cesium carbonate using a solvent which is inactive to the reaction, such as acetonitrile.

### <step 1-4>

A compound represented by formula (ii-b) can be produced by conducting a reaction using a compound represented by formula (ii-c) and a compound represented by formula (i-c) (for example, a known amine) as follows. When a compound represented by formula (ii-c) is a carboxylic acid, a compound represented by formula (ii-b) can be produced by allowing a compound represented by formula (ii-c) to react with a compound represented by formula (i-c) by a process similar to that described in published documents, for example, Jikken Kagaku Koza (Experimental Chemistry Series), 4th edition, 22, Organic synthesis IV, Acids, amino acids, and peptides, pp. 191-309, 1992. Maruzen Co., Ltd., in the presence of a condensing agent such as 1,3-dicyclohexylcarbodiimide (DCC), 1-ethyl-3-(3'-dimethylaminopropyl)carbodiimide hydrochloride (WSC·HCl), benzotriazol-1-yloxy tris(dimethylamino)phosphonium hexafluorophosphate (BOP reagent), bis(2-oxo-3-oxazolidinyl)phosphinic chloride (BOP-Cl), 2-chloro-1,3-dimethylimidazolinium hexafluorophosphate (CIP), or 4-(4,6-dimethoxy-)-1,3,5-triazin-2-yl)-4-methylmorpholinium chloride, in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, a polar solvent, e.g., N,N-dimethylformamide, or an alcoholic solvent, e.g., methanol, ethanol, or 2-propanol, in the presence or absence of a base such as triethylamine or pyridine at a temperature in the range of 0°C to the solvent-reflux temperature. When a compound represented by formula (ii-c) is an acid halide, a compound represented by formula (ii-b) can be similarly produced by conducting a reaction with a compound represented by formula (i-c) by a process similar to that described in, for example, Jikken Kagaku Koza (Experimental Chemistry Series), 4th edition, 22, Organic synthesis IV, Acids, amino acids, and peptides, pp. 144-146, 1992, Maruzen Co., Ltd., in the presence of a base such as triethylamine or pyridine in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, or a polar solvent, e.g., N,N-dimethylformamide at a temperature in the range of 0°C to the solvent-reflux temperature.

### <step 1-5>

A compound represented by formula (ii-c) can be produced by the same process as that used in <Step 1-1> of (Reaction Scheme 1) using a compound represented by formula (i-d) and compound represented by formula (i-e).

### <step 1-6>

A compound represented by formula (ii-c) can be produced from a compound represented by formula (i-f) by a process similar to that described in published documents, for example, Jikken Kagaku Koza (Experimental Chemistry Series), 4th edition, 22, Organic synthesis IV, Acids, amino acids, and peptides, pp. 1-43, 1992, Maruzen Co.. Ltd., in the presence of a base such as lithium hydroxide, sodium hydroxide, potassium hydroxide, lithium carbonate, sodium carbonate, or potassium carbonate using water and a solvent which is inactive to the reaction, such as methanol, ethanol, 2-propanol. N,N-dimethylformamide, dioxane, or tetrahydrofuran, or a mixed solvent thereof at a temperature in the range of 0°C to the solvent-reflux temperature.

### <step 2-1>

A compound represented by formula (iii-a) can be produced by a process similar to that described in published documents, for example, Zhurnal Organicheskoi Khimii, (6), 1305-8, 1970*,* or by a similar process as that used in <Step 1-4> and <step 1-5> of (Reaction Scheme 1) using a compound represented by formula (i-e) as an acid halide, and compound represented by formula (ii-a) as a suitable aminoalcohol or aminothiol. When aminothiol (ii-a) was used, cyclization process of producing a compound represented by formula (iii-a) can be also conducted step by step process using different base or different solvent system.

### <step 2-2>

Alternatively, a compound represented by formula (iii-a) can be produced by the same process as that used in <Step 2-1> of (Reaction Scheme 1) using a compound represented by formula (ii-b).

### <step 2-3>

Protective groups of a compound represented by formula (iii-a) can be introduced and removed between (iii-a) and (iii-b) hy techniques which are well-known or described here (see Greene, T.W., et. al., Protective Groups in Organic Synthesis (2007), 4th Ed., Wiley, New York, or Kocienski, P., Protecting Groups (1994), Thieme).

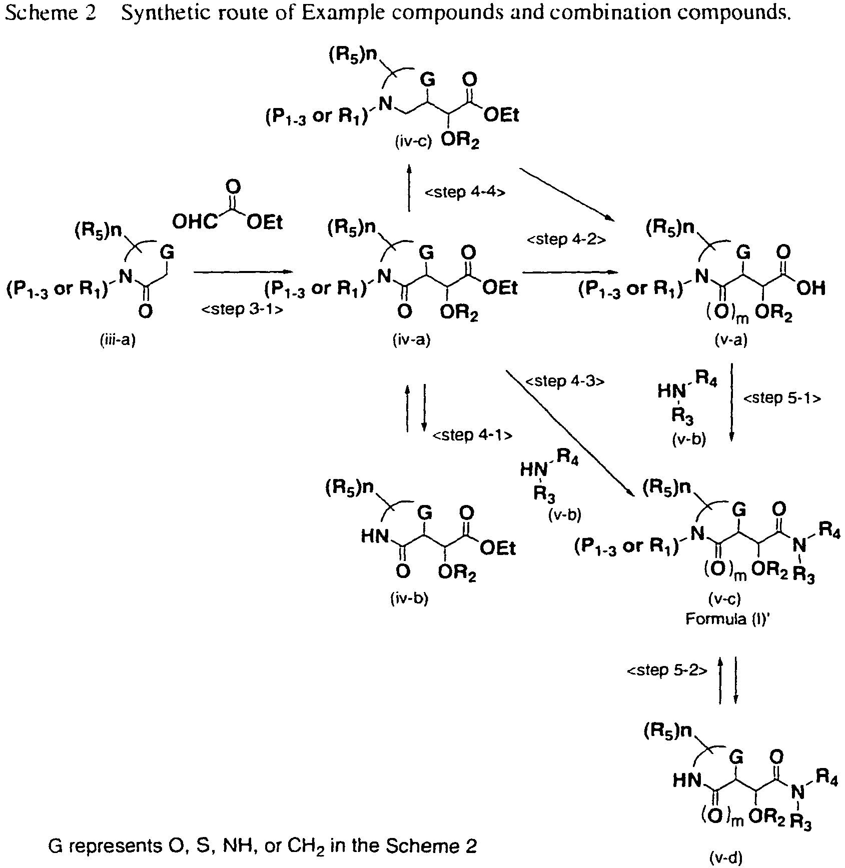

### (Reaction scheme 2)

### <step 3-1>

A compound represented by formula (iv-a) can be produced by allowing a compound represented by formula (iii-a) or (iii-b), which was produced in the Reaction scheme I or commercially available, to react with alkyl glyoxylate, such as ethyl glyoxylate by a process similar to that described in published documents, for example, Journal of Medicinal Chemistry. 31(1), pp. 230-243, 1988*,* in the presence of a base such as lithium hexamethyldisiladide, sodium hydride, lithium hydroxide, sodium hydroxide, potassium hydroxide, lithium carbonate, sodium carbonate, or potassium carbonate using a solvent which is inactive to the reaction, such as tetrahydrofuran. N,N-dimethylformamide, dioxane, or a mixed solvent thereof at a temperature in the range of room temperature to the solvent-reflux temperature. The resulting alcoholic compound (iv-a) wherein R2 represents hydrogen can be protected in any step described hereafter by techniques which are well-known or described here (see Greene, T.W., et. al.. Protective Groups in Organic Synthesis (2007), 4th Ed., Wiley, New York, or Kocienski. P., Protecting Groups (1994), Thieme), to be, for example, alcoxy groups or ester groups.

### <step 4-1>

Protective groups of a compound represented by formula (iv-a) can be introduced and removed between (iv-a) and (iv-b) by techniques which are well-known or described here (see Greene, T.W., et. al., Protective Groups in Organic Synthesis (2007). 4th Ed., Wiley, New York, or Kocienski, P., Protecting Groups (1994), Thieme).

### <step 4-2>

A compound represented by formula (v-a) can be produced by the same process as that used in <Step 1-6> of (Reaction Scheme 1) using a compound represented by formula (iv-a) or (iv-c).

### <step 4-3>

A compound represented by formula (v-c) can be produced by allowing a compound represented by formula (iv-a) to react with a compound represented by formula (v-b) by a process similar to that described in published documents, for example, Organic synthesis IV, Acids, amino acids, and peptides, pp. 191-309, 1992, Maruzen Co.. Ltd., in the presence of a condensing agent, in a solvent which is inactive to the reaction, such as an alcoholic solvent, e.g., methanol, ethanol, or 2-propanol, in the presence or absence of a base such as triethylamine or pyridine at a temperature in the range of 0°C to the solvent-reflux temperature.

### <step 4-4>

A compound represented by formula (iv-c) can be produced by a process similar to that described in published documents, for example, Organic synthesis, Collective Vol. 7, pp.221, 1990*,* Organic synthesis , Collective Vol. 7, pp.530, 1990*,* Jikken Kagaku Koza (Experimental Chemistry Series), 4th edition, 26, Reduction by borane, hydrazine or diimide pp 237-248, using a compound represented by formula (iv-a) in the presence of borane-THF complex, borane-diethyl ether complex, borane-dimethyl sulfide complex, hydradine or hydroxylamine using a solvent such as an ethereal solvent, e.g., diethyl ether or tetrahydrofuran at a temperature in the range of -78°C to the solvent-reflux temperature.

### <step 5-1>

A compound represented by formula (v-c) can be produced by allowing a compound represented by formula (v-a) to react with a compound represented by formula (v-b) by a process similar to that described in published documents, for example, Organic synthesis IV, Acids, amino acids, and peptides, pp. 191-309, 1992, Maruzen Co., Ltd., in the presence of a condensing agent such as 1,3-dicyclohexylcarbodiimide (DCC), 1-ethyl-3-(3'-dimethylaminopropyl)carbodiimide hydrochloride (WSC·HCl), benzotriazol-1-yloxy tris(dimethylamino)phosphonium hexafluorophosphate (BOP reagent), bis(2-oxo-3-oxazolidinyl)phosphinic chloride (BOP-Cl), 2-chloro-1,3-dimethylimidazolinium hexafluorophosphate (CIP), or 4-(4,6-dimethoxy-1,3,5-triazin-2-yl)-4-methylmorpholinium chloride, in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, a polar solvent, e.g., N,N-dimethylformamide, or an alcoholic solvent, e.g., methanol, ethanol, or 2-propanol, in the presence or absence of a base such as triethylamine or pyridine at a temperature in the range of 0°C to the solvent-reflux temperature. When a compound represented by formula (v-a) is converted to an acid halide, a compound represented by formula (v-c) can be similarly produced by conducting a reaction by a process similar to that described in, for example, Organic synthesis IV, Acids, amino acids, and peptides, pp. 144-146, 1992, Maruzen Co., Ltd., in the presence of a base such as triethylamine or pyridine in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, or a polar solvent, e.g., N,N-dimethylformamide at a temperature in the range of 0°C to the solvent-reflux temperature.

Alternatively, a compound represented by formula (v-c) can be produced by using triphosgene by a process similar to that described in published documents, for example, Letters in Organic Chemistry, 4, 20-22, 2007*,* in the presence of a base such as triethyl amine using a solvent which is inactive to the reaction, such as tetrahydrofuran, N,N-dimethylformamide, dioxane, CH2Cl2 or a mixed solvent thereof at a temperature in the range of room temperature to the solvent-reflux temperature.

### <step 5-2>

Protective groups of a compound represented by formula (v-c) can be introduced and removed between (v-c) and (v-d) by techniques which are well-known or described here (see Greene, T.W., et. al., Protective Groups in Organic Synthesis (2007), 4th Ed., Wiley, New York, or Kocienski, P., Protecting Groups (1994), Thieme).

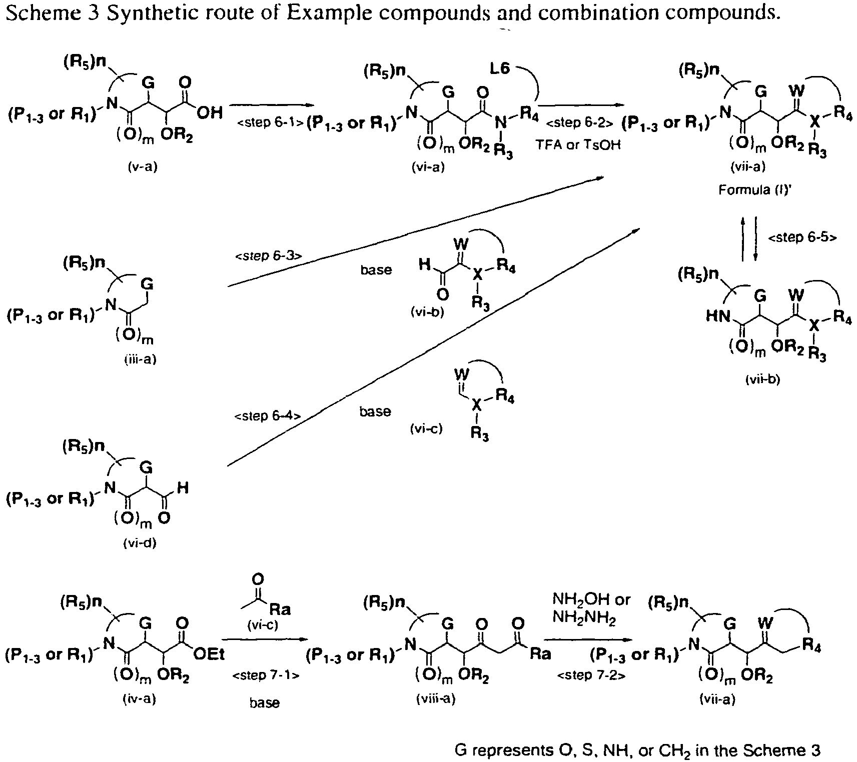

### (Reaction scheme 3)

### <step 6-1>

A compound represented by formula (vi-d) can be produced by the similar process as that used in <Step 5-1> of (Reaction Scheme 2) using a compound represented by formula (v-a).

### <step 6-2>

A compound represented by formula (vii-a) can be produced by allowing a compound represented by formula (vi-a) to react with by a process similar to that described in published documents, for example, Synthetic Communications, 37(24), 2007*,* in the presence of an acid such as acetic acid, trifluoroacetic acid or p-toluenesulfonic acid using a solvent such as acetic acid, trifluoroacetic acid, or a mixed solvent thereof at a temperature in the range of room temperature to the solvent-reflux temperature.

### <step 6-3>

Alternately, a compound represented by formula (vii-a) can be produced by the similar process as that used in <Step 3-1> of (Reaction Scheme 2) using a compound represented by formula (iii-a) and a compound represented by formula (vi-b).

### <step 6-4>

Alternatively, a compound represented by formula (vii-a) can be produced by a process similar to that described in published documents, for example, Bioorganic & Medicinal Chemistry Letters. 17(14), 2007, 3860 using a compound represented by formula (vi-d) and a compound represented by formula (vi-c), wherein Ra represents a suitable substituent..

### <step 6-5>

Protective groups of a compound represented by formula (vi-a) can be introduced and removed between (vii-a) and (vii-b) by techniques which are well-known or described here (see Greene, T.W., et. al., Protective Groups in Organic Synthesis (2007), 4th Ed., Wiley, New York, or Kocienski, P., Protecting Groups (1994), Thieme).

### <step 7-1>

A compound represented by formula (viii-a) can be produced by allowing a compound represented by formula (iv-a) to react with by a process similar to that described in published documents, for example, Organic synthesis, Collective Vol 1, pp.238. 1941. Maruzen Co., Ltd, in the presence of a base such as sodium ethoxide using a solvent such as ethanol at a temperature in the range of room temperature to the solvent-reflux temperature.

Ra of the formula (vi-c) represents a suitable substituent or leaving group of L1 to L4 described above.

### <step 7-2>

A compound represented by formula (vii-a) can be produced by a process similar to that described in published documents, for example, Journal of Medicinal Chemistry, 48, 14, pp4541, 2005*,* using a compound represented by formula (viii-a) in the presence of hydradine or hydroxylamine using a solvent such as ethanol at a temperature in the range of room temperature to the solvent-reflux temperature.

Furthermore, in the case of some of the compounds according to the invention the possibility arises of employing diastereomerically or enantiomerically pure starting products for the preparation of the ring structures. By this means, other or simplified processes can be employed for the purification of the final products. These starting products were prepared beforehand in enantiomerically or diastereomerically pure form according to processes known from the literature. This can mean, in particular, that in the synthesis of the scaffold structures either enantioselective processes are used, or else an enantiomeric (or diastereomeric) separation is carried out at an earlier stage of the synthesis and not only at the stage of the final products. Likewise, a simplification of the separations can be achieved by proceeding in two or more stages.

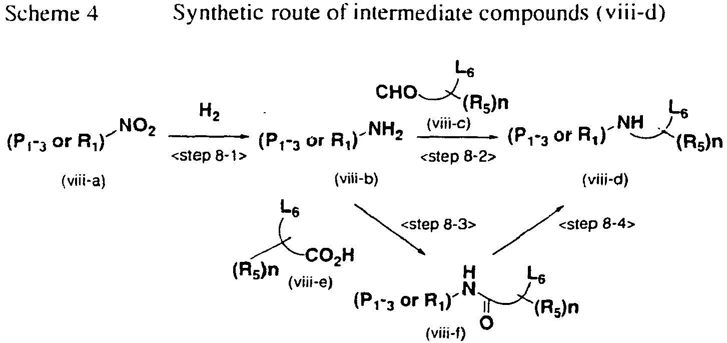

### (Reaction scheme 4)

### <step 8-1>

A compound represented by formula (viii-b) can be produced from (viii-a) by conducting a reaction using the compound represented by formula (viii-a) by a process similar to that described in published documents, for example, Jikken Kagaku Koza (Experimental Chemistry Series). 4th edition, 26, Organic synthesis VIII, Asymmetric synthesis, reduction, sugar, and labeled compound, pp. 159-266, 1992, Maruzen Co., Ltd., in the presence of a catalyst such as palladium-carbon (Pd-C), Raney-Ni, platinum oxide (PtO2), or dichloro triphenyl phosphine ruthenium, under hydrogen atmosphere, using a solvent which is inactive to the reaction, such as an alcoholic solvent, e.g., methanol, ethanol, or 2-propanol, an ethereal solvent, e.g., diethyl ether or tetrahydrofuran, 1,2-dimethoxyethane, or 1,4-dioxane, a polar solvent, e.g., ethyl acetate or methyl acetate, or a mixed solvent thereof at a temperature in the range of room temperature to the solvent-reflux temperature.

Alternatively, a compound represented by formula (viii-b) can be produced from (viii-a) by using Fe, or Sn, in hydrochloric acid or acetic acid, at a temperature in the range of 0°C to the solvent-reflux temperature. Furthermore, a compound represented by formula (viii-b) can also be produced from (viii-a) by using sodium borohydride in the presence of Lewis Acid, e.g., Nickel(II)chloride (NiCl₂), Tin(II)chloride (SnCl₂) using a solvent which is inactive to the reaction, such as an alcoholic solvent, e.g., methanol, ethanol, or 2-propanol, an ethereal solvent. e.g., diethyl ether or tetrahydrofuran, 1.2-dimethoxyethane, or 1,4-dioxane, or a mixed solvent thereof at a temperature in the range of 0°C to the solvent-reflux temperature.

### <step 8-2>

A compound represented by formula (viii-d) can be produced by conducting a reaction using a compound represented by formula (viii-b) by a process of reductive amination. After a compound represented by formula (viii-c) is converted to an imine with a suitable amine (viii-b) using a solvent which is inactive to the reaction, such as an alcoholic solvent, e.g., methanol, ethanol, or 2-propanol, such as a halogenated solvent, e.g., dichloromethane or chloroform, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0°C to the solvent-reflux temperature, a compound represented by formula (viii-d) can be produced by a process similar to that described in published documents, for example, Journal of Medical Chemistry, 23(12), pp. 1405-1410, 1980 in the presence of a reductive reagent such as sodium borohydride or sodium triacetoxy borohydride in the presence of acid such as acetyl alcohol, using a solvent which is inactive to the reaction, such as an alcoholic solvent. e.g., methanol, ethanol, 2-propanol, an ethereal solvent, e.g., diethyl ether, tetrahydrofuran, 1,2-dimethoxyethane, 1,4-dioxane, or an aromatic hydrocarbon solvent, e.g., toluene or benzene or a mixed solvent thereof at a temperature in the range of 0°C to the solvent-reflux temperature.

Alternatively, hydrogen gas can be used to hydrogenate an imine with a suitable process similar to that described in published documents, for example, Jikken Kagaku Koza (Experimental Chemistry Series), 4th edition, 26. Asymmetric synthesis, reduction, sugar, and labeled compound, pp. 251-266, 1992, Maruzen Co., Ltd., in the presence of a catalyst such as palladium-carbon (Pd-C), Raney-Ni, or platinum oxide (PtO₂) in a solvent which is inactive to the reaction, such as an alcoholic solvent, e.g., methanol, ethanol, or 2-propanol, a halogenated solvent, e.g., dichloromethane or chloroform, an ethereal solvent, e.g., diethyl ether or tetrahydrofuran, a polar solvent, e.g., ethyl acetate or acetonitrile, an aromatic hydrocarbon solvent, e.g., toluene or benzene, or an acid solvent. e.g., acetic acid at a temperature in the range of room temperature to the solvent-reflux temperature, thereby producing a compound represented by formula (viii-d).

### <step 8-3>

A compound represented by formula (viii-f) can be produced by the similar process as that used in <Step 5-1> of (Reaction Scheme 2) using a compound represented by formula (viii-e).

### <step 8-4>

A compound represented by formula (viii-d) can be produced by a process similar to that described in published documents, for example, Jikken Kagaku Koza (Experimental Chemistry Series), 4th edition, 26. Reduction by borane, hydrazine or diimide pp 237-248, using a compound represented by formula (viii-f) in the presence of hydradine or hydroxylamine using a solvent such as ethanol at a temperature in the range of room temperature to the solvent-reflux temperature.

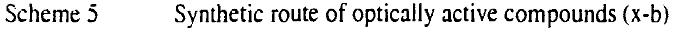

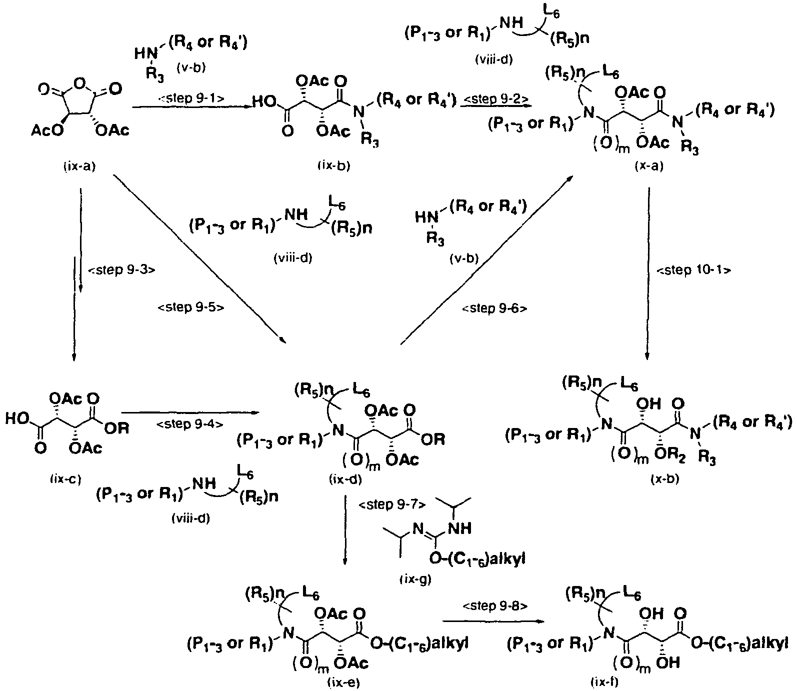

### (Reaction scheme 5)

### <step 9-1>

A compound represented by formula (ix-b) can be produced by conducting a reaction using (*2R,3R*)-2,3-diacetoxysuccinic anhydride represented by formula (ix-a) in the presence of amine (v-b) by a process similar to that described in published documents, for example, Organic Synthesis, Collective Vol.3. pp.169 1955*,* using a solvent which is inactive to the reaction, such as tetrahydrofuran, N, N-dimethylformamide, dioxane, CH2Cl2 or a mixed solvent thereof at a temperature in the range of room temperature to the solvent-reflux temperature.

### <step 9-2>

A compound represented by formula (x-a) can be produced by allowing a compound represented by formula (ix-b) to react with a compound represented by formula (viii-d) by a process similar to that described in published documents, for example, Jikken Kugaku Koza (Experimental Chemistry Series). 4th edition. 26, Acids, amino acids, and peptides, pp. 193-309, 1992, Maruzen Co., Ltd., in the presence of a condensing agent such as 1,3-dicyclohexylcarbodiimide (DCC), 1-ethyl-3-(3'-dimethylaminopropyl)carbodiimide hydrochloride (WSC·HCl), benzotriazol-1-yloxy tris(dimethylamino)phosphonium hexafluorophosphate (BOP reagent), bis(2-oxo-3-oxazolidinyl)phosphinic chloride (BOP-Cl), 2-chloro-1,3-dimethylimidazolinium hexafluorophosphate (CIP), or 4-(4,6-dimethoxy-1,3,5-triazin-2-yl)-4-methylmorpholinium chloride, in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g., toluene or benzene, a polar solvent, e.g., N,N-dimethylformamide, or an alcoholic solvent, e.g., methanol, ethanol, or 2-propanol, in the presence or absence of a base such as triethylamine or pyridine at a temperature in the range of -78°C to the solvent-reflux temperature. When a compound represented by formula (ix-b) is converted to an acid halide, a compound represented by formula (x-a) can be similarly produced by conducting a reaction by a process similar to that described in, for example, Jikken Kagaku Koza (Experimental Chemistry Series), 4th edition, 26, Acids, amino acids, and peptides, pp. 144-146, 1992, Maruzen Co., Ltd., in the presence of a base such as triethylamine or pyridine in a solvent which is inactive to the reaction, such as a halogenated solvent, e.g., dichloromethane or chloroform, an ethereal solvent, e.g., diethyl ether or tetrahydrofuran, an aromatic hydrocarbon solvent, e.g.. toluene or benzene, or a polar solvent, e.g., N,N-dimethylformamide at a temperature in the range of -78°C to the solvent-reflux temperature.

Alternatively, a compound represented by formula (x-a) can be produced by using triphosgene with (ix-b) by a process similar to that described in published documents, for example, Letters in Organic Chemistry, 4, 20-22. 2007*,* in the presence of a base such as triethyl amine using a solvent which is inactive to the reaction, such as tetrahydrofuran, N,N-dimethylformamide, dioxane, CH2Cl2 or a mixed solvent thereof at a temperature in the range of room temperature to the solvent-reflux temperature.

### <step 9-3>

A compound represented by formula (ix-c) ,wherein R represents hydrogen or C1-6 alkyl group, can be produced by conducting a reaction using (*2R,3R*)-2,3-diacetoxysuccinic anhydride represented by formula (ix-a) in the presence of suitable alcoholic solvent by a process similar to that described in published documents, for example, Organic Synthesis, Collective Vol.3. pp.169 1955*,* using a solvent, such as an alcoholic solvent, e.g., benzyl alcohol, tert-buthyl alcohol, methanol, ethanol, 2-propanol, or tetrahydrofuran, N,N-dimethylformamide, dioxane, CH2Cl2 or a mixed solvent thereof in the presence of DMAP at a temperature in the range of 0°C to the solvent-reflux temperature.

### <step 9-4>

A compound represented by formula (ix-d) can be produced by the same process as that used in <Step 9-2> of (Reaction Scheme 5) using a compound represented by formula (ix-c).

### <step 9-5>

A compound represented by formula (ix-d) wherein R represents hydrogen atom can be produced by allowing (*2R,3R*)-2,3-diacetoxysuccinic anhydride represented by formula (ix-a) to react with a compound represented by formula (viii-d) by a process similar to that described in published documents, for example, Organic Synthesis, Collective Vol.3, pp.169 1955*,* Organic Synthesis, Collective Vol.5, pp.944, 1973*,* Vol.41, 93, 1961*,* or Jikken Kagaku Koza (Experimental Chemistry Series). 4th edition. 26. Acids, amino acids, and peptides, pp. 146-148, 1992, Maruzen Co., Ltd., using a solvent, such as an alcoholic solvent, e.g., methanol, ethanol, or 2-propanol, a halogenated solvent, e.g., dichloromethane or chloroform, an ethereal solvent, e.g., diethyl ether or tetrahydrofuran, a polar solvent, e.g., DMF, ethyl acetate or acetonitrile, an aromatic hydrocarbon solvent, e.g., toluene or benzene, or an acid solvent, e.g., acetic acid or a mixed solvent thereof in the presence of DMAP, Pyridine or sulfuric acid as a catalyst if needed at a temperature in the range of 0°C to the solvent-reflux temperature.

### <step 9-6>

A compound represented by formula (x-a) can also be produced by the same process as that used in <Step 4-3> of (Reaction Scheme 2) using a compound represented by formula (ix-d) wherein R represents C1-6 alkyl group in the presence of amine (v-b), and also be produced by the same process as that used in <step 5-1> of (Reaction scheme 2) or <step 13-3> and <step 13-4> of (Reaction scheme 11) using a compound represented by formula (ix-d) wherein R represents hydrogen atom, in the presence of amine (v-b).

Protective groups of a compound in the process of producing a compound represented by formula (x-a) can be introduced and removed by techniques which are well-known or described here (see Greene, T.W., et. al., Protective Groups in Organic Synthesis (2007), 4th Ed., Wiley, New York, or Kocienski, P., Protecting Groups (1994), Thieme).

### <step 9-7>

A compound represented by formula (ix-e) can be produced by allowing a compound represented by formula (ix-d) to react with a compound represented by formula (ix-g) by a process similar to that described in published documents, for example, Jikken Kagaku Koza (Experimental Chemistry Series), 4th edition, 22, Organic synthesis IV, Acids, amino acids, and peptides, pp. 1-82, 1992, Maruzen Co., Ltd., in the presence of an acidic reagent such as hydrochloric acid, sulfuric acid, thionyl chloride, or acetyl chloride, using a solvent which is inactive to the reaction, such as an alcoholic solvent, e.g., methanol, ethanol, or 2-propanol, a halogenated solvent, e.g., dichloromethane or chloroform, an ethereal solvent, e.g., diethyl ether, dioxane, or tetrahydrofuran, or a mixed solvent thereof at a temperature in the range of -78°C to the room temperature.

### <step 9-8>

A compound represented by formula (ix-d) can be produced by conducting a reaction using a compound represented by formula (ix-e) by a process similar to that described in published documents, for example, Can. J. Chem., 49, 493 (1971*)* or Greene, T.W.. et. al., Protective Groups in Organic Synthesis (2007), 4th Ed., in the presence of ammonia, using a solvent which is inactive to the reaction, such as an alcoholic solvent, e.g., methanol, ethanol, or 2-propanol, a halogenated solvent, e.g., dichloromethane or chloroform, an ethereal solvent, e.g., diethyl ether, dioxane, or tetrahydrofuran, or a mixed solvent thereof at a temperature in the range of -78°C to the room temperature.

### <step 10-1>

A compound represented by formula (x-b) can be produced by the similar process as that used in <Step 9-8> of (Reaction Scheme 6) using a compound represented by formula (x-a).

Protective groups of a compound in the process of producing a compound represented hy formula (x-b) can be introduced and removed by techniques which are well-known or described here (see Greene, T.W., et. al., Protective Groups in Organic Synhesis (2007), 4th Ed., Wiley, New York, or Kocienski, P., Protecting Groups (1994), Thieme).

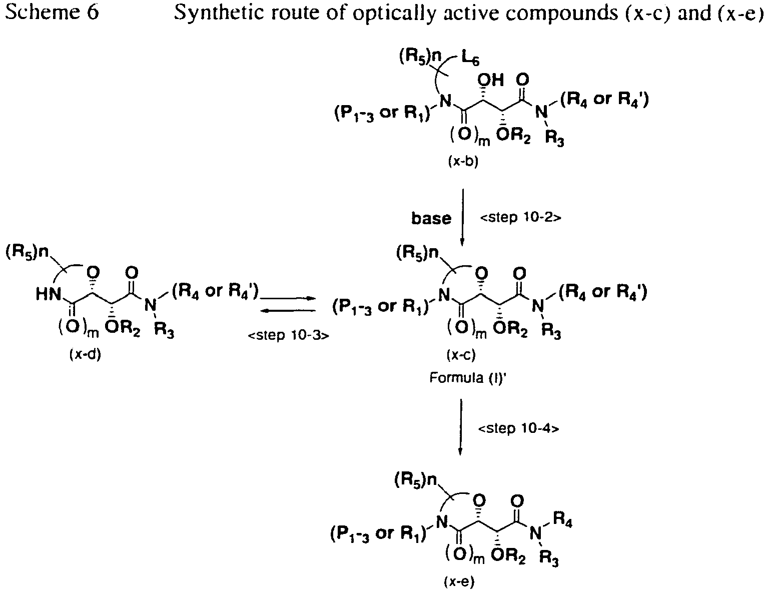

### (Reaction scheme 6)

### <step 10-2>

A compound represented by formula (x-c) can be produced by allowing a compound represented by formula (x-b) by a process similar to that described in published documents, for example, Organic Synthesis, Collective Vol.6, pp.301. 395, 1988*, or* Jikken Kagaku Koza (Experimental Chemistry Series), 4th edition, 20. alcohol and amine, pp. 187-194, 1992, Maruzen Co.. Ltd., in the presence of a base such as potassium *tert*-buthoxide, sodium hydride, lithium hydroxide, sodium hydroxide, potassium hydroxide, lithium carbonate, sodium carbonate, or potassium carbonate using a solvent which is inactive to the reaction, such as an alcoholic solvent, e.g., methanol, ethanol, or 2-propanol, tert-buthanol, or a polar solvent, e.g., DMF, DMSO, ethyl acetate, or acetonitril, or an aromatic hydrocarbon solvent, e.g., toluene or benzene, or acetone, dioxane or tetrahydrofuran, or a mixed solvent thereof at a temperature in the range of 0°C to the solvent-reflux temperature.

### <step 10-3>

Protective groups of a compound represented by formula (x-c) can be introduced and removed by techniques which are well-known or described here (see Greene, T.W., et. al., Protective Groups in Organic Synthesis (2007), 4th Ed., Wiley, New York, or Kocienski, P., Protecting Groups (1994),Thieme).

### <step 10-4>

When R4' is used as a precursor for R4 representing 4-amidino-phenyl group or its analogue, R4' represents 4-cyano-phenyl group or 1,2,4-oxadiazol-5-one-3-yl phenyl group or their analogues, which can be converted to 4-amidino-phenyl group R4 or its analogue by techniques which are well-known or described:

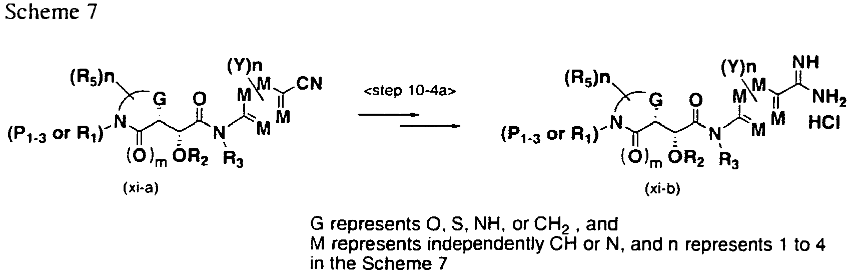

### (Reaction scheme 7)

### <step 10-4a>

When R4' of a compound represented by formula (x-c) of Scheme 6 represents 4-cyano-(aryl or heteroaryl) group or its analogue wherein 4-cyano-(aryl or heteroaryl) ring is optionally substituted with one to four Y, a compound represented by formula (xi-a) which corresponds to a compound (x-c) can be converted to a compound represented by formula (xi-b) via its imidate compound.

4-cyano-(aryl or heteroaryl) group or, R4' of a compound represented by formula (xi-a), can be converted to its imidate by allowing a compound represented by formula (xi-a) to acidic condition such as HCl gas solution of, such as an alcoholic solvent, e.g., methanol, ethanol, or 2-propanol, a halogenated solvent, e.g., dichloromethane or chloroform, or a mixed solvent thereof at a temperature in the range of 0°C to the room temperature.

Resulting imidate compound is converted to 4-amidino-(aryl or heteroaryl) compound (xi-b) or its analogue by conducting an imidate compound to ammonium or ammonium carbonate alcoholic solvent, e.g. methanol, ethanol, tert-buthanol or in a mixed solvent thereof at a temperature in the range of 0°C to the solvent-reflux temperature in a sealed tube.

Alternatively, when R4' represents 4-cyano-(aryl or heteroaryl) group or its analogue wherein 4-cyano-(aryl or heteroaryl) is optionally substituted with one to four Y, a compound represented by formula (xi-a) can be converted to 4-amidino-(aryl or heteroaryl) group R4 via its N-hydroxy amidine compound.

4-cyano group, R4' of a compound represented by formula (xi-a), can be converted to its N-hydroxy amidino group by allowing a compound (xi-a) in the presence of a base such as triethyl amine, hunig base, potassium *tert*-buthoxide, sodium hydride, lithium hydroxide, sodium hydroxide, potassium hydroxide, lithium carbonate, sodium carbonate, or potassium carbonate using a solvent which is inactive to the reaction, such as water, methanol, ethanol, acetone, N,N-dimethylformamide, dioxane or tetrahydrofuran, or a mixed solvent thereof at a temperature in the range of 0°C to the solvent-reflux temperature in a sealed tube.

Resulting N-hydroxy amidino group can be converted to its amidine compound represented by formula (xi-b) by a suitable process similar to that described in published documents, for example, Jikken Kagaku Koza (Experimental Chemistry Series), 4th edition, 26, Asymmetric synthesis, reduction, sugar, and labeled compound, pp. 251-266, 1992, Maruzen Co., Ltd., in the presence of a catalyst such as palladium-carbon (Pd-C), Raney-Ni, or platinum oxide (PtO₂) in a solvent which is inactive to the reaction, such as an alcoholic solvent, e.g., methanol, ethanol, or 2-propanol, a halogenated solvent, e.g., dichloromethane or chloroform, an ethereal solvent, e.g., diethyl ether or tetrahydrofuran, a polar solvent, e.g., ethyl acetate or acetonitrile, an aromatic hydrocarbon solvent, e.g., toluene or benzene, or an acid solvent, e.g., acetic acid or a mixed solvent thereof at a temperature in the range of room temperature to the solvent-reflux temperature.

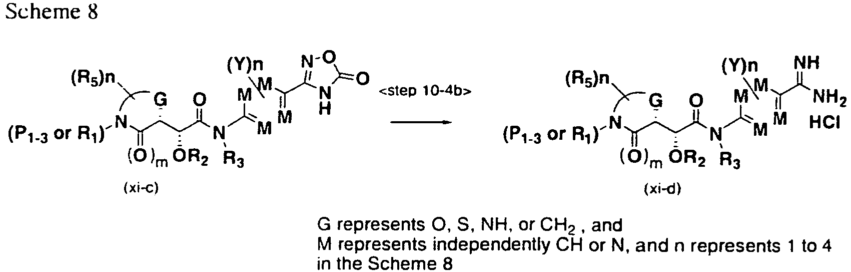

### (Reaction scheme 8)

### <step 10-4b>

When R4' is used as a precursor for R4 representing 4-amidino-(aryl or heteroaryl) group or its analogue wherein 4-amidino-(aryl or heteroaryl) group is optionally substituted with one to four Y, R4' also represents 1,2,4-oxadiazol-5-one-3-yl (aryl or heteroaryl) group or its analogues wherein phenyl group is optionally substituted with one to four Y, which can be converted to 4-amidino-(aryl or heteroaryl) group R4 by techniques which are well-known or described here.

A compound represented by formula (xi-d) can be produced by the same process as that used in <Step 8-1> of (Reaction Scheme 4) using a compound represented by formula (xi-c).

When G in the formula (xi-c) represents sulfur atom, sulfur can be oxidized to its sulfone or sulfoxide with Oxone^{®} by a process similar to that described in published documents, for example, Shin-Jikken Kagaku Kouza, Vol. 14- III, p1759*,* R. J. Kennedy, J. Org. Chem., 25, 1901 (1960*),* B.M. Trost, Tetrahedron Lett., 22,1287(1981*),* in the presence of Oxone^{®} using a solvent which is inactive to the reaction, such as water, or an alcoholic solvent, e.g., methanol, ethanol, or 2-propanol, a halogenated solvent, e.g., dichloromethane or chloroform, an ethereal solvent, e.g., diethyl ether, dioxane, or tetrahydrofuran, or a mixed solvent thereof at a temperature in the range of 0°C to the solvent-reflux temperature.

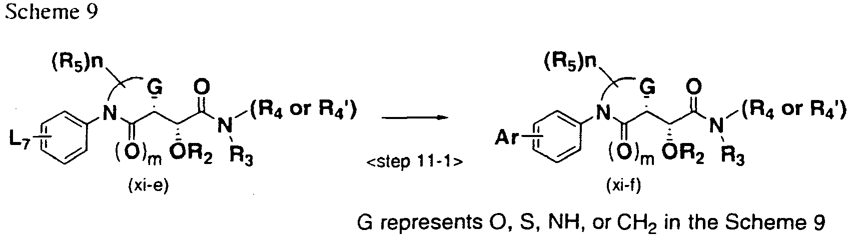

### (Reaction scheme 9)

### <step 11-1>

When R1 in the Formula (I) represents biaryl groups optionally substituted with one to four Y, such as, for example, 4-thienyl phenyl group or 4-phenyl phenyl group, a compound represented by formula (xi-f) can be produced by conducting a reaction using a compound represented by formula (xi-e) by a process of Suzuki-Miyaura coupling similar to that described in published documents, for example, Miyauru, N, et.al., Tetrahedron Lett., 1979, 3437*,* J. Chem. Soc. Chem. Commun., 1979, 866*,* Chem. Rev. 1995. 95, 2457*.* in the presence of catalyst such as palladium-carbon (Pd-C), Raney-Ni, or platinum oxide (PtO₂), and in the presence of a base such as potassium *tert*-buthoxide, sodium hydride, lithium hydroxide, sodium hydroxide, potassium hydroxide, lithium carbonate, sodium carbonate, or potassium carbonate with a corresponding arylboronic acid using a solvent which is inactive to the reaction, such as water, acetone, toluene, dioxane or tetrahydrofuran, or a mixed solvent thereof at a temperature in the range of room temperature to the solvent-reflux temperature.

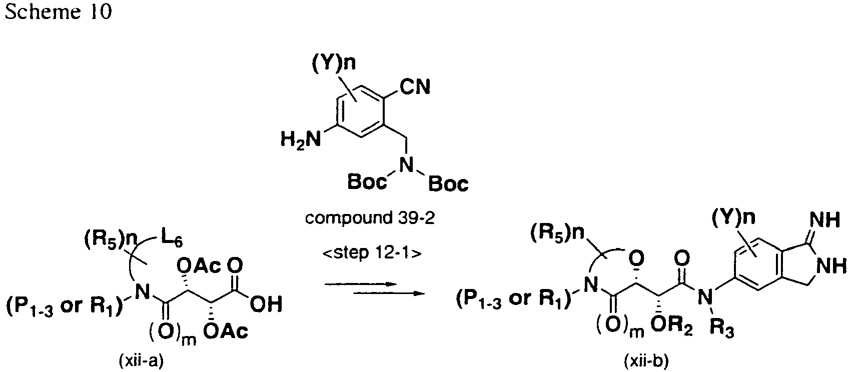

### (Reaction scheme 10)

### <step 12-1>

When R4 represents 1-imino-2,3-dihydroisoindol-5-yl group, or its heteroaryl analogue wherein M represents nitrogen atom, are optionally substituted with one to four Y, which is shown as a partial structure of a compound represented by formula (xii-b) in Scheme 10 or its analogue, a compound represented by formula (xii-b) can be produced from a compound represented by formula (xii-a) which is identical to the comound represented by formula (ix-d) in the Scheme 5, wherein its R is hydrogen atom, and from a compound 39-2 (N-[(5-Amino-2-cyanophenyl)-methyl]-N-[(2-methylpropan-2-yl) oxycarbonyl] carbamate) which is described in Experimental section 39. A compound represented by formula (ix-d) is converted to its analogous compound of 39-3 in the Example 39 procedure similar to that used in <Step 5-1> of (Reaction Scheme 2), which is followed by the conversion to compounds analogous to compounds 39-4, 39-5 and 39-6 by a process similar to that used in <Step 10-1> of (Reaction Scheme 5), <Step 10-2> of (Reaction Scheme 6) and deprotection of Boc group by a process similar to that used in the Experimental section Example 38-6 or by techniques which are well-known or described here (see Greene, T.W., et. al., Protective Groups in Organic Synthesis (2007), 4th Ed., Wiley, New York, or Kocienski, P., Protecting Groups (1994),Thieme). A compound represented by formula (xii-b) can be produced with resulting amine compound analogous to the compound 39-6 by a process similar to that described in the Experimental section 39 [step 39-7] using alcoholic solvent, other solvent or a mixed solvent thereof at a temperature in the range of room temperature to the solvent-reflux temperature.

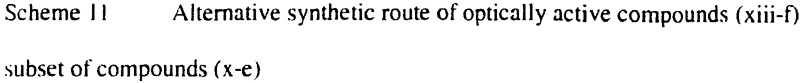

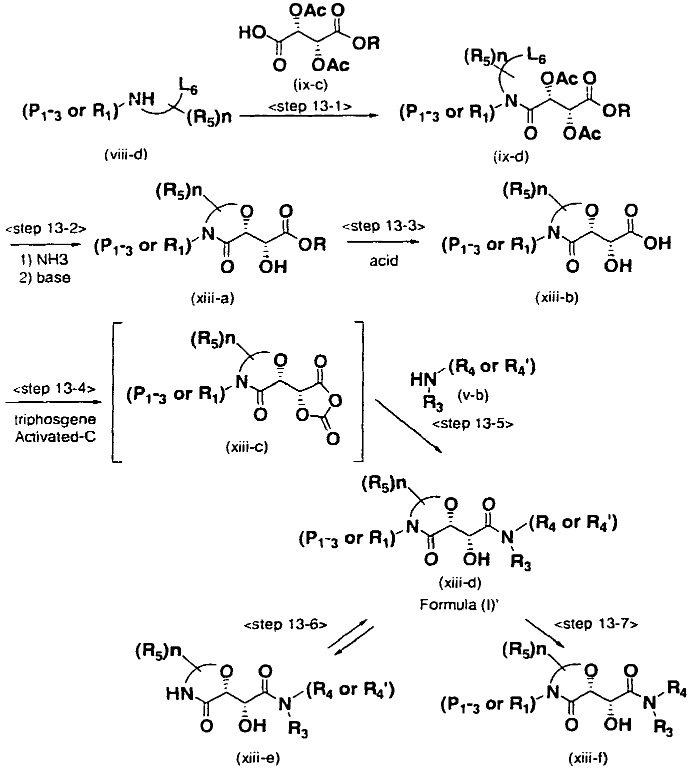

### (Reaction scheme 11)

### <step 13-1>

A compound represented by formula (ix-d) can be produced by the similar process as that used in <Step 5-1> of (Reaction Scheme 2) using compounds represented by formula (viii-d) and represented by formula (ix-c). When R represents *tert*-butyl group, a compound represented by formula (ix-c) could be prepared by a similar process that described in published document, for example, Tetrahedron, 45, 3071-3080, 1989.

### <step 13-2>

A compound represented by formula (xiii-a) can be produced by the similar process as that used in <Step 10-1> of (Reaction Scheme 5) using a compound represented by formula (ix-d), and followed by the similar process as that used in <step 10-2> of (Reaction Scheme 6) using the resulting alcoholic compound from a compound represented by formula (ix-d).

### <step 13-3>

A compound represented by formula (xiii-b) can be produced from a compound represented by formula (xiii-a) by a well-known or similar process that described in published documents, for example, Jikken Kagaku Koza (Experimental Chemistry Series), 4th edition, 22, Organic synthesis IV, Acids, amino acids, and peptides, pp. 1-43, 1992, Maruzen Co., Ltd.. in the presence of inorganic or organic acids such as hydrochloric, hydrobromic, sulfuric, hemisulfuric, phosphoric, methanesulfonic, benzenesulfonic, p-toluenesulfonic, 4-bromobenzenesulfonic, cyclohexylamidosulfonic, trifluoromethylsulfonic, 2-hydroxyethanesulfonic, acetic, oxalic, tartaric, succinic, glycerolphosphoric, lactic, malic, adipic, citric, fumaric, maleic, gluconic, glucuronic, palmitic or trifluoroacetic acid using water and a solvent which is inactive to the reaction, such as methanol, ethanol. 2-propanol, N,N-dimethylformamide, dioxane, or tetrahydrofuran, or a mixed solvent thereof at a temperature in the range of 0°C to the solvent-reflux temperature.

### <step 13-4> and <step 13-5>

A compound represented by formula (xiii-d) can be produced by the similar process as that used in <Step 5-1> of (Reaction Scheme 2) using a compound represented by formula (xiii-b) via its intermediate represented by formula (xiii-c), with a compound represented by formula (v-b).

### <step 13-6>

Protective groups of a compound represented by formula (xiii-d) can be introduced and removed between (xiii-d) and (xiii-e) by techniques which are well-known or described here (see Greene, T.W., et. al., Protective Groups in Organic Synthesis (2007), 4th Ed.. Wiley, New York, or Kocienski, P., Protecting Groups (1994), Thieme).

### <step 13-7>

A compound represented by formula (xiii-f), which is identical to the compound represented by formula (x-e) in Scheme 6 wherein m is 1 and R is hydrogen atom, can be produced by a similar process as that used in <Step 10-4> of (Reaction Scheme 6) using a compound represented by formula (xiii-d).

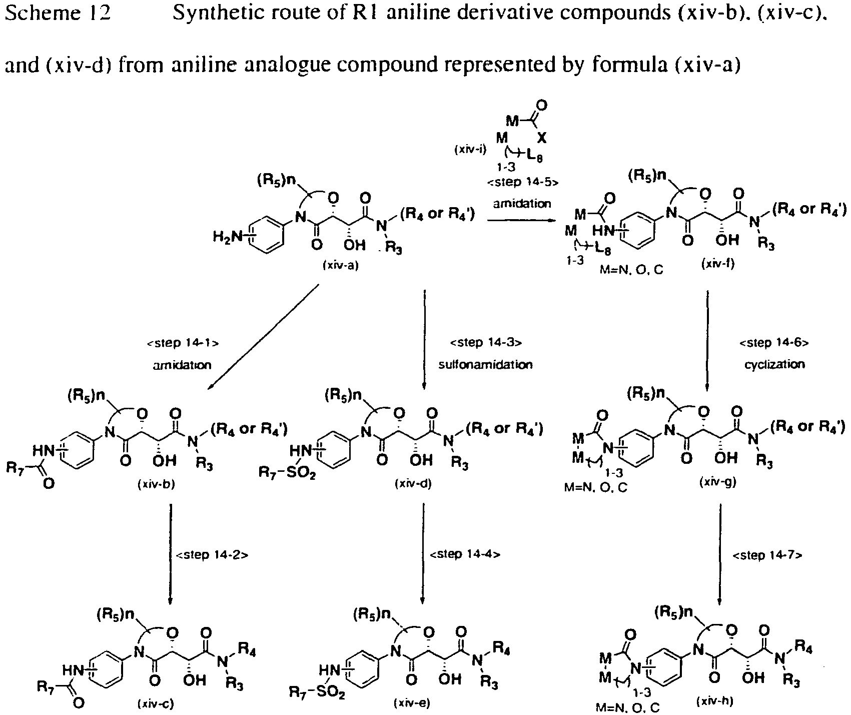

### (Reaction scheme 12)

### <step 14-1>

A compound represented by formula (xiv-b) can be produced by the similar process as that used in <Step 5-1> of (Reaction Scheme 2) using a compound represented by formula (xiv-a).

### <step 14-2>

A compound represented by formula (xiv-c) can be produced by the similar process as that used in <Step 10-4> of (Reaction Scheme 6) using a compound represented by formula (xiv-b).

### <step 14-3>

A compound represented by formula (xiv-d) can be produced by the similar process as that used in <Step 5-1> of (Reaction Scheme 2) using a compound represented by formula (xiv-a) and corresponding sulfonyl halide such as sulfonyl chloride reagent.

### <step 14-4>

A compound represented by formula (xiv-e) can be produced by the similar process as that used in <Step 10-4> of (Reaction Scheme 6) using a compound represented by formula (xiv-d).

### <step 14-5>

A compound represented by formula (xiv-f) wherein each M represents independently oxygen atom, nitrogen atom or carbon atom, can be produced with step by step cyclization process as that used in <Step 5-1> of (Reaction Scheme 2) using a compound represented by formula (xiv-a) and a compound represented by formula (xiv-i) denoting acid halide or acid reagent wherein X represents halogen or hydroxyl group, such as 2-chloroethoxy acetic acid.

### <step 14-6>

The resulting compound represented by formula (xiv-f) can he cyclized to produce a compound represented by formula (xiv-g) by the same prosess as that used in

### <Step 10-2> of (Reaction Scheme 6).

### <step 14-7>

A compound represented by formula (xiv-g) can be produced by the similar process as that used in <Step 10-4> of (Reaction Scheme 6) using a compound represented by formula (xiv-f).

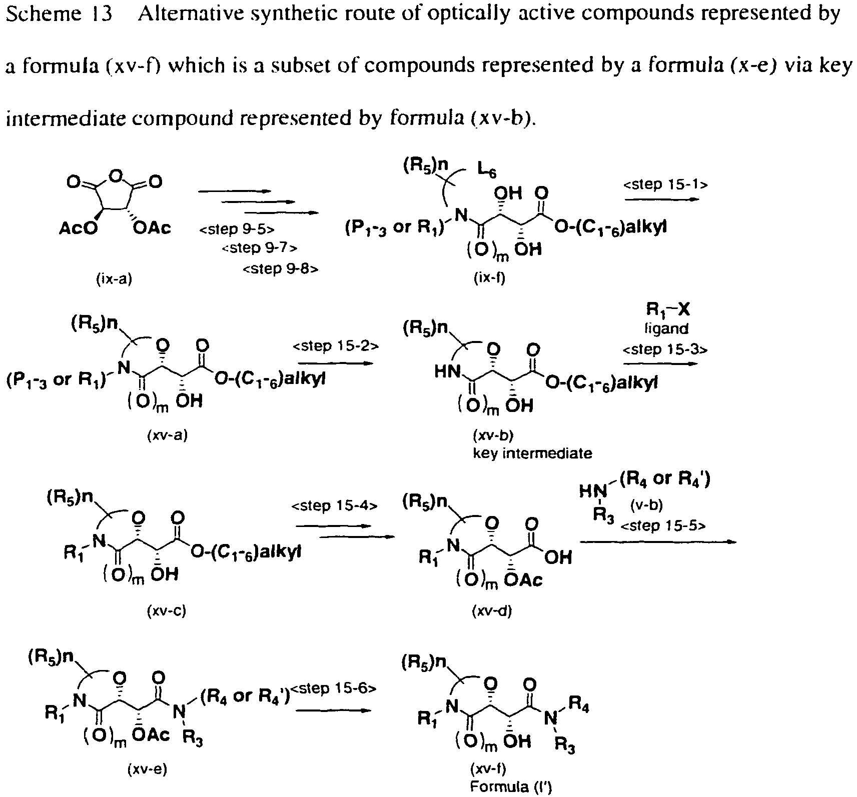

### (Reaction scheme 13)

### <step 15-1>

A compound represented by formula (xv-a) can be produced by the similar process as that used in <Step 10-2> of (Reaction Scheme 6) using a compound represented by formula (ix-f) in the Scheme 5.

### <step 15-2>

A compound represented by formula (xv-b), a key intermediate to produce compounds represented by formula (xv-f) which corresponds to the compounds represented by Formula (I), can be produced by deprotection of the compound represented by formula (xv-a) using CAN (ceric ammonium nitrate) using a solvent which is inactive to the reaction, such as a polar solvent, e.g., DMF, DMSO, ethyl acetate, water, or acetonitril, or an ethereal solvent, e.g., diethyl ether, tetrahydrofuran. 1,2-dimethoxyethane, 1,4-dioxane, or a mixed solvent thereof at a temperature in the range of 0°C to the solvent-reflux temperature, or by techniques which are well-known or described here (see Greene, T.W., et. al., Protective Groups in Organic Synthesis (2007), 4th Ed., Wiley, New York, or Kocienski, P., Protecting Groups (1994),Thieme).

### <step 15-3>

A compound represented by formula (xv-c) can be produced by allowing a key intermediate compound represented by formula (xv-b) to react with a compound represented by R1-X (aryl halide or heteroaryl halide, wherein X represents halogen atom) by a process known as Goldberg reaction which are similar to that described in published documents, for example, JACS, 2002, 124, 7421 in the presence of a base such as potassium phosphate, cesium carbonate, potassium *tert*-buthoxide, sodium hydride, lithium hydroxide, sodium hydroxide, potassium hydroxide, lithium carbonate, sodium carbonate, or potassium carbonate in the presence of 1,2-diamine ligand such as trans-1,2-cyclohexanediamine, trans-N.N'-dimethylcyclohexane-1,2-diamine, or ethylene diamine, and in the presence of catalytic amount of cupper iodide using a solvent which is inactive to the reaction, such as an ethereal solvent, e.g., diethyl ether, tetrahydrofuran, 1,3-dimethoxyethane, 1,4-dioxane, polar solvents such as DMF, and DMSO; or an aromatic hydrocarbon solvent, e.g., toluene or benzene or a mixed solvent thereof at a temperature in the range of room temperature to the solvent-reflux temperature.

### <step 15-4>

A compound represented by formula (xv-c) can be produced by allowing a compound represented by formula (xv-b) to react with acetic anhydride by a process similar to that described in published documents, for example, Jikken Kagaku Koza (Experimental Chemistry Series), 4th edition, 22, Organic synthesis IV, Acids, amino acids, and peptides, pp. 191-309, 1992, Maruzen Co., Ltd., and resulting acetylated alcohol comound can be hydrolyzed by the similar process as that used in <Step 4-2> of (Reaction Scheme 2).

### <step 15-5>

A compound represented by formula (xv-e) can be produced by the similar process as that used in <Step 5-1> of (Reaction Scheme 2) using a compound represented by formula (xv-d) in the Scheme 13 and a compound represented by formula (v-b).

### <step 15-6>

A compound represented by formula (xv-f) can be produced by a similar process as that used in <Step 10-4> of (Reaction Scheme 6) using a compound represented by formula (xv-e).

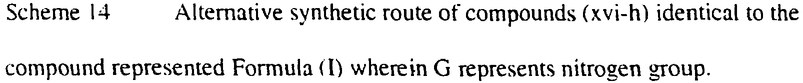

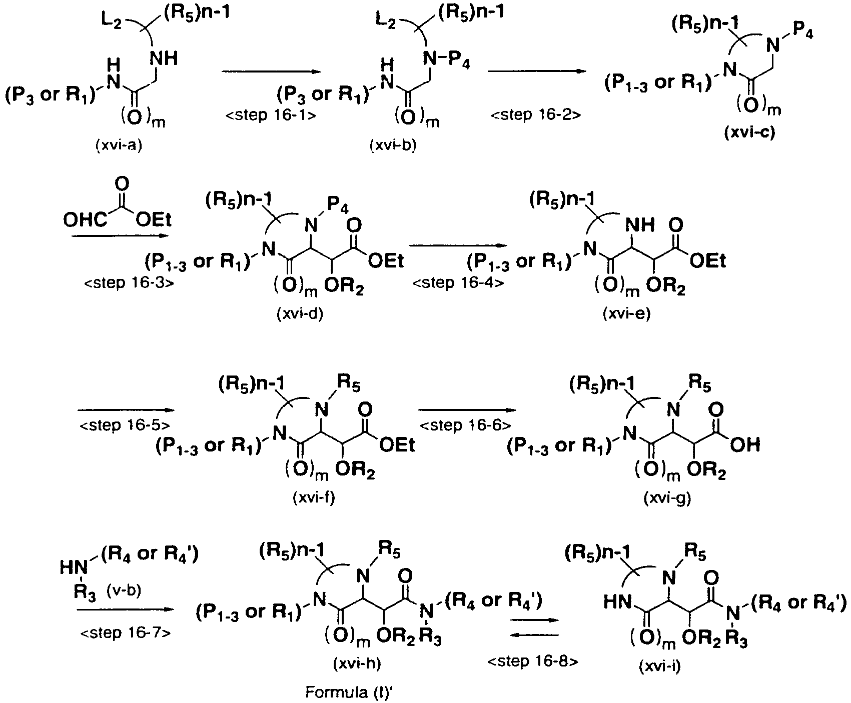

### (Reaction scheme 14)

### <step 16-1>

A compound represented by formula (xvi-a) are commercially available, or capable of being readily synthesized by the method as identical to the route descrived in Scheme 1 to synthesize a compound represented by formula (ii-b), or commonly used in the organic chemistry from commercially available products.

A compound represented by formula (xvi-b) can be produced by the similar process as that used in <Step 2-3> of (Reaction Scheme 1) using a compound represented by formula (xvi-a) in the Scheme 14.

### <step 16-2>

A compound represented by formula (xvi-c) can be produced by a similar process as that used in <Step 2-2> of (Reaction Scheme 1) using a compound represented by formula (xvi-b) in the Scheme 14.

### <step 16-3>

A compound represented by formula (xvi-d) can be produced by the similar process as that used in <Step 3-1> of (Reaction Scheme 2) using a compound represented by formula (xvi-c) in the Scheme 14.

### <step 16-4>

A compound represented by formula (xvi-e) can be produced by a similar process as that used in <Step 2-2> of (Reaction Scheme 1) using a compound represented by formula (xvi-d) in the Scheme 14.

### <step 16-5>

A compound represented by formula (xvi-f) can be produced by the similar process as that used in <Step 5-1> of (Reaction Scheme 2) using a compound represented by formula (xvi-e) in the Scheme 14 when R5 represents acyl group such as, for example, acetyl group or benzyl group.

### <step 16-6>

A compound represented by formula (xvi-g) can be produced by a similar process as that used in <Step 1-6> of (Reaction Scheme 1) using a compound represented by formula (xvi-f) in the Scheme 14.

### <step 16-7>

A compound represented by formula (xvi-h) can be produced by a similar process as that used in <Step 5-1> of (Reaction Scheme 2) using a compound represented by formula (xvi-g) in the Scheme 14.

### <step 16-8>

A compound represented by formula (xvi-i) can be produced by a similar process as that used in <Step 5-2> of (Reaction Scheme 2) using a compound represented by formula (xvi-h) in the Scheme 14.

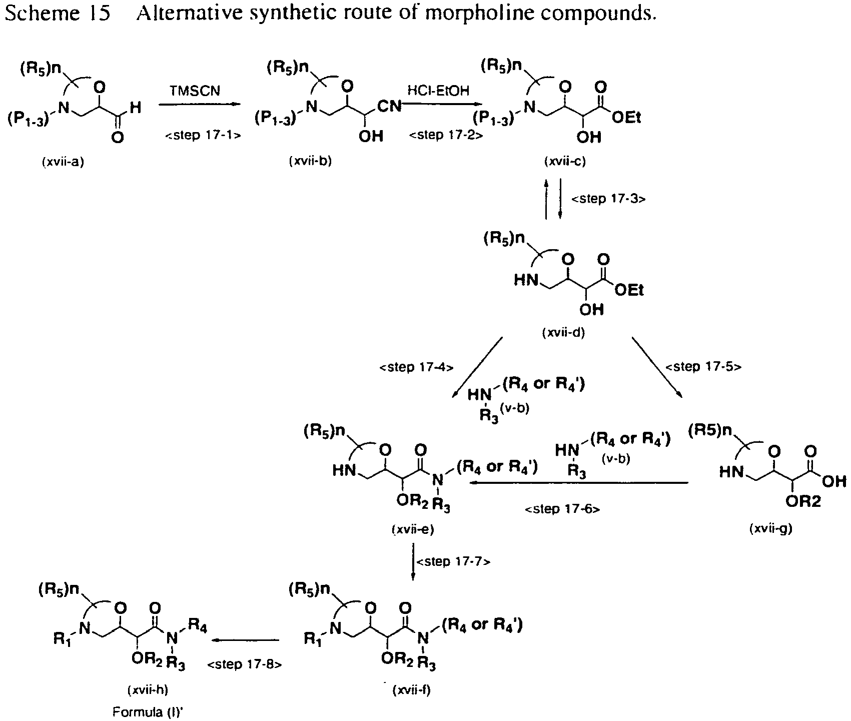

### <step 17-1>

A compound represented by formula (xvii-b) can be produced by allowing a compound represented by formula (xvii-a) to react with TMSCN(trimethylsilyl cyanide) by a process similar to that described in published documents, for example, Organic synthesis Collective Vol. 1, pp.336 (1941), Collective Vol. 2, pp.7 (1943), Colletive Vol. 7, pp-521 (1990) using a solvent which is inactive to the reaction, such as an alcoholic solvent, e.g., methanol, ethanol, 2-propanol, or a mixed solvent thereof at a temperature in the range of -78 °C to the solvent-reflux temperature. P₁₋₃ represents typically a benzyl group and deprotection of benzyl group can be reductive deprotection by a similar process of <step 8-1>.

### <step 17-2>

A compound represented by formula (xvii-c) can be produced by allowing a compound represented by formula (xvii-b) by a process similar to that described in published documents, for example, Organic synthesis Collective Vol. 1, pp.270 (1941), Collective Vol. 2, pp.310 (1943) in the presence of concentrated HCl using a solvent such as an alcoholic solvent containing hydrogen chloride, e.g., methanol-HCl, ethanol-HCl, or a mixed solvent thereof at a temperature in the range of 0°C to the solvent-reflux temperature.

### <step 17-3>

Protective groups of a compound represented by formula (xvii-c) can be introduced and removed by techniques which are well-known or described here (see Greene, T.W., et. al., Protective Groups in Organic Synthesis (2007), 4th Ed., Wiley, New York. or Kocienski, P., Protecting Groups (1994),Thieme).

### <step 17-4>

A compound represented by formula (xvii-e) can be produced by the same process as that used in <Step 4-3> of (Reaction Scheme 2) using a compound represented by formula (xvii-c) and a compound represented by formula (v-b).

### <step 17-5>

A compound represented by formula (xvii-g) can be produced by a similar process as that used in <Step 1-6> of (Reaction Scheme 1) using a compound represented by formula (xvii-c) in the Scheme 15.

### <step 17-6>

A compound represented by formula (xvii-e) can be produced by the same process as that used in <Step 5-1> of (Reaction Scheme 2) using a compound represented by formula (xvii-g) and a compound represented by formula (v-b).

### <step 17-7>

A compound represented by formula (xvii-f) can be produced by the same process as that used in <step 14-1> or <step 14-3> of (Reaction Scheme 12), or <step 15-3> of (Reaction Scheme 13) using a compound represented by formula (xvii-f).

### <step 17-8>

A compound represented by formula (zvii-h) can be produced by a similar process as that used in <Step 10-4> of (Reaction Scheme 6) using a compound represented by formula (xvii-g).

(*S*, *S*), (*R,S*) and (*S,R*) forms of compounds represented by Formula (I) can also be made from corresponding starting materials. The required starting materials for the synthesis of *(S,S), (R.S)* and *(S,R)* isoforms of compound (ix-a) are either commercially available, or capable of being readily synthesized by the method commonly used in the organic chemistry from commercially available products.

Acidic or basic products of the compound of the Formula (I) can be present in the form of their salts or in free form. Pharmacologically acceptable salts are preferred, for example alkali metal or alkaline earth metal salts such as hydrochlorides, hydrobromides, sulfates, hemisulfates, all possible phosphates, and salts of the amino acids, natural bases or carboxylic acids.

The preparation of pharmacologically acceptable salts from compounds of the Formula (I) capable of salt formation, including their stereoisomeric forms is carried out in a manner known per se. With basic reagents such as hydroxides, carbonates, hydrogencarbonates, alkoxides and ammonia or organic bases, for example, trimethyl- or triethylamine, ethanolamine, diethanolamine or triethanolamine, trometamol or alternatively basic amino acids, for example lysine, ornithine or arginine, the compounds of the Formula (I) form stable alkali metal, alkaline earth metal or optionally substituted ammonium salts. If the compounds of the Formula (I) have basic groups, stable acid addition salts can also be prepared using strong acids. For this, inorganic and organic acids such as hydrochloric, hydrobromic, sulfuric, hemisulfuric, phosphoric, methanesulfonic, benzenesulfonic, p-toluenesulfonic, 4-bromobenzenesulfonic, cyclohexylamidosulfonic, trifluoromethylsulfonic, 2-hydroxyethanesulfonic, acetic, oxalic, tartaric, succinic, glycerolphosphoric, lactic, malic, adipic, citric, fumaric, maleic, gluconic, glucuronic, palmitic or trifluoroacetic acid are suitable.

### REFERENCE EXAMPLES

### (EXAMPLE 1)

### Synthesis of N-(1-aminoisoquinolin-6-yl)-2-hydroxy-2-(3-oxo-4-p-tolylmorpholin-2-yl)acetamide trifluoroacetate

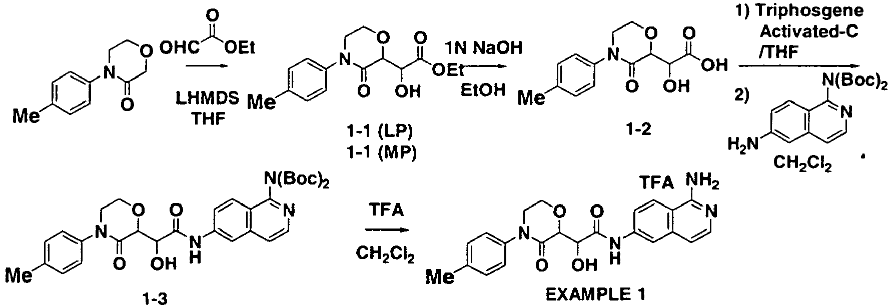

### [Step 1-1] Synthesis of ethyl 2-hydroxy-2-(3-oxo-4-p-tolylmorpholin-2-yl)acetate (compound 1-1 (LP) and compound 1-1 (MP))

To a solution of 4-(4-Methylphenyl)-3-morpholinone (Zhurnal Organicheskoi Khimii, 6(6), 1305-8, 1970) (1.08 g) in THF (21.6 ml), was added 1 M lithium hexamethyldisilazide solution (7.34 ml) in THF at -78 °C. The mixture was stirred at -78 °C for 15 minutes then 0 °C for 1 hour. Then the reaction mixture was cooled down at -78 °C and ethyl glyoxylate solution (1.84 ml) in toluene was added into the reaction mixture. The reaction mixture was stirred at 0 °C overnight. At the end of the reaction, saturated NH₄Cl aqueous solution was added into the reaction mixture. The mixture was concentrated in vacuo and the resulting mixture was extracted with AcOEt. The organic layer was washed with brine and dried with anhydrous Na₂SO₄. The solvent was removed under reduced pressure and the resulting residue was purified by silica gel flash column chromatography (eluent: n-Hex/AcOEt = 50/50 - 0/100) to obtain two diastereomers, compound 1-1 (LP) (405 mg; Rf value = 0.36 on TLC (n-Hex/AcOEt=1/2)) as a pale yellow amorphous solid and 1-1 (MP) (287 mg; Rf value = 0.27 on TLC (n-Hex/AcOEt=1/2)) as yellow oil. LP indicates a less polar spot on TLC. MP indicates a more polar spot on TLC.

### [Step 1-2] Synthesis of 2-hydroxy-2-(3-oxo-4-p-tolylmorpholin-2-yl)acetic acid (compound 1-2)

To a solution of compound 1-1 (LP) (100 mg) in EtOH (1 mL), was added 1 N NaOH aqueous solution (1 mL) at 0 °C. The reaction mixture was stirred at room temperature for I hour. Then DowEx^{®}-50Wx8-200 was added into the reaction mixture, then the mixture was filtered to remove DowEx^{®}-50Wx8-200. The filtrate was concentrated in vacuo to obtain compound 1-2 (90 mg) as a colorless amorphous solid. Compound 1-2 was used in the next step without further purification.

### [Step 1-3] Synthesis of N-[N,N-bis(tert-butoxylcarbonyl)-1-aminoisoquinolin-6-yl]-2-hydroxy-2-(3-oxo-4-p-tolymorpholin-2-yl)acetamide (compound 1-3)

To a solution of compound 1-2 (90 mg) in THF (2 ml), were added activated-charcoal (4.5 mg) and triphosgene (403 mg) at 0 °C. The reaction mixture was stirred at room temperature for 15 hours. Then activated-charcoal was removed by filtration and the filtrate was concentrated in vacuo. The resulting residue was resolved in CH₂Cl₂ (2 ml), 6-Amino-1-bis(tert-butoxyl carbonyl)aminoisoquinoline (146 mg) was added into the CH₂Cl₂ solution at 0°C. The reaction mixture was stirred at room temperature for 4 hours. The reaction mixture was concentrated in vacuo and a half volume of the resulting residue was purified by LC/MS to obtain compound 1-3 (30.6 mg) as a colorless amorphous solid.

### [Step 1-4]Synthesis of N-(1-aminoisoquinolin-6-yl)-2-hydroxy-2-(3-oxo-4-p-tolylmorpholin-2-yl)acetamide trifluoroacetate (EXAMPLE 1)

To a solution of compound 1-3 (30.6 mg) in CH₂Cl₂ (1.5 ml), was added trifluoroacetic acid (0.5 ml) at 0 °C. The reaction mixture was stirred at room temperature for 1 hour and the mixture was concentrated in vacuo. To the resulting residue, Et₂O was added and the residue was triturated. Then the precipitate was collected by filtration to obtain EXAMPLE 1 (19.3 mg) as a colorless amorphous solid.

### (EXAMPLE 7)

### Synthesis of (2R)-N-(4-amidinophenyl)-2-hydroxy-2-[(2R)-3-oxo-4-p-tolylmorpholin-2-yl]acetamide hydrochloride (EXAMPLE 7)

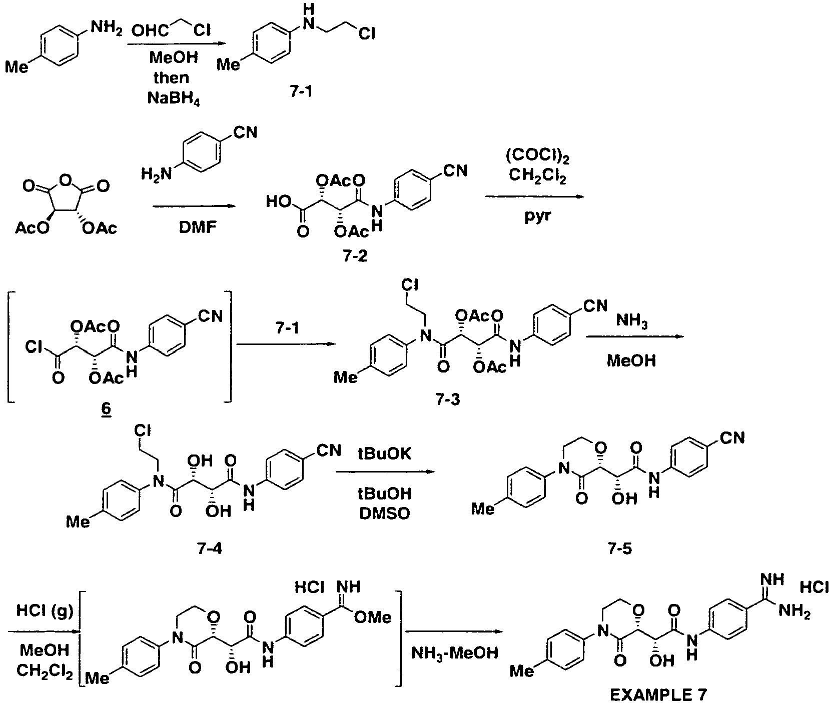

### [Step 7-1] Synthesis of N-(2-chloroethyl)-4-methylaniline (compound 7-1)

To a solution of 4-methylaniline (100 mg) in MeOH (2.0 mL) was added 40% chroloacetaldehyde solution in water (0.17 mL) at 0 °C. The mixture was stirred for 45 minutes at the same temperature, sodium borohydride (NaBH₄ ; 70.6 mg) was added into the reaction mixture at one portion and the mixture was stirred for 1 hour.

The reaction mixture was diluted with water and was extracted with EtOAc. The extract was washed with water, sat.NaHCO₃ and brine. The organic layer was dried with anhyd. Na₂SO₄. It was filtrated to remove insoluble matters and it was concentrated in vacuo. The residue was purified by silica gel flush chromatography (eluent : Hexane : EtOAc = 95 : 5 ~ 75 : 25) to obtain 7-1 (27 mg) as brown oil.

### [Step 7-2] Synthesis of (2R,3R)-2,3-diacetyloxy-4-(4-cyanoanilino)-4-oxobutanoic acid (compound 7-2)

To a solution of (+)-Diacetyl-L-tartaric anhydride (9.15 g) in dry DMF (100 mL), was added 4-aminobenzonitrile (5 g,) under ice cooling and the reaction mixture was stirred to obtain compound 7-2 at room temperature overnight. The solution of compound 7-2 was used in the next step without any treatment.

### [Step 7-3] Synthesis of [(2R,3R)-3-acetyloxy-1-[N-(2-chloroethyl)-4-methylanilino]-4-(4-cyanoanilino)-1,4-dioxobutan-2-yl] acetate (compound 7-3)

The above DMF solution of 7-2 (13.8 mL) was diluted with CH₂Cl₂ (13.8 mL). The internal temperature of the mixture was kept below -60 °C over all additions with dry ice bath.

Oxalyl chloride (0.55 mL) in CH₂Cl₂ (1.7 mL) was added dropwise into the reaction mixture. After stirring for 1 hour, pyridine (1.99 mL) was added dropwise thereto and stirred for 15 min. Then 7-1 (0.99 g) in CH₂Cl₂ (6 mL) was added dropwise into the reaction mixture. The mixture was stirred below -60 °C for 20 min, then it was stirred at -30 °C for 15 hours.

The reaction mixture was quenched with water and was extracted with EtOAc. The extract was washed with water, 1N HCl, sat.NaHCO₃ and brine. The organic layer was dried with anhyd. Na₂SO₄. It was filtrated and was concentrated in vacuo. The residue was purified by silica gel flush chromatography (eluent : Hexane : EtOAc = 75 : 25 ~ 25 : 75) to obtain 7-3 (1.70 g) as a light brown solid.

### [Step 7-4] Synthesis of (2R,3R)-N-(2-chloroethyl)-N'-(4-cyanophenyl)-2,3-dihydroxy-N-p-tolylbutanediamide (compound 7-4)

To a solution of 7-3 (0.20 g) in MeOH (4 mL), was added 8N NH₃ / MeOH (0.26 mL) at 0 °C and the mixture was stirred for 10 minutes in the same temperature. The mixture was concentrated and was dried in vacuo to obtain crude 7-4. The crude 7-4 was used in the next step without further purification.

### [Step 7-5]Synthesis of (2R)-N-(4-cyanophenyl)-2-hydroxy-2-[(2R)-3-oxo-4-p-tolylmorpholin-2-yl]acetamide (compound 7-5)

The crude 7-4 was dissolved in t-BuOH (12 mL)-DMSO (8 mL), and t-BuOK (554 mg) was added portionwise into the reaction mixture at 0 °C. The mixture was stirred for 10 minutes in the same temperature.
To the reaction mixture was added 1N HCl and Et₂O to obtain precipitate. Then the precipitate was collected by filtration, was rinsed with water, was washed with Et₂O and was dried in vacuo to obtain 7-5 (603 mg) as a white solid.

### [Step 7-6] Synthesis of (2R)-N-(4-amidinophenyl)-2-hydroxy-2-[(2R)-3-oxo-4-p-tolylmorpholin-2-yl]acetamide hydrochloride (EXAMPLE 7)

Compound 7-5 (27 mg) was suspended in MeOH(15 mL)-CH₂Cl₂(7mL). The suspension was saturated with HCl gas by bubbling at 0 °C for 0.5 hours. Then the mixture was stood to form the imidate at the same temperature overnight. The reaction mixture was concentrated and was dried in vacuo to obtain crude imidate. The crude imidate was dissolved in MeOH (10 mL), then 8N NH₃-MeOH (2 mL) was added into the above MeOH solution. The reaction mixture was stirred in sealed tube at 80 °C for 3 hours to convert EXAMPLE 7. The reaction mixture was stirred for 1 day at room temperature then it was concentrated in vacuo. The resulting residue was dissolved in 1N HCl-MeOH, then the mixture was purified by preparative LC/MS to obtain EXAMPLE 7 (7 mg) as a colorless amorphous solid.

### (EXAMPLE 20)

### (2R)-N-(4-Amidinophenyl)-2-hydroxy-2-[(2R)-3-oxo-4-(2-oxo-1,3-dihydrobenzimidaz ol-5-yl)morpholin-2-yl]acetamide hydrochloride (EXAMPLE 20)

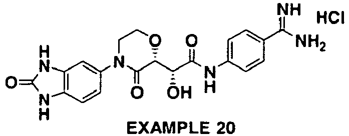

### [Step 20-1] Synthesis of 2-chloro-N-(2-oxo-1,3-dihydrobenzimidazol-5-yl)acetamide 20-1

To a suspension of 5-amino-1,3-dihydro-2H-benzimidazol-2-one (1 g) and K₂CO₃ (1.02 g) in DMF (20 mL), was added dropwise a solution of chloroacetylchloride (0.59 mL) in DMF (10 mL) at 0 °C. The reaction mixture was stirred at room temperature for 3 hours. Then the mixture was diluted with water to precipitate. The precipitate was collected by filtration, rinsed with H₂O to obtain compound 20-1 (1.2 g) as a colorless amorphous solid.

### [Step 20-2] Synthesis of 5-(2-chloroethylamino)-1,3-dihydrobenzimidazol-2-one (compound 20-2)

To a suspension of compound 20-1 (1 g) in THF (10 mL), was added dropwise 1M BH₃-THF complex at 0 °C. The reaction mixture was stirred at room temperature for 3 hours to complete the reaction. Then MeOH was carefully added to decompose an excess of BH₃ and then conc. HCl was added at 0 °C. After stirring under reflux condition for 20 minutes, the mixture was diluted with water. It was extracted with EtOAc and the organic layer was washed with brine and dried with anhyd. Na₂SO₄. The solvent was removed under reduced pressure to obtain compound 20-2 (0.85 g) as a colorless amorphous solid.

### [Step 20-3] Synthesis of [(2R,3R)-3-acetyloxy-4-[2-chloroethyl-(2-oxo-1,3-dihydrobenzimidazo-5-yl)amino]-1-(4-cyanoanilino)-1,4-dioxobutan-2-yl] acetate (compound 20-3)

According to the Step 7-3 in synthetic method for EXAMPLE 7, compound 20-2 (0.8 g) was used instead of compound 7-2 to obtain compound 20-3 (1.6 g) as a colorless amorphous solid.

### [Step 20-4] Synthesis of (2R,3R)-N-(2-chloroethyl)-N'-(4-cyanophenyl)-2,3-dihydroxy-N-(2-oxo-1,3-dihydrobenzimidazol-5-yl)butanediamide (compound 20-4)

According to the Step 7-4 in synthetic method for EXAMPLE 7, compound 20-3 (1.6 g) was used instead of compound 7-3 to obtain crude 20-4. The crude 20-4 was used in the next step without further purification.

### [Step 20-5] Synthesis of (2R)-N-(4-cyanophenyl)-2-hydroxy-2-[(2R)-3-oxo-4-(2-oxo-1,3-dihydrobenzimidazol-5-yl)morpholin-2-yl]acetamide (compound 20-5)

According to the Step 7-5 in synthetic method for EXAMPLE 7, crude 20-4 was used instead of compound 7-4 to obtain compound 20-5 (100 mg) as a brown amorphous solid.

### [Step 20-6] Synthesis of (2R)-N-(4-amidinophenyl)-2-hydroxy-2-[(2R)-3-oxo-4-(2-oxo-1,3-dihydrobenzimidazol-5-yl)morpholin-2-yl]acetamide hydrochloride (EXAMPLE 20)

According to the Step 7-6 in synthetic method for EXAMPLE 7, compound 20-5 (50 mg) was used instead of compound 7-5 to obtain EXAMPLE 20 (8 mg) as a pale yellow amorphous solid.

### (EXAMPLE 26)

### Synthesis of (2R)-2-[(2R)-4-(4-acetamidophenyl)-3-oxomorpholin-2-yl]-N-(4-amidinophenyl)-2-hydroxyacetamide hydrochloride (EXAMPLE 26)

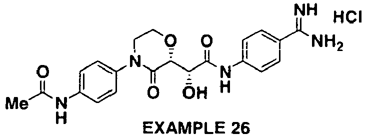

### [Method A]

### [Step 26-1] Synthesis of tert-butyl N-[4-(2-chloroethylamino)phenyl]carbamate (compound 26-1)

According to the Step 7-1 in synthetic method for EXAMPLE 7, 4-(tert-butoxycarbonylamino)aniline (11.6 g) was used instead of 4-methylaniline to obtain compound 26-1 (3,2 g) as a yellow brown amorphous solid.

### [Step 26-2] Synthesis of [(2R,3R)-3-acetyloxy-1-[N-(2-chloroethyl)-4-[(2-methylpropan-2-yl)oxycarbonylamino]anilino]-4-(4-cyanoanilino)-1,4-dioxobutan-2-yl ] acetate (compound 26-2)

According to the Step 7-3 in synthetic method for EXAMPLE 7, compound 26-1 (3.2 g) was used instead of compound 7-1 to obtain compound 26-2 (3.2 g) as colorless amorphous solid.

### [Step 26-3] Synthesis of tert-butyl N-[4-[2-chloroethyl-[(2R,3R)-4-(4-cyanoanilino)-2,3-dihydroxy-4-oxobutanoyl]amino]phenyl]carbamate (compound 26-3)

According to the Step 7-4 in synthetic method for EXAMPLE 7, compound 26-2 (3.2 g) was used instead of compound 7-3 to obtain crude 26-3. The crude 26-3 was used in the next step without further purification.

### [Step 26-4] Synthesis of tert-butyl N-[4-[(2R)-2-[(1R)-2-(4-cyanoanilino)-1-hydroxy-2-oxoethyl]-3-oxomorpholin-4-yl]phenyl]carbamate (compound 26-4)

According to the Step 7-5 in synthetic method for EXAMPLE 7, crude 26-3 was used instead of compound 7-4 to obtain compound 26-4 (1.68 g) as a colorless amorphous solid.

### [Step 26-5] Synthesis of (2R)-2-[(2R)-4-(4-aminophenyl)-3-oxomorpholin-2-yl]-N-(4-cyanophenyl)-2-hydroxyacetamide (compound 26-5)

To compound 26-4 (1.65 g), was added trifluoroacetic acid (10 mL) with anisole (0.2 mL) at 0 °C. The reaction mixture was stirred at 0 °C for 1 hour then Et₂O was added into the mixture to precipitate. The precipitate was collected by filtration and washed with Et₂O. Then the precipitate was solved in water and the solution was basified with sat. NaHCO₃ aq. The precipitate was collected by filtration and washed with H₂O to obtain compound 26-5 (1.2 g) as a pale brown amorphous solid.

### [Step 26-6] Synthesis of [(1R)-1-[(2R)-4-(4-acetamidophenyl)-3-oxomorpholin-2-yl]-2-(4-cyanoanilino)-2-oxoethyl] acetate (compound 26-6)

To a suspension of compound 26-5 (70 mg) in pyridine (1 mL), was added Ac₂O (43.4 microL). The mixture was stirred at room temperature overnight. The reaction mixture was diluted with water to precipitate. The precipitate was collected by filtration and rinsed with water to obtain compound 26-6 (85 mg) as a colorless amorphous solid.

### [Step 26-A] Synthesis of (2R)-2-[(2R)-4-(4-acetamidophenyl)-3-oxomorpholin-2-yl]-N-(4-amidinophenyl)-2-hydroxyacelamide hydrochloride (EXAMPLE 26)

According to the Step 7-6 in synthetic method for EXAMPLE 7, compound 26-6 (50mg) was used instead of compound 7-5 to obtain EXAMPLE 26 (2 mg) as a colorless amorphous solid.

### [Method B]

### [Step 26-7] Synthesis of tert-butyl N-[4-[(2-chloroacetyl)amino]phenyl]carbamate (compound 26-7)

According to the Step 20-1 in synthetic method for EXAMPLE 20, 4-(tert-butoxycarbonylamino)aniline (15 g) was used instead of 5-amino-1,3-dihydro-2*H*-benzimidazol-2-one to obtain compound 26-7 (19.5 g) as a gray amorphous solid.

### [Step 26-8] Synthesis of tert-butyl N-[4-(2-chloroethylamino)phenyl]carbamate (compound 26-1)

According to the Step 20-2 in synthetic method for EXAMPLE 20, compound 26-7 (4 g) was used instead of compound 20-1 to obtain compound 26-1 (3.84 g).

### [Step 26-9] Synthesis of (2R,3R)-2,3-diacetyloxy-4-[N-(2-chloroethyl)-4-[(2-methylpropan-2-yl)oxycarbonylamino]anilino]-4-oxobutanoic acid (compound 26-9)

To a solution of (+)-diacetyl-L-tartaric anhydride (3.01 g) in CH₂Cl₂ (40 mL), was added compound 26-1 (3.77 g) at 0 °C. The mixture was stirred at room temperature for 1 hour. The solvent was removed under reduced pressure to obtain compound 26-9 (7.41 g) as a gray amorphous solid.

### [Step 26-10] Synthesis of [(2R,3R)-3-acetyloxy-4-[N-(2-chloroethyl)-4-[(2-methylpropan-2-yl)oxycarbonylamino]anilino]-1,4-dioxo-1-[4-(5-oxo-4H-1,2,4-oxadia zol-3-yl)anilino]butan-2-yl] acetate (compound 26-10)

To a solution of compound 26-9 (4 g) in CH₂Cl₂ (40 mL), were added 3-(4-aminophenyl)-1,2,4-oxadiazol-5(2*H*)-one (1.46 g; EP1574516A1), 1-hydroxybenzotriazole hydrate (HOBt-H₂O; 0.13g), and WSC-HCl (1.73 g). The reaction mixture was stirred at room temperature for 1.5 hours and it was concentrated in vacuo. The resulting residue was solved in EtOAc, the organic layer was washed with brine and dried with anhyd. Na₂SO₄. The solvent was removed under reduced pressure and the resulting residue was suspended in Hex-Et₂O = 1-1. The precipitate was collected by filtration and rinsed with the above solvent to obtain compound 26-10 (4.45 g) as a pale brown amorphous solid.

### [Step 26-11] Synthesis of tert-butyl N-[4-[2-chloroethyl-[(2R,3R)-2,3-dihydroxy-4-oxo-4-[4-(5-oxo-4H-1,2,4-oxadiazol-3-yl)anilino]butanoyl]amino]phenyl]carbamate ammonium salt (compound 26-11)

According to the Step 7-4 in synthetic method for EXAMPLE 7, compound 26-10 (3.5 g) was used instead of compound 7-3 to obtain compound 26-11 (3.04 g) as a pale brown amorphous solid.

### [Step 26-12] Synthesis of tert-butyl N-[4-[(2R)-2-[(1R)-1-Hydroxy-2-oxo-2-[4-(5-oxo-4H-1,2,4-oxadiazol-3-yl)anilino]ethyl]-3-oxomorpholin-4-yl]phenyl]carbamate (compound 26-12)

According to the Step 7-5 in synthetic method for EXAMPLE 7, compound 26-11 (2.8 g) was used instead of compound 7-4 to obtain compound 26-12 (1.24 g) as an ivory whight amorphous solid.

### [Step 26-13] Synthesis of (2R)-2-[(2R)-4-(4-aminophenyl)-3-oxomorpholin-2-yl]-2-hydroxy-N-[4-(5-oxo-4H-1,2,4-oxadiazol-3-yl)phenyl]acetamide hydrochloride (compound 26-13)

According to the Step 26-5 in synthetic method for EXAMPLE 26, compound 26-12 (1 g) was used instead of compound 26-4 to obtain compound 26-13 (830 mg) as a pale brown amorphous solid.

### [Step 26-14] Synthesis of (2R)-2-[(2R)-4-(4-acetamidophenyl)-3-oxomorpholin-2-yl]-2-hydroxy-N-[4-(5-oxo-4H-1,2,4-oxadiazol-3-yl)phenyl]acetamide (compound 26-14)

According to the Step 26-6 in synthetic method for EXAMPLE 26, compound 26-13 (0.1 g) was used instead of compound 26-5 to obtain compound 26-14 (83 mg) as a colorless amorphous solid.

### [Step 26-B] Synthesis of (2R)-2-1(2R)-4-(4-acetamidophenyl)-3-oxomorpholin-2-yl]-N-(4-amidinophenyl)-2-hydroxyacetamide hydrochloride (EXAMPLE 26)

To a suspension of compound 26-14 (80 mg) in MeOH - I N HCl (8 mL - 8 mL), was added 10% Pd-C (80 mg) at room temperature. The reaction mixture was stirred under H₂ gas atmosphere at room temperature overnight. The reaction mixture was filtered with Celite^{®} pad. The Celite^{®} pad was washed with DMF and the filtrate was concentrated in vacuo. The resulting residue was suspended in MeOH and the precipitate was collected by filtration to obtain EXAMPLE 26 (38 mg) as a colorless amorphous solid.

### (EXAMPLE 38)

### Synthesis of (2R)-2-[(2R)-4-(4-acetamidophenyl)-3-oxomorpholin-2-yl]-N-(1-aminoisoquinolin-6-yl)-2-hydroxyacetamide hydrochloride (EXAMPLE 38)

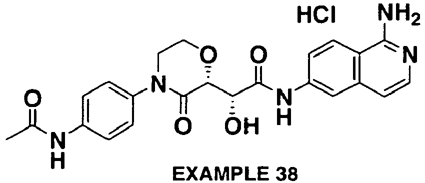

### [Step 38-1] Synthesis of N-[4-(2-chloroethylamino)phenyl]acetamide (compound 38-1)

According to the Step 7-1 in synthetic method for EXAMPLE 7. N-(4-aminophenyl)acetamide (10 g) was used instead of 4-methylaniline to obtain compound 38-1 (7.9 g) as a pale yellow amorphous solid.

### [Step 38-2] Synthesis of (2R,3R)-4-[4-acetamido-N-(2-chloroethyl)anilino]-2,3-diacetyloxy-4-oxobutanoic acid (compound 38-2)

According to the Step 26-9 in synthetic method for EXAMPLE 26, compound 38-1 (7.5 g) was used instead of compound 26-1 to obtain compound 38-2 (15.1 g) as a beige amorphous solid.

### [Step 38-3] Synthesis of [(2R,3R)-1-[4-acetamido-N-(2-chloroethyl)anilino]-3-acetyloxy-4-[[1-[bis[(2-methylpropan-2-yl)oxycarbonyl]amino]isoquinolin-6-yl]amino] -1,4-dioxobutan-2-yl]acetate (compound 38-3)

To a solution of compound 38-2 (0.7 g) in CH₂Cl₂-DMF (30 - 2 mL), was added oxalyl chloride (0.25 mL) at 0 °C. The reaction mixture was stirred for 1 hour in the same temperature. Then the mixture was concentrated in vacuo to remove excess oxalyl chloride. The resulting residue was resolved in CH₂Cl₂ (20 mL) and pyridine (0.19 mL) was added to the above solution at 0 °C. The mixture was stirred for 10 minutes at the same temperature and then a solution of 6-amino-1-bis(tert-butoxy carbonyl)aminoisoquinoline (0.52 g) in CH₂Cl₂ (10 mL) was added to the mixture at 0 °C. The reaction mixture was stirred for 1 hour at 0 °C, then for 2 days at room temperature. MeOH was added to the reaction mixture and the mixture was concentrated in vacuo. Then sat. NaHCO₃ aq. was added to residue and the mixture was extracted with EtOAc. The organic layer was washed with water, brine and dried with anhyd. Na₂SO₄. The solvent was removed under reduced pressure and the resulting residue was purified by silica gel flash chromatography (eluent : Hexane/EtOAc = 1/4) to obtain compound 38-3 (0.52 g) as a pale yellow amorphous solid.

### [Step 38-4] Synthesis of tert-butyl N-[6-[[(2R,3R)-4-[4-acetamido-N-(2-chloroethyl)anilino]-2,3-dihydroxy-4-oxobutanoyl]amino]isoquinolin-1-yl]-N-[(2-meth ylpropan-2-yl)oxycarbonyl]carbamate (compound 38-4)

According to the Step 7-4 in synthetic method for EXAMPLE 7, compound 38-3 (0.5 g) was used instead of 7-3 to obtain compound 38-4 (0.42 g) as a yellow amorphous solid.

### [Step 38-5] Synthesis of (2R)-2-[(2R)-4-(4-acetamidophenyl)-3-oxomorpholin-2-yl]-N-[N,N-bis(tert-butoxycarbonyl)-1-aminoisoquinolin-6-yl]-2-hydroxyacetamide (compound 38-5)

According to the Step 7-5 in synthetic method for EXAMPLE 7, compound 38-4 (0.4 g) and DMF were used instead of 7-4 and t-BuOH-DMSO to obtain compound 38-5 (0.13 g) as a yellow amorphous solid.

### [Step 38-6] Synthesis of (2R)-2-[(2R)-4-(4-acetamidophenyl)-3-oxomorpholin-2-yl]-N-(1-aminoisoquinolin-6-yl)-2-hydroxyacetamide hydrochloride (EXAMPLE 38)

To a solution of compound 38-5 (40 mg) in MeOH (0.4 mL), was added 4N-HCl/EtOAc (1 mL) at 0 °C. The reaction mixture was stirred for 21 hours at room temperature. After the reaction, the precipitate was collected by filtration to obtain EXAMPLE 38 (22 mg) as a pale yellow amorphous solid.

### (EXAMPLE 52)

### Synthesis of (2R)-N-(1-aminoisoquinolin-6-yl)-2-[(2R)-4-(4-tert-butylphenyl)-3-oxomorpholin-2-yl]-2-hydroxyacetamide hydrochloride (EXAMPLE 52)

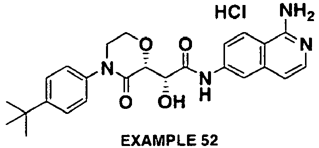

### [Step 52-1] Synthesis of tert-butyl (2R,3R)-2,3-diacetyloxy-4-[4-tert-butyl-N-(2-chloroethyl)anilino]-4-oxobutanoate (compound 52-1)

To a solution of (R, R)-2,3-bis(acetyloxy)-bucanedioic acid mono tert-butyl ester (9 g: Tetrahedron, 45, 3071-3080, 1989) in CH₂Cl₂ (90 mL), were added compound 11-1 (6.6 g) and 1-ethyl-3-(3'-dimethylaminopropyl)carbodiimide hydrochloride at 0 °C. The reaction mixture was stirred for 5 hours at room temperature. Then water was added into the mixture and it was extracted with CH₂Cl₂. The organic layer was washed with brine, dried with anhyd. Na₂SO₄. The solvent was removed under reduced pressure to obtain compound 52-1 (15.8 g) as a brown amorphous solid.

### [Step 52-2] Synthesis of tert-butyl (2R,3R)-4-[4-tert-butyl-N-(2-chloroethyl)anilino]-2.3-dihydroxy-4-oxobutanoate (compound 52-2)

According to the Step 7-4 in synthetic method for EXAMPLE 7, compound 52-1 (15.5 g) was used instead of 7-3 to obtain compound 52-2 (13 g) as a brown oil.

### [Step 52-3] Synthesis of tert-butyl (2R)-2-[(2R)-4-(4-tert-butylphenyl)-3-oxomorpholin-2-yl]-2-hydroxyacetate (compound 52-3)

According to the Step 7-5 in synthetic method for EXAMPLE 7, compound 52-2 (12.8 g) was used instead of 7-4 to obtain compound 52-3 (4.85 g) as a pale yellow amorphous solid.

### [Step 52-4] Synthesis of (2R)-2-[(2R)-4-(4-tert-butylphenyl]-3-oxomorpholin-2-yl]-2-hydroxyacetic acid (compound 52-4)

Compound 52-3 (1.5 g) was resolved in 4N HCl-dioxane (30 mL). The reaction mixture was stirred at room temperature overnight. The mixture was concentrated in vacuo to obtain compound 52-4 (1.44 g) as a beige amorphous hygroscopic solid.

### [Step 52-5] Synthesis of (2R)-N-[N,N-bis(tert-butoxycarbonyl)-1-aminoisoquinolin-6-yl]-2-[(2R)-4-(4-tert-butylphenyl)-3-oxomorpholin-2-yl]-2-hydroxyacetamide (compound 52-5)

According to the Step 1-3 in synthetic method for EXAMPLE 1, 52-4 (0.38 g) was used instead of 1-2 to obtain compound 52-5 (92 mg) as a colorless amorphous solid.

### [Step52-6] Synthesis of (2R)-N-(1-aminoisoquinolin-6-yl)-2-[(2R)-4-(4-tert-butylphenyl)-3-oxomorpholin-2-yl]-2-hydroxyacetamide hydrochloride (EXAMPLE 52)

According to the Step 38-6 in synthetic method for EXAMPLE 38. 52-5 (91 mg) was used instead of 38-5 to obtain EXAMPLE 52 (60 mg) as a colorless amorphous solid.

### (EXAMPLE 54)

### Synthesis of (2R)-N-(1-aminoisoquinolin-6-yl)-2-hydroxy-2-[(2R)-3-oxo-4-[4-(trifluoromethyl)phenyl]morpholin-2-yl]acetamide hydrochloride (EXAMPLE 54)

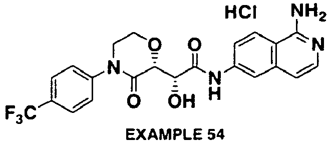

### [Step 54-1] Synthesis of N-(2-chloro-N-[4-(trifluoromethyl)phenyl]acetamide (compound 54-1)

According to the Step 20-1 in synthetic method for EXAMPLE 20, 4-trifluoromethyl aniline (5 g) was used instead of 5-amino-1,3-dihydro-2H-benzimidazol-2-one to obtain compound 54-1 (6.94 g) as a brown amorphous solid.

### [Step 54-2] Synthesis of N-(2-chloroethyl)-4-(trifluoromethyl)aniline (compound 54-2)

According to the Step 20-2 in synthetic method for EXAMPLE 20, compound 54-1 (3.5 g) was used instead of 20-1 to obtain compound 54-2 (3.36 g) as brown oil.

### [Step 54-3] Synthesis of tert-butyl (2R,3R)-2,3-diacetyloxy-4-[N-(2-chloroethyl)-4-(trifluoromethyl)anilino]-4-oxobutanoate (compound 54-3)

To a solution of (R, R)-2,3-bis(acetyloxy)-butanedioic acid mono tert-butyl ester (3.25 g: Tetrahedron, 45, 3071-3080, 1989) in CH₂Cl₂ (65 mL), were added oxalyl chloride (1.06 mL) and DMF (50 microL) at 0 °C. The reaction mixture was stirred at 0 °C for 30 minutes then pyridine (3.82 mL) was added into the mixture at the same temperature. The reaction mixture was stirred at the same temperature for 5 minutes. To the mixture, was added a solution of compound 54-2 (2.5 g) in CH₂Cl₂ (12.5 mL) at 0 °C. The mixture was stirred for 1 hour at the same temperature. Then the mixture was concentrated in vacuo and the resulting residue was suspended in water. The mixture was extracted with EtOAc and the organic layer was washed with I N HCl aq., sat. NaHCO₃ aq., brine, and it was dried with anhyd. Na₂SO₄. The solvent was removed under reduced pressure to obtain compound 54-3 (5.7 g) as brown oil.

### [Step 54-4] Synthesis of tert-butyl (2R,3R)-4-[N-(2-chloroethyl)-4-(trifluoromethyl)anilino]-2,3-dihydroxy-4-oxobutanoate (compound 54-4)

According to the Step 7-4 in synthetic method for EXAMPLE 7, compound 54-3 (5.5 g) was used instead of 7-3 to obtain compound 54-4 (4.57 g) as a brown amorphous solid.

### [Step 54-5] Synthesis of tert-butyl (2R)-2-hydroxy-2-[(2R)-3-oxo-4-[4-(trifluoromethyl)phenyl]morpholin-2-yl]acetate (compound 54-5)

According to the Step 7-5 in synthetic method for EXAMPLE 7, compound 54-4 (3 g) was used instead of 7-4 to obtain compound 54-5 (220 mg) as a pale yellow amorphous solid.

### [Step 54-6] Synthesis of (2R)-2-hydroxy-2-[(2R)-3-oxo-4-[4-(trifluoromethyl)phenyl]morpholin-2-y]acetic acid (compound 54-6)

According to the Step 52-4 in synthetic method for EXAMPLE 52, compound 54-5 (0.2 g) was used instead of 52-3 to obtain compound 54-6 (128 mg) as a colorless amorphous solid.

### [Step 54-7] Synthesis of (2R)-N-[N,N-bis(tert-butoxycarbonyl)-1-aminoisoquinolin-6-yl]-2-hydroxy-2-[(2R)-3-oxo-4-[4-(trifluoromethyl)phenyl]morpholin-2-yl]acetamide (compound 54-7)

According to the Step 1-3 in synthetic method for EXAMPLE 1, compound 54-6 (128 mg) was used instead of 1-2 to obtain compound 54-7 (107 mg) as a beige amorphous solid.

### [Step 54-8] Synthesis of (2R)-N-(-aminoisoquinolin-6-yl)-2-hydroxy-2-[(2R)-3-oxo-4-[4-(trifluoromethyl)phenyl]morpholin-2-yl]acetamide hydrochloride (EXAMPLE 54)

According to the Step 38-6 in synthetic method for EXAMPLE 38, 54-7 (50 mg) was used instead of 38-5 to obtain EXAMPLE 54 (28.3 mg) as a leaf green amorphous solid.

### (EXAMPLE 57)

### Synthesis of (2R)-2-hydroxy-N-(1-imino-2,3-dihydroisoindol-5-yl)-2-[(2R)-3-oxo-4-[4-(trifluoromethoxy)phenyl]morpholin-2-yl]acetamide hydrochloride (EXAMPLE 57)

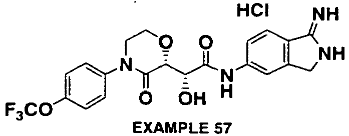

### [Step 57-1] Synthesis of N-[[2-cyano-5-[[(2R)-2-hydroxy-2-[(2R)-3-oxo-4-[4-(trifluoromethoxy)phenyl]morpholin-2-yl]acetyl]amino]phenyl]methyl]-N-[(2-methylp ropan-2-yl)oxycarbonyl]carbamate (compound 56-1)

According to the Step 1-3 in synthetic method for EXAMPLE 1, a cyclic carbonate analogue (0.15 g) derived from compound 55-6 and 39-2 (0.14 g) were used instead of 1-2 and 6-amino-1-bis(tert-butoxycarbonyl)aminoisoquinoline to obtain compound 57-1 (147 mg) as a pale yellow amorphous solid.

### [Step 57-2] Synthesis of (2R)-N-(3-(aminomethyl)-4-cyanophenyl]-2-hydroxy-2-[(2R)-3-oxo-4-[4-(trifluoromethoxy)phenyl]morpholin-2-yl]acetamide hydrochloride (compound 57-2)

According to the Step 38-6 in synthetic method for EXAMPLE 38, 57-1 (0.14 g) was used instead of 38-5 to obtain compound 57-2 (93 mg) as a pale yellow amorphous solid.

### [Step 57-3] Synthesis of (2R)-2-hydroxy-N-(-imino-2,3-dihydroisoindol-5-yl)-2-[(2R)-3-oxo-4-[4-(trifluoromethoxy)phenyl)morpholin-2-yl]acetamide hydrochloride (EXAMPLE 57)

According to the Step 39-7 in synthetic method for EXAMPLE 39, 57-3 (89 mg) was used instead of 39-6 to obtain EXAMPLE 57 (66.4 mg) as a pale yellow amorphous solid.

### (EXAMPLE 68)

### Synthesis of (R)-N-(4-carbamimidoylphenyl)-2-hydroxy-2-((R)-3-oxo-4-(3-oxo-2-propylisoindolin-5-yl)morpholin-2-yl)acetamide hydrochloride (EXAMPLE 68)

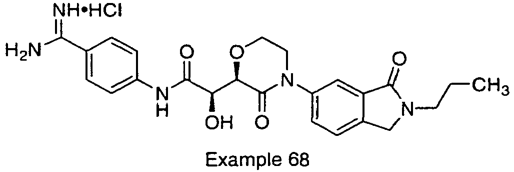

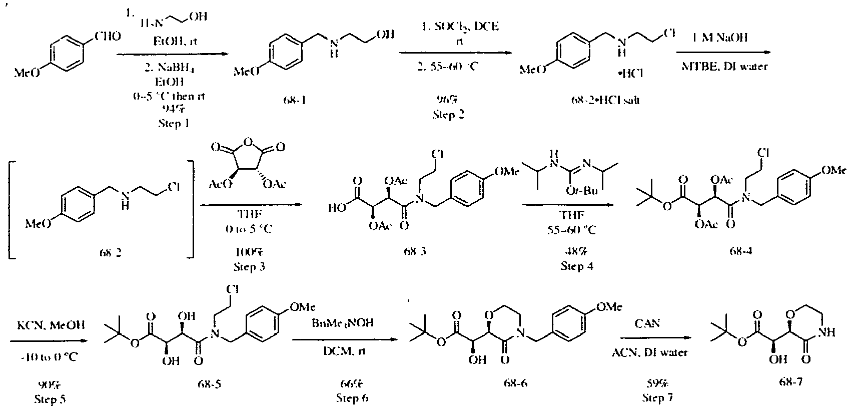

### [Step 68-1] Synthesis of compound 68-1.

A 100-L glass-jacketed reactor was charged with 4-methoxybenzaldehyde (3440 g, 25.3 mol, Aldrich lot # 05826MH and 20098PJ) and absolute ethanol (34.4 L). 2-Aminoethanol (1840 mL, 30.0 mol, Aldrich lot # 06201PE) was added over 30 minutes while maintaining the temperature of the batch between 20 and 30 °C. After the addition was complete, the batch was held at 20-25 °C for 2 hours until formation of the imine intermediate was deemed complete by ¹H NMR analysis (DMSO-d₆, aldehyde peak at 9.8 ppm not observed). The batch was cooled to 0-5 °C and sodium borohydride (1050 g, 27.8 mol, Aldrich lot # 10106TC) was added portionwise over 2.8 hours while maintaining the temperature of the batch between 0 and 10 °C. Once the addition was complete, the batch was allowed to gradually warm to 20-25 °C (20 °C/hour) and was held at this temperature for 16 hours until the reduction of the imine intermediate was deemed complete by HPLC analysis [<1.0% (AUC) of imine by HPLC]. The reaction mixture was quenched by carefully adding 1 M aqueous sodium hydroxide (25.0 L) to the batch. This process led to the formation of insoluble masses of solid. These solids were dissolved by adding DI water (25.0 L) and the quenched was resumed. The batch was extracted with dichloromethane (CH₂Cl₂, 3 × 17.2 L). The combined organic extracts were concentrated on a rotary evaporator at 40-45 °C until distillation ceased and then the concentrate was slurried in DI water (17.2 L). The batch was cooled to 10-15 °C and the pH was adjusted to 1-2 using concentrated hydrochloric acid (2.2 L). The batch was washed with tert-butyl methyl ether (MTBE, 3 × 10.3 L), cooled to 10-15 °C and the pH was adjusted to 13-14 using 6 M aqueous sodium hydroxide (6.0 L). The batch was extracted with CH₂Cl₂ (2 × 10.3 L). The combined organic extracts were dried over anhydrous sodium sulfate, filtered and the filter cake was washed with CH₂Cl₂ (6.0 L). The filtrate was concentrated on a rotary evaporator at 30-35 °C until distillation ceased to afford compouond 68-1 (4325 g, 94%).

### [step 68-2] Synthesis of compound 68-2.

A 100-L glass-jacketed reactor was charged with compound 68-1 (4325 g, 23.9 mol) and 1,2-dichloroethane (86.5 L). Thionyl chloride (1900 mL, 26.1 mol, Aldrich lot # 05497DJ) was added over 50 minutes while maintaining the temperature of the batch between 20 and 30 °C. Once the addition was complete, the batch was heated to 55-60 °C and held at this temperature for 5.5 hours until the reaction was deemed complete by ¹H NMR analysis (DMSO-d₆, doublets at 7.3 ppm and 6.9 ppm shifted to 7.5 ppm and 7.0 ppm respectively, and doublets at 9.2 min and multiplet at 4.4 ppm disappeared). The batch was cooled to 20-25 °C and concentrated using a rotavap at 40-45 °C until distillation ceased. The concentrate was swapped once with MTBE (22.0 L), slurried in MTBE (21.7 L) and filtered to afford 68-2•HCl (5420 g, 96%) as white solids after drying in a vacuum oven at 20-30 °C for 17 hours.

### [Step 68-3] Synthesis of compound 68-3.

A 50-L glass-jacketed reactor was charged with compound 68-2•HCl (1940 g, 8.2 mol), DI water (19.4 L) and MTBE (19.4 L). The pH of the aqueous layer was adjusted to 11-12 using 1 M aqueous sodium hydroxide (10.5 L) and maintaining the temperature of the batch between 15 and 30 °C. The phases were separated and the aqueous layer was extracted with MTBE (2 × 9.7 L). The combined organic extracts were dried over anhydrous sodium sulfate, filtered, washed with MTBE (8.0 L) and the filtrate was concentrated on a rotary evaporator at 20-25 °C until distillation ceased, affording free amine 68-2 (1720 g, containing 6.8 wt % of MTBE by ¹H NMR (DMSO-d₆), corrected weight: 1604 g, 98%)
A 50-L glass-jacketed reactor was charged with di-O-acetyl-L-tartaric anhydride (1775 g, 8.2 mol, Alfa Aesar lot # E13U033) and tetrahydrofuran (17.5 L, THF). The batch was cooled to 0-5 °C and a solution of compound 68-2 (1720 g) in THF (2.0 L) was added over 1.3 hours while maintaining the temperature of the batch between 0 and 10 °C. The batch was held at 0-5 °C for 18 hours until the reaction was deemed complete by HPLC analysis [2.8% (AUC) of compound 68-2 remaining] and then it was concentrated on a rotary evaporator at 20-25 °C until distillation ceased to afford compound 68-3 [4485 g, containing 22.7 wt % of THF by ¹H NMR (DMSO-d₆), corrected weight: 3467 g, 102%, 86.9 % (AUC) by HPLC].

### [Step 68-4] Synthesis of compound 68-4.

A 50-L glass-jacketed reactor was charged with compound 68-3 (3520 g, assuming theoretical yield for step 3, 8.5 mol) and THF (35.0 L), and the batch was heated to 50-60 °C. Two portions of *O-tert*-butyl-*N,N*-diisopropylurea (2115 g, 10.6 mol, and 1700 g, 8.9 mol) were each added dropwise over 30 minutes while maintaining the temperature of the batch between 50 and 60 °C. In-process assay by HPLC analysis after these additions were complete indicated that 19.5% (AUC) of compound 68-3 remained and that 70.9% (AUC) of compound 68-4 had formed. Additional *O-tert*-butyl-*N,N*-diisopropylurea (2 × 425 g, 4.2 mol) was added to the batch until the reaction was deemed complete by HPLC analysis [4.4% (AUC) of compound 68-3 remaining]. The batch was cooled to 15-25 °C and MTBE (19.4 L) was added. The batch was filtered over Celite^{®} and washed with MTBE (15.0 L). The combined filtrate and washes were concentrated on a rotary evaporator at 40-45 °C until distillation ceased to afford crude compound 68-4 [4675 g, containing 10.6 wt % of THF by ¹H NMR (DMSO-d₆), corrected weight 4180 g, 105%, 55.7% (AUC) by HPLC]. This material was purified by silica-gel column chromatography (Four 1.1 to 1.3-kg batches using 5.5 kg of silica gel each, 20 to 60% EtOAc in heptane) to afford compound 68-4 [1915 g, 48%, 96.7-97.1% (AUC) by HPLC] as well as mixed fractions that were combined with other lots for further purification.

### [Step 68-5] Synthesis of compound 68-5.

A 50-L glass-jacketed reactor was charged with compound compound 68-4 (1100 g, 2.3 mol) and methanol (10.2 L), and the batch was cooled to -10 to 0 °C. A slurry of potassium cyanide (80 g, 1.2 mol, Aldrich lot # 14614KA) in methanol (800 mL) was added over 5 minutes while maintaining the temperature of the batch between -10 and 0 °C. The batch was held at -10 to 0 °C for 3.3 hours until the reaction was deemed complete by HPLC analysis [5.3% (AUC) of compound 68-4 remaining]. Solid sodium bicarbonate (200 g, 2.4 mol, Natrium Products lot # 01096A) was added and the batch was concentrated on a rotary evaporator at 20-25 °C until distillation ceased. MTBE (11.0 L) and DI water (11.0 L) were added to the concentrate, and the layers were separated. The organic layer was washed with saturated aqueous sodium bicarbonate (6.0 L), dried over anhydrous sodium sulfate, filtered, washed with MTBE (7.0 L) and concentrated on a rotary evaporator at 20-25 °C until distillation ceased to yield compound 68-5 [910 g, containing 10.2 wt % of MTBE by ¹H NMR (CDCl₃), corrected weight 817 g, 90%, 82.4% (AUC) by HPLC]. This material was stored in the freezer.

### [Step 68-6] Synthesis of compound 68-6.

A 50-L glass-jacketed reactor was charged with compound 68-5 (817 g, 2.1 mol), CH₂Cl₂ (8.2 L) and deionized water (1.9 L). Benzyltrimethylammonium hydroxide (1912 mL, 40 wt % in methanol, 4.2 mol, Aldrich lot # 10896HJ) was added to the batch over 10 minutes while maintaining the temperature between 20 and 25 °C. The batch was held at 20-25 °C for 1.5 hours until the reaction was deemed complete by HPLC analysis [<1.0% (AUC) of compound 68-5 remaining]. At completion of the reaction, DI water (6.5 L) was added and the layers were separated. The aqueous layer was extracted with CH₂Cl₂ (8.2 L). The combined organic extracts were washed with brine (8.2 L), dried over anhydrous sodium sulfate, filtered and washed with CH₂Cl₂ (2.5 L). The combined filtrate and washes were concentrated on a rotary evaporator at 30-35 °C until distillation ceased to afford crude compound 68-6 [625 g, 84%, 82.5% (AUC) by HPLC]. This material was purified by silica-gel column chromatography [5 kg of silica gel, 20 to 60% EtOAc in heptane] and the pure fractions were slurried in 1:4 MTBE/heptane to yield compound 68-6 [Two lots: 155 g, 21%, >99% (AUC) by HPLC, and 335 g, 45%, >99% (AUC) by HPLC] as white solids.

### [Step 68-7] Synthesis of compound 68-7.

A 50-L glass-jacketed reactor was charged with compound 68-6 (490 g, 1.4 mol), acetonitrile (10.1 L) and DI water (2.0 L). The batch was cooled to 0-5 °C and a slurry of cerium (IV) ammonium nitrate (3060 g, 5.6 mol, Alfa Aesar lot # H22T016) in acetonitrile (8.0 L) was added while maintaining the temperature of the batch between 0 and 5 °C. The batch was held at 0-5 °C for 30 minutes, warmed to 20-25 °C and held at this temperature for 3.5 hours until the reaction was deemed complete by HPLC analysis. Saturated aqueous sodium bicarbonate (17.0 L) was added until the pH of the reaction mixture reached 4.5 to 5. The resulting suspension was filtered over Celite^{®} and the filter cake was washed with CH₂Cl₂ (2 × 10.0 L) followed by 5% methanol in CH₂Cl₂ (10.0 L). The combined filtrate and washes were transferred to a 50-L, glass-jacketed reactor and the phases were separated. The aqueous layer was extracted with CH₂Cl₂ (15.0 L). The combined organic extracts were washed with brine, dried over anhydrous sodium sulfate, filtered and the filter cake was washed with CH₂Cl₂ (10.0 L). The combined filtrate and washes were concentrated on a rotary evaporator at 30-35 °C until distillation ceased to give crude compound 68-7 [700 g, >100%]. This material was purified by silica-gel column chromatography [4 kg of silica gel, I to 5% methanol in dichloromethane]. The pure fractions were slurried in 1:4 CH₂Cl₂/MTBE to yield compound 68-7 [Two lots: 189 g, 59%] as white to off-white solids.

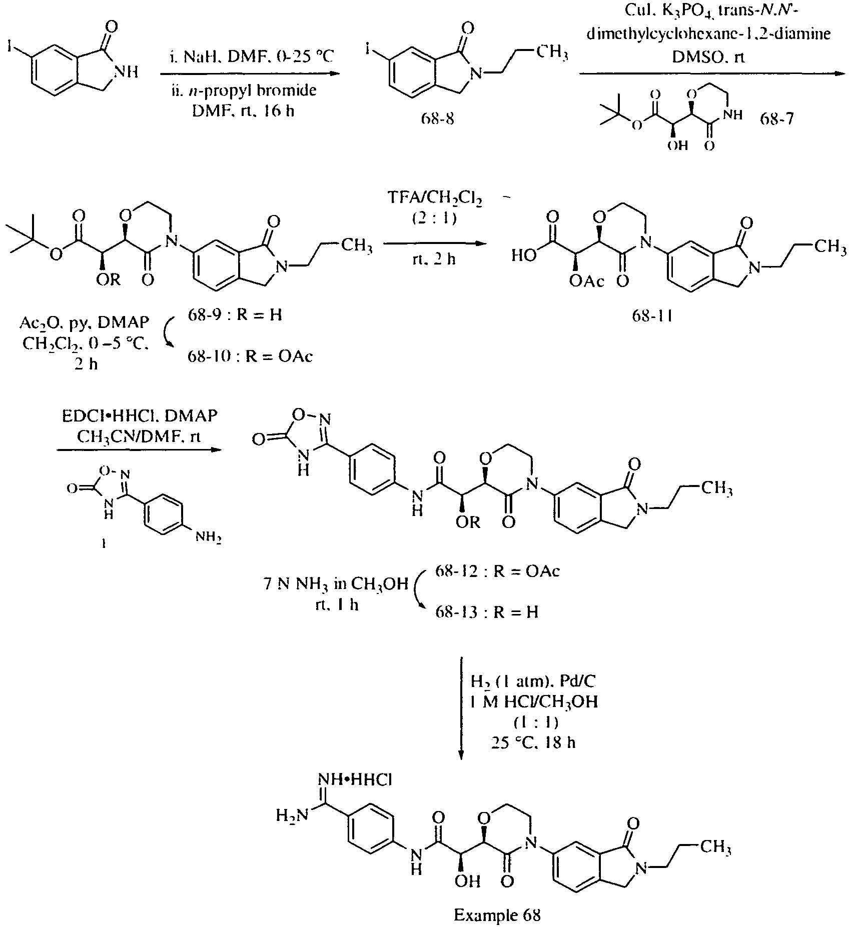

### [Step 68-8] Synthesis of compound 68-8.

To a 50 mL round bottom flask was added 5-iodo-3-oxo-isoindolin (1.00 g, 3.86 mmol) in DMF (10.0 mL) and the reaction mixture was stirred at 0-5 °C. NaH (60% in oil, 186 mg, 4.65 mmol) was added, and the reaction mixture was allowed to warm to room temperature. After 20 min, the reaction obtained a green color, and a solution of *n*-propyl bromide (706 mg, 5.78 mmol) in DMF (2.00 mL) was added dropwise over a period of 15 min. The reaction mixture was stirred overnight at room temperature, then diluted with EtOAc (100 mL), washed with saturated aqueous NH₄Cl (2 × 25 mL), saturated aqueous LiCl (25 mL), brine (25 mL), dried over Na₂SO₄, filtered and concentrated under reduced pressure. The resulting residue was purified by CombiFlash (80 g, Hex/EtOAc, 100:0 to 30:70 over 30 min) to provide compound 68-8 (700 mg, 60%) as a white solid.

### [Step 68-9] Synthesis of compound 68-9.

A mixture of compound 68-7 (100 mg, 0.432 mmol), aryl iodide compound 68-8 (156 mg, 0.518 mmol), CuI (8.1 mg, 43 µmol), K₃PO₄ (183 mg, 0.861 mmol), DMSO (1.5 mL), and *trans*-*N,N*'-dimethylcyclohexane-1,2-diamine (13.6 µL, 86.1 µmol) were stirred at room temperature under nitrogen in the dark. After 14 h, additional CuI (8.1 mg, 43 µmol) and *trans*-*N,N*'-dimethylcyclohexane-1,2-diamine (13.6 µL, 86.1 µmol) were added and the mixture was stirred for an additional 2.5 h. The mixture was diluted with EtOAc (100 mL), washed with water (3 × 25 mL), brine (25mL), dried over Na₂SO₄, filtered, and concentrated under reduced pressure. The crude product was purified on CombiFlash (12 g SiO₂, Hex/EtOAc, 100:0 to 0:100 over 45 min) to provide pure product compound 68-9 (120 mg, 69%) as a yellow solid.

### [Step 68-10]

### Synthesis of compound 68-10.

To a 50 mL round bottom flask was added compound 68-9 (114 mg, 0.281 mmol), DMAP (3.4 mg, 28 µmol), pyridine (45 µL, 0.56 mmol) and CH₂Cl₂ (5.00 mL). The reaction mixture was cooled to 0-5 °C, Ac₂O (53 µL, 0.56 mmol) was added, stirred at 0-5 °C for 2 h, and then diluted with EtOAc (40 mL), washed with saturated aqueous CuSO₄ (2 × 25 mL), brine (25 mL), dried over Na₂SO₄, filtered and concentrated under reduced pressure to provide crude compound 68-10 (125 mg, quant) as a yellow solid. The product was used in the next reaction without any further purification.

### [Step 68-11] Synthesis of compound 68-11.

In a 50 mL round bottom flask containing compound 68-10 (125 mg, 0.281 mmol) was added CH₂Cl₂ (1.00 mL) and TFA (2.00 mL). The reaction mixture was stirred at room temperature for 2 h, then TFA and CH₂Cl₂ were removed under reduced pressure. The crude product was triturated with ether to provide pure 68-11 (110 mg, quant.) as a yellow solid.

### [Step 68-12] Synthesis of compound 68-12.

To a 100 mL round bottom flask was added compound 68-11 (110 mg, 0.281 mmol), DMAP (3.4 mg, 28 µmol), and 3-(4-aminophenyl)-1,2,4-oxadiazol-5(2*H*)-one 1 (53.1 mg, 0.299 mmol) in CH₃CN (3.00 mL). The reaction mixture was cooled to 0-5 °C, then EDCI•HCl (57.5 mg, 0.299 mmol) was added and the reaction mixture was warmed to room temperature. After 30 min DMF (1.00 mL) was added to dissolve the precipitate and stirring was continued for an additional 3 h. The solvents were removed under reduced pressure and the residue was triturated with ether (20.0 mL) then decanted. The undissolved material was washed with water (2 × 5 mL) and acetonitrile (2 × 5 mL) then dried on under vacuum to provide pure product 68-12 (95 mg, 62%) as off-white solid.

### [Step 68-13] Synthesis of compound 68-13.

To a 50 mL round bottom flask was added compound 68-12 (95 mg, 0.17 mmol CH₃OH (2.00 mL) and 7 N NH₃ in CH₃OH (6.00 mL). The reaction mixture was stirred at room temperature for 1 h then the volatiles were removed under reduced pressure. Additional CH₃OH (2 × 50 mL) was used to strip off excess NH₃. The crude product was redissolved in CH₃OH (50 mL) and the solvent degassed with N₂ to remove trace ammonia then concentrated under reduced pressure to provide compound 68-13 (85 mg, 99%) as off-white solid.

### [Step 68-14] Synthesis of (R)-N-(4-carbamimidoylphenyl)-2-hydroxy-2-((R)-3-oxo-4-(3-oxo-2-propylisoindolin-5-yl)morpholin-2-yl)acetamide hydrochloride (EXAMPLE 68)

To a 250 mL round bottom flask was added compound 68-13 (84 mg, 0.17 mmol) in CH₃OH (6.00 mL) and 1 M HCl (6.00 mL). The solvent was degassed for 10 min with N₂, then 10% Pd/C (84 mg, 39 µmol) was added and the reaction mixture was hydrogenated at I atm overnight. The mixture was diluted with hot CH₃OH (250 mL), filtered and the filtrate was concentrated under reduced pressure. The residue was triturated with CH₃OH (5.00 mL) and filtered to provide pure product compound 68 (51 mg, 64%) as off-white solid.

### (EXAMPLE 70)

### Synthesis of (R)-N-(4-Carbamimidoylphenyl)-2-hydroxy-2-((R)-4-(2-methyl-3-oxoisoindolin-5-yl)-3-oxomorpholin-2-yl)acetamide hydrochloride (EXAMPLE 70)

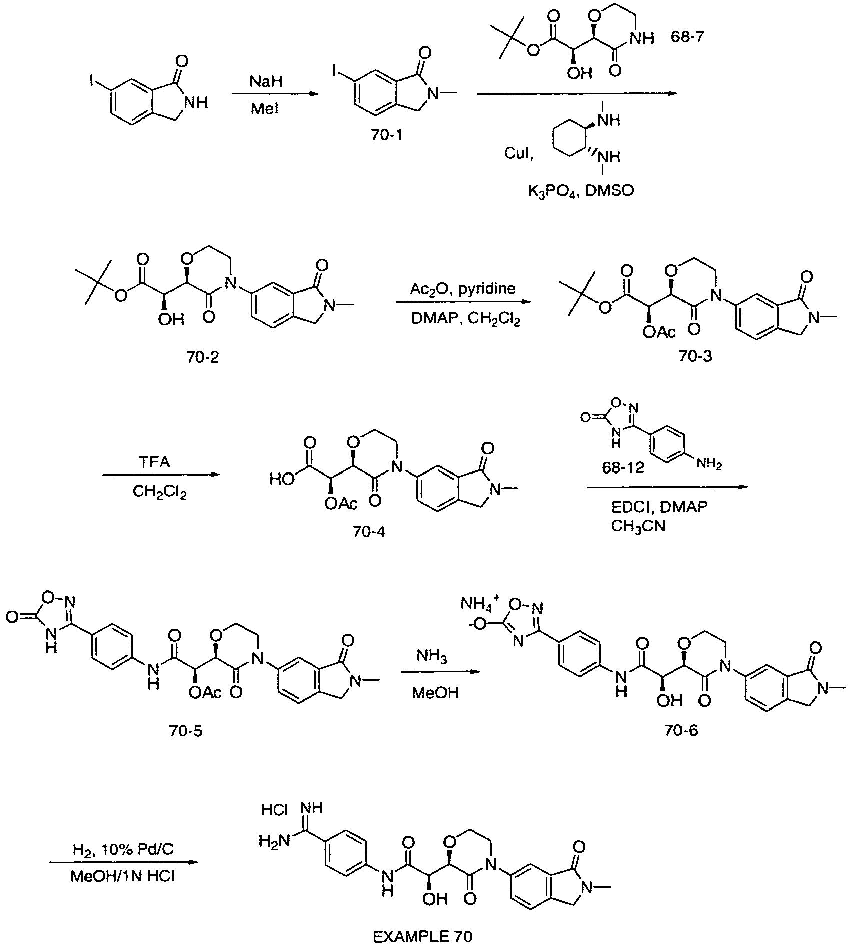

### [Step 70-1] Synthesis of compound 70-1.

To a mixture of 2,3-dihydro-6-iodo-1H-isoindol-1-one (1.0 g) in DMF (20 mL) at 0 °C was added NaH (97 mg) in a single portion. The resulting mixture was stirred for 30 min at 0 °C whereupon MeI (0.25 mL) was added dropwise. The mixture was allowed to warm to rt and was stirred for 72 h. The mixture was quenched by addition of sat. aq. NH₄Cl (- 3 mL) and was diluted with EtOAc (10 mL). The layers were separated and the aqueous layer was extracted with EtOAc. The organic layers were combined and washed sequentially with water and brine. The organic layer was dried (Na₂SO₄), filtered, and concentrated under reduced pressure. The crude material was purified The crude product was purified by flash chromatography (ISCO, 120 g) using a gradient of 100% hexanes to 80:20 hexanes/EtOAc to afford compound 70-1 (0.84 g) as a yellow solid. LC-MS: M+H = 274.

### [Step 70-2] Synthesis of (R)-tert-butyl 2-hydroxy-2-((R)-4-(2-methyl-3-oxoisoindolin-5-yl)-3-oxomorpholin-2-yl)acetate (compound 70-2)

To a round bottom flask charged with a stir bar was added morpholinone compound 68-7 (0.28 g) and compound 70-1 (0.40 g) in DMSO (8 mL) at rt was added K₃PO₄ (0.51 g), and CuI (23 mg) under N₂. *trans-N,N'*-Dimethylcyclohexane-1,2-diamine (37 µL) was added dropwise and the mixture was affixed with a condenser. The mixture was degassed under vacuum (- 20 mm), filled with N₂, and heated to 80 °C. The mixture stirred for 2.5 h at 80 °C, cooled to rt, and was diluted with EtOAc. The mixture was then sequentially washed with conc NH₄OH, water, and brine. The organic layer was dried (Na₂SO₄), filtered, and concentrated under reduced pressure to afford a yellow oil. The crude product was purified by flash chromatography using a gradient of 100% CH₂Cl₂ to 60% CH₂Cl₂/40% MeOH to afford compound 70-2 (0.23 g) of a yellow solid. LC-MS: M+H = 377.

### [Step 70-3] Synthesis of (R)-tert-butyl 2-acetoxy-2-((R)-4-(2-methyl-3-oxoisoindolin-5-yl)-3-oxomorpholin-2-yl)acetate (compound 70-3)

To a solution of compound 70-2 (80 mg) in CH₂Cl₂ (2 ml) at 0°C was added pyridine (26 µL), Ac₂O (30 µl), and DMAP (4 mg). The mixture was stirred for 1 hour at 0°C, warmed to rt, and stirred for an additional 12 h. The mixture was diluted with EtOAc and the organic layer was washed sequentially with sat. aq. CuSO₄ solution, water, and brine. The organic layer was dried (Na₂SO₄), filtered, and concentrated under reduced pressure to afford compound 70-3 (85 mg) as a light yellow semisolid. LC-MS: M+H = 419. This material was used without further purification.

### [Step 70-4] Synthesis of (R)-2-acetoxy-2-((R)-4-(2-methyl-3-oxoisoindolin-5-yl)-3-oxomorpholin-2-yl)acetic acid (compound 70-4)

To a solution of compound 70-3 (85 mg) in CH₂Cl₂ (2.5 mL) at 0°C was added TFA (0.5 mL) dropwise. The mixture was stirred for 1h at 0°C and at rt for 30 min whereupon an additional portion of TFA (0.5 mL) was added. After an additional 1 h at rt, the mixture was diluted with CH₂Cl₂ and concentrated to dryness under reduced pressure. The crude mixture was redissolved in CH₂Cl₂ and concentrated and this protocol was repeated 5 times with to afford compound 70-4 (65 mg) as a light semisolid. LC-MS: M+H = 363. This material was used without further purification.

### [Step 70-5] Synthesis of (R)-1-((R)-4-(2-methyl-3-oxoisoindolin-5-yl)-3-oxomorpholin-2-yl)-2-oxo-2-(4-(5-oxo-4,5-dihydro-1,2,4-oxadiazol-3-yl)phenylamino) ethyl acetate (compound 70-5)

To a solution of compound 70-4 (40 mg) in CH₃CN (1 mL) at 0 °C was added 4-(5-oxo-4,5-dihydro-[1,2,4]oxadiazol-3-yl)-phenyl amide (21 mg) followed by EDCI (25 mg). The reaction mixture was warmed to rt and stirred for 72 h. The mixture was concentrated under reduced pressure and placed under high vacuum. The crude material was purified by reverse phase HPLC using a C18 column and a gradient of (89.95:9.95:0.1 H₂O:MeCN:HCO₂H to 9.95:89.95:0.1 H₂O:MeCN:HCO₂H) to afford compound 70-5 (30 mg) as a white solid. LC-MS: M+H = 522.

### [Step 70-6] Synthesis of ammonium 3-(4-((R)-2-hydroxy-2-((R)-4-(2-methyl-3-oxoisoindolin-5-yl)-3-oxomorpholin-2-yl)acetamido)phenyl)-1,2,4-oxadiazol-5-olate (compound 70-6)

To a solution of the compound 70-5 (30 mg) in MeOH (2 mL) at 0 "C was added 7M NH₃/MeOH (0.3 mL) dropwise. The mixture was stirred for 1 h at 0 °C and an additional hour at rt. The mixture was concentrated under reduced pressure and placed under high vacuum to afford compound 70-6 (27 mg) as a clear glass. LC-MS: M+H = 480 (free base).

### [Step 70-7] Synthesis of (R)-N-(4-carbamimidoylphenyl)-2-hydroxy-2-((R)-4-(-methyl-3-oxoisoindolin-5-yl)-3-oxomorpholin-2-yl)acetamide hydrochloride (EXAMPLE 70)

To a solution of the compound 70-6 (27 mg) in MeOH (2 mL) was added I N HCl (2 mL) followed by 10% Pd/C (50 mg). The mixture was stirred under a H₂ balloon for 3 h and was filtered through a pad of Celite^{®}. The Celite^{®} pad was washed with MeOH and the resultant filtrate was concentrated under reduced pressure. The crude residue was treated with MeOH followed by dilution with Et₂O and the resultant solid was collected by filtration and dried under vacuum to afford 17 mg of Example 70 as a maize solid. LC-MS: M+H = 438 (free base).

### (EXAMPLE 73)

### Synthesis of (R)-2-((R)-4-(3,5-bis(trifluoromethyl)phenyl)-3-oxomorpholin-2-yl)-N-(4-carbamimidoylphenyl)-2-hydroxyacetamide hydrochloride (EXAMPLE 73)

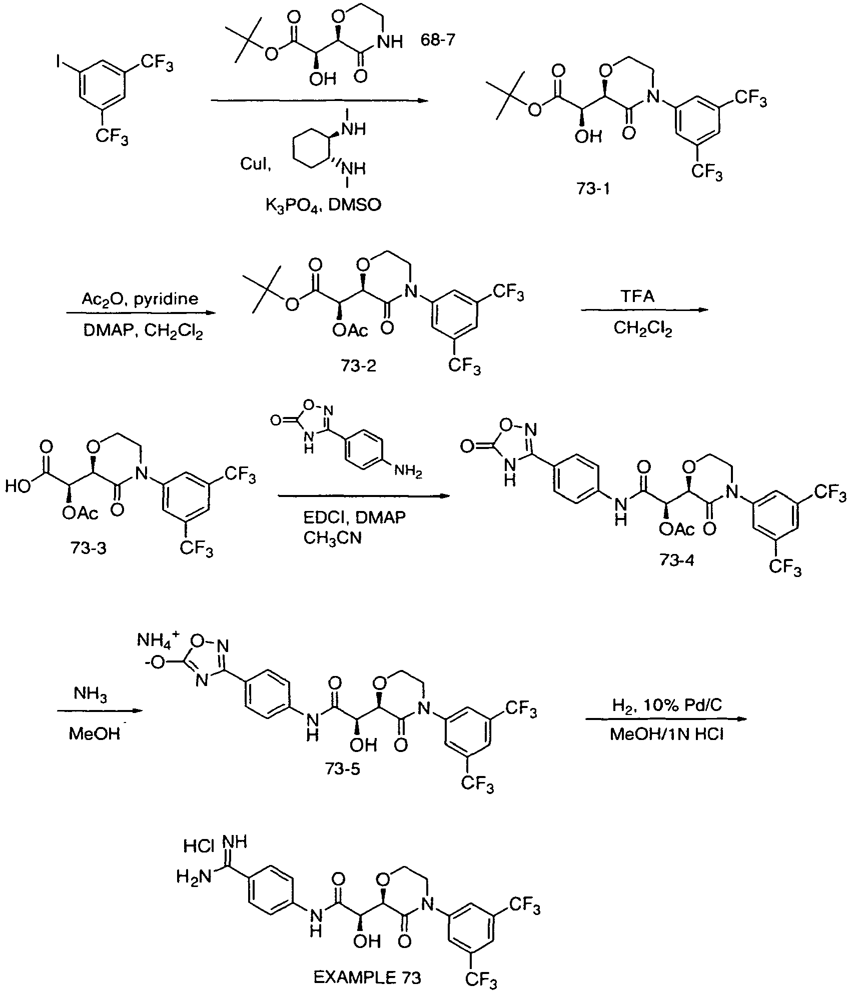

### [Step 73-1] Synthesis of (R)-tert-butyl 2-((R)-4-(3,5-bis(trifluoromethyl)phenyl)-3-oxomorpholin-2-yl)-2-hydroxyacetate (compound 73-1)

According to the Step 70-2 in the synthetic method for Example 70, 3,5-(bistrifluoromethyl)iodobenzene (0.1 mL) was used instead of compound 70-1 to obtain compound 73-1 (0.13 g) as a white solid after reverse-phase (C 18) purification.

### [Step 73-2] Synthesis of (R)-tert-butyl 2-acetoxy-2-((R)-4-(3,5-bis(trifiuoromethyl)phenyl)-3-oxomorpholin-2-yl)acetate (compound 73-2)

According to the Step 70-3 in the synthetic method for EXAMPLE 70, 73-1 (0.13 g) was used instead of compound 70-2 to obtain compound 73-2 (0.14 g) as a yellow semisolid that was used without further purification.

### [Step 73-3] Synthesis of (R)-2-acetoxy-2-((R)-4-(3,5-bis(trifluoromethyl)phenyl)-3-oxomorpholin-2-yl)acetic acid (compound 73-3)

According to the Step 70-4 in the synthetic method for EXAMPLE 70, compound 73-2 (0.14 g) was used instead of compound 70-3 to afford compound 73-3 (0.12 g) as a light yellow solid that was used without further purification.

### [Step 73-4] Synthesis of (R)-1-((R)-4-(3,5-bis(trifluoromethyl)phenyl)-3-oxomorpholin-2-yl)-2-oxo-2-(4-(5-oxo-4,5-dihydro-1,2,4-oxadiazol-3-yl)phenylamino) ethyl acetate (compound 73-4)

According to the Step 70-5 in the synthetic method for EXAMPLE 70, compound 73-3 (65 mg) was used instead of compound 70-4 to obtain compound 73-4 (69 mg) as a white solid after reverse-phase (C18) HPLC purification.

### [Step 73-5] Synthesis of ammonium 3-(4-((R)-2-((R)-4-(3,5-bis(trifluoromethyl)-phenyl)-3-oxomorpholin-2-y)-2-hydroxyacetamido)phenyl)-1,2,4-oxadiazol-5-olate (compound 73-5)

According to the Step 70-6 in the synthetic method for EXAMPLE 70, compound 73-4 (69 mg) was used instead of compound compound 70-5 to obtain compound 73-5 (58 mg) as a white solid that was used without further purification.

### [Step 73-6] Synthesis of (R)-2-((R)-4-(3,5-bis(trifluoromethyl)phenyl)-3-oxomorpholin-2-yl)-N-(4-carbamimidoylphenyl)-2-hydroxyacetamide hydrochloride (EXAMPLE 73)

According to the Step 70-7 in the synthetic method for EXAMPLE 70, compound 73-5 (53 mg) was used instead of compound 70-6 to obtain EXAMPLE 73 (37 mg) as a maize solid.

### (EXAMPLE 98)

### Synthesis of N-(4-Amino-7-quinazolinyl)-alpha(R)-hydroxy-3-oxo-4-[4-(trifluoromethyl)phenyl]-2(R)-morpholineacetamide (Example 98)

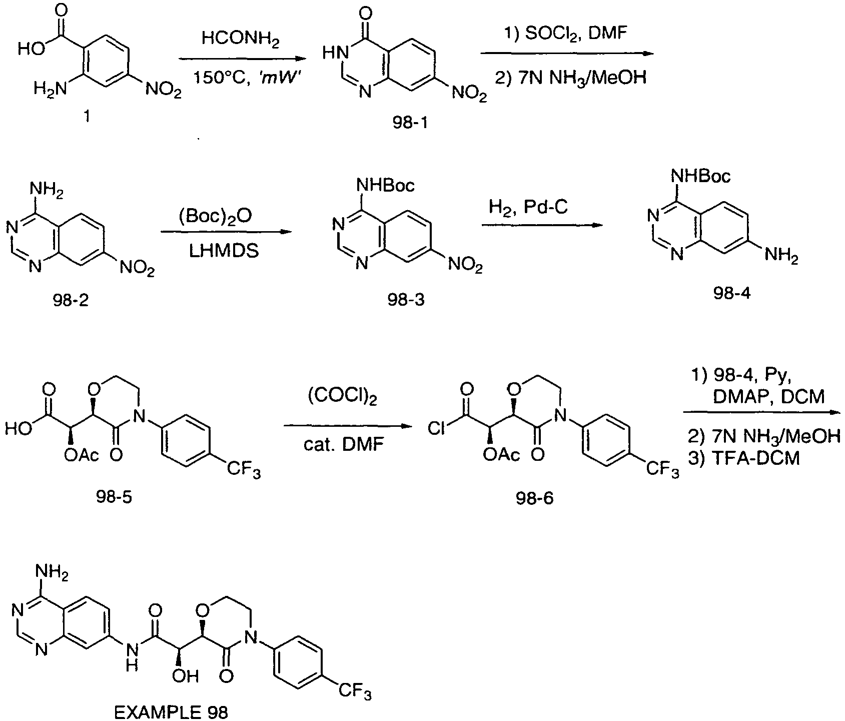

### [Step 98-1] Synthesis of 7-Nitroquinazolin-4(3H)-one (compound 98-1)

A mixture of 2-amino-4-nitrobenzoic acid (5.0g, 27.5 mmol) and formamide (8.0 ml, 201.5 mmol) in a microwave reaction vessel was heated in a microwave reactor at 150 °C for 1 hr. The slurry was cooled to rt, stirred with aq. NaHCO₃, filtered, washed with water followed by ether and dried in vacuum oven to provide 3.7 g of 7-nitroquinazolin-4(3H)-one (compound 98-1).

### [Step 98-2] Synthesis of 7-Nitroquinazolin-4-amine (compound 98-2)

To a solution of 7-nitroquinazolin-4(3H)-one (2.0 g, 10.5 mmol) in 40 ml thionyl chloride was added 0.8 ml of DMF and the mixture was heated at reflux overnight then evaporated to dryness to provide crude 4-chloro-7-nitroquinazoline.

A mixture of 1.6 g of 4-chloro-7-nitroquinazoline in 50 ml 7N ammonia in methanol was stirred overnight at rt and concentrated to dryness. The solid was suspended in water, filtered, rinsed with water followed by ether and dried overnight in vacuum oven to provide 0.98 g of 7-nitroquinzolin-4-amine (compound 98-2).

### [Step 98-3] Synthesis of tert-Butyl 7-nitroquinazolin-4-ylcarbamate (compound 98-3)

To a suspension of 7-nitroquinazolin-4-amine (0.98 g, 5.2 mmol) in 10 ml THF at rt was added a 1M solution of di-tert-butyldicarbonate in THF (10.3 ml, 10.3 mmol, 2 eq.) followed by a 1M solution of LHMDS in THF (8.8 mmol, 1.7 eq.). The resultant clear solution was stirred for 10 min. quenched with aq. NH₄Cl, extracted 3x with ethyl acetate, the combined organic layers washed with brine, dried over MgSO₄, filtered, concentrated and purified by chromatography eluting with 30% ethyl acetate in hexanes to provide 713 mg of tert-butyl 7-nitroquinazolin-4-ylcarbamate (compound 98-3).

### [Step 98-4] Synthesis of tert-butyl 7-aminoquinazolin-4-ylcarbamate (compound 98-4)

A mixture of 600 mg of *tert*-butyl 7-nitroquinazolin-4-ylcarbamate and 150 mg of 10% Pd-C in 15 ml each of THF and MeOH was stirred overnight under a hydrogen balloon, filtered through a Celite^{®} pad, concentrated and purified by chromatography eluting with 5% methanol in dichloromethane to provide 385 mg of *tert*-butyl 7-aminoquinazolin-4-ylcarbamate (compound 98-4). MS m/e = 261.1 (MH⁺)

### [Step 98-5]

### Synthesis of compound 98-5.

Compound 98-5 was prepared using a procedure similar to the preparation of compound 93-3.

### [Step 98-6] Synthesis of (R)-2-chloro-2-oxo-1-((R)-3-oxo-4-(4-(trifluoromethyl)-phenyl)morpholin-2-yl)ethyl acetate (compound 98-6)

To a solution of compound 98-5 (0.30 mmol) in 4 ml of dichloromethane was added oxalylchloride (75 µl, 0.886 mmol, 3 eq.) followed by 1 drop of DMF. Stirred at rt for 1 hr, added toluene and evaporated to dryness to provide crude compound 98-6 which was used as such for the next step.

### [Step 98-7] Synthesis of N-(4-Amino-7-quinazolinyl)-alpha(R)-hydroxy-3-oxo-4-[4-(trifluoromethyl)phenyl]-2(R)-morpholineacetamide (EXAMPLE 98)

To a solution of compound 98-6 (∼0.15 mmol) in 2 ml dichloromethane at 0 °C was added *tert*-butyl 7-aminoquinazolin-4-ylcarbamate (compound 98-4) (77 mg, 0.30 mmol, 2 eq.) followed by pyridine (24 µl, 0.30 mmol, 2 eq.) and DMAP (2 mg, 0.016 mmol, 0.1 eq.). To the mixture was added 1 ml of acetonitrile and stirred overnight while warming to rt. It was diluted with ethyl acetate, washed with aq. NaHCO₃, water and brine, dried over MgSO₄, filtered, concentrated to dryness. The residue was stirred overnight with 5 ml of 7N ammonia in methanol. The methanol was evaporated and the residue was stirred with 2 ml each of dichloromethane and trifluoroacetic acid for about 75 min. The mixture was evaporated to dryness and the residue was purified by RPHPLC to provide 14 mg of EXAMPLE 98.

### EXAMPLES

The present invention will now be described in more detail using examples, but the present invention is not limited to the examples.

The measurement of nuclear magnetic resonance (NMR) spectrum (Table 1) was performed using a JEOL JNM-ECX300 FT-NMR (manufactured by JEOL Ltd.) or a JEOL JNM-ECX400 FT-NMR (manufactured by JEOL Ltd.).

Liquid chromatography-mass spectrometry (LC-MS, Table 2) was performed using a Waters FractionLynx MS system (manufactured by Waters Corporation) from the Example 1 to Example 38. A SunFire column™ (4.6 mm × 5 cm, 5 µm) (manufactured by Waters Corporation) was used as an analytical column. A SunFire column™ (19 mm × 5 cm, 5 µm) (manufactured by Waters Corporation) was used as a preparative column. Methanol and 0.05% aqueous acetic acid solution or 0.05% aqueous trifluoroacetic acid solution were used as the mobile phase. Acetonitrile and 0.05% aqueous acetic acid solution or 0.05% aqueous trifluoroacetic acid solution were also used as the mobile phase. The analysis was performed under the following gradient conditions: Methanol: 0.05% aqueous acetic acid solution or 0.05% aqueous trifluoroacetic acid solution = 1:9 (0 minutes), 10:0 (5 minutes), and 10:0 (7 minutes). Acetonitrile: 0.05% aqueous acetic acid solution or 0.05% aqueous trifluoroacetic acid solution = 1:9 (0 minutes), 9:1 (5 minutes), and 9:1 (7 minutes). The solvent systems are described as the followings: A indicates MeOH-AcOH, B indicates MeOH-TFA, C indicates MeCN-AcOH, and D indicates MeCN-TFA.

### Examples aa1 - aa38

### EXAMPLE aa1

### Synthesis of (2R)-2-[(2R)-4-(4-tert-butylphenyl)-3-oxomorpholin-2-yl]-2-hydroxy-N-[4-(N-methylcarbamimidoyl)phenyl]acetamide hydrochloride (EXAMPLE aa1)

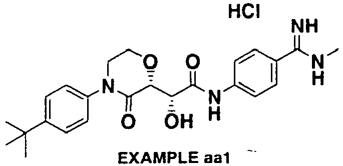

### [Step 1-1] Synthesis of 4-tert-butyl-N-(2-chloroethyl)aniline (compound aa1-1)

To a solution of 4-tert-butylaniline (5.00 g) in MeOH (100 mL) was added 40% chroloacetaldehyde solution in water (8.24 mL) and AcOH (3.84 mL) at 0 °C. The mixture was stirred for 15 minutes at the same temperature, sodium acetoxyborohydride (NaBH(OAc)₃ ; 14.2 g) was added into the reaction mixture at one portion and the mixture was stirred for 15 minutes.

The reaction mixture was diluted with water and was extracted with EtOAc. The extract was washed with water, sat.NaHCO₃ and brine. The organic layer was dried with anhyd. Na₂SO₄. It was filtrated to remove insoluble matters and it was concentrated in vacuo. The residue was purified by silica gel flush chromatography (NH-type, eluent: Hexane) to obtain aa1 - 1 (4.8 g) as pale yellow oil.

### [Step 1-2] Synthesis of (2R,3R)-2,3-diacetyloxy-4-(4-cyanoanilino)-4-oxobutanoic acid (compound aa1-2)

To a solution of (+)-Diacetyl-L-tartaric anhydride (9.15 g) in dry DMF (100 mL), was added 4-aminobenzonitrile (5 g,) under ice cooling and the reaction mixture was stirred at room temperature overnight to obtain compound aa1-2. The solution of compound aa1-2 was used in the next step without any treatment.

### [Step 1-3] Synthesis of [(2R,3R)-3-acetyloxy-1-[4-tert-butyl-N-(2-chloroethyl)anilino]-4-(4-cyanoanilino)-1,4-dioxobutan-2-yl] acetate (compound aa1-3)

The above DMF solution of aa1-2 (25.4 mL) was diluted with CH₂Cl₂ (25.4 mL). The internal temperature of the mixture was kept below -70 °C over all additions with dry ice bath.

Oxalyl chloride (1.0 mL) in CH₂Cl₂ (3 mL) was added dropwise into the reaction mixture. After stirring for 1 hour, pyridine (3.67 mL) was added dropwise thereto and stirred for 15 min. Then compound aa1-1 (2.28 g) in CH₂Cl₂ (13.7 mL) was added dropwise into the reaction mixture. The mixture was stirred below -70 °C for 12 hours.

The reaction mixture was quenched with water and was extracted with EtOAc. The extract was washed with water, 1N HCl, sat.NaHCO₃ and brine. The organic layer was dried with anhyd. Na₂SO₄. It was filtrated and was concentrated in vacuo. The residue was purified by silica gel flush chromatography (eluent : Hexane : EtOAc = 90 : 10 - 25 : 75) to obtain 1-3 (1.19 g) as a white amorphous solid.

### [Step 1-4] Synthesis of (2R,3R)-N-(4-tert-butylphenyl)-N-(2-chloroethyl)-N'-(4-cyanophenyl)-2,3-dihydroxybutanediamide (compound aa1-4)

To a solution of aa1-3 (0.3 g) in MeOH (6 mL), was added 8N NH₃ / MeOH (0.36 mL) at 0 °C and the mixture was stirred for 10 minutes in the same temperature. The mixture was concentrated and was dried in vacuo to obtain crude aa 1-4. The crude aa 1-4 was used in the next step without further purification.

### [Step 1-5] Synthesis of (2R)-2-[(2R)-4-(4-tert-butylphenyl)-3-oxomorpholin-2-yl]-N-(4-cyanophenyl)-2-hydroxyacetamide (compound aa1-5)

The crude aa1-4 was dissolved in t-BuOH (4.5 mL)-DMSO (3 mL), and t-BuOK (191 mg) was added portionwise into the reaction mixture at 0 °C. The mixture was stirred for 5 minutes in the same temperature.
To the reaction mixture was added 1N HCl and Et₂O to obtain precipitate. Then the precipitate was collected by filtration, was rinsed with water, was washed with Et₂O and was dried in vacuo to obtain aa1-5 (115 mg) as a white amorphous solid.

### [Step 1-6] Synthesis of (2R)-2-[(2R)-4-(4-tert-butylphenyl)-3-oxomorpholin-2-yl]-2-hydroxy-N-[4-(N-methylcarbamimidoyl)phenyl]acetamide hydrochloride (EXAMPLE aa1)

A mixture of compound aa1-5 (0.15 g) in CH₂Cl₂-MeOH (10-50 mL) was bubbled with HCl gas at 0 °C for 1h. Then the mixture was left at room temperature overnight. The mixture was concentrated in vauo. Then the resulting mixture was solved in MeOH (10 mL) and 2M MeNH₂ in THF (0.74 mL) were added into the mixture at 0 °C. The solution was stirred at room temperature overnight. The solvent was removed and the resulting residue was purified by preparative LC/MS to obtain EXAMPLE aa1 (15 mg) as a beige amorphous solid.

### EXAMPLE aa2

### Synthesis of (2R)-2-hydroxy-N-(1-imino-2,3-dihydroisoindol-5-yl)-2-[(2R)-3-oxo-4-(1-oxo-2,3-dihydroisoindol-5-yl)morpholin-2-yl]acetamide hydrochloride (EXAMPLE aa2)

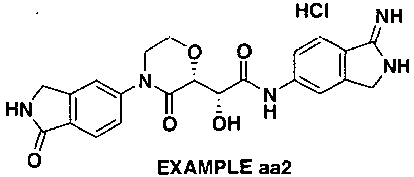

### [Step 2-1] Synthesis of 2-[tert-butyl(diphenyl)silyl]-5-iodo-3H-isoindol-1-one (compound aa2-1)

To a suspension of 5-iodo-2,3-dihydroisoindol-1-one (3 g) in DMF (60 mL), were added N,N-dimthylaminopyridine (DMAP: 14 mg), Et₃N (4.84 mL) and tert-butyl-diphenylsilyl chloride (9.0 mL). The mixture was stirred at 50 °C for 2h. After cooling, saturated NaHCO₃ aq. was added into the reaction mixture. Then it was extracted with EtOAc. The organic layer was washed with water and brine, then was dried over anhydrous Na₂SO₄. The solvent was removed under reduced pressure. The tritulation with Et₂O followed by filtration gave compound aa2-1 (2.78 g) as a yellow amorphous solid.

### [Step 2-2] Synthesis of tert-butyl (2R)-2-[(2R)-4-[2-[tert-butyl(diphenyl)silyl]-1-oxo-3H-isoindol-5-yl]-3-oxomorpholin-2-yl]-2-hydroxyacetate (compound aa2-2)

The mixture of (R)-tert-butyl (2-hydroxy-2-(R)-3-oxomorpholin-2-yl)acetate (1.2 g) and compound 2-1 (2.71 g) were solved in degassed DMSO (17 mL). Then crushed K₃PO₄ (2.2 g) and CuI (0.2 g) were added into the mixture. Then trans-N,N-dimethyl cyclohexanediamine (0.49 mL) was immediately added into the mixture. The mixture was stirred at room temperature for 3 h, then CuI (0.1 g) and trans-N,N-dimethyl cyclohexanediamine (0.29 mL) were added to complete the reaction. The reaction mixture was stirred at room temperature for more 2 h. Then, 1N HCl was added to the mixture and it was extracted with EtOAc. The organic solvent was washed with water and brine. It was dried over anhydrous Na₂SO₄ and the solvent was removed under reduced pressure. The resulting residue was purified by silica gel flash column chromatography (Hexane/EtOAc =100/0 - 50/50) to obtain compound aa2-2 (1.40 g) as a pale yellow amorphous solid.

### [Step 2-3] Synthesis of tert-butyl (2R)-2-acetyloxy-2-[(2R)-4-[2-tert-butyl(diphenyl)silyl]-1-oxo-3H-isoindol-5-yl]-3-oxomorpholin-2-yl]acetate (compound aa2-3)

To the solution of compound aa2-2 (0.1 g) and DMAP (2.0 mg) in CH₂Cl₂ (1.7 mL), were added pyridine (27 uL) and Ac₂O (31 uL) at 0°C. The mixture was stirred at 0 °C for 75 min. Then the mixture was diluted with EtOAc, the organic layer was washed with 5% CuSO₄ aq., water and brine. It was dried over anhydrous Na₂SO₄. The solvent was removed under reduced pressure to obtain compound aa2-3 (101 mg) as a white amorophous solid. It was used to the next step without further purification.

### [Step 2-4] Synthesis of (2R)-2-acetyloxy-2-[(2R)-3-oxo-4-(1-oxo-2,3-dihydroisoindol-5-yl)morpholin-2-yl]acetic acid (compound aa2-4)

To a solution of compound aa2-3 (91 mg) in CH₂Cl₂ (0.6 mL), was added trifluoroacetic acid (TFA: 2.4 mL) at 0 °C. The reaction mixture was stirred at room temperature for 2 h. The solvent was removed in vacuo. The resulting residue was co-evaporated with CH₂Cl₂, several times. Then trituration with Et₂O gave compound aa2-4 (46 mg) as a white amorphous solid.

### [Step 2-5] Synthesis of [(1R)-2-[3-[[bis[(2-methylpropan-2-yl)oxycarbonyl]amino]-methyl]-4-cyanoanilino]-2-oxo-1-[(2R)-3-oxo-4-(1-oxo-2,3-dihydroisoindol-5-yl)morp holin-2-yl]ethyl]acetate (compound aa2-5)

To the suspension of compound aa2-4 (40 mg), N-[(5-amino-2-cyanophenyl)methyl]-N-[(2-methylpropan-2-yl)oxycarbonyl]carbamate (39.9 mg) and DMAP (1.4 mg) in CH₂Cl₂ (1.1 mL), were added WSC-HCl (28.6 mg) at 0°C. The reaction mixture was stirred at 0 °C for 4 h. The mixture was diluted with EtOAc and the organic layer was washed with 1N HCl and brine. It was dried over anhydrous Na₂SO₄ and the solvent was removed under reduced pressure. The resulting residue was purified by silica gel flash column chromatography (EtOAc /MeOH= 100/0 - 90/10) to obtain compound aa2-5 (26 mg) as a colorless amorphous solid.

### [Step 2-6] Synthesis of tert-butyl N-[[2-cyano-5-[[(2R)-2-hydroxy-2-[(2R)-3-oxo-4-(1-oxo-2,3-dihydroisoindol-5-yl)morpholin-2-yl]acetyl]amino]phenyl]methyl]-N-[(2-methylpropan-2-yl)oxycarbonyl]carbamate (compound aa2-6)

A solution of compound aa2-5 (25.9 mg) in 8N NH₃-MeOH (1 mL) was stirred at room temperature for 2 h. Then the solvent was removed in vacuo. Acetonitrile was added to the resulting residue and the mixture was concentrated in vacuo to obtain compound aa2-6 (23 mg) as a white amorphous solid.

### [Step 2-7] Synthesis of (2R)-N-[3-(aminomethyl)-4-cyanophenyl]-2-hydroxy-2-[(2R)-3-oxo-4-(1-oxo-2,3-dihydroisoindol-5-yl)morpholin-2-yl]acetamide hydrochloride (compound aa2-7)

Compound aa2-6 (23 mg) was suspended in 4N HCl-dioxane (2 mL). The reaction mixture was stirred at room temperature for 2 h and evaporated. The residue was co-evaporated with toluene (2 times) to obtain compound aa2-7 (17 mg) as a colorless amorphous solid.

### [Step 2-8] Synthesis of (2R)-2-hydroxy-N-(1-imino-2,3-dihydroisoindol-5-yl)-2-[(2R)-3-oxo-4-(1-oxo-2,3-dihydroisoindol-5-yl)morpholin-2-yl]acetamide hydrochloride (EXAMPLE aa2)

A solution of compound aa2-7 (15 mg) in EtOH (3 mL) was refluxed for 7 h 30 m. After cooling, the mixture was concentrated in vacuo and trituration with Et₂O followed by filtration gave EXAMPLE aa2 (14.8 mg) as a colorless amorphous solid.

### EXAMPLE aa3

### Synthesis of (2R)-2-hydroxy-N-(1-imino-2,3-dihydroisoindol-5-yl)-2-[(2R)-3-oxo-4-(2-oxo-1H-quinolin-6-yl)morpholin-2-yl]acetamide hydrochloride (EXAMPLE aa3)

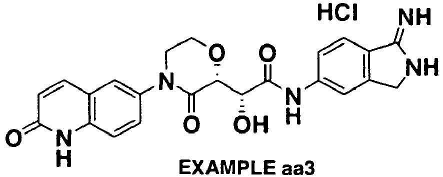

### [Step 3-1] Synthesis of tert-butyl (2R)-2-hydroxy-2-[(2R)-3-oxo-4-(2-oxo-1H-quinolin-6-yl)morpholin-2-yl]acetate (compound aa3-1)

According to the Step 2-2 in synthetic method for EXAMPLE aa2, 6-iodo-1H-quinolin-2-one (2.00 g) was used instead of aa2-1 to obtain compound aa3-1 (1.13 g) as a colorless amorphous solid.

### [Step 3-2] Synthesis of tert-butyl (2R)-2-acetyloxy-2-[(2R)-3-oxo-4-(2-oxo-1H-quinolin-6-yl)morpholin-2-yl]acetate (compound aa3-2)

According to the Step 2-3 in synthetic method for EXAMPLE aa2, compound aa3-1 (1.00 g) was used instead of aa2-2 to obtain compound aa3-2 (0.85 g) as a colorless amorphous solid.

### [Step 3-3] Synthesis of (2R)-2-acetyloxy-2-[(2R)-3-oxo-4-(2-oxo-1H-quinolin-6-yl)morpholin-2-yl]acetic acid (compound aa3-3)

According to the Step 2-4 in synthetic method for EXAMPLE aa2, compound aa3-2 (0.80 g) was used instead of aa2-3 to obtain compound aa3-3 (0.56 g) as a pale brown amorphous solid.

### [Step 3-4] Synthesis of [(1R)-2-[3-[[bis[(2-methylpropan-2-yl)oxycarbonyl]-amino]methyl]-4-cyanoanilino]-2-oxo-1-[(1R)-3-oxo-4-(2-oxo-1H-quinolin-6-yl)morp holin-2-yl]ethyl] acetate (compound aa3-4)

According to the Step 2-5 in synthetic method for EXAMPLE aa2, compound aa3-3 (0.10 g) was used instead of aa2-4 to obtain compound aa3-4 (47 mg) as a colorless amorphous solid.

### [Step 3-5] Synthesis of tert-butyl N-[[2-cyano-5-[[(2R)-2-hydroxy-2-[(2R)-3-oxo-4-(2-oxo-1H-quinolin-6-yl)morpholin-2-yl]acetyl]amino]phenyl]methyl]-N-[(2-me thylpropan-2-yl)oxycarbonyl]carbamate (compound aa3-5)

According to the Step 2-6 in synthetic method for EXAMPLE aa2, compound aa3-4 (45 mg) was used instead of aa2-5 to obtain compound aa3-5 (35 mg) as a colorless amorphous solid.

### [Step 3-6] Synthesis of methyl (2R)-2-hydroxy-2-[(2R)-4-(4-iodophenyl)-3-oxomorpholin-2-yl]acetate hydrochloride (compound aa3-6)

According to the Step 2-7 in synthetic method for EXAMPLE aa2, compound aa3-5 (35 mg) was used instead of aa2-6 to obtain compound aa3-6 (25 mg) as a colorless amorphous solid.

### [Step 3-7] Synthesis of (2R)-2-hydroxy-N-(1-imino-2,3-dihydroisoindol-5-yl)-2-[(2R)-3-oxo-4-(2-oxo-1H-quinolin-6-yl)morpholin-2-yl]acetamide hydrochloride (EXAMPLE aa3)

According to the Step 2-8 in synthetic method for EXAMPLE aa2, compound aa3-6 (25 mg) was used instead of aa2-7 to obtain EXAMPLE aa3 (20 mg) as a pale brown amorphous solid.

### EXAMPLE aa4

### Synthesis of (2R)-2-[(2R)-4-[7-(difluoromethoxy)-2-oxo-1H-quinolin-6-yl]-3-oxomorpholin-2-yl]-2-hydroxy-N-(1-imino-2,3-dihydroisoindol-5-yl)acetamide hydrochloride (EXAMPLE aa4)

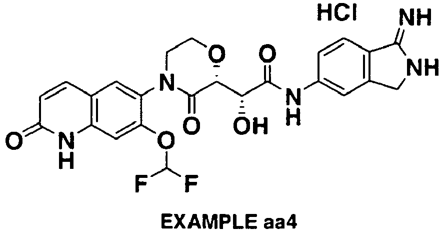

### [Step 4-1] Synthesis of 2-(difluoromethoxy)-1-iodo-4-nitrobenzene (compound aa4-1)

A mixture of 2-iodo-5-nitrophenol (8.98 g), K₂CO₃ (18.7 g) and 2-chloro-2,2-difluoro-acetic acid sodium salt (5.17 g) in DMF-H₂O (60-6 mL) was stirred at 110°C for 3.5 h and at room temperature for 12 h. The mixture was diluted with EtOAc and washed with I H HCl, saturated NaHCO₃ aq., water and brine, and dried over anhydrous Na₂SO₄. The solvent was removed under reduced pressure. The resulting residue was purified by silica gel column chromatography (Hexane/EtOAc = 9/1) to obtain compound aa4-1 (9.17 g) as a brown amorphous solid.

### [Step 4-2] Synthesis of 3-(difluoromethoxy)-4-iodoaniline (compound aa4-2)

A mixture of compound aa4-1 (8 g) and Na₂S₂O₄ in THF-EtOH-H₂O (150-75-225 mL) was stirred at 0 °C for I h and at room temperature for 6 h.The mixture was extracted with EtOAc and the organic layer was washed with H₂O, brine. It was dried over anhydrous Na₂SO₄. The solvent was removed under reduced pressure to obtain compound aa4-2 (2.36 g) as brown oil. It was used to the next step without further purification.

### [Step 4-3] Synthesis of (E)-N-[3-(difluoromethoxy)-4-iodophenyl]-3-ethoxyprop-2-enamide (compound aa4-3)

To a solution of compound aa4-2 (2.35 g) and pyridine (1.33 mL) in CH₂Cl₂ (60 mL), was added dropwise 3-ethoxy-2-propenoyl chloride (1.33 g) at 0°C. The reaction mixture was stirred at 0°C for 5 min and at room temperature for 20 min. The mixture was concentrated in vacuo, and the resulting residue was solved in EtOAt and the organic layer was washed with 1N HCl, saturated NaHCO₃ aq., water and brine, and dried over anhydrous Na₂SO₄. The solvent was removed under reduced pressure to obtain compound aa4-3 (3.2 g) as brown oil. It was used to the next step without further purification.

### [Step 4-4] Synthesis of 7-(difluoromethoxy)-6-iodo-1H-quinolin-2-one (compound aa4-4)

To compound aa4-3 (3.15 g), conc. H₂SO₄ was added dropwise at 0 °C. The mixture was stirred at 0 °C for 10 min then it was poured into crashed ice. The mixture was diluted with water and it was extracted with EtOAc. The organic layer was washed with saturated NaHCO₃ aq., water and brine, and dried over anhydrous Na₂SO₄. The solvent was removed under reduced pressure to obtain compound aa4-4 (2.06 g) as a pale brown amorphous solid. It was used to the next step without further purification.

### [Step 4-5] Synthesis of tert-butyl (2R)-2-[(2R)-4-[7-(difluoromethoxy)-2-oxo-1H-quinolin-6-yl]-3-oxomorpholin-2-yl]-2-hydroxyacetate (compound aa4-5)

According to the Step 2-2 in synthetic method for EXAMPLE aa2, compound aa4-4 (1.91 g) was used instead of aa2-1 to obtain compound aa4-5 (0.6 g) as a brown amorphous solid.

### [Step 4-6] Synthesis of tert-butyl (2R)-2-acetyloxy-2-[(2R)-4-[7-(difluoromethoxy)-2-oxo- I H-quinolin-6-yl]-3-oxomorpholin-2-yl]acetate (compound aa4-6)

According to the Step 2-3 in synthetic method for EXAMPLE aa2, compound aa4-5 (0.59 g) was used instead of aa2-2 to obtain compound aa4-6 (0.58 g) as a brown amorphous solid.

### [Step 4-7] Synthesis of (2R)-2-acetyloxy-2-[(2R)-4-[7-(difluoromethoxy)-2-oxo-1H-quinolin-6-yl]-3-oxomorpholin-2-yl]acetic acid (compound aa4-7)

According to the Step 2-4 in synthetic method for EXAMPLE aa2, compound aa4-6 (0.57 g) was used instead of aa2-3 to obtain compound aa4-7 (0.53 g) as a brown amorphous solid.

### [Step 4-8] Synthesis of [(1R)-2-[3-[[bis[(2-methylpropan-2-yl)oxycarbonyl]amino]-methyl]-4-cyanoanilino[-1-[(2R)-4-[7-(difluoromethoxy)-2-oxo-1H-quinolin-6-yl]-3-ox omorpholin-2-yl]-2-oxoethyl]acetate (compound aa4-8)

According to the Step 2-5 in synthetic method for EXAMPLE aa2, compound aa4-7 (0.35 g) was used instead of aa2-4 to obtain compound aa4-8 (97 mg) as a pale yellow amorphous solid.

### [Step 4-9] Synthesis of tert-butyl N-[[2-cyano-5-[[(2R)-2-[(2R)-4-[7-(difluoromethoxy)-2-oxo-1H-quinolin-6-yl]-3-oxomorpholin-2-yl]-2-hydroxyacetyl]am ino]phenyl]methyl]-N-[(2-methylpropan-2-yl)oxycarbonyl]carbamate (compound aa4-9)

According to the Step 2-6 in synthetic method for EXAMPLE aa2, compound aa4-8 (96 mg) was used instead of aa2-5 to obtain compound aa4-9 (88 mg) as a white amorphous solid.

### [Step 4-10] Synthesis of (2R)-N-13-(aminomethyl)-4-cyanophenyl]-2-[(2R)-4-[7-(difluoromethoxy)-2-oxo-1H-quinolin-6-yl]-3-oxomorpholin-2-yl]-2-hydroxyacetamid e hydrochloride (compound aa4-10)

According to the Step 2-7 in synthetic method for EXAMPLE aa2, compound aa4-9 (82 mg) was used instead of aa2-6 to obtain compound aa4-10 (59 mg) as a white amorphous solid.

### [Step 4-11] Synthesis of (2R)-2-[(2R)-4-[7-(difluoromethoxy)-2-oxo-1H-quinolin-6-yl]-3-oxomorpholin-2-yl]-2-hydroxy-N-(1-imino-2,3-dihydroisoindol-5-yl)acetamide hydrochloride (EXAMPLE aa4)

According to the Step 2-8 in synthetic method for EXAMPLE aa2, compound aa4-10 (57 mg) was used instead of aa2-7 to obtain EXAMPLE aa4 (39 mg) as a white amorphous solid.

### EXAMPLE aa5

### Synthesis of (2R)-N-(4-carbamimidoylphenyl)-2-hydroxy-2-[(2R)-3-oxo-4-(2-oxo-1H-quinolin-6-yl)morpholin-2-yl]acetamide hydrochloride (EXAMPLE aa5)

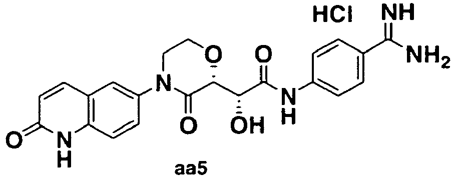

### [Step 5-1] Synthesis of [(1R)-2-oxo-2-[4-(5-oxo-2H-1,2,4-oxadiazol-3-yl)anilino]-1-[(2R)-3-oxo-4-(2-oxo-1H-quinolin-6-yl)morpholin-2-yl]ethyl] acetate (compound aa5-1)

To a solution of compound aa3-3 (100 mg) in DMF (2.5 mL), was added oxalyl chrolide (48 uL) at 0 °C. The reaction mixture was stirred at 0°C for 0.5 h, then CH₂Cl₂ (2.5 mL) and pyridine (95.5 uL) were added into the reaction mixture at 0°C. The mixture was stirred at 0 °C for 0.5h, then 3-(4-aminophenyl)-1,2,4-oxadiazol-5(2H)-one (60.2 mg) was added into the mixture at 0 °C. The mixture was stirred at room temperature for 3 days. Then water and 1N HCl were added into the reaction mixture and the mixture was extracted with EtOAc. The organic layer was washed with water and brine, and was dried over anhydrous Na₂SO₄. The solvent was removed under reduced pressure to obtain compound aa5-1 (77 mg) as a beige amorphous solid. It was used to the next step without further purification.

### [Step 5-2] Synthesis of (2R)-2-hydroxy-N-[4-(5-oxo-2H-1,2,4-oxadiazol-3-yl)phenyl]-2-[(2R)-3-oxo-4-(2-oxo-1H-quinolin-6-yl)morpholin-2-yl]acetamide ammonium salt (compound aa5-2)

According to the Step 2-6 in synthetic method for EXAMPLE aa2, compound aa5-1 (75 mg) was used instead of aa2-5 to obtain compound aa5-2 (44 mg) as a beige amorphous solid.

### [Step 5-3] Synthesis of (2R)-N-(4-carbamimidoylphenyl)-2-hydroxy-2-[(2R)-3-oxo-4-(2-oxo-1H-quinolin-6-yl)morpholin-2-yl]acetamide hydrochloride (EXAMPLE aa5)

To the solution of compound aa5-2 (40 mg) in 1N HCl-dioxane/MeOH - MeOH (= 0.34 - 40 mL), was added 10 % palladium charcoal (Pd/C: 40 mg). The reaction mixture was stirred under hydrogen gas atmosphere for 1 h. MeOH (20 mL) was added into the reaction mixture and the mixture was stirred for more 1.5 h to complete the reaction. Then Pd/C was removed by filtration with Celite^{®} pad and rinsed with MeOH. The filtrate was concentrated in vacuo and trituration with MeOH/Et₂O followed by filtration gave EXAMPLE aa5 (30 mg) as a beige amorphous solid.

### EXAMPLE aa6

### Synthesis of (2R)-N-(4-carbamimidoyl-3-fluorophenyl)-2-hydroxy-2-[(2R)-3-oxo-4-(2-oxo-1H-quinolin-6-yl)morpholin-2-yl]acetamide hydrochloride (EXAMPLE aa6)

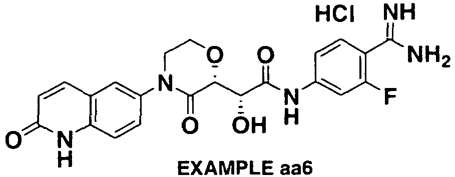

### [Step 6-1] Synthesis of [(1R)-2-[3-fluoro-4-(5-oxo-2H-1,2,4-oxadiazol-3-yl)anilino]-2-oxo-1-[(2R)-3-oxo-4-(2-oxo-1H-quinolin-6-yl)morpholin-2-yl]ethyl] acetate (compound aa6-1)

According to the Step 5-1 in synthetic method for EXAMPLE aa5. 3-fluoro-4-(5-oxo-2H-1,2,4-oxadiazol-3-yl)aniline (55 mg) was used instead of 4-(5-oxo-2H-1,2,4-oxadiazol-3-yl)aniline to obtain compound aa6-1 (31 mg) as a colorless amorphous solid.

### [Step 6-2] Synthesis of (2R)-N-[3-fluoro-4-(5-oxo-2H-1,2,4-oxadiazol-3-yl)phenyl]-2-hydroxy-2-[(2R)-3-oxo-4-(2-oxo-1H-quinolin-6-yl)morpholin-2-yl]acetamide ammonium salt (compound aa6-2)

According to the Step 2-6 in synthetic method for (EXAMPLE aa2, compound aa6-1 (30 mg) was used instead of aa2-5 to obtain compound aa6-2 (28 mg) as a beige amorphous solid.

### [Step 6-3] Synthesis of (2R)-N-(4-carbamimidoyl-3-fluorophenyl)-2-hydroxy-2-[(2R)-3-oxo-4-(2-oxo-1H-quinolin-6-yl)morpholin-2-yl]acetamide hydrochloride (EXAMPLE aa6)

According to the Step 5-3 in synthetic method for EXAMPLE aa5, compound aa6-2 (22 mg) was used instead of aa5-2 to obtain EXAMPLE aa6 (19 mg) as a beige amorphous solid.

### EXAMPLE aa7

### Synthesis of (2R)-2-hydroxy-N-(1-imino-2,3-dihydroisoindol-5-yl)-2-[(2R)-3-oxo-4-(2-oxo-1H-quinolin-7-yl)morpholin-2-yl]acetamide hydrochloride (EXAMPLE aa7)

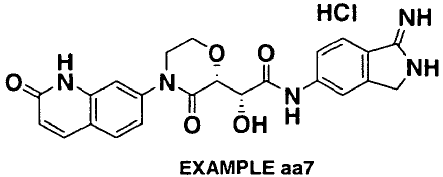

### [Step 7-1] Synthesis of 7-iodo-1H-quinolin-2-one (compound aa7-1)

According to the Step 4-4 in synthetic method for EXAMPLE aa4, (E)-N-(3-iodophenyl)-3-ethoxyprop-2-enamide (13.00 g) was used instead of aa4-3 to obtain compound aa7-1 (11.2 g) as a colorless powder.

### [Step 7-2] Synthesis of tert-butyl (2R)-2-hydroxy-2-[(2R)-3-oxo-4-(2-oxo-1H-quinolin-7-yl)morpholin-2-yl]acetate (compound aa7-2)

According to the Step 2-2 in synthetic method for EXAMPLE aa2, compound aa7-1 (4.00 g) was used instead of aa2-1 to obtain compound aa7-2 (1.76 g) as a yellow amorphous solid.

### [Step 7-3] Synthesis of tert-butyl (2R)-2-acetyloxy-2-[(2R)-3-oxo-4-(2-oxo-1H-quinolin-7-yl)morpholin-2-yl]acetate (compound aa7-3)

According to the Step 2-3 in synthetic method for EXAMPLE aa2, compound aa7-2 (0.83 g) was used instead of aa2-2 to obtain compound aa7-3 (0.64 g) as a yellow amorphous solid.

### [Step 7-4] Synthesis of (2R)-2-acetyloxy-2-[(2R)-3-oxo-4-(2-oxo-1H-quinolin-7-yl)morpholin-2-yl]acetic acid (compound aa7-4)

According to the Step 2-4 in synthetic method for EXAMPLE aa2, compound aa7-3 (0.63 g) was used instead of aa2-3 to obtain compound aa7-4 (0.50 g) as a pale yellow amorphous solid.

### [Step 7-5] Synthesis of N-[[2-cyano-5-[[(2R)-2-hydroxy-2-[(2R)-3-oxo-4-(2-oxo-1H-quinolin-7-yl)morpholin-2-yl]acetyl]amino]phenyl]methyl]-N-[(2-methylpr opan-2-yl)oxycarbonyl]carbamate (compound aa7-5)

According to the Step 2-5 and 2-6 in synthetic method for EXAMPLE aa2, compound aa7-4 (1.0 g) was used instead of aa2-4 to obtain compound aa7-5 (0.39 g) as a pale yellow amorphous solid.

### [Step 7-6] Synthesis of (2R)-N-[3-(aminomethyl)-4-cyanophenyl]-2-hydroxy-2-[(2R)-3-oxo-4-(2-oxo-1H-quinolin-7-yl)morpholin-2-yl]acetamide hydrochloride (compound aa7-6)

According to the Step 2-7 in synthetic method for EXAMPLE aa2. compound aa7-5 (0.38 g) was used instead of aa2-6 to obtain compound aa7-6 (0.27 g) as a yellow amorphous solid.

### [Step 7-7] Synthesis of (2R)-2-hydroxy-N-(1-imino-2,3-dihydroisoindol-5-yl)-2-[(2R)-3-oxo-4-(2-oxo-1H-quinolin-7-yl)morpholin-2-yl]acetamide hydrochloride (EXAMPLE aa7)

According to the Step 2-8 in synthetic method for EXAMPLE aa2, compound aa7-6 (0.27 g) was used instead of aa2-7 to obtain EXAMPLE aa7 (0.27 g) as a yellow amorphous solid.

### EXAMPLE aa8

### Synthesis of (2R)-2-hydroxy-N-(1-imino-2.3-dihydroisoindol-5-yl)-2-[(2R)-4-(1-methyl-2-oxoquinolin-7-yl)-3-oxomorpholin-2-yl]acetamide hydrochloride (EXAMPLE aa8)

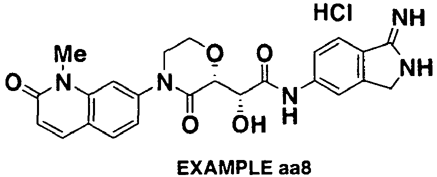

### [Step 8-1] Synthesis of 7-iodo-1-methylquinolin-2-one (compound aa8-1)

To a solution of compound aa7-1 (1 g) in DMF (18 mL), was added 60% NaH (0.22 g) at 0°C and the mixture was stirred at 0°C for 15 min. Then MeI (0.46 mL) was added into the reaction mixture at 0°C and the mixture was stirred at room temperature for 3 h. After the reaction, water and 1N HCl were added into the reaction mixture at 0 °C. The mixture was extracted with EtOAc and the organic layer was washed with saturated NaHCO₃ aq. and brine, and dried over anhydrous Na₂SO₄. The solvent was removed under reduced pressure to obtain the crude product. Trituration with hexane followed by filtration gave compound aa8-1 (0.73 g) as a yellow amorphous solid.

### [Step 8-2] Synthesis of tert-butyl (2R)-2-hydroxy-2-[(3R)-4-(1-methyl-2-oxoquinolin-7-yl)-3-oxomorpholin-2-yl]acetate (compound aa8-2)

According to the Step 2-2 in synthetic method for EXAMPLE aa2, compound aa8-1 (0.71 g) was used instead of aa2-1 to obtain compound aa8-2 (0.43 g) as a yellow amorphous solid.

### [Step 8-3] Synthesis of tert-butyl (2R)-2-acetyloxy-2-[(2R)-4-(1-methyl-2-oxoquinolin-7-yl)-3-oxomorpholin-2-yl]acetate (compound aa8-3)

According to the Step 2-3 in synthetic method for EXAMPLE aa2, compound aa8-2 (0.45 g) was used instead of aa2-2 to obtain compound aa8-3 (0.46 g) as a yellow amorphous solid.

### [Step 8-4] Synthesis of (2R)-2-acetyloxy-2-[(2R)-4-(1-methyl-2-oxoquinolin-7-yl)-3-oxomorpholin-2-yl]acetic acid (compound aa8-4)

According to the Step 2-4 in synthetic method for EXAMPLE aa2, compound aa8-3 (0.45 g) was used instead of aa2-3 to obtain compound aa8-4 (0.48 g) as a pale brown amorphous solid.

### [Step 8-5] Synthesis of [(1R)-2-[3-[[bis[(2-methylpropan-2-yl)oxycarbonyl] amino]methyl]-4-cyanoanilino]-1-[(2R)-4-(1-methyl-2-oxoquinolin-7-yl)-3-oxomorpho lin-2-yl]-2-oxoethyl] acetate (compound aa8-5)

According to the Step 2-5 in synthetic method for EXAMPLE aa2, compound aa8-4 (0.47 g) was used instead of aa2-4 to obtain compound aa8-5 (0.52 g) as a yellow amorphous.

### [Step 8-6] Synthesis of tert-butyl N-[[2-cyano-5-[[(2R)-2-hydroxy-2-[(2R)-4-(1-methyl-2-oxoquinolin-7-yl)-3-oxomorpholin-2-yl]acetyl]amino]phenyl]methyl]-N-[(2-methylpropan-2-yl)oxycarbonyl]carbamate (compound aa8-6)

According to the Step 2-6 in synthetic method for EXAMPLE aa2, compound aa8-5 (0.51 g) was used instead of aa2-5 to obtain compound aa8-6 (0.47 g) as a yellow amorphous solid.

### [Step 8-7] Synthesis of (2R)-N-[3-(aminomethyl)-4-cyanophenyl]-2-hydroxy-2-[(2R)-4-(1-methyl-2-oxoquinolin-7-yl)-3-oxomorpholin-2-yl]acetamide hydrochloride (compound aa8-7)

According to the Step 2-7 in synthetic method for EXAMPLE aa2, compound aa8-6 (0.46 g) was used instead of aa2-6 to obtain compound aa8-7 (0.35 g) as a pale yellow amorphous solid.

### [Step 8-8] Synthesis of (2R)-2-hydroxy-N-(1-imino-2,3-dihydroisoindol-5-yl)-2-[(2R)-4-(1-methyl-2-oxoquinolin-7-yl)-3-oxomorpholin-2-yl]acetamide hydrochloride (EXAMPLE aa8)

According to the Step 2-8 in synthetic method for EXAMPLE aa2, compound aa8-7 (0.34 g) was used instead of aa2-7 to obtain EXAMPLE aa8 (0.33 g) as a pale yellow amorphous solid.

### EXAMPLE aa9

### Synthesis of (2R)-2-[(2R)-4-[1-(cyclopropylmethyl)-2-oxoquinolin-7-yl]-3-oxomorpholin-2-yl]-2-hydroxy-N-(1-imino-2,3-dihydroisoindol-5-yl)acetamide hydrochloride (EXAMPLE aa9)

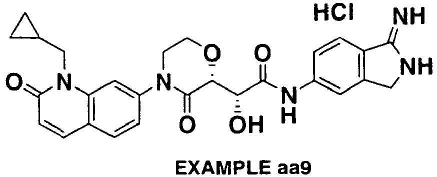

### [Step 9-1] Synthesis of 1-(cyclopropylmethyl)-7-iodoquinolin-2-one (compound aa9-1)

According to the Step 8-1 in synthetic method for EXAMPLE aa8, (bromomethyl)cycloprolane (1.00 g) was used instead of iodomethane to obtain compound aa9-1 (0.48 g) as a colorless amorphous solid.

### [Step 9-2] Synthesis of tert-butyl (2R)-2-[(2R)-4-[1-(cyclopropylmethyl)-1-oxoquinolin-7-yl]-3-oxomorpholin-2-yl]-2-hydroxyacetate (compound aa9-2)

According to the Step 2-2 in synthetic method for EXAMPLE aa2, compound aa9-1 (0.47 g) was used instead of aa2-1 to obtain compound aa9-2 (0.34 g) as a colorless amorphous solid.

### [Step 9-3] Synthesis of tert-butyl (2R)-2-acetyloxy-2-[(2R)-4-[1-(cyclopropylmethyl)-2-oxoquinolin-7-yl]-3-oxomorpholin-2-yl]acetate (compound aa9-3)

According to the Step 2-3 in synthetic method for EXAMPLE aa2, compound aa9-2 (0.34 g) was used instead of aa2-2 to obtain compound aa9-3 (0.37 g) as a colorless amorphous solid.

### [Step 9-4] Synthesis of (2R)-2-acetyloxy-2-[(2R)-4-[1-(cyclopropylmethyl)-2-oxoquinolin-7-yl]-3-oxomorpholin-2-yl]acetic acid (compound aa9-4)

According to the Step 2-4 in synthetic method for EXAMPLE aa2, compound aa9-3 (0.36 g) was used instead of aa2-3 to obtain compound aa9-4 (0.32 g) as a colorless amorphous solid.

### [Step 9-5] Synthesis of [(1R)-2-[3-[[bis[(2-methylpropan-2-yl)oxycarbony]amino]-methyl]-4-cyanoanilino]-1-[(2R)-4-[1-(cyclopropylmethyl)-2-oxoquinolin-7-yl]-3-oxo morpholin-2-yl]-2-oxoethyl] acetate (compound aa9-5)

According to the Step 2-5 in synthetic method for EXAMPLE aa2, compound aa9-4 (0.32 g) was used instead of aa2-4 to obtain compound aa9-5 (0.42 g) as a colorless amorphous solid.

### [Step 9-6] Synthesis of tert-butyl N-[[2-cyano-5-[[(2R)-2-[(2R)-4-[1-(cyclopropylmethyl)-2-oxoquinolin-7-yl]-3-oxomorpholin-2-yl]-2-hydroxyacetyl]amin o]phenyl]methyl]-N-[(2-methylpropan-2-yl)oxycarbonyl]carbamate (compound aa9-6)

According to the Step 2-6 in synthetic method for EXAMPLE aa2, compound aa9-5 (0.41 g) was used instead of aa2-5 to obtain compound aa9-6 (0.38 g) as a colorless amorphous solid.

### [Step 9-7] Synthesis of (2R)-N-[3-(aminomethyl)-4-cyanophenyl]-2-[(2R)-4-[1-(cyclopropylmethyl)-2-oxoquinolin-7-yl]-3-oxomorpholin-2-yl]-2-hydroxyacetamid e hydrochloride (compound aa9-7)

According to the Step 2-7 in synthetic method for EXAMPLE aa2, compound aa9-6 (0.39 g) was used instead of aa2-6 to obtain compound aa9-7 (0.30 g) as a colorless amorphous solid.

### [Step 9-8] Synthesis of (2R)-2-[(2R)-4-[1-(cyclopropylmethyl)-2-oxoquinolin-7-yl)-3-oxomorpholin-2-yl]-2-hydroxy-N-(1-imino-2,3-dihydroisoindol-5-yl)acetamide hydrochloride (EXAMPLE aa9)

According to the Step 2-8 in synthetic method for EXAMPLE aa2, compound aa9-7 (0.30 g) was used instead of aa2-7 to obtain EXAMPLE aa9 (0.24 g) as a yellow amorphous solid.

### EXAMPLE aa10

### Synthesis of methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetate hydrochloride (EXAMPLE aa10)

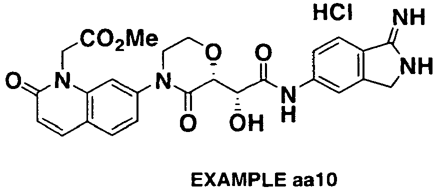

### [Step 10-1] Synthesis of methyl 2-(7-iodo-2-oxoquinolin-1-yl)acetate (compound aa10-1)

According to the Step 8-1 in synthetic method for EXAMPLE aa8, methyl bromoacetate (1.75 ml) was used instead of iodomethane to obtain compound aa 10-1 (1.76 g) as a pale yellow amorphous solid.

### [Step 10-2] Synthesis of tert-butyl (2R)-2-hydroxy-2-[(2R)-4-[1-(2-methoxy-2-oxoethyl)-2-oxoquinolin-7-yl]-3-oxomorpholin-2-yl]acetate (compound aa10-2)

According to the Step 2-2 in synthetic method for EXAMPLE aa2, compound aa10-1 (1.69 g) was used instead of aa2-1 to obtain compound aa10-2 (1.52 g) as a yellow amorphous solid.

### [Step 10-3] Synthesis of tert-butyl (2R)-2-acetyloxy-2-[(2R)-4-[1-(2-methoxy-2-oxoethyl)-2-oxoquinolin-7-yl]-3-oxomorpholin-2-yl]acetate (compound aa10-3)

According to the Step 2-3 in synthetic method for EXAMPLE aa2, compound aa10-2 (1.50 g) was used instead of aa2-2 to obtain compound aa10-3 (1.77 g) as a pale yellow amorphous.

### [Step 10-4] Synthesis of (2R)-2-acetyloxy-2-[(2R)-4-[1-(2-methoxy-2-oxoethyl)-2-oxoquinolin-7-yl]-3-oxomorpholin-2-yl]acetic acid (compound aa10-4)

According to the Step 2-4 in synthetic method for EXAMPLE aa2, compound aa10-3 (1.63 g) was used instead of aa2-3 to obtain compound aa10-4 (1.69 g) as a pale yellow amorphous.

### [Step 10-5] Synthesis of methyl 2-[7-[(2R)-2-[(1R)-1-acetyloxy-2-[3-[[bis[(2-methylpropan-2-yl)oxycarbonyl]amino]methyl]-4-cyanoanilino]-2-oxoethyl]-3-oxomor pholin-4-yl]-2-oxoquinolin-1-yl]acetate (compound aa10-5)

According to the Step 2-5 in synthetic method for EXAMPLE aa2, compound aa 10-4 (1.00 g) was used instead of aa2-4 to obtain compound aa 10-5 (1.13 g) as a pale yellow amorphous.

### [Step 10-6] Synthesis of methyl 2-[7-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpholi n-4-yl]-2-oxoquinolin-1-yl]acetate (compound aa10-6)

To a solution of compound aa10-5 (0.4 g) in MeOH (5.3 mL), was added 28% NaOMe in MeOH (0.13 mL) at 0 °C. The reaction mixture was stirred at 0 °C for 15 min. Then water was added into the mixture and it was extracted with EtOAc. The organic layer was washed with brine, dried over anhydrous Na₂SO₄. The solvent was removed under reduced pressure to obtain compound aa10-6 (0.34 g) as a pale yellow amorphous solid. It was used to the next step without further purification.

### [Step 10-7] Synthesis of methyl 2-[7-[(2R)-2-[(1R)-2-[3-(aminomethyl)-4-cyanoanilino]-1-hydroxy-2-oxoethyl]-3-oxomorpholin-4-yl]-2-oxoquinolin-1-yl]acetate hydrochloride (compound aa10-7)

According to the Step 2-7 in synthetic method for EXAMPLE aa2, compound aa10-6 (0.32 g) was used instead of aa2-6 to obtain compound aa10-7 (0.22 g) as a pale yellow amorphous solid.

### [Step 10-8] Synthesis of methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]ace tate hydrochloride (EXAMPLE aa10)

According to the Step 2-8 in synthetic method for EXAMPLE aa2, compound aa10-7 (0.21 g) was used instead of aa2-7 to obtain EXAMPLE aa10 (0.20 g) as a pale yellow amorphous solid.

### EXAMPLE aa11

### Synthesis of 2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetic acid hydrochloride (EXAMPLE aa11)

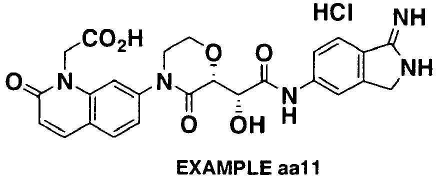

### [Step 11-1] Synthesis of 2-[7-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpholi n-4-yl]-2-oxoquinolin-1-yl]acetic acid (compound aa11-1)

To a solution of compound aa 10-5 (0.4 g) in MeOH (5 mL), was added 1N NaOH (5 mL) at 0 °C. The reaction mixture was stirred at room temperature for 35 min. Then water and 1N HCl were added into the mixture and it was extracted with EtOAc. The organic layer was washed with brine, dried over anhydrous Na₂SO₄. The solvent was removed under reduced pressure. The resulting residue was purified by silica gel column chromatography (CH₂Cl₂/MeOH = 95/5) to obtain compound aa11-1 (0.18 g) as a white amorphous solid.

### [Step 11-2] Synthesis of 2-[7-[(2R)-2-[(1R)-2-[3-(aminomethyl)-4-cyanoanilino]-1-hydroxy-2-oxoethyl]-3-oxomorpholin-4-yl]-2-oxoquinolin-1-yl]acetic acid hydrochloride (compound aa11-2)

According to the Step 2-7 in synthetic method for EXAMPLE aa2, compound aa 11-1 (0.18 g) was used instead of aa2-6 to obtain compound aa 11-2 (0.13 g) as a white amorphous solid.

### [Step 11-3] Synthesis of 2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]ace tic acid hydrochloride (EXAMPLE aa11)

According to the Step 2-8 in synthetic method for EXAMPLE aa2. compound aa 11-2 (0.13 g) was used instead of aa2-7 to obtain EXAMPLE aa11 (0.11 g) as a white amorphous solid.

### EXAMPLE aa 12

### Synthesis of (2R)-2-hydroxy-N-(1-imino-2.3-dihydroisoindol-5-yl)-2-[(2R)-4-(8-methyl-2-oxo-1H-quinolin-7-yl)-3-oxomorpholin-2-yl]acetamide hydrochloride (EXAMPLE aa12)

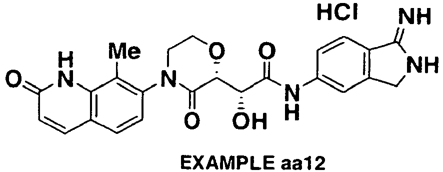

### [Step 12-1] Synthesis of (E)-3-ethoxy-N-(3-iodo-2-methylphenyl)prop-2-enamide (compound aa12-1)

According to the Step 4-3 in synthetic method for EXAMPLE aa4, 3-Iodo-2-methylaniline (0.50 g) was used instead of aa4-2 to obtain compound aa12-1 (0.68 g) as a brown amorphous solid.

### [Step 12-2] Synthesis of 7-iodo-8-methyl-1H-quinolin-2-one (compound aa12-2)

According to the Step 4-4 in synthetic method for EXAMPLE aa4, compound aa12-1 (0.68 g) was used instead of aa4-3 to obtain compound aa12-2 (0.47 g) as a pale yellow amorphous solid.

### [Step 12-3] Synthesis of tert-butyl (2R)-2-hydroxy-2-[(2R)-4-(8-methyl-2-oxo-1H-quinolin-7-yl)-3-oxomorpholin-2-yl]acetate (compound aa12-3)

According to the Step 2-2 in synthetic method for EXAMPLE aa2. compound aa12-2 (0.20 g) was used instead of aa2-1 to obtain compound aa12-3 (89 mg) as a pale yellow amorphous solid.

### [Step 12-4] Synthesis of tert-butyl (2R)-2-acetyloxy-2-[(2R)-4-(8-methyl-2-oxo-1H-quinolin-7-yl)-3-oxomorpholin-2-yl]acetate (compound aa12-4)

According to the Step 2-3 in synthetic method for EXAMPLE aa2, compound aa 12-3 (1.11 g) was used instead of aa2-2 to obtain compound aa 12-4 (1.23 g) as a yellow amorphous solid.

### [Step 12-5] Synthesis of (2R)-2-acetyloxy-2-[(2R)-4-(8-methyl-2-oxo-1H-quinolin-7-yl)-3-oxomorpholin-2-yl]acetic acid (compound aa12-5)

According to the Step 2-4 in synthetic method for EXAMPLE aa2, compound aa12-4 (1.22 g) was used instead of aa2-3 to obtain compound aa12-5 (1.39 g) as a colorless amorphous solid.

### [Step 12-6] Synthesis of [(1R)-2-(3-[[bis[(2-methylpropan-2-yl)oxycarbonyl]-amino]methyl]-4-cyanoanilino]-1-((2R)-4-(8-methyl-2-oxo-1H-quinolin-7-yl)-3-oxomo rpholin-2-yl]-2-oxoethyl] acetate (compound aa12-6)

According to the Step 2-5 in synthetic method for EXAMPLE aa2, compound aa12-5 (1.38 g) was used instead of aa2-4 to obtain compound aa12-6 (0.89 g) as a pale yellow amorphous solid.

### [Step 12-7] Synthesis of tert-butyl N-[[2-cyano-5-[[(2R)-2-hydroxy-2-[(2R)-4-(8-methyl-2-oxo-1H-quinolin-7-yl)-3-oxomorpholin-2-yl]acetyl]amino]phenyl]methyl]-N-[(2-methylpropan-2-yl)oxycarbonyl]carbamate (compound aa 12-7)

According to the Step 2-6 in synthetic method for EXAMPLE aa2, compound aa12-6 (0.88 g) was used instead of aa2-5 to obtain compound aa12-7 (0.80 g) as a pale yellow amorphous solid.

### [Step 12-8] Synthesis of (2R)-N-[3-(aminomethyl)-4-cyanophenyl]-2-hydroxy-2-[(2R)-4-(8-methyl-2-oxo-1H-quinolin-7-yl)-3-oxomorpholin-2-yl]acetamide hydrochloride (compound aa12-8)

According to the Step 2-7 in synthetic method for EXAMPLE aa2. compound aa12-7 (0.78 g) was used instead of aa2-6 to obtain compound aa12-8 (0.57 g) as a pale yellow amorphous solid.

### [Step 12-9] Synthesis of (2R)-2-hydroxy-N-(1-imino-2,3-dihydroisoindol-5-yl)-2-[(2R)-4-(8-methyl-2-oxo-1H-quinolin-7-yl)-3-oxomorpholin-2-yl]acetamide hydrochloride (EXAMPLE aa12)

According to the Step 2-8 in synthetic method for EXAMPLE aa2. compound aa12-8 (0.56 g) was used instead of aa2-7 to obtain EXAMPLE aa12 (0.55 g) as a pale yellow amorphous solid.

### EXAMPLE aa 13

### Synthesis of (2R)-2-hydroxy-N-(1-imino-2,3-dihydroisoindol-5-yl)-2-[(2R)-4-(2-methoxyquinolin-7-yl)-3-oxomorpholin-2-yl]acetamide hydrochloride (EXAMPLE aa13)

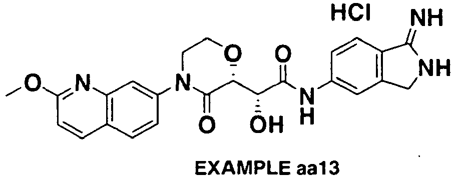

### [Step 13-1] Synthesis of 7-iodo-2-methoxyquinoline (compound aa13-1)

To a solution of compound aa7-1 (1.00 g) in CH₂Cl₂ (100 mL), was added trimethyloxonium tetrafluoroborate (1.09 g) at 0 °C. The reaction mixture was stirred at room temperature for 4 days. The mixture was concentrated in vacuo, then water was added to the resulting residue. The mixture was extracted with EtOAc and the organic layer was washed with brine and was dried over anhydrous Na₂SO₄. The solvent was removed under reduced pressure to obtain compound aa13-1 (0.95 g) as a colorless amorphous solid. It was used to the next step without further purification.

### [Step 13-2] Synthesis of tert-butyl (2R)-2-hydroxy-2-[(2R)-4-(2-methoxyquinolin-7-yl)-3-oxomorpholin-2-yl]acetate (compound aa13-2)

According to the Step 2-2 in synthetic method for EXAMPLE aa2, compound aa13-1 (0.93 g) was used instead of aa2-1 to obtain compound aa13-2 (0.28 g) as a colorless amorphous solid.

### [Step 13-3] Synthesis of tert-butyl (2R)-2-acetyloxy-2-[(2R)-4-(2-methoxyquinolin-7-yl)-3-oxomorpholin-2-yl]acetate (compound aa13-3)

According to the Step 2-3 in synthetic method for EXAMPLE aa2, compound aa13-2 (0.28 g) was used instead of aa2-2 to obtain compound aa13-3 (0.32 g) as a colorless amorphous solid.

### [Step 13-4] Synthesis of (2R)-2-acetyloxy-2-[(2R)-4-(2-methoxyquinolin-7-yl)-3-oxomorpholin-2-yl]acetic acid (compound aa13-4)

According to the Step 2-4 in synthetic method for EXAMPLE aa2, compound aa13-3 (0.31 g) was used instead of aa2-3 to obtain compound aa 13-4 (0.27 g) as a colorless amorphous solid.

### [Step 13-5] Synthesis of [(1R)-2-[3-[[bis[(2-methylpropan-2-yl)oxycarbonyl]-amino]methyl]-4-cyanoanilino]-1-[(2R)-4-(2-methoxyquinolin-7-yl)-3-oxomorpholin-2 -yl]-2-oxoethyl] acetate (compound aa13-5)

According to the Step 2-5 in synthetic method for EXAMPLE aa2, compound aa13-4 (0.27 g) was used instead of aa2-4 to obtain compound aa 13-5 (0.33 g) as a colorless amorphous solid.

### [Step 13-6] Synthesis of tert-butyl N-[[2-cyano-5-[[(2R)-2-hydroxy-2-[(2R)-4-(2-methoxyquinolin-7-yl)-3-oxomorpholin-2-yl]acetyl]amino]phenyl]methyl]-N-[(2-m ethylpropan-2-yl)oxycarbonyl]carbamate (compound aa13-6)

According to the Step 2-6 in synthetic method for EXAMPLE aa2, compound aa13-5 (0.32 g) was used instead of aa2-5 to obtain compound aa13-6 (0.28 g) as a colorless amorphous solid.

### [Step 13-7] Synthesis of (2R)-N-[3-(aminomethyl)-4-cyanophenyl]-2-hydroxy-2-[(2R)-4-(2-methoxyquinolin-7-yl)-3-oxomorpholin-2-yl]acetamide hydrochloride (compound aa13-7)

According to the Step 2-7 in synthetic method for EXAMPLE aa2, compound aa13-6 (0.30 g) was used instead of aa2-6 to obtain compound aa13-7 (0.23 g) as a colorless amorphous solid.

### [Step 13-8] Synthesis of (2R)-2-hydroxy-N-(1-imino-2,3-dihydroisoindol-5-yl)-2-[(2R)-4-(2-methoxyquinolin-7-yl)-3-oxomorpholin-2-yl]acetamide hydrochloride (EXAMPLE aa 13)

According to the Step 2-8 in synthetic method for EXAMPLE aa2, compound aa13-7 (0.22 g) was used instead of aa2-7 to obtain EXAMPLE aa13 (45 mg) as a yellow amorphous solid.

### EXAMPLE aa 14

### Synthesis of (2R)-2-hydroxy-N-(1-imino-2,3-dihydroisoindol-5-yl)-2-[(2R)-3-oxo-4-quinolin-7-ylmorpholin-2-yl]acetamide dihydrochloride (EXAMPLE aa14)

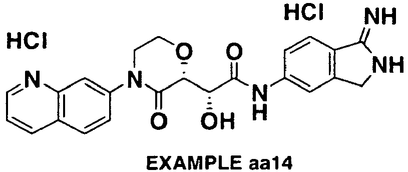

### [Step 14-1] Synthesis of tert-butyl (2R)-2-hydroxy-2-[(2R)-3-oxo-4-quinolin-7-ylmorpholin-2-yl]acetate (compound aa14-1)

According to the Step 2-2 in synthetic method for EXAMPLE aa2, 7-Iodoquinoline (2.76 g) was used instead of aa2-1 to obtain compound aa14-1 (2.50 g) as a colorless amorphous solid.

### [Step 14-2] Synthesis of tert-butyl (2R)-2-acetyloxy-2-[(2R)-3-oxo-4-quinolin-7-ylmorpholin-2-yl]acetate (compound aa14-2)

According to the Step 2-3 in synthetic method for EXAMPLE aa2, compound aa14-1 (1.25 g) was used instead of aa2-2 to obtain compound aa14-2 (1.32 g) as a colorless amorphous solid.

### [Step 14-3] Synthesis of (2R)-2-acetyloxy-2-[(2R)-3-oxo-4-quinolin-7-ylmorpholin-2-yl]acetic acid trifluoroacetic acid salt (compound aa14-3)

According to the Step 2-4 in synthetic method for EXAMPLE aa2, compound aa14-2 (1.3 g) was used instead of aa2-3 to obtain compound aa14-3 (1.4 g) as a colorless amorphous solid.

### [Step 14-4] Synthesis of [(1R)-2-[3-[[bis[(2-methylpropan-2-yl)oxycarbonyl] amino]methyl]-4-cyanoanilino]-2-oxo-1-[(2R)-3-oxo-4-quinolin-7-ylmorpholin-2-yl]et hyl] acetate (compound aa14-4)

According to the Step 2-5 in synthetic method for EXAMPLE aa2, compound aa14-3 (0.5 g) was used instead of aa2-4 to obtain compound aa14-4 (0.64 g) as a colorless amorphous solid.

### [Step 14-5] Synthesis of tert-butyl N-[[2-cyano-5-[[(2R)-2-hydroxy-2-[(2R)-3-oxo-4-quinolin-7-ylmorpholin-2-yl]acetyl]amino]phenyl]methyl]-N-[(2-methylpropan-2-yl)ox ycarbonyl]carbamate (compound aa14-5)

According to the Step 2-6 in synthetic method for EXAMPLE aa2, compound aa14-4 (0.63 g) was used instead of aa2-5 to obtain compound aa14-5 (0.59 g) as a colorless amorphous solid.

### [Step 14-6] Synthesis of (2R)-N-[3-(aminomethyl)-4-cyanophenyl]-2-hydroxy-2-[(2R)-3-oxo-4-quinolin-7-ylmorpholin-2-yl]acetamide dihydrochloride (compound aa14-6)

According to the Step 2-7 in synthetic method for EXAMPLE aa2, compound aa14-5 (0.58 g) was used instead of aa2-6 to obtain compound aa14-6 (0.46 g) as a colorless amorphous solid.

### [Step 14-7] Synthesis of (2R)-2-hydroxy-N-(1-imino-2,3-dihydroisoindol-5-yl)-2-[(2R)-3-oxo-4-quinolin-7-ylmorpholin-2-yl]acetamide dihydrochloride (EXAMPLE aa 14)

According to the Step 2-8 in synthetic method for EXAMPLE aa2, compound aa14-6 (0.45 g) was used instead of aa2-7 to obtain EXAMPLE aa14 (0.31 g) as a yellow amorphous solid.

### EXAMPLE aa 15

### Synthesis of (2R)-N-(3-amino-1,2-benzisoxazol-6-yl)-2-hydroxy-2-[(2R)-3-oxo-4-quinolin-7-ylmorpholin-2-yl]acetamide hydrochloride (EXAMPLE aa15)

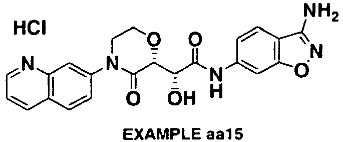

### [Step 15-1] Synthesis of [(1R)-2-[[3-(1,3-dioxoisoindol-2-yl)-1,2-benzisoxazol-6-yl]amino]-2-oxo-1-[(2R)-3-oxo-4-quinolin-7-ylmorpholin-2-yl]ethyl] acetate (compound aa15-1)

According to the Step 2-5 in synthetic method for EXAMPLE aa2, compound aa14-3 (0.5 g) and 6-amino-3-(1,3-dioxoisoindol-2-yl)-1,2-benzisoxazole (0.61 g) were used instead of aa2-4 and tert-butyl N-(2-cyano-5-aminophenyl)methyl-N-[(2-methylpropan-2-yl)oxycarbonyl]carbamate to obtain compound 15-1 (0.42 g) as a colorless amorphous solid.

### [Step 15-2] Synthesis of (2R)-N-(3-amino-1,2-benzisoxazol-6-yl)-2-hydroxy-2-[(2R)-3-oxo-4-quinolin-7-ylmorpholin-2-yl]acetamide hydrochloride (EXAMPLE aa15)

To a solution of compound aa15-1 (400 mg) in CH₂Cl₂-MeOH (14-14 mL). was added NH₂NH₂-H₂O (0.32 mL) at 0 °C. The reaction mixture was stirred at room temperature for 3 h. The precipitate was appeared. The precipitate was collected by filtration and the filtrate was concentrated in vacuo. The resulting residue was suspended in CH₂Cl₂-MeOH. then the precipitate was collected by filtration and combined the first crops. Then the precipitate was solved in 10% HCl-MeOH then the solvent was removed in vacuo to obtain EXAMPLE aa15 (240 mg) as a pale yellow amorphous solid.

### EXAMPLE aa16

### Synthesis of (2R)-2-hydroxy-N-(1-imino-2,3-dihydroisoindol-5-yl)-2-[(2R)-3-oxo-4-(2-oxo-1H-quinolin-5-yl)morpholin-2-yl]acetamide hydrochloride (EXAMPLE aa16)

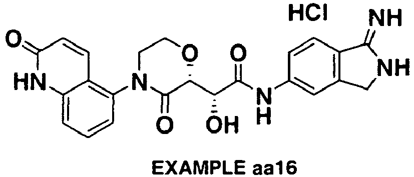

### [Step 16-1] Synthesis of 5-iodo-1H-quinolin-2-one (compound aa 16-1)

To the mixture of 5-aminoquinolin-2(1H)-one (0.1 g) in AcOH-H₂O (0.73 - 0.3 mL), was added dropwise conc. H₂SO₄ (66 uL) at 0 °C. Then a solution of NaNO₂ (86 mg) in water (0.38 mL) was added dropwise into the reaction mixture at 0 °C. The mixture was stirred at 0 °C for 30 min, then a solution of KI (0.31 g) in water (0.25 mL) was added dropwise at 0 °C. The reaction mixture was stirred at room temperature for 4 h. Water was added to the reaction mixture and it was extracted with EtOAc. The organic layer was washed with water, saturated NaHCO₃ aq. and brine, dried over anhydrous Na₂SO₄. The solvent was removed under reduced pressure. Trituration with Et₂O followed by filtration gave a16-1 (0.14 g) as a brown amorphous solid. It was used to the next step without further purification.

### [Step 16-2] Synthesis of tert-butyl (2R)-2-hydroxy-2-[(2R)-3-oxo-4-(2-oxo-1H-quinolin-5-yl)morpholin-2-yl]acetate (compound aa16-2)

According to the Step 2-2 in synthetic method for EXAMPLE aa2, compound aa 16-1 (1.20 g) was used instead of aa2-1 to obtain compound aa 16-2 (0.48 g) as an orange amorphous solid.

### [Step 16-3] Synthesis of tert-butyl (2R)-2-acetyloxy-2-[(2R)-3-oxo-4-(2-oxo-1H-quinolin-5-yl)morpholin-2-yl]acetate (compound aa16-3)

According to the Step 2-3 in synthetic method for EXAMPLE aa2, compound aa16-2 (0.47 g) was used instead of aa2-2 to obtain compound aa16-3 (0.55 g) as a brown amorphous solid.

### [Step 16-4] Synthesis of (2R)-2-acetyloxy-2-[(2R)-3-oxo-4-(2-oxo-1H-quinolin-5-yl)morpholin-2-yl]acetic acid (compound aa16-4)

According to the Step 2-4 in synthetic method for EXAMPLE aa2, compound aa16-3 (0.51 g) was used instead of aa2-3 to obtain compound aa16-4 (0.38 g) as an orange amorphous solid.

### [Step 16-5] Synthesis of [(1R)-2-[3-[[bis[(2-methylpropan-2-yl)oxycarbonyl]-aminoymethyl]-4-cyanoanilino]-2-oxo-1-[(2R)-3-oxo-4-(2-oxo-1H-quinolin-5-yl)morp holin-2-yl]ethyl] acetate (compound aa16-5)

According to the Step 2-5 in synthetic method for EXAMPLE aa2, compound aa16-4 (0.37 g) was used instead of aa2-4 to obtain compound aa16-5 (0.19 g) as an orange amorphous solid.

### [Step 16-6] Synthesis of tert-butyl N-[[2-cyano-5-[[(2R)-2-hydroxy-2-[(2R)-3-oxo-4-(2-oxo-1H-quinolin-5-yl)morpholin-2-yl]acetyl]amino]phenyl]methyl]-N-[(2-methyl propan-2-yl)oxycarbonyl]carbamate (compound aa16-6)

According to the Step 2-6 in synthetic method for EXAMPLE aa2, compound aa16-5 (0.18 g) was used instead of aa2-5 to obtain compound aa16-6 (0.16 g) as an orange amorphous solid.

### [Step 16-7] Synthesis of (2R)-N-[3-(aminomethyl)-4-cyanophenyl]-2-hydroxy-2-[(2R)-3-oxo-4-(2-oxo-1H-quinolin-5-yl)morpholin-2-yl]acetamide hydrochloride (compound aa16-7)

According to the Step 2-7 in synthetic method for EXAMPLE aa2, compound aa16-6 (0.15 g) was used instead of aa2-6 to obtain compound aa16-7 (0.13 g) as a pale yellow amorphous solid.

### [Step 16-8] Synthesis of (2R)-2-hydroxy-N-(1-imino-2,3-dihydroisoindol-5-yl)-2-[(2R)-3-oxo-4-(2-oxo-1H-quinolin-5-yl)morpholin-2-yl]acetamide hydrochloride (EXAMPLE aa16)

According to the Step 2-8 in synthetic method for EXAMPLE aa2, compound aa16-7 (0.12 g) was used instead of aa2-7 to obtain EXAMPLE aa16 (0.10 g) as a pale yellow amorphous solid.

### EXAMPLE aa 17

### Synthesis of (2R)-2-hydroxy-N-(1-imino-2,3-dihydroisoindol-5-yl)-2-[(2R)-3-oxo-4-(1-oxo-3,4-dihydro-2H-isoquinolin-6-yl)morpholin-2-yl]acetamide hydrochloride (EXAMPLE aa17)

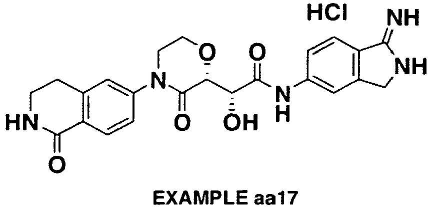

### [Step 17-1] Synthesis of tert-butyl (2R)-2-hydroxy-2-[(2R)-3-oxo-4-(1-oxo-3,4-dihydro-2H-isoquinolin-6-yl]morpholin-2-yl]acetate (compound aa17-1)

According to the Step 2-2 in synthetic method for EXAMPLE aa2, 6-bromo-3,4-dihydroisoquinolin-1(2H)-one (500 mg) was used instead of aa2-1 to obtain compound aa17-1 (288 mg) as a yellow amorphous solid (contained diastereomer).

### [Step 17-2] Synthesis of tert-butyl (2R)-2-acetyloxy-2-[(2R)-3-oxo-4-(1-oxo-3,4-dihydro-2H-isoquinolin-6-yl)morpholin-2-yl]acetate (compound aa17-2)

According to the Step 2-3 in synthetic method for EXAMPLE aa2, compound aa17-1 (231 mg) was used instead of aa2-2 to obtain compound aa17-2 (313 g) as a white amorphous solid (contained diastereomer).

### I Step 17-3] Synthesis of (2R)-2-acetyloxy-2-[(2R)-3-oxo-4-(1-oxo-3,4-dihydro-2H-isoquinolin-6-yl)morpholin-2-yl]acetic acid (compound aa17-3)

According to the Step 2-4 in synthetic method for EXAMPLE aa2, compound aa17-2 (270 mg) was used instead of aa2-3 to obtain compound aa17-3 (254 mg) as a white amorphous solid (contained diastereomer).

### [Step 17-4] Synthesis of [(1R)-2-[3-[[bis[(2-methylpropan-2-yl)oxycarbonyl]-amino]methyl]-4-cyanoanilino]-2-oxo-1-[(2R)-3-oxo-4-(1-oxo-3,4-dihydro-2H-isoquin olin-6-yl)morpholin-2-yl]ethyl] acetate (compound aa17-4)

According to the Step 2-5 in synthetic method for EXAMPLE aa2, compound aa17-3 (250 mg) was used instead of aa2-4 to obtain compound aa17-4 (120 mg) as a white amorphous solid.

### [Step 17-5] Synthesis of tert-butyl N-[[2-cyano-5-[[(2R)-2-hydroxy-2-[(2R)-3-oxo-4-(1-oxo-3,4-dihydro-2H-isoquinolin-6-yl)morpholin-2-yl]acetyl]amino]phenyl]methyl ]-N-[(2-methylpropan-2-yl)oxycarbonyl]carbamate (compound aa17-5)

According to the Step 2-6 in synthetic method for EXAMPLE aa2, compound aa17-4 (98 mg) was used instead of aa2-5 to obtain compound aa17-5 (83 mg) as a white amorpous solid.

### [Step 17-6]

### Synthesis of (2R)-N-[3-(aminomethyl)-4-cyanophenyl]-2-hydroxy-2-[(2R)-3-oxo-4-(1-oxo-3,4-dihy dro-2H-isoquinolin-6-yl)morpholin-2-yl]acetamide hydrochloride (compound aa17-6)

According to the Step 2-7 in synthetic method for EXAMPLE aa2, compound aa17-5 (79 mg) was used instead of aa2-6 to obtain compound aa17-6 (75 mg) as a white amorphous solid.

### [Step 17-7] Synthesis of (2R)-2-hydroxy-N-(1-imino-2,3-dihydroisoindol-5-yl)-2-[(2R)-3-oxo-4-(1-oxo-3.4-dihydro-2H-isoquinolin-6-yl)morpholin-2-yl]acetami de hydrochloride (EXAMPLE aa17)

According to the Step 2-8 in synthetic method for EXAMPLE aa2. compound aa17-6 (55 mg) was used instead of aa2-7 to obtain EXAMPLE aa17 (47 mg) as a white amorphous solid.

### EXAMPLE aa 18

### Synthesis of (2R)-2-hydroxy-N-(1-imino-2,3-dihydroisoindol-5-yl)-2-[(2R)-3-oxo-4-(2-oxo-3,4-dihydro-1H-quinolin-7-yl)morpholin-2-yl]acetamide hydrochloride (EXAMPLE aa18)

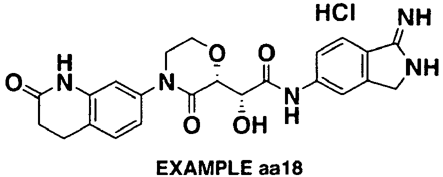

### [Step 18-1] Synthesis of tert-butyl (2R)-2-hydroxy-2-[(2R)-3-oxo-4-(2-oxo-3,4-dihydro-1H-quinolin-7-yl)morpholin-2-yl]acetate (compound aa18-1)

According to the Step 2-2 in synthetic method for EXAMPLE aa2, 3,4-dihydro-7-iodo-2(1H)-quinolinone (3.40 g) was used instead of aa2-1 to obtain compound aa18-1 (0.59 g) as a brown amorphous solid.

### [Step 18-2] Synthesis of tert-butyl (2R)-2-acetyloxy-2-[(2R)-3-oxo-4-(2-oxo-3,4-dihydro-1H-quinolin-7-yl)morpholin-2-yl]acetate (compound aa18-2)

According to the Step 2-3 in synthetic method for EXAMPLE aa2, compound aa18-1 (0.57 g) was used instead of aa2-2 to obtain compound aa18-2 (0.59 g) as a white amorphous solid.

### [Step 18-3] Synthesis of (2R)-2-acetyloxy-2-[(2R)-3-oxo-4-(2-oxo-3,4-dihydro-1H-quinolin-7-yl)morpholin-2-yl]acetic acid (compound aa18-3)

According to the Step 2-4 in synthetic method for EXAMPLE aa2, compound aa18-2 (0.28 g) was used instead of aa2-3 to obtain compound aa18-3 (0.21 g) as a white amorphous solid.

### [Step 18-4] Synthesis of [(1R)-2-[3-[[bis[(2-methylpropan-2-yl)oxycarbonyl]-amino]methyl]-4-cyanoanilino]-2-oxo-1-[(2R)-3-oxo-4-(2-oxo-3,4-dihydro-1H-quinoli n-7-yl)morpholin-3-yl]ethyl] acetate (compound aa18-4)

According to the Step 2-5 in synthetic method for EXAMPLE aa2, compound aa18-3 (75 mg) was used instead of aa2-4 to obtain compound aa18-4 (62 mg) as a white amorphous solid.

### [Step 18-5] Synthesis of tert-butyl N-[[2-cyano-5-[[(2R)-2-hydroxy-2-[(2R)-3-oxo-4-(2-oxo-3,4-dihydro-1H-quinolin-7-yl)morpholin-2-yl]acetyl]amino]phenyl]me thyl]-N-[(2-methylpropan-2-yl)oxycarbonyl]carbamate (compound aa18-5)

According to the Step 2-6 in synthetic method for EXAMPLE aa2, compound aa18-4 (52 mg) was used instead of aa2-5 to obtain compound aa18-5 (47 mg) as a white amorphous solid.

### [Step 18-6] Synthesis of (2R)-N-[3-(aminomethyl)-4-cyanophenyl]-2-hydroxy-2-[(2R)-3-oxo-4-(2-oxo-3,4-dihydro-1H-quinolin-7-yl)morpholin-2-yl]acetam ide hydrochloride (compound aa18-6)

According to the Step 2-7 in synthetic method for EXAMPLE aa2, compound aa18-5 (44 mg) was used instead of aa2-6 to obtain compound aa18-6 (33 mg) as a white amorphous solid.

### [Step 18-7]

### Synthesis of (2R)-2-hydroxy-N-(1-imino-2,3-dihydroisoindol-5-yl)-2-[(2R)-3-oxo-4-(2-oxo-3,4-dih ydro-1H-quinolin-7-yl)morpholin-2-yl]acetamide hydrochloride (EXAMPLE aa18)

According to the Step 2-8 in synthetic method for EXAMPLE aa2, compound aa18-6 (31 g) was used instead of aa2-7 to obtain EXAMPLE aa18 (26 g) as a white amorphous solid.

### EXAMPLE aa19

### Synthesis of (2R)-2-hydroxy-N-(1-imino-2,3-dihydroisoindol-5-yl)-2-[(2R)-4-(1-methyl-2-oxo-3,4-dihydroquinolin-7-yl)-3-oxomorpholin-2-yl]acetamide hydrochloride (EXAMPLE aa19)

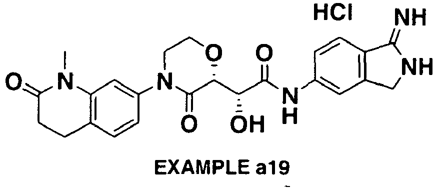

### [Step 19-1] Synthesis of 7-iodo-1-methyl-3,4-dihydroquinolin-2-one (compound aa19-1)

According to the Step 8-1 in synthetic method for EXAMPLE aa8, 3,4-dihydro-7-iodo-2(1H)-quinolinone (1.50 g) was used instead of aa7-1 to obtain compound aa19-1 (1.23 g) as a pale yellow amorphous solid.

### [Step 19-2] Synthesis of tert-butyl (2R)-2-hydroxy-2-[(2R)-4-(1-methyl-2-oxo-3,4-dihydroquinolin-7-yl)-3-oxomorpholin-2-yl]acetate (compound aa19-2)

According to the Step 2-2 in synthetic method for EXAMPLE aa2, compound aa19-1 (1.22 g) was used instead of aa2-1 to obtain compound aa19-2 (0.95 g) as a pale yellow amorphous solid.

### [Step 19-3] Synthesis of tert-butyl (2R)-2-acetyloxy-2-[(2R)-4-(1-methyl-2-oxo-3,4-dihydroquinolin-7-yl)-3-oxomorpholin-2-yl]acetate (compound aa19-3)

According to the Step 2-3 in synthetic method for EXAMPLE aa2, compound aa19-2 (0.60 g) was used instead of aa2-2 to obtain compound aa19-3 (0.68 g) as a pale yellow amorphous solid.

### [Step 19-4] Synthesis of (2R)-2-acetyloxy-2-[(2R)-4-(1-methyl-2-oxo-3,4-dihydroquinolin-7-yl)-3-oxomorpholin-2-yl]acetic acid (compound aa19-4)

According to the Step 2-4 in synthetic method for EXAMPLE aa2, compound aa19-3 (0.65 g) was used instead of aa2-3 to obtain compound aa19-4 (0.74 g) as a pale brown amorphous solid.

### [Step 19-5] Synthesis of [(1R)-2-[3-[[bis[(2-methylpropan-2-yl)oxycarbonyl]-amino]methyl]-4-cyanoanilino]-1-[(2R)-4-(1-methyl-2-oxo-3,4-dihydroquinolin-7-yl)-3 -oxomorpholin-2-yl]-2-oxoethyl] acetate (compound aa19-5)

According to the Step 2-5 in synthetic method for EXAMPLE aa2, compound aa19-4 (0.56 g) was used instead of aa2-4 to obtain compound aa19-5 (0.78 g) as a white amorphous solid.

### [Step 19-6] Synthesis of tert-butyl N-[[2-cyano-5-[[(2R)-2-hydroxy-2-[(2R)-4-(1-methyl-2-oxo-3,4-dihydroquinolin-7-yl)-3-oxomorpholin-2-yl]acetyl]amino]phenyl] methyl]-N-[(2-methylpropan-2-yl)oxycarbonyl]carbamate (compound aa19-6)

According to the Step 2-6 in synthetic method for EXAMPLE aa2, compound aa19-5 (0.75 g) was used instead of aa2-5 to obtain compound aa 19-6 (0.69 g) as a white amorphous solid.

### [Step 19-7] Synthesis of (2R)-N-[3-(aminomethyl)-4-cyanophenyl]-2-hydroxy-2-[(2R)-4-(1-methyl-2-oxo-3,4-dihydroquinolin-7-yl)-3-oxomorpholin-2-yl]ac etamide hydrochloride (compound aa19-7)

According to the Step 2-7 in synthetic method for EXAMPLE aa2, compound aa19-6 (0.67 g) was used instead of aa2-6 to obtain compound aa 19-7 (0.50 g) as a white amorphous solid.

### [Step 19-8] Synthesis of (2R)-2-hydroxy-N-(1-imino-2,3-dihydroisoindol-5-yl)-2-[(2R)-4-(1-methyl-2-oxo-3,4-dihydroquinolin-7-yl)-3-oxomorpholin-2-yl]acetami de hydrochloride (EXAMPLE aa19)

According to the Step 2-8 in synthetic method for EXAMPLE aa2, compound aa19-7 (0.49 g) was used instead of aa2-7 to obtain EXAMPLE aa19 (0.35 g) as a white amorphous solid.

### EXAMPLE aa20

### Synthesis of (2R)-2-[(2R)-4-[1-(cyclopropylmethyl)-2-oxo-3,4-dihydroquinolin-7-yl]-3-oxomorpholin-2-yl]-2-hydroxy-N-(1-imino-2,3-dihydroisoindol-5-yl)acetamide hydrochloride (EXAMPLE aa20)

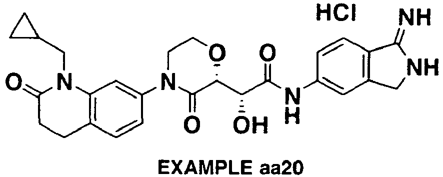

### [Step 20-1] Synthesis of 1-(cyclopropylmethyl)-7-iodo-3,4-dihydroquinolin-2-one (compound aa20-1)

According to the Step 8-1 in synthetic method for EXAMPLE aa8, 3,4-dihydro-7-iodo-2(1H)-quinolinone (1.50 g) and cyclopropylmethyl bromide (0.80 mL) were used instead of a7-1 and MeI to obtain compound aa20-1 (1.23 g) as a pale red amorphous solid.

### [Step 20-2] Synthesis of tert-butyl (2R)-2-[(2R)-4-[1-(cyclopropylmethyl)-2-oxo-3,4-dihydroquinolin-7-yl]-3-oxomorpholin-2-yl]-2-hydroxyacetate (compound aa20-2)

According to the Step 2-2 in synthetic method for EXAMPLE aa2, compound aa20-1 (1.22 g) was used instead of aa2-1 to obtain compound aa20-2 (1.16 g) as a pale brown amorphous solid.

### [Step 20-3] Synthesis of tert-butyl (2R)-2-acetyloxy-2-[(2R)-4-[1-(cyclopropylmethyl)-2-oxo-3,4-dihydroquinolin-7-yl]-3-oxomorpholin-2-yl]acetate (compound aa20-3)

According to the Step 2-3 in synthetic method for EXAMPLE aa2, compound aa20-2 (0.60 g) was used instead of aa2-2 to obtain compound aa20-3 (0.65 g) as a pale yellow amorphous solid.

### [Step 20-4] Synthesis of (2R)-2-acetyloxy-2-[(2R)-4-[1-(cyclopropylmethyl)-2-oxo-3,4-dihydroquinolin-7-yl]-3-oxomorpholin-2-yl]acetic acid (compound aa20-4)

According to the Step 2-4 in synthetic method for EXAMPLE aa2, compound aa20-3 (0.64 g) was used instead of aa2-3 to obtain compound aa20-4 (0.70 g) as a pale brown amorphous solid.

### [Step 20-5] Synthesis of [(1R)-2-[3-[[bis[(2-methylpropan-2-yl)oxycarbonyl] amino]methyl]-4-cyanoanilino]-1-[(2R)-4-[1-(cyclopropylmethyl)-2-oxo-3,4-dihydroqu inolin-7-yl]-3-oxomorpholin-2-yl]-2-oxoethyl] acetate (compound aa20-5)

According to the Step 2-5 in synthetic method for EXAMPLE aa2, compound aa20-4 (0.55 g) was used instead of aa2-4 to obtain compound aa20-5 (0.67 g) as a white amorphous solid.

### [Step 20-6] Synthesis of tert-butyl N-[[2-cyano-5-[[(2R)-2-[(2R)-4-[1-(cyclopropylmethyl)-2-oxo-3,4-dihydroquinolin-7-yl]-3-oxomorpholin-2-yl]-2-hydroxy acetyl]amino]phenyl]methyl]-N-[(2-methylpropan-2-yl)oxycarbonyl]carbamate (compound aa20-6)

According to the Step 2-6 in synthetic method for EXAMPLE aa2, compound aa20-5 (0.66 g) was used instead of aa2-5 to obtain compound aa20-6 (0.62 g) as a white amorphous solid.

### [Step 20-7] Synthesis of (2R)-N-[3-(aminomethyl)-4-cyanophenyl]-2-[(2R)-4-[1-(cyclopropylmethyl)-2-oxo-3,4-dihydroquinolin-7-yl]-3-oxomorpholin-2-yl]-2-hydr oxyacetamide hydrochloride (compound aa20-7)

According to the Step 2-7 in synthetic method for EXAMPLE aa2, compound aa20-6 (0.61 g) was used instead of aa2-6 to obtain compound aa20-7 (0.47 g) as a white amorphous solid.

### [Step 20-8] Synthesis of (2R)-2-[(2R)-4-[1-(cyclopropylmethyl)-2-oxo-3,4-dihydroquinolin-7-yl]-3-oxomorpholin-2-yl]-2-hydroxy-N-(1-imino-2,3-dihydroisoindo 1-5-yl)acetamide hydrochloride (EXAMPLE aa20)

According to the Step 2-8 in synthetic method for EXAMPLE aa2, compound aa20-7 (0.46 g) was used instead of aa2-7 to obtain EXAMPLE aa20 (0.43 g) as a white amorphous solid.

### EXAMPLE aa21

### Synthesis of methyl 2-[7-[(2R)-2-[(1R)-1-hydroxy-2-[(-amino-2,3-dihydroisoindol-5-yl)amino]-2-oxoethyl]-3-oxomorpholin-4-yl]-2-oxo-3,4-dihydroquin olin-1-yl]acetate hydrochloride (EXAMPLE a21)

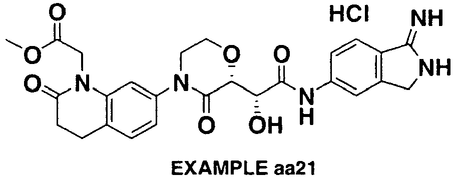

### [Step 21-1] Synthesis of methyl 2-(7-iodo-2-oxo-3,4-dihydroquinolin-1-yl)acetate (compound aa21-1)

According to the Step 8-1 in synthetic method for EXAMPLE aa8, 3,4-dihydro-7-iodo-2(1H)-quinolinone (2.12 g) and bromoacetic acid methyl ester (1.10 mL) were used instead of aa7-1 and MeI to obtain compound aa21-1 (2.24 g) as a white amorphous solid.

### [Step 21-2] Synthesis of tert-butyl (2R)-2-hydroxy-2-[(2R)-4-[1-(2-methoxy-2-oxoethyl)-2-oxo-3,4-dihydroquinolin-7-yl]-3-oxomorpholin-2-yl]acetate (compound aa21-2)

According to the Step 2-2 in synthetic method for EXAMPLE aa2, compound aa21-1 (2.23 g) was used instead of aa2-1 to obtain compound aa21-2 (2.27 g) as a pale brown amorphous solid.

### [Step 21-3] Synthesis of tert-butyl (2R)-2-acetyloxy-2-[(2R)-4-[1-(2-methoxy-2-oxoethyl)-2-oxo-3,4-dihydroquinolin-7-yl]-3-oxomorpholin-2-yl]acetate (compound aa21-3)

According to the Step 2-3 in synthetic method for EXAMPLE aa2, compound aa21-2 (2.27 g) was used instead of aa2-2 to obtain compound aa21-3 (2.17 g) as a pale yellow amorphous solid.

### [Step 21-4] Synthesis of (2R)-2-acetyloxy-2-[(2R)-4-[1-(2-methoxy-2-oxoethyl)-2-oxo-3,4-dihydroquinolin-7-yl]-3-oxomorpholin-2-yl]acetic acid (compound aa21-4)

According to the Step 2-4 in synthetic method for EXAMPLE aa2, compound aa21-3 (2.15 g) was used instead of aa2-3 to obtain compound aa21-4 (2.11 g) as a white amorphous.

### [Step 21-5] Synthesis of methyl 2-[7-[(2R)-2-[(1R)-1-acetyloxy-2-[3-[[bis[(2-methylpropan-2-yl)oxycarbonyl]amino]methyl]-4-cyanoanilino]-2-oxoethyl]-3-oxomor pholin-4-yl]-2-oxo-3,4-dihydroquinolin-1-yl]acetate (compound aa21-5)

According to the Step 2-5 in synthetic method for EXAMPLE aa2, compound aa21-4 (1.90 g) was used instead of aa2-4 to obtain compound aa21-5 (2.44 g) as a white amorphous solid.

### [Step 21-6] Synthesis of methyl 2-[7-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpholi n-4-yl]-2-oxo-3,4-dihydroquinolin-1-yl]acetate (compound a21-6)

According to the Step 10-6 in synthetic method for EXAMPLE aa 10, compound aa21-5 (0.54 g) was used instead of aa10-5 to obtain compound aa21-6 (0.50 g) as a white amorphous solid.

### [Step 21-7] Synthesis of methyl 2-[7-[(2R)-2-[(1R)-2-[3-(aminomethyl)-4-cyanoanilino]-1-hydroxy-2-oxoethyl]-3-oxomorpholin-4-yl]-2-oxo-3,4-dihydroquinolin -1-yl]acetate hydrochloride (compound aa21-7)

According to the Step 2-7 in synthetic method for EXAMPLE aa2, compound aa21-6 (0.40 g) was used instead of aa2-6 to obtain compound aa21-7 (0.29 g) as a white amorphous solid.

### [Step 21-8] Synthesis of methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquin olin-1-yl]acetate hydrochloride (EXAMPLE aa21)

According to the Step 2-8 in synthetic method for EXAMPLE aa2, compound aa21-7 (0.28 g) was used instead of aa2-7 to obtain EXAMPLE aa21 (0.12 g) as a white amorphous solid.

### EXAMPLE aa22

### Synthesis of 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquinolin-1-yl]acetic acid hydrochloride (EXAMPLE aa22)

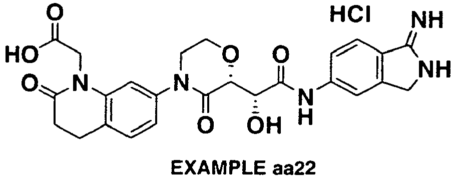

### [Step 22-1] Synthesis of 2-[7-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpholi n-4-yl]-2-oxo-3,4-dihydroquinolin-1-yl]acetic acid (compound aa22-1)

According to the Step 11-1 in synthetic method for EXAMPLE aa11, compound aa21-5 (1.20 g) was used instead of aa10-5 to obtain compound aa22-1 (0.99 g) as a pale yellow amorphous solid.

### [Step 22-2] Synthesis of 2-[7-[(2R)-2-[(1R)-2-[3-(aminomethyl)-4-cyanoanilino]-1-hydroxy-2-oxoethyl]-3-oxomorpholin-4-yl]-2-oxo-3,4-dihydroquinolin-1-yl]acetic acid hydrochloride (compound aa22-2)

According to the Step 2-7 in synthetic method for EXAMPLE aa2, compound aa22-1 (0.40 g) was used instead of aa2-6 to obtain compound aa22-2 (0.30 g) as a white amorphous solid.

### [Step 22-3] Synthesis of 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquin olin-1-yl]acetic acid hydrochloride (EXAMPLE aa22)

According to the Step 2-8 in synthetic method for EXAMPLE aa2, compound aa22-2 (0.29 g) was used instead of aa2-7 to obtain EXAMPLE aa22 (0.19 g) as a white amorphous solid.

### EXAMPLE aa23

### Synthesis of (2R)-N-(4-carbamimidoyl-3-fluorophenyl)-2-hydroxy-2-[(2R)-3-oxo-4-(2-oxo-3,4-dihydro-1H-quinolin-7-yl)morpholin-2-yl]acetam ide hydrochloride (EXAMPLE aa23)

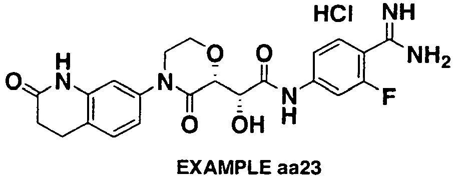

### [Step 23-1] Synthesis of [(1R)-2-[3-fluoro-4-(5-oxo-2H-1,2,4-oxadiazol-3-yl)anilino]-2-oxo-1-[(2R)-3-oxo-4-(2-oxo-1H-quinolin-7-yl)morpholin-2-yl]ethyl] acetate (compound aa23-1)

According to the Step 5-1 in synthetic method for EXAMPLE aa5, aa7-4 (0.20 g) was used instead of aa3-3 to obtain compound aa23-1 (0.12 g) as a beige amorphous solid.

### [Step 23-2] Synthesis of (2R)-N-[3-fluoro-4-(5-oxo-2H-1,2,4-oxadiazol-3-yl)phenyl]-2-hydroxy-2-[(2R)-3-oxo-4-(2-oxo-1H-quinolin-7-yl)morpholin-2-yl]aceta mide ammonium salt (compound aa23-2)

According to the Step 2-6 in synthetic method for EXAMPLE aa2, compound aa23-1 (0.18 g) was used instead of aa2-5 to obtain compound aa23-2 (0.12 g) as a beige amorphous solid.

### [Step 23-3] Synthesis of (2R)-N-(4-carbamimidoyl-3-fluorophenyl)-2-hydroxy-2-[(2R)-3-oxo-4-(2-oxo-3,4-dihydro-1H-quinolin-7-yl)morpholin-2-yl]acetamide hydrochloride (EXAMPLE aa23)

According to the Step 5-3 in synthetic method for EXAMPLE aa5, compound aa23-2 (0.12 g) was used instead of aa5-2 to obtain EXAMPLE aa23 (88 mg) as a beige amorphous solid.

### EXAMPLE aa24

### Synthesis of (2R)-N-(3-amino-1,2-benzisoxazol-6-yl)-2-hydroxy-2-[(2R)-3-oxo-4-(2-oxo-3,4-dihydro-1H-quinolin-7-yl)morpholin-2-yl]acetamide (EXAMPLE aa24)

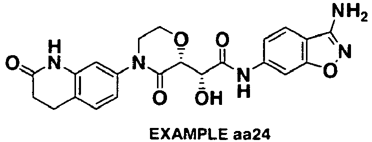

### [Step 24-1] Synthesis of [(1R)-2-[[3-(1,3-dioxoisoindol-2-yl)-1,2-benzisoxazol-6-yl]-amino]-2-oxo-1-[(2R)-3-oxo-4-(2-oxo-3,4-dihydro-1H-quinolin-7-yl)morpholin-2-yl]et hyl] acetate (compound aa24-1)

According to the Step 2-5 in synthetic method for EXAMPLE aa2, compound aa18-3 (0.50 g) and 6-amino-3-(1,3-dioxoisoindol-2-yl)-1,2-benzisoxazole (0.50 g) were used instead of aa2-4 and tert-butyl N-(2-cyano-5-aminophenyl)methyl-N-[(2-methylpropan-2-yl)oxycarbonyl]carbamate to obtain compound aa24-1 (0.40 g) as a pale yellow amorphous solid.

### [Step 24-2] Synthesis of (2R)-N-(3-amino-1,2-benzisoxazol-6-yl)-2-hydroxy-2-[(2R)-3-oxo-4-(2-oxo-3,4-dihydro-1H-quinolin-7-yl)morpholin-2-yl]acetamide (EXAMPLE aa24)

According to the Step 15-2 in synthetic method for EXAMPLE aa15, compound aa24-1 (0.35 g) was used instead of aa15-1 to obtain EXAMPLE aa24 (0.27 g) as a colorless amorphous solid.

### EXAMPLE aa25

### Synthesis of (2R)-N-(1-aminoisoquinolin-6-yl)-2-hydroxy-2-[(2R)-3-oxo-4-(2-oxo-3,4-dihydro-1H-quinolin-7-yl)morpholin-2-yl]acetamide hydrochloride (EXAMPLE aa25)

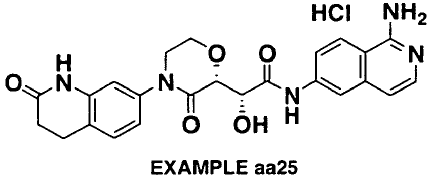

### [Step 25-1] Synthesis of [(1R)-2-[[1-[bis[(2-methylpropan-2-yl)oxycarbonyl]-amino]isoquinolin-6-yl]aminol-2-oxo-1-[(2R)-3-oxo-4-(2-oxo-3,4-dihydro-1H-quinolin -7-yl)morpholin-2-yl]ethyl] acetate (compound aa25-1)

According to the Step 2-5 in synthetic method for EXAMPLE aa2, compound aa18-3 (0.50 g), 6-amino-1-bis(tert-butoxycarbonyl)aminoisoquinoline (0.5 g), (1-cyano-2-ethoxy-2-oxoethylidenaminooxy)dimethylamino-morpholino-carbenium hexafluorophosphate (COMU: 1.18 g) and diisopropylethylamine (0.96 mL) were used instead of 2-4, N-[(5-Amino-2-cyanophenyl)methyl]-N-[(2-methylpropan-2-yl)oxycarbonyl]carbamate, WSC-HCl, and DMAP to obtain compound aa25-1 (0.49 g) as a brown amorphous solid.

### [Step 25-2] Synthesis of tert-butyl N-[6-[[(2R)-2-hydroxy-2-[(2R)-3-oxo-4-(2-oxo-3,4-dihydro-1H-quinolin-7-yl)morpholin-2-yl]acetyl]amino]isoquinolin-1-yl]-N-[(2-methyl propan-2-yl)oxycarbonyl]carbamate (compound aa25-2)

According to the Step 2-6 in synthetic method for EXAMPLE aa2, compound aa25-1 (0.47 g) was used instead of aa2-5 to obtain compound aa25-2 (0.43 g) as a pale yellow amorphous solid.

### [Step 25-3] Synthesis of (2R)-N-(1-aminoisoquinolin-6-yl)-2-hydroxy-2-[(2R)-3-oxo-4-(2-oxo-3,4-dihydro-1H-quinolin-7-yl)morpholin-2-yl]acetamide hydrochloride (EXAMPLE aa25)

According to the Step 2-7 in synthetic method for EXAMPLE aa2, compound aa25-2 (0.41 g) was used instead of aa2-6 to obtain EXAMPLE aa25 (0.25 g) as a pale brown amorphous solid.

### EXAMPLE aa26

### Synthesis of (2R)-N-(4-aminoquinazolin-7-yl)-2-hydroxy-2-[(2R)-3-oxo-4-(2-oxo-3,4-dihydro-1H-quinolin-7-yl)morpholin-2-yl]acetamide (EXAMPLE aa26)

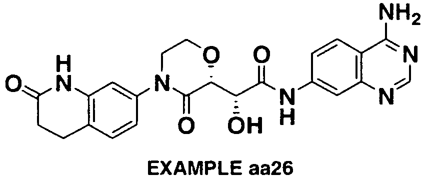

### [Step 26-1] Synthesis of [(1R)-2-[[4-[(2-methylpropan-2-yl)oxycarbonylaminol-quinazolin-7-yl]amino]-2-oxo-1-[(2R)-3-oxo-4-(2-oxo-3,4-dihydro-1H-quinolin-7-yl)m orpholin-2-yl]ethyl] acetate (compound aa26-1)

According to the Step 2-5 in synthetic method for EXAMPLE aa2, compound aa18-3 (0.50 g), 7-amino-4-(tert-butoxycarbonyl)aminoquinazoline (0.36 g), O-(7-azabenzotriazol-1-yl)-N,N,N',N'-tetramethyluronium hexafluorophosphate (HATU: 1.05 g) and diisopropylethylamine (0.96 mL) were used instead of 2-4, N-[(5-amino-2-cyanophenyl)methyl]-N-[(2-methylpropan-2-yl)oxycarbonyl]carbamate, WSC-HCl, and DMAP to obtain compound aa26-1 (0.21 g) as a pale yellow amorphous solid.

### [Step 26-2] Synthesis of tert-butyl N-[7-[[(2R)-2-hydroxy-2-[(2R)-3-oxo-4-(2-oxo-3,4-dihydro-1H-quinolin-7-yl)morpholin-2-yl]acetyl]amino]quinazolin-4-yl]carbamate (compound aa26-2)

According to the Step 2-6 in synthetic method for EXAMPLE aa2, compound aa26-1 (0.21 g) was used instead of aa2-5 to obtain compound aa26-2 (0.19 g) as a white amorphous solid.

### [Step 26-3] Synthesis of (2R)-N-(4-aminoquinazolin-7-yl)-2-hydroxy-2-[(2R)-3-oxo-4-(2-oxo-3,4-dihydro-1H-quinolin-7-yl)morpholin-2-yl]acetamide (EXAMPLE aa26)

According to the Step 2-7 in synthetic method for EXAMPLE aa2. compound aa26-2 (0.18 g) was used instead of aa2-6 to obtain EXAMPLE aa26 (20 mg) as a pale brown amorphous solid.

### EXAMPLE aa27

### Synthesis of (2R)-2-hydroxy-N-(1-imino-2,3-dihydroisoindol-5-yl)-2-[(2R)-3-oxo-4-(2-oxo-3,4-dih ydro-1H-quinolin-5-yl)morpholin-2-yl]acetamide hydrochloride (EXAMPLE aa27)

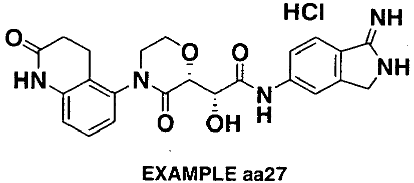

### [Step 27-1] Synthesis of tert-butyl (2R)-2-hydroxy-2-[3-oxo-4-(2-oxo-3,4-dihydro-1H-quinolin-5-yl)morpholin-2-yl]acetate (compound aa27-1)

According to the Step 2-2 in synthetic method for EXAMPLE aa2, 5-bromo-3,4-dihydroquinolin-2(1H)-one (2.0 g) was used instead of aa2-1 to obtain compound aa27-1 (0.34 g) as a white amorphous solid (contained diastereomer).

### [Step 27-2] Synthesis of tert-butyl (2R)-2-acetyloxy-2-[3-oxo-4-(2-oxo-3,4-dihydro-1H-quinolin-5-yl)morpholin-2-yl]acetate (compound aa27-2)

According to the Step 2-3 in synthetic method for EXAMPLE aa2, compound aa27-1 (0.30 g) was used instead of aa2-2 to obtain compound aa27-2 (0.32 g) as a white amorphous solid (contained diastereomer).

### [Step 27-3] Synthesis of (2R)-2-acetyloxy-2-[3-oxo-4-(2-oxo-3.4-dihydro-1H-quinolin-5-yl)morpholin-2-yl]acetic acid (compound aa27-3)

According to the Step 2-4 in synthetic method for EXAMPLE aa2, compound aa27-2 (0.30 g) was used instead of aa2-3 to obtain compound aa27-3 (0.24 g) as a white amorphous solid (contained diastereomer).

### [Step 27-4] Synthesis of tert-butyl N-[[2-cyano-5-[[(2R)-2-hydroxy-2-[(2R)-3-oxo-4-(2-oxo-3,4-dihydro-1H-quinolin-5-yl)morpholin-2-yl]acetyl]amino]phenyl]methyl]-N-[(2-methylpropan-2-yl)oxycarbonyl]carbamate (compound aa27-4)

According to the Step 2-5 and 2-6 in synthetic method for EXAMPLE aa2, compound aa27-3 (0.24 g) was used instead of aa2-4 to obtain compound aa27-4 (38 mg) as a white amorphous solid.

### [Step 27-5] Synthesis of (2R)-N-[3-(aminomethyl)-4-cyanophenyl]-2-hydroxy-2-[(2R)-3-oxo-4-(2-oxo-3,4-dihydro-1H-quinolin-5-yl)morpholin-2-yl]acetamide hydrochloride (compound aa27-5)

According to the Step 2-7 in synthetic method for EXAMPLE aa2, compound aa27-4 (36 mg) was used instead of aa2-6 to obtain compound aa27-5 (33 mg) as a white amorphous solid.

### [Step 27-6] Synthesis of (2R)-2-hydroxy-N-(1-imino-2,3-dihydroisoindol-5-yl)-2-[(2R)-3-oxo-4-(2-oxo-3,4-dihydro-1H-quinolin-5-yl)morpholin-2-yl]acetamide hydrochloride (EXAMPLE aa27)

According to the Step 2-8 in synthetic method for EXAMPLE aa2, compound aa27-5 (30 mg) was used instead of aa2-7 to obtain EXAMPLE aa27 (22 mg) as a white amorphous solid.

### EXAMPLE aa28

### Synthesis of (2R)-2-hydroxy-2-[(2R)-4-[2-(3-hydroxy-3-methylbutoxy)phenyl]-3-oxomorpholin-2-yl]-N-(1-imino-2,3-dihydroisoindol-5-yl)acet amide hydrochloride (EXAMPLE aa28)

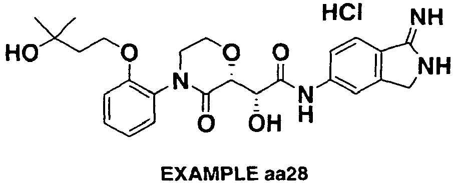

### [Step 28-1] Synthesis of 4-(2-iodophenoxy)-2-methylbutan-2-ol (compound aa28-1)

A mixture of 2-iodophenol (6 g), 3-hydroxy-3-methylbutyl tosylate (7.4 g) and Cs₂CO₃ (13.3 g) in DMF (120 mL) was stirred at 0 °C for 2 h and at room temperature for 10 h. The mixture was diluted with EtOAc and washed with 1N HCl. saturated NaHCO₃ aq., H₂O, and brine, and dried over anhydrous Na₂SO₄. The solvent was removed under reduced pressure. The resulting residue was purified by silica gel column chromatography (Hexane/EtOAc = 7/3) to obtain compound aa28-1 (8.02 g) as pale yellow oil.

### [Step 28-2] Synthesis of tert-butyl (2R)-2-hydroxy-2-[(2R)-4-[2-(3-hydroxy-3-methylbutoxy)phenyl]-3-oxomorpholin-2-yl]acetate (compound aa28-2)

According to the Step 2-2 in synthetic method for EXAMPLE aa2, compound aa28-1 (2.20 g) was used instead of aa2-1 to obtain compound aa28-2 (1.69 g) as a pale brown amorphous solid.

### [Step 28-3] Synthesis of tert-butyl (2R)-2-acetyloxy-2-[(2R)-4-[2-(3-hydroxy-3-methylbutoxy)phenyl]-3-oxomorpholin-2-yl]acetate (compound aa28-3)

According to the Step 2-3 in synthetic method for EXAMPLE aa2, compound aa28-2 (1.60 g) was used instead of aa2-2 to obtain compound aa28-3 (1.72 g) as a white amorphous solid.

### [Step 28-4] Synthesis of (3R)-2-acetyloxy-2-[(2R)-4-[2-(3-hydroxy-3-methylbutoxy)phenyl]-3-oxomorpholin-2-yl]acetic acid (compound aa28-4)

According to the Step 2-4 in synthetic method for EXAMPLE aa2, compound aa28-3 (0.85 g) was used instead of aa2-3 to obtain compound aa28-4 (0.85 g) as a white amorphous solid.

### [Step 28-5] Synthesis of [(1R)-2-[3-[[bis[(2-methylpropan-2-yl)oxycarbonyl]-amino]methyl]-4-cyanoanilino]-1-[(2R)-4-[2-(3-hydroxy-3-methylbutoxy)phenyl]-3-ox omorpholin-2-yl]-2-oxoethyl] acetate (compound aa28-5)

According to the Step 2-5 in synthetic method for EXAMPLE aa2, compound aa28-4 (0.40 g) was used instead of aa2-4 to obtain compound aa28-5 (0.33 g) as a white amorphous solid.

### [Step 28-6] Synthesis of tert-butyl N-[[2-cyano-5-[[(2R)-2-hydroxy-2-[(2R)-4-[2-(3-hydroxy-3-methylbutoxy)phenyl]-3-oxomorpholin-2-yl]acetyl]amino]phenyl]methyl ]-N-[(2-methylpropan-2-yl)oxycarbonyl]carbamate (compound aa28-6)

According to the Step 2-6 in synthetic method for EXAMPLE aa2, compound aa28-5 (0.30 g) was used instead of aa2-5 to obtain compound aa28-6 (87 mg) as a white amorphous solid.

### [Step 28-7] Synthesis of (2R)-N-[3-(aminomethyl)-4-cyanophenyl[-2-hydroxy-2-[(2R)-4-[2-(3-hydroxy-3-methylbutoxy)phenyl]-3-oxomorpholin-2-yl]acetamide hydrochloride (compound aa28-7)

According to the Step 2-7 in synthetic method for EXAMPLE aa2, compound aa28-6 (83 mg) was used instead of aa2-6 to obtain compound aa28-7 (63 mg) as a white amorphous solid.

### [Step 28-8] Synthesis of (2R)-2-hydroxy-2-[(2R)-4-[2-(3-hydroxy-3-methylbutoxy)phenyl]-3-oxomorpholin-2-yl]-N-(1-imino-2,3-dihydroisoindol-5-yl)acet amide hydrochloride (EXAMPLE aa28)

According to the Step 2-8 in synthetic method for EXAMPLE aa2, compound aa28-7 (61 mg) was used instead of aa2-7 to obtain EXAMPLE aa28 (24 mg) as a white amorphous solid.

### EXAMPLE aa29

### Synthesis of (2R)-2-hydroxy-N-(1-imino-2,3-dihydroisoindol-5-yl)-2-[(2R)-4-[2-(3-methoxy-3-methylbutoxy)phenyl]-3-oxomorpholin-2-yl]acetamide hydrochloride (EXAMPLE aa29)

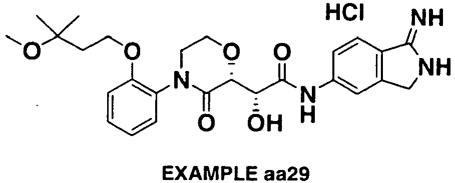

### [Step 29-1] Synthesis of 1-iodo-2-(3-methoxy-3-methylbutoxy)benzene (compound aa29-1)

According to the Step 28-1 in synthetic method for EXAMPLE aa28, 3-methoxy-3-methylbutyl p-toluenesulfonate (6.50 g) was used instead of 3-hydroxy-3-methylbutyl p-toluenesulfonate to obtain compound aa29-1 (5.77 g) as colorless oil.

### I Step 29-2] Synthesis of tert-butyl (2R)-2-hydroxy-2-[(2R)-4-[2-(3-methoxy-3-methylbutoxy)phenyl]-3-oxomorpholin-2-yl]acetate (compound aa29-2)

According to the Step 2-2 in synthetic method for EXAMPLE aa2, compound aa29-1 (4.00 g) was used instead of aa2-1 to obtain compound aa29-2 (2.43 g) as pale yellow oil.

### [Step 29-3] Synthesis of tert-butyl (2R)-2-acetyloxy-2-[(2R)-4-[2-(3-methoxy-3-methylbutoxy)phenyl]-3-oxomorpholin-2-yl]acetate (compound aa29-3)

According to the Step 2-3 in synthetic method for EXAMPLE aa2, compound aa29-2 (2.42 g) was used instead of aa2-2 to obtain compound aa29-3 (2.61 g) as a white amorphous solid.

### [Step 29-4] Synthesis of (2R)-2-acetyloxy-2-[(2R)-4-[2-(3-methoxy-3-methylbutoxy)phenyl]-3-oxomorpholin-2-yl]acetic acid (compound aa29-4)

According to the Step 2-4 in synthetic method for EXAMPLE aa2, compound aa29-3 (2.50 g) was used instead of aa2-3 to obtain compound aa29-4 (2.78 g) as pale brown oil.

### [Step 29-5] Synthesis of [(1R)-2-[3-[[bis[(2-methylpropan-2-yl)oxycarbonyl] amino]methyl]-4-cyanoanilino]-1-[(2R)-4-[2-(3-methoxy-3-methylbutoxy)phenyl]-3-o xomorpholin-2-yl]-2-oxoethyl] acetate (compound aa29-5)

According to the Step 2-5 in synthetic method for EXAMPLE aa2, compound aa29-4 (1.00 g) was used instead of aa2-4 to obtain compound aa29-5 (0.96 g) as a white amorphous solid.

### [Step 29-6] Synthesis of tert-butyl N-[[2-cyano-5-[[(2R)-2-hydroxy-2-[(2R)-4-[2-(3-methoxy-3-methylbutoxy)phenyl]-3-oxomorpholin-2-yl]acetyl]amino]phenyl]met hyl]-N-[(2-methylpropan-2-yl)oxycarbonyl]carbamate (compound aa29-6)

According to the Step 2-6 in synthetic method for EXAMPLE aa2, compound aa29-5 (0.70 g) was used instead of aa2-5 to obtain compound aa29-6 (0.64 g) as a white amorphous solid.

### [Step 29-7] Synthesis of (2R)-N-[3-(aminomethyl)-4-cyanophenyl]-2-hydroxy-2-[(2R)-4-[2-(3-methoxy-3-methylbutoxy)phenyl]-3-oxomorpholin-2-yl]acetamide hydrochloride (compound aa29-7)

According to the Step 2-7 in synthetic method for EXAMPLE aa2, compound aa29-6 (0.62 g) was used instead of aa2-6 to obtain compound aa29-7 (0.45 g) as a cream amorphous solid.

### [Step 29-8] Synthesis of (2R)-2-hydroxy-N-(1-imino-2,3-dihydroisoindol-5-yl)-2-[(2R)-4-[2-(3-methoxy-3-methylbutoxy)phenyl]-3-oxomorpholin-2-yl]acetamide hydrochloride (EXAMPLE aa29)

According to the Step 2-8 in synthetic method for EXAMPLE aa2, compound aa29-7 (0.25 g) was used instead of aa2-7 to obtain EXAMPLE aa29 (0.21 g) as a white amorphous solid.

### EXAMPLE aa30

### Synthesis of (2R)-2-hydroxy-N-(1-imino-2,3-dihydroisoindol-5-yl)-2-[(2R)-3-oxo-4-[2-(2-oxopiperidin-1-yl)phenyl]morpholin-2-yl]acetamide hydrochloride (EXAMPLE aa30)

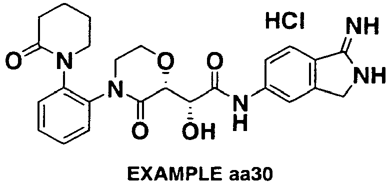

### [Step 30-1] Synthesis of tert-butyl (2R)-2-hydroxy-2-[(2R)-3-oxo-4-[2-(2-oxopiperidin-1-yl)phenyl]morpholin-2-yl]acetate (compound aa30-1)

According to the Step 2-2 in synthetic method for EXAMPLE aa2, 1-(2-iodophenyl)-2-piperidinone (2.73 g) was used instead of aa2-1 to obtain compound aa30-1 (0.85 g) as a brown amorphous solid.

### [Step 30-2] Synthesis of tert-butyl (2R)-2-acetyloxy-2-[(2R)-3-oxo-4-[2-(2-oxopiperidin-1-yl)phenyl]morpholin-2-yl]acetate (compound aa30-2)

According to the Step 2-3 in synthetic method for EXAMPLE aa2, compound aa30-1 (0.85 g) was used instead of aa2-2 to obtain compound aa30-2 (0.80 g) as a pale yellow amorphous solid.

### [Step 30-3] Synthesis of (2R)-2-acetyloxy-2-[(2R)-3-oxo-4-[2-(2-oxopiperidin-1-yl)phenyl]morpholin-2-yl]acetic acid (compound aa30-3)

According to the Step 2-4 in synthetic method for EXAMPLE aa2, compound aa30-2 (0.79 g) was used instead of aa2-3 to obtain compound aa30-3 (0.58 g) as a colorless amorphous solid.

### [Step 30-4] Synthesis of [(1R)-2-[3-[[bis[(2-methylpropan-2-yl)oxycarbonyl]-amino]methyl]-4-cyanoanilino]-2-oxo-1-[(2R)-3-oxo-4-[2-(2-oxopiperidin-1-yl)phenyl ]morpholin-2-yl]ethyl] acetate (compound aa30-4)

According to the Step 2-5 in synthetic method for EXAMPLE aa2, compound aa30-3 (0.30 g) was used instead of aa2-4 to obtain compound aa30-4 (0.16 g) as a pale yellow amorphous solid.

### [Step 30-5] Synthesis of tert-butyl N-[[2-cyano-5-[[(2R)-2-hydroxy-2-[(2R)-3-oxo-4-[2-(2-oxopiperidin-1-yl)phenyl]morpholin-2-yl]acetyl]amino]phenyl]methyl]-N-[(2-me thylpropan-2-yl)oxycarbonyl]carbamate (compound aa30-5)

According to the Step 2-6 in synthetic method for EXAMPLE aa2, compound aa30-4 (0.15 g) was used instead of aa2-5 to obtain compound aa30-5 (0.14 g) as pale yellow oil.

### [Step 30-6] Synthesis of (2R)-N-[3-(aminomethyl)-4-cyanophenyl]-2-hydroxy-2-[(2R)-3-oxo-4-[2-(2-oxopiperidin-1-yl)phenyl]morpholin-2-yl]acetamide hydrochloride (compound aa30-6)

According to the Step 2-7 in synthetic method for EXAMPLE aa2, compound aa30-5 (0.14 g) was used instead of aa2-6 to obtain compound aa30-6 (78 mg) as a colorless amorphous solid.

### [Step 30-7] Synthesis of (2R)-2-hydroxy-N-(1-imino-2,3-dihydroisoindol-5-yl)-2-[(2R)-3-oxo-4-[2-(2-oxopiperidin-1-yl)phenyl[morpholin-2-yl[acetamide hydrochloride (EXAMPLE aa30)

According to the Step 2-8 in synthetic method for EXAMPLE aa2, compound aa30-6 (78 mg) was used instead of aa2-7 to obtain EXAMPLE aa30 (72 mg) as a colorless amorphous solid.

### EXAMPLE aa31

### Synthesis of (2R)-2-[(2R)-4-(2-(difluoromethoxy)-5-fluorophenyl]-3-oxomorpholin-2-yl ]-2-hydroxy-N-(1-imino-2,3-dihydroisoindol-5-yl)acetamide hydrochloride (EXAMPLE aa31)

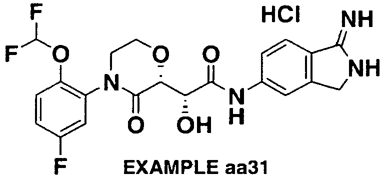

### [Step 31-1] Synthesis of 2-bromo-1-(difluoromethoxy)-4-fluorobenzene (compound aa31-1)

According to the Step 4-1 in synthetic method for EXAMPLE aa4, 2-bromo-4-fluorophenol (5.0 g) was used instead of 2-iodo-5-nitrophenol to obtain compound aa31-1 (5.25 g) as a red amorphous solid.

### [Step 31-2] Synthesis of tert-butyl (2R)-2-[(2R)-4-[2-(difluoromethoxy)-5-f7uorophenyl]-3-oxomorpholin-2-yl]--2-hydroxyacetate (compound aa31-2)

According to the Step 2-2 in synthetic method for EXAMPLE aa2, compound aa31-1 (2.08 g) was used instead of aa2-1 to obtain compound aa31-2 (0.75 g) as pale yellow oil.

### [Step 31-3] Synthesis of tert-butyl (2R)-2-acetyloxy-2-[(2R)-4-[2-(difluoromethoxy)-5-fluorophenyl]-3-oxomorpholin-2-yl]acetate (compound aa31-3)

According to the Step 2-3 in synthetic method for EXAMPLE aa2, compound aa31-2 (0.74 g) was used instead of aa2-2 to obtain compound aa31-3 (0.82 g) as pale yellow oil.

### [Step 31-4] Synthesis of (2R)-2-acetyloxy-2-[(2R)-4-[2-(difluoromethoxy)-5-fluorophenyl]-3-oxomorpholin-2-yl]acetic acid (compound aa31-4)

According to the Step 2-4 in synthetic method for EXAMPLE aa2, compound aa31-3 (0.80 g) was used instead of aa2-3 to obtain compound aa31-4 (0.56 g) as a colorless amorphous solid.

### [Step 31-5] Synthesis of [(1R)-2-[3-[[bis[(2-methylpropan-2-yl)oxycarbonyl]-amino]methyl]-4-cyanoanilino]-1-[(2R)-4-[2-(difluoromethoxy)-5-fluorophenyl]-3-oxo morpholin-2-yl]-2-oxoethyl] acetate (compound aa31-5)

According to the Step 2-5 in synthetic method for EXAMPLE aa2, compound aa31-4 (0.20 g) was used instead of aa2-4 to obtain compound aa31-5 (0.19 g) as a colorless amorphous solid.

### [Step 31-6] Synthesis of tert-butyl N-[[2-cyano-5-[[(2R)-2-[(2R)-4-[2-(difluoromethoxy)-5-fluorophenyl]-3-oxomorpholin-2-yl]-2-hydroxyacetyl]amino]phen yl]methyl]-N-[(2-methylpropan-2-yl)oxycarbonyl]carbamate (compound aa31-6)

According to the Step 2-6 in synthetic method for EXAMPLE aa2, compound aa31-5 (0.18 g) was used instead of aa2-5 to obtain compound aa31-6 (0.11 g) as colorless oil.

### [Step 31-7] Synthesis of (2R)-N-[3-(aminomethyl)-4-cyanophenyl]-2-[(2R)-4-[2-(difluoromethoxy)-5-fluorophenyl]-3-oxomorpholin-2-yl]-1-hydroxyacetam ide hydrochloride (compound aa31-7)

According to the Step 2-7 in synthetic method for EXAMPLE a2, compound aa31-6 (95 mg) was used instead of aa2-6 to obtain compound aa31-7 (70 mg) as a pale yellow amorphous solid.

### [Step 31-8] Synthesis of (2R)-2-[(2R)-4-[2-(difluoromethoxy)-5-fluorophenyl]-3-oxomorpholin-2-yl]-2-hydroxy-N-(1-imino-2,3-dihydroisoindol-5-yl)acetamide hydrochloride (EXAMPLE aa31)

According to the Step 2-8 in synthetic method for EXAMPLE aa2. compound aa31-7 (70 mg) was used instead of aa2-7 to obtain EXAMPLE aa31 (57 mg) as a pale yellow amorphous solid.

### EXAMPLE aa32

### Synthesis of methyl 2-(dif]uoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoate hydrochloride (EXAMPLE aa32)

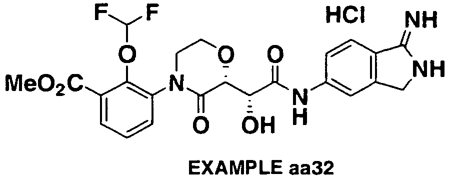

### [Step 32-1] Synthesis of methyl 2-(difluoromethoxy)-3-nitrobenzoate (compound aa32-1)

According to the Step 4-1 in synthetic method for EXAMPLE aa4, 2-hydroxy-3-nitrobenzoic acid methyl ester (118.3g) was used instead of 2-iodo-5-nitrophenol to obtain compound aa32-1 (3.70 g) as a colorless amorphous solid.

### [Step 32-2] Synthesis of methyl 3-amino-2-(difluoromethoxy)benzoate (compound aa32-2)

According to the Step 5-3 in synthetic method for EXAMPLE aa5, compound aa32-1 (3.75 g) was used instead of aa5-2 to obtain compound aa32-2 (3.17 g) as colorless amorphous solid.

### [Step 32-3] Synthesis of methyl 2-(difluoromethoxy)-3-iodobenzoate (compound aa32-3)

According to the Step 16-1 in synthetic method for EXAMPLE aa16, compound aa32-2 (2.87 g) was used instead of 5-Aminoquinolin-2(H)-one to obtain compound aa32-3 (3.99 g) as a yellow amorphous solid.

### [Step 32-4] Synthesis of methyl 2-(difluoromethoxy)-3-[(2R)-2-[(1R)-1-hydroxy-2-[(2-methylpropan-2-yl)oxy]-2-oxoethyl]-3-oxomorpholin-4-yl]benzoate (compound aa32-4)

According to the Step 2-2 in synthetic method for EXAMPLE aa2, compound aa32-3 (3.98 g) was used instead of aa2-1 to obtain compound aa32-4 (1.21 g) as a yellow amorphous solid.

### [Step 32-5] Synthesis of methyl 3-[(2R)-2-[(1R)-1-acetyloxy-2-[(2-methylpropan-2-yl)oxy]-2-oxoethyl[-3-oxomorpholin-4-yl]-2-(difluoromethoxy)benzoate (compound aa32-5)

According to the Step 2-3 in synthetic method for EXAMPLE aa2, compound aa32-4 (1.20 g) was used instead of aa2-2 to obtain compound aa32-5 (1.32 g) as a yellow amorphous solid.

### [Step 32-6] Synthesis of (2R)-2-acetyloxy-2-[(2R)-4-(2-(difluoromethoxy)-3-methoxycarbonylphenyl]-3-oxomorpholin-2-yl lacetic acid (compound aa32-6)

According to the Step 2-4 in synthetic method for EXAMPLE aa2, compound aa32-5 (1.32 g) was used instead of aa2-3 to obtain compound aa32-6 (1.16 g) as a pink amorphous solid.

### [Step 32-7] Synthesis of methyl 3-[(2R)-2-[(1R)-1-acetyloxy-2-[3-[[bis[(2-methylpropan-2-yl)oxycarbonyl]amino]methyl]-4-cyanoanilino]-2-oxoethyl]-3-oxomor pholin-4-yl]-2-(difluoromethoxy)benzoate (compound aa32-7)

According to the Step 2-5 in synthetic method for EXAMPLE aa2, compound aa32-6 (0.93 g) was used instead of aa2-4 to obtain compound aa32-7 (0.60 g) as a colorless amorphous solid.

### [Step 32-8] Synthesis of methyl 3-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpho lin-4-yl]-2-(difluoromethoxy)benzoate (compound aa32-8)

According to the Step 10-6 in synthetic method for EXAMPLE aa10, compound aa32-7 (0.12 g) was used instead of aa10-5 to obtain compound aa32-8 (0.11 g) as a colorless amorphous solid.

### [Step 32-9] Synthesis of methyl 3-[(2R)-2-[(1R)-2-[3-(aminomethyl)-4-cyanoanilino]-1-hydroxy-2-oxoethyl]-3-oxomorpholin-4-yl]-2-(difluoromethoxy)benzoate hydrochloride (compound aa32-9)

According to the Step 2-7 in synthetic method for EXAMPLE aa2, compound aa32-8 (0.10 g) was used instead of aa2-6 to obtain compound aa32-9 (70 mg) as a yellow amorphous solid.

### [Step 32-10] Synthesis of methyl 2-(difluoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoate hydrochloride (EXAMPLE aa32)

According to the Step 2-8 in synthetic method for EXAMPLE aa2, compound aa32-9 (70 mg) was used instead of aa2-7 to obtain EXAMPLE aa32 (57 mg) as a yellow amorphous solid.

### EXAMPLE aa33

### Synthesis of 2-(difluoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoic acid hydrochloride (EXAMPLE aa33)

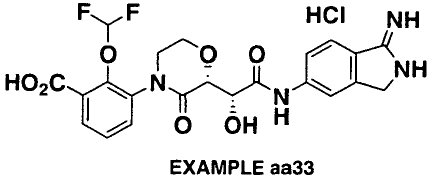

### [Step 33-1] Synthesis of 3-[(2R)-2-[(R)-2-[3-[[bis[(2-methylpropan-2-yl)oxycarbonyl]amino]methyl]-4-cyanoanilino]-1-hydroxy-2-oxoethyl]-3-oxomorpholi n-4-yl]-2-(difluoromethoxy)benzoic acid (compound aa33-1)

According to the Step 11-1 in synthetic method for EXAMPLE aa11, compound aa32-7 (0.30 g) was used instead of aa10-5 to obtain compound aa33-1 (0.26 g) as a colorless amorphous solid.

### [Step 33-2] Synthesis of 3-[(2R)-2-[(1R)-2-[3-(aminomethyl)-4-cyanoanilino]-1-hydroxy-2-oxoethyl]-3-oxomorpholin-4-yl]-2-(difluoromethoxy)benzoic acid hydrochloride (compound aa33-2)

According to the Step 2-7 in synthetic method for EXAMPLE aa2, compound aa33-1 (0.10 g) was used instead of aa2-6 to obtain compound aa33-2 (76 mg) as a colorless amorphous solid.

### [Step 33-3] Synthesis of 2-(difluoromethoxy)-3-[(2R)-2-[(1R)-1-hydroxy-2-[(1-imino-2,3-dihydroisoindol-5-yl)aminol-2-oxoethyl]-3-oxomorpholin-4-yl]benzoic acid hydrochloride (EXAMPLE aa33)

According to the Step 2-8 in synthetic method for EXAMPLE aa2, compound aa33-2 (76 mg) was used instead of aa2-7 to obtain EXAMPLE aa33 (60 mg) as a colorless amorphous solid.

### EXAMPLE aa34

### Synthesis of 2-(difluoromethoxy)-3-[(2R)-2-[(R)-1-hydroxy-2-[(1-imino-2,3-dihydroisoindol-5-yl)amino]-2-oxoethyl]-3-oxomorpholin-4-yl]-N,N-di methylbenzamide hydrochloride (EXAMPLE aa34)

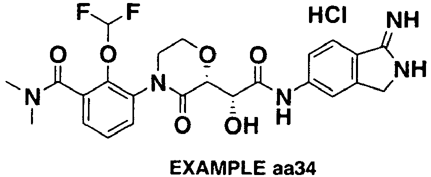

### [Step 34-1] Synthesis of tert-butyl N-[[2-cyano-5-[[(2R)-2-[(2R)-4-[2-(difluoromethoxy)-3-(dimethylcarbamoyl)phenyl]-3-oxomorpholin-2-yl]-2-hydroxyace tyl]amino]phenyl]methyl]-N-[(2-methylpropan-2-yl)oxycarbonyl]carbamate (compound aa34-1)

According to the Step 2-5 in synthetic method for EXAMPLE aa2, compound aa33-1 (0.12 g), dimethylamine (2M in THF, 174 µl), and HOBt (3 mg) were used instead of 2-4, tert-butyl N-(5-amino-2-cyanophenyl)methyl-N-[(2-methylpropan-2-yl)oxycarbonyl]carbamate, and DMAP to obtain compound aa34-1 (24 mg) as a colorless amorphous solid.

### [Step 34-2] Synthesis of 3-[(2R)-2-[(1R)-2-[3-(aminomethyl)-4-cyanoanilino]-1-hydroxy-2-oxoethyl]-3-oxomorpholin-4-yl]-2-(difluoromethoxy)-N,N-dimethylbenza mide hydrochloride (compound aa34-2)

According to the Step 2-7 in synthetic method for EXAMPLE aa2, compound aa34-1 (24 mg) was used instead of aa2-6 to obtain compound aa34-2 (18 mg) as a colorless amorphous solid.

### [Step 34-3] Synthesis of 2-(difluoromethoxy)-3-[(2R)-2-[(1R)-1-hydroxy-2-[(1-imino-2,3-dihydroisoindol-5-yl)amino]-2-oxoethyl]-3-oxomorpholin-4-yl]-N,N-di methylbenzamide hydrochloride (EXAMPLE aa34)

According to the Step 2-8 in synthetic method for EXAMPLE aa2, compound aa34-2 (18 mg) was used instead of aa2-7 to obtain EXAMPLE aa34 (15 mg) as a colorless amorphous solid.

### EXAMPLE aa35

### Synthesis of 2-(difluoromethoxy)-3-[2-[(1-hydroxy-2-[(1-amino-2,3-dihydroisoindol-5-yl)amino]-2-oxoethyl]-3-oxomorpholin-4-yl]benzamide hydrochloride (EXAMPLE aa35)

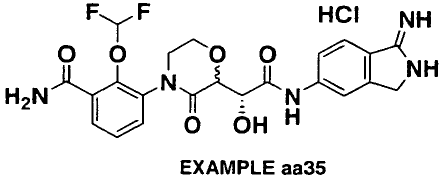

### [Step 35-1] Synthesis of tert-butyl N-[[5-[[2-[4-[3-carbamoyl-2-(difluoromethoxy)phenyl]-3-oxomorpholin-3-yl]-2-hydroxyacetyl]amino]-2-cyanophen yl]methyl]-N-[(2-methylpropan-2-yl]oxycarbonyl]carbamate (compound aa35-1)

According to the Step 2-6 in synthetic method for EXAMPLE aa2, compound aa32-7 (0.12 g) was used instead of aa2-5 to obtain compound aa35-1 (40 mg) as a colorless amorphous solid (diastereomer mixture).

### [Step 35-2] Synthesis of 3-[2-[2-[3-(aminomethyl)-4-cyanoanilino]-1-hydroxy-2-oxoethyl]-3-oxomorpholin-4-yl]-2-(difluoromethoxy)benzamide hydrochloride (compound aha35-2)

According to the Step 2-7 in synthetic method for EXAMPLE aa2, compound aa35-1 (39 mg) was used instead of aha2-6 to obtain compound aa35-2 (30 mg) as a colorless amorphous solid (diastereomer mixture).

### [Step 35-3] Synthesis of 2-(difluoromethoxy)-3-[2-[(1R)-1-hydroxy-2-[(1-imino-2,3-dihydroisoindol-5-yl)amino]-2-oxoethyl]-3-oxomorphol in-4-yl]benzamide hydrochloride (EXAMPLE aa35)

According to the Step 2-8 in synthetic method for EXAMPLE aa2, compound aa35-2 (54 mg) was used instead of aa2-7 to obtain EXAMPLE aa35 (49 mg) as a pale yellow amorphous solid (diastereomer mixture).

### EXAMPLE aa36

### Synthesis of methyl 2-[(2R)-2-[(1R)-1-hydroxy-2-[(1-imino-2,3-dihydroisoindol-5-yl)amino]-2-oxoethyl]-3-oxomorpholin-4-yl]-5-(trifluoromethoxy)be nzoate hydrochloride (EXAMPLE aa36)

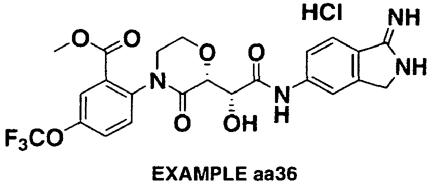

### [Step 36-1] Synthesis of methyl 2-iodo-5-(trifluoromethoxy)benzoate (compound aa36-1)

According to the Step 16-1 in synthetic method for EXAMPLE aa16, methyl 2-amino-5-trifluoromethoxybenzoate (4.0 g) was used instead of 5-Aminoquinolin-2(1H)-one to obtain compound aa36-1 (4.73 g) as pale yellow oil.

### [Step 36-2] Synthesis of methyl 2-[(2R)-2-[(1R)-1-hydroxy-2-[(2-methylpropan-2-yl)oxy]-2-oxoethyl]-3-oxomorpholin-4-yl)-5-(trifluoromethoxy)benzoate (compound aa36-2)

According to the Step 2-2 in synthetic method for EXAMPLE aa2, compound aa36-1 (2.35 g) was used instead of aa2-1 to obtain compound aa36-2 (0.74 g) as a white amorphous solid.

### [Step 36-3] Synthesis of methyl 2-[(2R)-2-[(1R)-1-acetyloxy-2-[(2-methylpropan-2-yl)oxy]-2-oxoethyl]-3-oxomorpholin-4-yl]-5-(trifluoromethoxy)benzoate (compound aa36-3)

According to the Step 2-3 in synthetic method for EXAMPLE aa2, compound as36-2 (0.74 g) was used instead of aa2-2 to obtain compound aa36-3 (0.86 g) as colorless oil.

### [Step 36-4] Synthesis of (2R)-2-acetyloxy-2-[(2R)-4-[2-methoxycarbonyl-4-(trifluoromethoxy)phenyl]-3-oxomorpholin-2-yl]acetic acid (compound a36-4)

According to the Step 2-4 in synthetic method for EXAMPLE aa2, compound aa36-3 (0.86 g) was used instead of aa2-3 to obtain compound aa36-4 (0.74 g) as a white amorphous solid.

### [Step 36-5] Synthesis of methyl 2-[(2R)-2-[(1R)-1-acetyloxy-2-[3-[[bis[(2-methylpropan-2-yl)oxycarbonyl]amino]methyl]-4-cyanoanilino]-2-oxoethyl]-3-oxomor pholin-4-yl]-5-(trifluoromethoxy)benzoate (compound aa36-5)

According to the Step 2-5 in synthetic method for EXAMPLE aa2, compound aa36-4 (0.35 g) was used instead of aa2-4 to obtain compound aa36-5 (0.43 g) as a white amorphous solid.

### [Step 36-6] Synthesis of methyl 2-[(2R)-2-[(1R)-2-[3-[[bis[(2-methylpropan-2-yl)oxycarbonyl]amino]methyl]-4-cyanoanilino]-1-hydroxy-2-oxoethyl]-3-oxomorpholi n-4-yl)-5-(trifluoromethoxy)benzoate (compound aa36-6)

According to the Step 2-6 in synthetic method for EXAMPLE aa2, compound aa36-5 (70 mg) was used instead of aa2-5 to obtain compound aa36-6 (63 mg) as a white amorphous solid.

### [Step 36-7] Synthesis of methyl 2-[(2R)-2-[(1R)-2-[3-(aminomethyl)-4-cyanoanilino]-1-hydroxy-2-oxoethyl]-3-oxomorpholin-4-yl]-5-(trifluoromethoxy)benzo ate hydrochloride (compound aa36-7)

According to the Step 2-7 in synthetic method for EXAMPLE aa2, compound aa36-6 (59 mg) was used instead of aa2-6 to obtain compound aa36-7 (45 mg) as a white solid.

### [Step 36-8] Synthesis of methyl 2-[(2R)-2-[(1R)-1-hydroxy-2-[(1-imino-2,3-dihydroisoindol-5-yl)amino [-2-oxoethyl]-3-oxomorpholin-4-yl]-5-(trifluoromethoxy)be nzoate hydrochloride (EXAMPLE aa36)

According to the Step 2-8 in synthetic method for EXAMPLE aa2, compound aa36-7 (43 mg) was used instead of aa2-7 to obtain EXAMPLE aa36 (32 mg) as a white amorphous solid.

### EXAMPLE aa37

### Synthesis of 2-[(2R)-2-[(1R)-1-hydroxy-2-[(1-imino-2,3-dihydroisoindol-5-yl)amino]-2-oxoethyl]-3-oxomorpholin-4-yl]-5-(trifluoromethoxy)benzoic acid hydrochloride (EXAMPLE aa37)

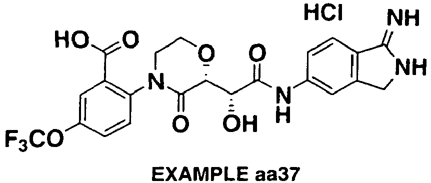

### [Step 37-1] Synthesis of 2-[(2R)-2-[(1R)-2-[3-[[bis[(2-methylpropan-2-yl)oxycarbonyl]amino[methyl]-4-cyanoanilino]-1-hydroxy-2-oxoethyl]-3-oxomorpholi n-4-yl]-5-(trifluoromethoxy)benzoic acid (compound aa37-1)

According to the Step 11-1 in synthetic method for EXAMPLE aa11, compound aa36-5 (0.35 g) was used instead of aa10-5 to obtain compound aa37-1 (0.33 g) as a white amorphous solid.

### [Step 37-2] Synthesis of 2-[(2R)-2-[(1R)-2-[3-(aminomethyl)-4-cyanoanilino]-1-hydroxy-2-oxoethyl]-3-oxomorpholin-4-yl]-5-(trifluoromethoxy)benzoic acid hydrochloride (compound aa37-2)

According to the Step 2-7 in synthetic method for EXAMPLE aa2, compound aa37-1 (33 mg) was used instead of aa2-6 to obtain compound aa37-2 (26 mg) as a white amorphous solid.

### [Step 37-3] Synthesis of 2-[(2R)-2-[(1R)-1-hydroxy-2-[(-imino-2,3-dihydroisoindol-5-yl)amino]-2-oxoethyl]-3-oxomorpholin-4-yl]-5-(trifluoromethoxy)be nzoic acid hydrochloride (EXAMPLE aa37)

According to the Step 2-8 in synthetic method for EXAMPLE aa2, compound aa37-2 (24 mg) was used instead of aa2-7 to obtain EXAMPLE aa37 (16 mg) as a white amorphous solid.

### EXAMPLE aa38

### Synthesis of 2-[(2R)-2-[(1R)-1-hydroxy-2-[(1-imino-2,3-dihydroisoindol-5-yl)amino]-2-oxoethyl]-3-oxomorpholin-4-yl]-5-(trifluoromethoxy)benzamide hydrochloride (EXAMPLE aa38)

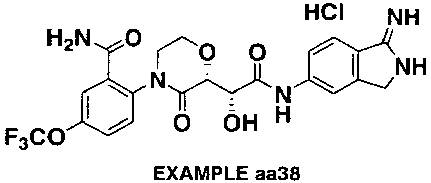

### [Step 38-1] Synthesis of tert-butyl N-[[5-[[(2R)-2-[(2R)-4-[2-carbamoyl-4-(trifluoromethoxy)phenyl]-3-oxomorpholin-2-yl]-2-hydroxyacetyl]amino]-2-cyanophe nyl]methyl]-N-[(2-methylpropan-2-yl)oxycarbonyl]carbamate (compound aa38-1)

According to the Step 2-5 in synthetic method for EXAMPLE aa2, compound aa37-1 (88 mg), NH₄Cl (33 mg), HOBt (30 mg), and diisopropylethylamine (0.11 ml) were used instead of compound aa37-1, tert-butyl N-(5-amino-2-cyanophenyl)methyl-N-[(2-methylpropan-2-yl)oxycarbonyl]carbamate, and DMAP to obtain compound aa38-1 (9.8 mg) as a white amorphous solid.

### [Step 38-2] Synthesis of 2-[(2R)-2-[(1R)-2-[3-(aminomethyl)-4-cyanoanilino]-1-hydroxy-2-oxoethyl]-3-oxomorpholin-4-yl]-5-(trifluoromethoxy)benzamide hydrochloride (compound aa38-2)

According to the Step 2-7 in synthetic method for EXAMPLE aa2, compound aa38-1 (9 mg) was used instead of aa2-6 to obtain compound aa38-2 (9 mg) as a white amorphous solid.

### [Step 38-3] Synthesis of 2-[(2R)-2-[(R)-1-hydroxy-2-[(1-imino-2,3-dihydroisoindol-5-yl)amino]-2-oxoethyl]-3-oxomorpholin-4-yl]-5-(tritluoromethoxy)be nzamide hydrochloride (EXAMPLE aa38)

According to the Step 2-8 in synthetic method for EXAMPLE aa2, compound aa38-2 (8 mg) was used instead of aa2-7 to obtain EXAMPLE aa38 (5 mg) as a white amorphous solid.

**Table 1**

| EXAMPLE | NMR (ppm) (No mark : 400MHz, * : 300MHz) |
|---|---|
| aa1 | * *DMSO-d₆* : 10.19 (1H, s), 8.80-7.68 (3H, m), 8.03 (2H, d, J=9Hz), 7.74 (2H, d, 9Hz), 7.49-7.40 (2H, m), 7.35-7.25 (2H, m), 6.49 (1H, d, J=8Hz), 4.72-4.62 (2H, m), 4.18-4.08 (1H, m), 4.01-3.77 (2H, m), 3.71-3.56 (1H, m), 3.00 (3H, s), 1.31 (9H, s) |
| aa2 | *DMSO-d₆* : 10.25 (1H, s), 9.50-8.90 (3H, m), 8.62 (1H, s), 8.32 (1H, s), 8.10 (1H, d, J=9Hz), 7.94 (1H, d, J=9Hz), 7.72 (1H, d, J=8Hz), 7.63 (1H, s), 7.52 (1H, d, J=8Hz), 6.52 (1H, d, J=7Hz), 4.78 (2H, s), 4.72 (1H, d, J=2Hz), 4.72-4.66 (1H, m), 4.40 (2H, s), 4.24-4.07 (1H, m), 4.06-3.83 (2H, m), 3.78-3.64 (1H, m) |
| aa3 | - *DMSO-d₆* : 11.86 (1H, s), 10.26 (1H, s), 10.19 (1H, s), 9.59 (1H, s), 9.13 (1H, s), 8.36-8.32 (1H, m), 8.15 (1H, d, J=9Hz), 7.99-7.87 (2H, m), 7.70 (1H, d, 2Hz), 7.55 (1H, dd, J=9, 2Hz), 7.34 (1H, d, J=9Hz), 6.64-6.41 (2H, m), 4.80 (2H, s), 4.71 (2H, s), 4.23-4.12 (1H, m), 4.03-3.93 (2H, m), 3.73-3.61 (1H, m) |
| aa4 | * *DMSO-d₆* : 11.93 (1H, s), 10.30 (1H, s), 10.22 (1H, s), 9.63 (1H, s), 9.16 (1H, s), 8.34 (1H, s), 8.18 (1H, d, J=9Hz), 8.00-7.88 (2H, m), 7.75 (1H, s), 7.22 (1H, s), 7.19 (1H, t, J=73Hz), 6.59-6.37 (2H, m), 4.80 (2H, s), 4.73-4.64 (2H, m), 4.26-4.14 (1H, m), 4.00-3.77 (2H, m), 3.56-3.45 (1H, m) |
| aa4-1 | **CDCl₃* : 8.12-8.05 (1H, m), 8.04-7.98 (1H, m), 7.89-7.81 (1H, m), 6.66 (1H, t, J=72Hz) |
| aa5 | * *DMSO-d₆* : 11.85 (1H, brs), 10.20 (1H, s), 9.30-8.82 (4H, m), 8.03 (2H, d, J=8Hz), 7.90 (1H, d, J=9Hz), 7.80 (2H, d, J=8Hz), 7.68 (1H, s), 7.57-7.49 (1H, m), 7.32 (1H, d, J=9Hz), 6.59-6.42 (2H, m), 4.73-4.62 (2H, m), 4.22-4.08 (1H, m), 4.02-3.82 (2H, m), 3.70-3.58 (1H, m) |
| aa6 | * *DMSO-d₆* : 11.87 (1H, d, J=1Hz), 10.38 (1H, s), 9.33 (2H, s), 9.08 (2H, s), 7.99 (1H, dd, J=13, 2Hz), 7.91 (1H, d, J=10Hz), 7.84 (1H, dd, J=9, 2Hz), 7.73-7.62 (2H, m), 7.55 (1H, dd, J=9, 2Hz), 7.38-7.30 (H, m), 6.61-6.51 (2H, m), 4.75-4.63 (2H, m), 4.23-4.10 (1H, m), =1.04-3.83 (2H, m), 3.72-3.62 (1H, m) |
| aa7 | *DMSO-d₆* (100degC): 11.44 (1H, s), 10.00 (2H, s), 9.16 (2H, brs), 8.21 (1H, s), 8.16 (1H, d, J=9Hz), 7.90 (1H, d, J=9Hz), 7.83 (1H, d, J=10Hz), 7.64 (1H, d, J=9Hz), 7.39 (1H, s), 7.25-7.18 (1H, m), 6.44 (1H, d, J=10Hz), 4.79-4.69 (4H, m), 4.22-4.13 (1H, m), 4.01-3.87 (2H, m), 3.70-3.62 (1H, m) |
| aa8 | *DMSD-d₆* : 10.27 (1H, s), 10.21 (1H, s), 9.61 (1H, s), 9.14 (1H, s), 8.33 (1H, s), 8.16 (1H, d, J=9Hz), 7.95 (1H, d, J=9Hz), 7.92 (1H, d, J=10Hz), 7.77 (1H, d, J=8Hz), 7.58 (1H, s), 7.34 (1H, d, J=8Hz), 6.62 (1H, d, J=10Hz), 6.57 (1H, d, J=7Hz), 4.79 (2H, s), 4.77-4.68 (2H, m), 4.25-4.10 (1H, m), 4.07-3.90 (2H, m), 3.88-3.74 (1H, m), 3.61 (3H, s) |
| aa9 | * *DMSO-d₆* : 10.30-10.21 (2H, m), 9.69 (1H, s), 9.19 (1H, s), 8.33 (1H, s), 8.24-8.16 (1H, m), 7.98-7.88 (2H, m), 7.81-7.72 (2H, m), 7.31 (1H, dd, J=8, 2Hz), 6.65-6.36 (2H, m), 4.82-4.71 (4H, m), 4.25-4.12 (3H, m), 4.05-3.93 (2H, m), 3.83-3.75 (1H, m), 1.34-1.22 (1H, m), 0.55-0.40 (4H, m) |
| aa10 | *DMSO-d₆* : 10.26 (1H, s), 10.21 (1H, s), 9.63 (1H, s), 9.15 (1H, s), 8.32 (1H, s), 8.17 (1H, d, J=9Hz), 8.02-7.97 (1H, m), 7.94 (1H, d, J=9Hz), 7.80 (1H, d, J=8Hz), 7.56 (1H, d, J=1Hz), 7.35 (1H, dd, J=8, 1Hz), 6.65 (1H, d, J=9Hz), 6.54 (1H, d, J=7Hz), 5.17-4.99 (2H, m), 4.79 (2H, s), 4.76-4.68 (2H, m), 4.25-4.10 (1H, m), 4.04-3.86 (2H, m), 3.85-3.73 (1H, m), 3.71 (3.H, s) |
| aa11 | *DMSO-d₆* : 13.08 (1H, s), 10.25 (1H, s), 10.20(1H, s), 9.57 (1H, s), 9.13 (1H, s), 8.33 (1H, s), 8.14 (1H, d, J=9Hz), 8.02-7.90 (2H, m), 7.79 (1H, d, J=9Hz), 7.53 (1H, d, J=1Hz), 7.38-7.28 (1H, m), 6.64 (1H, d, J=9Hz), 6.56 (1H, d, J=6Hz), 5.04-4.89 (2H, m), 4.79 (2H, s), 4.73 (1H, d, J=2Hz), 4.73-4.68 (1H, m), 4.23-4.09 (1H, m), 4.03-3.85 (2H, m), 3.83-3.71 (1H, m) |
| aa12 | *DMSO-d₆* (100degC) : 10.53 (1H, brs), 10.20-9.79 (2H, m), 9.59-8.73 (2H, m), 8.22 (1H, s), 8.18(1H, d, J=9Hz), 7.90 (1H, d, J=9Hz), 7.85 (1H, d, J=10Hz), 7.54 (1H, d, J=8Hz), 7.15-6.91 (1H, m), 6.50 (1H, d, J=10Hz), 6.42-5.52 (1H, m), 4.83-4.57 (4H, m), 4.33-3.26 (4H, m), 2.40-2.15 (3H, m) |
| aa13 | * *DMS0-d₆* : 9.95 (1H, s), 8.24 (1H, d, J=9Hz), 8.12 (1H, s), 7.92 (1H, d, J=9Hz), 7.85-7.75 (3H, m), 7.58-7.52 (1H, m), 7.06-7.00 (1H, m), 4.77-4.68 (2H, m), 4.50 (2H, s), 4.24-4.14 (1H, m), 4.04-3.93 (5H, m), 3.88-3.78 (1H, m) |
| aa14 | * *DMSO-d₆* : 10.32 (1H, s), 10.28 (1H, s), 9.76 (1H, s), 9.23 (1H, s), 9.16 (1H, d, J=4Hz), 8.88 (1H, d, J=8Hz), 8.41-8.19 (4H, m), 8.03-7.82 (3H, m), 4.86-4.71 (4H, m), 4.27-4.17 (1H, m), 4.13-3.98 (2H, m), 3.92-3.83 (1H, m) |
| aa15 | * *DMSO-d₆* : 10.06 (1H, s), 9.28-9.18 (1H, m), 9.03 (1H, d, J=8Hz), 8.43 (1H, s), 8.35 (1H, d, J=9Hz), 8.12-8.01 (2H, m), 7.97 (1H, dd, J = 8. 5Hz), 7.72 (1H, d, J=9Hz), 7.58 (1H, dd, J=9, 1Hz), 4.84 (1H, d, J=2Hz), 4.71 ( 1H, d, J=2Hz), 4.33-4.17 (1H, m), 4.17-3.95 (2H, m), 3.94-3.73 (1H, m) |
| aa16 | * *DMSO-d₆* (100degC) : 11.53 (1H, brs), 10.16-9.82 (2H, m), 9.43-8.86 (2H, m), 8.23 (1H, s), 8.18 (1H, d, J=8Hz), 7.99-7.69 (2H, m), 7.59-7.47 (1H, m), 7.34 (1H, d, J=8Hz), 7.12 (1H, s), 6.48 (1H, d, J=9Hz), 4.93-4.61 (4H, m), 4.38-3.34 (4H, m) |
| aa17 | * *DMSO-d₆* : 10.26 (1H, s), 8.33 (1H, s), 8.16 (1H, d, J=9Hz), 8.00-7.84 (3H, m), 7.44-7.38 (2H, m), 6.51 (1H, d, J=7Hz), 4.83-4.66 (4H, m), 4.21-4.08 (1H, m), 3.99-3.83 (2H, m), 3.74-3.63 (1H, m), 3.43-3.35 (2H, m), 2.97-2.87 (2H, m) |
| aa18 | * *DMSO-d₆* : 10.35-10.12 (3H, m), 9.66 (1H, brs), 9.18 (1H, brs), 8.33 (1H, s), 8.19 (1H, d, J=8Hz), 8.00-7.88 (1H, m), 7.22 (1H, d, J=7Hz), 6.98-6.83 (2H, m), 6.45 (1H, d, J=6Hz), 4.79 (2H, s), 4.72-4.62 (2H, m), 4.19-4.05 (1H, m), 4.01-3.75 (2H, m), 3.63-3.49 (1H, m), 2.95-2.82 (2H, m), 2.51-2.40 (2H, m) |
| aa19 | *DMSO-d₆* : 10.40-8.99 (4H, m), 8.32 (1H, s), 8.22-8.12 (1H, m), 7.94 (1H, d, J=7Hz), 7.31-7.22 (1H, m), 7.11 (1H, s), 7.0 1(1H, d, J=6Hz), 6.54 (1H, brs), 4.79 (2H, s), 4.69 (2H, s), 4.19-4.08 (1H, m), 4.00-3.89 (1H, m), 3.88-3.78 (1H, m), 3.71-3.61 (1H, m), 3.24 (3H, s), 2.92-2.82 (2H, m), 2.61-2.53 (2H, m) |
| aa20 | * *DMSO-d₆* : 10.32-10.06 (2H, m), 9.64 (1H, brs), 9.17 (1H, brs), 8.33-8.32 (1H, m), 8.17 (1H, d, J=9Hz), 7.94 (1H, dd, J=9, 2Hz), 7.35-7.22 (2H, m), 6.99 (1H, dd, J=8, 2Hz), 6.55-6.45 (1H, m), 4.78 (2H, s), 4.72-4.66 (2H, m), 4.20-4.08 (1H, m), 4.00-3.72 (4H, m), 3.70-3.60 (1H, m), 2.93-2.80 (2H, m), 2.64-2.53 (2H, m), 1.18-1.04 (1H, m), 0.50-0.28 (4H, m) |
| aa21 | * *DMSO-d₆* : 10.34-9.93 (2H, m), 9.45 (2H, brs), 8.32 (1H, s), 8.18 (1H, d, J=9Hz), 7.94 (1H, d, J=9Hz), 7.29 (1H, d, J=8Hz), 7.09-6.97 (2H, m), 6.49 (1H, d, J=7Hz), 4.78 (2H, s), 4.69-4.58 (4H, m), 4.20-4.07 (1H, m), 3.99-3.76 (2H, m), 3.74-3.58 (4H, m), 2.98-2.83 (2H, m), 2.66-2.56 (2H, m) |
| aa22 | *DMSO-d₆* : 12.92 (1H, brs), 10.25 (2H, brs), 9.62 (1H, brs), 9.18 (1H, brs), 8.32 (1H, s), 8.16(1H, d, J=8Hz), 7.98-7.88 (1H, m), 7.28 (1H, d, J=7Hz), 7.06-6.94 (2H, m), 6.53 (1H, d, J=6Hz), 4.78 (2H, s), 4.67 (2H, s), 4.61-4.45 (2H, m), 4.18-4.06 (1H, m), 3.99-3.88 (1H, m), 3.86-3.75 (1H, m), 3.69-3.59 (1H, m), 2.96-2.82 (2H, m), 2.65-2.55 (2H, m) |
| aa23 | * *DMSO-d₆* : 10.37 (1H, s), 10.18 (1H, s), 9.33 (2H, s), 9.15 (2H, s), 7.96 (1H, dd, J=13, 2Hz), 7.81 (1H, dd, J=9, 2Hz), 7.68-7.60 (1H, m), 7.36 (1H, s), 7.25-7.14 (2H, m), 7.02 (1H, s), 6.94-6.83 (2H, m), 6.49 (1H, d, J = 7Hz), 4.71-4.58 (2H, m), 4.17-3.72 (3H, m), 3.59-3.46 (1H, m), 2.93-2.80 (2H, m), 2.49-2.39 (2H, m) |
| aa24 | *DMSO-d₆* : 10.18 (1H, s), 10.01 (1H, s), 8.03 (1H, d, J=1Hz), 7.70 (1H, d, J=9Hz), 7.57 (1H, dd, J=9, 1Hz), 7.21 (1H, d, J=8Hz), 6.91 (1H, dd, J=8, 2Hz), 6.88 (1H, d, J=2Hz), 4.67 (1H, d, J=2Hz), 4.64 (1H, d, J=2Hz), 4.17-4.07 (1H, m), 3.99-3.88 (1H, m), 3.88-3.74 (1H, m), 3.60-3.47 (1H, m), 2.87 (2H, t, J=7Hz), 2.49-2.42 (2H, m) |
| aa25 | * *DMSO-d₆* : 13.02 (1H, brs), 10.39 (1H, s), 10.19 (1H, s), 8.93 (2H, brs), 8.59-8.41 (2H, m), 8.04 (1H, d, J=10Hz), 7.63 (1H, d, J=7Hz), 7.29-7.08 (2H, m), 6.99-6.81 (2H, m), 6.50 (1H, d, J=7Hz), 4.69 (2H, s), 4.20-4.05 (1H, m), 4.01-3.74 (2H, m), 3.62-3.50 (1H, m), 2.95-2.80 (2H, m), 2.50-2.38 (2H, m) |
| aa26 | *DMSO-d₆* : 10.18 (1H, s), 10.07 (1H, s), 8.32 (1H, s), 8.23 (1H, s), 8.12 (1H, d, J=9Hz), 7.80 (1H, d, J=8Hz), 7.64 (2H, brs), 7.22 (1H, d, J=8Hz), 6.97-6.84 (2H, m), 6.39 (1H, d, J=4Hz), 4.75-4.60 (2H, m), 4.19-4.06 (1H, m), 4.01-3.89 (1H, m), 3.87-3.76 (1H, m), 3.62-3.50 (1H, m), 2.94-2.81 (2H, m), 2.49-2.40 (2H, m) |
| aa27 | *DMSO-d₆* : 10.34-10.07 (3H, m), 9.59 (1H, brs), 9.13 (1H, brs), 8.33 (1H, s), 8.14 (1H, d, J=9Hz), 8.00-7.83 (1H, m), 7.27-7.18 (1H, m), 6.98-6.78 (2H, m), 6.64-6.46 (1H, m), 4.83-4.60 (4H, m), 4.18-3.39 (4H, m), 2.88-2.62 (2H, m), 2.49-2.27 (2H, m) |
| aa28 | ** DMSO-d₆* : 10.28 (1H, s), 10.21 (1H, brs), 9.62 (1H, brs), 9.15 (1H, brs), 8.34 (1H, s), 8.17 (1H, d, J=9Hz), 7.96 (1H, d, J=9Hz), 7.39-7.29 (1H, m), 7.25-7.10 (2H, m), 7.05-6.93 (1H, m), 6.48 (1H, d, J=7Hz), 4.80 (2H, s), 4.71-4.60 (2H, m), 4.40 (1H, s), 4.25-4.05 (3H, m), 3.95-3.82 (1H, m), 3.80-3.65 (1H, m), 3.50-3.39 (1H, m), 1.91-1.81 (2H, m), 1.18 (6H, s) |
| aa29 | ** DMSO-d₆* : 10.37-10.12 (2H, m), 9.69 (1H, brs), 9.19 (1H, brs), 8.33 (1H, s), 8.20 (1H, d, J=9Hz), 7.94 (1H, d, J=9Hz), 7.38-7.26 (1H, m), 7.23-7.09 (2H, m), 7.03-6.92 (1H, m), 6.54-6.39 (1H, m), 4.78 (2H, s), 4.70-4.57 (2H, m), 4.20-3.99 (3H, m), 3.94-3.80 (1H, m), 3.79-3.64 (1H, m), 3.50-3.30 (1H, m), 3.11 (3H, s), 1.98-1.87 (2H, m), 1.17 (6H, s) |
| aa30 | ** DMSO-d₆* (100degC) : 10.10-9.83 (2H, m), 9.39-8.92 (2H, m), 8.19 (1H, s), 8.17 (1H, d, J=9Hz), 7.89 (1H, d, J=9Hz), 7.45-7.22 (5H, m), 4.76 (2H, s), 4.69 (1H, d, J=2Hz), 4.66 (1H, d, J=2Hz), 4.15-4.07 (1H, m), 3.90-3.69 (2H, m), 3.68-3.42 (3H, m), 2.52-2.33 (2H, m), 1.99-1.68 (4H, m) |
| aa31 | *DMSO-d₆* : 10.27 (1H, s), 9.61-9.02 (3H, m), 8.34-8.32 (1H, m), 8.14 (1H, d, J=9Hz), 7.94 (1H, dd, J=9, 2Hz), 7.44-7.29 (3H, m), 7.05 (1H, t. J=74Hz), 6.47 (1H, d, J=6Hz), 4.78 (2H, s), 4.73-4.62 (2H, m), 4.22-4.12 (1H, m), 3.99-3.75 (2H, m), 3.55-3.47 (1H, m) |
| aa31-1 | **CDCl₃* : 7.36 (1H, dd, J=8, 3Hz), 7.27-7.18 (1H, m), 7.08-6.99 (1H, m), 6.48 (1H, t, J=73Hz) |
| aa32 | ** DMSO-d₆* (100degC) : 9.99 (1H, s), 9.27 (3H, brs), 8.20 (1H, s), 8.16 (1H, d, J=8Hz), 7.92-7.86 (1H, m), 7.81 (1H, dd, J=8, 2Hz), 7.63 (1H, dd, J=8, 2Hz), 7.49 (1H, t, J=8Hz), 6.80 (1H, t, J=74Hz), 6.03 (1H, s), 4.76 (2H, s), 4.70 (2H, s), 4.25-4.16 (1H, m), 3.97-3.79 (2H, m), 3.86 (3H, s), 3.58-3.48 (1H, m) |
| aa33 | • *DMSO-d₆* (100degC) : 9.99 (1H, s), 9.35 (3H, brs), 8.23-8.13 (2H, m), 7.89 (1H, dd, J=8, 2Hz), 7.79 (1H, dd, J=8, 2Hz). 7.56 (1H, dd, J=8, 2Hz), 7.44 (1H, t, J=8Hz), 6.82 (1H, t, J=75Hz), 6.02 (1H, s), 4.76 (2H, s), 4.73-4.67 (2H, m), 4.25-4.16 (1H, m), 3.98-3.79 (2H, m), 3.58-3.49 (1H, m) |
| aa34 | ** DMSO-d₆ :* 10.27 (1H, s), 9.36 (3H, brs), 8.33 (1H, s), 8.14 (1H, d, J=9Hz), 7.97-7.91 (1H, m), 7.54-7.36 (3H, m), 7.03-6.44 (2H, m), 4.78 (2H, s), 4.72-4.64 (2H, m), 4.24-4.12 (1H, m), 3.98-3.80 (2H, m), 3.60-3.48 (1H, m), 2.99 (3H, s), 2.80 (3H, s) |
| aa35 | ** DMSO-d₆:* 10.33-9.99 (2H, m), 9.70-9.60 (1H, m), 9.20-9.10 (1H, m), 8.36-8.26 (1H, m), 8.21-8.13 (1H, m), 7.99-7.77 (2H, m), 7.71-7.62 (1H, m), 7.57-7.40 (3H, m), 7.10-6.39 (2H, m), 4.79 (2H, s), 4.67-4.59 (2H, m), 4.24-4.12 (1H, m), 4.01-3.36 (3H, m) |
| aa36 | *DMSO-d₆ :* 10.27 (1H, s), 8.32 (1H, s), 8.14 (1H, d, J=9Hz), 7.94 (1H, dd, J=9, 2Hz), 7.83-7.73 (2H, m), 7.57 (1H, d, J=9Hz), 6.42 (1H, d, J=7Hz), 4.83-4.59 (4H, m), 4.24-4.13 (1H, m), 4.01-3.89 (2H, m), 3.85 (3H, s), 3.59-3.47 (1H, m) |
| aa37 | *DMSO-d₆ :* 10.29-10.22 (2H, m), 9.64 (1H, s), 9.18 (1H, s), 8.32 (1H, s), 8.17 (1H, d, J=9Hz), 7.93 (1H, dd, J=9, 1Hz), 7.82-7.67 (2H, m), 7.49 (1H, d, J=9Hz), 6.48 (1H, brs), 4.78 (2H, s), 4.68-4.55 (2H, m), 4.23-4.14 (1H, m), 3.98-3.83 (2H, m), 3.55-3.46 (1H, m) |
| aa38 | ** DMSO-d₅:* 10.25 (1H, s), 8.32 (1H, s), 8.20-8.06 (1H, m), 7.98-7.88 (2H, m), 7.65-7.40 (4H, m), 6.38-6.26 (1H, m), 4.78 (2H, s), 4.68-4.62 (1H, m), 4.61-4.56 (1H, m), 4.21-4.07 (1H, m), 3.96-3.81 (2H, m), 3.62-3.48 (1H, m) |

**Table 2**

| | | | | | | | | |
|---|---|---|---|---|---|---|---|---|
| EXAMPLE | LC/MS | | | | aa18 | 450 | 2.47 | A |
| | m/z | RT | solvent system | | aa18-1 | 399 [M+Na]⁺ | 4.27 | A |
| | [M+1]⁺ | min | | | aa18-2 | 419 | 4.52 | A |
| aa1 | 439 | 4.30 | B | | aa18-3 | 363 | 3.27 | B |
| aa1-1 | 212 | 5.40 | C | | aa18-4 | 692 | 5.52 | A |
| aa1-2 | 335 | 2.82 | D | | aa18-5 | 650 | 5.52 | A |
| aa1-3 | 528 | 5.33 | C | | aa18-6 | 450 | 3.15 | B |
| aa1-4 | 444 | 4.63 | C | | aa19 | 464 | 2.57 | A |
| aa1-5 | 408 | 4.30 | C | | aa19-1 | 288 | 5.27 | A |
| aa2 | 436 | 2.85 | B | | aa19-2 | 391 | 4.37 | A |
| aa2-1 | 498 | 6.87 | A | | aa19-3 | 433 | 4.60 | A |
| aa2-2 | 601 | 6.33 | A | | aa19-4 | 377 | 3.35 | B |
| aa2-3 | 643 | 6.37 | A | | aa19-5 | 728 [M+Na]⁺ | 5.57 | A |
| aa2-4 | 349 | 2.52 | B | | aa19-6 | 686 [M+Na]⁺ | 5.55 | A |
| aa2-5 | 700 [M+Na]⁺ | 5.40 | A | | aa19-7 | 464 | 2.53 | A |
| aa2-6 | 658 [M+Na]⁺ | 5.42 | A | | aa20 | 504 | 2.88 | A |
| aa2-7 | 436 | 0.27 | A | | aa20-1 | 328 | 5.87 | A |
| aa3 | 448 | 3.12 | B | | aa20-2 | 453 [M+Na]⁺ | 4.93 | A |
| aa3-1 | 375 | 4.13 | A | | aa20-3 | 473 | 5.13 | A |
| aa3-2 | 417 | 4.37 | B | | aa20-4 | 417 | 4.07 | B |
| aa3-3 | 361 | 2.90 | B | | aa20-5 | 768 [M+Na]⁺ | 5.78 | A |
| aa3-4 | 712 [M+Na]⁺ | 5.47 | B | | aa20-6 | 726 [M+Na]⁺ | 5.82 | A |
| aa3-5 | 670 [M+Na]⁺ | 5.42 | B | | aa20-7 | 504 | 2.87 | A |
| aa3-6 | 448 | 3.03 | B | | aa21 | 522 | 3.37 | B |
| aa4 | 514 | 3.40 | B | | aa21-1 | 346 | 4.97 | A |
| | | | | | | | | |
| aa4-2 | 286 | 4.97 | A | | aa21-2 | 449 | 4.38 | A |
| aa4-3 | 384 | 5.67 | A | | aa21-3 | 491 | 4.62 | A |
| aa4-4 | 338 | 5.10 | A | | aa21-4 | 435 | 3.40 | B |
| aa4-5 | 441 | 4.57 | A | | aa21-5 | 786 [M+Na]⁺ | 5.62 | A |
| aa4-6 | 483 | 4.83 | A | | aa21-6 | 744 [M+Na]⁺ | 5.57 | A |
| aa4-7 | 427 | 3.48 | B | | aa21-7 | 522 | 2.57 | A |
| aa4-8 | 778 [M+Na]⁺ | 5.60 | A | | aa22 | 508 | 3.23 | B |
| aa4-9 | 736 [M+Na]⁺ | 5.58 | A | | aa22-1 | 730 [M+Na]⁺ | 5.50 | B |
| aa4-10 | 514 | 3.37 | B | | aa22-2 | 508 | 3.12 | B |
| aa5 | 436 | 2.95 | B | | aa23 | 456 | 3.15 | B |
| aa5-1 | 520 | 4.18 | B | | aa23-1 | 538 | 4.25 | B |
| aa5-2 | 478 | 4.05 | B | | aa23-2 | 496 | 4.12 | B |
| aa6 | 454 | 3.00 | B | | aa24 | 452 | 3.68 | A |
| aa6-1 | 538 | 4.17 | B | | aa24-1 | 624 | 4.53 | A |
| aa6-2 | 496 | 3.98 | B | | aa25 | 462 | 3.45 | B |
| aa7 | 448 | 3.08 | B | | aa25-1 | 704 | 5.17 | A |
| aa7-1 | 272 | 4.70 | A | | aa25-2 | 662 | 5.05 | A |
| aa7-2 | 375 | 4.20 | A | | aa26 | 463 | 3.27 | B |
| aa7-3 | 417 | 4.50 | A | | aa26-1 | 605 | 4.77 | A |
| aa7-4 | 361 | 3.27 | B | | aa26-2 | 563 | 4.63 | A |
| aa7-5 | 648 | 5.48 | A | | aa27 | 450 | 2.87 | B |
| aa7-6 | 448 | 3.12 | B | | aa27-1 | 377 | 4.03 | A |
| aa8 | 462 | 3.27 | B | | aa27-2 | 419 | 4.28 | A |
| aa8-1 | 286 | 4.92 | A | | aa27-3 | 363 | 2.90 | B |
| aa8-2 | 389 | 4.35 | A | | aa27-4 | 672 [M+Na]⁺ | 5.80 | A |
| aa8-3 | 431 | 4.58 | A | | aa27-5 | 450 | 2.80 | A |
| aa8-4 | 375 | 3.37 | B | | aa28 | 483 | 3.73 | B |
| aa8-5 | 726 [M+Na]⁺ | 5.52 | A | | aa28-1 | 329 [M+Na]⁺ | 5.65 | A |
| aa8-6 | 684 [M+Na]⁺ | 5.48 | A | | aa28-2 | 410 | 4.92 | A |
| aa8-7 | 462 | 3.12 | B | | aa28-3 | 474 [M+Na]⁺ | 5.07 | A |
| aa9 | 502 | 3.70 | B | | aa28-4 | 396 | 3.98 | B |
| aa9-1 | 326 | 5.58 | A | | aa28-5 | 747 [M+Na]⁺ | 5.80 | A |
| aa9-2 | 429 | 4.87 | A | | aa28-6 | 705 | 5.80 | A |
| aa9-3 | 471 | 5.05 | A | | aa28-7 | 483 | 2.87 | A |
| aa9-4 | 415 | 3.97 | B | | aa29 | 497 | 3.97 | B |
| aa9-5 | 744 | 5.75 | A | | aa29-1 | 343 [M+Na]⁺ | 6.22 | A |
| aa9-6 | 702 | 5.73 | A | | aa29-2 | 424 | 5.32 | A |
| aa9-7 | 502 | 2.87 | A | | aa29-3 | 466 | 5.35 | A |
| aa10 | 520 | 3.30 | B | | aa29-4 | 432 [M+Na]⁺ | 4.47 | B |
| aa10-1 | 344 | 4.70 | A | | aa29-5 | 761 [M+Na]⁺ | 6.05 | A |
| aa10-2 | 447 | 4.30 | A | | aa29-6 | 719 | 5.97 | A |
| aa10-3 | 489 | 4.58 | A | | aa29-7 | 497 | 3.12 | A |
| aa10-4 | 433 | 3.33 | B | | aa30 | 478 | 3.15 | B |
| aa10-5 | 784 [M+Na]⁺ | 5.53 | A | | aa30-1 | 405 | 4.38 | A |
| aa10-6 | 742 [M+Na]⁺ | 5.55 | A | | aa30-2 | 447 | 4.65 | A |
| aa10-7 | 520 | 3.18 | B | | aa30-3 | 391 | 3.23 | B |
| aa11 | 506 | 3.17 | B | | aa30-4 | 742 [M+Na]⁺ | 5.63 | A |
| aa11-1 | 728 [M+Na]⁺ | 5.50 | B | | aa30-5 | 700 [M+Na]⁺ | 5.57 | A |
| aa11-2 | 506 | 3.15 | B | | aa30-6 | 478 | 0.37 | B |
| aa12 | 462 | 3.27 | B | | aa31 | 465 | 3.62 | B |
| aa12-1 | 332 | 5.22 | A | | aa31-2 | 414 [M+Na]⁺ | 4.95 | A |
| aa12-2 | 286 | 5.07 | A | | aa31-3 | 456 [M+Na]⁺ | 5.18 | A |
| aa12-3 | 389 | 4.15 | A | | aa31-4 | 378 | 4.15 | B |
| aa12-4 | 431 | 4.45 | A | | aa31-5 | 729 [M+Na]⁺ | 5.83 | A |
| aa12-5 | 375 | 3.09 | B | | aa31-6 | 687 [M+Na]⁺ | 5.82 | A |
| aa12-6 | 704 | 5.50 | A | | aa31-7 | 465 | 3.58 | B |
| aa12-7 | 662 | 5.47 | A | | aa32 | 505 | 3.58 | B |
| aa12-8 | 462 | 3.10 | B | | aa32-1 | 270 [M+Na]⁺ | 4.58 | B |
| aa13 | 462 | 3.78 | B | | aa32-2 | 240 [M+Na]⁺ | 4.03 | A |
| aa13-1 | 286 | 6.15 | A | | aa32-3 | 351 [M+Na]⁺ | 5.25 | A |
| aa13-2 | 389 | 5.05 | A | | aa32-4 | 432 | 4.97 | A |
| aa13-3 | 431 | 5.23 | A | | aa32-5 | 474 | 5.12 | A |
| aa13-4 | 375 | 4.20 | B | | aa32-6 | 418 | 4.02 | B |
| aa13-5 | 704 | 5.90 | A | | aa32-7 | 769 [M+Na]⁺ | 5.88 | B |
| aa13-6 | 662 | 5.87 | A | | aa32-8 | 727 [M+Na]⁺ | 5.85 | B |
| aa13-7 | 462 | 2.92 | A | | aa32-9 | 505 | 3.72 | B |
| aa14 | 432 | 2.72 | B | | aa33 | 491 | 3.22 | B |
| aa14-1 | 359 | 4.27 | A | | aa33-1 | 713 [M+Na]⁺ | 5.70 | B |
| aa14-2 | 401 | 3.92 | B | | aa33-2 | 491 | 3.37 | B |
| aa14-3 | 345 | 2.02 | B | | aa34 | 518 | 3.23 | B |
| aa14-4 | 674 | 5.28 | B | | aa34-1 | 740 [M+Na]⁺ | 5.65 | A |
| aa14-5 | 632 | 5.62 | A | | aa34-2 | 518 | 3.20 | B |
| aa14-6 | 432 | 2.72 | B | | aa35 | 490 | 2.90 | B |
| aa15 | 434 | 2.97 | B | | aa35-1 | 712 [M+Na]⁺ | 5.45 | A |
| aa15-1 | 606 | 4.43 | B | | aa35-2 | 490 | 2.82 | B |
| aa16 | 448 | 3.07 | B | | aa36 | 523 | 4.18 | B |
| aa16-1 | 272 | 4.82 | A | | aa36-1 | 346 | 5.93 | A |
| aa16-2 | 375 | 4.08 | A | | aa36-2 | 450 | 5.43 | A |
| aa16-3 | 417 | 4.28 | A | | aa36-3 | 492 | 5.55 | A |
| aa16-4 | 361 | 2.77 | B | | aa36-4 | 436 | 4.85 | B |
| aa16-5 | 690 | 5.42 | A | | aa36-5 | 787 [M+Na]⁺ | 6.10 | A |
| aa16-6 | 648 | 5.42 | A | | aa36-6 | 745 [M+Na]⁺ | 5.97 | A |
| aa16-7 | 448 | 3.03 | B | | aa36-7 | 523 | 4.15 | B |
| aa17 | 450 | 2.97 | B | | aa37 | 509 | 4.00 | B |
| aa17-1 | 377 | 4.10 | A | | aa37-1 | 731 [M+Na]⁺ | 5.93 | B |
| aa17-2 | 419 | 4.35 | A | | aa37-2 | 509 | 4.02 | B |
| aa17-3 | 363 | 3.03 | B | | aa38 | 508 | 3.62 | B |
| aa17-4 | 714 [M+Na]⁺ | 5.43 | A | | aa38-1 | 730 [M+Na]⁺ | 5.73 | A |
| aa17-5 | 650 | 5.40 | A | | aa38-2 | 508 | 3.58 | B |
| aa17-6 | 450 | 3.05 | B | | | | | |

### EXAMPLE ap1

### Synthesis of (2R)-N-(3-amino-1,2-benzisoxazol-6-yl)-2-hydroxy-2-[(2R)-3-oxo-4-(2-oxo-1H-quinolin-6-yl)morpholin-2-yl]acetamide (EXAMPLE ap1)

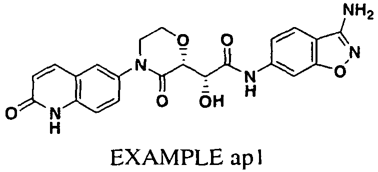

According to the Step 15-1 in the synthetic method for Example aa15, (2R)-2-acetyloxy-2-[(2R)-3-oxo-4-(2-oxo-1H-quinolin-6-yl)morpholin-2-yl]acetic acid (compound aa3-3) and 6-amino-3-(1,3-dioxoisoindol-2-yl)-1,2-benzisoxazole can be used to obtain [(1R)-2-[[3-(1,3-dioxoisoindol-2-yl)-1,2-benzisoxazol-6-yl]amino]-1-oxo-1-[(2R)-3-oxo-4-(2-oxo-1H-quinolin-6-yl)morpholin-2-yl]ethyl]acetat e (compound ap1-1), then further treatment can be achieved according to the Step 15-2 to obtain the title compound ap1.

### EXAMPLE ap2

### Synthesis of (2R)-N-(3-amino-1,2-benzisoxazol-6-yl)-2-hydroxy-2-[(2R)-3-oxo-4-(2-oxo-1H-quinolin-7-yl)morpholin-2-yl]acetamide (EXAMPLE ap2)

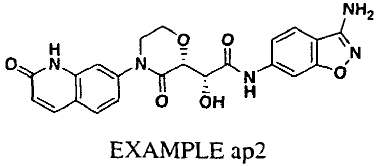

According to the Step 15-1 in the synthetic method for Example aa15, (2R)-2-acetyloxy-2-[(2R)-3-oxo-4-(2-oxo-1H-quinolin-7-yl)morpholin-2-yl]acetic acid (compound aa7-4) and 6-amino-3-(1,3-dioxoisoindol-2-yl)-1,2-benzisoxazole can be used to obtain [(1R)-2-[[3-(1,3-dioxoisoindol-2-yl)-1,2-benzisoxazol-6-yl]amino]-2-oxo-1-[(2R)-3-oxo-4-(2-oxo-1H-quinolin-7-yl)morpholin-2-yl]ethyl]acetat e (compound ap2-1), then further treatment can be achieved according to the Step 15-2 to obtain the title compound ap2.

### EXAMPLE ap3

### Synthesis of (2R)-N-(3-amino-1,2-benzisoxazol-6-yl)-2-hydroxy-2-[(2R)-3-oxo-4-(2-oxo-1H-quinolin-5-yl)morpholin-2-yl]acetamide (EXAMPLE ap3)

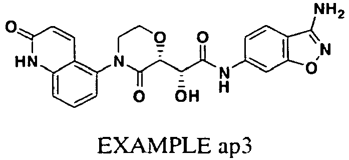

According to the Step 15-1 in the synthetic method for Example aa15, (2R)-2-acetyloxy-2-[(2R)-3-oxo-4-(2-oxo-1H-quinolin-5-yl)morpholin-2-yl]acetic acid (compound aa16-4) and 6-amino-3-(1,3-dioxoisoindol-2-yl)-1,2-benzisoxazole can be used to obtain [(1R)-2-[[3-(1,3-dioxoisoindol-2-yl)-1,2-benzisoxazol-6-yl]amino]-2-oxo-1-[(2R)-3-oxo-4-(2-oxo-1H-quinolin-5-yl)morpholin-3-yl]ethyl]acet ate (compound ap3-1), then further treatment can be achieved according to the Step 15-2 to obtain the title compound ap3.

### EXAMPLES aa39 - aa94

| Structure | EXAMPLE | M+H |
|---|---|---|
| 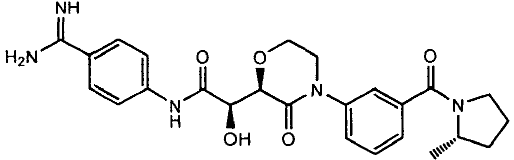 | aa39 | 480.3 |
| 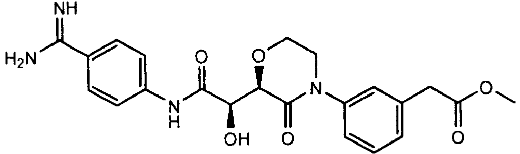 | aa40 | 441.2 |
| 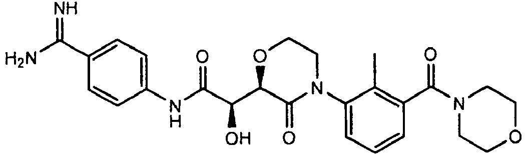 | aa41 | 496.3 |
| 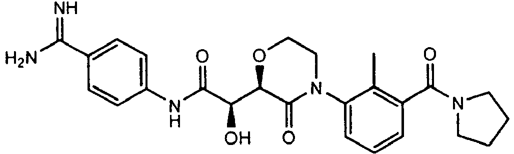 | aa42 | 480.3 |
| 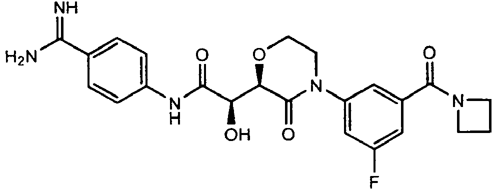 | aa43 | 470.3 |
| 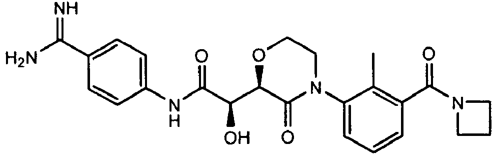 | aa44 | 466.3 |
| 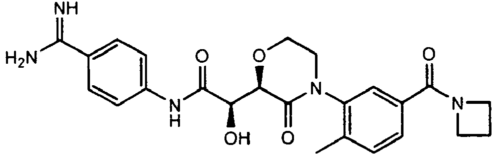 | aa45 | 466.3 |
| 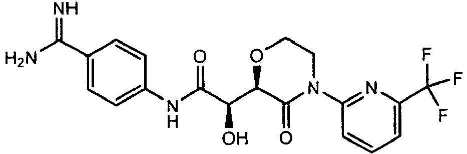 | aa46 | 438.2 |
| 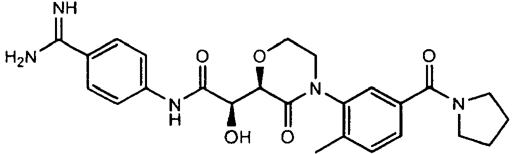 | aa47 | 480.3 |
| 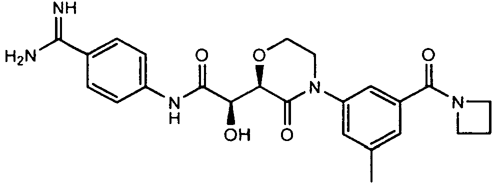 | aa48 | 466.3 |
| 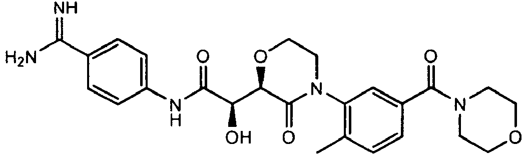 | aa49 | 496.3 |
| 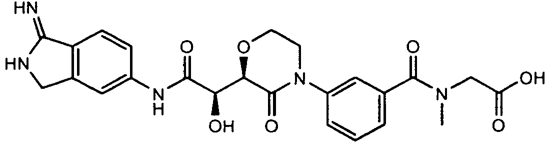 | aa50 | 496.2 |
| 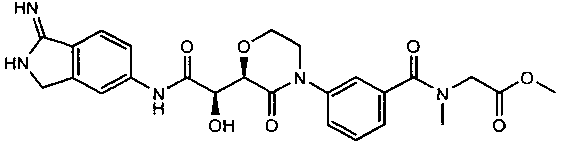 | aa51 | 510.2 |
| 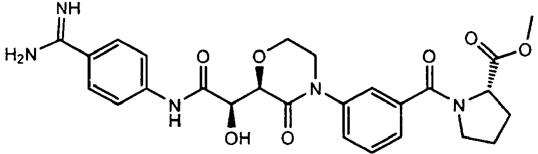 | aa52 | 524.2 |
| 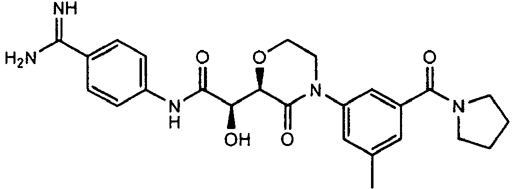 | aa53 | 480.3 |
| 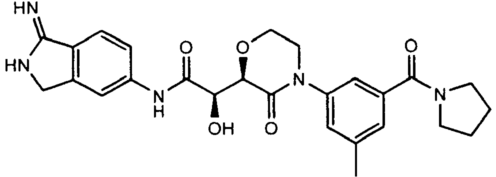 | aa54 | 492.2 |
| 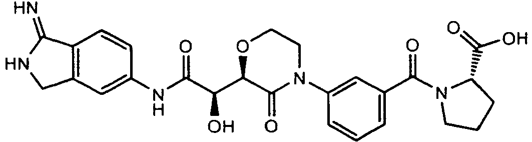 | aa55 | 522.6 |
| 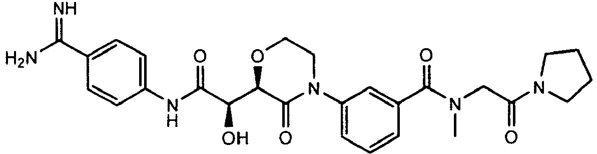 | aa56 | 537.3 |
| 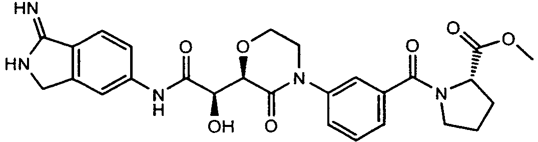 | aa57 | 536.6 |
| 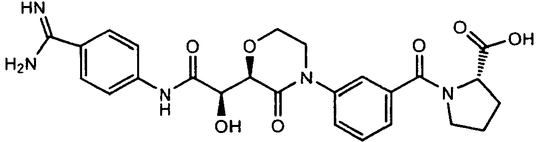 | aa58 | 510.7 |
| 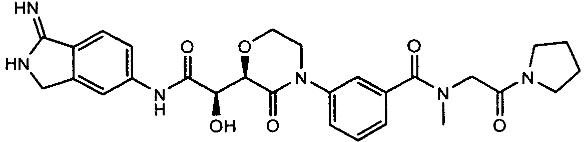 | aa59 | 549.3 |
| 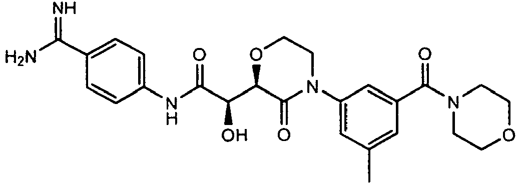 | aa60 | 496.3 |
| 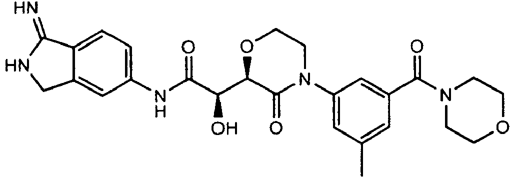 | aa61 | 508.3 |
| 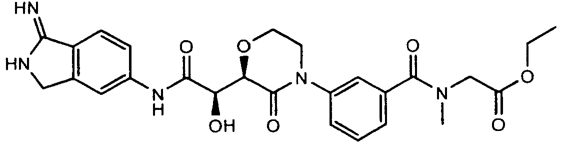 | aa62 | 524.2 |
| 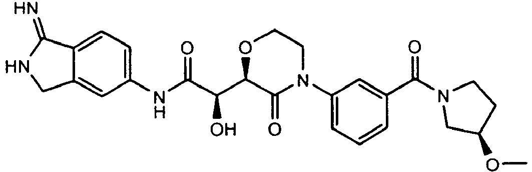 | aa63 | 508.3 |
| 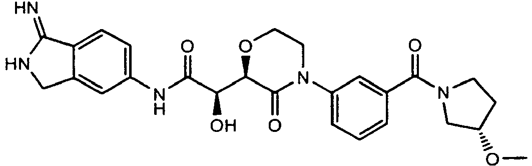 | aa64 | 508.3 |
| 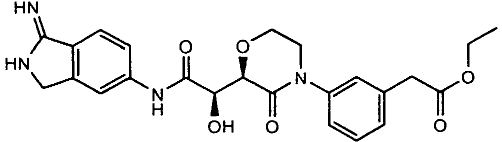 | aa65 | 467.2 |
| 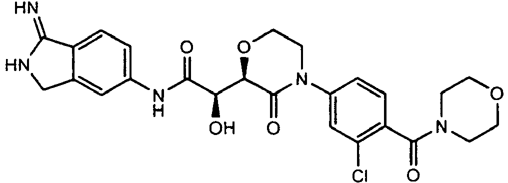 | aa66 | 528.3 |
| 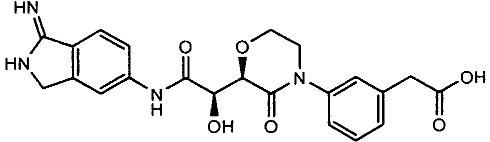 | aa67 | 439.2 |
| 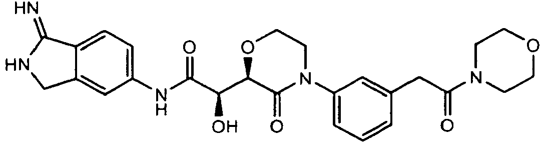 | aa68 | 508.3 |
| 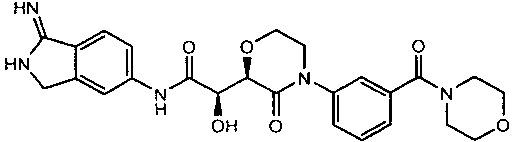 | aa69 | 494.3 |
| 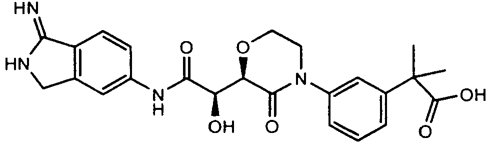 | aa70 | 467.2 |
| 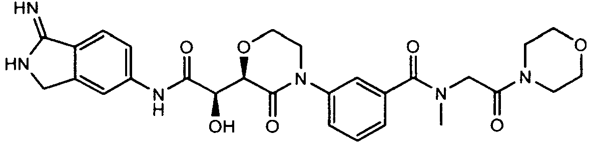 | aa71 | 565.3 |
| 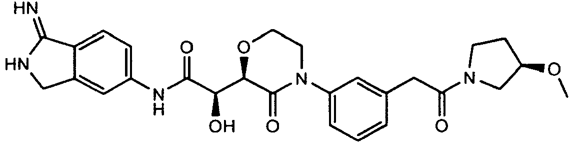 | aa72 | 522.3 |
| 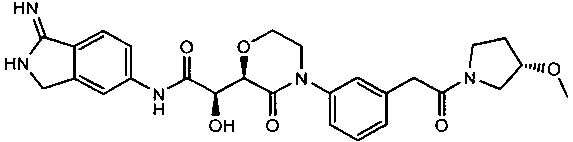 | aa73 | 522.3 |
| 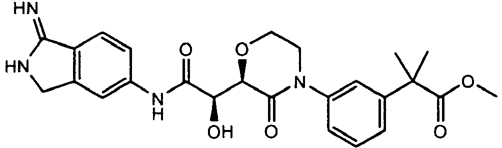 | aa74 | 481.2 |
| 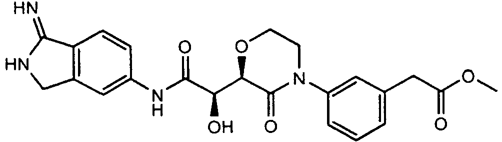 | aa75 | 453.2 |
| 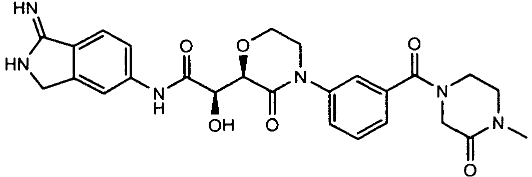 | aa76 | 521.3 |
| 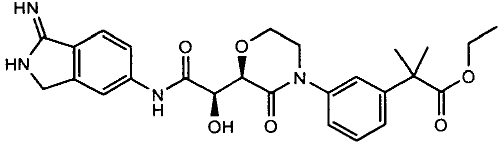 | aa77 | 495.2 |
| 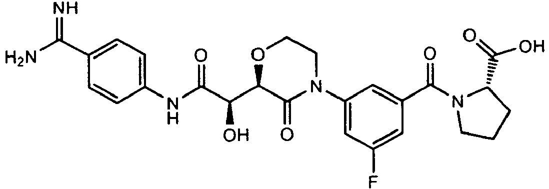 | aa78 | 528.2 |
| 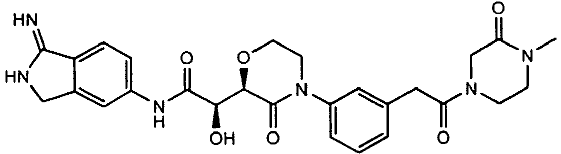 | aa79 | 535.3 |
| 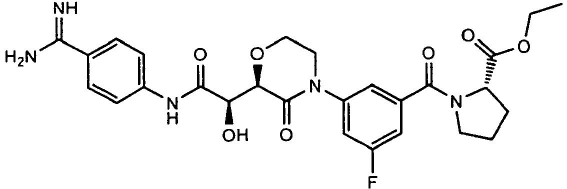 | aa80 | 556.2 |
| 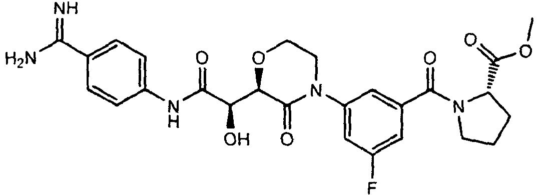 | aa81 | 542.2 |
| 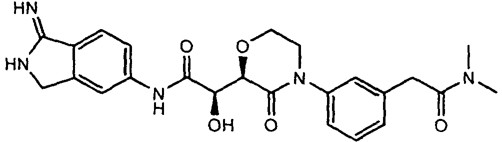 | aa82 | 466.3 |
| 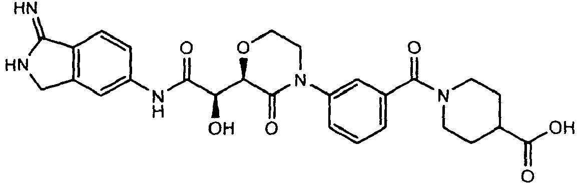 | aa83 | 536.2 |
| 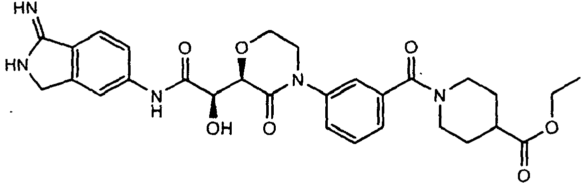 | aa84 | 564.2 |
| 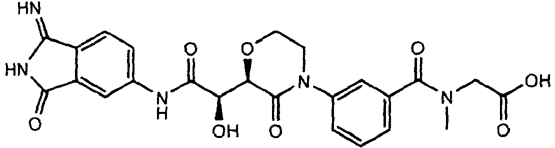 | aa85 | 510.2 |
| 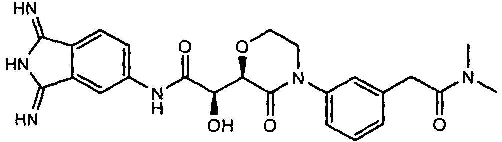 | aa86 | 479.3 |
| 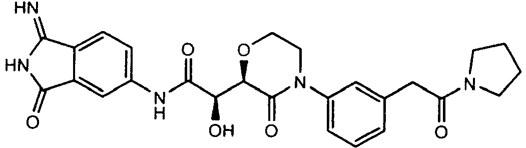 | aa87 | 506.3 |
| 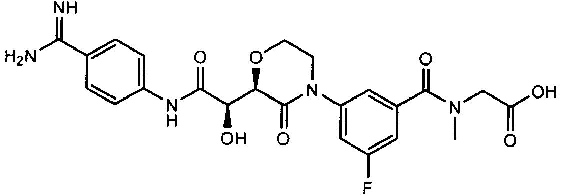 | aa88 | 502.2 |
| 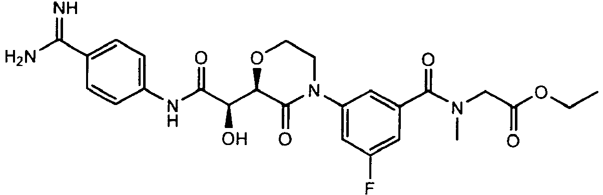 | aa89 | 530.2 |
| 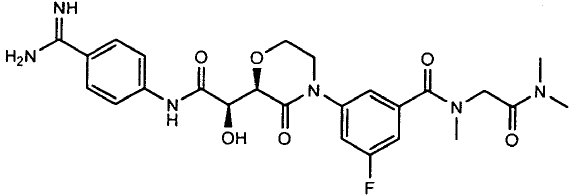 | aa90 | 529.3 |
| 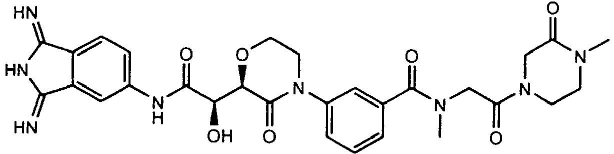 | aa91 | 605.3 |
| 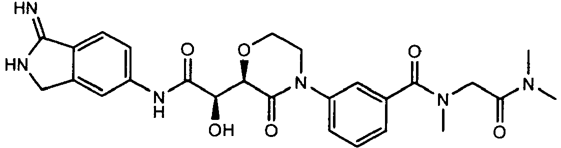 | aa92 | 523.3 |
| 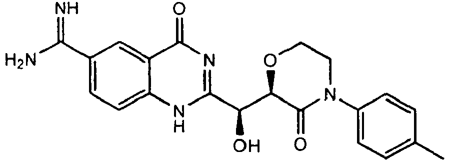 | aa93 | 408.2 |
| 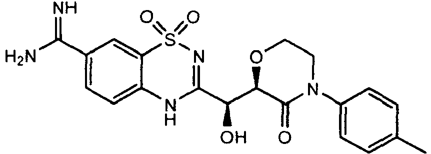 | aa94 | 444.2 |

### (EXAMPLE aa39)

### Preparation of (R)-N-(4-carbamimidoylphenyl)-2-hydroxy-2-((R)-4-(3-((S)-2-methylpyrrolidine-1-car bonyl)phenyl)-3-oxomorpholin-2-yl)acetamide EXAMPLE aa39

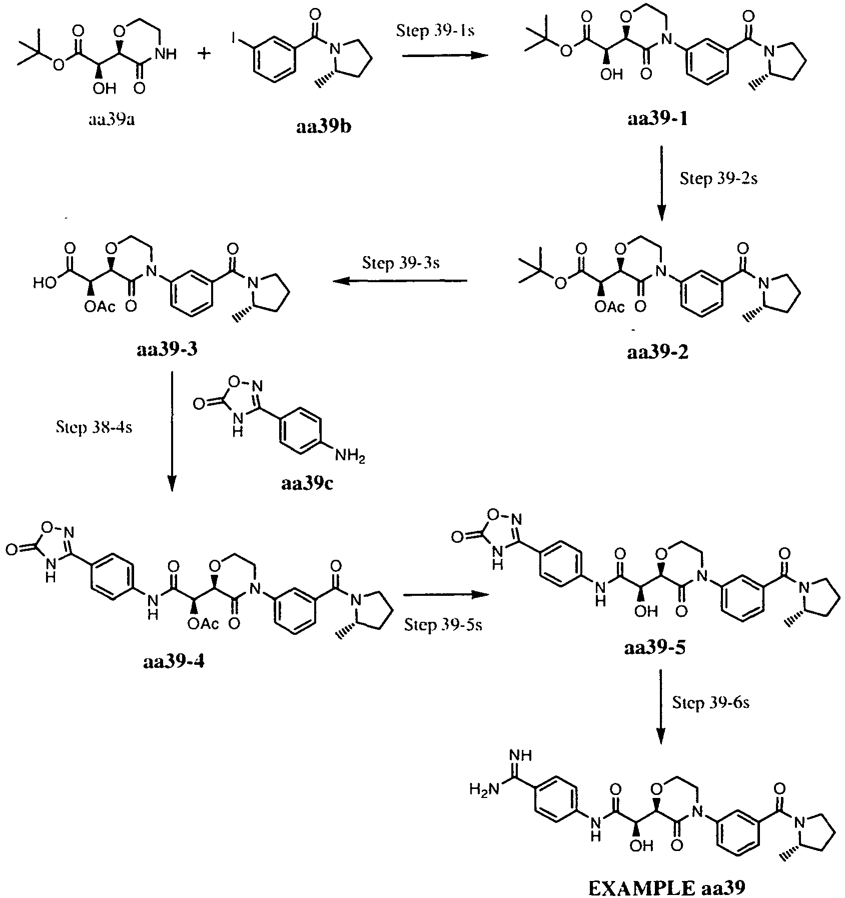

### [Step 39-1s]

To a solution of aa**39a** (150 mg, 0.65 mmol) in anhydrous DMSO (8 mL) under a nitrogen atmosphere was added aa**39b** (225 mg, 0.71 mmol), potassium phosphate (275 mg, 1.30 mmol), copper (I) iodide (12 mg, 0.063 mmol) and trans-N,N'-dimethylcyclohexane-1,2-diamine (18 mg, 0.13 mmol). The reaction mixture was heated at 80 °C for 2 hours. Ethyl acetate (100 mL) was added and the organic layer was washed with water and brine. The organic layer was dried over anhydrous sodium sulfate. The organic solvent was evaporated under reduced pressure. The crude product was purified by flash column chromatography to afford the desired aa**39-1** (170 mg, 0.41 mmol).

### [Step 39-2s]

To a solution of aa**39-1** (170 mg, 0.41 mmol) in anhydrous dichloromethane (6 mL) under a nitrogen atmosphere was added acetic anhydride (84 mg. 0.82 mmol), DMAP (5.0 mg, 0.041 mmol) and triethylamine (124 mg, 1.23 mmol). The reaction mixture was stirred at room temperature for 1 hour. Ethyl acetate (100 mL) was added and the organic layer was washed with water and brine. The organic layer was dried over anhydrous sodium sulfate. The organic solvent was evaporated under reduced pressure. The crude product was purified by flash column chromatography to afford the desired aa**39-2** (179 mg, 0.39 mmol).

### [Step 39-2s]

To aa**39-2** (179 mg, 0.39 mmol) was added a 50% solution of trifluoroacetic acid in dichloromethane (8 mL). The reaction mixture was stirred at room temperature for 16 hours. The organic solvent was evaporated under reduced pressure to afford the desired aa**39-3** (0.39 mmol) which was used in the next step without further purification.

### [Step 39-4s]

To a solution of aa**39-3** (0.39 mmol) in acetonitrile (8 mL) was added aa**39c** (114 mg, 0.64 mmol). EDCI (107 mg, 0.56 mmol) and DMAP (5 mg, 0.041 mmol). The reaction mixture was stirred at room temperature for 2 hour. The organic solvent was evaporated under reduced pressure. The crude product was purified by flash column chromatography to afford the desired aa**39-4** (208 mg, 0.37 mmol).

### [Step 39-5s]

To aa**39-4** (208 mg, 0.37 mmol) was added a solution of 7 N ammonia in methanol (8 mL). The reaction mixture was stirred at room temperature for 40 minutes. The organic solvent was evaporated under reduced pressure to afford the desired aa**39-5** (0.37 mmol) which was used in the next step without further purification.

### [Step 39-6s]

To a solution of aa**39-5** (0.37 mmol) in a 50% solution of 1 N hydrochloric acid in methanol (5 mL) was added palladium-charcoal (10%, 200 mg). The reaction mixture was stirred at room temperature under a hydrogen atmosphere for 16 hours. The reaction mixture was filtered. The filtrate was evaporated under reduced pressure. The crude product was purified by RP-HPLC to afford the desired **EXAMPLE aa39** (171 mg, 0.36 mmol) as a white amorphous solid.

### (EXAMPLE aa40)

### Preparation of methyl 2-(3-((R)-2-((R)-2-(4-carbamimidoylphenylamino)-1-hydroxy-2-oxoethyl)-3-oxomorp holino)phenyl)acetate EXAMPLE aa40.

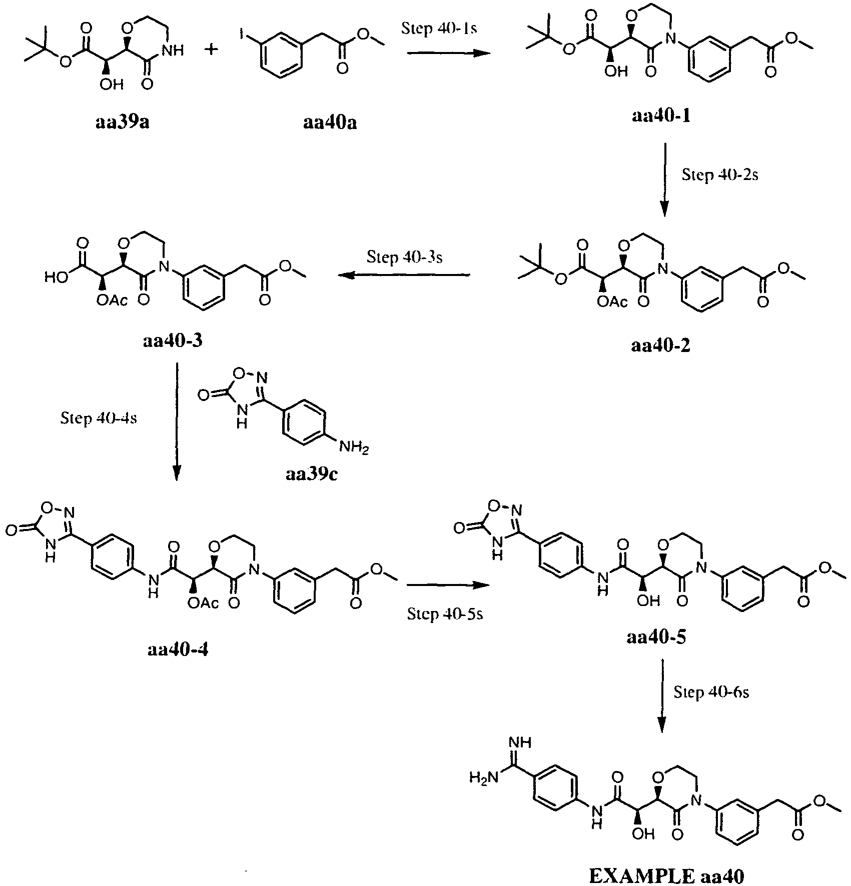

### [Step 40-1s]

According to Step 39-1s in the synthetic method for **EXAMPLE aa39,** compound aa**40a** (420 mg, 1.52 mmol) was used instead of compound aa**39b** to obtain compound aa**40-1** (468 mg, 1.23 mmol).

### [Step 40-2s]

According to Step 39-2s in the synthetic method for **EXAMPLE aa39,** compound aa**40-1** (468 mg, 1.23 mmol) was used instead of compound aa**39-1** to obtain compound aa**40-2** (484 mg, 1.15 mmol).

### [Step 40-3s]

According to Step 39-3s in the synthetic method for **EXAMPLE aa39,** compound aa**40-2** (332 mg, 0.79 mmol) was used instead of compound aa**39-2** to obtain compound aa**40-3** (0.79 mmol) which was used in the next step without further purification.

### [Step 40-4s]

According to Step 39-4s in the synthetic method for **EXAMPLE aa39,** compound aa**40-3** (0.79 mmol) was used instead of compound aa**39-3** to obtain compound aa**40-4** (381 mg, 0.73 mmol).

### [Step 40-5s]

According to Step 39-5s in the synthetic method for **EXAMPLE aa39,** compound aa**40-4** (190 mg, 0.36 mmol) was used instead of compound aa**39-4** to obtain compound aa**40-5** (93 mg, 0.19 mmol) which was used in the next step without further purification.

### [Step 40-6s]

According to Step 39-6s in the synthetic method for **EXAMPLE aa39,** compound aa**40-5** (93 mg, 0.19 mmol) was used instead of compound aa**39-5** to obtain **EXAMPLE aa40** (65 mg, 0.15 mmol) as a white amorphous solid.

### (EXAMPLE aa41)

### Preparation of (R)-N-(4-carbamimidoylphenyl)-2-hydroxy-2-((R)-4-(2-methyl-3-(morpholine-4-carbo nyl)phenyl)-3-oxomorpholin-2-yl)acetamide EXAMPLE aa41

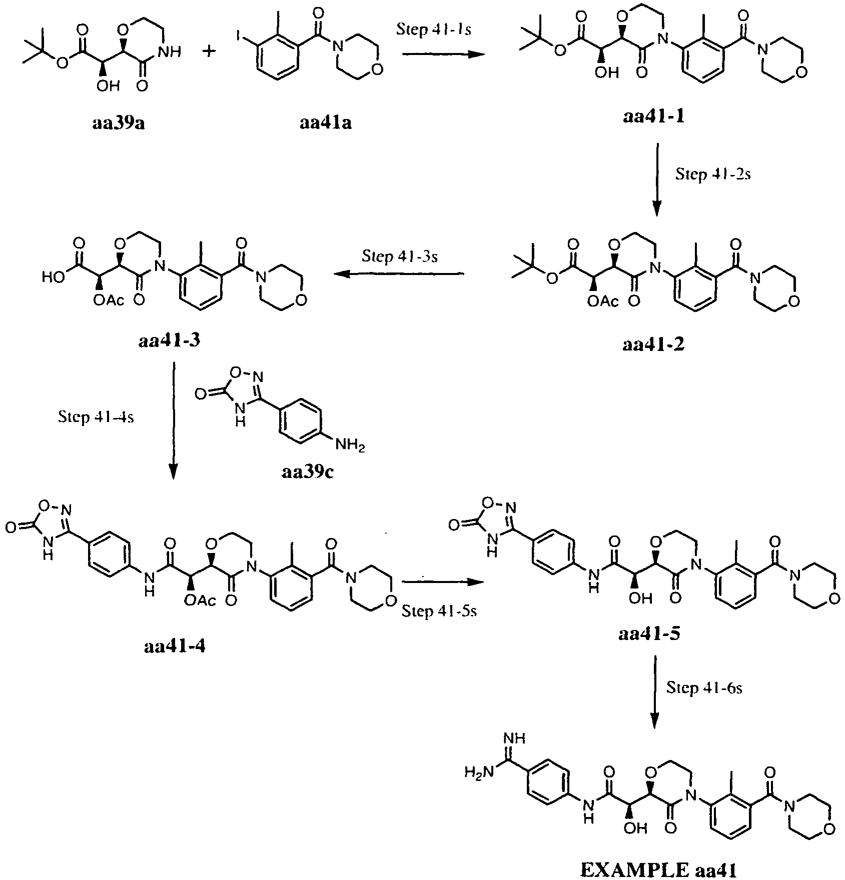

### [Step 41-1s]

According to Step 39-1s in the synthetic method for **EXAMPLE aa39,** compound aa**41a** (236 mg, 0.71 mmol) was used instead of compound aa**39b** to obtain compound aa**41-1** (81 mg, 0.19 mmol).

### [Step 41-2s]

According to Step 39-2s in the synthetic method for **EXAMPLE aa39,** compound aa**41-1** (81 mg, 0.19 mmol) was used instead of compound aa**39-1** to obtain compound aa**40-2** (82 mg, 0.17 mmol).

### [Step 41-3s]

According to Step 39-3s in the synthetic method for **EXAMPLE aa39,** compound aa**41-2** (82 mg, 0.17 mmol) was used instead of compound aa**39-2** to obtain compound aa41-3 (0.17 mmol) which was used in the next step without further purification.

### [Step 41-4s]

According to Step 39-4s in the synthetic method for **EXAMPLE aa39,** compound aa**41-3** (0.17 mmol) was used instead of compound aa**39-3** to obtain compound aa**41-4** (50 mg, 0.086 mmol).

### [Step 41-5s]

According to Step 39-5s in the synthetic method for **EXAMPLE aa39,** compound aa**41-4** (50 mg, 0.086 mmol) was used instead of compound aa**39-4** to obtain compound aa**41-5** (0.086 mmol) which was used in the next step without further purification.

### [Step 41-6s]

According to Step 39-6s in the synthetic method for **EXAMPLE aa39,** compound aa**41-5** (0.086 mmol) was used instead of compound aa**39-5** to obtain **EXAMPLE aa41** (21 mg, 0.042 mmol) as a white amorphous solid.

### (EXAMPLE aa42)

### Preparation of (R)-N-(4-carbamimidoylphenyl)-2-hydroxy-2-((R)-4-(2-methyl-3-(pyrrolidine-1-carbo nyl)phenyl)-3-oxomorpholin-2-yl)acetamide EXAMPLE aa42

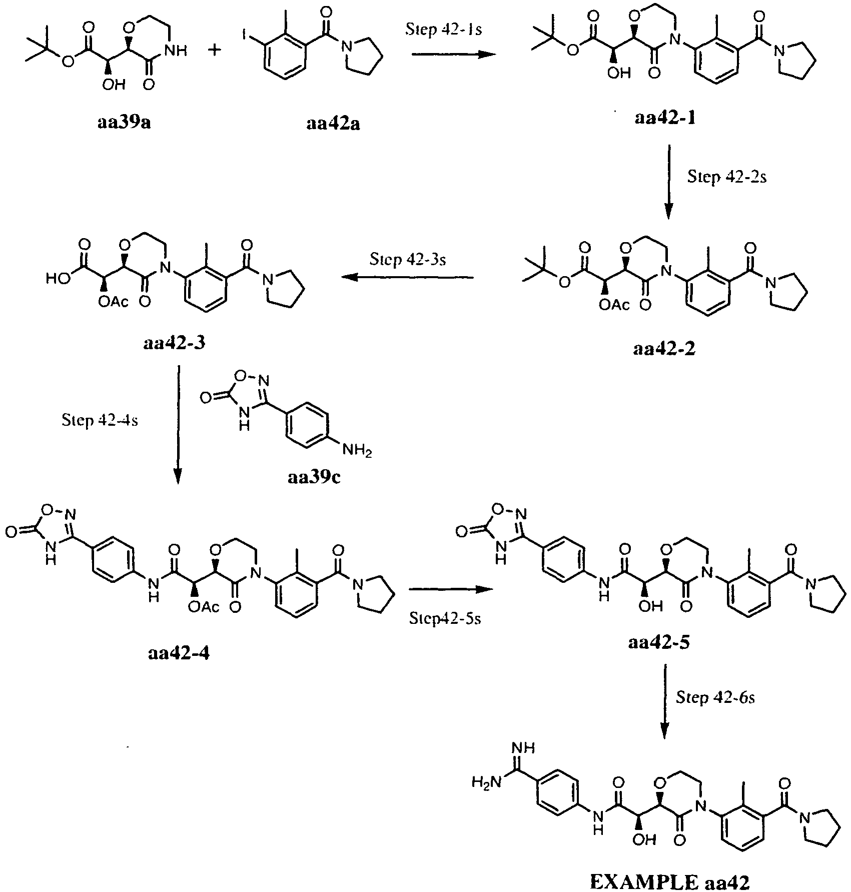

### [Step 42-1s]

According to Step 39-1s in the synthetic method for **EXAMPLE aa39,** compound aa**42a** (225 mg, 0.71 mmol) was used instead of compound aa**39b** to obtain compound aa**42-1** (92 mg, 0.22 mmol).

### [Step 42-2s]

According to Step 39-2s in the synthetic method for **EXAMPLE aa39,** compound aa**42-1** (92 mg, 0.22 mmol) was used instead of compound aa**39-1** to obtain compound aa**42-2** (88 mg, 0.19 mmol).

### [Step 42-3s]

According to Step 39-3s in the synthetic method for **EXAMPLE aa39,** compound aa**42-2** (88 mg, 0.19 mmol) was used instead of compound aa**39-2** to obtain compound aa**42-3** (0.19 mmol) which was used in the next step without further purification.

### [Step 42-4s]

According to Step 39-4s in the synthetic method for **EXAMPLE aa39**, compound aa**42-3** (0.19 mmol) was used instead of compound aa**39-3** to obtain compound aa**42-4** (70 mg, 0.12 mmol).

### [Step 42-5s]

According to Step 39-5s in the synthetic method for **EXAMPLE aa39**, compound aa**42-4** (70 mg, 0.12 mmol) was used instead of compound aa**39-4** to obtain compound aa42-5 (0.12 mmol) which was used in the next step without further purification.

### [Step 42-6s]

According to Step 39-6s in the synthetic method for **EXAMPLE aa39,** compound aa**42-5** (0.12 mmol) was used instead of compound aa**39-5** to obtain **EXAMPLE aa42** (28 mg, 0.058 mmol) as a white amorphous solid.

### (EXAMPLE aa43)

### Preparation of (R)-2-((R)-4-(3-(azetidine-1-carbonyl)-5-fluorophenyl)-3-oxomorpholin-2-yl)-N-(4-car bamimidoylphenyl)-2-hydroxyacetamide EXAMPLE aa43

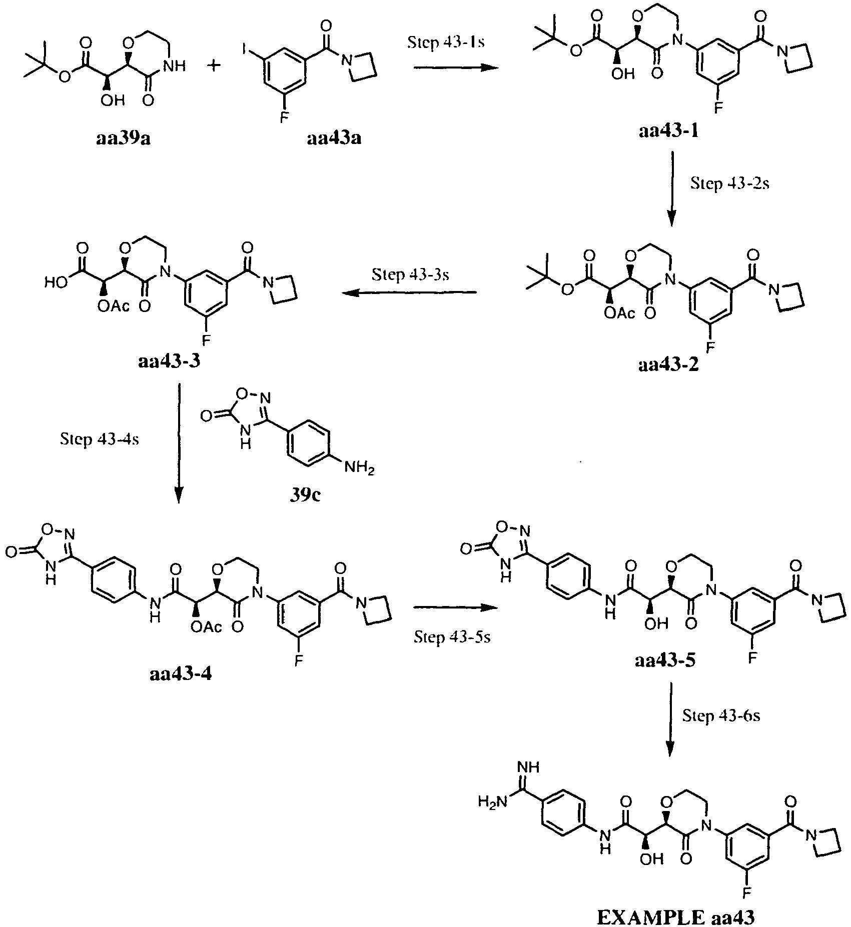

### [Step 43-1s]

According to Step 39-1s in the synthetic method for **EXAMPLE aa39,** compound aa**42a** (290 mg, 0.95 mmol) was used instead of compound aa**39b** to obtain compound aa**43-1** (243 mg, 0.60 mmol).

### [Step 43-2s]

According to Step 39-2s in the synthetic method for **EXAMPLE aa39,** compound aa**43-1** (243 mg, 0.60 mmol) was used instead of compound aa**39-1** to obtain compound aa**43-2** (0.60 mmol).

### [Step 43-3s]

According to Step 39-3s in the synthetic method for **EXAMPLE aa39,** compound aa**43-2** (0.60 mmol) was used instead of compound aa**39-2** to obtain compound aa**43-3** (0.60 mmol) which was used in the next step without further purification.

### [Step 43-4s]

According to Step 39-4s in the synthetic method for **EXAMPLE aa39**, compound aa**43-3** (0.60 mmol) was used instead of compound aa**39-3** to obtain compound aa**43-4** (316 mg, 0.57 mmol).

### [Step 43-5s]

According to Step 39-5s in the synthetic method for **EXAMPLE aa39**, compound aa**43-4** (316 mg, 0.57 mmol) was used instead of compound aa39-4 to obtain compound aa**43-5** (0.57 mmol) which was used in the next step without further purification.

### [Step 43-6s]

According to Step 39-6s in the synthetic method for **EXAMPLE aa39**, compound aa**43-5** (0.57 mmol) was used instead of compound aa**39-5** to obtain **EXAMPLE aa43** (237 mg, 0.51 mmol) as a white amorphous solid.

### (EXAMPLE aa44)

### Preparation of (R)-2-((R)-4-(3-(azetidine-1-carbonyl)-2-methylphenyl)-3-oxomorpholin-2-yl)-N-(4-carbamimidoylphenyl)-2-hydroxyacetamide EXAMPLE aa44

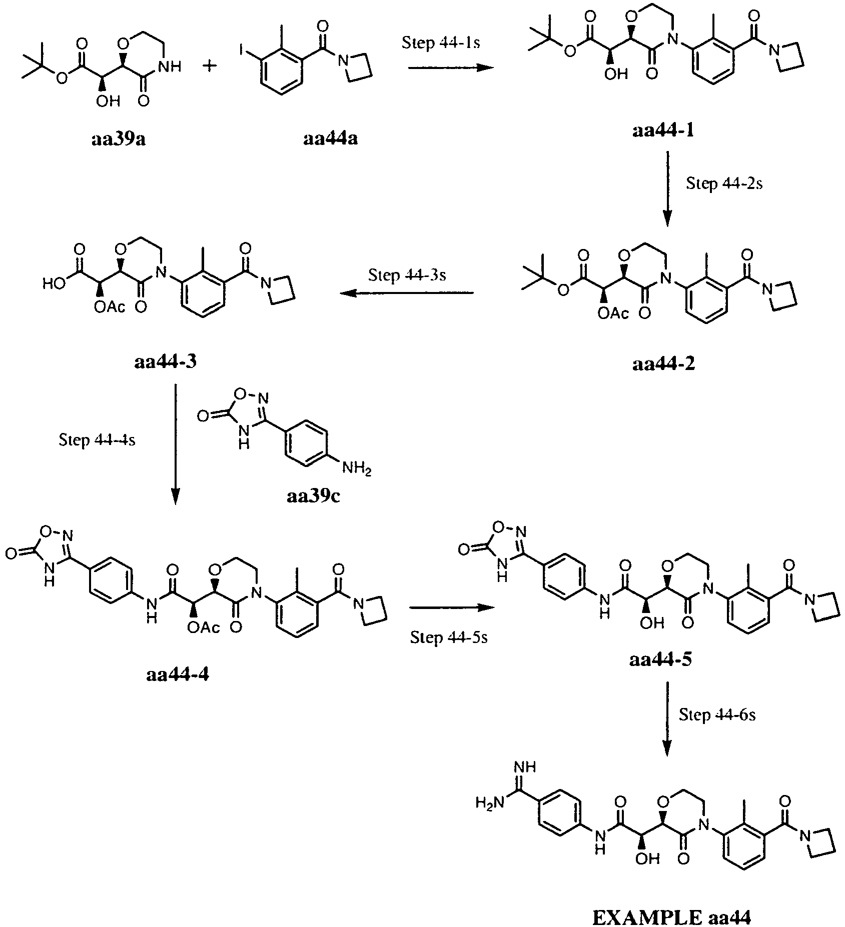

### [Step 44-1s]

According to Step 39-1s in the synthetic method for **EXAMPLE aa39**. compound aa**44a** (300 mg, 1.00 mmol) was used instead of compound aa**39b** to obtain compound aa**44-1** (129 mg, 0.32 mmol).

### [Step 44-2s]

According to Step 39-2s in the synthetic method for **EXAMPLE aa39**, compound aa**44-1** (129 mg, 0.32 mmol) was used instead of compound aa39-1 to obtain compound aa**44-2** (144 mg, 0.32 mmol).

### [Step 44-3s]

According to Step 39-3s in the synthetic method for **EXAMPLE aa39**, compound aa**44-2** (144 mg, 0.32 mmol) was used instead of compound aa39-2 to obtain compound aa**44-3** (0.32 mmol) which was used in the next step without further purification.

### [Step 44-4s]

According to Step 39-4s in the synthetic method for **EXAMPLE aa39,** compound aa**44-3** (0.32 mmol) was used instead of compound aa**39-3** to obtain compound aa**44-4** (48 mg, 0.087 mmol).

### [Step 44-5s]

According to Step 39-5s in the synthetic method for **EXAMPLE aa39,** compound aa**44-4** (48 mg, 0.087 mmol) was used instead of compound aa**39-4** to obtain compound aa**44-5** (0.087 mmol) which was used in the next step without further purification.

### [Step 44-6s]

According to Step 39-6s in the synthetic method for **EXAMPLE aa39,** compound aa**44-5** (0.087 mmol) was used instead of compound aa**39-5** to obtain **EXAMPLE aa44** (33 mg, 0.071 mmol) as a white amorphous solid.

### (EXAMPLE aa45)

### Preparation of (R)-2-((R)-4-(5-(azetidine-1-carbonyl)-2-methylphenyl)-3-oxomorpholin-2-yl)-N-(4-carbamimidoylphenyl)-2-hydroxyacetamide EXAMPLE aa45

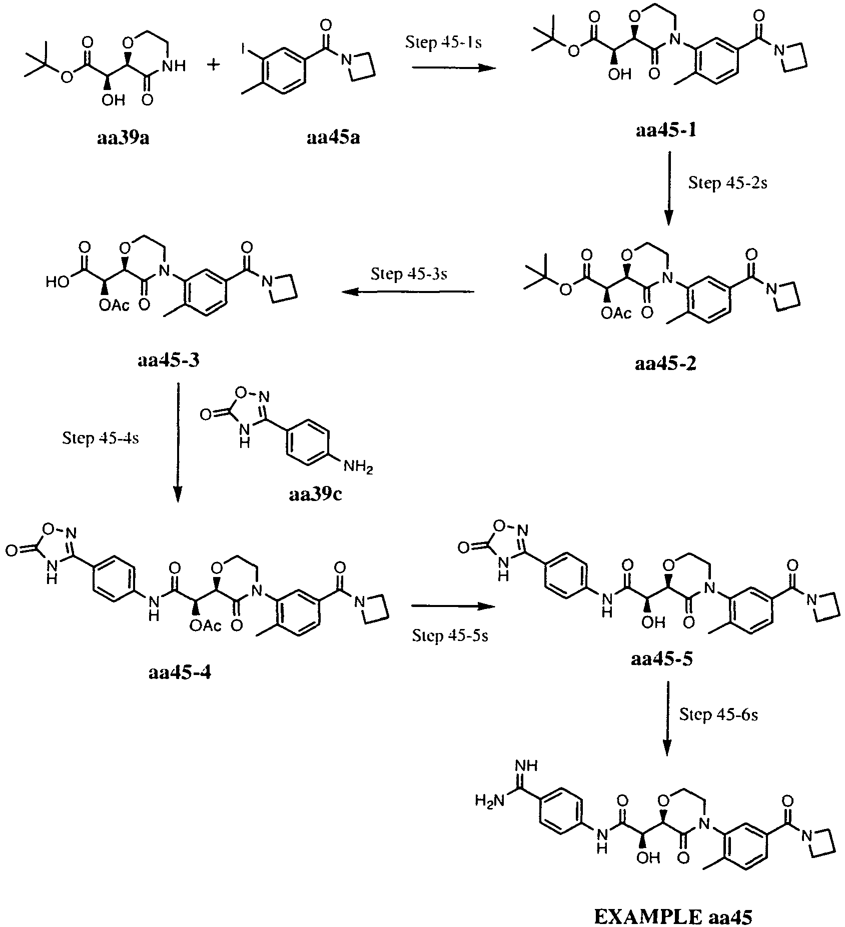

### [Step 45-1s]

According to Step 39-1s in the synthetic method for **EXAMPLE aa39,** compound aa**45a** (282 mg, 0.94 mmol) was used instead of compound aa**39b** to obtain compound aa**45-1** (186 mg, 0.46 mmol).

### [Step 45-2s]

According to Step 39-2s in the synthetic method for **EXAMPLE aa39,** compound aa**45-1** (186 mg, 0.46 mmol) was used instead of compound aa**39-1** to obtain compound aa**45-2** (96 mg, 0.22 mmol).

### [Step 45-3s]

According to Step 39-3s in the synthetic method for **EXAMPLE aa39,** compound aa**45-2** (96 mg, 0.22 mmol) was used instead of compound aa39-2 to obtain compound aa**45-3** (0.22 mmol) which was used in the next step without further purification.

### [Step 45-4s]

According to Step 39-4s in the synthetic method for **EXAMPLE aa39,** compound aa**45-3** (0.22 mmol) was used instead of compound aa**39-3** to obtain compound aa**45-4** (117 mg, 0.21 mmol).

### [Step 45-5s]

According to Step 39-5s in the synthetic method for **EXAMPLE aa39,** compound aa**45-4** (117 mg, 0.21 mmol) was used instead of compound aa**39-4** to obtain compound aa**45-5** (0.21 mmol) which was used in the next step without further purification.

### [Step 45-6s]

According to Step 39-6s in the synthetic method for **EXAMPLE aa39,** compound aa**45-5** (0.21 mmol) was used instead of compound aa39-5 to obtain **EXAMPLE aa45** (48 mg, 0.10 mmol) as a white amorphous solid.

### (EXAMPLE aa46)

### Preparation of (R)-N-(4-carbamimidoylphenyl)-2-hydroxy-2-((R)-3-oxo-4-(6-(trifluoromethyl)pyridin-2-yl)morpholin-2-yl)acetamide EXAMPLE aa46

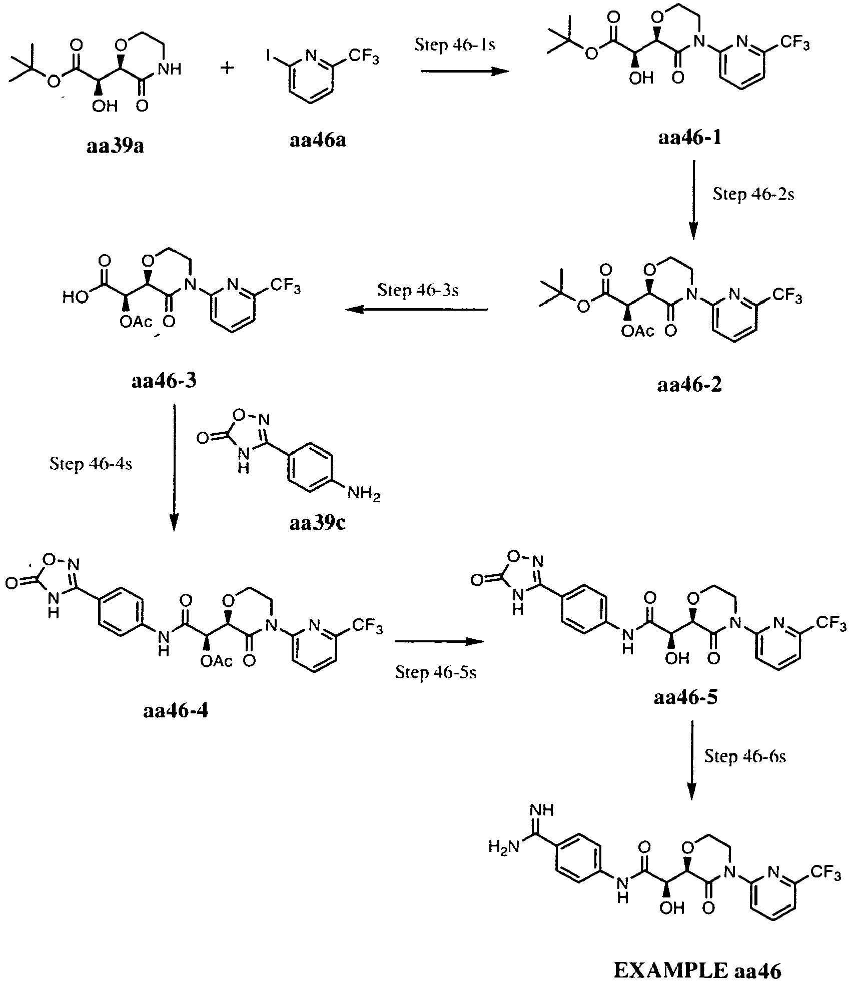

### [Step 46-1s]

According to Step 39-1s in the synthetic method for **EXAMPLE aa39,** compound aa**46a** (500 mg, 1.83 mmol) was used instead of compound aa**39b** to obtain compound aa**46-1** (380 mg, 1.01 mmol).

### [Step 46-2s]

According to Step 39-2s in the synthetic method for **EXAMPLE aa39,** compound aa**46-1** (380 mg, 1.01 mmol) was used instead of compound aa**39-1** to obtain compound aa**46-2** (1.01 mmol).

### [Step 46-3s]

According to Step 39-3s in the synthetic method for **EXAMPLE aa39,** compound aa**46-2** (222 mg, 0.53 mmol) was used instead of compound aa**39-2** to obtain compound aa**46-3** (0.53 mmol) which was used in the next step without further purification.

### [Step 46-4s]

According to Step 39-4s in the synthetic method for **EXAMPLE aa39,** compound aa**46-3** (0.53 mmol) was used instead of compound aa39-3 to obtain compound aa**46-4** (155 mg, 0.30 mmol).

### [Step 46-5s]

According to Step 39-5s in the synthetic method for **EXAMPLE aa39,** compound aa**46-4** (155 mg, 0.30 mmol) was used instead of compound aa**39-4** to obtain compound aa**46-5** (0.30 mmol) which was used in the next step without further purification.

### [Step 46-6s]

According to Step 39-6s in the synthetic method for **EXAMPLE aa39,** compound aa**46-5** (0.30 mmol) was used instead of compound aa**39-5** to obtain **EXAMPLE aa46** (59 mg, 0.14 mmol) as a white amorphous solid.

### (EXAMPLE aa47)

Preparation of (R)-N-(4-carbamimidoylphenyl)-2-hydroxy-2-((R)-4-(2-methyl-5-(pyrrolidine-1-carbonyl)phenyl)-3-oxomorpholin-2-yl)acetamide **EXAMPLE aa47**

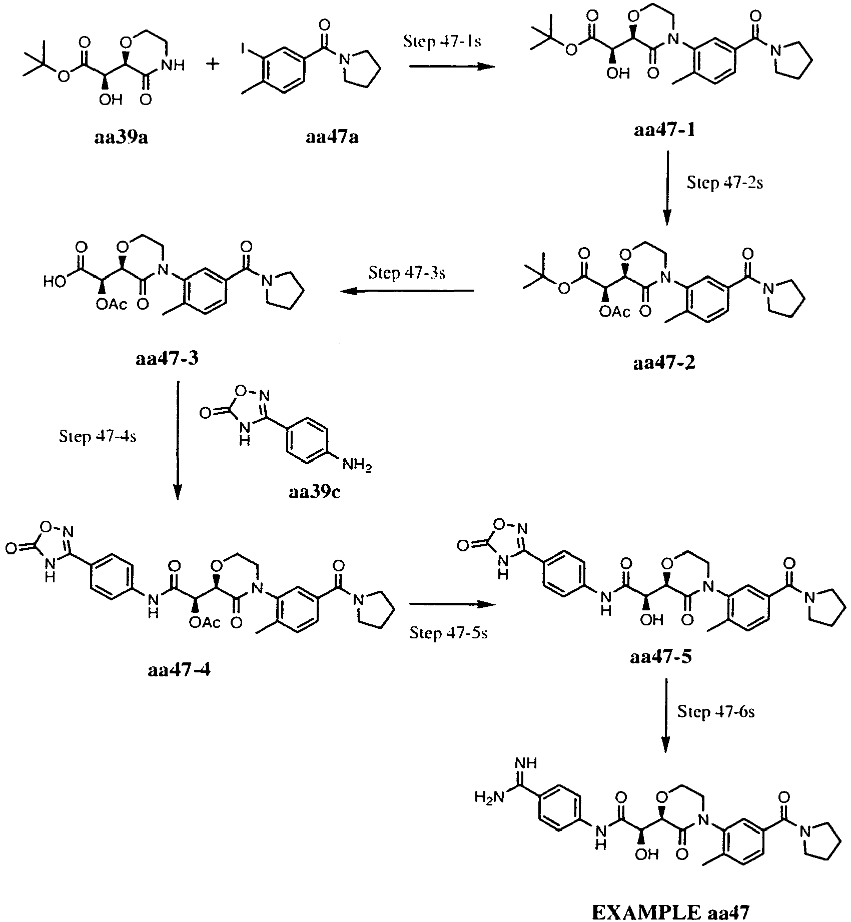

### [Step 47-1s]

According to Step 39-1s in the synthetic method for **EXAMPLE aa39,** compound aa**47a** (300 mg, 0.95 mmol) was used instead of compound aa39b to obtain compound aa**47-1** (338 mg, 0.81 mmol).

### [Step 47-2s]

According to Step 39-2s in the synthetic method for **EXAMPLE aa39,** compound aa**47-1** (338 mg, 0.81 mmol) was used instead of compound aa**39-1** to obtain compound aa**47-2** (146 mg, 0.32 mmol).

### [Step 47-3s]

According to Step 39-3s in the synthetic method for **EXAMPLE aa39,** compound aa**47-2** (146 mg, 0.32 mmol) was used instead of compound aa39-2 to obtain compound aa**47-3** (0.32 mmol) which was used in the next step without further purification.

### [Step 47-4s]

According to Step 39-4s in the synthetic method for **EXAMPLE aa39,** compound aa**47-3** (0.32 mmol) was used instead of compound aa**39-3** to obtain compound aa**47-4** (163 mg, 0.29 mmol).

### [Step 47-5s]

According to Step 39-5s in the synthetic method for **EXAMPLE aa39,** compound aa**47-4** (163 mg, 0.29 mmol) was used instead of compound aa39-4 to obtain compound aa**47-5** (0.29 mmol) which was used in the next step without further purification.

### [Step 47-6s]

According to Step 39-6s in the synthetic method for **EXAMPLE aa39,** compound aa**47-5** (0.29 mmol) was used instead of compound aa**39-5** to obtain **EXAMPLE aa47** (115 mg, 0.24 mmol) as a white amorphous solid.

### (EXAMPLE aa48)

### Preparation of (R)-2-((R)-4-(3-(azetidine-1-carbonyl)-5-methylphenyl)-3-oxomorpholin-2-yl)-N-(4-carbamimidoylphenyl)-2-hydroxyacetamide EXAMPLE aa48

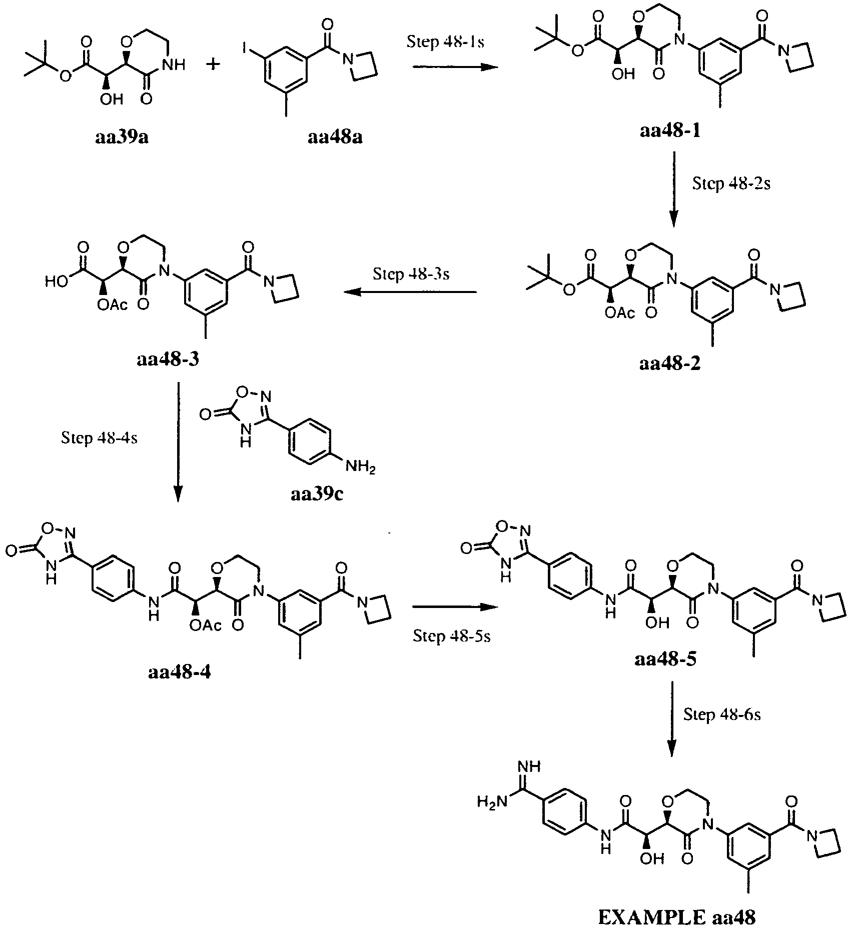

### [Step 48-1s]

According to Step 39-1s in the synthetic method for **EXAMPLE aa39,** compound aa**48a** (205 mg, 0.68 mmol) was used instead of compound aa**39b** to obtain compound aa**48-1** (222 mg, 0.55 mmol).

### [Step 48-2s]

According to Step 39-2s in the synthetic method for **EXAMPLE aa39,** compound aa**48-1** (222 mg, 0.55 mmol) was used instead of compound aa**39-1** to obtain compound aa**48-2** (219 mg, 0.49 mmol).

### [Step 48-3s]

According to Step 39-3s in the synthetic method for **EXAMPLE aa39,** compound aa**48-2** (219 mg, 0.49 mmol) was used instead of compound aa**39-2** to obtain compound aa**48-3** (0.49 mmol) which was used in the next step without further purification.

### [Step 48-4s]

According to Step 39-4s in the synthetic method for **EXAMPLE aa39,** compound aa**48-3** (0.49 mmol) was used instead of compound aa**39-3** to obtain compound aa48-4 (182 mg, 0.33 mmol).

### [Step 48-5s]

According to Step 39-5s in the synthetic method for **EXAMPLE aa39,** compound aa**48-4** (182 mg, 0.33 mmol) was used instead of compound aa**39-4** to obtain compound aa**48-5** (0.33 mmol) which was used in the next step without further purification.

### [Step 48-6s]

According to Step 39-6s in the synthetic method for **EXAMPLE aa39,** compound aa**48-5** (0.33 mmol) was used instead of compound aa**39-5** to obtain **EXAMPLE aa48** (118 mg, 0.25 mmol) as a white amorphous solid.

### (EXAMPLE aa49)

### Preparation of (R)-N-(4-carbamimidoylphenyl)-2-hydroxy-2-((R)-4-(2-methyl-5-(morpholine-4-carbo nyl)phenyl)-3-oxomorpholin-2-yl)acetamide EXAMPLE aa49

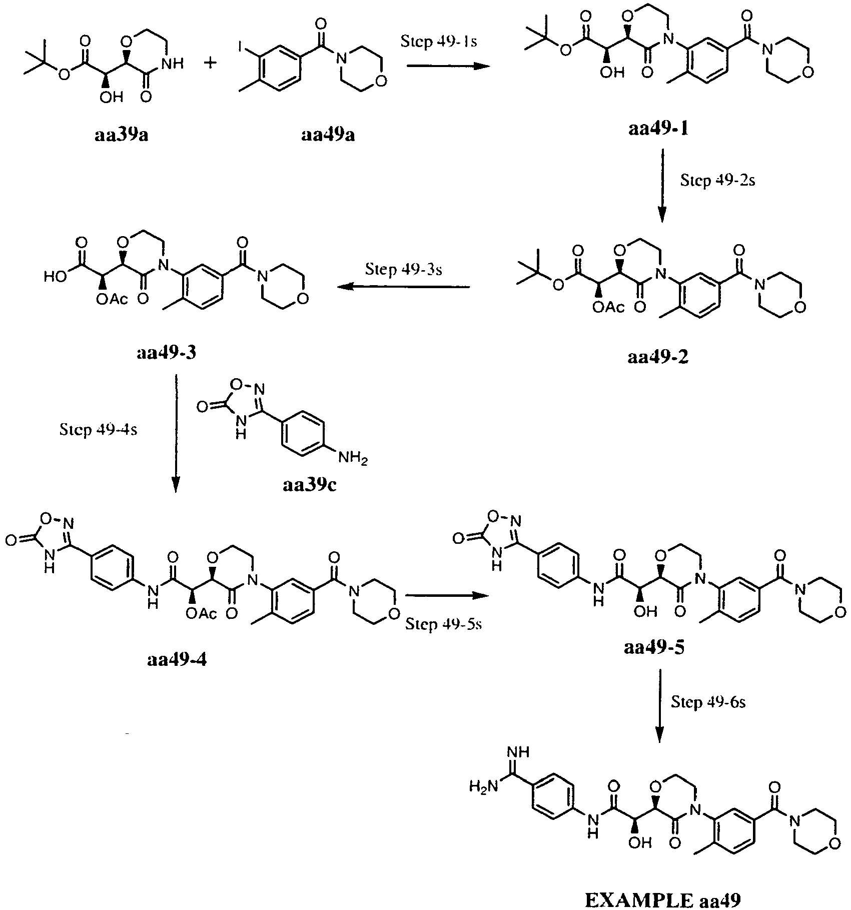

### [Step 49-1s]

According to Step 39-1s in the synthetic method for **EXAMPLE aa39,** compound aa**49a** (315 mg, 0.95 mmol) was used instead of compound aa**39b** to obtain compound aa**49-1** (177 mg, 0.41 mmol).

### [Step 49-2s]

According to Step 39-2s in the synthetic method for **EXAMPLE aa39,** compound aa**49-1** (177 mg, 0.41 mmol) was used instead of compound aa**39-1** to obtain compound aa**49-2** (160 mg, 0.34 mmol).

### [Step 49-3s]

According to Step 39-3s in the synthetic method for **EXAMPLE aa39,** compound aa**49-2** (160 mg, 0.34 mmol) was used instead of compound aa**39-2** to obtain compound aa**49-3** (0.34 mmol) which was used in the next step without further purification.

### [Step 49-4s]

According to Step 39-4s in the synthetic method for **EXAMPLE aa39,** compound aa**49-3** (0.34 mmol) was used instead of compound aa**39-3** to obtain compound aa**49-4** (90 mg, 0.16 mmol).

### [Step 49-5s]

According to Step 39-5s in the synthetic method for **EXAMPLE aa39,** compound aa**49-4** (90 mg, 0.16 mmol) was used instead of compound aa**39-4** to obtain compound aa**49-5** (0.16 mmol) which was used in the next step without further purification.

### [Step 49-6s]

According to Step 39-6s in the synthetic method for **EXAMPLE aa39,** compound aa**49-5** (0.16 mmol) was used instead of compound aa**39-5** to obtain **EXAMPLE aa49** (60 mg, 0.12 mmol) as a white amorphous solid.

### (EXAMPLE aa53)

### Preparation of (R)-N-(4-carbamimidoylphenyl)-2-hydroxy-2-((R)-4-(3-methyl-5-(pyrrolidine-1-carbo nyl)phenyl)-3-oxomorpholin-2-yl)acetamide EXAMPLE aa53

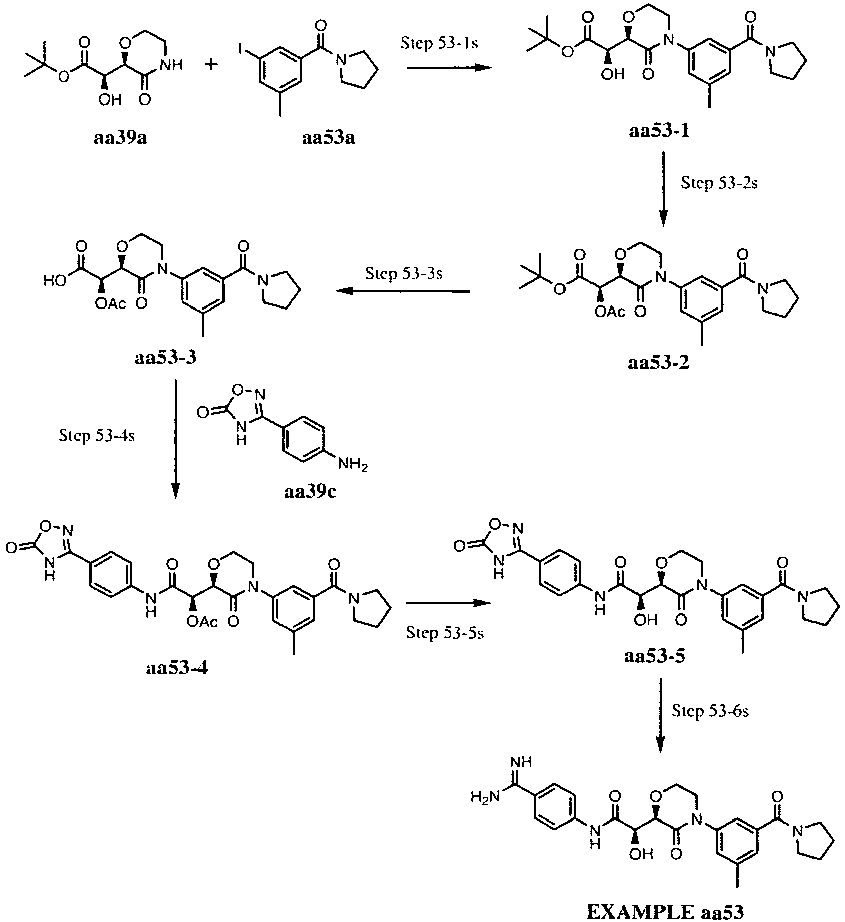

### [Step 53-1s]

According to Step 39-1s in the synthetic method for **EXAMPLE aa39,** compound aa**53a** (225 mg, 0.71 mmol) was used instead of compound aa**39b** to obtain compound aa**53-1** (222 mg, 0.53 mmol).

### [Step 53-2s]

According to Step 39-2s in the synthetic method for **EXAMPLE aa39,** compound aa**53-1** (222 mg, 0.53 mmol) was used instead of compound aa**39-1** to obtain compound aa**53-2** (207 mg, 0.45 mmol).

### [Step 53-3s]

According to Step 39-3s in the synthetic method for **EXAMPLE aa39,** compound aa**53-2** (207 mg, 0.45 mmol) was used instead of compound aa**39-2** to obtain compound aa**53-3** (0.45 mmol) which was used in the next step without further purification.

### [Step 53-4s]

According to Step 39-4s in the synthetic method for **EXAMPLE aa39,** compound aa**53-3** (106 mg, 0.26 mmol) was used instead of compound aa**39-3** to obtain compound aa**53-4** (147 mg, 0.26 mmol).

### [Step 53-5s]

According to Step 39-5s in the synthetic method for **EXAMPLE aa39,** compound aa**53-4** (147 mg, 0.26 mmol) was used instead of compound aa**39-4** to obtain compound aa**53-5** (0.26 mmol) which was used in the next step without further purification.

### [Step 53-6s]

According to Step 39-6s in the synthetic method for **EXAMPLE aa39,** compound aa**53-5** (0.26 mmol) was used instead of compound aa**39-5** to obtain **EXAMPLE aa53** (83 mg, 0.17 mmol) as a white amorphous solid.

### (EXAMPLE aa54)

### Preparation of (R)-2-hydroxy-N-(1-iminoisoindolin-5-yl)-2-((R)-4-(3-methyl-5-(pyrrolidine-1-carbonyl)phenyl)-3-oxomorpholin-2-yl)acetamide EXAMPLE aa54

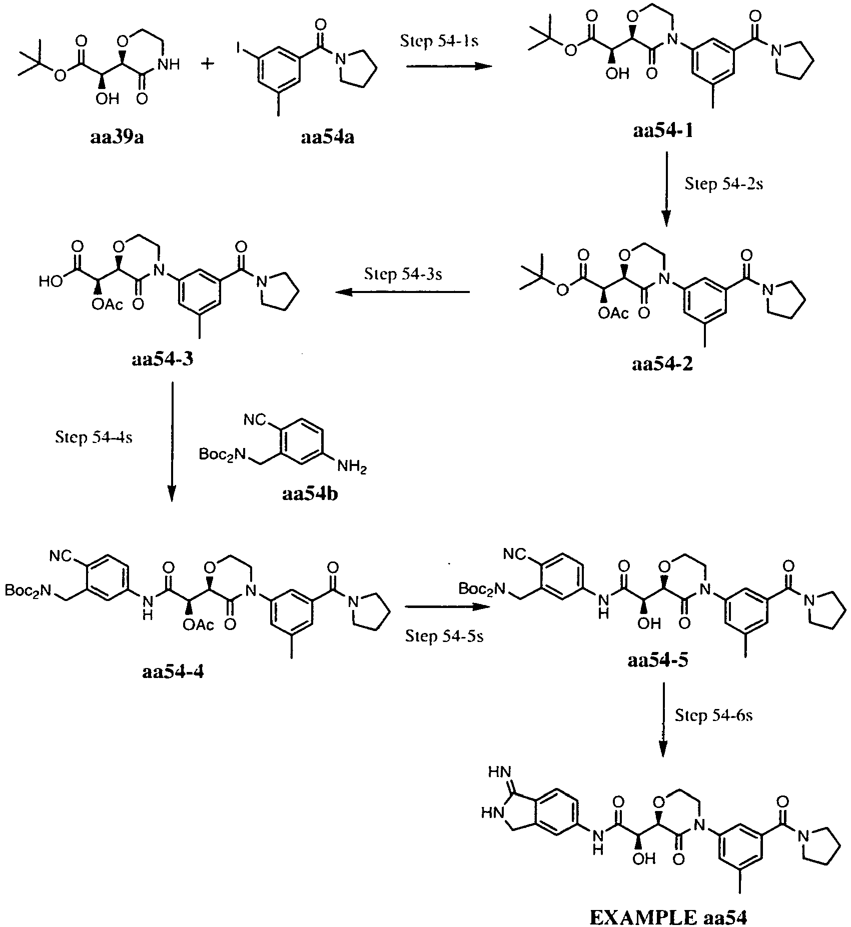

### [Step 54-1s]

According to Step 39-1s in the synthetic method for **EXAMPLE aa39,** compound aa**54a** (225 mg, 0.71 mmol) was used instead of compound aa**39b** to obtain compound aa54-1 (222 mg, 0.53 mmol).

### [Step 54-2s]

According to Step 39-2s in the synthetic method for **EXAMPLE aa39,** compound aa**54-1** (222 mg, 0.53 mmol) was used instead of compound aa**39-1** to obtain compound aa**54-2** (207 mg, 0.45 mmol).

### [Step 54-3s]

According to Step 39-3s in the synthetic method for **EXAMPLE aa39,** compound aa**54-2** (207 mg, 0.45 mmol) was used instead of compound aa**39-2** to obtain compound aa**54-3** (0.45 mmol) which was used in the next step without further purification.

### [Step 54-4s]

According to Step 39-4s in the synthetic method for **EXAMPLE aa39,** compound aa**53-3** (109 mg, 0.27 mmol) was used instead of compound aa39-3 and compound aa**54b** (140 mg, 0.40 mmol) was instead of compound aa39c to obtain compound aa**54-4** (116 mg, 0.16 mmol).

### [Step 54-5s]

According to Step 39-5s in the synthetic method for **EXAMPLE aa39,** compound aa**54-4** (116 mg, 0.16 mmol) was used instead of compound aa**39-4** to obtain compound aa**54-5** (0.16 mmol) which was used in the next step without further purification.

### [Step 54-6s]

To a solution of compound aa**54-5** (0.16 mmol) was added a 4 N solution of hydrogen chloride in dioxane (10 mL). The reaction mixture was stirred at room temperature for 2 hours. The organic solvent was evaporated under reduced pressure. Anhydrous ethanol (12 mL) was added and the reaction mixture was heated under reflux for 16 hours. The organic solvent was evaporated under reduced pressure. The crude product was purified by RP-HPLC to afford the desired **EXAMPLE aa54** (55 mg, 0.11 mmol) as a white amorphous solid.

### (EXAMPLE aa50)

### Preparation of 2-(3-((R)-2-((R)-1-hydroxy-2-(1-iminoisoindolin-5-ylamino)-2-oxoethyl)-3-oxomorph olino)-N-methylbenzamido)acetic acid EXAMPLE aa50

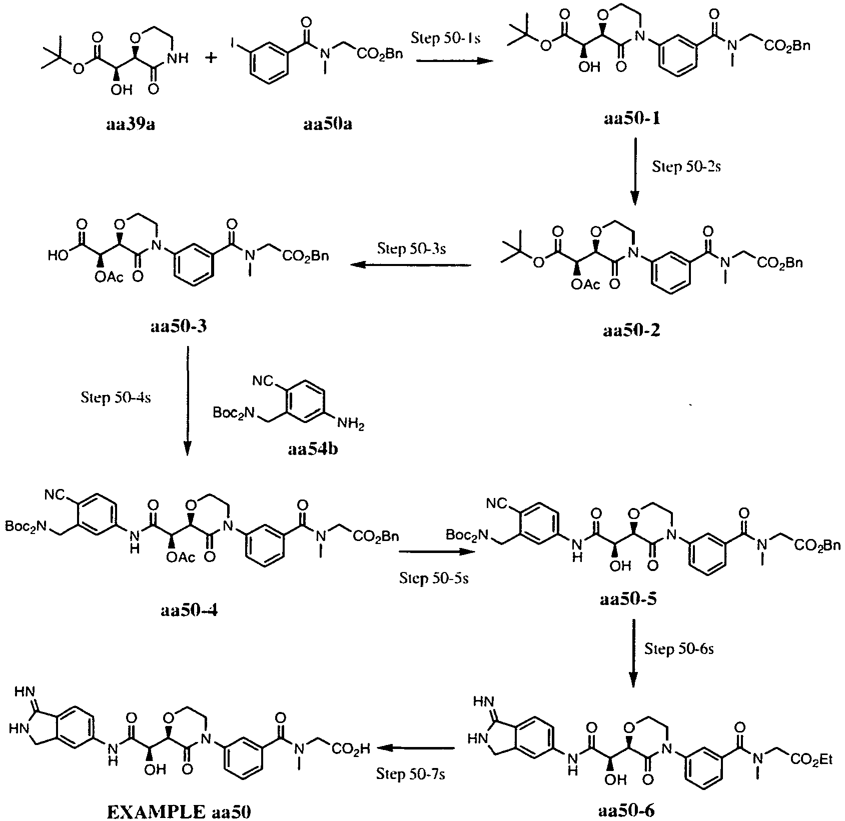

### [Step 50-1s]

According to Step 39-1s in the synthetic method for **EXAMPLE aa39,** compound aa**50a** (1.24 g, 3.03 mmol) was used instead of compound aa**39b** to obtain compound aa**50-1** (1.16 g, 2.27 mmol).

### [Step 50-2s]

According to Step 39-2s in the synthetic method for **EXAMPLE aa39,** compound aa**50-1** (1.16 g, 2.27 mmol) was used instead of compound aa**39-1** to obtain compound aa**50-2** (1.16 g, 2.09 mmol).

### [Step 50-3s]

According to Step 39-3s in the synthetic method for **EXAMPLE aa39,** compound aa**50-2** (250 mg, 0.50 mmol) was used instead of compound aa39-2 to obtain compound aa**50-3** (0.50 mmol) which was used in the next step without further purification.

### I Step 50-4s]

According to Step 54-4s in the synthetic method for **EXAMPLE aa54,** compound aa**50-3** (0.50 mmol) was used instead of compound aa**54-3** to obtain compound aa**50-4** (255 mg, 0.31 mmol).

### [Step 50-5s]

According to Step 39-5s in the synthetic method for **EXAMPLE aa39,** compound aa**50-4** (255 mg, 0.31 mmol) was used instead of compound aa**39-4** to obtain compound aa**40-5** (0.31 mmol) which was used in the next step without further purification.

### [Step 50-6s]

According to Step 54-6s in the synthetic method for **EXAMPLE aa54,** compound aa**50-5** (0.31 mmol) was used instead of compound aa**54-5** to obtain compound aa**50-6** (0.31 mmol) which was used in the next step without further purification.

### I Step 50-7s]

To a solution of compound aa**50-6** (0.31 mmol) in methanol (2 mL) and water (2 mL) was added triethylamine (0.43 mL). The reaction mixture was stirred at room temperature for 16 hours. The organic solvent was evaporated under reduced pressure. The crude product was purified by RP-HPLC to afford the desired **EXAMPLE aa50** (153 mg, 0.31 mmol) as a white amorphous solid.

### (EXAMPLE aa51)

### Preparation of methyl 2-(3-((R)-2-((R)-1-hydroxy-2-(1-iminoisoindolin-5-ylamino)-2-oxoethyl)-3-oxomorpholino)-N-methylbenzamido)acetate EXAMPLE aa51

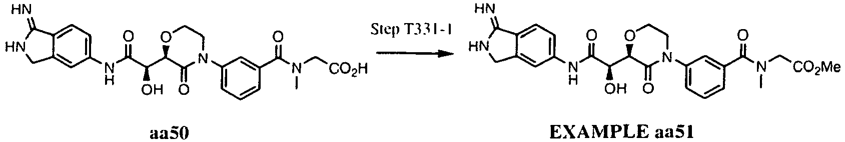

### [Step 51-1s]

To a solution of compound aa**50** (90 mg, 0.18 mmol) in methanol (4 mL) was added 1 N hydrochloric acid (1 mL). The reaction mixture was stirred at room temperature for 3 days. The organic solvent was evaporated under reduced pressure. The crude product was purified by RP-HPLC to afford the desired **EXAMPLE aa51** (48 mg, 0.094 mmol) as a white amorphous solid.

### (EXAMPLE aa58)

### Preparation of (S)-1-(3-((R)-2-((R)-2-(4-carbamimidoylphenylamino)-1-hydroxy-2-oxoethyl)-3-oxomorpholino)benzoyl)pyrrolidine-2-carboxylic acid EXAMPLE aa58

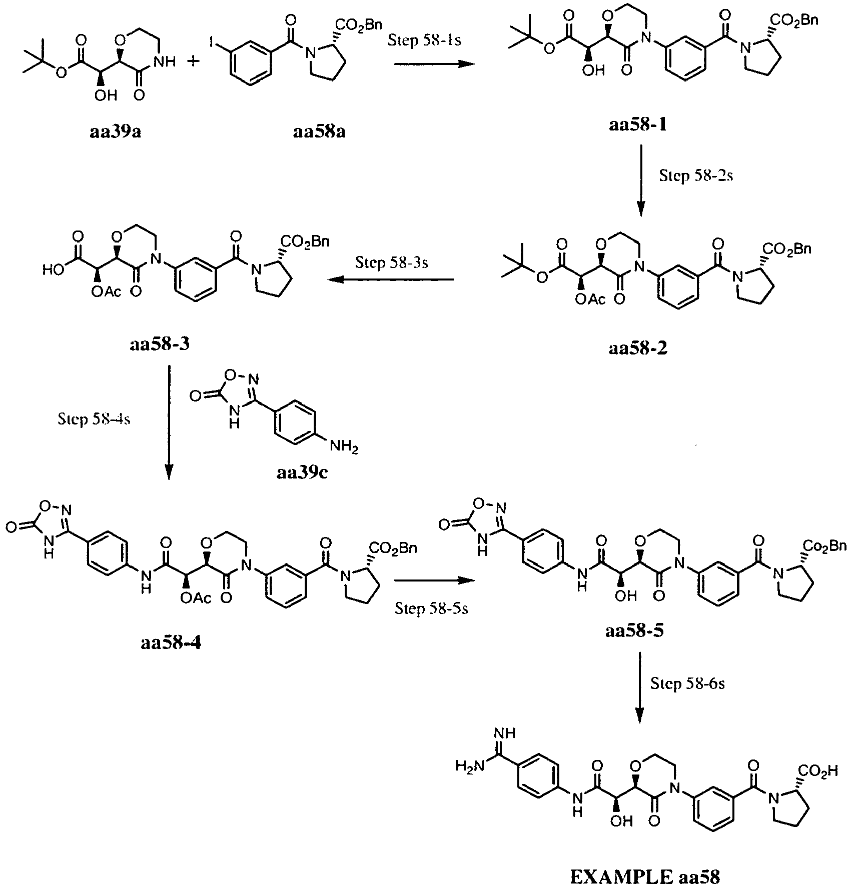

### [Step 58-1s]

According to Step 39-1s in the synthetic method for **EXAMPLE aa39**, compound aa**58a** (616 mg, 1.42 mmol) was used instead of compound aa**39B** to obtain compound aa58-1 (710 mg, 1.32 mmol).

### [Step 58-2s]

According to Step 39-2s in the synthetic method for EXAMPLE **aa39,** compound aa**58-1** (710 mg, 1.32 mmol) was used instead of compound aa**39-1** to obtain compound aa**58-2** (707 mg, 1.22 mmol).

### [Step 58-3s]

According to Step 39-3s in the synthetic method for **EXAMPLE aa39,** compound aa**58-2** (0.66 mmol) was used instead of compound aa39-2 to obtain compound aa**58-3** (0.66 mmol) which was used in the next step without further purification.

### [Step 58-4s]

According to Step 39-4s in the synthetic method for **EXAMPLE aa39,** compound aa**58-3** (0.66 mmol) was used instead of compound aa**39-3** to obtain compound aa**58-4** (445 mg, 0.65 mmol).

### [Step 58-5s]

According to Step 39-5s in the synthetic method for **EXAMPLE aa39,** compound aa**58-4** (445 mg, 0.65 mmol) was used instead of compound aa**39-4** to obtain compound aa**58-5** (0.65 mmol) which was used in the next step without further purification.

### [Step 58-6s]

According to Step 39-6s in the synthetic method for **EXAMPLE aa39,** compound aa**58-5** (0.65 mmol) was used instead of compound aa39-5 to obtain **EXAMPLE aa58** (42 mg, 0.083 mmol) as a white amorphous solid.

### (EXAMPLE aa52)

### Preparation of (S)-methyl 1-(3-((R)-2-((R)-2-(4-carbamimidoylphenylamino)-1-hydroxy-2-oxoethyl)-3-oxomorpholino)benzoyl)pyrrolidine-2-carboxylate EXAMPLE aa52

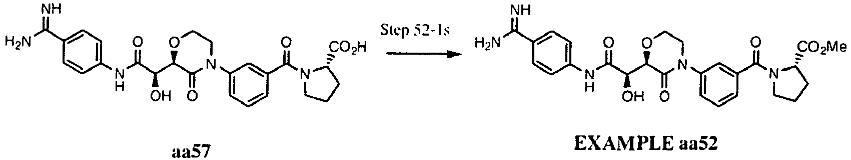

### [Step 52-1s]

According to Step 51-1s in the synthetic method for **EXAMPLE aa51,** compound aa**58** (24 mg, 0.047 mmol) was used instead of compound aa**50** to obtain **EXAMPLE** aa**52** (24 mg, 0.046 mmol) as a white amorphous solid.

### (EXAMPLE aa56)

### Preparation of 3-((R)-2-((R)-2-(4-carbamimidoylphenylamino)-1-hydroxy-2-oxoethyl)-3-oxomorpholino)-N-methyl-N-(2-oxo-2-(pyrrolidin-1-yl)ethyl)benzamide EXAMPLE aa56

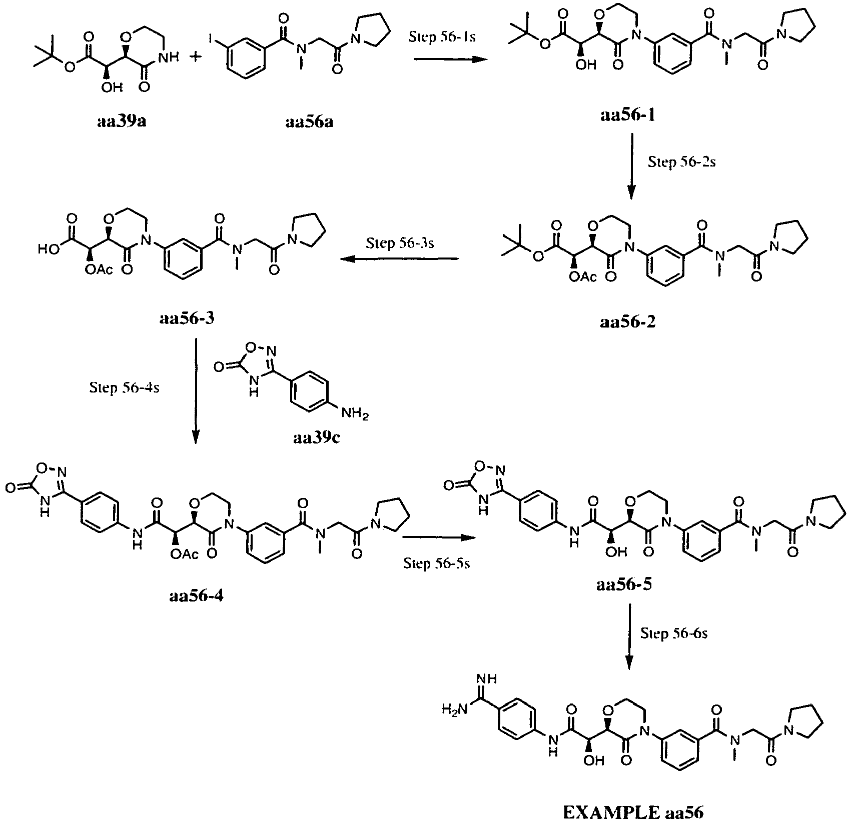

### [Step 56-1s]

According to Step 39-1s in the synthetic method for **EXAMPLE aa39,** compound aa**56a** (886 mg, 2.38 mmol) was used instead of compound aa**39b** to obtain compound aa**56-1** (669 mg, 1.41 mmol).

### [Step 56-2s]

According to Step 39-2s in the synthetic method for **EXAMPLE aa39,** compound aa**56-1** (669 mg, 1.41 mmol) was used instead of compound aa**39-1** to obtain compound aa**56-2** (477 mg, 0.92 mmol).

### [Step 56-3s]

According to Step 39-3s in the synthetic method for **EXAMPLE aa39,** compound aa**56-2** (477 mg, 0.92 mmol) was used instead of compound aa**39-2** to obtain compound aa**40-3** (0.92 mmol) which was used in the next step without further purification.

### [Step 56-4s]

According to Step 39-4s in the synthetic method for **EXAMPLE aa39,** compound aa**40-3** (235 mg, 0.51 mmol) was used instead of compound aa**39-3** to obtain compound aa**56-4** (244 mg, 0.39 mmol).

### [Step 56-5s]

According to Step 39-5s in the synthetic method for **EXAMPLE aa39,** compound aa**56-4** (244 mg, 0.39 mmol) was used instead of compound aa**39-4** to obtain compound aa**56-5** (0.39 mmol) which was used in the next step without further purification.

### [Step 56-6s]

According to Step 39-6s in the synthetic method for **EXAMPLE aa39,** compound aa**56-5** (0.39 mmol) was used instead of compound aa**39-5** to obtain **EXAMPLE aa56** (160 mg, 0.30 mmol) as a white amorphous solid.

### (EXAMPLE aa59)

### Preparation of 3-((R)-2-((R)-1-hydroxy-2-(1-iminoisoindolin-5-ylamino)-2-oxoethyl)-3-oxomorpholino)-N-methyl-N-(2-oxo-2-(pyrrolidin-1-yl)ethyl)benzamide EXAMPLE aa59

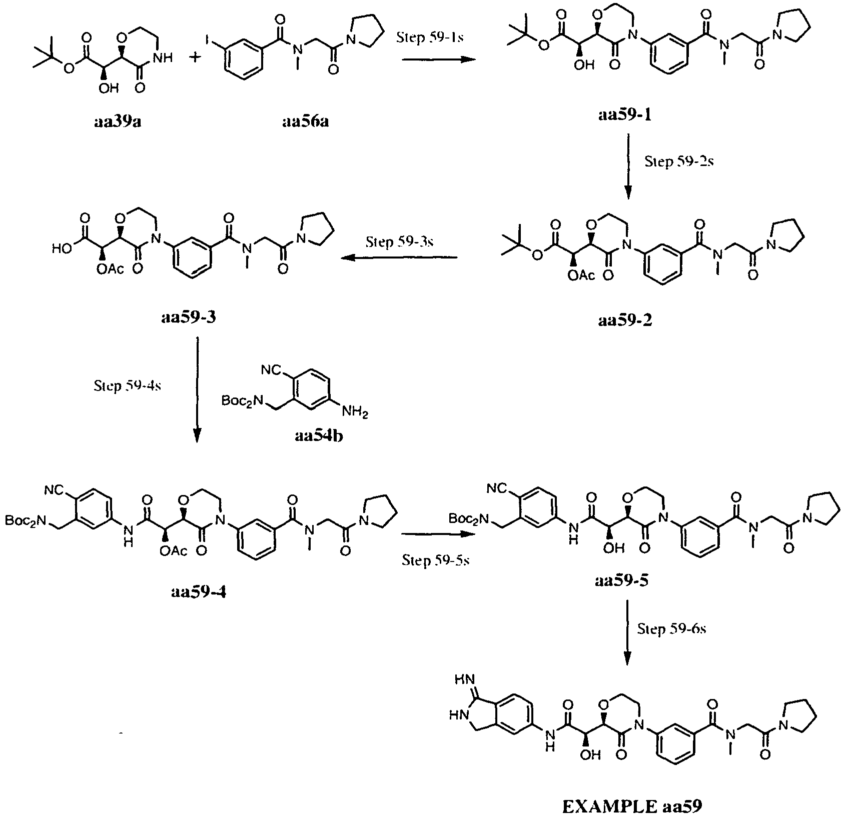

### [Step 59-1s]

According to Step 39-1s in the synthetic method for **EXAMPLE aa39,** compound aa**56a** (886 mg, 2.38 mmol) was used instead of compound aa**39b** to obtain compound aa**59-1** (669 mg, 1.41 mmol).

### [Step 59-2s]

According to Step 39-2s in the synthetic method for **EXAMPLE aa39,** compound aa**59-1** (669 mg, 1.41 mmol) was used instead of compound aa39-1 to obtain compound aa**59-2** (477 mg, 0.92 mmol).

### [Step 59-3s]

According to Step 39-3s in the synthetic method for **EXAMPLE aa39,** compound aa**59-2** (477 mg, 0.92 mmol) was used instead of compound aa**39-2** to obtain compound aa59-3 (0.92 mmol) which was used in the next step without further purification.

### [Step 59-4s]

According to Step 54-4s in the synthetic method for **EXAMPLE aa54,** compound **aa54b** (235 mg, 0.51 mmol) was used instead of compound aa**54-3** to obtain compound aa**59-4** (224 mg, 0.28 mmol).

### [Step 59-5s]

According to Step 39-5s in the synthetic method for **EXAMPLE aa39,** compound aa**59-4** (224 mg, 0.28 mmol) was used instead of compound aa**39-4** to obtain compound aa**59-5** (0.28 mmol) which was used in the next step without further purification.

### [Step 59-6s]

According to Step 54-6s in the synthetic method for **EXAMPLE aa54,** compound aa**59-5** (0.28 mmol) was used instead of compound aa**54-5** to obtain **EXAMPLE aa59** (57 mg, 0.10 mmol) as a white amorphous solid.

### (EXAMPLE aa60)

### Preparation of (R)-N-(4-carbamimidoylphenyl)-2-hydroxy-2-((R)-4-(3-methyl-5-(morpholine-4-carbonyl)phenyl)-3-oxomorpholin-2-yl)acetamide EXAMPLE aa60

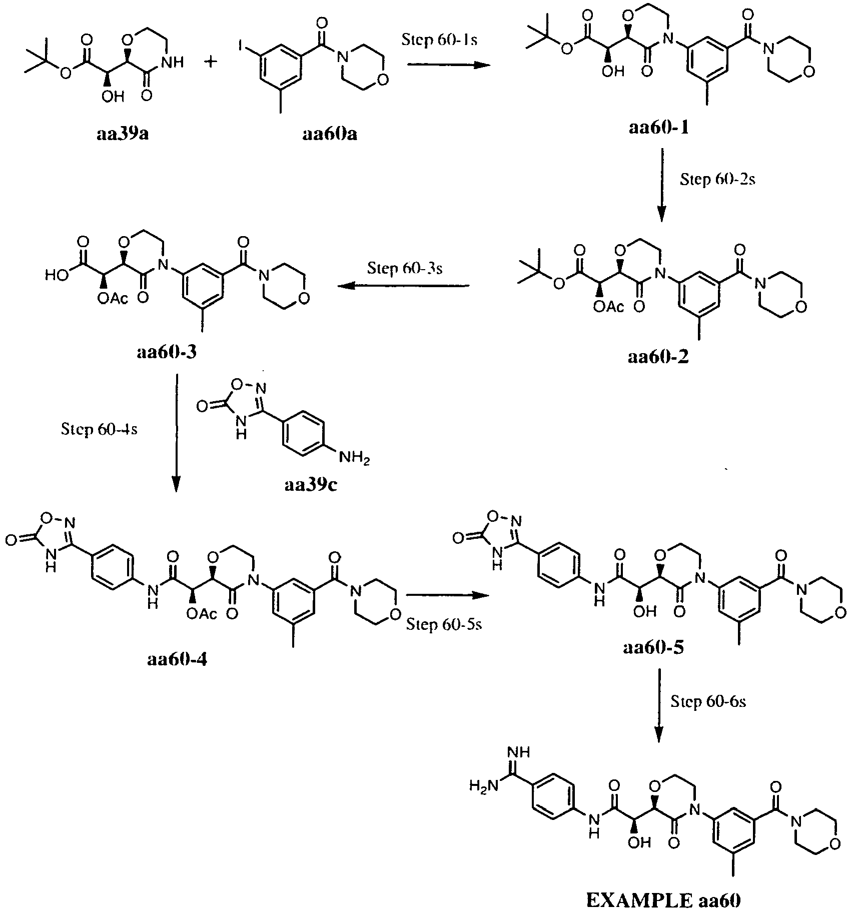

### [Step 60-1s]

According to Step 39-1s in the synthetic method for **EXAMPLE aa39,** compound aa**60a** (315 mg, 0.95 mmol) was used instead of compound aa**39b** to obtain compound aa**60-1** (218 mg, 0.50 mmol).

### [Step 60-2s]

According to Step 39-2s in the synthetic method for **EXAMPLE aa39,** compound aa**60-1** (218 mg, 0.50 mmol) was used instead of compound aa**39-1** to obtain compound aa**60-2** (226 mg, 0.47 mmol).

### [Step 60-3s]

According to Step 39-3s in the synthetic method for **EXAMPLE aa39,** compound aa**60-2** (226 mg, 0.47 mmol) was used instead of compound aa**39-2** to obtain compound aa**60-3** (0.47 mmol) which was used in the next step without further purification.

### [Step 60-4s]

According to Step 39-4s in the synthetic method for **EXAMPLE aa39,** compound aa**60-3** (100 mg, 0.24 mmol) was used instead of compound aa**39-3** to obtain compound aa**60-4** (87 mg, 0.15 mmol).

### [Step 60-5s]

According to Step 39-5s in the synthetic method for **EXAMPLE aa39,** compound aa**60-4** (87 mg, 0.15 mmol) was used instead of compound aa**39-4** to obtain compound aa**60-5** (0.15 mmol) which was used in the next step without further purification.

### [Step 60-6s]

According to Step 39-6s in the synthetic method for **EXAMPLE aa39,** compound aa**60-5** (0.15 mmol) was used instead of compound aa**39-5** to obtain **EXAMPLE aa60** (71 mg, 0.14 mmol) as a white amorphous solid.

### (EXAMPLE aa61)

### Preparation of (R)-2-hydroxy-N-(1-iminoisoindolin-5-yl)-2-((R)-4-(3-methyl-5-(morpholine-4-carbonyl)phenyl)-3-oxomorpholin-2-yl)acetamide EXAMPLE aa61

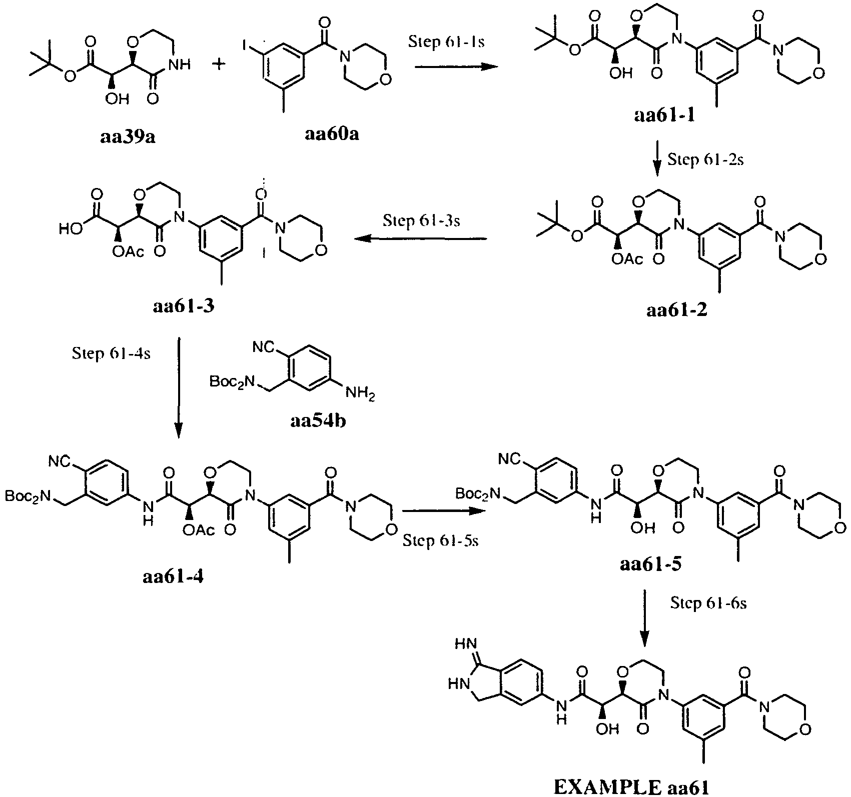

### [Step 61-1s]

According to Step 39-1s in the synthetic method for **EXAMPLE aa39,** compound aa**60a** (315 mg, 0.95 mmol) was used instead of compound aa39b to obtain compound aa**61-1** (218 mg, 0.50 mmol).

### [Step 61-2s]

According to Step 39-2s in the synthetic method for **EXAMPLE aa39,** compound aa**61-1** (218 mg, 0.50 mmol) was used instead of compound aa**39-1** to obtain compound aa**61-2** (226 mg, 0.47 mmol).

### [Step 61-3s]

According to Step 39-3s in the synthetic method for **EXAMPLE aa39,** compound aa**61-2** (226 mg, 0.47 mmol) was used instead of compound aa39-2 to obtain compound aa**61-3** (0.47 mmol) which was used in the next step without further purification.

### [Step 61-4s]

According to Step 54-4s in the synthetic method for **EXAMPLE aa54,** compound aa**61-3** (124 mg, 0.30 mmol) was used instead of compound aa54-3 to obtain compound aa**61-4** (118 mg, 0.16 mmol).

### [Step 61-5s]

According to Step 39-5s in the synthetic method for **EXAMPLE aa39,** compound aa**61-4** (118 mg, 0.16 mmol) was used instead of compound aa**39-4** to obtain compound aa**61-5** (0.16 mmol) which was used in the next step without further purification.

### [Step 61-6s]

According to Step 54-6s in the synthetic method for **EXAMPLE aa54,** compound aa**61-5** (0.16 mmol) was used instead of compound aa**54-5** to obtain **EXAMPLE aa61** (71 mg, 0.14 mmol) as a white amorphous solid.

### (EXAMPLE aa63)

### Preparation of (R)-2-hydroxy-N-(1-iminoisoindolin-5-yl)-2-((R)-4-(3-((R)-3-methoxypyrrolidine-1-carbonyl)phenyl)-3-oxomorpholin-2-yl)acetamide EXAMPLE aa63

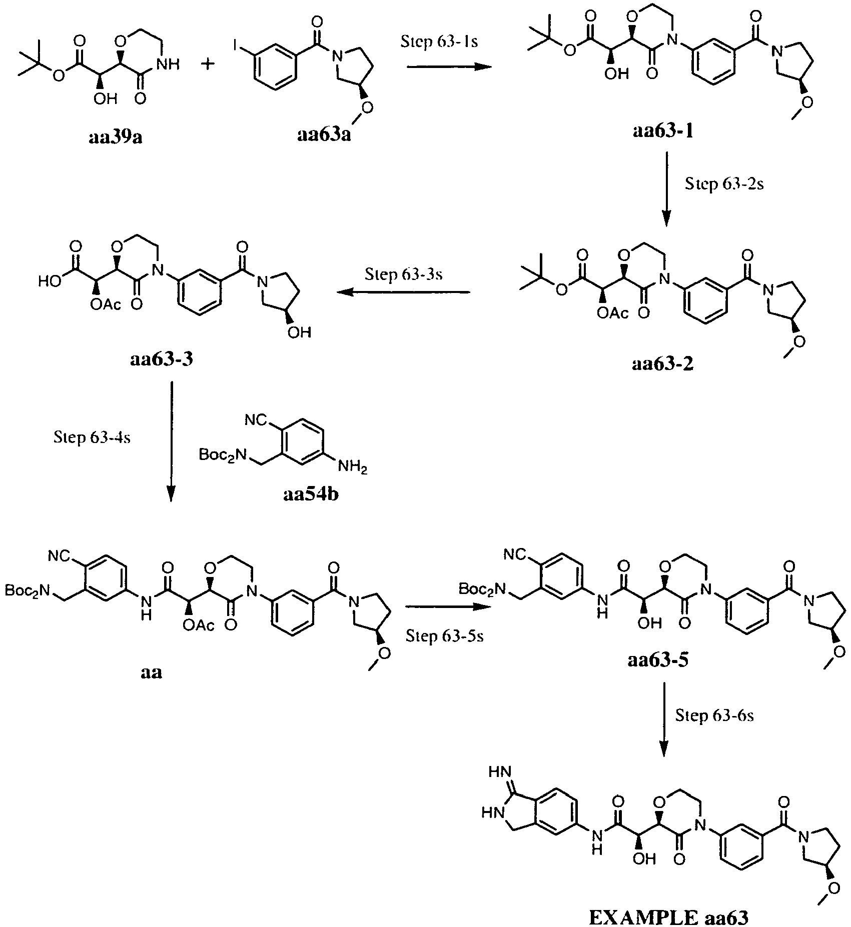

### [Step 63-1s]

According to Step 39-1s in the synthetic method for **EXAMPLE aa39,** compound aa**63a** (158 mg, 0.48 mmol) was used instead of compound aa**39b** to obtain compound aa**63-1** (124 mg, 0.29 mmol).

### [Step 63-2s]

According to Step 39-2s in the synthetic method for **EXAMPLE aa39,** compound aa**63-1** (124 mg, 0.29 mmol) was used instead of compound aa**39-1** to obtain compound aa**63-2** (132 mg, 0.28 mmol).

### [Step 63-3s]

According to Step 39-3 in the synthetic method for **EXAMPLE aa39,** compound aa**63-2** (132 mg, 0.28 mmol) was used instead of compound aa**39-2** to obtain compound aa**63-3** (0.28 mmol) which was used in the next step without further purification.

### [Step 63-4s]

According to Step 54-4s in the synthetic method for **EXAMPLE aa54,** compound aa**63-3** (0.28 mmol) was used instead of compound aa**54-3** to obtain compound aa**63-4** (88 mg, 0.12 mmol).

### [Step 63-5s]

According to Step 39-5s in the synthetic method for **EXAMPLE aa39,** compound aa**63-4** (88 mg, 0.12 mmol) was used instead of compound aa**39-4** to obtain compound **63-5** (0.12 mmol) which was used in the next step without further purification.

### [Step 63-6s]

According to Step 54-6s in the synthetic method for **EXAMPLE aa54,** compound aa**63-5** (0.12 mmol) was used instead of compound aa**54-5** to obtain **EXAMPLE aa63** (26 mg, 0.051 mmol) as a white amorphous solid.

### (EXAMPLE aa64)

### Preparation of (R)-2-hydroxy-N-(1-iminoisoindolin-5-yl)-2-((R)-4-(3-((S)-3-methoxypyrrolidine-1-carbonyl)phenyl)-3-oxomorpholin-2-yl)acetamide EXAMPLE aa64

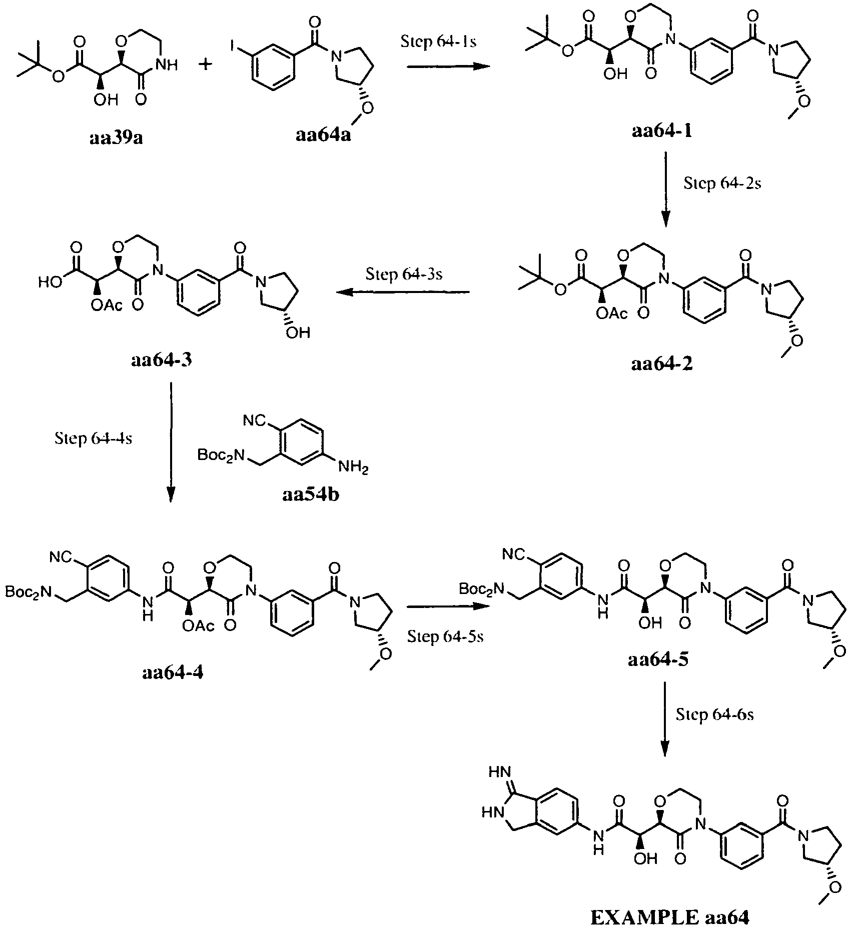

### [Step 64-1s]

According to Step 39-1s in the synthetic method for **EXAMPLE aa39,** compound aa**64a** (158 mg, 0.48 mmol) was used instead of compound aa**39b** to obtain compound aa**64-1** (125 mg, 0.29 mmol).

### [Step 64-2s]

According to Step 39-2s in the synthetic method for **EXAMPLE aa39,** compound aa**64-1** (125 mg, 0.29 mmol) was used instead of compound aa**39-1** to obtain compound aa**64-2** (136 mg, 0.29 mmol).

### [Step 64-3s]

According to Step 39-3s in the synthetic method for **EXAMPLE aa39,** compound aa**64-2** (136 mg, 0.29 mmol) was used instead of compound aa39-2 to obtain compound aa**64-3** (0.29 mmol) which was used in the next step without further purification.

### [Step 64-4s]

According to Step 54-4s in the synthetic method for **EXAMPLE aa54,** compound aa**64-3** (0.29 mmol) was used instead of compound aa**54-3** to obtain compound aa**64-4** (109 mg, 0.15 mmol).

### [Step 64-5s]

According to Step 39-5s in the synthetic method for **EXAMPLE aa39,** compound aa**64-4** (109 mg, 0.15 mmol) was used instead of compound aa**39-4** to obtain compound aa**64-5** (0.15 mmol) which was used in the next step without further purification.

### [Step 64-6s]

According to Step 54-6s in the synthetic method for **EXAMPLE aa54,** compound aa**64-5** (0.15 mmol) was used instead of compound aa**54-5** to obtain **EXAMPLE aa64** (70 mg, 0.14 mmol) as a white amorphous solid.

### (EXAMPLE aa66)

### Preparation of (R)-2-((R)-4-(3-chloro-4-(morpholine-4-carbonyl)phenyl)-3-oxomorpholin-2-yl)-2-hydroxy-N-(1-iminoisoindolin-5-yl)acetamide EXAMPLE aa66

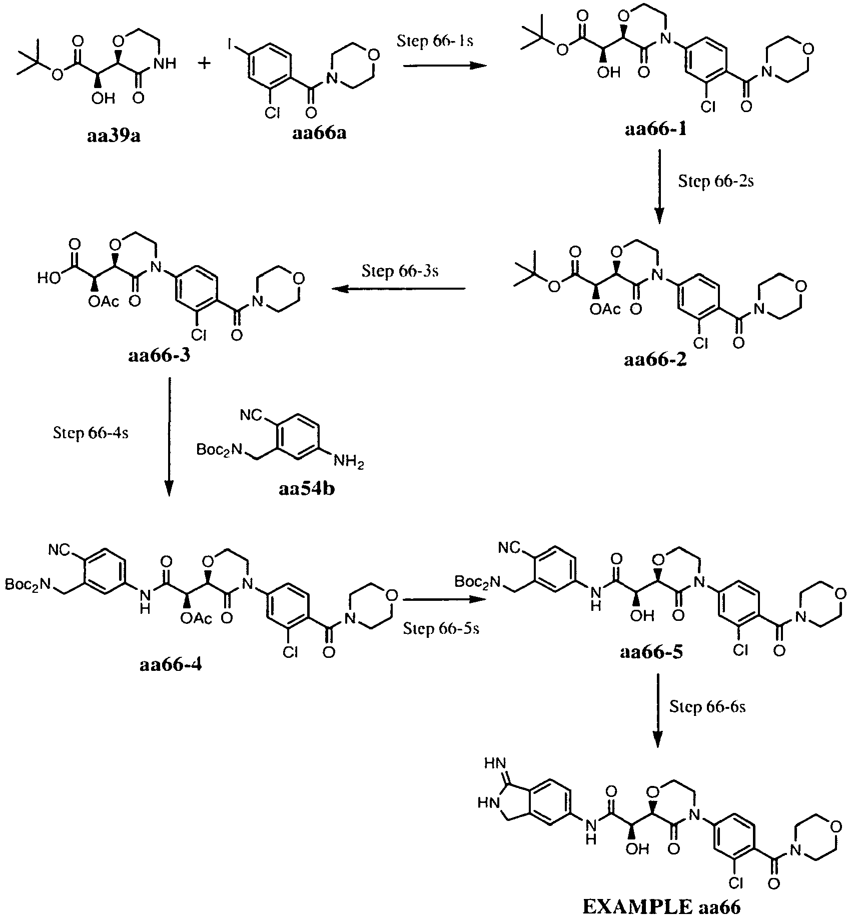

### [Step 66-1s]

According to Step 39-1s in the synthetic method for **EXAMPLE aa39,** compound aa**66a** (334 mg, 0.95 mmol) was used instead of compound aa**39B** to obtain compound aa**66-1** (276 mg, 0.61 mmol).

### [Step 66-2s]

According to Step 39-2s in the synthetic method for **EXAMPLE aa39,** compound aa**66-1** (276 mg, 0.61 mmol) was used instead of compound aa**39-1** to obtain compound aa**66-2** (0.61 mmol).

### [Step 66-3s]

According to Step 39-3s in the synthetic method for **EXAMPLE aa39,** compound aa**66-2** (0.61 mmol) was used instead of compound aa**39-2** to obtain compound aa**66-3** (0.61 mmol) which was used in the next step without further purification.

### [Step 66-4s]

According to Step 54-4s in the synthetic method for **EXAMPLE aa54,** compound aa**66-3** (213 mg, 0.48 mmol) was used instead of compound aa**54-3** to obtain compound aa**66-4** (310 mg. 0.40 mmol).

### [Step 66-5s]

According to Step 39-5s in the synthetic method for **EXAMPLE aa39,** compound aa**66-4** (310 mg, 0.40 mmol) was used instead of compound aa**39-4** to obtain compound aa66-5 (0.40 mmol) which was used in the next step without further purification.

### [Step 66-6s]

According to Step 54-6s in the synthetic method for **EXAMPLE aa54,** compound aa**66-5** (0.40 mmol) was used instead of compound aa**54-5** to obtain **EXAMPLE aa66** (208 mg, 0.39 mmol) as a white amorphous solid.

### (EXAMPLE aa68)

### Preparation of (R)-2-hydroxy-N-(1-iminoisoindolin-5-yl)-2-((R)-4-(3-(2-morpholino-2-oxoethyl)phenyl)-3-oxomorpholin-2-yl)acetamide EXAMPLE aa68

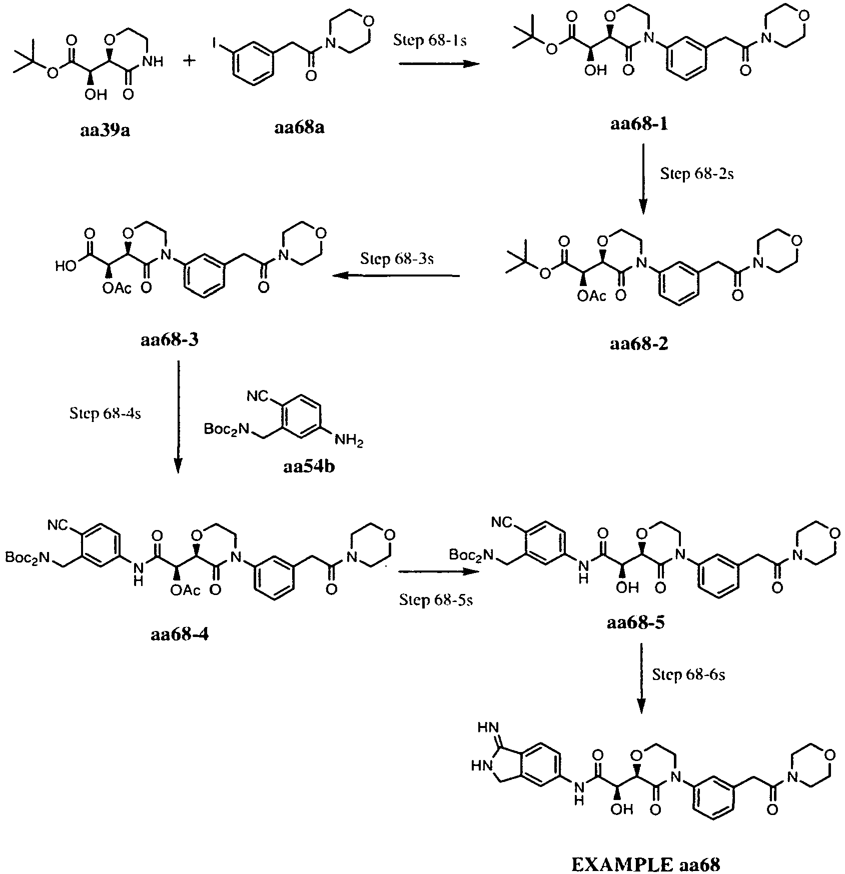

### [Step 68-1s]

According to Step 39-1s in the synthetic method for **EXAMPLE aa39,** compound aa**68a** (923 mg, 2.38 mmol) was used instead of compound aa**39b** to obtain compound aa68-1 (701 mg, 1.43 mmol).

### [Step 68-2s]

According to Step 39-2s in the synthetic method for **EXAMPLE aa39,** compound aa**68-1** (485 mg, 0.99 mmol) was used instead of compound aa**39-1** to obtain compound aa**68-2** (477 mg, 0.89 mmol).

### [Step 68-3s]

According to Step 39-3s in the synthetic method for **EXAMPLE aa39,** compound aa**68-2** (477 mg, 0.89 mmol) was used instead of compound aa39-2 to obtain compound aa**68-3** (0.89 mmol) which was used in the next step without further purification.

### [Step 68-4s]

According to Step 54-4s in the synthetic method for **EXAMPLE aa54,** compound aa**68-3** (0.89 mmol) was used instead of compound aa**54-3** to obtain compound aa**68-4** (536 mg, 0.67 mmol).

### [Step 68-5s]

According to Step 39-5s in the synthetic method for **EXAMPLE aa39,** compound aa**68-4** (536 mg, 0.67 mmol) was used instead of compound aa**39-4** to obtain compound aa**68-5** (0.67 mmol) which was used in the next step without further purification.

### [Step 68-6s]

According to Step 54-6s in the synthetic method for **EXAMPLE aa54,** compound aa**68-5** (0.67 mmol) was used instead of compound aa**54-5** to obtain **EXAMPLE aa68** (291 mg, 0.36 mmol) as a white amorphous solid.

### (EXAMPLE aa55)

### Preparation of (S)-1-(3-((R)-2-((R)-1-hydroxy-2-(1-iminoisoindolin-5-ylamino)-2-oxoethyl)-3-oxomorpholino)benzoyl)pyrrolidine-2-carboxylic acid EXAMPLE aa55

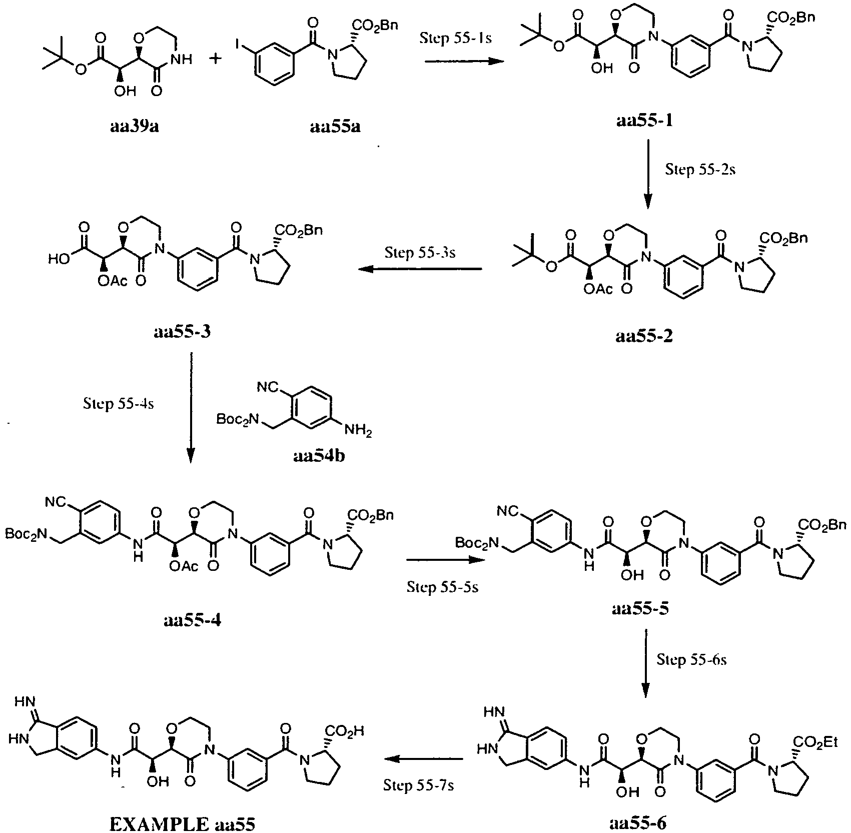

### [Step 55-1s]

According to Step 39-1s in the synthetic method for **EXAMPLE aa39,** compound aa**55a** (616 mg, 1.42 mmol) was used instead of compound aa**39b** to obtain compound aa**55-1** (710 mg, 1.32 mmol).

### [Step 55-2s]

According to Step 39-2s in the synthetic method for **EXAMPLE aa39,** compound aa**55-1** (710 mg, 1.32 mmol) was used instead of compound aa**39-1** to obtain compound aa**55-2** (707 mg, 1.22 mmol).

### [Step 55-3s]

According to Step 39-3s in the synthetic method for **EXAMPLE aa39,** compound aa**55-2** (707 mg, 1.22 mmol) was used instead of compound aa**39-2** to obtain compound aa**55-3** (1.22 mmol) which was used in the next step without further purification.

### [Step 55-4s]

According to Step 54-4 in the synthetic method for **EXAMPLE aa54,** compound aa**55-3** (1.22 mmol) was used instead of compound aa**54-3** to obtain compound aa**55-4** (409 mg, 0.48 mmol).

### [Step 55-5s]

According to Step 39-5s in the synthetic method for **EXAMPLE aa39,** compound aa**55-4** (409 mg, 0.48 mmol) was used instead of compound aa**39-4** to obtain compound aa**55-5** (0.48 mmol) which was used in the next step without further purification.

### [Step 55-6s]

According to Step 54-6s in the synthetic method for **EXAMPLE aa54,** compound aa**55-5** (0.48 mmol) was used instead of compound aa**54-5** to obtain compound aa**55-6** (0.48 mmol) which was used in the next step without further purification.

### [Step 55-7s]

According to Step 50-7s in the synthetic method for **EXAMPLE aa50,** compound aa**55-6** (0.48 mmol) was used instead of compound aa**50-6** to obtain **EXAMPLE aa55** (80 mg, 0.15 mmol) as a white amorphous solid.

### (EXAMPLE aa57)

### Preparation of (S)-methyl 1-(3-((R)-2-((R)-1-hydroxy-2-(1-iminoisoindolin-5-ylamino)-2-oxoethyl)-3-oxomorpholino)benzoyl)pyrrolidine-2-carboxylate EXAMPLE aa57

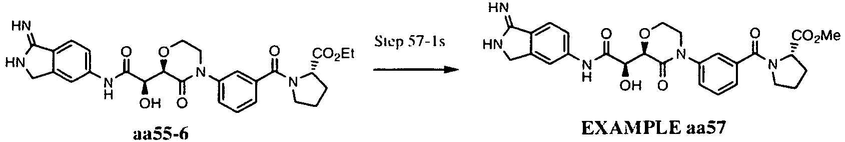

### [Step 57-1s]

According to Step 50-7s in the synthetic method for **EXAMPLE aa50,** compound aa**55-6** (0.48 mmol) was used instead of compound aa**50-6** to obtain **EXAMPLE aa57** (31 mg, 0.058 mmol) as a white amorphous solid.

### (EXAMPLE aa65)

### Preparation of ethyl 2-(3-((R)-2-((R)-1-hydroxy-2-(1-iminoisoindolin-5-ylamino)-2-oxoethyl)-3-oxomorpholino)phenyl)acetate EXAMPLE aa65

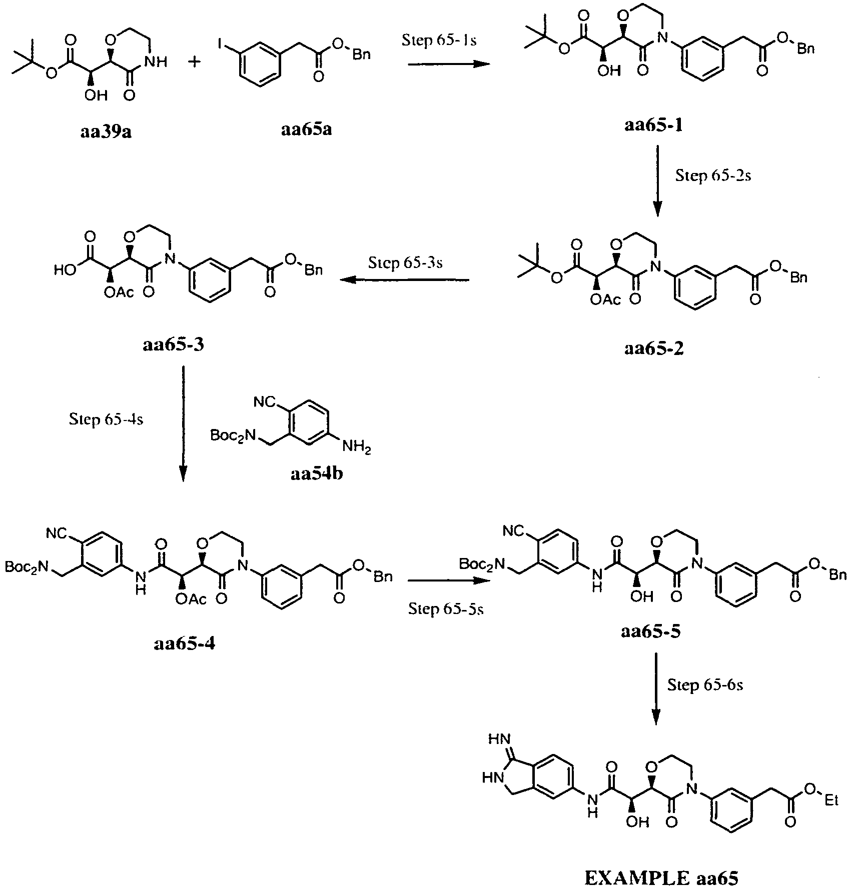

### [Step 65-1s]

According to Step 39-1s in the synthetic method for **EXAMPLE aa39**, compound aa**65a** (636 mg, 1.81 mmol) was used instead of compound aa**39b** to obtain compound aa**65-1** (694 mg, 1.53 mmol).

### [Step 65-2s]

According to Step 39-2s in the synthetic method for **EXAMPLE aa39,** compound aa**65-1** (694 mg, 1.53 mmol) was used instead of compound aa**39-1** to obtain compound aa**65-2** (723 mg, 1.45 mmol).

### [Step 65-3s]

According to Step 39-3s in the synthetic method for **EXAMPLE aa39**, compound aa**65-2** (723 mg, 1.45 mmol) was used instead of compound aa**39-2** to obtain compound aa**65-3** (1.45 mmol) which was used in the next step without further purification.

### [Step 65-4s]

According to Step 54-4s in the synthetic method for **EXAMPLE aa54**, compound aa**65-3** (1.45 mmol) was used instead of compound aa**54-3** to obtain compound aa**65-4** (715 mg, 0.93 mmol).

### [Step 65-5s]

According to Step 39-5s in the synthetic method for **EXAMPLE aa39**. compound aa**65-4** (715 mg. 0.93 mmol) was used instead of compound aa**39-4** to obtain compound aa**65-5** (0.93 mmol) which was used in the next step without further purification.

### [Step 65-6s]

According to Step 54-6s in the synthetic method for **EXAMPLE aa54**, compound aa**65-5** (0.93 mmol) was used instead of compound aa**54-5** to obtain **EXAMPLE aa65** (261 mg, 0.56 mmol) as a white amorphous solid.

### (EXAMPLE aa67)

### Preparation of 2-(3-((R)-2-((R)-1-hydroxy-2-(1-iminoisoindolin-5-ylamino)-2-oxoethyl)-3-oxomorpholino)phenyl)acetic acid EXAMPLE aa67

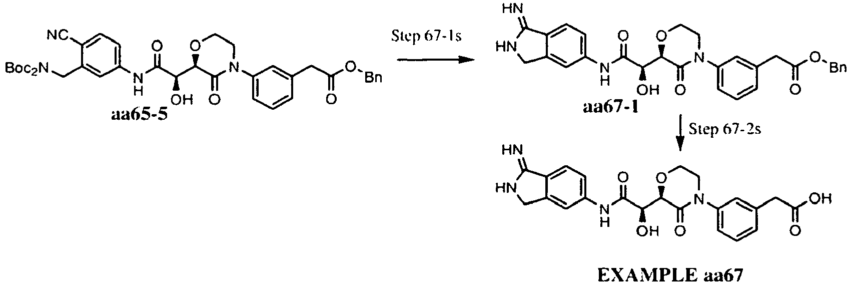

### [Step 67-1s]

According to Step 54-6s in the synthetic method for **EXAMPLE aa54**, compound aa**65-5** (715 mg, 0.93 mmol) was used instead of compound aa**54-5** to obtain aa**67-1** (64 mg, 0.12 mmol).

### [Step 67-2s]

According to Step 39-6s in the synthetic method for **EXAMPLE aa39**, compound aa**67-1** (64 mg, 0.12 mmol) was used instead of compound aa**39-5** to obtain **EXAMPLE aa67** (30 mg, 0.068 mmol) as a white amorphous solid.

### (EXAMPLE aa62)

### Preparation of ethyl 2-(3-((R)-1-((R)-1-hydroxy-2-(1-iminoisoindolin-5-ylamino)-2-oxoethyl)-3-oxomorpholino)-N-methylbenzamido)acetate EXAMPLE aa62

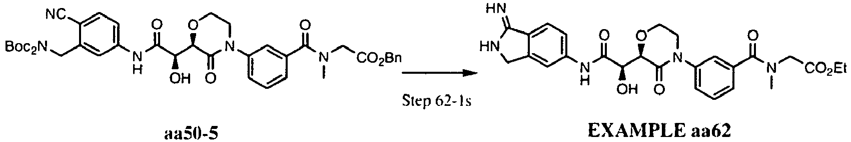

### [Step 62-1]

According to Step 54-6s in the synthetic method for **EXAMPLE aa54**, compound aa**50-5** (276 mg, 0.35 mmol) was used instead of compound aa**54-5** to obtain **EXAMPLE aa62** (65 mg, 0.12 mmol) as a white amorphous solid.

### (EXAMPLE aa70)

### Preparation of benzyl 2-(3-((R)-2-((R)-1-hydroxy-2-(1-iminoisoindolin-5-ylamino)-2-oxoethyl)-3-oxomorpholino)phenyl)-2-methylpropanoate EXAMPLE aa70

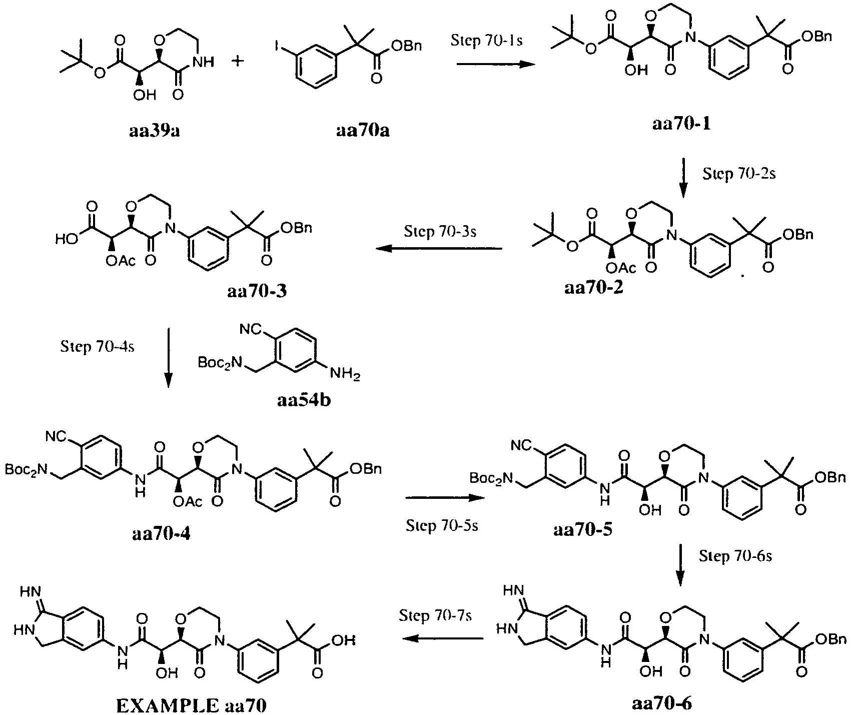

### [Step 70-1s]

According to Step 39-1s in the synthetic method for **EXAMPLE aa39**, compound aa**70a** (559 mg, 1.47 mmol) was used instead of compound aa**39b** to obtain compound aa**70-1** (524 mg, 1.08 mmol).

### [Step 70-2s]

According to Step 39-2s in the synthetic method for **EXAMPLE aa39,** compound aa**70-1** (524 mg, 1.08 mmol) was used instead of compound aa**39-1** to obtain compound aa**70-2** (558 mg, 1.06 mmol).

### [Step 70-3s]

According to Step 39-3s in the synthetic method for **EXAMPLE aa39,** compound aa**70-2** (558 mg, 1.06 mmol) was used instead of compound aa**39-2** to obtain compound aa**70-3** (1.06 mmol) which was used in the next step without further purification.

### [Step 70-4s]

According to Step 54-4s in the synthetic method for **EXAMPLE aa54**, compound aa**70-3** (1.06 mmol) was used instead of compound aa**54-3** to obtain compound aa**70-4** (415 mg, 0.54 mmol).

### [Step 70-5s]

According to Step 39-5s in the synthetic method for **EXAMPLE aa39**, compound aa**70-4** (415 mg, 0.54 mmol) was used instead of compound aa**39-4** to obtain compound aa**70-5** (0.54 mmol) which was used in the next step without further purification.

### [Step 70-6s]

According to Step 54-6s in the synthetic method for **EXAMPLE aa54**, compound aa**70-5** (0.54 mmol) was used instead of compound aa**54-5** to obtain **aa70-6** (0.54 mmol).

### [Step 70-7s]

According to Step 39-6s in the synthetic method for **EXAMPLE aa39**, compound aa**70-6** (0.54 mmol) was used instead of compound aa**39-5** to obtain **EXAMPLE aa70** (83 mg, 0.18 mmol) as a white amorphous solid.

### (EXAMPLE aa69)

### Preparation of (R)-2-hydroxy-N-(1-iminoisoindolin-5-yl)-2-((R)-4-(3-(morpholine-4-carbonyl)phenyl)-3-oxomorpholin-2-yl)acetamide EXAMPLE aa69

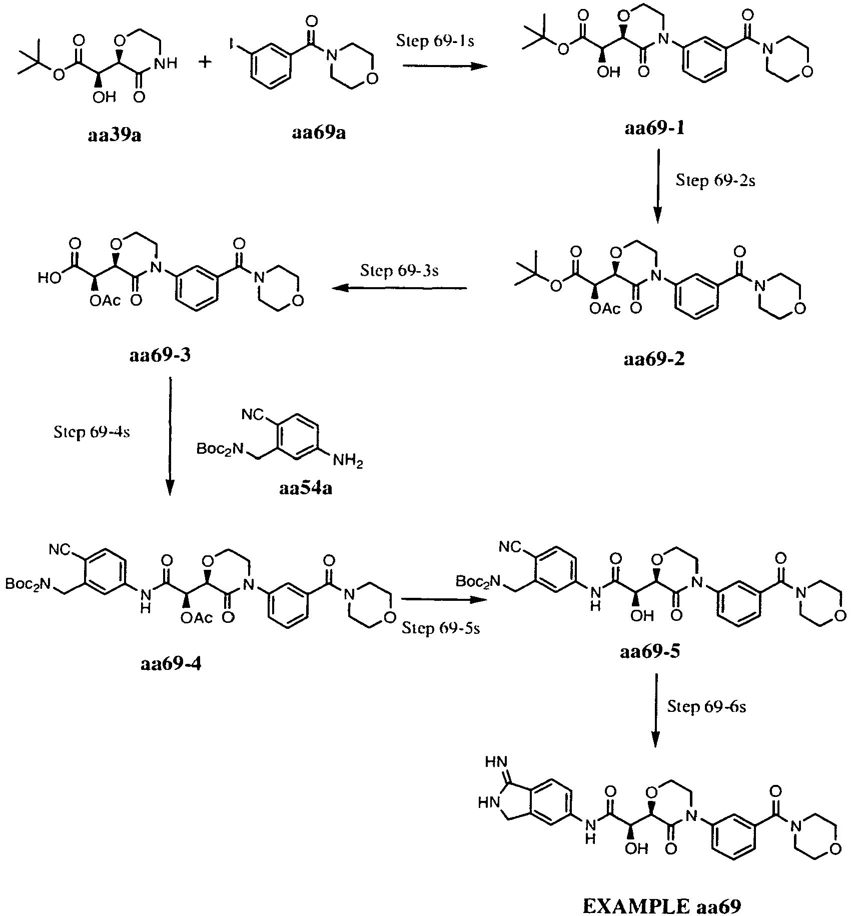

### [Step 69-1s]

According to Step 39-1s in the synthetic method for **EXAMPLE aa39**, compound aa**69a** (296 mg, 0.93 mmol) was used instead of compound aa**39b** to obtain compound aa**69-1** (345 mg, 0.82 mmol).

### [Step 69-2s]

According to Step 39-2s in the synthetic method for **EXAMPLE aa39**, compound aa**69-1** (345 mg, 0.82 mmol) was used instead of compound aa**39-1** to obtain compound aa**69-2** (372 mg, 0.81 mmol).

### [Step 69-3s]

According to Step 39-3s in the synthetic method for **EXAMPLE aa39,** compound aa**69-2** (372 mg, 0.81 mmol) was used instead of compound aa**39-2** to obtain compound aa**69-3** (0.81 mmol) which was used in the next step without further purification.

### [Step 69-4s]

According to Step 54-4s in the synthetic method for **EXAMPLE aa54**, compound aa**69-3** (0.81 mmol) was used instead of compound aa**54-3** to obtain compound aa**69-4** (523 mg, 0.71 mmol).

### [Step 69-5s]

According to Step 39-5s in the synthetic method for **EXAMPLE aa39**, compound aa**69-4** (523 mg, 0.71 mmol) was used instead of compound aa**39-4** to obtain compound aa**69-5** (0.71 mmol) which was used in the next step without further purification.

### [Step 69-6s]

According to Step 54-6s in the synthetic method for **EXAMPLE aa54**, compound aa**69-5** (0.71 mmol) was used instead of compound aa**54-5** to obtain **EXAMPLE aa69** (274 mg, 0.56 mmol) as a white amorphous solid.

### (EXAMPLE aa71)

### Preparation of 3-((R)-2-((R)-1-hydroxy-2-(1-iminoisoindolin-5-ylamino)-2-oxoethyl)-3-oxomorpholino)-N-methyl-N-(2-morpholino-2-oxoethyl)benzamide EXAMPLE aa71

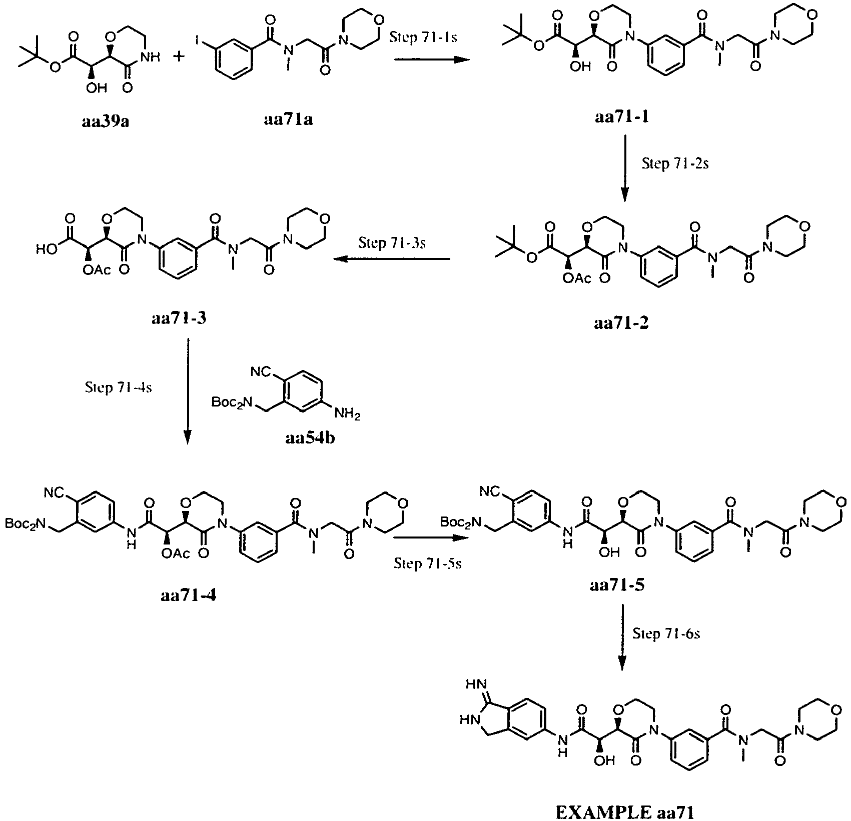

### [Step 71-1s]

According to Step 39-1s in the synthetic method for **EXAMPLE aa39**, compound aa**71a** (923 mg, 2.38 mmol) was used instead of compound aa**39b** to obtain compound aa**71-1** (701mg, 1.43 mmol).

### [Step 71-2s]

According to Step 39-2s in the synthetic method for **EXAMPLE aa39,** compound aa**71-1** (485 mg, 0.99 mmol) was used instead of compound aa**39-1** to obtain compound aa**71-2** (477 mg, 0.89 mmol).

### [Step 71-3s]

According to Step 39-3s in the synthetic method for **EXAMPLE aa39,** compound aa**71-2** (477 mg, 0.89 mmol) was used instead of compound aa**39-2** to obtain compound aa**71-3** (0.89 mmol) which was used in the next step without further purification.

### [Step 71-4s]

According to Step 54-4s in the synthetic method for **EXAMPLE aa54,** compound aa**71-3** (345 mg, 0.85 mmol) was used instead of compound aa**54-3** to obtain compound aa**71-4** (523 mg, 0.71 mmol).

### [Step 71-5s]

According to Step 39-5s in the synthetic method for **EXAMPLE aa39**, compound aa**71-4** (523 mg, 0.71 mmol) was used instead of compound aa**39-4** to obtain compound aa**71-5** (0.71 mmol) which was used in the next step without further purification.

### [Step 71-6s]

According to Step 54-6s in the synthetic method for **EXAMPLE aa54**, compound aa**71-5** (0.71 mmol) was used instead of compound aa**54-5** to obtain **EXAMPLE** aa**71** (274 mg, 0.56 mmol) as a white amorphous solid.

### (EXAMPLE aa72)

### Preparation of (R)-2-hydroxy-N-(1-iminoisoindolin-5-yl)-2-((R)-4-(3-(2-((R)-3-methoxypyrrolidin-1-yl)-2-oxoethyl)phenyl)-3-oxomorpholin-2-yl)acetamide EXAMPLE aa72

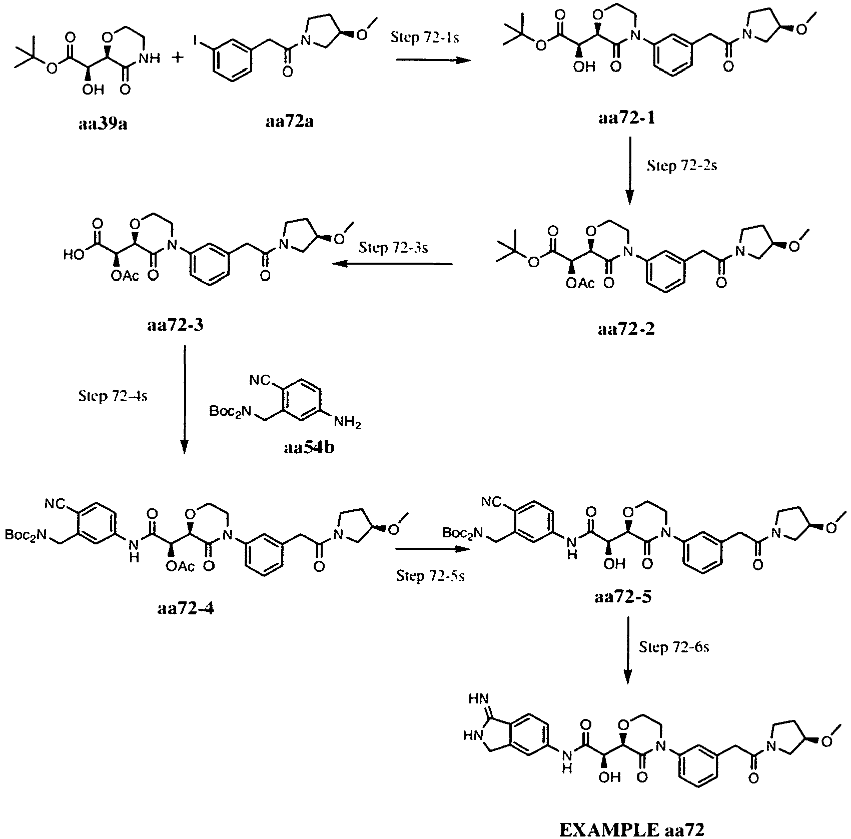

### [Step 72-1s]

According to Step 39-1 s in the synthetic method for **EXAMPLE aa39,** compound aa**72a** (356 mg, 1.03 mmol) was used instead of compound aa**39b** to obtain compound aa**72-1** (358 mg, 0.80 mmol).

### [Step 72-2s]

According to Step 39-2s in the synthetic method for **EXAMPLE aa39,** compound aa**72-1** (358 mg, 0.80 mmol) was used instead of compound aa**39-1** to obtain compound aa**72-2** (391 mg, 0.80 mmol).

### [Step 72-3s]

According to Step 39-3s in the synthetic method for **EXAMPLE aa39,** compound aa**72-2** (391 mg, 0.80 mmol) was used instead of compound aa**39-2** to obtain compound aa**72-3** (0.80 mmol) which was used in the next step without further purification.

### [Step 72-4s]

According to Step 54-4s in the synthetic method for **EXAMPLE aa54**, compound aa**72-3** (0.80 mmol) was used instead of compound aa**54-3** to obtain compound aa**72-4** (534 mg, 0.70 mmol).

### [Step 72-5s]

According to Step 39-5s in the synthetic method for **EXAMPLE aa39**, compound aa**72-4** (534 mg, 0.70 mmol) was used instead of compound aa**39-4** to obtain compound aa**72-5** (0.70 mmol) which was used in the next step without further purification.

### [Step 72-6s]

According to Step 54-6s in the synthetic method for **EXAMPLE aa54,** compound aa**72-5** (0.70 mmol) was used instead of compound aa**54-5** to obtain **EXAMPLE aa72** (283 mg, 0.56 mmol) as a white amorphous solid.

### (EXAMPLE aa73)

### Preparation of (R)-2-hydroxy-N-(1-iminoisoindolin-5-yl)-2-((R)-4-(3-(2-((S)-3-methoxypyrrolidin-1-yl)-2-oxoethyl)phenyl)-3-oxomorpholin-2-yl)acetamide EXAMPLE aa73

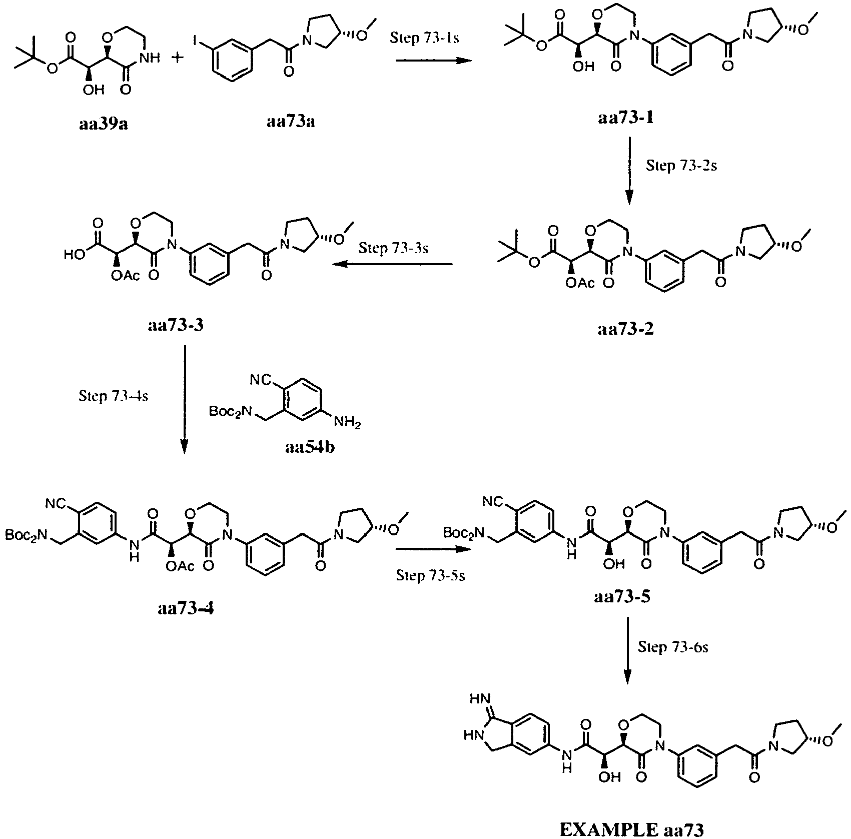

### [Step 73-1s]

According to Step 39-1 s in the synthetic method for **EXAMPLE aa39,** compound aa**73a** (724 mg, 2.10 mmol) was used instead of compound aa**39b** to obtain compound aa**73-1** (331 mg, 0.74 mmol).

### [Step 73-2s]

According to Step 39-2s in the synthetic method for **EXAMPLE aa39**, compound aa**73-1** (331 mg, 0.74 mmol) was used instead of compound aa**39-1** to obtain compound aa**73-2** (359 mg, 0.73 mmol).

### [Step 73-3s]

According to Step 39-3s in the synthetic method for **EXAMPLE aa39,** compound aa**73-2** (359 mg, 0.73 mmol) was used instead of compound aa**39-2** to obtain compound aa**73-3** (0.73 mmol) which was used in the next step without further purification.

### [Step 73-4s]

According to Step 54-4s in the synthetic method for **EXAMPLE aa54**, compound aa**73-3** (0.73 mmol) was used instead of compound aa**54-3** to obtain compound aa**73-4** (395 mg, 0.52 mmol).

### [Step 73-5s]

According to Step 39-5s in the synthetic method for **EXAMPLE aa39**, compound aa**73-4** (395 mg, 0.52 mmol) was used instead of compound aa**39-4** to obtain compound aa**73-5** (0.52 mmol) which was used in the next step without further purification.

### [Step 73-6s]

According to Step 54-6s in the synthetic method for **EXAMPLE aa54**, compound aa**73-5** (0.52 mmol) was used instead of compound aa**54-5** to obtain **EXAMPLE aa73** (222 mg, 0.43 mmol) as a white amorphous solid.

### (EXAMPLE aa74)

### Preparation of methyl 2-(3-((R)-2-((R)-1-hydroxy-2-(1-iminoisoindolin-5-ylamino)-2-oxoethyl)-3-oxomorpholino)phenyl)-2-methylpropanoate EXAMPLE aa74

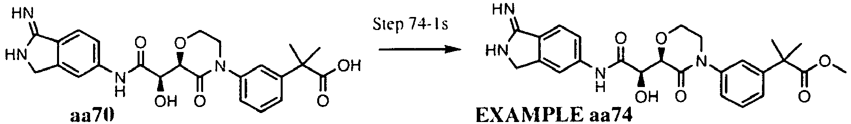

### [Step 74-1s]

To a solution of compound aa**70** (44 mg, 0.094 mmol) in methanol (4 mL) was concentrated sulfuric acid (0.08 mL). The reaction mixture was heated at 80°C for 16 hours. Triethylamine (0.5 mL) was added. The organic solvent was evaporated under reduced pressure. The crude product was purified RP-HPLC to afford the desired **EXAMPLE aa74** (20 mg, 0.042 mmol) as a white amorphous solid.

### (EXAMPLE aa75)

### Preparation of methyl 2-(3-((R)-2-((R)-1-hydroxy-2-(1-iminoisoindolin-5-ylamino)-2-oxoethyl)-3-oxomorpholino)phenyl)acetate EXAMPLE aa75

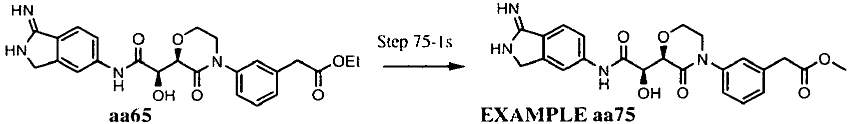

### [Step 75-1s]

To a solution of compound aa**65** (237 mg, 0.51 mmol) in methanol (5 mL) and water (5 mL) was added triethylamine (0.5 mL). The reaction mixture was stirred at room temperature for 3 days. The organic solvent was evaporated under reduced pressure. The residue was dissolved in methanol (5 mL) and concentrated sulfuric acid (0.1 mL) was added. The reaction mixture was heated at 80 °C for 16 hours. Triethylamine (1 mL) was added. The organic solvent was evaporated under reduced pressure. The crude product was purified RP-HPLC to afford the desired **EXAMPLE aa75** (20 mg, 0.042 mmol) as a white amorphous solid.

### (EXAMPLE aa76)

### Preparation of (R)-2-hydroxy-N-(1-iminoisoindolin-5-yl)-2-((R)-4-(3-(4-methyl-3-oxopiperazine-1-ca rbonyl)phenyl)-3-oxomorpholin-2-yl)acetamide EXAMPLE aa76

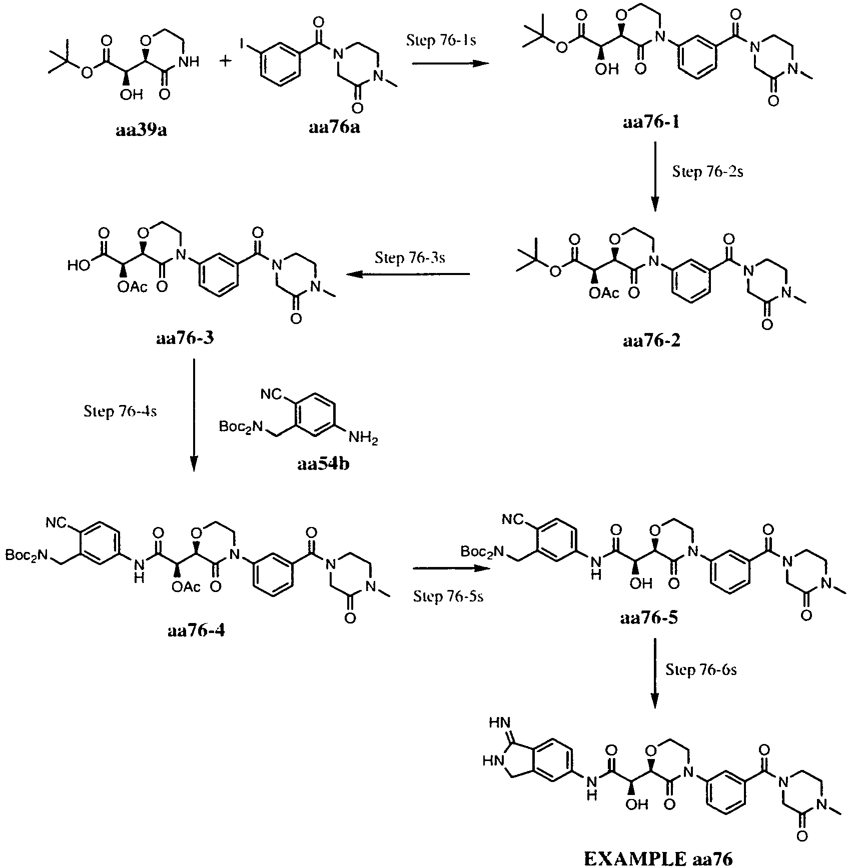

### [Step 76-1s]

According to Step 39-1s in the synthetic method for **EXAMPLE aa39,** compound aa**76a** (328 mg, 0.95 mmol) was used instead of compound aa**39b** to obtain compound aa**76-1** (347 mg, 0.71 mmol).

### [Step 76-2s]

According to Step 39-2s in the synthetic method for **EXAMPLE aa39,** compound aa**76-1** (347 mg, 0.71 mmol) was used instead of compound aa**39-1** to obtain compound aa**76-2** (265 mg, 0.54 mmol).

### [Step 76-3s]

According to Step 39-3s in the synthetic method for **EXAMPLE aa39,** compound aa**76-2** (265 mg, 0.54 mmol) was used instead of compound aa**39-2** to obtain compound aa**76-3** (0.54 mmol) which was used in the next step without further purification.

### [Step 76-4s]

According to Step 54-4s in the synthetic method for **EXAMPLE aa54,** compound aa**76-3** (0.54 mmol) was used instead of compound aa**54-3** to obtain compound aa**76-4** (282 mg, 0.37 mmol).

### [Step 76-5s]

According to Step 39-5s in the synthetic method for **EXAMPLE aa39,** compound aa**76-4** (282 mg, 0.37 mmol) was used instead of compound aa**39-4** to obtain compound aa**76-5** (0.37 mmol) which was used in the next step without further purification.

### [Step 76-6s]

According to Step 54-6s in the synthetic method for **EXAMPLE aa54**, compound aa**76-5** (0.37 mmol) was used instead of compound aa**54-5** to obtain **EXAMPLE aa76** (135 mg, 0.26 mmol) as a white amorphous solid.

### (EXAMPLE aa77)

### Preparation of ethyl 2-(3-((R)-2-((R)-1-hydroxy-2-(1-iminoisoindolin-5-ylamino)-2-oxoethyl)-3-oxomorph olino)phenyl)-2-methylpropanoate EXAMPLE aa77

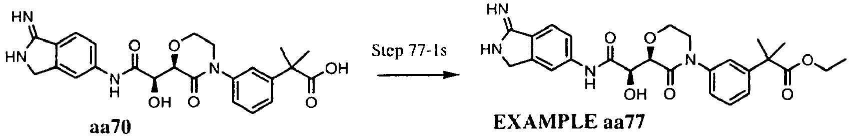

### [Step 77-1s]

To a solution of compound aa**70** (55 mg, 0.12 mmol) in absolute ethanol (4 mL) was added concentrated sulfuric acid (0.05 mL). The reaction mixture was heated at 80°C for 16 hours. Triethylamine (0.5 mL) was added. The organic solvent was evaporated under reduced pressure. The crude product was purified RP-HPLC to afford the desired **EXAMPLE aa77** (24 mg, 0.049 mmol) as a white amorphous solid.

### (EXAMPLE aa78)

### Preparation of (S)-1-(3-((R)-2-((R)-2-(4-carbamimidoylphenylamino)-1-hydroxy-2-oxoethyl)-3-oxom orpholino)-5-fluorobenzoyl)pyrrolidine-2-carboxylic acid EXAMPLE aa78

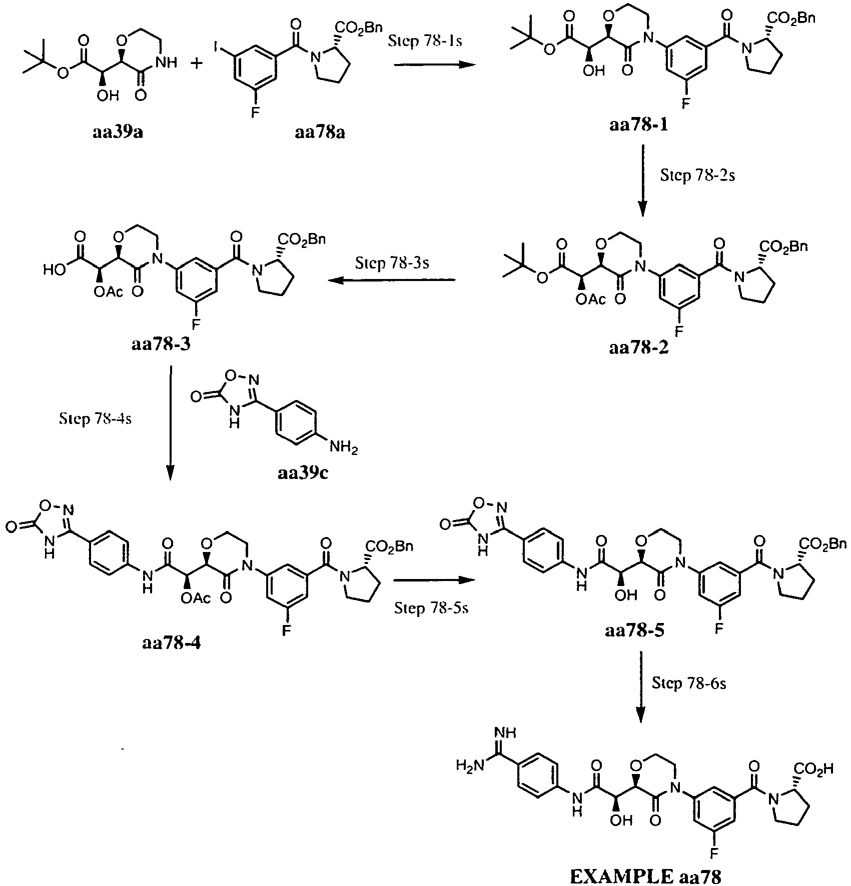

### [Step 78-1s]

According to Step 39-1s in the synthetic method for **EXAMPLE aa39**, compound aa**78a** (1.59 g, 3.51 mmol) was used instead of compound aa**39b** to obtain compound aa**78-1** (895 mg, 1.61 mmol).

### [Step 78-2s]

According to Step 39-2s in the synthetic method for **EXAMPLE aa39**, compound aa**78-1** (895 mg, 1.61 mmol) was used instead of compound aa**39-1** to obtain compound aa**78-2** (854 mg, 1.43 mmol).

### [Step 78-3s]

According to Step 39-3s in the synthetic method for **EXAMPLE aa39,** compound aa**78-2** (854 mg, 1.43 mmol) was used instead of compound aa**39-2** to obtain compound aa**78-3** (1.43 mmol) which was used in the next step without further purification.

### [Step 78-4s]

According to Step 39-4s in the synthetic method for **EXAMPLE aa39**, compound aaa**78-3** (1.43 mmol) was used instead of compound aa**39-3** to obtain compound aa**78-4** (968 mg, 1.38 mmol).

### [Step 78-5s]

According to Step 39-5s in the synthetic method for **EXAMPLE aa39,** compound **aa78-4** (968 mg, 1.38 mmol) was used instead of compound aa**39-4** to obtain compound aa**78-5** (1.38 mmol) which was used in the next step without further purification.

### [Step 78-6s]

According to Step 39-6s in the synthetic method for **EXAMPLE aa39**, compound aa**78-5** (1.38 mmol) was used instead of compound aa**39-5** to obtain **EXAMPLE aa78** (542 mg, 1.03 mmol) as a white amorphous solid.

### (EXAMPLE aa79)

### Preparation of (R)-2-hydroxy-N-(1-iminoisoindolin-5-yl)-2-((R)-4-(3-(2-(4-methyl-3-oxopiperazin-1-yl)-2-oxoethyl)phenyl)-3-oxomorpholin-2-yl)acetamide EXAMPLE aa79

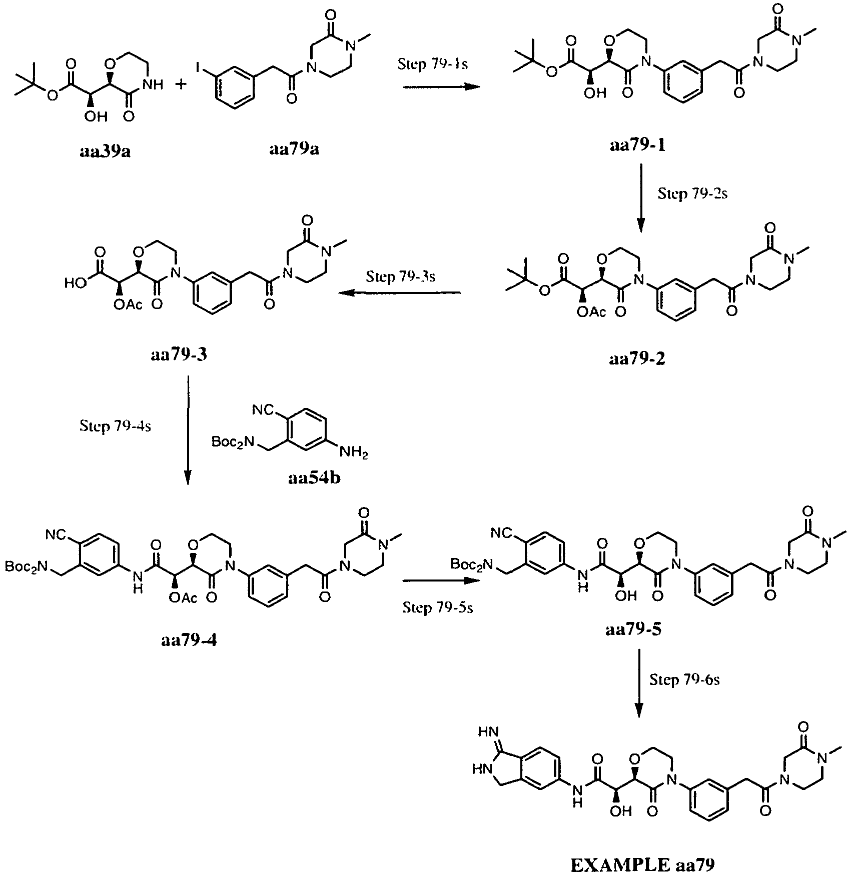

### [Step 79-1s]

According to Step 39-1s in the synthetic method for **EXAMPLE aa39**, compound aa**79a** (341 mg, 0.95 mmol) was used instead of compound aa**39b** to obtain compound aa**79-1** (214 mg, 0.46 mmol).

### [Step 79-2s]

According to Step 39-2s in the synthetic method for **EXAMPLE aa39**, compound aa**79-1** (214 mg, 0.46 mmol) was used instead of compound **aa39-1** to obtain compound aa**79-2** (207 mg, 0.41 mmol).

### [Step 79-3s]

According to Step 39-3s in the synthetic method for **EXAMPLE aa39**, compound aa**79-2** (207 mg, 0.41 mmol) was used instead of compound aa**39-2** to obtain compound aa**79-3** (0.41 mmol) which was used in the next step without further purification.

### [Step 79-4s]

According to Step 54-4s in the synthetic method for **EXAMPLE aa54**, compound aa**79-3** (0.41 mmol) was used instead of compound aa**54-3** to obtain compound aa**79-4** (277 mg, 0.36 mmol).

### [Step 79-5s]

According to Step 39-5s in the synthetic method for **EXAMPLE aa39,** compound aa**79-4** (277 mg, 0.36 mmol) was used instead of compound aa**39-4** to obtain compound aa**79-5** (0.36 mmol) which was used in the next step without further purification.

### [Step 79-6s]

According to Step 54-6s in the synthetic method for **EXAMPLE aa54**, compound aa**79-5** (0.36 mmol) was used instead of compound aa**54-5** to obtain **EXAMPLE aa79** (147 mg, 0.28 mmol) as a white amorphous solid.

### (EXAMPLE aa80)

### Preparation of (S)-ethyl 1-(3-((R)-2-((R)-2-(4-carbamimidoylphenylamino)-1-hydroxy-2-oxoethyl)-3-oxomorpholino)-5-fluorobenzoyl)pyrrolidine-2-carboxylate EXAMPLE aa80

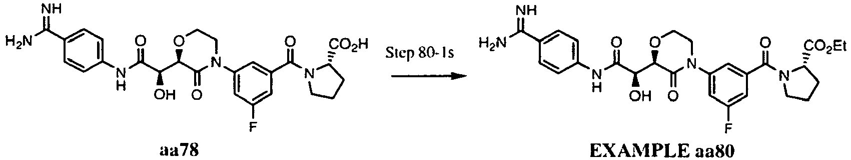

According to Step 77-1s in the synthetic method for **EXAMPLE aa77,** compound aa**78** (60 mg, 0.11 mmol) was used instead of compound aa**70** to obtain **EXAMPLE aa80** (29 mg, 0.049 mmol) as a white amorphous solid.

### (EXAMPLE aa81)

### Preparation of (S)-methyl 1-(3-((R)-2-((R)-2-(4-carbamimidoylphenylamino)-1-hydroxy-2-oxoethyl)-3-oxomorpholino)-5-fluorobenzoyl)pyrrolidine-2-carboxylate EXAMPLE aa81

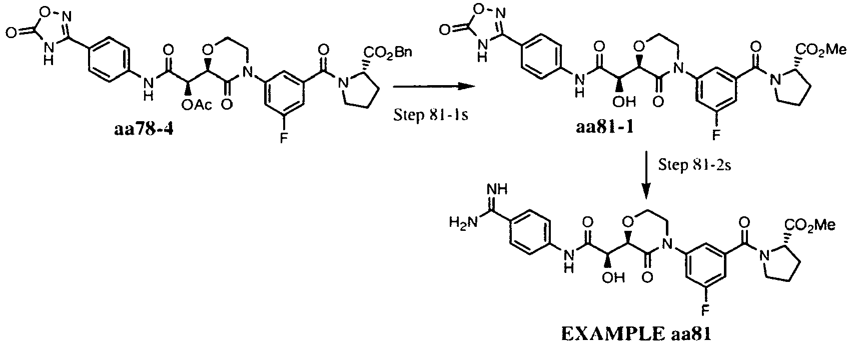

### [Step 81-1s]

According to Step 39-5s in the synthetic method for **EXAMPLE aa39,** compound aa**78-4** (968 mg, 1.38 mmol) was used instead of compound aa**39-4** to obtain compound aa**81-1** which was used in the next step without further purification.

### [Step 81-2s]

According to Step 39-6s in the synthetic method for **EXAMPLE aa39**, compound aa**81-1** was used instead of compound aa**39-5** to obtain **EXAMPLE aa81** (50 mg, 0.092 mmol) as a white amorphous solid.

### (EXAMPLE aa82)

### Preparation of (R)-2-((R)-4-(3-(2-(dimethylamino)-2-oxoethyl)phenyl)-3-oxomorpholin-2-yl)-2-hydroxy-N-(1-iminoisoindolin-5-yl)acetamide EXAMPLE aa82

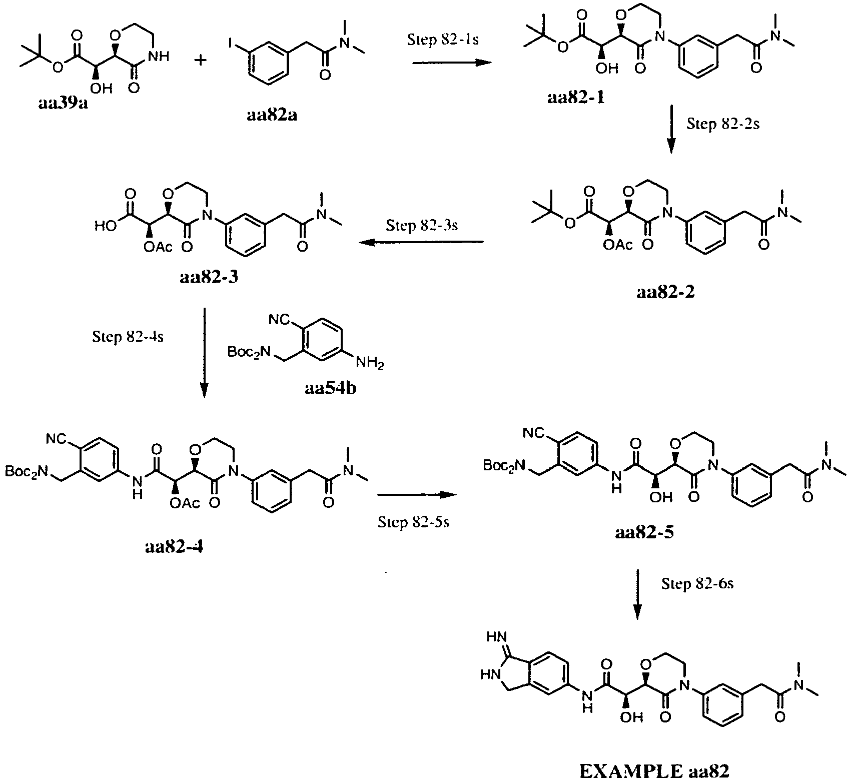

### [Step 82-1s]

According to Step 39-1 s in the synthetic method for **EXAMPLE aa39**, compound aa**82a** (275 mg, 0.95 mmol) was used instead of compound aa**39b** to obtain compound aa**82-1** (304 mg. 0.78 mmol).

### [Step 82-2s]

According to Step 39-2s in the synthetic method for **EXAMPLE aa39**, compound aa82-1 (304 mg, 0.78 mmol) was used instead of compound aa**39-1** to obtain compound aa**82-2** (302 mg, 0.70 mmol).

### [Step 82-3s]

According to Step 39-3s in the synthetic method for **EXAMPLE aa39,** compound aa**82-2** (302 mg, 0.70 mmol) was used instead of compound aa**39-2** to obtain compound aa**82-3** (0.70 mmol) which was used in the next step without further purification.

### [Step 82-4s]

According to Step 54-4s in the synthetic method for **EXAMPLE aa54**, compound aa**82-3** (104 mg, 0.28 mmol) was used instead of compound aa**54-3** to obtain compound aa**82-4** (143 mg, 0.20 mmol).

### [Step 82-5s]

According to Step 39-5s in the synthetic method for **EXAMPLE aa39**, compound aa**82-4** (143 mg, 0.20 mmol) was used instead of compound aa**39-4** to obtain compound aa**82-5** (0.20 mmol) which was used in the next step without further purification.

### [Step 82-6s]

According to Step 54-6s in the synthetic method for **EXAMPLE aa54**, compound aa**82-5** (0.20 mmol) was used instead of compound aa**54-5** to obtain **EXAMPLE** aa**82** (61 mg, 0.13 mmol) as a white amorphous solid.

### (EXAMPLE aa83)

### Preparation of 1-(3-((R)-2-((R)-1-hydroxy-2-(1-iminoisoindolin-5-ylamino)-2-oxoethyl)-3-oxomorpholino)benzoyl)piperidine-1-carboxylic acid EXAMPLE aa83

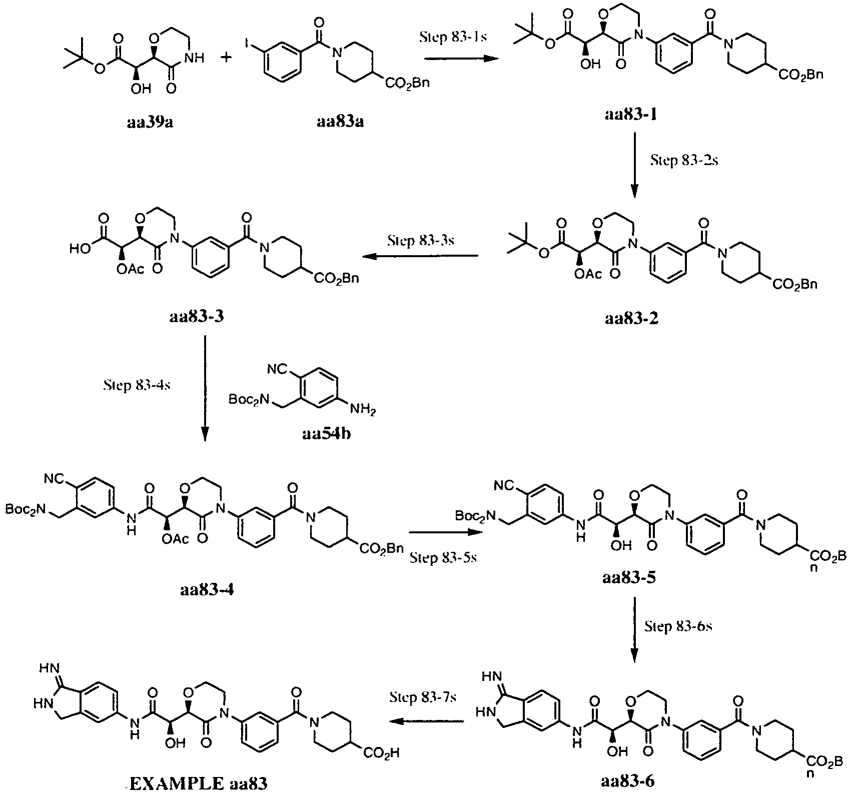

### [Step 83-1s]

According to Step 39-1s in the synthetic method for **EXAMPLE aa39**, compound aa**83a** (616 mg, 1.37 mmol) was used instead of compound aa**39b** to obtain compound aa**83-1** (592 mg, 1.07 mmol).

### [Step 83-2s]

According to Step 39-2s in the synthetic method for **EXAMPLE aa39**, compound aa**83-1** (592 mg, 1.07 mmol) was used instead of compound aa39-1 to obtain compound aa**83-2** (608 mg, 1.02 mmol).

### [Step 83-3s]

According to Step 39-3s in the synthetic method for **EXAMPLE aa39,** compound aa**83-2** (608 mg, 1.02 mmol) was used instead of compound aa**39-2** to obtain compound aa**83-3** (1.02 mmol) which was used in the next step without further purification.

### [Step 83-4s]

According to Step 54-4s in the synthetic method for **EXAMPLE aa54**, compound aa**83-3** (1.02 mmol) was used instead of compound aa**54-3** to obtain compound aa**83-4** (612 mg, 0.71 mmol).

### [Step 83-5s]

According to Step 39-5s in the synthetic method for **EXAMPLE aa39**, compound aa**83-4** (612 mg, 0.71 mmol) was used instead of compound aa**39-4** to obtain compound aa**83-5** (0.71 mmol) which was used in the next step without further purification.

### [Step 83-6s]

According to Step 54-6s in the synthetic method for **EXAMPLE aa54**, compound aa**83-5** (0.71 mmol) was used instead of compound **aa54-5** to obtain compound aa**83-6** (0.71 mmol) which was used in the next step without further purification.

### [Step 83-7s]

According to Step 39-6s in the synthetic method for **EXAMPLE aa39,** compound aa**83-6** (0.71 mmol) was used instead of compound aa**39-5** to obtain **EXAMPLE aa83** (124 mg, 0.23 mmol) as a white amorphous solid.

### (EXAMPLE aa84)

### Preparation of ethyl 1-(3-((R)-2-((R)-1-hydroxy-2-(1-iminoisoindolin-5-ylamino)-2-oxoethyl)-3-oxomorph olino)benzoyl)piperidine-4-carboxylate EXAMPLE aa84

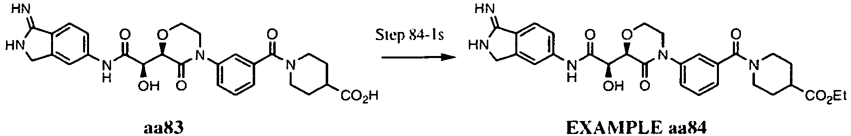

### [Step 84-1s]

According to Step 77-1s in the synthetic method for **EXAMPLE aa77,** compound aa**83** (90 mg, 0.17 mmol) was used instead of compound aa**70** to obtain **EXAMPLE aa84** (92 mg, 0.16 mmol) as a white amorphous solid.

### (EXAMPLE aa85)

### Preparation of 2-(3-((R)-2-((R)-1-acetoxy-2-(1-imino-3-oxoisoindolin-5-ylamino)-2-oxoethyl)-3-oxo morpholino)-N-methylbenzamido)acetic acid EXAMPLE aa85

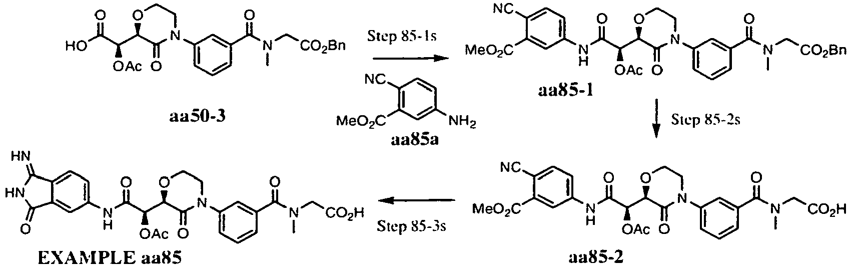

### [Step 85-1s]

According to Step 39-4s in the synthetic method for **EXAMPLE aa39,** compound aa**50-3** (250 mg, 0.50 mmol) was used instead of compound aa**39-3** and compound aa**85a** (105 mg, 0.60 mmol) was used instead of compound aa**39c** to obtain compound aa**85-1** (254 mg, 0.39 mmol).

### [Step 85-2s]

To a solution of aa**85-1** (254 mg, 0.39 mmol) in methanol (8 mL) was added palladium-charcoal (10%, 26 mg). The reaction mixture was stirred at room temperature under a hydrogen atmosphere for 16 hours. The reaction mixture was filtered. The filtrate was evaporated under reduced pressure to obtain compound aa**85-2** (0.39 mmol) which was used in the next step without further purification.

### [Step 85-3s]

To compound aa**85-2** (0.39 mmol) was added a 7 N solution of ammonia in methanol (2 mL). The reaction mixture was stirred at room temperature for 5 days. The organic solvent was evaporated under reduced pressure. The crude product was purified by high pH RP-HPLC to afford the desired **EXAMPLE aa85** (4 mg, 0.0073 mmol) as a white amorphous solid.

### (EXAMPLE aa86)

### Preparation of (R)-N-(1,3-diiminoisoindolin-5-yl)-2-((R)-4-(3-(2-(dimethylamino)-2-oxoethyl)phenyl)-3-oxomorpholin-2-yl)-2-hydroxyacetamide EXAMPLE a86

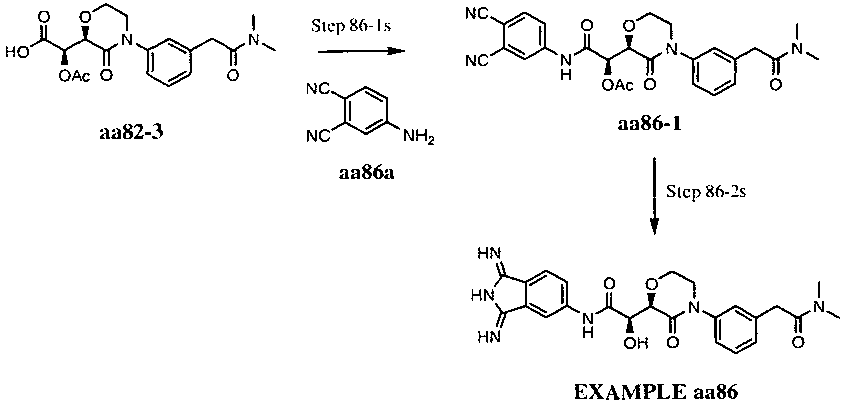

### [Step 86-1s]

According to Step 39-4s in the synthetic method for **EXAMPLE aa39,** compound aa**82-3** (175 mg, 0.46 mmol) was used instead of compound aa**39-3** and compound aa**86a** (100 mg, 0.70 mmol) was used instead of compound aa**39c** to obtain compound aa**86-1** (81 mg, 0.16 mmol).

### [Step 86-2s]

To compound aa**86-1** (81 mg, 0.16 mmol) was added a 7 N solution of ammonia in methanol (10 mL). The reaction mixture was stirred at room temperature for 4 days. The organic solvent was evaporated under reduced pressure. The crude product was purified by high pH RP-HPLC to afford the desired **EXAMPLE aa86** (45 mg, 0.094 mmol) as a white amorphous solid.

### (EXAMPLE aa87)

### Preparation of (R)-2-(1-imino-3-oxoisoindolin-5-ylamino)-1-((R)-4-(3-(methyl(2-oxo-2-(pyrrolidin-1-yl)ethyl)carbamoyl)phenyl)-3-oxomorpholin-2-yl)-2-ox oethyl acetate EXAMPLE aa87

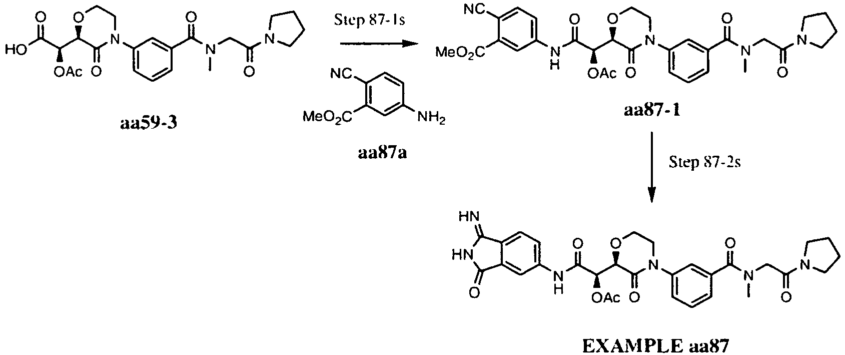

### [Step 87-1s]

According to Step 39-4s in the synthetic method for **EXAMPLE aa39**, compound aa**59-3** (179 mg, 0.44 mmol) was used instead of compound aa**39-3** and compound aa**87a** (117 mg, 0.66 mmol) was used instead of compound aa**39c** to obtain compound aa**87-1** (163 mg, 0.29 mmol).

### [Step 87-2s]

To compound aa**87-1** (163 mg, 0.29 mmol) was added a 7 N solution of ammonia in methanol (20 mL). The reaction mixture was stirred at room temperature for 3 days. The organic solvent was evaporated under reduced pressure to afford the desired **EXAMPLE aa87** (144 mg, 0.29 mmol) as a white amorphous solid.

### (EXAMPLE aa88)

### Preparation of 2-(3-((R)-2-((R)-2-(4-carbamimidoylphenylamino)-1-hydroxy-2-oxoethyl)-3-oxomorpholino)-5-fluoro-N-methylbenzamido)acetic acid EXAMPLE aa88

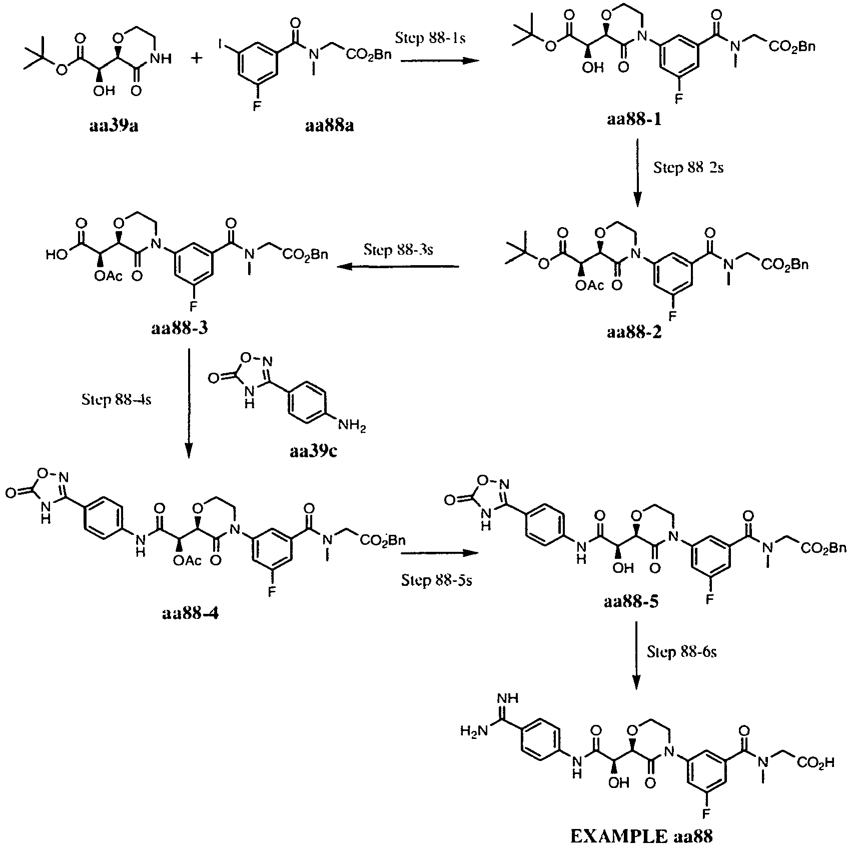

### [Step 88-1s]

According to Step 39-1 s in the synthetic method for **EXAMPLE aa39**, compound aa**88a** (760 mg, 1.78 mmol) was used instead of compound aa**39b** to obtain compound aa**88-1** (618 mg, 1.17 mmol).

### [Step 88-2s]

According to Step 39-2s in the synthetic method for **EXAMPLE aa39**, compound aa**88-1** (618 mg, 1.17 mmol) was used instead of compound aa**39-1** to obtain compound aa**88-2** (550 mg, 0.99 mmol).

### [Step 88-3s]

According to Step 39-3s in the synthetic method for **EXAMPLE aa39,** compound aa**88-2** (550 mg, 0.99 mmol) was used instead of compound aa**39-2** to obtain compound aa**88-3** (0.99 mmol) which was used in the next step without further purification.

### [Step 88-4s]

According to Step 39-4s in the synthetic method for **EXAMPLE aa39,** compound aa**88-3** (0.99 mmol) was used instead of compound aa**39-3** to obtain compound aa**88-4** (649 mg, 0.96 mmol).

### [Step 88-5s]

According to Step 39-5s in the synthetic method for **EXAMPLE aa39,** compound aa**88-4** (649 mg, 0.96 mmol) was used instead of compound aa**39-4** to obtain compound aa**88-5** (0.96 mmol) which was used in the next step without further purification.

### [Step 88-6s]

According to Step 39-6s in the synthetic method for **EXAMPLE aa39**, compound aa**88-5** (0.96 mmol) was used instead of compound aa**39-5** to obtain **EXAMPLE aa88** (156 mg, 0.31 mmol) as a white amorphous solid.

### (EXAMPLE aa89)

### Preparation of ethyl 2-(3-((R)-2-((R)-2-(4-carbamimidoylphenylamino)-1-hydroxy-2-oxoethyl)-3-oxomorpholino)-5-fluoro-N-methylbenzamido)acetate EXAMPLE aa89

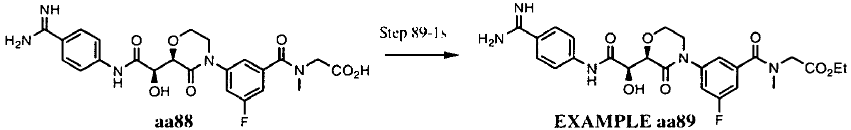

### [Step 89-1s]

According to Step 77-1s in the synthetic method for **EXAMPLE aa77**, compound aa**88** (150 mg, 0.30 mmol) was used instead of compound aa**70** to obtain **EXAMPLE aa89** (136 mg, 0.28 mmol) as a white amorphous solid.

### (EXAMPLE aa90)

### Preparation of 3-((R)-2-((R)-2-(4-carbamimidoylphenylamino)-1-hydroxy-2-oxoethyl)-3-oxomorpholino)-N-(2-(dimethylamino)-2-oxoethyl)-5-fluoro-N-methylbenzamide EXAMPLE aa90

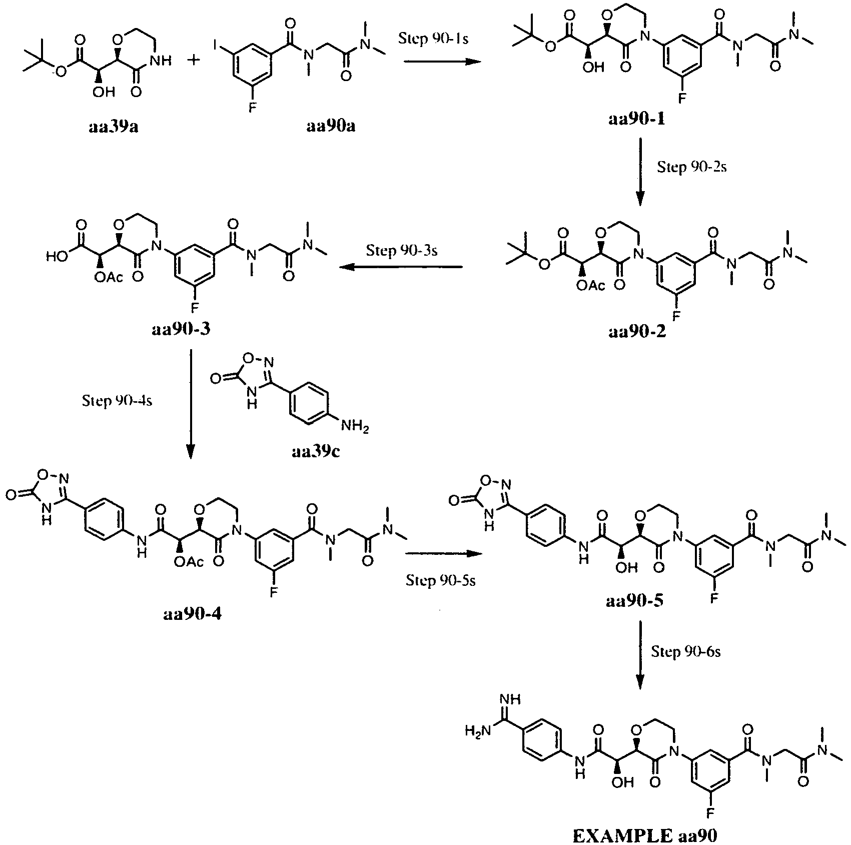

### [Step 90-1s]

According to Step 39-1s in the synthetic method for **EXAMPLE aa39**, compound aa**90a** (293 mg, 0.80 mmol) was used instead of compound aa**39b** to obtain compound aa**90-1** (347 mg, 0.74 mmol).

### [Step 90-2s]

According to Step 39-2s in the synthetic method for **EXAMPLE aa39,** compound aa**90-1** (347 mg, 0.74 mmol) was used instead of compound aa**39-1** to obtain compound aa**90-2** (282 mg, 0.55 mmol).

### [Step 90-3s]

According to Step 39-3s in the synthetic method for **EXAMPLE aa39**, compound aa**90-2** (282 mg, 0.55 mmol) was used instead of compound aa**39-2** to obtain compound aa**90-3** (0.55 mmol) which was used in the next step without further purification.

### [Step 90-4s]

According to Step 39-4s in the synthetic method for **EXAMPLE aa39,** compound aa**90-3** (0.55 mmol) was used instead of compound aa**39-3** to obtain compound aa**90-4** (219 mg, 0.36 mmol).

### [Step 90-5s]

According to Step 39-5s in the synthetic method for **EXAMPLE aa39,** compound aa**90-4** (219 mg, 0.36 mmol) was used instead of compound aa**39-4** to obtain compound aa**90-5** (0.36 mmol) which was used in the next step without further purification.

### [Step 90-6s]

According to Step 39-6s in the synthetic method for **EXAMPLE aa39,** compound aa**90-5** (0.36 mmol) was used instead of compound aa**39-5** to obtain **EXAMPLE aa90** (156 mg, 0.30 mmol) as a white amorphous solid.

### (EXAMPLE aa91)

### Preparation of (R)-N-(1.3-diiminoisoindolin-5-yl)-2-hydroxy-2-((R)-4-(3-(2-(4-methyl-3-oxopiperazin-1-yl)-2-oxoethyl)phenyl)-3-oxomorpholin-2-yl)acetamide EXAMPLE aa91

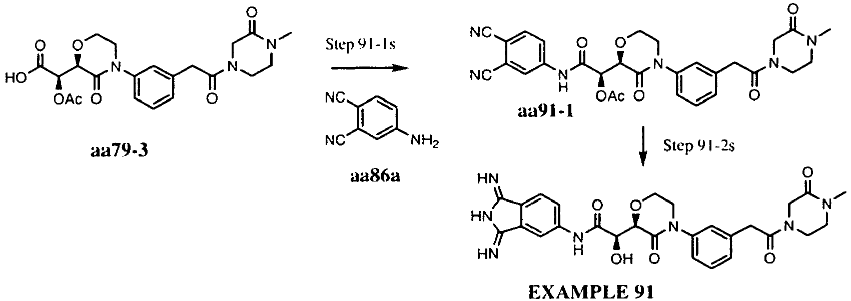

### [Step 91-1s]

According to Step 86-1s in the synthetic method for **EXAMPLE aa86,** compound aa**79-3** (176 mg, 0.35 mmol) was used instead of compound aa**82-3** to obtain compound aa**91-1** (50 mg, 0.079 mmol).

### [Step 91-2s]

According to Step 86-2s in the synthetic method for **EXAMPLE aa86**. compound aa**91-1** (50 mg, 0.079 mmol) was used instead of compound aa**86-1** to obtain **EXAMPLE** aa**91** (29 mg, 0.048 mmol).

### (EXAMPLE aa92)

### Preparation of N-(2-(dimethylamino)-2-oxoethyl)-3-((R)-2-((R)-1-hydroxy-2-(1-iminoisoindolin-5-ylamino)-2-oxoethyl)-3-oxomorpholino)-N-methylbenzamide EXAMPLE aa92

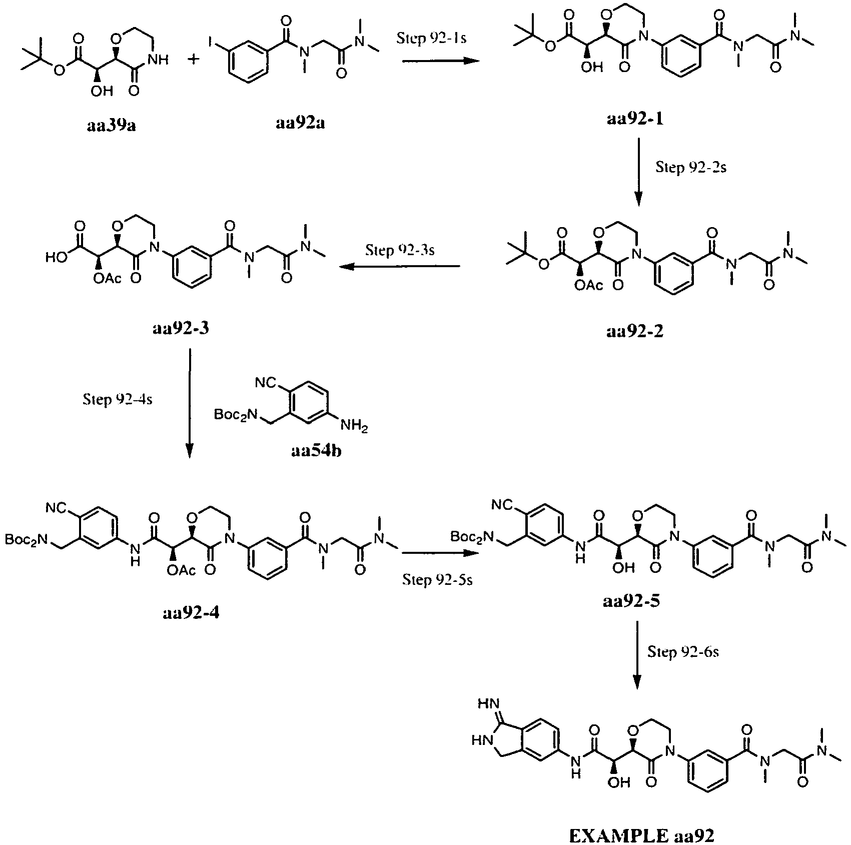

### [Step 92-1s]

According to Step 39-1 s in the synthetic method for **EXAMPLE aa39**, compound aa**92a** (330 mg, 0.95 mmol) was used instead of compound aa**39b** to obtain compound aa**92-1** (223 mg, 0.45 mmol).

### [Step 92-2s]

According to Step 39-2s in the synthetic method for **EXAMPLE aa39**, compound aa**92-1** (223 mg, 0.45 mmol) was used instead of compound **aa39-1** to obtain compound aa**92-2** (234 mg, 0.48 mmol).

### [Step 92-3s]

According to Step 39-3s in the synthetic method for **EXAMPLE aa39**, compound aa**92-2** (210 mg, 0.48 mmol) was used instead of compound aa**39-2** to obtain compound aa**92-3** (0.48 mmol) which was used in the next step without further purification.

### [Step 92-4s]

According to Step 54-4s in the synthetic method for **EXAMPLE aa54**, compound aa**92-3** (0.48 mmol) was used instead of compound aa**54-3** to obtain compound aa**92-4** (292 mg, 0.38 mmol).

### [Step 92-5s]

According to Step 39-5s in the synthetic method for **EXAMPLE aa39**, compound aa**92-4** (292 mg, 0.38 mmol) was used instead of compound **aa39-4** to obtain compound aa**92-5** (0.38 mmol) which was used in the next step without further purification.

### [Step 92-6s]

According to Step 54-6s in the synthetic method for **EXAMPLE aa54,** compound aa**92-5** (0.38 mmol) was used instead of compound aa**54-5** to obtain **(EXAMPLE aa92** (160 mg, 0.0.31 mmol) as a white amorphous solid.

### (EXAMPLE aa93)

### Preparation of 2-((S)-hydroxy((R)-3-oxo-4-p-tolylmorpholin-2-yl)methyl)-4-oxo-1.4-dihydroquinazoline-6-carboximidamide EXAMPLE aa93

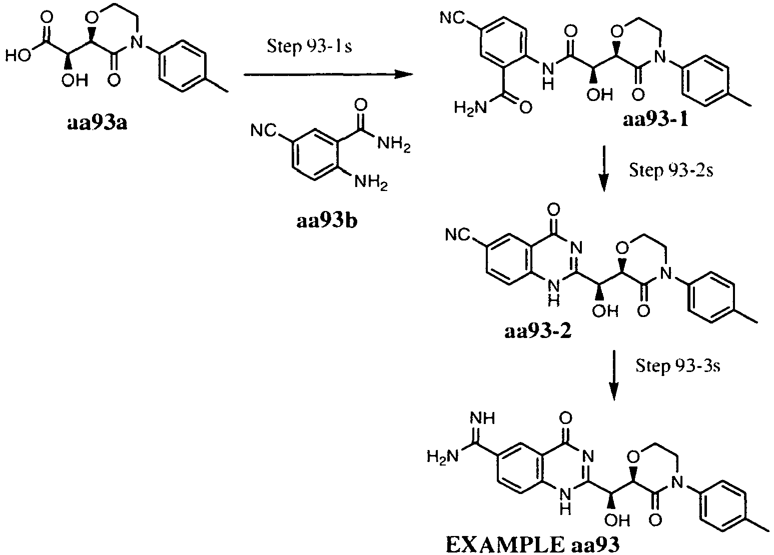

### [Step 93-1s]

To a solution of imidazole (258 mg, 3.79 mmol) in anhydrous DCM (4 mL) cooled at -10°C was added thionyl chloride (113 mg, 0.95 mmol). The reaction mixture was stirred at room temperature for 10 minutes and was added to a solution of compound **93b** (304 mg, 1.89 mmol) in anhydrous DCM (3 mL) cooled at -40 °C. The reaction mixture was stirred at room temperature for 30 minutes. The reaction mixture was filtered. The filtrate was added to a solution of compound aa**93a** (100 mg, 0.38 mmol) and 1.2,4-triazole (39 mg, 0.57 mmol) in anhydrous DCM (3 mL) cooled at 0 °C. The reaction mixture was stirred at room temperature for 16 hours. Ethyl acetate (100 mL) was added and the organic layer was washed with 2 N hydrochloric acid, saturated sodium bicarbonate solution and brine. The organic layer was dried over anhydrous sodium sulfate. The organic solvent was evaporated under reduced pressure. The crude product was purified by flash column chromatography to afford the desired aa**93-1** (15 mg, 0.037 mmol).

### [Step 93-2s]

To a solution of aa**93-1** (5 mg, 0.012 mmol) in 1,4-dioxane was added a 1 N sodium hydroxide solution (0.1 mL). The reaction mixture was stirred at room temperature for 2 hours. Ethyl acetate (50 mL) was added and the organic layer was washed with 1 N hydrochloric acid, water and brine. The organic solvent was evaporated under reduced pressure to afford the desired aa**93-2** (0.012 mmol). The crude product was used in the next step without further purification.

### [Step 93-3s]

Acetyl chloride (3 mL) was added to absolute ethanol (1 mL) cooled at 0 °C to generate a hydrogen chloride solution. To the hydrogen chloride solution was added a solution of aa**93-2** (0.012 mmol) in absolute ethanol (2 mL). The reaction mixture was stirred at room temperature for 2 days. The organic solvent was evaporated under reduced pressure. To the residue was added a 7 N solution of ammonia in methanol (5 mL). The reaction mixture was stirred at room temperature for 16 hours. The organic solvent was evaporated under reduced pressure. The crude product was purified by RP-HPLC to afford the desired EXAMPLE **aa93** (1 mg, 0.0023 mmol).

### (EXAMPLE aa94)

### Preparation of 1,1-dioxo-3-((S)-hydroxy((R)-3-oxo-4-p-tolylmorpholin-2-yl)methyl)-4H-benzo[e][1,2,4]thiadiazine-7-carboximidamide EXAMPLE aa94

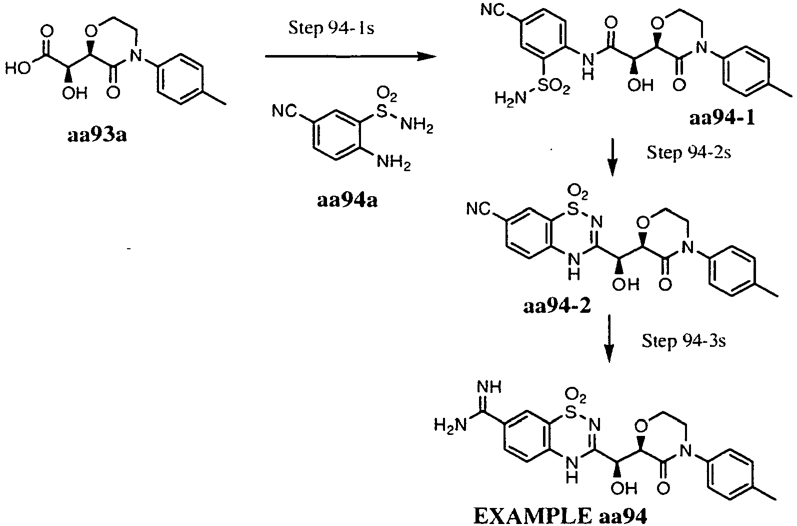

### [Step 94-1s]

According to Step 93-1s in the synthetic method for **EXAMPLE aa93**, compound aa**94a** (606 mg, 3.08 mmol) was used instead of compound aa**93b** to obtain compound aa**94-1** (86 mg, 0.19 mmol).

[Step 94-2s] To compound aa**94-1** (86 mg, 0.19 mmol) was added saturated ammonium hydroxide solution (8 mL). The reaction mixture was heated under reflux for 2 days. The solvent was evaporated under reduced pressure. The crude product was purified by RP-HPLC to afford the desired compound aa**94-2** (8.5 mg, 0.020 mmol).

[Step 94-3s] According to Step 93-3s in the synthetic method for **EXAMPLE aa93**, compound aa**94-2** (8.5 mg, 0.020 mmol) was used instead of compound aa**93-2** to obtain **EXAMPLE** aa**94** (7.2 mg, 0.016 mmol).

### EXAMPLES aa95 - aa116

### EXAMPLE aa95

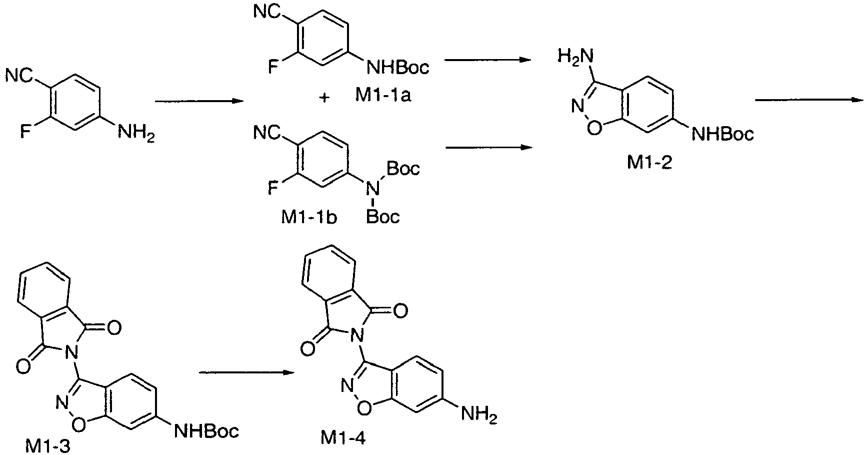

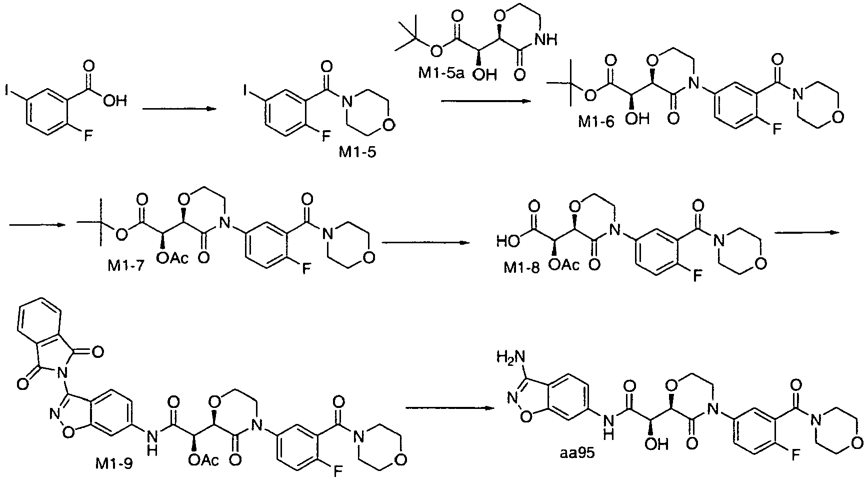

### Synthesis of tert-butyl 4-cyano-3-fluorophenylcarbamate (compound M1-1a) and (compound M1-1b)

4-amino-2-fluorobenzonitrile (10g, 0.0735mol) was dissolved in THF (50ml), NaH (2.94g of a 60% dispersion, 1eq) was added. After 30 minutes the resulting mixture was added to a mixture of Boc₂O (16g, 1eq) and DMAP (0.897g, 10%) in THF (100ml). This mixture was stirred for 2 hours and was diluted with EtOAc. The mixture was washed with NH₄Cl₍ₛₐₜ₎. dried (MgSO₄) and concentrated. The residue was purified by silica gel chromatography (0-60% EtOAc in hexane) to give, in order of elution. 7.7g (31%) of M-1-1b and 4.4g (25%) of M1-1a.

### Synthesis of tert-butyl 3-aminobenzo[d]isoxazol-6-ylcarbamate (compound M1-2)

Compound M1-1a or M1-1b (0.0229mol) was dissolved in 10:1 DMF/H₂O (114ml). Acetohydroxamic acid (10.3g, 6eq) and K₂CO₃ (38g, 12eq) were added and the mixture heated at 55°C overnight. After cooling to room temperature the mixture was diluted with EtOAc. washed with NH₄Cl₍ₛₐₜ₎ and dried (MgSO₄). The residue was purified by silica gel chromatography (0-70% EtOAc in hexane) to give 5g (87%) of M1-2.

### Synthesis of tert-butyl 3-(1,3-dioxoisoindolin-2-yl)benzo[d]isoxazol-6-ylcarbamate (compound M1-3)

Compound M 1-2 (5.3g, 0.0213mol) was dissolved in CH₂Cl₂ (106ml) and cooled to 0°C. o-phthaloyl dichloride (3.7ml, 1.2 eq) was added followed by Et₃N (7.18ml. 2.4eq). The mixture was stirred overnight. The mixture was diluted with EtOAc, washed with NH₄Cl₍ₛₐₜ₎ and dried (MgSO4). The residue was purified by silica gel chromatography (0-100% EtOAc in hexane) to give 5.6g of M1-3.

### Synthesis of 2-(6-aminobenzo[d]isoxazol-3-yl)isoindoline-1,3-dione (compound M1-4)

Compound M1-3 (1.37g, 0.00363mol) was dissolved in 1,4-dioxane (18ml). 4N HCl in 1,4-dioxane (18ml) was added and the mixture stirred overnight. The mixture was concentrated and the residue suspended in EtOAc. NaHCO₃(sat) was added and stirred until a clear organic layer persisted. The organic layer was dried (MgSO₄), and concentrated to give 1g of M 1-4 (98%).

### Synthesis of (2-fluoro-5-iodophenyl)(morpholino)methanone (compound M1-5)

2-Fluoro-5-iodobenzoic acid (2g, 0.0088mol) was dissolved in MeCN (44ml) and the mixture cooled to 0°C. Morpholine (0.929ml, 1.2eq) followed by EDCI.HCI (2.03g, 1.2eq) and DMAP (108mg, 0.1eq) were added and the mixture stirred overnight. The mixture was diluted with EtOAc, washed with NH₄Cl(sat), dried (MgSO₄), and concentrated. The resulting residue was then purified by silica gel chromatography (0-30% EtOAc in hexane) gave 2g of M 1-5. LCMS MH⁺ = 336.

### Synthesis of (R)-tert-butyl 2-((R)-4-(4-fluoro-3-(morpholine-4-carbonyl)phenyl)-3-oxomorpholin-2-yl)-2-hydroxy acetate. (compound M 1-6).

Compound M 1-5a (1.37g, 0.0059mol) and compound M1-1 (1.99g, 1eq) were dissolved in 1,4-dioxane (60ml), to this mixture were added CuI (339 mg, 0.2eq), Cs₂CO₃ (3.87g, 2eq), and *trans*-*N,N*-dimethylcyclohexane-1,2-diamine (0.281ml, 0.3eq). The resulting solution was degassed and heated at 90°C for 6.5 hours. The mixture was cooled to rt, NH₄Cl(sat) was added and the mixture extracted with EtOAc. The extracts were washed with NH₄Cl(sat), dried (MgSO₄), and concentrated. The resulting residue was then purified by silica gel chromatography (0-100% EtOAc in hexane) gave 1.6g of M1-6.

### Synthesis of (R)-tert-butyl 2-acetoxy-2-((R)-4-(4-fluoro-3-(morpholine-4-carbonyl)phenyl)-3-oxomorpholin-2-yl) acetate (compound M1-7)

M1-6 (1.6g, 0.00365mol) was dissolved in CH₂Cl₂ (18.25ml) and cooled to 0°C, Ac₂O (0.69ml, 2eq), pyridine (0.59ml, 2eq), and DMAP (45mg, 0.1eq) was added. The mixture was stirred for 1 hours, diluted with EtOAc, washed with CuSO₄ solution, water, dried, and concentrated to give 1.75g of M1-7.

### Synthesis of (R)-2-acetoxy-2-((R)-4-(4-fluoro-3-(morpholine-4-carbonyl)phenyl)-3-oxomorpholin-2 -yl)acetic acid (compound M1-8)

M1-7 (1.75g, 0.00364mol) was dissolved in 1:1 CH₂Cl₂/TFA (40ml) and stirred for 30 minutes. The mixture was concentrated to give 1.6g of M1-8.

### Synthesis of (R)-2-(3-(1,3-dioxoisoindolin-2-yl)benzo[d]isoxazol-6-ylamino)-1((R)-4-(4-fluoro-3-( morpholine-4-carbonyl)phenyl)-3-oxomorpholin-2-yl)-2-oxoethyl acetate (compound M1-9)

Compound M1-8 (1g, 0.00235mol) and compound M1-4 (724mg, 1.1eq) were dissolved in MeCN (4.71ml, 0.5M) and the mixture cooled to 0°C. EDCI.HCl (0.542g, 1.1eq) and DMAP (29mg, 10%) were added and the mixture stirred overnight. The mixture was diluted with EtOAc, washed with NH₄Cl₍ₛₐₜ₎, dried (MgSO₄). and concentrated. The residue was purified by silica gel chromatography (0-5% MeOH in CH₂Cl₂) to give 1g (63%) of M1-9.

### Synthesis of (R)-N-(3-aminobenzo[d]isoxazol-6-yl)-2-((R)-4-(4-fluoro-3-(morpholine-4-carbonyl)p henyl)-3-oxomorpholin-2-yl)-2-hydroxyacetamide (example aa95)

Compound M1-9 (1g, 0.00146mol) was dissolved in a 1:1 mixture of CH₂Cl₂/MeOH (58ml), NH₂NH₂ (0.458ml, 10eq) was added and the mixture stirred for 2 hours. The mixture was concentrated. The residue was purified by silica gel chromatography (0-5% MeOH in CH₂Cl₂) to give 0.5g (67%) of Example aa95.

### EXAMPLE aa96

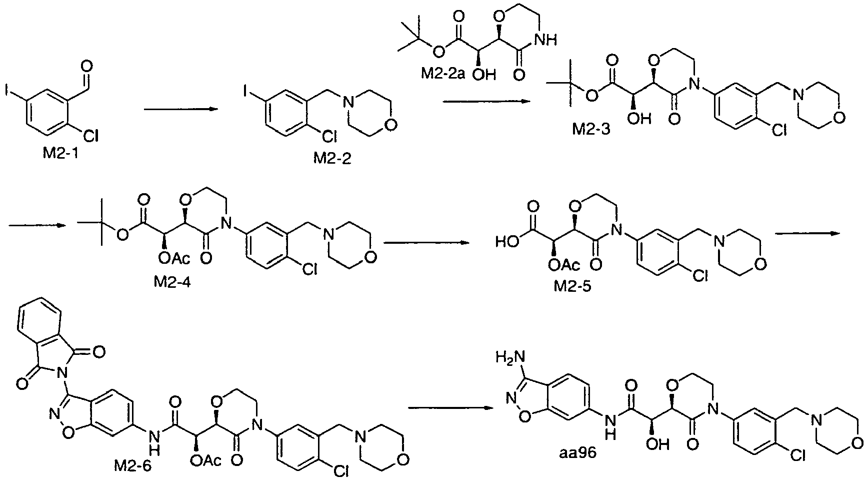

### Synthesis of 2-chloro-5-iodobenzaldehyde (compound M2-1)

M2-1 was synthesized using the procedure described in WO 2008/077009, page 39, example 27, steps (a) to (b).

### Synthesis of 4-(2-chloro-5-iodobenzyl)morpholine (compound M2-2)

M2-1 (0.875g, 0.0033mol) was dissolved in DCE (8.2ml), morpholine (0.373ml, 1.3eq) and AcOH (0.94ml, 5eq) were added and the mixture stirred for 30 minutes. Na(OAc)₃BH (1.392g, 2eq) was added and the mixture stirred overnight. The mixture was diluted with EtOAc and washed with NaHCO₃₍ₛₐₜ₎. dried (MgSO4), and concentrated. The residue was dissolved in ether and acidified with 1M HCl in ether. The resulting precipitate was collected, suspended in EtOAc, and neutralized with NaHCO₃₍ₛₐₜ₎. The organic layer was dried (MgSO4) and concentrated to give 305mg of M2-2.

### Synthesis of (R)-tert-butyl 2-((R)-4-(4-chloro-3-(morpholinomethyl)phenyl)-3-oxomorpholin-2-yl)-2-hydroxyacet ate (compound M2-3).

Compound M2-2a (0.23g, 0.001 mol) and compound M2-2 (0.305g, 1eq) were dissolved in DMSO (10ml), to this mixture were added CuI (57 mg, 0.2eq), K₃PO₄ (422mg, 2eq), and *trans*-*N,N*-dimethylcyclohexane-1,2-diamine (0.047ml, 0.3eq). The resulting solution was degassed and heated at 90°C for 1.5 hours. The mixture was cooled to rt, NH₄Cl(sat) was added and the mixture extracted with EtOAc. The extracts were washed with NH₄Cl(sat), dried (MgSO₄), and concentrated. The resulting residue was then purified by silica gel chromatography (0-100% EtOAc in hexane) gave 266mg of M2-3.

### Synthesis of (R)-tert-butyl 2-acetoxy-2-((R)-4-(4-chloro-3-(morpholinomethyl)phenyl)-3-oxomorpholin-2-yl)aceta te (compound M2-4).

Compound M2-4 was synthesized using a procedure similar to the synthesis of compound M1-7.

### Synthesis of (R)-2-acetoxy-2-((R)-4-(4-chloro-3-(morpholinomethyl)phenyl)-3-oxomorpholin-2-yl) acetic acid (compound M2-5).

M2-4 (230mg, 0.000476) was treated with 4M HCl in dioxane for 16 hours. The mixture was then concentrated to give M2-5 as a hydrochloride salt.

### Synthesis of (R)-1-((R)-4-(4-chloro-3-(morpholinomethyl)phenyl)-3-oxomorpholin-2-yl)-2-(3-(1,3-dioxoisoindolin-2-yl)benzo[d]isoxazol-6-ylamino)-2-oxoethyl acetate (compound M2-6)

M2-5.HCl (100mg, 0.000216) and M1-4 (66mg, 1.1eq) were dissolved in MeCN and cooled to 0°C. DMAP (3 mg, 0.1eq), pyridine (0.017ml, 1eq), and EDCI.HCl (50 mg, 1.2eq) were added and the mixture stirred for 2 hours. The mixture was diluted with EtOAc, washed with NH₄Cl₍ₛₐₜ₎. dried (MgSO₄). and concentrated. The residue was purified by silica gel chromatography (0-5% MeOH in CH₂Cl₂) to give 40mg of M2-6.

### Synthesis of (R)-N-(3-aminobenzo[d]isoxazol-6-yl)-2-((R)-4-(4-chloro-3-(morpholinomethyl)pheny l)-3-oxomorpholin-2-yl)-2-hydroxyacetamide (Example aa96)

Example aa96 was synthesized using a procedure similar to the synthesis of compound aa95. Additionally an ether solution of aa96 was converted to the hydrochloride salt by treatment with 1M HCl in ether and isolated by filtration.

### EXAMPLE aa97

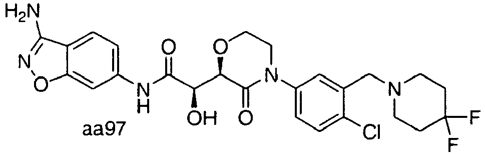

(R)-N-(3-aminobenzo[d]isoxazol-6-yl)-2-((R)-4-(4-chloro-3-((4,4-difluoropiperidin-1-y l)methyl)phenyl)-3-oxomorpholin-2-yl)-2-hydroxyacetamide (Example aa97) was synthesized in a similar manner to example aa96 using previously described procedures.

### EXAMPLE aa98

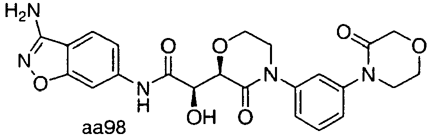

(R)-N-(3-aminobenzo[d]isoxazol-6-yl)-2-hydroxy-2-((R)-3-oxo-4-(3-(3-oxomorpholin o)phenyl)morpholin-2-yl)acetamide (Example aa98) was synthesized in a similar manner to example aa95 using previously described procedures.

### EXAMPLE aa99

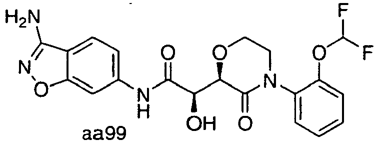

(R)-N-(3-aminobenzo[d]isoxazol-6-yl)-2-((R)-4-(2-(difluoromethoxy)phenyl)-3-oxomo rpholin-2-yl)-2-hydroxyacetamide (Example aa99) was synthesized in a similar manner to example aa95 using previously described procedures.

### EXAMPLE aa100

(R)-N-(3-aminobenzo[d]isoxazol-6-yl)-2-hydroxy-2-((R)-4-(2-isopropoxyphenyl)-3-ox omorpholin-2-yl)acetamide (Example aa100) was synthesized in a similar manner to example aa95 using previously described procedures.

### EXAMPLE aa101

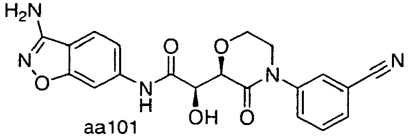

(R)-N-(3-aminobenzo[d]isoxazol-6-yl)-2-((R)-4-(3-cyanophenyl)-3-oxomorpholin-2-yl )-2-hydroxyacetamide (Example aa101) was synthesized in a similar manner to example aa95 using previously described procedures.

### EXAMPLE

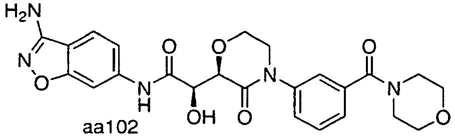

(R)-N-(3-aminobenzo[d]isoxazol-6-yl)-2-hydroxy-2-((R)-4-(3-(morpholine-4-carbonyl) phenyl)-3-oxomorpholin-2-yl)acetamide (Example aa102) was synthesized in a similar manner to example aa95 using previously described procedures.

### EXAMPLE aa 103

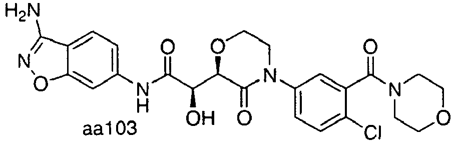

(R)-N-(3-aminobenzo[d]isoxazol-6-yl)-2-((R)-4-(4-chloro-3-(morpholine-4-carbonyl)p henyl)-3-oxomorpholin-2-yl)-2-hydroxyacetamide (Example aa103) was synthesized in a similar manner to example aa95 using previously described procedures.

### EXAMPLE aa 104

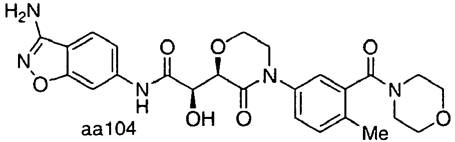

(R)-N-(3-aminobenzo[d]isoxazol-6-yl)-2-hydroxy-2-((R)-4-(4-methyl-3-(morpholine-4 -carbonyl)phenyl)-3-oxomorpholin-2-yl)acetamide (Example aa104) was synthesized in a similar manner to example aa95 using previously described procedures.

### EXAMPLE aa 105

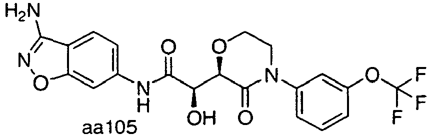

(R)-N-(3-aminobenzo[d]isoxazol-6-yl)-2-hydroxy-2-((R)-3-oxo-4-(3-(trifluoromethoxy )phenyl)morpholin-2-yl)acetamide (Example aa105) was synthesized in a similar manner to example aa95 using previously described procedures.

### EXAMPLE aa 106

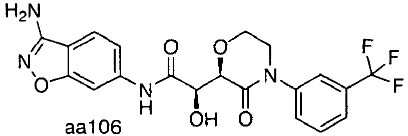

(R)-N-(3-aminobenzo[d]isoxazol-6-yl)-2-hydroxy-2-(R)-3-oxo-4-(3-(trifluoromethyl)p henyl)morpholin-2-yl)acetamide (Example aa106) was synthesized in a similar manner to example aa95 using previously described procedures.

### EXAMPLE aa 107

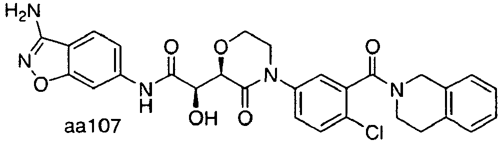

(R)-N-(3-aminobenzo[d]isoxazol-6-yl)-2-((R)-4-(4-chloro-3-(1,2,3,4-tetrahydroisoquin oline-2-carbonyl)phenyl)-3-oxomorpholin-2-yl)-2-hydroxyacetamide (Example aa107) was synthesized in a similar manner to example aa95 using previously described procedures.

### EXAMPLE aa108

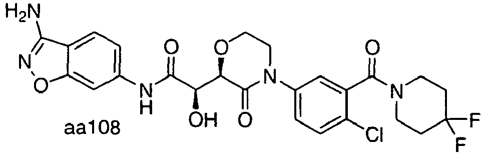

(R)-N-(3-aminobenzo[d]isoxazol-6-yl)-2-((R)-4-(4-chloro-3-(4,4-difluoropiperidine-1-carbonyl)phenyl)-3-oxomorpholin-2-yl)-2-hydroxyacetamide (Example aa108) was synthesized in a similar manner to example a95 using previously described procedures.

### EXAMPLE aa 109

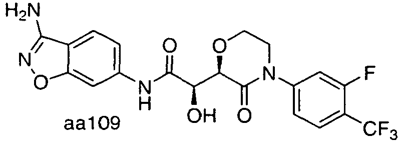

(R)-N-(3-aminobenzo[d]isoxazol-6-yl)-2-((R)-4-(3-fluoro-4-(trifluoromethyl)phenyl)-3 -oxomorpholin-2-yl)-2-hydroxyacetamide (Example aa109) was synthesized in a similar manner to example a95 using previously described procedures.

### EXAMPLE aa110

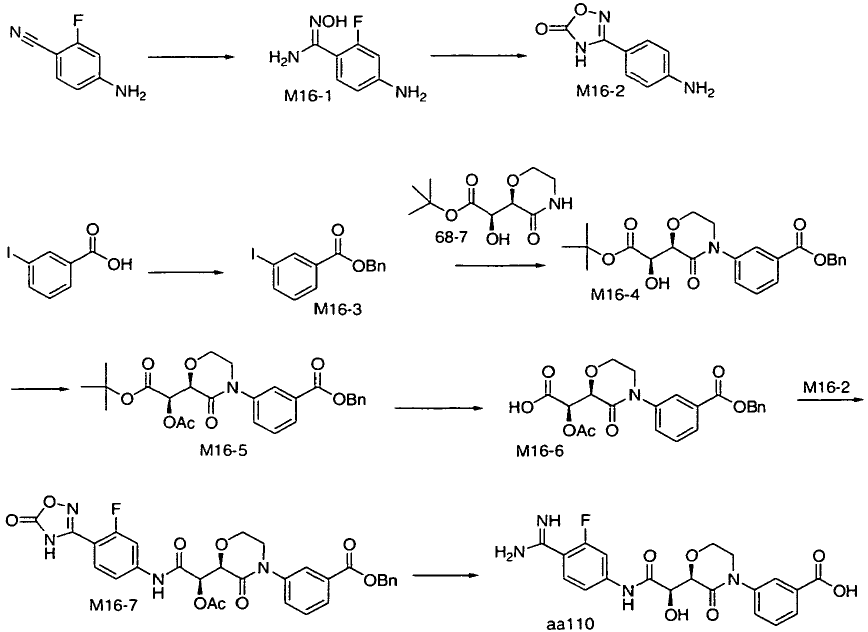

### Synthesis of 4-amino-2-fluoro-N'-hydroxybenzimidamide (compound M16-1)

4-cyano-3-fluoroaniline (10g, 0.073mol) dissolved in EtOH (36ml). Water (7.3mg) was added followed by Na₂CO₃ (5.06g, 0.65eq) and the temperature of the mixture raised to 60°C. NH₂OH.HCl (5.6g, 1.1eq) in water (7.3ml) was added slowly, and the mixture heated at 60°C overnight. The mixture was cooled to rt, the solid was collected by filtration, washed with, water (7ml), EtOH (7ml), ether (20ml), and dried to give 7.5g of M16-1.

### Synthesis of 3-(4-aminophenyl)-1,2,4-oxadiazol-5(4H)-one (compound M16-2)

M 6-1 (7.5g. 0.044mol) was suspended in EtOH (26ml), diethyl carbonate (5.34ml, 1 eq) was added and the mixture warmed to 65°C. NaOEt (16.5g of a 21% wt solution in EtOH, 1.15eq) added slowly and the temperature of the mixture raised to 70°C for 2 hours. The mixture was cooled to rt, concentrated and dissolved in water (25 ml) at 70°C, HCl(c) added to bring the PH to 2, the mixture was cooled to 0°C. The solid was collected by filtration and washed with water (20ml), EtOH (7 ml), and ether (20 ml) to give 6.4g of M 16-2.

### Synthesis of 1-(3-iodophenyl)-2-phenylethanone (compound M16-3)

m-Iodobenzoic acid (1g, 0.004mol) was dissolved in MeCN (20ml), Cs₂CO₃ (2.63g, 2eq) and benzyl bromide (0.528ml, 1.1eq) added. The mixture was heated at reflux overnight. The mixture was concentrated, taken up in EtOAc, washed with water, dried (MgSO₄), and concentrated. The residue was purified by silica gel chromatography (0-60% EtOAc in hexane) to give 1.6g of M 16-3.

### Synthesis of benzyl 3-((R)-2-((R)-1-acetoxy-2-(3-fluoro-4-(5-oxo-4,5-dihydro-1,2,4-oxadiazol-3-yl)phenyl amino)-2-oxoethyl)-3-oxomorpholino)benzoate (compound M16-7)

Compound M16-3 was converted to compound M 16-7 using previously described procedures.

### Synthesis of 3-((R)-2-((R)-2-(4-carbamimidoyl-3-fluorophenylamino)-1-hydroxy-2-oxoethyl)-3-oxo morpholino)benzoic acid (Example aa110)

Compound M16-7 (200mg, 0.00033mol) was dissolved in MeOH (5.5ml), 7N NH₃ (0.284ml, 6eq) was added, and the mixture stirred for 1 hour. The mixture was concentrated, taken up in MeOH (5ml). 1M HCl (0.662ml, 2eq) was added followed by 10% Pd(C) (100mg). The mixture was put under H₂ (1 atm) for 1 hour. The solids were removed by filtration, the mixture was concentrated. The residue was triturated with ether/MeOH, and solid collected to give 143 mg of Example aa110.

### EXAMPLE aa111

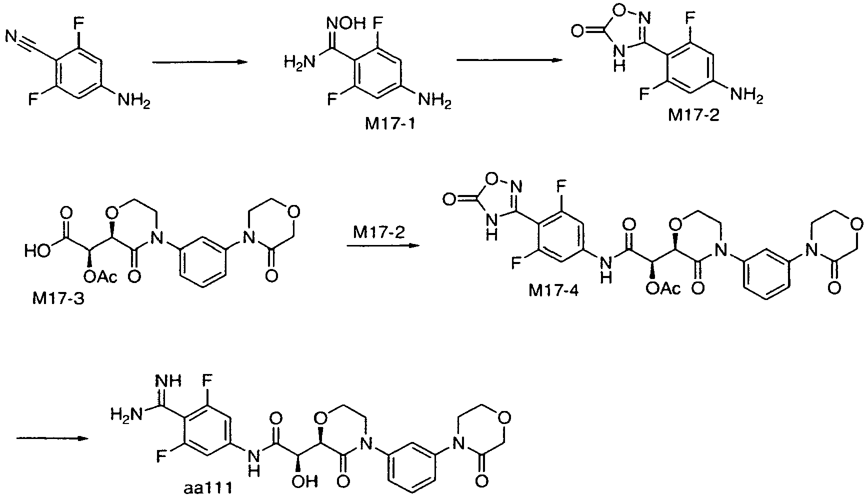

(R)-N-(4-carbamimidoyl-3.5-difluorophenyl)-2-hydroxy-2-((R)-3-oxo-4-(3-(3-oxomor pholino)phenyl)morpholin-2-yl)acetamide (Example aa111) was synthesized in a similar manner to previously described examples.

### EXAMPLE aa112

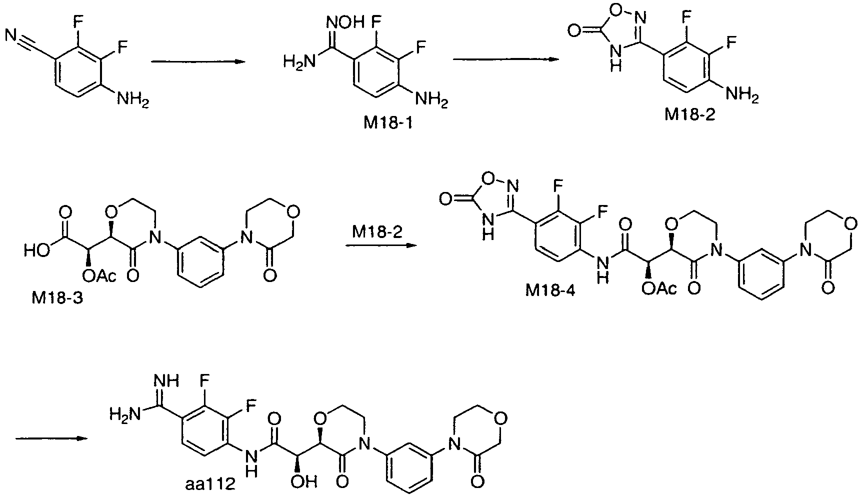

(R)-N-(4-carbamimidoyl-2,3-difluorophenyl)-2-hydroxy-2-((R)-3-oxo-4-(3-(3-oxomor pholino)phenyl)morpholin-2-yl)acetamide (Example aa112) was synthesized in a similar manner to previously described examples.

### EXAMPLE aa113

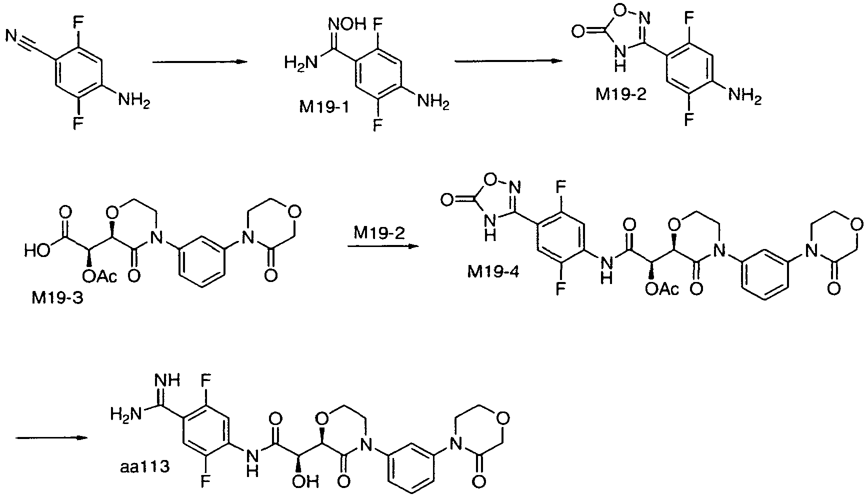

(R)-N-(4-carbamimidoyl-2,5-difluorophenyl)-2-hydroxy-2-((R)-3-oxo-4-(3-(3-oxomor pholino)phenyl)morpholin-2-yl)acetamide (Example aa113) was synthesized in a similar manner to previously described examples.

### EXAMPLE aa114

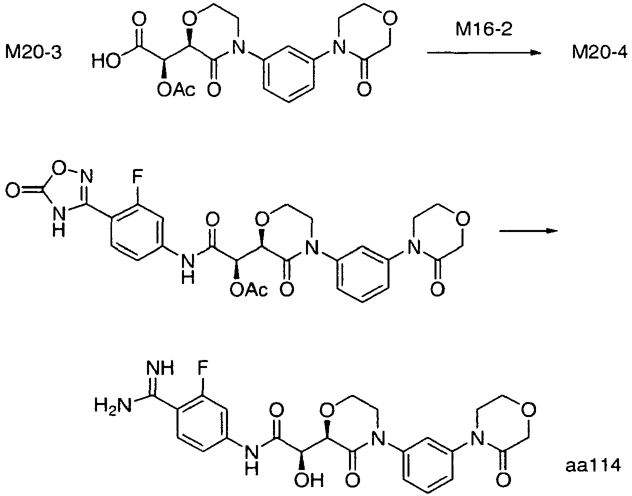

(R)-N-(4-carbamimidoyl-3-fluorophenyl)-2-hydroxy-2-((R)-3-oxo-4-(3-(3-oxomorphol ino)phenyl)morpholin-2-yl)acetamide (Example aa114) as synthesized in a similar manner to previously described examples.

### EXAMPLES aa115 and aa 116

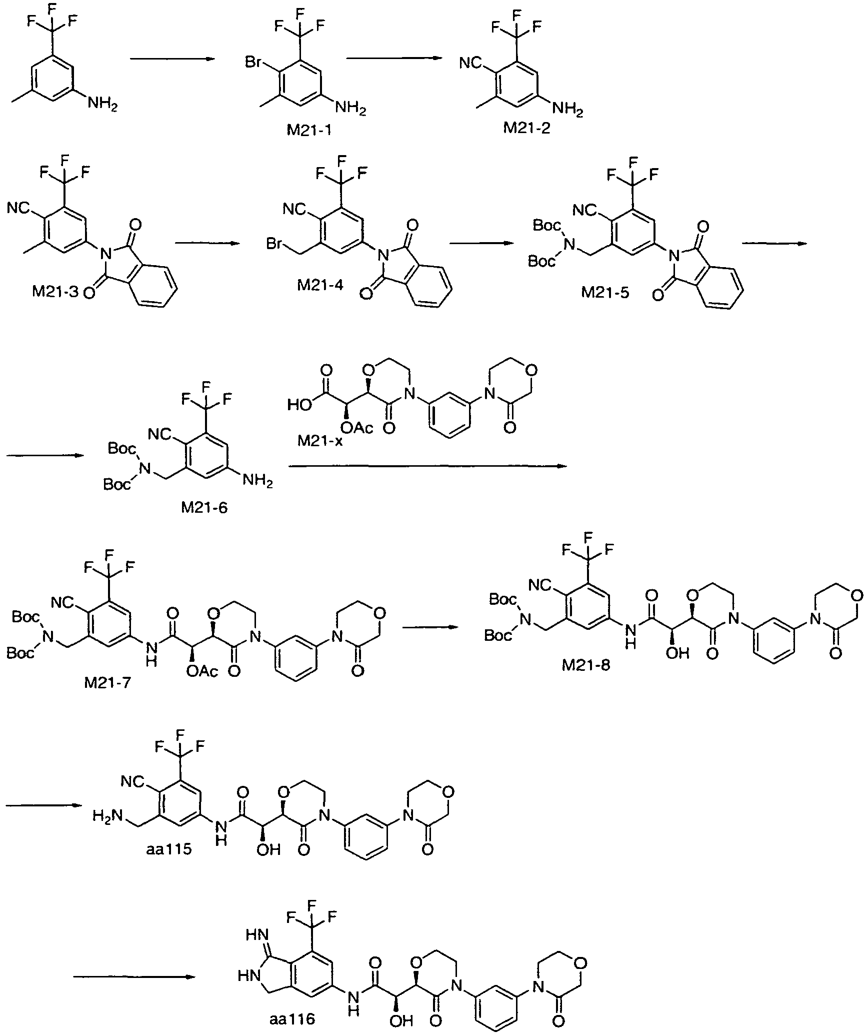

### Synthesis of 4-bromo-3-methyl-5-(trifluoromethyl)aniline (Compound M21-1)

3-methyl-5-(tritluoromethyl)aniline (4.84g, 0.011mol) was dissolved in CH₂Cl₂ and cooled to 0°C. NBS (4.92g, 1eq) was added and the mixture stirred overnight. The reaction mixture was loaded onto silica gel and eluted with 0-20% EtOAc in hexane to give 2.3g of M21-1.

### Synthesis of 4-amino-2-methyl-6-(trifluoromethyl)benzonitrile (Compound M21-2)

M21-1 (2.3g, 0.009053mol) was dissolved in NMP (45ml). Pd(Ph₃P)₄ (0.937mg, 0.1eq), Zn(CN)₂ (1.27g, 1.2eq), and Zn powder (0.592g, 1eq) were added. The mixture was degassed and heated at 110°C overnight. After cooling to rt the mixture was diluted with EtOAc and washed with saturated aqueous NaHCO₃ solution. The extracts were dried (MgSO4) and concentrated. The residue was purified by silica gel chromatography (0-30%, EtOAc in hexane) to give 1.5g of M21-2

### Synthesis of 4-(1,3-dioxoisoindolin-2-yl)-2-methyl-6-(trifluoromethyl)benzonitrile (Compound M21-3)

M21-2 (1.5g, 0.00614mol), was dissolved in AcOH (30ml), phthalic anhydride (1g, 1.1eq) was added and the mixture heated at 130°C for 2 hours. The mixture was cooled to rt and concentrated. The residue was purified by silica gel chromatography (0-30% EtOAc in hexane) to give 2.0g of M21-3.

### Synthesis of 2-(bromomethyl)-4-(1,3-dioxoisoindolin-2-yl)-6-(trifluoromethyl)benzonitrile (Compound M21-4)

M21-3 (2.6g, 0.0078mol) was dissolved in CCl₄ (39ml). AIBN (0.259g, 0.2eq) and NBS (1.822g, 1.3 eq) was added and the mixture heated at reflux while being irradiated with a 250W lamp for 2 days. The mixture was concentrated and the residue was purified by silica gel chromatography (0-30% EtOAc in hexane) to give 2.15g of M21-4.

### Synthesis of Compound M21-5

M21-4 (2.15g, 0.00525mol) was dissolved in DMF (52ml), K₂CO₃ (1.45g, 2eq) and (Boc)₂NH (2.28g, 2eq) was added and the mixture stirred overnight. The mixture was diluted with EtOAc and washed with NH₄Cl₍ₛₐₜ₎. The organic layers were dried (MgSO₄) concentrated and the residue was purified by silica gel chromatography (0-30% EtOAc in hexane) to give 1.4g of M21-5.

### Synthesis of Compound M21-6

M21-5 (1.4g, 0.00257) was dissolved in 1:1I MeOH/CH₂Cl₂ (25ml) and cooled to 0°C. Hydrazine (1.6ml, 20eq) was added, after 15 minutes the mixture was allowed to warm to rt and then stirred for an additional 1.5 hours. The resulting suspension was filtered and the filtrate purified by silica gel chromatography (0-40% EtOAc in hexane) to give 0.583g of M21-6.

### Synthesis of Compound M20-7

M21-6 (0.538g, 0.0013mol) and M21-x (disclosed previously in this filing) (0.661g, 1.3eq) were dissolved in MeCN (2.6ml) and cooled to 0°C. EDCI.HCl(0.372g, 1.5eq) and DMAP (16mg, 0,1eq) were added and the mixture stirred overnight. The mixture was diluted with EtOAc, washed with NH₄Cl₍ₛₐₜ₎, the extracts were dried (MgSO₄), and concentrated. The residue was purified by silica gel chromatography (0-100% EtOAc in hexane) to give 167mg of M21-7.

### Synthesis of Compound M21-8

M21-7 (167mg, 0.00021mol) was treated with 7N NH₃ in MeOH for 1 hour. The mixture was concentrated and the residue was purified by silica gel chromatography (0-5% MeOH in CH₂Cl₂) to give 60mg of M21-8.

### EXAMPLE aa115

Compound M21-8 (60 mg) was treated with 4M HCl in dioxane for 1 hour. The mixture was concentrated to give 60mg of (R)-N-(3-(aminomethyl)-4-cyano-5-(trifluoromethyl)phenyl)-2-hydroxy-2-((R)-3-oxo-4-(3-(3-oxomorpholino)phenyl)morpholin-2-yl)acetamide (Example aa115).

### EXAMPLE aa116

Example aa 115 (5mg) was dissolved in EtOH and heated at 90°C for 2 hours. After cooling to rt the mixture was concentrated to give 4 mg of (R)-2-hydroxy-N-(1-imino-7-(trifluoromethyl)isoindolin-5-yl)-2-((R)-3-oxo-4-(3-(3-ox omorpholino)phenyl)morpholin-2-yl)acetamide (example aa 116).

| EXAMPLE | LC/MS | | |
|---|---|---|---|
| | m/z | RT | Solvent system |
| | *[M+1]⁺ | min | |
| aa95 | 514 | 1.29 | A |
| aa96 | 516 | 1.27 | B |
| aa97 | 550 | 1.55 | A |
| aa98 | 482 | 1.26 | A |
| aa99 | 448 | 1.44 | A |
| aa100 | 441 | 1.54 | A |
| aa101 | 430[MNa]⁺ | 1.70 | B |
| aa102 | 496 | 1.30 | A |
| aa103 | 530 | 1.34 | A |
| aa104 | 510 | 1.32 | A |
| aa105 | 467 | 1.80 | A |
| aa106 | 451 | 1.86 | B |
| aa107 | 576 | 1.84 | A |
| aa108 | 564 | 0.81 | B |
| aa109 | 469 | 1.81 | A |
| aa110 | 431 | 1.19 | A |
| aa111 | 504 | 1.18 | A |
| aa112 | 504 | 1.18 | A |
| aa113 | 504 | 1.48 | B |
| aa114 | 486 | 1.23 | A |
| aa115 | 548 | 1.34 | A |
| aa116 | 548 | 1.29 | A |

| | | | |
|---|---|---|---|
| * except when noted. Solvent A: Column: Agilent SBC (3.0x50 mm, 1.8u); Flow: 1.0 ml/min; solvent A: H2O-0.1% TFA: Solvent B: ACN-0.1% TFA; Gradient Table : 0.1 min: 5%B, 2.3 min: 99%B, 2.90 min: 99%B, 3.0 min: 5%B stop time 3.50 min. Solvent B: Column: Agilent SBC (3.0x50 mm, 1.8u); Flow: 1.0 ml/min; solvent A: H2O-0.1% TFA: Solvent B: ACN-0.1% TFA; Gradient Table : 0 min: 10%B, 1.5 min: 95%B, 2.76 min: 10%B, stop time 3.60 min, Post Time 0.70 min. | | | |

| EXAMPLE | NMR ppm 400 MHz |
|---|---|
| aa95 | *DMSO-d₆* 9.98 (1H, s), 8.01 (1H, m), 7.68 (1H, d. J = 8.8 Hz), 7.58-7.55 (1H, m), 7.54-7.48 (2H, m), 7.37 (1H, t, J = 9.1 Hz), 6.43 (1H, J = 6.6 Hz), 6.31 (2H, s), 4.68-4.66 (1H, m), 4.65-4.63 (1H, m), 4.14-4.10 (1H, m), 3.95-3.83 (2H, m), 3.66-3.61 (5H, m), 3.54-3.52 (2H, m), 3.26-3.23 (2H, m). |
| aa96 | *DMSO-d₆* 10.00 (1H, s), 8.02 (1H, m), 7.97 (1H, s), 7.69 (1H, d, J = 8.5 Hz), 7.63 (2H, m), 7.56-7.54 (1H, m), 4.72 (1H, m), 4.64 (1H, m), 4.48 (1H, br s), 4.20-4.13 (1H, m), 3.98-3.69 (11H, m), 3.33-3.21 (4H, m). |
| aa97 | *DMSO-d₆* 10.00 (1H, s), 8.02 (1H, m), 7.96-7.90 (1H, m), 7.69 (1H, d, = 8.5 Hz), 7.64-7.61 (1H, m), 7.56-7.54 (1H, m), 4.72 (1H, m), 4.65 (1H, m), 4.58-4.51 (1H, m), 4.18-4.14 (1H, m), 3.97-3.70 (9H, m), 3.55-3.24 (4H, m), 2.40-2.30 (3H, m). |
| aa98 | *DMSO-d₆* 9.98 (1H, s), 8.02 (1H, m), 7.68 (1H, d, J = 8.5Hz), 7.56 (1H, dd, J = 7.8, 1.5 Hz), 7.48-7.46 (1H, m), 7.43 (1H, d, J = 7.8 Hz), 7.33-7.29 (2H, m), 6.43 (1H, d, J = 6.5 Hz), 6.32 (2H, s), 4.67-4.63 (2H, m), 4.2 (2H, s), 4.15-4.10 (1H, m), 3.98-3.95 (2H, m), 3.93-3.82 (2H, m), 3.75-3.71 (2H, m), 3.70-3.62 (1H, m). |
| aa99 | *DMSO-d₆* 10.01 (1H, s), 8.02 (1H, m), 7.68 (1H, d, J = 8.05), 7.58 (1H, m), 7.45-7.38 (2H, m), 7.35-7.27 (2H, m), 7.37 (1H, d, J = 7.01 Hz), 6.31 (2H, m), 4.66 (1H, m), 4.63-4.61 (1H, m), 4.18-4.13 (1H, m), 3.92-3.76 (2H, m), 3.45-3.4 (1H, m). |
| aa100 | *DMSO-d₆* 9.99 (1H, s), 8.02 (1H, m), 7.68 (1H, d, J = 8.4 Hz), 7.59-7.56 (1H, m), 7.30-7.26 (1H, m), 7.18-7.15 (1H, m), 7.13-7.11 (1H, m), 7.96-7.92 (1H, m), 6.36-6.30 (3H, m), 4.63-4.56 (3H, m), 4.16-4.10 (1H, m), 3.89-3.33 (1H, m), 3.79-3.67 (1H, m), 3.40-3.35 (1H, m), 1.25 (3H, s), 1.24 (3H, s). |
| aa101 | *DMSO-d₆* 9.98 (1H, s), 8.02 (1H, m), 7.95 (1H, m), 7.81-7.79 (1H, m), 7.75-7.73 (1H, m), 7.69-7.61 (2H, m), 7.58-7.55 (1H, m), 6.44 (1H, d, J = 7.0 Hz), 6.32 (2H, m), 4.7 (1H, m), 4.66-4.64 (1H, m), 4.17-4.12 (1H, m) 3.96-3.87 (2H, m), 3.74-3.68 (1H, m). |
| aa102 | *DMSO-d₆* 9.99 (1H, s), 8.07-8.02 (2H, m), 7.87-7.85 (1H, m), 7.68 (1H, d, J = 8.9 Hz), 7.57 (1H, d, J = 8.76 Hz), 7.48 (2H, m), 7.32-7.26 (1H,m), 7.46-7.41 (1H, m), 6.32 (2H, s), 4.69 (1H, s), 4.66-4.60 (1H, m), 4.15-4.09 (1H, m), 3.96-3.85 (2H, m), 3.69-3.5 (8H, m). |
| aa103 | *DMSO-d₆* 9.97 (1H, s), 8.01 (1H, m), 7.68 (1H, d, J = 8.5 Hz), 7.58-7.50 (4H, m), 6.43 (1H, dd, J = 9.0, 6.5 Hz), 6.31 (2H, s). 4.68 (1H, m), 4.64 (1H, d, J = 7.0 Hz), 4.15-4.06 (2H, m), 3.95-3.84 (2H, m), 3.70-3.53 (6H, m), 3.16-3.14 (2H, m). |
| aa104 | *DMSO-d₆* 9.99 (1H, s). 8.04 (1H, m), 7.71 (1H, d, J = 8.5 Hz), 7.60-7.58 (1H, m), 7.38-7.29 (3H, m), 6.44-6.42 (1H, s), 6.33 (2H, s), 4.70-4.65 (2H, m), 4.16-4.11 (1H, m), 3.98-3.83 (2H, m), 3.70-3.62 (4H, m). 3.56-3.48 (2H, m), 3.20-3.14 (2H, m), 2.24 (3H, s). |
| aa105 | *DMSO-d₆* 9.98 (1H, s), 8.01 (1H, m), 7.68 (1H, d, J = 8.66 Hz), 7.58-7.53 (3H, m), 7.47 (1H, d, J = 6.2 Hz), 7.28 (1H, d, J = 8 Hz), 6.44 (1H, d, J = 6.88 Hz), 6.32 (2H, s), 4.70 (1H, m), 4.66-4.63 (1H, m), 4.15-4.10 (1H, m), 3.97-3.86 (2H, m), 3.73-3.65 (1H, m), |
| aa106 | *DMSO-d₆* 9.98 (1H, s), 8.02 (1H, m), 7.84 (1H, m), 7.44-7.62 (4H, m), 7.56 (1H, dd. J = 8.5, 1.5 Hz), 6.46 (1H, d, J = 6.8 Hz), 6.32 (2H, m), 4.71 (1H, m), 4.66-4.64 (1H, m), 4.16-4.12 (1H, m), 3.97-3.87 (2H, m), 3.75-3.69 (1H, m). |
| aa107 | *DMSO-d₆* 9.97 (1H, s), 8.0 (1H, m), 7.67 (1H, d, J = 8.4 Hz), 7.61 (1H, d, J = 9.2 Hz), 7.58-7.53 (3H, m), 7.27-7.10 (4H, m), 6.46-6.42 (1H, m), 6.31 (2H, s), 4.70-4.62 (2H, m), 4.37 (1H, m), 4.14-4.10 (1H, m), 3.95-3.83 (3H, m), 3.72-3.65 (1H, m), 3.44-3.41 (1H, m), 3.29 (1H, m). 2.90-2.86 (1H, m), 2.82-2.79 (1H, m). |
| aa108 | *DMSO-d₆* 9.97 (1H, s), 8.01 (1H, m), 7.68 (1H, d, J = 8.44 Hz), 7.60-7.51 (4H, m), 6.43 (1H, t, J = 5.5 Hz), 6.32 (2H, s), 4.70 (1H, s), 4.64 (1H, d, J= 6.7 Hz), 4.16-4.10 (1H, m), 3.96-3.81 (3H, m), 3.29-3.24 (2H, m), 2.15-2.89 (5H, m). |
| aa109 | *DMSO-d₆* 9.98 (1H, s), 8.01 (1H, m), 7.84 (1H, t, J = 8.5 Hz), 7.75-7.71 (1H, m), 7.68 (1H, d, J = 8.8 Hz), 7.56 (2H, d, J = 8.3 Hz), 6.44 (1H, d, J = 6.88 Hz), 6.32 (2H, s), 4.74 (1H, m), 4.66-4.64 (1H, m), 4.17-4.14 (1H, m), 3.93 (2H, q, J = 9.5 Hz), 3.76-3.73 (1H, m). |
| aa110 | *CD₃OD* 10.01 (1H, s), 8.05 (1H, m), 7.99-7.95 (2H, m), 7.67-7.62 (3H, m), 7.56 (1H, t, J = 7.5Hz), 4.83-4.81 (2H, m,), 4.25-4.21 (1H, m), 3.70-3.66 (1H, m). 3.34 (2H, m). |
| aa111 | *CD₃OD* 7.71 (2H, d, J = 10.64), 7.54-7.45 (2H, m), 7.38-7.33 (2H, m), 4.82-4.78 (2H, m), 4.28 (2H, m), 4.24-4.18 (1H, m), 4.07-3.97 (4H, m), 3.82-3.78 (2H, m). 3.73-3.68 (1H, m). |
| aa112 | *CD₃OD* 8.31-8.27 (1H, m), 7.54-7.46 (3H, m), 7.38-7.33 (2H, m), 4.81 (2H, m), 4.31-4.27 (2H, m), 4.23-4.20 (1H, m), 4.07-4.00 (4H, m), 3.83-3.79 (2H, m), 3.70-3.67 (1H, m). |
| aa113 | *CD₃OD* 8.46-8.41 (1H, m), 7.66-7.62 (1H, m), 7.52 (1H, t, J = 8.05 Hz), 7.47 (1H, m), 7.38-7.34 (2H, m), 4.81 (2H, m), 4.28 (2H, m), 4.20 (1H, dd, J = 2.5, 8.4 Hz), 4.07-4.02 (4H, m), 3.82-3.78 (2H, m), 3.67-3.62 (1H, m). |
| aa114 | *CD₃OD* 7.96 (1H, d, J = 13.9Hz), 7.66-7.65 (2H, m), 7.52 (1H, t, J = 8.06Hz), 7.47 (1H, m), 7.38 (2H, m), 4.82-4.80 (2H, m), 4.28 (2H, m), 4.25-4.18 (1H, m), 4.06-4.00 (4H, m), 3.82-3.79 (2H, m), 3.72-3.66 (1H, m). |
| aa115 | *DMSO-d₆* 10.66 (1H, s), 8.69 (1H, s), 8.44 (2H, s), 8.39 (1H, m), 7.42 (1H, m), 7.44 (1H, d, J = 8.1Hz), 7.24-7.12 (1H, m), 7.33-7.29 (2H, m), 6.64 (1H, d, J = 6.7Hz), 4.71-4.67 (2H, m), 4.24 (1H, s), 4.19 (2H, s), 4.13-4.09 (1H, m), 3.97 (1H, t, J = 5Hz), 3.91-3.82 (2H, m). 3.74-3.6 (3H, m). 3.54 (2H, s). |
| aa116 | *DMSO-d₆* 10.60 (1H, s), 8.64 (1H, s), 8.48 (1H, s), 7.48-7.43 (2H, m0, 7.32-7.29 (2H, m), 6.59 (1H, d, J = 6.5Hz), 4.83 (1H, s), 4.72-4.67 (2H, m), 4.19 (1H, s), 4.13-4.10 (1H, m), 3.97 (1H, t, J = 4.7Hz), 3.95-3.82 (2H, m), 3.74-3.64 (3H, m). |

### EXAMPLES aa117 - aa135

### (EXAMPLE aa117)

### (R)-2-Hydroxy-N-(1-iminoisoindolin-5-yl)-2-((R)-3-oxo-4-(3-(trifluoromethoxy)phenyl )morpholin-2-yl)acetamide EXAMPLE aa117

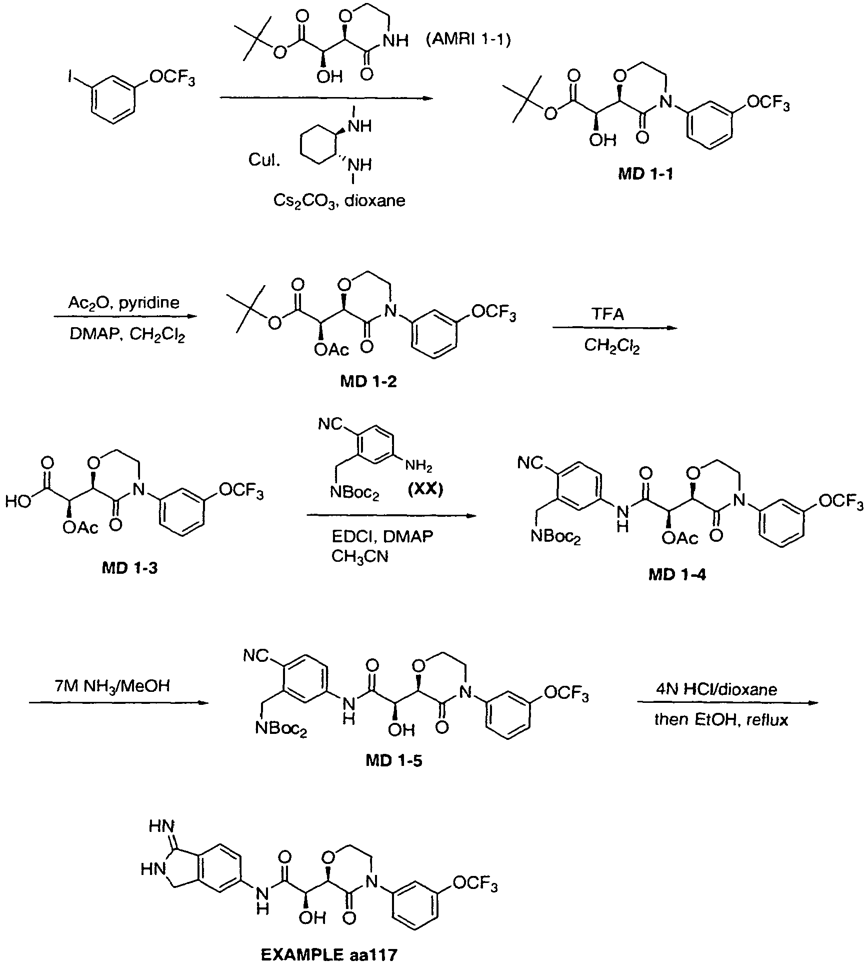

### [Step MD 1-1]

### (R)-tert-Butyl 2-hydroxy-2-((R)-3-oxo-4-(3-(trifluoromethoxy)phenyl)morpholin-2-yl)acetate MD 1-1

To a round bottom flask charged with a stir bar was added morpholinone (0.15 g) AMRI 1-1 and 3-trifluoromethoxyiodobenzene (0.12 mL) in dioxane (4 mL) at rt was added Cs₂-CO₃ (0.42 g), and CuI (37 mg) under N₂. *trans*-*N.N'*-Dimethylcyclohexane-1,2-diamine (31 L) was added dropwise and the mixture was affixed with a condenser. The mixture was degassed under vacuum (- 20 mm), filled with N₂, and heated to 90 °C. The mixture stirred for 3 h at 90 °C, cooled to rt, and was diluted with conc NH₄OH and water, EtOAc. The mixture was extracted with EtOAc three times and the organic layers were combined. The organic layer was washed with brine, dried (Na₂SO₄), filtered, and concentrated under reduced pressure to afford a yellow oil. The crude product was purified by flash chromatography using a 95% CH₂Cl₂/5% MeOH mixture to afford MD 1-1 (0.21 g) as a white solid.

### [Step MD 1-2]

### (R)-tert-butyl 2-acetoxy-2-((R)-3-oxo-4-(3-(trifluoromethoxy)phenyl)morpholin-2-yl)acetate MD 1-2

To a solution of MD 1-1 (0.21 g) in CH₂Cl₂ (2.5 ml) at 0°C was added pyridine (63 µL), Ac₂O (74 µl), and DMAP (5 mg). The mixture was stirred for 1 hour at 0°C, warmed to rt, and stirred for an additional 12 h. The mixture was diluted with EtOAc and the organic layer was washed sequentially with sat. aq. CuSO₄ solution, water, and brine. The organic layer was dried (Na₂SO₄), filtered, and concentrated under reduced pressure to afford MD1-2 (0.22 g) as a light yellow semisolid. This material was used without further purification.

### [Step MD 1-3]

### (R)-2-acetoxy-2-((R)-3-oxo-4-(3-(trifluoromethoxy)phenyl)morpholin-2-yl)acetic acid MD 1-3

To a solution of MD 1-2 (0.22 g) in CH₂Cl₂ (2 mL) at 0°C was added TFA (0.6 mL) dropwise. The mixture was stirred for 1h at 0°C and at rt for 30 min whereupon an additional portion of TFA (0.4 mL) was added. After an additional 1 h at rt, the mixture was diluted with CH₂Cl₂ and concentrated to dryness under reduced pressure. The crude mixture was redissolved in a 10:1 mixture of toluene/CH₂Cl₂ and concentrated and this protocol was repeated 5 times with to afford MD1-3 (0.18 g) as a light yellow solid. This material was used without further purification.

### [Step MD 1-4]

### (R)-2-(3-((Bis(tert-butoxycarbonyl)amino)methyl)-4-cyanophenylamino)-2-oxo-1-((R)-3-oxo-4-(3-(trifluoromethoxy)phenyl)morpholin-2-yl)ethyl acetate MD 1-4

To a solution of MD 1-3 (0.41 g) in CH₂Cl₂ (5 mL) at 0 °C was added 3-bis(tert-butoxycarbonylaminomethyl)-4-cyanophenylamine XX (0.42 g) followed by EDCI (0.27 g) and DMAP (13 mg). The reaction mixture was warmed to rt and stirred for 12 h. The mixture was diluted with EtOAc (15 mL) and the organic layer was washed with 1N HCl (2 x 3 mL) and brine (1 x 2 mL). The organic layer was dried (Na₂SO₄), filtered, and concentrated under reduced pressure. The crude product was purified by flash chromatography (ISCO, 20 g) using a gradient of 100% CH₂Cl₂ to 90:10 CH₂Cl₂ /MeOH to afford MD 1-4 (0.35 g) as a white solid.

### [Step MD 1-5]

### (R)-2-(3-((Bis(tert-butoxycarbonyl)amino)methyl)-4-cyanophenylamino)-2-oxo-1-((R)-3-oxo-4-(3-(trifluoromethoxy)phenyl)morpholin-2-yl)ethyl MD 1-5

To a solution of the MD 1-4 (0.35 g) in MeOH (0.5 mL) at rt was added 7M NH₃/MeOH (2.5 mL) dropwise. The mixture was stirred for 45 min at rt and the mixture was concentrated under reduced pressure and placed under high vacuum. The crude product was purified by flash chromatography (ISCO, 12 g) using a gradient of 100% CH₂Cl₂ to 90:10 CH₂Cl₂ /MeOH to afford MD 1-5 (0.26 g) as a white solid.

### [Step MD 1-6]

### (R)-2-Hydroxy-N-(1-iminoisoindolin-5-yl)-2-((R)-3-oxo-4-(3-(trifluoromethoxy)phenyl )morpholin-2-yl)acetamide EXAMPLE aa117

To a flask containing MD 1-5 (0.26 g) and a stir bar was added 4N HCl in dioxane (4 mL) at rt. The mixture was stirred for 1.5 h and was concentrated to - 2 mL. Toluene (8 mL) was added and the mixture was concentrated under reduced pressure. The crude material was treated with toluene (2 x 8 mL) and concentrated under reduced pressure. The crude product was taken up in EtOH (8 mL) and was heated at reflux for 12 h. The mixture was cooled to rt, concentrated under reduced pressure, and placed under high vacuum. The crude residue was treated with MeOH followed by dilution with Et₂O and the resultant solid was collected by filtration and dried under vacuum to afford Example aa117 (106 mg) hydrochloride as a white solid.

### (EXAMPLE aa118)

### (R)-2-Hydroxy-N-(1-iminoisoindolin-5-yl)-2-((R)-4-(2-methyl-1-oxoisoindolin-5-yl)-3-oxomorpholin-2-yl)acetamide EXAMPLE aa118

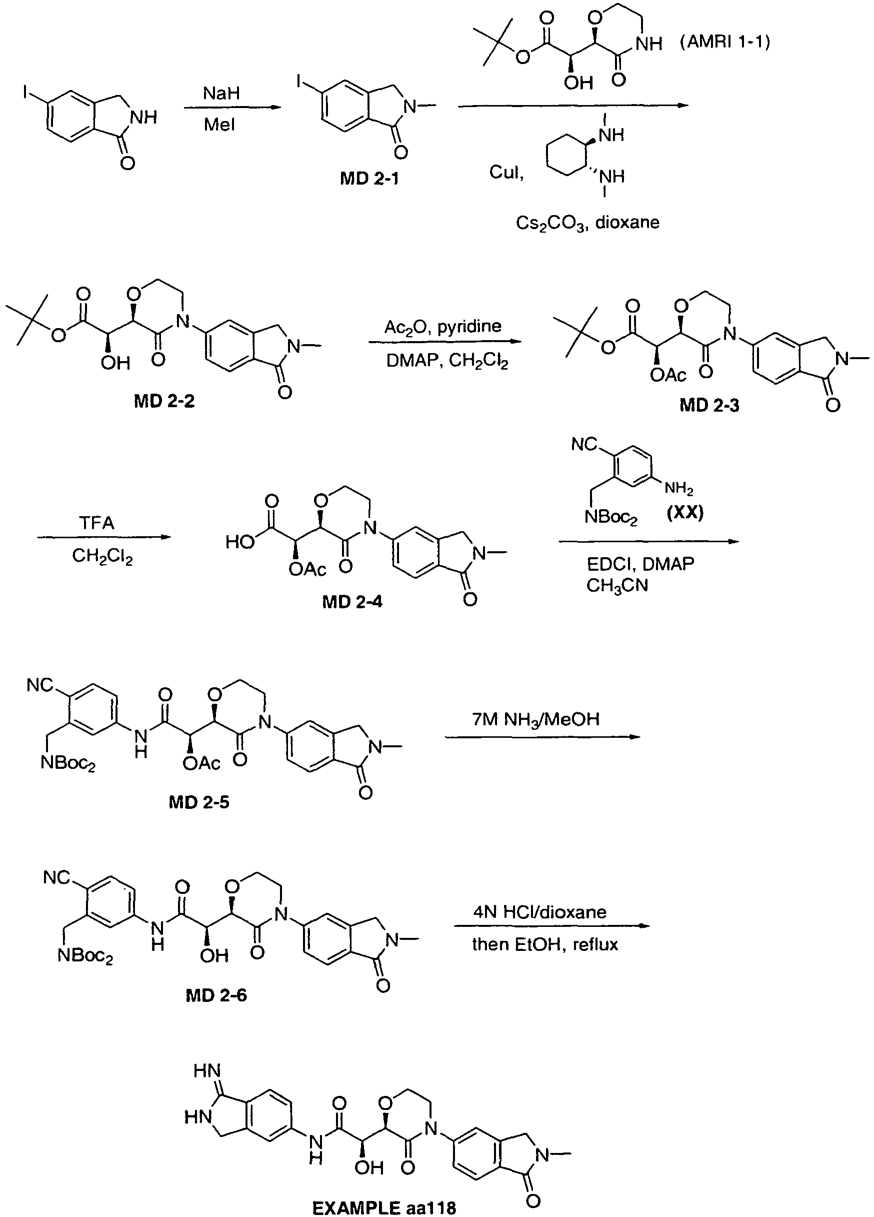

### [Step MD 2-1]

### 5-Iodo-2-methylisoindolin-1-one MD 2-1

To a mixture of 2,3-dihydro-5-iodo-1H-isoindol-1-one (1.0 g) in DMF (20 mL) at 0 °C was added NaH (97 mg) in a single portion. The resulting mixture was stirred for 30 min at 0 °C whereupon MeI (0.25 mL) was added dropwise. The mixture was allowed to warm to rt and was stirred for 72 h. The mixture was quenched by addition of sat. aq. NH₄Cl (- 3 mL) and was diluted with EtOAc (10 mL). The layers were separated and the aqueous layer was extracted with EtOAc. The organic layers were combined and washed sequentially with water and brine. The organic layer was dried (Na₂SO₄), filtered, and concentrated under reduced pressure. The crude material was purified The crude product was purified by flash chromatography (ISCO, 120 g) using a gradient of 100% hexanes to 80:20 hexanes/EtOAc to afford MD 2-1 (0.78 g) as a light yellow solid.

### [Step MD 2-2]

### (R)-tert-Butyl 2-hydroxy-2-((R)-4-(2-methyl-1-oxoisoindolin-5-yl)-3-oxomorpholin-2-yl)acetate MD 2-2

According to the Step MD 1-1 in the synthetic method for EXAMPLE aa117, compound MD 2-1 (0.20 g) was used instead of MD 1-1 to obtain MD 2-2 (0.29 g) as an off-white solid.

### [Step MD 2-3]

### (R)-tert-butyl 2-acetoxy-2-((R)-4-(2-methyl-1-oxoisoindolin-5-yl)-3-oxomorpholin-2-yl)acetate MD 2-3

According to the Step MD 1-2 in the synthetic method for EXAMPLE aa117, compound MD 2-2 (0.29 g) was used instead of MD 1-1 to obtain MD 2-3 (0.31 g) as an off-white solid which was used without further purification.

### [Step MD 2-4]

### (R)-2-Acetoxy-2-((R)-4-(2-methyl-1-oxoisoindolin-5-yl)-3-oxomorpholin-2-yl)acetic acid MD 2-4

According to the Step MD 1-3 in the synthetic method for EXAMPLE aa117, compound MD 2-3 (0.31 g) was used instead of MD 1-2 to obtain MD 2-4 (0.27 g) as a white solid which was used without further purification.

### [Step MD 2-5]

### (R)-2-(3-((Bis(tert-butoxycarbonyl)amino)methyl)-4-cyanophenylamino)-1-((R)-4-(2-m ethyl-1-oxoisoindolin-5-yl)-3-oxomorpholin-2-yl)-2-oxoethyl acetate MD 2-5

According to the Step MD 1-4 in the synthetic method for EXAMPLE aa117, compound MD 2-4 (0.11 g) was used instead of MD 1-3 to obtain MD 2-5 (0.14 g) as an off-white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 2-6]

### (R)-2-(3-((Bis(tert-butoxycarbonyl)amino)methyl)-4-cyanophenylamino)-1-((R)-4-(2-methyl-1-oxoisoindolin-5-yl)-3-oxomorpholin-2-yl)-2-oxoethyl MD 2-6

According to the Step MD 1-5 in the synthetic method for EXAMPLE aa117, compound MD 2-5 (0.14 g) was used instead of MD 1-4 to obtain MD 2-6 (0.13 mg) as a off-white solid which was used without further purification.

### [Step MD 2-7]

### (R)-2-Hydroxy-N-(1-iminoisoindolin-5-yl)-2-((R)-4-(2-methyl-1-oxoisoindolin-5-yl)-3-oxomorpholin-2-yl)acetamide EXAMPLE aa118

According to the Step MD 1-6 in the synthetic method for EXAMPLE aa117, compound MD 2-5 (0.13 g) was used instead of MD 1-5 to obtain EXAMPLE aa118 (25 mg) as a pale white solid as the hydrochloride salt after reverse phase HPLC purification using a C18 column and a gradient of 89.95:9.95:0.1 H₂O:MeCN:HCO₂H to 9.95:89.95:0.1 H₂O:MeCN:HCO₂H and treatment with HCl.

### (EXAMPLE aa119)

### (R)-2-Hydroxy-N-(1-iminoisoindolin-5-yl)-2-((R)-4-(2-methyl-3-oxoisoindolin-5-yl)-3-oxomorpholin-2-yl)acetamide hydrochloride EXAMPLE aa119

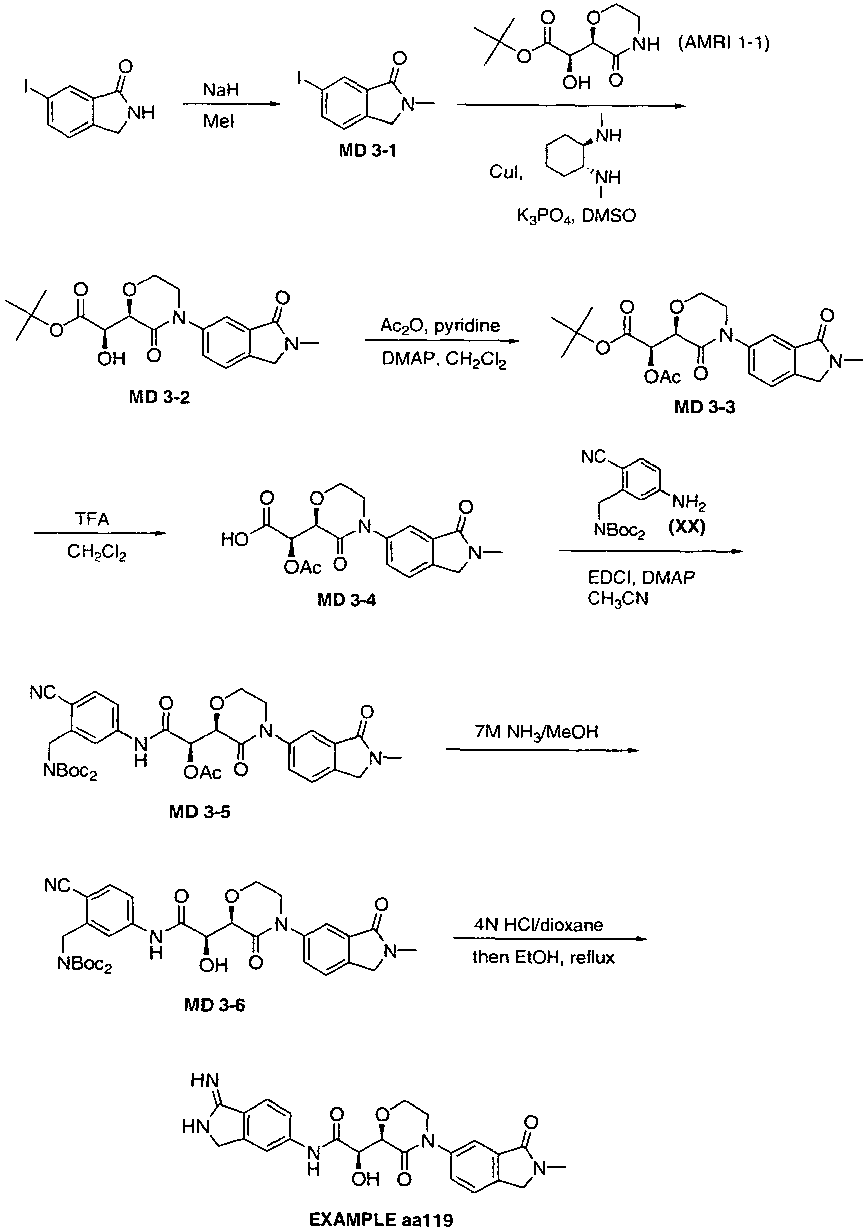

### [Step MD 3-1]

### 6-Iodo-2-methylisoindolin-1-one MD 3-1

To a mixture of 2,3-dihydro-6-iodo-1H-isoindol-1-one (1.0 g) in DMF (20 mL) at 0 °C was added NaH (97 mg) in a single portion. The resulting mixture was stirred for 30 min at 0 °C whereupon MeI (0.25 mL) was added dropwise. The mixture was allowed to warm to rt and was stirred for 72 h. The mixture was quenched by addition of sat. aq. NH₄Cl (- 3 mL) and was diluted with EtOAc (10 mL). The layers were separated and the aqueous layer was extracted with EtOAc. The organic layers were combined and washed sequentially with water and brine. The organic layer was dried (Na₂SO₄), filtered, and concentrated under reduced pressure. The crude material was purified The crude product was purified by flash chromatography (ISCO, 120 g) using a gradient of 100% hexanes to 80:20 hexanes/EtOAc to afford MD 3-1 (0.84 g) as a yellow solid.

### [Step MD 3-2]

### (R)-tert-Butyl 2-hydroxy-2-((R)-4-(2-methyl-3-oxoisoindolin-5-yl)-3-oxomorpholin-2-yl)acetate MD 3-2

To a round bottom flask charged with a stir bar was added AMRI 1-1 (0.28 g) and MD 11-1 (0.40 g) in DMSO (8 mL) at rt was added K₃PO₄ (0.51 g), and CuI (23 mg) under N₂. *trans-N,N'*-Dimethylcyclohexane-1,2-diamine (37 µL) was added dropwise and the mixture was affixed with a condenser. The mixture was degassed under vacuum (~ 20 mm), filled with N₂, and heated to 80 °C. The mixture stirred for 2.5 h at 80 °C, cooled to rt, and was diluted with EtOAc. The mixture was then sequentially washed with conc NH₄OH, water, and brine. The organic layer was dried (Na₂SO₄), filtered, and concentrated under reduced pressure to afford a yellow oil. The crude product was purified by flash chromatography using a gradient of 100% CH₂Cl₂ to 60% CH₂Cl₂/40% MeOH to afford MD3-2 (0.23 g) as a yellow solid.

### [Step MD 3-3]

### (R)-tert-Butyl 2-acetoxy-2-((R)-4-(2-methyl-3-oxoisoindolin-5-yl)-3-oxomorpholin-2-yl)acetate MD 3-3

According to the Step MD 1-2 in the synthetic method for EXAMPLE aa117, compound MD 3-2 (80 mg) was used instead of MD 1-1 to obtain MD 3-3 (85 mg) as an off-white solid which was used without further purification.

### [Step MD 3-4]

### (R)-2-Acetoxy-2-((R)-4-(2-methyl-3-oxoisoindolin-5-yl)-3-oxomorpholin-2-yl)acetic acid MD 3-4

According to the Step MD 1-3 in the synthetic method for EXAMPLE aa117, compound MD 3-3 (85 mg) was used instead of MD 1-2 to obtain MD 3-4 (65 mg) as a light yellow semisolid solid which was used without further purification.

### [Step MD 3-5]

### (R)-2-(3-((Bis(tert-butoxycarbonyl)amino)methyl)-4-cyanophenylamino)-1-((R)-4-(2-m ethyl-3-oxoisoindolin-5-yl)-3-oxomorpholin-2-yl)-2-oxoethyl acetate MD 3-5

According to the Step MD 1-4 in the synthetic method for EXAMPLE aa117, compound MD 3-4 (0.25 g) was used instead of MD 1-3 to in the presence of aniline XX (0.29 g) obtain MD 3-5 (0.23 g) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 3-6]

### (R)-2-(3-((Bis(tert-butoxycarbonyl)amino)methyl)-4-cyanophenylamino)-1-((R)-4-(2-m ethyl-3-oxoisoindolin-5-yl)-3-oxomorpholin-2-yl)-2-oxoethyl MD 3-6

According to the Step MD 1-5 in the synthetic method for EXAMPLE aa 117, compound MD 3-5 (0.23 g) was used instead of MD 1-4 to obtain MD 3-6 (0.16 g) as a white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 3-7]

### (R)-N-(4-Carbamimidoyl-2-ethylphenyl)-2-hydroxy-2-((R)-4-(2-methyl-3-oxoisoindoli n-5-yl)-3-oxomorpholin-2-yl)acetamide hydrochloride EXAMPLE aa119

According to the Step MD 1-6 in the synthetic method for EXAMPLE aa117, compound MD 3-6 (0.16 g) was used instead of MD 1-5 to obtain EXAMPLE aa119 (0.11 g) as a white solid as the hydrochloride salt after HCl treatment.

### (EXAMPLE aa120)

### Ethyl 2-(3-((R)-2-((R)-1-hydroxy-2-(1-iminoisoindolin-5-ylamino)-2-oxoethyl)-3-oxomorpho lino)phenoxy)acetate EXAMPLE aa120

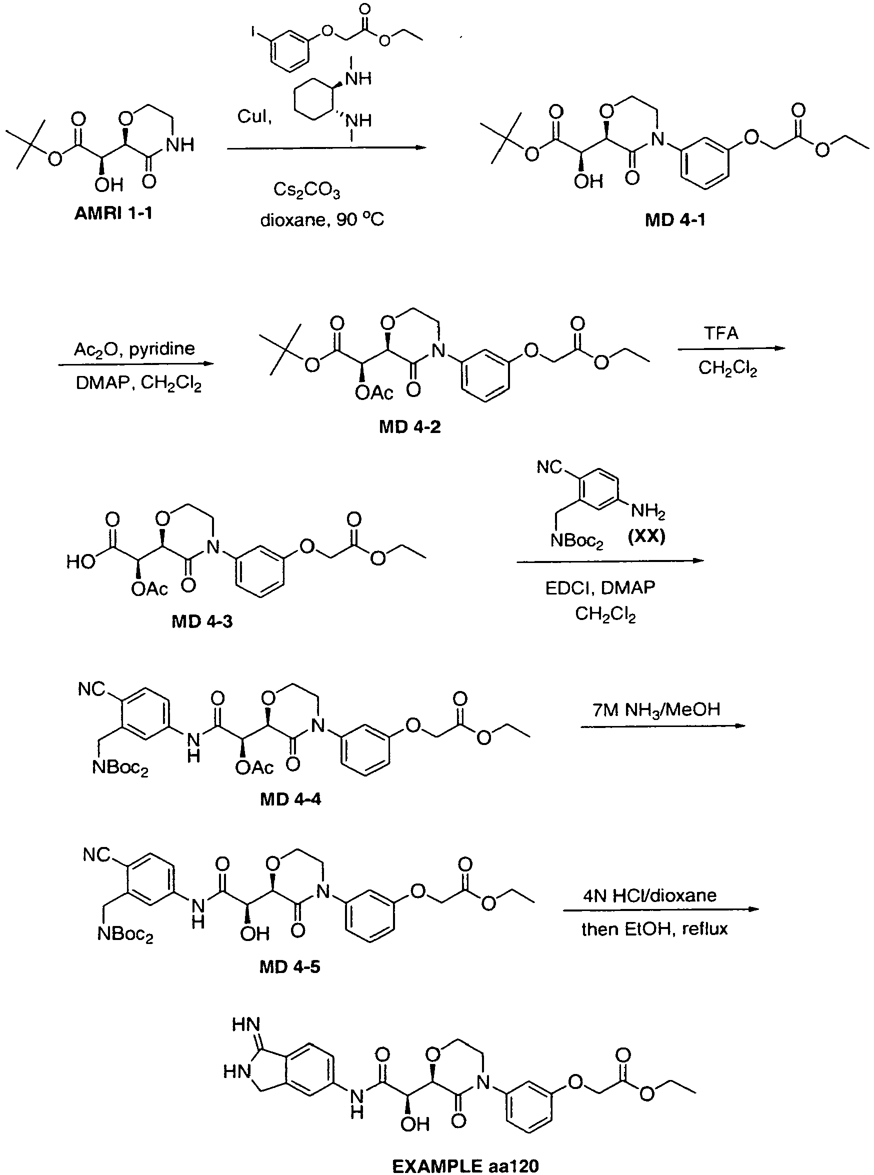

### [Step MD 4-1]

### (R)-tert-Butyl 2-((R)-4-(3-(2-ethoxy-2-oxoethoxy)phenyl)-3-oxomorpholin-2-yl)-2-hydroxyacetate MD 4-1

According to the Step MD 1-1 in the synthetic method for EXAMPLE aa117, AMRI 1-1 (0.35 g) was treated with ethyl 2-(3-iodophenoxy)acetate (0.56 g) from Eur. J. Org. Chem. 2008. 337 to obtain MD 4-1 (0.54 g) as an yellow solid after flash chromatography with 40:1 CH₂Cl₂/MeOH as eluent.

### [Step MD 4-2]

### (R)-tert-Butyl 2-acetoxy-2-((R)-4-(3-(2-ethoxy-2-oxoethoxy)phenyl)-3-oxomorpholin-2-yl)acetate MD 4-2

According to the Step MD 1-2 in the synthetic method for EXAMPLE aa117, compound MD 4-1 (0.54 g) was used instead of MD 1-1 to obtain MD 4-2 (0.56 g) as a yellow oil which was used without further purification.

### [Step MD 4-3]

### (R)-2-Acetoxy-2-((R)-4-(3-(2-ethoxy-2-oxoethoxy)phenyl)-3-oxomorpholin-2-yl)acetic acid MD 4-3

According to the Step MD 1-3 in the synthetic method for EXAMPLE aa 117, compound MD 9-2 (0.55 g) was used instead of MD 1-2 to obtain MD 4-3 (0.45 g) as a yellow solid which was used without further purification.

### [Step MD 4-4]

### Ethyl 2-(3-((R)-2-((R)-1-acetoxy-2-(3-((bis(tert-butoxycarbonyl)amino)methyl)-4-cyanophen ylamino)-2-oxoethyl)-3-oxomorpholino)phenoxy)acetate MD 4-4

According to the Step MD 1-4 in the synthetic method for EXAMPLE aa117, compound MD 4-3 (0.38 g) was used instead of MD 1-3 to obtain MD 3-4 (0.26 g) after flash chromatography using a gradient of 100% hexanes to 100% EtOAc.

### [Step MD 4-5]

### Ethyl 2-(3-((R)-2-((R)-1-hydroxyl-2-(3-((bis(tert-butoxycarbonyl)amino)methyl)-4-cyanophenylamino)-2-oxoethyl)-3-oxo morpholino)phenoxy)acetate MD 4-5

According to the Step MD 1-5 in the synthetic method for EXAMPLE aa 117 except using 2M NH₃ in EtOH, compound MD 4-4 (0.26 g) was used instead of MD 1-4 to obtain MD 5-5 (0.21 g) as an off-white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 4-6]

### Ethyl 2-(3-((R)-2-((R)-1-hydroxy-2-(1-iminoisoindolin-5-ylamino)-2-oxoethyl)-3-oxomorpho lino)phenoxy)acetate EXAMPLE aa120

According to the Step MD 1-6 in the synthetic method for EXAMPLE aa117 except substituting EtOH for MeOH as solvent, compound MD 4-5 (0.20 g) was used instead of MD 1-5 to obtain EXAMPLE aa120 (81 mg) as the hydrochloride salt after reverse phase HPLC purification using a C18 column and a gradient of 89.95:9.95:0.1 H₂O:MeCN:HCO₂H to 9.95:89.95:0.1 H₂O:MeCN:HCO₂H and subsequent treatment with HCl.

### (EXAMPLE aa121)

### (R)-2-Hydroxy-N-(1-iminoisoindolin-5-yl)-2-((R)-4-(4-methoxybenzyl)-3-oxomorpholi n-2-yl)acetamide EXAMPLE aa121

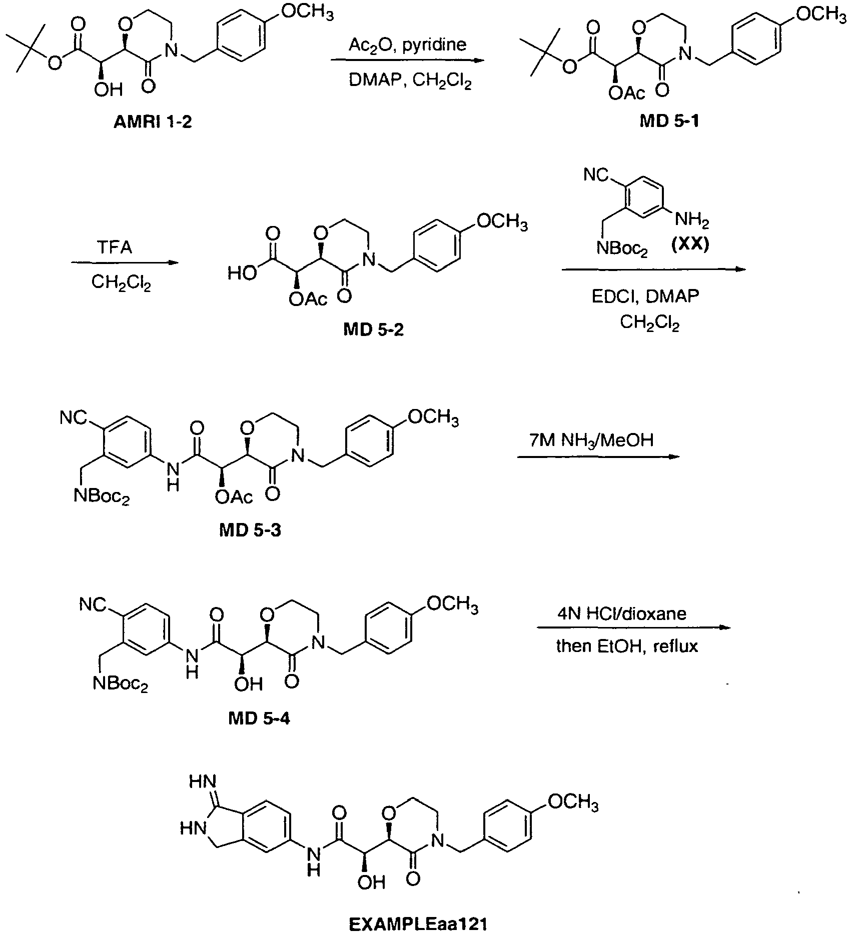

### [Step MD 5-1]

### (R)-tert-Butyl 2-acetoxy-2-((R)-4-(4-methoxybenzyl)-3-oxomorpholin-2-yl)acetate MD 5-1

According to the Step MD 1-2 in the synthetic method for EXAMPLE aa 117, compound AMRI 2-1 (0.35 g) was used instead of MD 1-1 to obtain MD 5-1 (0.37 g) as a yellow oil which was used without further purification.

### [Step MD 5-2]

### (R)-2-Acetoxy-2-((R)-4-(4-methoxybenzyl)-3-oxomorpholin-2-yl)acetic acid MD 5-2

According to the Step MD 1-3 in the synthetic method for EXAMPLE aa117, compound MD 5-1 (0.37 g) was used instead of MD 1-2 to obtain MD 5-2 (0.32 g) as a yellow solid which was used without further purification.

### [Step MD 5-3]

### (R)-2-(3-((bis(tert-Butoxycarbonyl)amino)methyl)-4-cyanophenylamino)-1-((R)-4-(4-methoxybenzyl)-3-oxomorpholin-2-yl)-2-oxoethyl acetate MD 5-3

According to the Step MD 1-4 in the synthetic method for EXAMPLE aa117, compound MD 5-2 (0.25 g) was used instead of MD 1-3 to obtain MD 5-3 (0.24 g) of a white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 5-4]

### (R)-2-(3-((bis(tert-Butoxycarbonyl)amino)methyl)-4-cyanophenylamino)-1-((R)-4-(4-methoxybenzyl)-3-oxomorpholin-2-yl)-2-hydroxyacetamide MD 5-4

According to the Step MD 1-5 in the synthetic method for EXAMPLE aa117, compound MD 5-3 (0.24 g) was used instead of MD 1-4 to obtain MD 5-4 (0.23 g) as an off-white solid which was used without further purification.

### [Step MD 5-5]

### (R)-2-Hydroxy-N-(1-iminoisoindolin-5-yl)-2-((R)-4-(4-methoxybenzyl)-3-oxomorpholi n-2-yl)acetamide EXAMPLE aa121

According to the Step MD 1-6 in the synthetic method for EXAMPLE aa117, compound MD 5-4 (0.23 g) was used instead of MD 1-5 to obtain EXAMPLE aa121 (0.15 g) as a pale yellow solid as the hydrochloride salt.

### (EXAMPLE aa122)

This Example number is intentionally left blank.

### (EXAMPLE aa123)

### (R)-2-((R)-4-(3-(Benzyloxy)phenyl)-3-oxomorpholin-2-yl)-2-hydroxy-N-(1-iminoisoin dolin-5-yl)acetamide EXAMPLE aa123

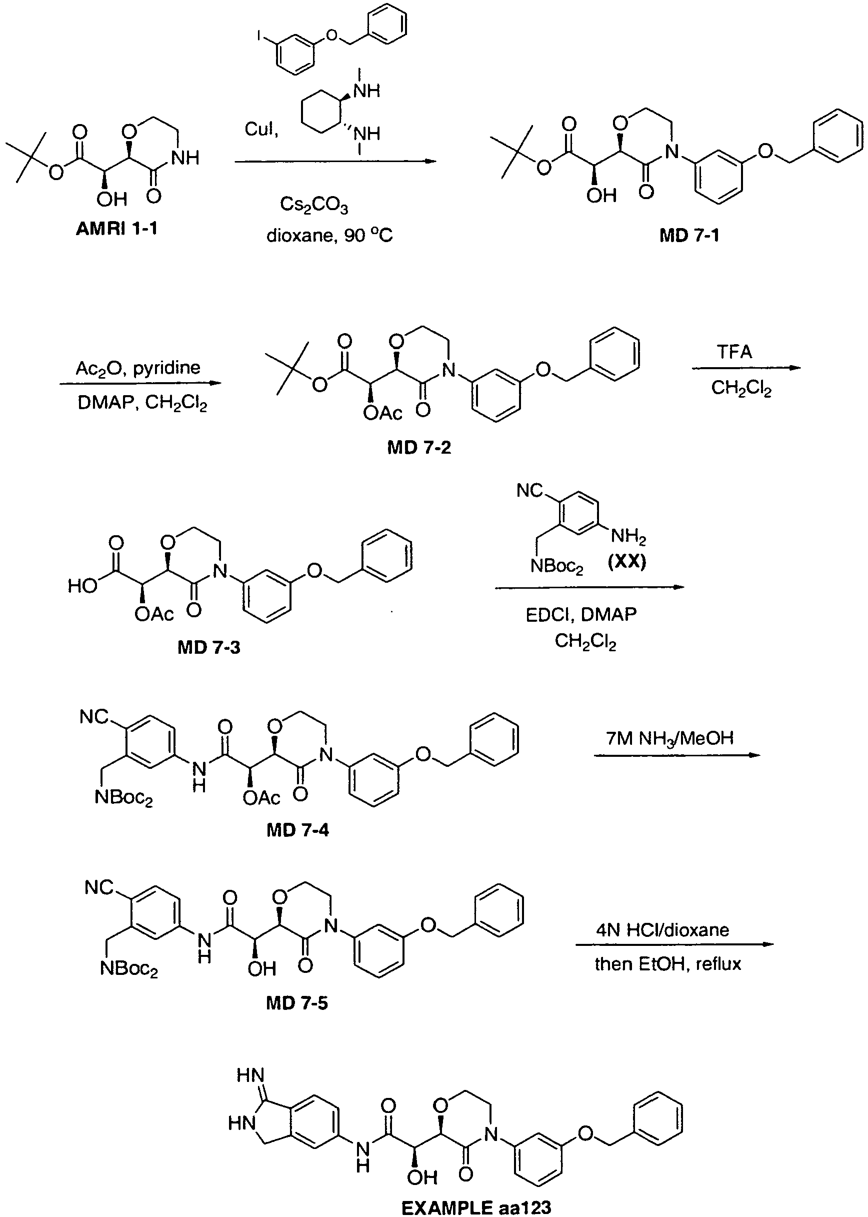

### [Step MD 7-1]

### (R)-teri-Butyl 2-((R)-4-(3-(benzyloxy)phenyl)-3-oxomorpholin-2-yl)-2-hydroxyacetate MD 7-1

According to the Step MD 1-1 in the synthetic method for EXAMPLE aa117, AMRI 1-1 (0.50 g) was treated with 1-benzyloxy-3-iodobenzene (0.81 g) to obtain MD 7-1 (0.71 g) as a clear oil after flash chromatography with 50:1 CH₂Cl₂/MeOH as eluent.

### [Step MD 7-2]

### (R)-tert-Butyl 2-acetoxy-2-((R)-4-(3-(benzyloxy)phenyl)-3-oxomorpholin-2-yl)acetate MD 7-2

According to the Step MD 1-2 in the synthetic method for EXAMPLE aa117, compound MD 7-1 (0.71 g) was used instead of MD 1-1 to obtain MD 7-2 (0.78 g) as a yellow oil which was used without further purification.

### [Step MD 7-3]

### (R)-2-Acetoxy-2-((R)-4-(3-(benzyloxy)phenyl)-3-oxomorpholin-2-yl)acetic acid MD 7-3

According to the Step MD 1-3 in the synthetic method for EXAMPLE aa117, compound MD 7-2 (0.78 g) was used instead of MD 1-2 to obtain MD 7-3 (0.68 g) as a yellow solid which was used without further purification.

### [Step MD 7-4]

### (R)-1-((R)-4-(3-(Benzyloxy)phenyl)-3-oxomorpholin-2-yl)-2-(3-((bis(tert-butoxycarbo nyl)amino)methyl)-4-cyanophenylamino)-2-oxoethyl acetate MD 7-4

According to the Step MD 1-4 in the synthetic method for EXAMPLE aa117, compound MD 7-3 (0.68 g) was used instead of MD 1-3 to obtain MD 7-4 (0.20 g) of an off-white solid after flash chromatography with a gradient of 100% hexanes to 50:50 hexanes/EtOAc.

### [Step MD 7-5]

### R)-1-((R)-4-(3-(Benzyloxy)phenyl)-3-oxomorpholin-2-yl)-2-(3-((bis(tert-butoxycarbon yl)amino)methyl)-4-cyanophenylamino)-2-hydroxyacetamide MD 7-5

According to the Step MD 1-5 in the synthetic method for EXAMPLE aa117, compound MD 7-4 (0.20 g) was used instead of MD 1-4 to obtain MD 7-5 (0.16 g) as an off-white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 7-6]

### (R)-2-((R)-4-(3-(Benzyloxy)phenyl)-3-oxomorpholin-2-yl)-2-hydroxy-N-(1-iminoisoin dolin-5-yl)acetamide EXAMPLE aa123

According to the Step MD 1-6 in the synthetic method for EXAMPLE aa117, compound MD 7-5 (0.16 g) was used instead of MD 1-5 to obtain EXAMPLE aa123 (86 mg) as the hydrochloride salt.

### (EXAMPLE aa 124)

### (R)-2-((R)-4-(2-(Cyclopropylmethyl)-3-oxoisoindolin-5-yl)-3-oxomorpholin-2-yl)-2-hy droxy-N-(1-iminoisoindolin-5-yl)acetamide EXAMPLE aa124

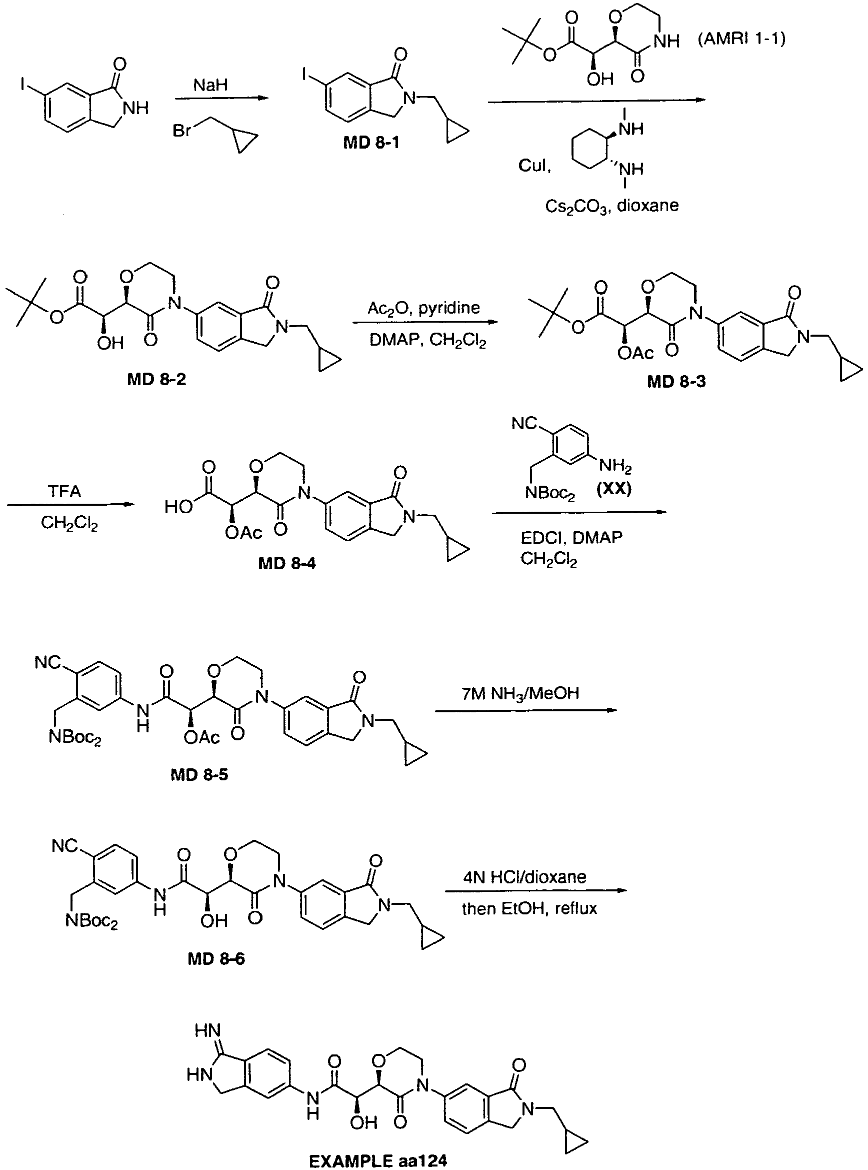

### [Step MD 8-1]

### 2-(Cyclopropylmethyl)-6-iodoisoindolin-1-one MD 8-1

According to the Step MD 3-1 in the synthetic method for EXAMPLE 3, 2.3-dihydro-6-iodo-1H-isoindol-1-one (0.50 g) was treated with cyclopropyl bromide (0.20 mL) to afford MD 8-1 (0.22 g) of a yellow solid after purification by flash chromatography (ISCO, 120 g) using a gradient of 100% hexanes to 40:60 hexanes/EtOAc.

### [Step MD 8-2]

### (R)-tert-Butyl 2-((R)-4-(2-(cyclopropylmethyl)-3-oxoisoindolin-5-yl)-3-oxomorpholin-2-yl)-2-hydrox yacetate MD 8-2

According to the Step MD 1-1 in the synthetic method for EXAMPLE aa117, AMRI 1-1 (0.14 g) was treated with MD 8-1 (0.22 g) to obtain MD 8-2 (0.22 g) as a white solid after flash chromatography with 50:1 CH₂Cl₂/MeOH as eluent.

### [Step MD 8-3]

### (R)-tert-Butyl 2-acetoxy-2-((R)-4-(2-(cyclopropylmethyl)-3-oxoisoindolin-5-yl)-3-oxomorpholin-2-yl )acetate MD 8-3

According to the Step MD 1-2 in the synthetic method for EXAMPLE aa117, compound MD 8-2 (0.22 g) was used instead of MD 1-1 to obtain MD 8-3 (0.24 g) as an off-white solid which was used without further purification.

### [Step MD 8-4]

### (R)-2-Acetoxy-2-((R)-4-(2-(cyclopropylmethyl)-3-oxoisoindolin-5-yl)-3-oxomorpholin -2-yl)acetic acid MD 8-4

According to the Step MD 1-3 in the synthetic method for EXAMPLE aa117, compound MD 8-3 (0.24 g) was used instead of MD 1-2 to obtain MD 8-4 (0.22 g) as a light yellow semisolid solid which was used without further purification.

### [Step MD 8-5]

### (R)-2-(3-((bis(tert-Butoxycarbonyl)amino)methyl)-4-cyanophenylamino)-1-((R)-4-(2-( cyclopropylmethyl)-3-oxoisoindolin-5-yl)-3-oxomorpholin-2-yl)-2-oxoethyl acetate MD 8-5

According to the Step MD 1-4 in the synthetic method for EXAMPLE aa117, compound MD 8-4 (0.22 g) was used instead of MD 1-3 to in the presence of aniline XX (0.22 g) obtain MD 8-5 (0.25 g) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 8-6]

### (R)-2-(3-((bis(tert-Butoxycarbonyl)amino)methyl)-4-cyanophenylamino)-1-((R)-4-(2-( cyclopropylmethyl)-3-oxoisoindolin-5-yl)-3-oxomorpholin-2-yl)-2-hydroxyacetamide MD 8-6

According to the Step MD 1-5 in the synthetic method for EXAMPLE aa117, compound MD 8-5 (0.25 g) was used instead of MD 1-4 to obtain MD 8-6 (0.18 g) as a white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 8-7]

### (R)-2-((R)-4-(2-(Cyclopropylmethyl)-3-oxoisoindolin-5-yl)-3-oxomorpholin-2-yl)-2-hy droxy-N-(1-iminoisoindolin-5-yl)acetamide EXAMPLE aa 124

According to the Step MD 1-6 in the synthetic method for EXAMPLE aa117, compound MD 8-6 (0.18 g) was used instead of MD 1-5 to obtain EXAMPLE aa124 (78 mg) as a white solid as the hydrochloride salt after HCl treatment.

### EXAMPLE aa 125

### (R)-N-(4-(Aminomethyl)-3-fluorophenyl)-2-hydroxy-2-((R)-3-oxo-4-(3-(3-oxomorphol ino)phenyl)morpholin-2-yl)acetamide EXAMPLE aa125

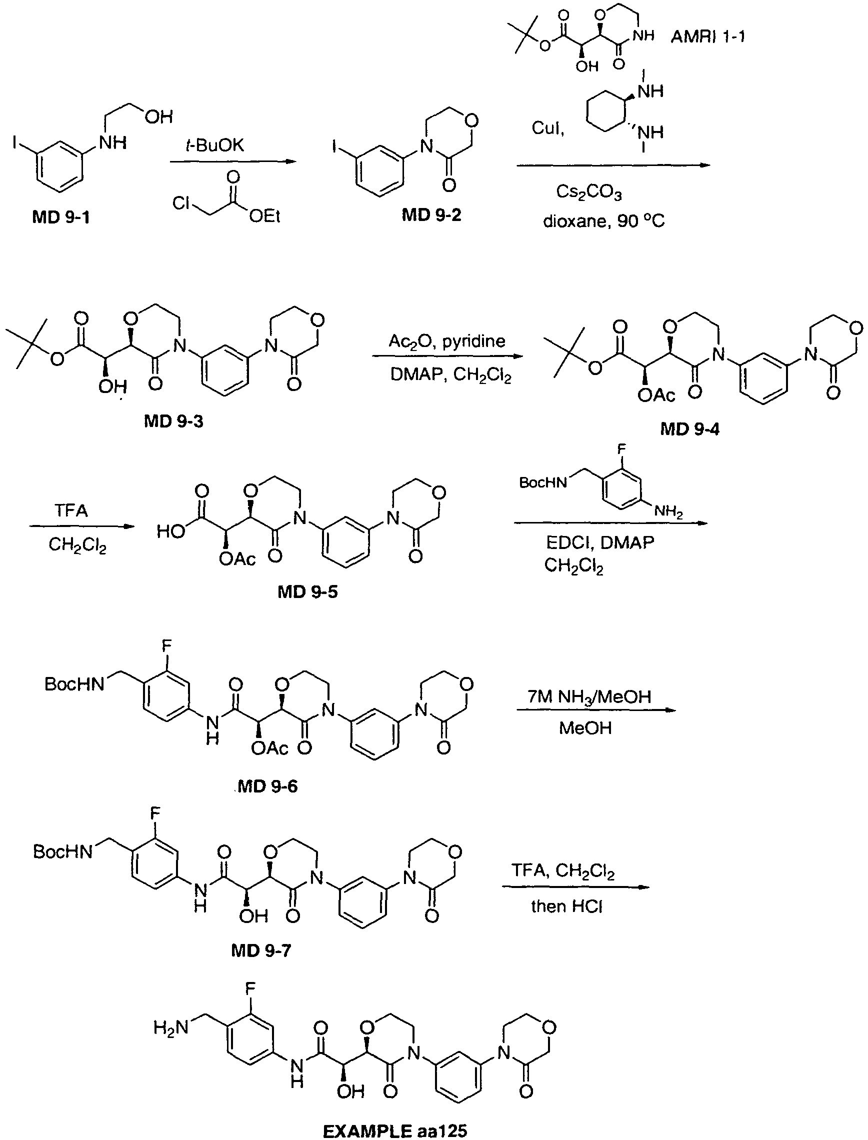

### [Step MD 9-2]

### 4-(3-Iodophenyl)morpholin-3-one MD 9-2

To a solution of *t*-BuOK (1.3 g) in THF (15 mL) at rt was added 2-(3-iodophenylamino)ethanol MD 9-1 (3.0 g) prepared from US 2004/0167188 followed by ethyl chloroacetate (1.1 mL). The resulting mixture was stirred for 12 h at rt whereupon an additional portion of *t*-BuOK (0.6 g) and ethyl chloroacetate (0.5 mL) was added. The mixture was heated to 55 °C, stirred for 12 h, and was cooled to rt. The mixture was treated with sat. aq NaHCO₃ and water and was extracted with EtOAc. The organic layers were combined, dried (Na₂SO₄), filtered, and concentrated under reduced pressure. The crude material was purified by flash chromatography using a gradient of 100% hexanes to 20% hexanes/80% EtOAc to afford MD 9-2 (1.5 g) of the title compound as a light yellow solid.

### [Step MD 9-3]

### (R)-tert-butyl 2-hydroxy-2-((R)-3-oxo-4-(3-(3-oxomorpholino)phenyl)morpholin-2-yl)acetate MD 9-3

According to the Step MD 1-1 in the synthetic method for EXAMPLE aa 117, compound MD 9-2 (0.72 g) was used in the presence of AMRI 1-1 (0.50 g) to obtain MD 9-3 (0.65 g) as yellow crystalline solid after flash chromatography using a 20:1 mixture of CH₂Cl₂/MeOH.

### [Step MD 9-4]

### (R)-tert-butyl 2-acetoxy-2-((R)-3-oxo-4-(3-(3-oxomorpholino)phenyl)morpholin-2-yl)acetate MD 9-4

According to the Step MD 1-2 in the synthetic method for EXAMPLE aa117, compound MD 9-3 (0.65 g) was used instead of MD 1-1 to obtain MD 9-4 (0.72 g) as an off-white solid which was used without further purification.

### [Step MD 9-5]

### (R)-2-acetoxy-2-((R)-3-oxo-4-(3-(3-oxomorpholino)phenyl)morpholin-2-yl)acetic acid MD 9-5

According to the Step MD 1-3 in the synthetic method for EXAMPLE aa117. compound MD 9-4 (0.72 g) was used instead of MD 1-2 to obtain MD 9-5 (0.60 g) as a light yellow solid which was used without further purification.

### [Step MD 9-6]

### (R)-2-(4-((tert-Butoxycarbonylamino)methyl)-3-fluorophenylamino)-2-oxo-1-((R)-3-ox o-4-(3-(3-oxomorpholino)phenyl)morpholin-2-yl)ethylacetate MD 9-6

According to the Step MD 1-4 in the synthetic method for EXAMPLE aa117. MD 4-5 (70 mg) was treated with *tert*-butyl-4-amino-2-fluorobenzylcarbamate (65 mg) to afford MD 9-6 (0.10 g) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 9-7]

### tert-Butyl 2-fluoro-4-((R)-2-hydroxy-2-((R)-3-oxo-4-(3-(3-oxomorpholino)phenyl)morpholin-2-y l)acetamido)benzylcarbamate MD 9-7

According to the Step MD 1-5 in the synthetic method for EXAMPLE aa117 , MD 9-6 (0.10 g) was used instead of compound MD 1-4 to obtain MD 9-7 (90 mg) as a white solid. Crude MD 9-7 was used without further purification in the next step.

### [Step MD 9-8]

### (R)-N-(4-(Aminomethyl)-3-fluorophenyl)-2-hydroxy-2-((R)-3-oxo-4-(3-(3-oxomorphol ino)phenyl)morpholin-2-yl)acetamide EXAMPLE aa125

To a solution of MD 9-7 (90 mg) in CH₂Cl₂ (5 mL) at 0°C was added TFA (1.5 mL) dropwise. The mixture was stirred for 3h at 0°C and concentrated to dryness and this protocol was repeated 5 times. The crude mixture was taken up in MeOH and treated with 1M HCl in Et₂O to afford after filtration EXAMPLE aa 125 (45 mg) as a pale yellow solid.

### EXAMPLE aa126

### (R)-N-(4-Guanidinophenyl)-2-hydroxy-2-((R)-3-oxo-4-(3-(3-oxomorpholino)phenyl)m orpholin-2-yl)acetamide EXAMPLE aa126

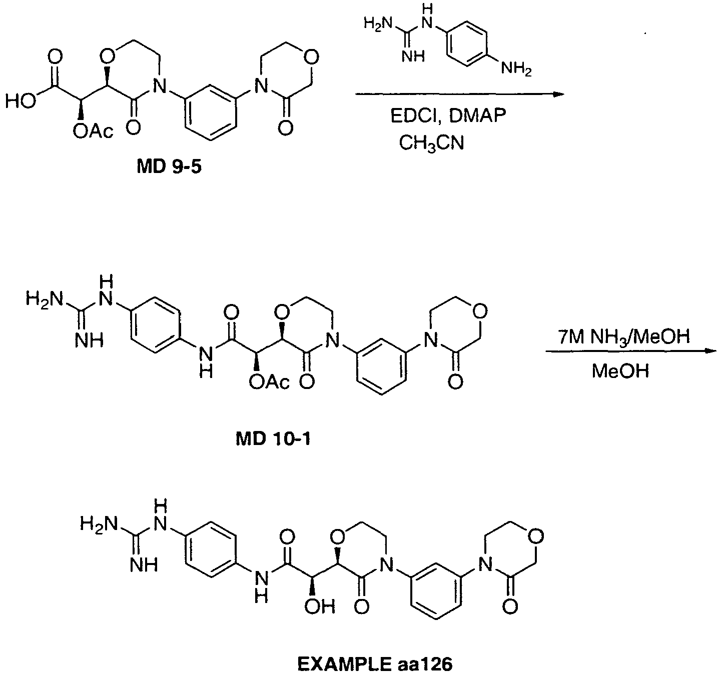

### [Step MD 10-1]

### (R)-2-(4-Guanidinophenylamino)-2-oxo-1-((R)-3-oxo-4-(3-(3-oxomorpholino)phenyl) morpholin-2-yl)ethyl acetate MD 10-1

According to the Step MD 1-4 in the synthetic method for EXAMPLE aa117, MD 9-5 (70 mg) was treated with *N*-(4-aminophenyl)guanidine (41 mg) to afford MD 10-1 (65 mg) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 10-2]

### (R)-N-(4-Guanidinophenyl)-2-hydroxy-2-((R)-3-oxo-4-(3-(3-oxomorpholino)phenyl)m orpholin-2-yl)acetamide EXAMPLE aa126

According to the Step MD 1-5 in the synthetic method for EXAMPLE aa117 , MD 10-1 (60 mg) was used instead of compound MD 1-4 to obtain EXAMPLE aa126 (35 mg) as a yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### EXAMPLE aa127

### (R)-N-(4-(Aminomethyl)phenyl)-2-((R)-4-(4-fluoro-3-(morpholine-4-carbonyl)phenyl)-3-oxomorpholin-2-yl)-2-hydroxyacetamide EXAMPLE aa127

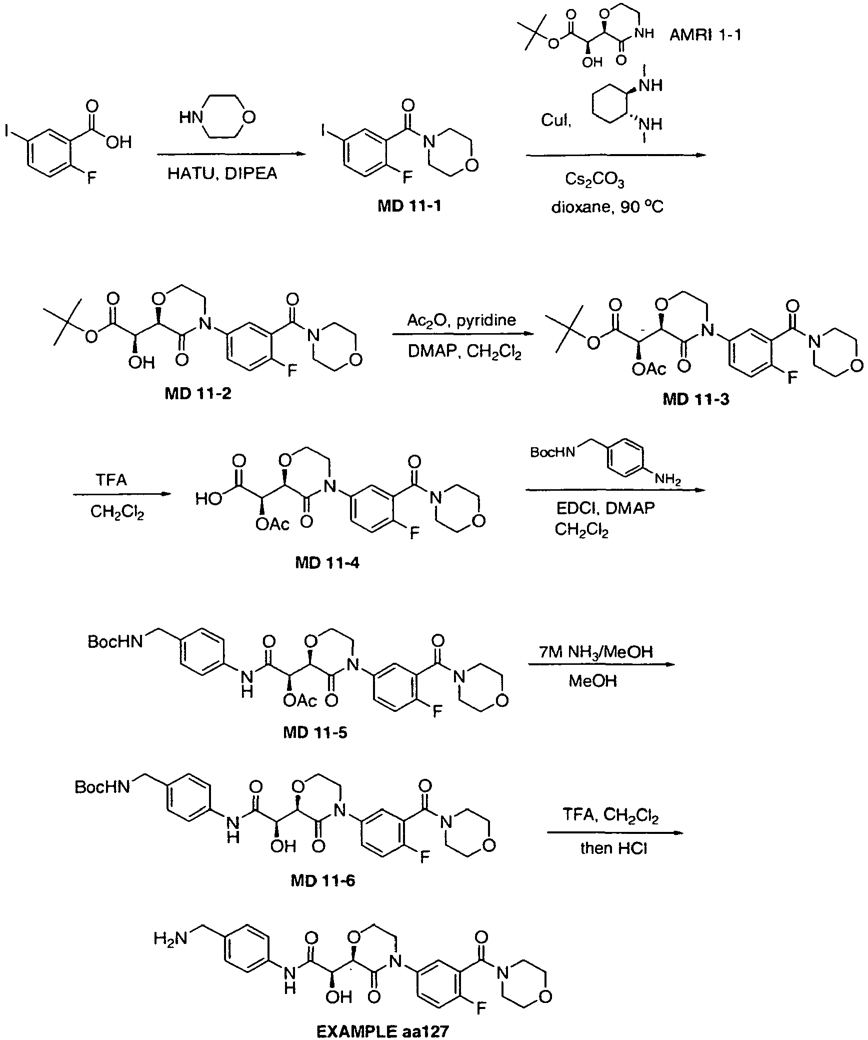

### [Step MD 11-1]

### (2-Fluoro-5-iodophenyl)(morpholino)methanone MD 11-1

To a solution of 3-iodo-6-fluorobenzoic acid (5.0 g) in DMF (50 mL) at rt was added morpholine (1.8 mL), HATU (8.6 g), and DIPEA (9.8 mL). The mixture was stirred for 12 h at rt whereupon the mixture was diluted with EtOAc. The organic layers were washed with 1N NaOH, 1M HCl, water, and brine. The organic layer was dried (Na₂SO₄), filtered, and concentrated under reduced pressure. The crude material was purified by flash chromatography using a gradient of 100% CH₂Cl₂ to 97.5% CH₂Cl₂/2.5 % MeOH to afford MD 11-1 (5.2 g) of the title compound as a brown solid.

### [Step MD 11-2]

### (R)-tert-Butyl 2-((R)-4-(4-fluoro-3-(morpholine-4-carbonyl)phenyl)-3-oxomorpholin-2-yl)-2-hydroxy acetate MD 11-2

According to the Step MD 1-1 in the synthetic method for EXAMPLE aa 117, compound MD 11-1 (0.87 g) was used in the presence of AMRI 1-1 (0.50 g) to obtain MD 11-2 (0.64 g) as yellow semisolid after flash chromatography using a 50:1 mixture of CH₂Cl₂/MeOH.

### [Step MD 11-3]

### (R)-tert-Butyl 2-acetoxy-2-((R)-4-(4-fluoro-3-(morpholine-4-carbonyl)phenyl)-3-oxomorpholin-2-yl) acetate MD 11-3

According to the Step MD 1-2 in the synthetic method for EXAMPLE aa117, compound MD 11-2 (0.64 g) was used instead of MD 1-1 to obtain MD 11-3 (0.71 g) as an light yellow oil which was used without further purification.

### [Step MD 11-4]

### (R)-2-Acetoxy-2-((R)-4-(4-fluoro-3-(morpholine-4-carbonyl)phenyl)-3-oxomorpholin-2-yl)acetic acid MD 11-4

According to the Step MD 1-3 in the synthetic method for EXAMPLE aa117, compound MD 11-3 (0.71 g) was used instead of MD 1-2 to obtain MD 11-4 (0.60 g) as a light yellow solid which was used without further purification.

### [Step MD 11-5]

### (R)-2-(4-((tert-Butoxycarbonylamino)methyl)phenylamino)-1-((R)-4-(4-fluoro-3-(morp holine-4-carbonyl)phenyl)-3-oxomorpholin-2-yl)-2-oxoethyl acetate MD 11-5

According to the Step MD 1-4 in the synthetic method for EXAMPLE aa117, MD 11-4 (0.62 g) was treated with *tert*-butyl-4-aminobenzylcarbamate (0.48 g) to afford MD 11-5 (0.71 g) as a pale yellow solid after flash chromatography using a 50:1 mixture of CH₂Cl₂/MeOH.

### [Step MD 11-6]

### tert-Butyl 4-((R)-2-((R)-4-(4-fluoro-3-(morpholine-4-carbonyl)phenyl)-3-oxomorpholin-2-yl)-2-h ydroxyacetamido)benzylcarbamate MD 11-6

According to the Step MD 1-5 in the synthetic method for EXAMPLE aa117 , MD 11-5 (0.71 g) was used instead of compound MD 1-4 to obtain MD 11-6 (0.66 g) as a white solid. Crude MD 9-7 was used without further purification in the next step.

### [Step MD 11-7]

### (R)-N-(4-(Aminomethyl)phenyl)-2-((R)-4-(4-fluoro-3-(morpholine-4-carbonyl)phenyl)-3-oxomorpholin-2-yl)-2-hydroxyacetamide EXAMPLE aa127

According to the Step MD 9-8 in the synthetic method for EXAMPLE aa 125, MD 11-6 (0.66 g) was used instead of compound MD 9-7 to obtain EXAMPLE aa127 (0.21 g) as a pale yellow hydrochloride salt upon treatment with HCl.

### EXAMPLE aa 128

### (R)-2-(4-Carbamoylphenylamino)-1-((R)-4-(4-fluoro-3-(morpholine-4-carbonyl)phenyl )-3-oxomorpholin-2-yl)-2-oxoethyl acetate EXAMPLE aa128

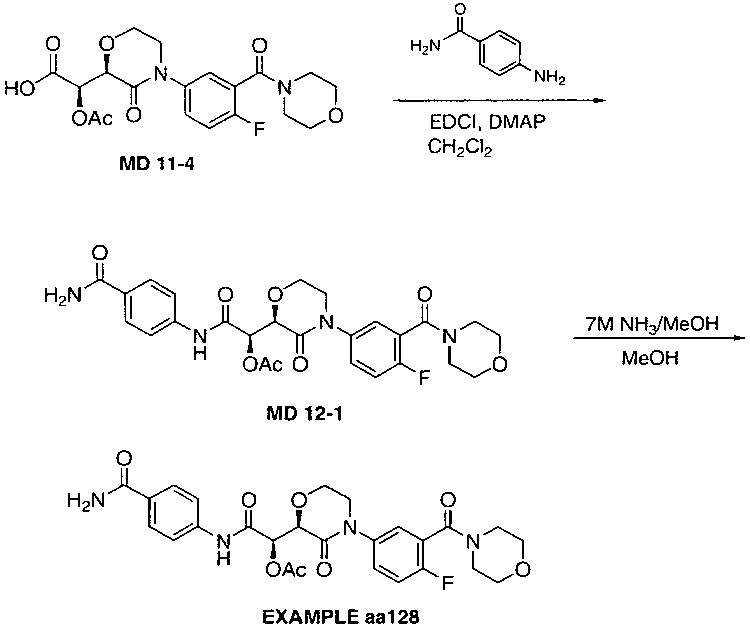

### [Step MD 12-1]

### (R)-2-(4-carbamoylphenylamino)-1-((R)-4-(4-fluoro-3-(morpholine-4-carbonyl)phenyl) -3-oxomorpholin-2-yl)-2-oxoethyl acetate MD 12-1

According to the Step MD 1-4 in the synthetic method for EXAMPLE aa117, MD 11-4 (0.10 g) was treated with 4-aminobenzamide (49 mg) to afford MD 12-1 (60 mg) as a yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step 12-2]

### (R)-2-(4-Carbamoylphenylamino)-1-((R)-4-(4-fluoro-3-(morpholine-4-carbonyl)phenyl )-3-oxomorpholin-2-yl)-2-oxoethyl acetate EXAMPLE aa128

According to the Step MD 1-5 in the synthetic method for EXAMPLE aa117 , MD 12-1 (60 mg) was used instead of compound MD 1-4 to obtain EXAMPLE aa128 (31 mg) as a pale white solid after reverse phase HPLC purification using a C18 column and a gradient of 89.95:9.95:0.1 H₂O:MeCN:HCO₂H to 9.95:89.95:0.1 H₂O:MeCN:HCO₂H.

### EXAMPLE aa129

### (R)-2-((R)-4-(4-Fluoro-3-(morpholine-4-carbonyl)phenyl)-3-oxomorpholin-2-yl)-2-hyd roxy-N-(1,2,3,4-tetrahydroisoquinolin-6-yl)acetamide EXAMPLE aa129

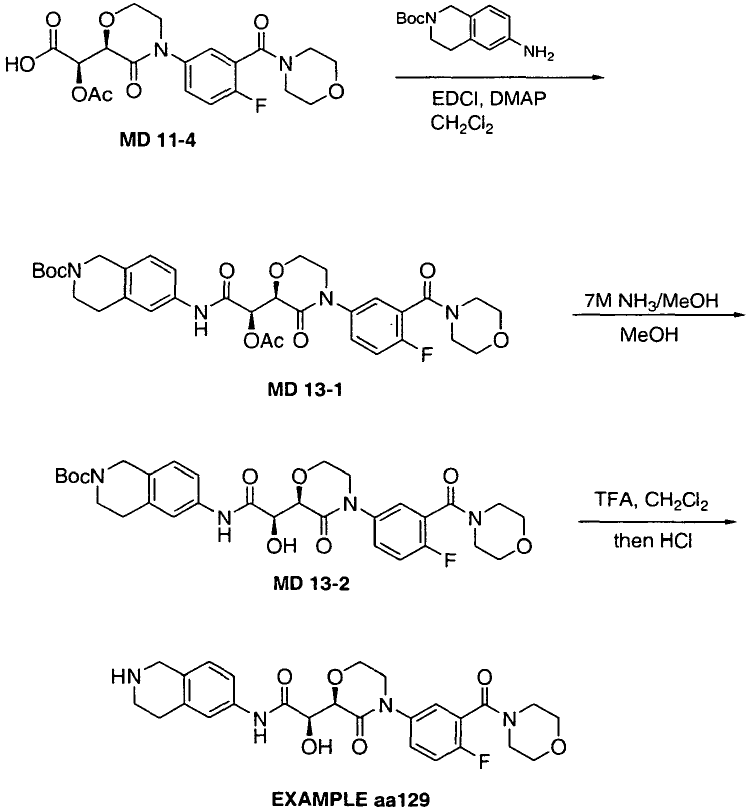

### [Step MD 13-1]

### tert-Butyl 6-((R)-2-acetoxy-2-((R)-4-(4-fluoro-3-(morpholine-4-carbonyl)phenyl)-3-oxomorpholi n-2-yl)acetamido)-3,4-dihydroisoquinoline-2(1H)-carboxylate MD 13-1

According to the Step MD 1-4 in the synthetic method for EXAMPLE aa117, MD 11-4 (0.10 g) was treated with *tert*-butyl 6-amino-3,4-dihydroisoquinoline-2(1*H*)-carboxylate (89 mg) to afford MD 13-1 (0.10 g) as a yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 13-2]

### tert-Butyl 6-((R)-2-((R)-4-(4-fluoro-3-(morpholine-4-carbonyl)phenyl)-3-oxomorpholin-2-yl)-2-h ydroxyacetamido)-3,4-dihydroisoquinoline-2(1H)-carboxylate MD 13-2

According to the Step MD 1-5 in the synthetic method for EXAMPLE aa117 , MD 13-1 (0.10 g) was used instead of compound MD 1-4 to obtain MD 13-2 (94 mg) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 13-3]

### (R)-2-((R)-4-(4-Fluoro-3-(morpholine-4-carbonyl)phenyl)-3-oxomorpholin-2-yl)-2-hyd roxy-N-(1,2,3,4-tetrahydroisoquinolin-6-yl)acetamide EXAMPLE aa129

According to the Step MD 9-8 in the synthetic method for EXAMPLE aa125, MD 13-2 (94 mg) was used instead of compound MD 9-7 to obtain EXAMPLE aa129 (90 mg) as a pale yellow solid after treatment with HCl.

### EXAMPLE aa130

### (R)-N-(4-(Aminomethyl)-3-fluorophenyl)-2-((R)-4-(4-fluoro-3-(morpholine-4-carbonyl )phenyl)-3-oxomorpholin-2-yl)-2-hydroxyacetamide EXAMPLE aa130

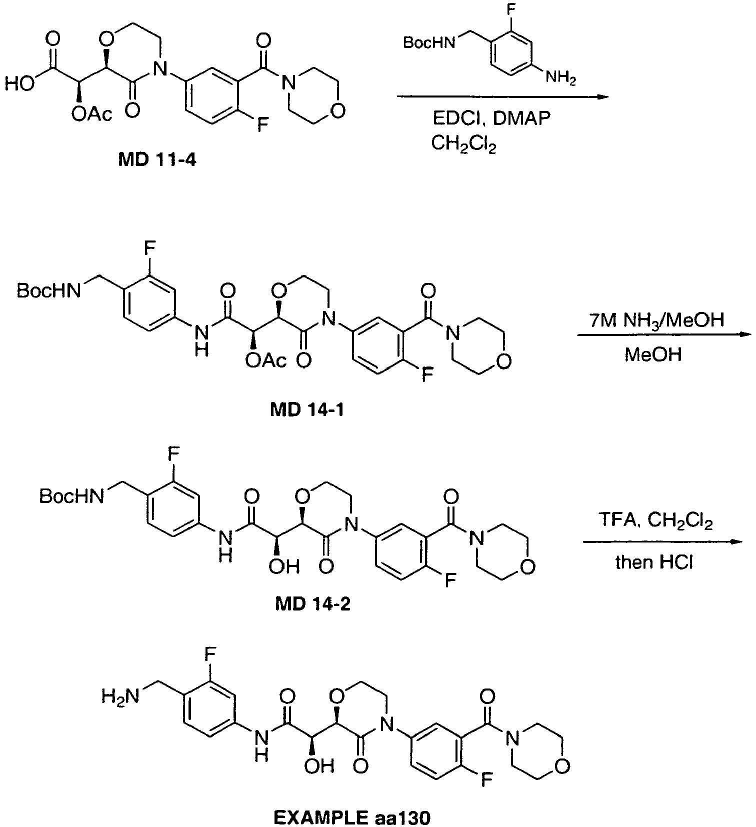

### [Step MD 14-1]

### (R)-2-(4-((tert-Butoxycarbonylamino)methyl)-3-fluorophenylamino)-1-((R)-4-(4-fluoro -3-(morpholine-4-carbonyl)phenyl)-3-oxomorpholin-2-yl)-2-oxoethyl acetate MD 14-1

According to the Step MD 1-4 in the synthetic method for EXAMPLE aa117, MD 11-4 (0.10 g) was treated with *tert*-butyl-4-amino-2-fluorobenzylcarbamate (86 mg) to afford MD 14-1 (0.13 g) as an off-white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 14-2]

### tert-Butyl 2-fluoro-4-((R)-2-((R)-4-(4-fluoro-3-(morpholine-4-carbonyl)phenyl)-3-oxomorpholin-2-yl)-2-hydroxyacetamido)benzylcarbamate MD 14-2

According to the Step MD 1-5 in the synthetic method for EXAMPLE aa117 , MD 14-1 (0.13 g) was used instead of compound MD 1-4 to obtain MD 14-2 (90 mg) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 14-3]

### (R)-N-(4-(Aminomethyl)-3-fluorophenyl)-2-((R)-4-(4-fluoro-3-(morpholine-4-carbonyl )phenyl)-3-oxomorpholin-2-yl)-2-hydroxyacetamide EXAMPLE aa130

According to the Step MD 9-8 in the synthetic method for EXAMPLE aa125, MD 14-2 (90 mg) was used instead of compound MD 9-7 to obtain EXAMPLE aa130 (95 mg) as a pale yellow solid after treatment with HCl.

### EXAMPLE aa 131

### (R)-N-(4-((R)-1-Aminoethyl)phenyl)-2-((R)-4-(4-fluoro-3-(morpholine-4-carbonyl)phe nyl)-3-oxomorpholin-2-yl)-2-hydroxyacetamide EXAMPLE aa 131

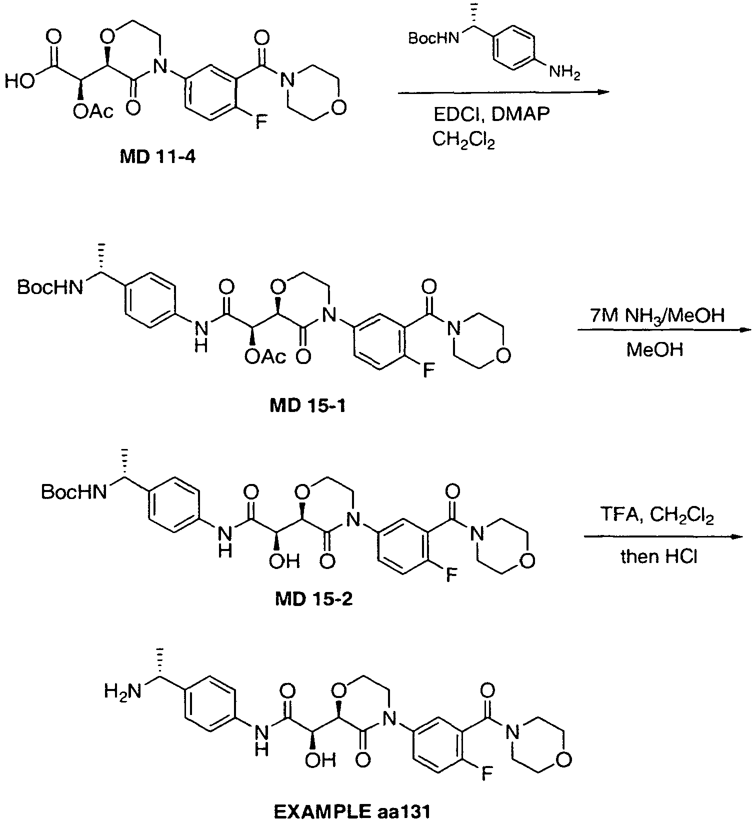

### [Step MD 15-1]

### (R)-2-(4-((R)-1-(tert-Butoxycarbonylamino)ethyl)phenylamino)-1-((R)-4-(4-fluoro-3-( morpholine-4-carbonyl)phenyl)-3-oxomorpholin-2-yl)-2-oxoethyl acetate MD 15-1

According to the Step MD 1-4 in the synthetic method for EXAMPLE aa117, MD 11-4 (0.10 g) was treated with (*R*)-[1-(4-amino-phenyl)-ethyl]-carbamic acid *tert*-butyl ester (85 mg) to afford MD 15-1 (0.12 g) as an off-white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 15-2]

### tert-Butyl (R)-1-(4-((R)-2-((R)-4-(4-fluoro-3-(morpholine-4-carbonyl)phenyl)-3-oxomorpholin-2-yl)-2-hydroxyacetamido)phenyl)ethylcarbamate MD 15-2

According to the Step MD 1-5 in the synthetic method for EXAMPLE aa117 , MD 15-1 (0.12 g) was used instead of compound MD 1-4 to obtain MD 15-2 (80 mg) as a pale yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 15-3]

### (R)-N-(4-((R)-1-Aminoethyl)phenyl)-2-((R)-4-(4-fluoro-3-(morpholine-4-carbonyl)phe nyl)-3-oxomorpholin-2-yl)-2-hydroxyacetamide EXAMPLE aa131

According to the Step MD 9-8 in the synthetic method for EXAMPLE aa 125, MD 15-3 (90 mg) was used instead of compound MD 9-7 to obtain EXAMPLE aa131 (80 mg) as a pale yellow solid after treatment with HCl.

### EXAMPLE aa132

### (R)-N-(1-Aminoisoquinolin-6-yl)-2-((R)-4-(4-fluoro-3-(morpholine-4-carbonyl)phenyl) -3-oxomorpholin-2-yl)-2-hydroxyacetamide EXAMPLE aa132

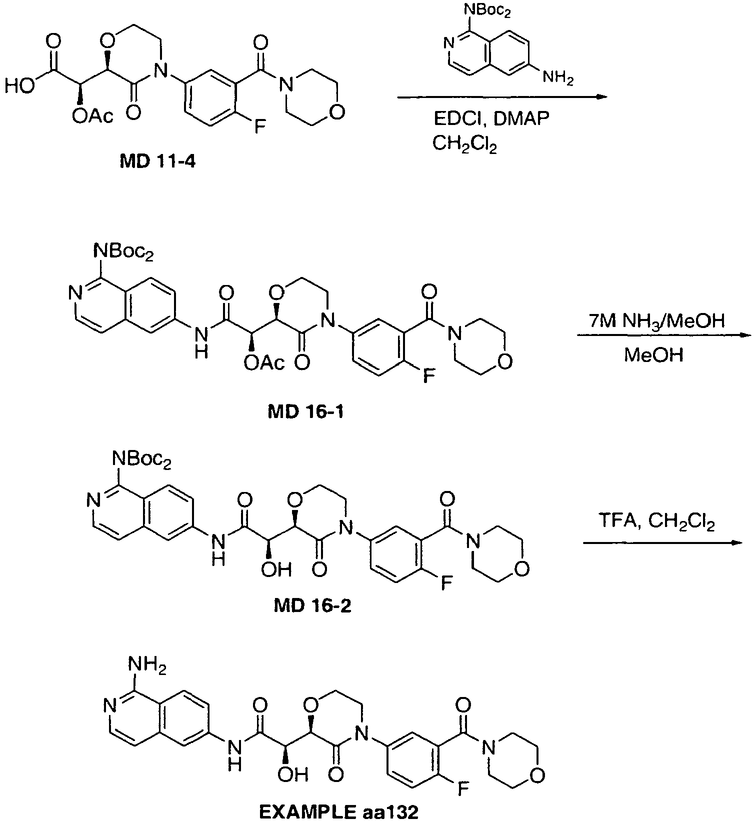

### [Step MD 16-1]

### (R)-2-(1-(bis(tert-Butoxycarbonyl)amino)isoquinolin-6-ylamino)-1-((R)-4-(4-fluoro-3-( morpholine-4-carbonyl)phenyl)-3-oxomorpholin-2-yl)-2-oxoethyl acetate MD 16-1

According to the Step MD 1-4 in the synthetic method for EXAMPLE aa 117, MD 11-4 (0.23 g) was treated with di-*tert*-butyl(6-aminoisoquinolin-1-yl)imidocarbonate (0.25 g) from WO 2006/062972 to afford MD 16-1 (0.29 g) as an yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 16-2]

### (R)-2-(1-(bis(tert-Butoxycarbonyl)amino)isoquinolin-6-ylamino)-1-((R)-4-(4-fluoro-3-( morpholine-4-carbonyl)phenyl)-3-oxomorpholin-2-yl)-2- hydroxyacetamide MD 16-2

According to the Step MD 1-5 in the synthetic method for EXAMPLE aa117 , MD 16-1 (0.29 g) was used instead of compound MD 1-4 to obtain MD 16-2 (0.27 g) as maize solid which was used without further purification.

### [Step MD 16-3]

### (R)-N-(1-Aminoisoquinolin-6-yl)-2-((R)-4-(4-fluoro-3-(morpholine-4-carbonyl)phenyl) -3-oxomorpholin-2-yl)-2-hydroxyacetamide EXAMPLE aa132

According to the Step MD 9-8 in the synthetic method for EXAMPLE aa 125, MD 16-3 (0.27 g) was used instead of compound MD 9-7 to obtain EXAMPLE aa132 (0.11 g) as a pale yellow solid after reverse phase HPLC purification using a C18 column and a gradient of 89.95:9.95:0.1 H₂O:MeCN:TFA to 9.95:89.95:0.1 H₂O:MeCN:TFA to afford the trifluoroacetate salt.

### EXAMPLE aa133

### (R)-N-((R)-1-amino-2,3-dihydro-1H-inden-5-yl)-2-((R)-4-(4-fluoro-3-(morpholine-4-ca rbonyl)phenyl)-3-oxomorpholin-2-yl)-2-hydroxyacetamide EXAMPLE aa133

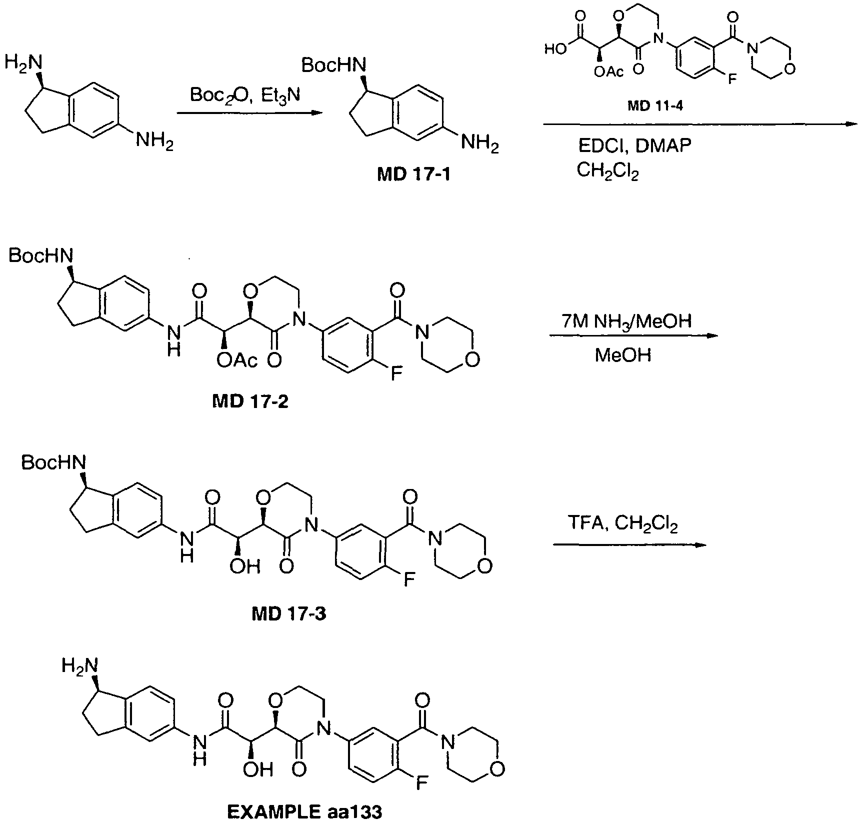

### [Step MD 17-1]

### (R)-tert-Butyl 5-amino-2,3-dihydro-1H-inden-1-ylcarbamate MD 17-1

To a solution of (1*R*)-indane-1,5-amine hydrochloride (0.60 g) in CH₂Cl₂ (16 mL) at 0 °C was added Et₃N (1.4 mL) followed by Boc₂O (0.85 g). The mixture was allowed to warm to rt and was stirred for 12 h. The mixture was diluted with CH₂Cl₂ (5 mL) and sat. aq. NaHCO₃ (3 mL) and the layers were separated. The aqueous layer was extracted with CH₂Cl₂ (2 x 5 mL) and the organic layers were combined. The organic layer was dried (Na₂SO₄), filtered and concentrated under reduced pressure. The crude material was purified by flash chromatography using a gradient of 100% hexanes to 20% hexanes/80% EtOAc to afford MD 17-1 (0.26 g) as a yellow/orange semisolid.

### [Step MD 17-2]

### (R)-2-((R)-1-(tert-Butoxycarbonylamino)-2,3-dihydro-1H-inden-5-ylamino)-1-((R)-4-( 4-fluoro-3-(morpholine-4-carbonyl)phenyl)-3-oxomorpholin-2-yl)-2-oxoethyl acetate MD 17-2

According to the Step MD 1-4 in the synthetic method for EXAMPLE aa117, MD 17-1 (0.11 g) was treated with MD 11-4 (0.15 g) to afford MD 17-2 (0.21 g) as a white solid afterreverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 17-3]

### tert-Butyl (R)-5-((R)-2-((R)-4-(4-fluoro-3-(morpholine-4-carbonyl)phenyl)-3-oxomorpholin-2-yl) -2-hydroxyacetamido)-2,3-dihydro-1H-inden-1-ylcarbamate MD 17-3

According to the Step MD 1-5 in the synthetic method for EXAMPLE aa117 , MD 17-2 (0.21 g) was used instead of compound MD 1-4 to obtain MD 17-2 (0.14 g) as a white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 17-4]

### (R)-N-((R)-1-amino-2,3-dihydro-1H-inden-5-yl)-2-((R)-4-(4-fluoro-3-(morpholine-4-ca rbonyl)phenyl)-3-oxomorpholin-2-yl)-2-hydroxyacetamide EXAMPLE aa133

According to the Step MD 9-8 in the synthetic method for EXAMPLE aa125, MD 17-3 (0.14 g) was used instead of compound MD 9-7 to obtain EXAMPLE aa133 (38 mg) as a clear glass after reverse phase HPLC purification using a C18 column and a gradient of 89.95:9.95:0.1 H₂O:MeCN:TFA to 9.95:89.95:0.1 H₂O:MeCN:TFA to afford the trifluoroacetate salt.

### EXAMPLE aa134

### (R)-N-((S)-1-Amino-2,3-dihydro-1H-inden-5-yl)-2-((R)-4-(4-fluoro-3-(morpholine-4-ca rbonyl)phenyl)-3-oxomorpholin-2-yl)-2-hydroxyacetamide EXAMPLE aa134

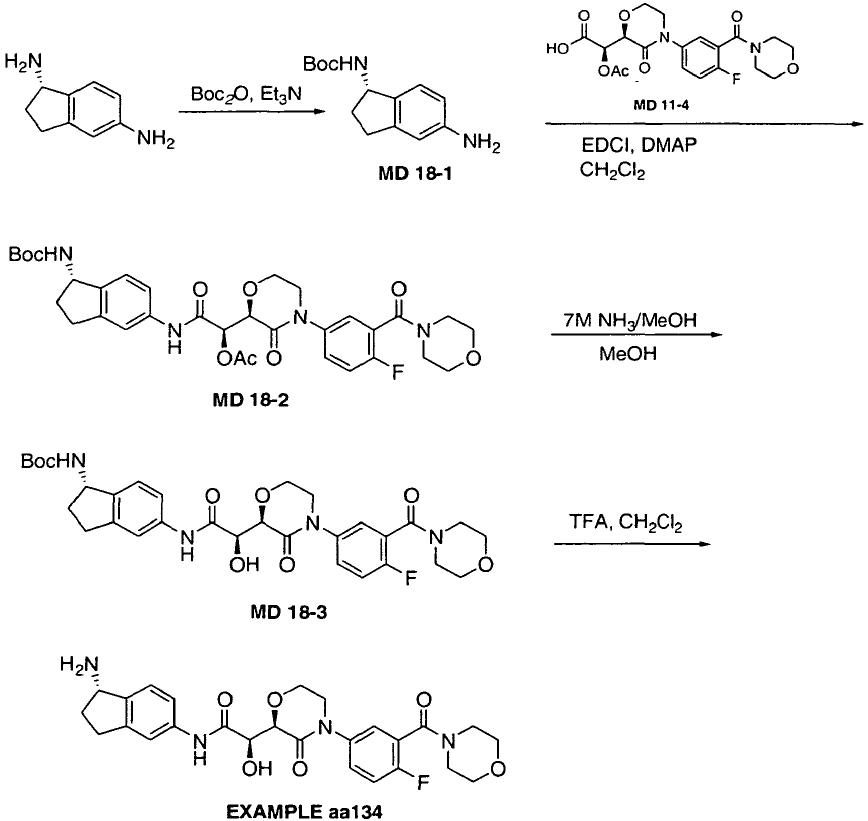

### [Step MD 18-1]

### (S)-tert-Butyl 5-amino-2,3-dihydro-1H-inden-1-ylcarbamate MD 18-1

According to the Step MD 17-1 in the synthetic method for EXAMPLE aa133, (1*S*)-indane-1,5-amine hydrochloride (0.72 g) was used to produce MD 18-1 (0.40g) as a yellow semisolid.

### [Step MD 18-2]

### (R)-2-((S)-1-(tert-Butoxycarbonylamino)-2,3-dihydro-1H-inden-5-ylamino)-1-((R)-4-(4 -fluoro-3-(morpholine-4-carbonyl)phenyl)-3-oxomorpholin-2-yl)-2-oxoethyl acetate MD 18-2

According to the Step MD 1-4 in the synthetic method for EXAMPLE aa117, MD 18-1 (0.11 g) was treated with MD 11-4 (0.15 g) to afford MD 17-2 (0.24 g) as a white solid afterreverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 18-3]

### tert-Butyl (S)-5-((R)-2-((R)-4-(4-fluoro-3-(morpholine-4-carbonyl)phenyl)-3-oxomorpholin-2-yl)-2-hydroxyacetamido)-2,3-dihydro-1H-inden-1-ylcarbamate MD 18-3

According to the Step MD 1-5 in the synthetic method for EXAMPLE aa117 , MD 18-2 (0.24 g) was used instead of compound MD 1-4 to obtain MD 18-3 (0.18 g) as a white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 18-4]

### (R)-N-((S)-1-Amino-2,3-dihydro-1H-inden-5-yl)-2-((R)-4-(4-fluoro-3-(morpholine-4-ca rbonyl)phenyl)-3-oxomorpholin-2-yl)-2-hydroxyacetamide EXAMPLE aa134

According to the Step MD 9-8 in the synthetic method for EXAMPLE aa125, MD 18-3 (0.14 g) was used instead of compound MD 9-7 to obtain EXAMPLE aa134 (56 mg) as a clear glass after reverse phase HPLC purification using a C18 column and a gradient of 89.95:9.95:0.1 H₂O:MeCN:TFA to 9.95:89.95:0.1 H₂O:MeCN:TFA to afford the trifluoroacetate salt.

### EXAMPLE aa 135

### (R)-N-(2-Aminoquinolin-6-yl)-2-((R)-4-(4-fluoro-3-(morpholine-4-carbonyl)phenyl)-3-oxomorpholin-2-yl)-2-hydroxyacetamide EXAMPLE aa135

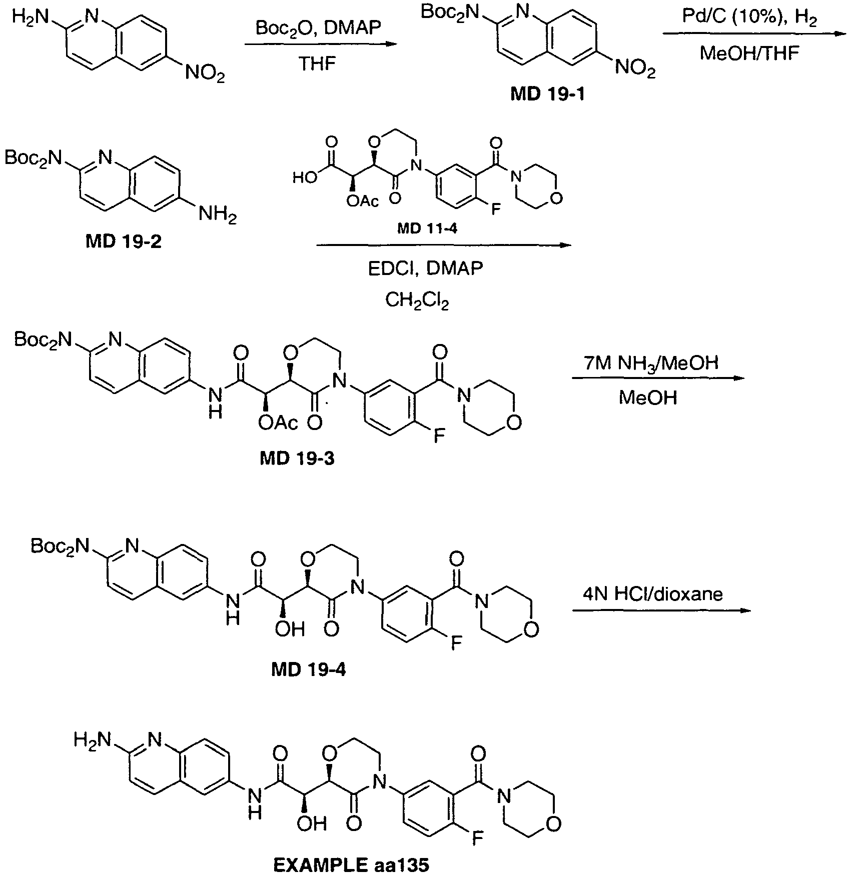

### [Step MD 19-1]

### 2-bis(tert-Butoxycarbonylamine)-6-nitroquinoline MD 19-1

To a solution of 6-nitroquinoline-2-amine (1.0 g) in THF (30 mL) was added Boc₂O (2.9 g) and DMAP (32 mg). The mixture was heated at refux for 12 h whereupon an additional portion of Boc₂O (0.7 g) and DMAP (60 mg) were added. The mixture was stirred at reflux for an additional 12 h whereupon the mixture was cooled and concentrated under reduced pressure. The resultant solid was dissolved in CH₂Cl₂ (50 mL) and was washed with sat. aq. NH₄Cl (1 x 15 mL) and sat. aq. NaHCO₃ (1 x 15 mL). The organic layer was dried (Na₂SO₄), filtered, and concentrated under reduced pressure. The crude material was purified by flash chromatography using a gradient of 100% hexanes to 50% hexanes/50% EtOAc to afford MD 19-1 (2.1 g) as a white solid.

### [Step MD 19-2]

### 2-(bis(tert-Butoxycarbonyl)amino)quinolin-6-ylamine MD 19-2

To a heterogenous mixture of MD 19-1 (2.0 g) in MeOH/THF (12 mL/12 mL) was added 10% Pd/C (100 mg). The mixture was stirred under a H₂ balloon for 12 h whereupon the mixture was purged to N₂. The mixture was filtered thru a pad of Celite and the pad was generously washed with MeOH (5 x 10 mL). The filtrate was concentrated under reduced pressure and placed under high vacuum to afford MD 19-2 (1.7 g) as a light yellow solid.

### [Step MD 19-3]

### (R)-2-(2-(bis(tert-Butoxycarbonyl)amino)quinolin-6-ylamino)-1-((R)-4-(4-fluoro-3-(m orpholine-4-carbonyl)phenyl)-3-oxomorpholin-2-yl)-2-oxoethyl acetate MD 19-3

According to the Step MD 1-4 in the synthetic method for EXAMPLE aa117, MD 11-4 (0.15 g) was treated with MD 19-2 (0.17 g) to afford MD 19-3 (0.13 g) as an yellow solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 19-4]

### (R)-2-(2-(bis(tert-Butoxycarbonyl)amino)quinolin-6-ylamino)-1-((R)-4-(4-fluoro-3-(m orpholine-4-carbonyl)phenyl)-3-oxomorpholin-2-yl)-2-hydroxyacetamide MD 19-4

According to the Step MD 1-5 in the synthetic method for EXAMPLE aa117, MD 19-3 (0.13 g) was used instead of compound MD 1-4 to obtain MD 19-4 (0.10 g) as white solid after reverse phase HPLC purification using a C18 column and a gradient of 89.95:9.95:0.1 H₂O:MeCN:HCO₂H to 9.95:89.95:0.1 H₂O:MeCN:HCO₂H.

### [Step MD 19-5]

### (R)-N-(2-Aminoquinolin-6-yl)-2-((R)-4-(4-fluoro-3-(morpholine-4-carbonyl)phenyl)-3-oxomorpholin-2-yl)-2-hydroxyacetamide EXAMPLE aa135

According to the Step MD 9-8 in the synthetic method for EXAMPLE aa125, MD 19-4 (0.10 g) was used instead of compound MD 9-7 using 4N HCl in dioxane obtain EXAMPLE aa135 (85 mg) as a yellow solid after treatment with HCl.

| EXAMPLE | LCMS | | |
|---|---|---|---|
| | m/z | RT | Method |
| | [M+1]⁺ | min | |
| aa117 | 465 | 2.48 | A |
| MD 1-1 | 392 | 4.86 | A |
| MD 1-2 | 434 | 1.95 | B |
| MD 1-3 | 378 | 1.64 | B |
| MD 1-4 | 729 (Na) | 1.99 | B |
| MD 1-5 | 687 | 1.94 | B |
| aa118 | 450 | 1.73 | A |
| MD 2-1 | 274 | 1.65 | B |
| MD 2-2 | 377 | 1.53 | B |
| MD 2-3 | 419 | 1.65 | B |
| MD 2-4 | 363 | 1.17 | B |
| MD 2-5 | 714 (Na) | 1.93 | B |
| MD 2-6 | 672 (Na) | 1.79 | B |
| aa119 | 450 | 1.70 | A |
| MD 3-1 | 274 | 3.36 | A |
| MD 3-2 | 321 | 3.08 | A |
| MD 3-3 | 419 | 3.23 | A |
| MD 3-4 | 363 | 1.18 | B |
| MD 3-5 | 714 (Na) | 1.81 | B |
| MD 3-6 | 672 (Na) | 1.77 | B |
| aa120 | 483 | 2.17 | A |
| MD 4-1 | 410 | 1.69 | B |
| MD 4-2 | 452 | 1.82 | B |
| MD 4-3 | 396 | 1.53 | B |
| MD 4-4 | 747 (Na) | 1.92 | B |
| MD 4-5 | 705 (Na) | 1.88 | B |
| aa121 | 425 | 2.12 | A |
| MD 5-1 | 416 (Na) | 1.71 | B |
| MD 5-2 | 338 | 1.35 | B |
| MD 5-3 | 481 (Na) | 1.68 | B |
| MD 5-4 | 647 (Na) | 1.87 | B |
| aa122 | 305 | 1.19 | A |
| MD 6-1 | 296 (Na) | 1.28 | B |
| MD 6-2 | 218 | -- | B |
| MD 6-3 | 569 (Na) | 1.71 | B |
| MD 6-4 | 527 (Na) | 1.67 | B |
| aa123 | 487 | 3.28 | A |
| MD 7-1 | 414 | 4.25 | A |
| MD 7-2 | 478 (Na) | 1.85 | B |
| MD 7-3 | 400 | 1.61 | B |
| MD 7-4 | 729 | 2.41 | B |
| MD 7-5 | 709 (Na) | 2.38 | B |
| aa124 | 490 | 2.18 | A |
| MD 8-1 | 314 | 1.84 | B |
| MD 8-2 | 417 | 1.93 | B |
| MD 8-3 | 459 | 1.92 | B |
| MD 8-4 | 403 | 1.39 | B |
| MD 8-5 | 754 (Na) | 1.89 | B |
| MD 8-6 | 712 (Na) | 1.96 | B |
| aa125 | 473 | 1.71 | A |
| MD 9-1 | 264 | 3.33 | A |
| MD 9-2 | 304 | 3.97 | A |
| MD 9-3 | 407 | 1.50 | B |
| MD 9-4 | 449 | 1.65 | B |
| MD 9-5 | 393 | 1.13 | B |
| MD 9-6 | 615 | 3.55 | A |
| MD 9-7 | 595 (Na) | 1.61 | A |
| aa126 | 483 | 1.61 | A |
| MD 10-1 | 525 | 1.82 | A |
| aa127 | 487 | 1.86 | A |
| MD 11-1 | 336 | 1.48 | B |
| MD 11-2 | 439 | 2.81 | A |
| MD 11-3 | 481 | 1.91 | B |
| MD 11-4 | 425 | 1.55 | B |
| MD 11-5 | 629 | 3.98 | A |
| MD 11-6 | 587 | 3.72 | A |
| aa128 | 501 | 2.08 | A |
| MD 12-1 | 543 | 1.30 | B |
| aa129 | 513 | 1.68 | A |
| MD 13-1 | 655 | 1.79 | B |
| MD 13-2 | 613 | 3.51 | A |
| aa130 | 505 | 1.76 | A |
| MD 14-1 | 665 (Na) | 1.72 | B |
| MD 14-2 | 505 (-Boc) | 1.65 | B |
| aa131 | 501 | 1.78 | A |
| MD 15-1 | 543 (-Boc) | 1.72 | B |
| MD 15-2 | 501 (-Boc) | 1.65 | B |
| aa132 | 524 | 1.95 | A |
| MD 16-1 | 788 (Na) | 2.22 | B |
| MD 16-2 | 724 | 1.01 | B |
| aa133 | 535 (Na) | 1.33 | B |
| MD 17-1 | 249 | 2.42 | A |
| MD 17-2 | 677 (Na) | 1.94 | B |
| MD 17-3 | 635 (Na) | 1.97 | B |
| aa134 | 535 (Na) | 1.34 | B |
| MD 18-1 | 249 | 2.38 | A |
| MD 18-2 | 677 (Na) | 2.48 | B |
| MD 18-3 | 635 (Na) | 1.97 | B |
| aa135 | 524 | 1.79 | A |
| MD 19-1 | -- | -- | -- |
| MD 19-2 | 360 | 1.01 | B |
| MD 19-3 | 766 | 2.16 | B |
| MD 19-3 | 724 | 2.04 | B |

### Method A:

### Electro Spray Ionization Liquid Chromatography-Mass Spectrometry (ESI-LC/MS)

| | |
|---|---|
| Column: | Phenomenex Gemini C18, 50 x 4.6 mm, 5 micron |
| Mobile Phase: | A: 0.05% Trifluoroacetic acid in water |
| | B: 0.05% Trifluoroacetic acid in acetonitrile |
| Gradient: | 90% A and 10% B to 5% A and 95% B over 5 minutes |
| Flow rate: | 1.0 ml/min |
| UV detection: | 254 nM |
| Spectrometer: | PE SCIEX API-150EX, single quadrupole mass spectrometer |
| | |
| Method B: | |
| Column: | Zorbax SB-C-18; 1.8 micron |
| Mobile Phase: | A: 0.1% Trifluoroacetic acid in water |
| | B: 0.1% Trifluoroacetic acid in acetonitrile |
| Gradient: | 0 min = 10 % B |
| | 1.3 min = 55 % B |
| | 2.7 min = 95 % B |
| | 2.8 min = 10 % B |
| Flow rate: | 1.0 ml/min |
| UV detection: | 254 nM |
| Spectrometer: | Agilent 6140 Quadrapole LC-MS, single quadrupole mass spectrometer |

| EXAMPLE | NMR (ppm)(400 MHz unless otherwise indicated) |
|---|---|
| aa117 | *DMSO-d*6: 10.3 (1H, s), 10.2 (1H, s), 9.68 (1H, s), 9.18 (1H, s), 8.33 (1H, s), 8.20 (1H, d, J = 8.3 Hz), 7.95 (1H, d, J = 8.2 Hz), 7.60-7.56 (2H, m), 7.49 (1H, d, J = 8.3 Hz), 7.31 (1H, d, J = 8.2 Hz), 6.52 (1H, d, J = 7.0 Hz), 4.79 (2H, s), 4.73 (1H, s), 4.70 (m, 1H), 4.14 (1H, m), 3.98-3.90 (2H, m), 3.72 (1H, m). |
| aa118 | *Cd3od-d4*: 8.25 (1H, s), 8.00 (1H, d, J = 8.4 Hz), 7.90-7.88 (1H, d, J = 1.8 Hz), 7.80-7.70 (1H, d, J = 1.8 Hz), 4.85 (2H, s), 4.83 (2H, s), 4.53 (2H, s), 4.25 (1H, m), 4.07 (2H, m), 3.71 (1H, m), 3.20 (3H, s).2H |
| aa119 | *Cd3od-d4*: 8.26 (1H, s), 8.05 (1H, d, J = 8.4 Hz), 7.90-7.88 (1H, d, J = 1.8 Hz), 7.70 (1H, s), 7.64 (2H, s), 4.85 (2H, s), 4.83 (2H, s), 4.53 (2H, s), 4.22 (1H, m), 4.06-4.03 (2H, m), 3.67 (1H, m), 3.21 (3H, s). |
| aa120 | *DMSO-d*6: 10.3 (1H, s), 9.80 (1H, br s), 8.43 (1H, s), 8.28 (1H, s), 8.05 (1H, d, J = 8.5 Hz), 7.95 (1H, d, J = 8.5 Hz), 7.34 (1H, t, J = 8.2 Hz), 7.05-7.01 (2H, m). 6.86 (1H, dd, J = 1.3, 8.2 Hz), 4.80 (2H, s), 4.74 (1H, s), 4.68 (1H, s), 4.19 (2H, q, J = 6.8 Hz). 4.13 (1H, m), 3.95-3.91 (m, 1H), 3.87-3.82 (1H, m), 3.69-3.60 (1H, m), 3.40 (1H, br s), 1.23 (3H, t, J = 6.8 Hz). |
| aa121 | *Cd3od-d4*: 8.21 (1H, s), 8.05 (1H, d, J = 8.4 Hz), 7.90 (1H, d, J = 8.1 Hz), 7.25-7.15 (2H, d, J = 8.5 Hz) 6.80-6.71 (2H, d, J = 8.5 Hz), 4.50 (2H, s), 4.22 (1H, s) 3.82-3.40 (6H, m), 3.20 (3H, s). |
| aa122 | *Cd3od-d4*: 8.21 (1H, s), 8.00 (1H, s), 7.81 (1H, s), 4.60 (2H, d, J = 1.7 Hz), 4.10 (2H, m), 3.85 (2H, m). |
| aa123 | *Cd3od-d4*: 8.21 (1H, s), 8.00 (1H, d. J = 8.6 Hz), 7.82 (1H, d, J = 1.8 Hz), 7.40-7.21 (6H, m), 7.10-7.05 (3H, m), 5.40 (2H, s), 4.80 (2H, s). 4.20 (1H, m), 4.05-3.95 (2H, m). 3.60 (1H, m), 3.35 (2H, s). |
| aa124 | *Cd3od-d4*: 8.25 (1H, s), 8.05 (1H, d, J = 8.6 Hz), 7.90-7.81 (1H, d, J = 1.9 Hz), 7.72 (1H, s), 7.60 (3H, m), 4.60 (2H, s), 4.22 (1H, m) 4.19-3.90 (2H, m), 3.65 (2H, m), 3.45 (2H, m), 1.10 (1H, t, J = 6.9 Hz), 0.55 (2H, d, J = 1.4 Hz), 0.45 (2H, d, J = 1.42 Hz). |
| aa125 | *Cd3od-d4:* 7.84-7.15 (7H, m), 4.79 (2H, s), 4.20-3.91 (7H, m), 3.81-3.62 (5H, m), 3.31 (2H, s). |
| aa126 | *Cd3od-d4*: 7.71-7.68 (2H, m), 7.53-7.45 (2H, m), 7.36-7.24 (4H, m), 5.62 (1H, s), 4.27 (3H, m), 4.11-4.08 (4H, m), 3.80 (4H, m) |
| aa127 | *Cd3od-d4*: 9.7 (1H, s), 7.75-7.73 (2H, d, J = 1.5 Hz), 7.58-7.41 (4H, m), 7.35-7.25 (1H, t, J = 9.2 Hz), 4.80 (2H, s), 4.28-3.93 (4H, m), 3.85-3.31 (8H, m). |
| aa128 | *Cd3od-d4*: 7.90-7.77 (4H, m), 7.62-7.53 (2H, m), 7.41-7.33 (1H, m), 4.80 (2H, s), 4.28-4.23 (1H, m), 4.19-4.02 (2H, m), 3.85-3.31 (7H, m). |
| aa129 | *Cd3od-d4*: 7.68-7.42 (5H, m), 7.30-7.20 (2H, m), 4.80 (2H, s), 4.35 (1H, s), 4.26 (2H, m), 4.10-4.05 (2H, m) 3.80-3.12 (5H, m). |
| aa130 | *Cd3od-d4:* 7.85-7.19 (7H, m), 4.79 (2H, s), 4.21-3.90 (7H, m), 3.81-3.62 (5H, m), 3.31 (2H, s). |
| aa131 | *Cd3od-d4:* 7.74 (2H, m), 7.48-7.43 (2H, m), 7.42 (2H, d, J = 8.4 Hz), 7.28 (1H, t, J = 8.8 Hz), 4.77 (2H, s), 4.42 (1H, m), 4.21-4.19 (2H, m), 3.99 (2H, m), 3.76-3.73 (4H, m), 3.64-3.61 (4H, m), 3.39 (2H, m), 1.61 (3H, d, J = 7.0 Hz). |
| aa132 | *Cd3OD-d4*: 8.45 (1H, s), 8.32 (2H, s), 7.96 (1H, d, J = 9.2 Hz), 7.59-7.49 (3H, m), 7.334-7.30 (1H, m), 7.08 (1H, d, J = 6.7 Hz), 4.88 (2H, s), 4.28-4.25 (1H, m), 4.10-4.02 (2H, m), 3.79-3.72 (4H, m), 3.69-3.65 (3H, m), 3.42 (2H, m). |
| aa133 | *Cd3OD-d4*: 8.30 (1H, br s), 7.72 (1H, s), 7.60-7.48 (4H, m), 7.33 (1H, t, J = 9.1 Hz), 4.82 (2H, m), 4.80-4.74 (2H, m), 4.24 (1H, m), 4.08-4.00 (2H, m), 3.79-3.73 (4H, m), 3.70-3.63 (3H, m), 3.43 (2H, s), 3.23-3.17 (1H, m), 3.05-3.00 (1H, m), 2.68-2.60 (1H, m), 2.17-2.06 (1H, m). |
| aa134 | *Cd3OD-d4*: 8.31 (1H, br s), 7.73 (1H, s), 7.59-7.48 (4H, m), 7.33 (1H, t, J = 8.9 Hz), 4.81 (2H, s), 4.77 (2H, m), 4.24-4.21 (1H, m), 4.08-4.00 (2H, m), 3.80-3.77 (4H, m), 3.70-3.64 (3H, m), 3.37 (2H, s), 3.23-3.17 (1H, m), 3.05-2.98 (1H, m), 2.68-2.60 (1H, m), 2.18-2.11 (1H, m). |
| aa135 | *DMSO-d*6: 10.1 (1H, s), 9.00 (1H, br s), 8.46 (1H, d, J = 1.6 Hz), 8.37 (1H, d, J = 9.4 Hz), 8.07 (1H, d, J = 1.6, 8.8 Hz), 7.68 (1H, d, J = 8.8 Hz), 7.58-7.52 (2H, m), 7.40 (1H, t, J = 8.8 Hz), 7.08 (1H, d, J = 9.4 Hz), 4.71 (1H, s), 4.68 (1H, s), 4.15 (1H, d, J = 11.6 Hz), 3.97-3.91 (2H, m), 3.87-3.30 (9H, m). |

Examples aa 136 - aa 153

### EXAMPLE aa 136

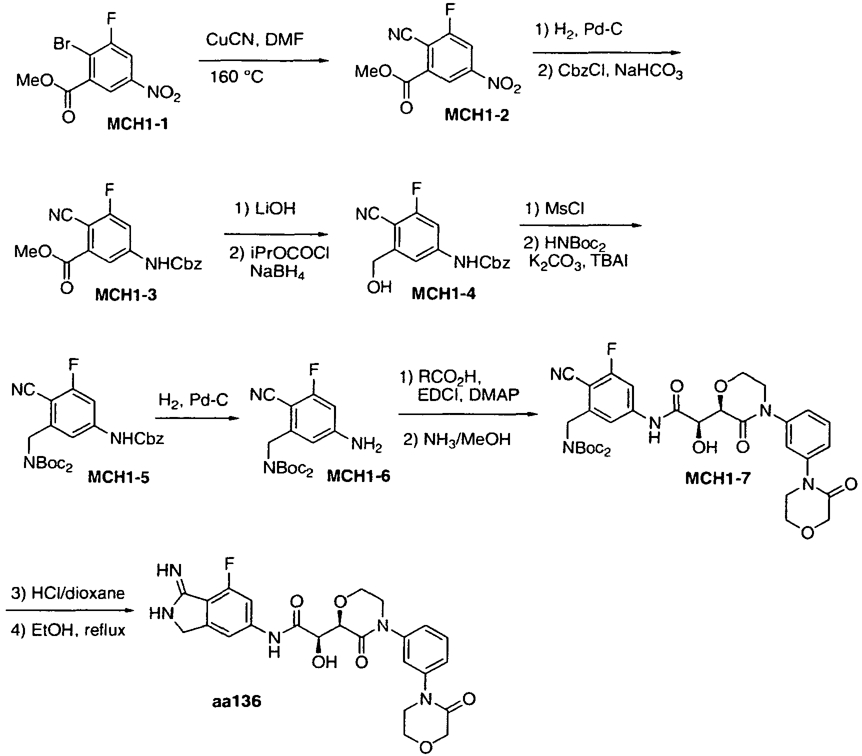

### [Step 1] Synthesis of

### Methyl 2-cyano-3-fluoro-5-nitrobenzoate (compound MCH1-2)

A mixture of **MCH1-1** (3.9 g, 14.03 mmol (prepared according to the procedure described in WO 2006/021457 A2)), CuCN (6.3 g, 70.34 mmol, 5 eq.) in 40 mL DMF was degassed under vacuum, filled with argon atmosphere and heated in an oil bath at ~160 °C (bath temp.) for 20 min. It was removed from the bath, cooled to rt then diluted with ethyl acetate while being stirred vigorously. The mixture was filtered through a pad of CELITE, rinsed with ethyl acetate and the filtrate was washed with water and brine. It was dried over MgSO₄, filtered and evaporated to dryness to give 3.02 g of **MCH1-2** as solid.

### [Step 2] Synthesis of

Methyl 5-(benzyloxycarbonylamino)-2-cyano-3-Fluorobenzoate (compound **MCH1-3)** A suspension of 3 (500 mg) and 10% Pd/C (50 mg) in 10 mL of 1:1 THF-EtOH was under overnight under a hydrogen balloon. It was filtered through a CELITE pad and evaporated to dryness to give 435 mg of aniline.

To a solution of the above aniline (430 mg, 2.22 mmol) and NaHCO3 (930 mg, 11.07 mmol, 5 eq.) in 10 mL THF at rt was added benzylchloroformate (0.95 mL, 0.66 mmol, 3 eq.). The mixture was stirred overnight at rt, diluted with water and extracted 3x with ethyl acetate. The combined organic layer was washed with brine, dried over MgSO₄, filtered and concentrated to a small volume to give a suspension. The suspension was diluted with ether and the solid was filtered, washed with ether and dried to give 485 mg **MCH1-3**.

### [Step 3] Synthesis of Benzyl 3-((di-tert-butoxycarbonyl)aminomethyl)-4-cyano-5-fluorophenylcarbamate (compound MCH1-5)

To a solution of **MCH1-3** (650 mg, 1.98 mmol), LiOH.H₂O (330 mg, 5.96 mmol, 3 eq.) in 10 mL THF and 2 mL water was stirred at rt for 45 min. and diluted with water. The solution was acidified with 1N HCl to -pH 1 and extracted 3x with ethyl acetate. The combined organic layer was washed with brine, dried over MgSO₄, filtered and evaporated to dryness to give 620g of the acid.

To a solution of the above acid (620 mg, 1.97 mmol) and triethyl amine (0.55 mL. 3.95 mmol, 2 eq.) in 10 mL THF at ∼-20 °C was added a I M solution of isopropylchlroformate in toluene (2.4 mL, 2.4 mmol, 1.2 eq.). The mixture was stirred for about 10 min and filtered through a fritted funnel and the precipitate rinsed with 10 mL THF. The filtrate was cooled to ∼-20°C and a solution of sodium borohydride (375 mmol, 9.91 mmol, 5 eq.) in 2 mL water was added. After being stirred for 10 min. at -20°C, the reaction mixture was diluted with water and extrated 3x with ethyl acetate. The combined organic layers was washed aq. NH₄Cl solution followed by brine, dried over MgSO₄, filtered and evaporated to dryness to provide the crude alcohol.

The above alcohol was dissolved in 10 mL of dichloromethane and cooled to 0°C. To this was added triethyl amine (0.55 mL, 3.95 mmol, 2 eq) followed by methane sulfonylchloride (0.185 mL, 2.39 mmol, 1.2 eq.). The mixture was stirred for 1 hr, diluted with ethyl acetate, washed with 1N HCl, 2x with aq. NaHCO₃ and brine. It was dried over MgSO₄, filtered and concentrated to provide the crude mesylate which was used for the subsequent step.

A solution of the above mesylate, di-*tert*-butyl iminodicarboxylate (560 mg, 2.58 mmol, 1.3 cq.), K₂CO₃ (540 mg, 3.97 mmol), and tetrabutylammonium iodide (973 mg, 0.198 mmol, 0.1 eq) in 10 mLDMF was stirred overnight at rt. The mixture was diluted with water, extracted 3x with ethyl acetate. The combined organic layer was washed with brine, dried over MgSO₄, filtered, concentrated and purified by chromatography eluting with 0% to 20% ethyl acetate in hexane to provide 49 mg of **MCH1-5**.

### [Step 4] Synthesis of 4-Amino-2-((di-tert-butoxycarbonyl)aminomethyl)-6-fluorobenzonitrile (compound MCH1-6)

A suspension of **5** (180 mg), 10% Pd-C (30 mg) in 2 mL THF and 2 mL ethanol was stirred under a hydrogen balloon for 6 hr, filtered through a CELITE pad, concentrated and purified by chromatography eluting with 100% hexane to 1:1:3 ethyl acetate/dichloromethane/hexane to provide 50 mg of **MCH1-6**.

### [Step 5] Synthesis of N-(7-fluoro-2,3-dihydro-1-imino-1H-isoindol-5-yl)-alpha(R)-hydroxy-3-oxo-4-[3-(3-o xo-4-morpholinyl)phenyl]-2(R)-morpholineacetamide (Example aa136)

Compound **MCH1-6** was converted to example aa136 using a procedure similar to the preparation of Example 57.

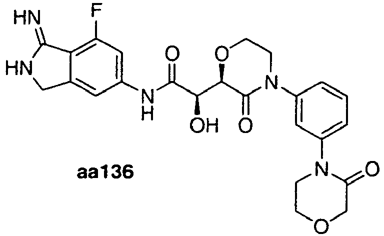

### EXAMPLE aa137

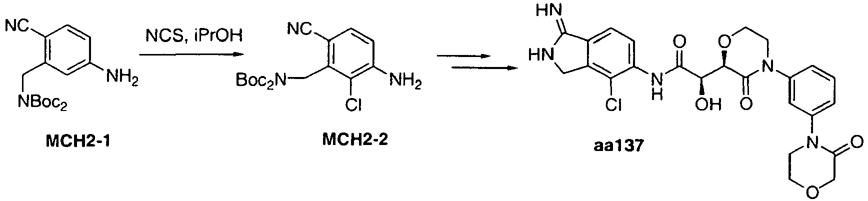

### [Step1] Synthesis of 4-Amino-2-((di-tert-butoxycarbonyl)aminomethyl)-3-chlorobenzonitrile (compound MCH 2-2)

To a solution of **MCH2-1** (950 mg, 2.73 mmol) in 10 ml isopropanol at 60°C was added N-chlorosuccinimide (400 mg, 2.99 mmol, 1.1 eq). The mixture was heated at reflux for 1.5 hr, left overnight at rt, concentrated and diluted with ethyl acetate. The solution was washed 2x with water, brine, dried over MgSO₄, filtered, concentrated and purified by flash chromatography using 40% ethyl acetate in hexanes to provide 288 mg of **MCH2-2.**

### [Step 2] Synthesis of N-(4-chloro-2,3-dihydro-1-imino-1H-isoindol-5-yl)-alpha(R)-hydroxy-3-oxo-4-[3-(3-o xo-4-morpholinyl)phenyl]-2(R)-morpholineacetamide (Example aa137)

Compound MCH2-2 was converted to example aa137 using procedure similar to the preparation of Example 57.

The following analogs were prepared using analogous procedure:

| **Example** | **Structure** | **Name** |
|---|---|---|
| **aa138** | 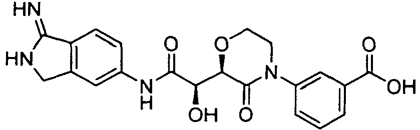 | 3-[2(R)-[2-[(2,3-Dih ydro-1-imino-1h-isoi ndol-5-yl)amino]-1( R)-hydroxy-2-oxoet hyl]-3-oxo-4-morph olinyl]benzoic acid |
| **aa139** | 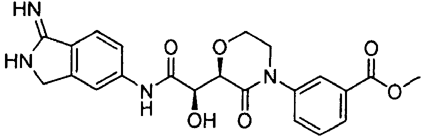 | Methyl 3-[2(R)-[2-[(2,3-dih ydro-1-imino-1H-iso indol-5-yl)amino[-1( R)-hydroxy-2-oxoet hyl]-3-oxo-4-morph olinyl]benzoate |
| **aa140** | 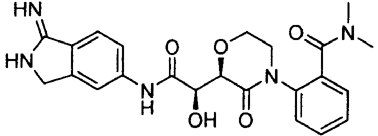 | N-(2,3-Dihydro-1-i mino-1h-isoindol-5-yl)-4-[2-[(dimethyla mino)carbonyl]phen yl]-alpha(R)-hydrox y-3-oxo-2(R)-morph olineacetamide |
| **aa141** | 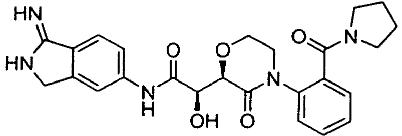 | N-(2,3-Dihydro-1-i mino-1H-isoindol-5-yl)-alpha(R)-hydrox y-3-oxo-4-[2-(1-pyrr olidinylcarbonyl)phe nyl]-2(R)-morpholin eacetamide |
| **aa142** | 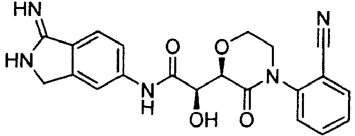 | 4-(2-Cyanophenyl)-N-(2,3-dihydro-1-im ino-1h-isoindol-5-yl) -alpha(R)-hydroxy-3 -oxo-2(R)-morpholi neacetamide |
| **aa143** | 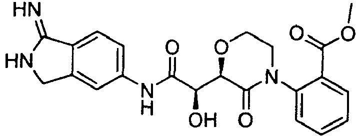 | Methyl 2-[2(R)-[2-[(2,3-dih ydro-1-imino-1h-isoi ndol-5-yl)amino]-1( R)-hydroxy-2-oxoet hyl]-3-oxo-4-morph olinyl]benzoate |
| **aa144** | 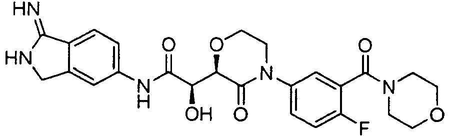 | N-(2,3-Dihydro-1-i mino-1h-isoindol-5-yl)-4-[4-fluoro-3-(4-morpholinylcarbonyl )phenyl]-alpha(R)-h ydroxy-3-oxo-2(R)-morpholineacetamid e |
| **aa145** | 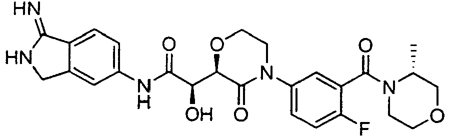 | N-(2,3-Dihydro-1-i mino-1h-isoindol-5-yl)-4-[4-fluoro-3-[(3 (R)-methyl-4-morph olinyl)carbonyl]phen yl]-alpha(R)-hydrox y-3-oxo-2(R)-morph olineacetamide |
| **aa146** | 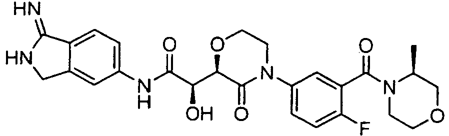 | N-(2,3-Dihydro-1-i mino-1h-isoindol-5-yl)-4-[4-fluoro-3-[(3 (S)-methyl-4-morph olinyl)carbonyl]phen yl]-alpha(R)-hydrox y-3-oxo-2(R)-morph olineacetamide |

### EXAMPLE aa147

### N-[4-(Aminoiminomethyl)phenyl]-4-(2-cyanophenyl)-alpha(R)-hydroxy-3-oxo-2(R)-m orpholineacetamide (Example aa147)

Example **aa147** was prepared using procedures similar to the preparation of Example 73.

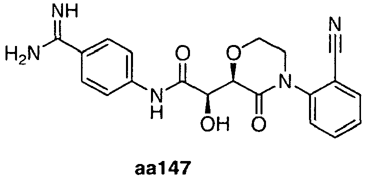

### EXAMPLE aa148

### N-(4-Amino-7-quinazolinyl)-4-[4-fluoro-3-(4-morpholinylcarbonyl)phenyl]-alpha(R)-h ydroxy-3-oxo-2(R)-morpholineacetamide (Example aa148)

Example **aa148** was prepared using procedures similar to the preparation of example 98.

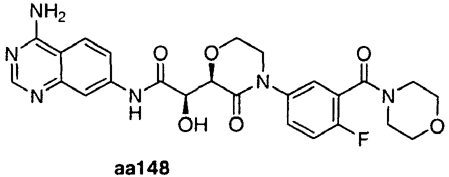

### EXAMPLE aa149

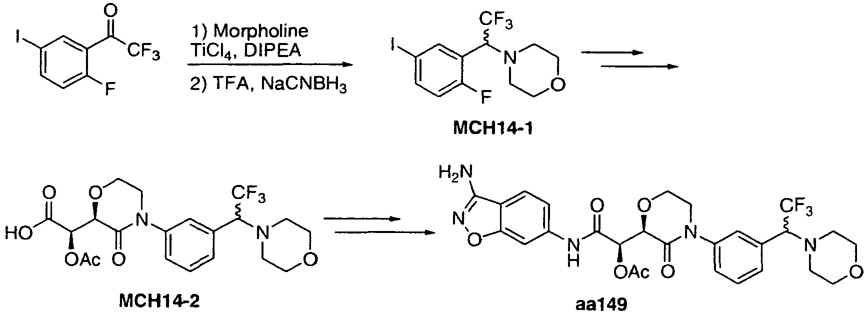

### [Step 1] Synthesis of 4-(2,2,2-trifluoro-1-(2-fluoro-5-iodophenyl)ethyl)morpholine (compound MCH14-1)

To a solution of commercially available 2,2,2-trifluoro-1-(2-fluoro-5-iodophenyl)ethanone (2 g, 6.29 mmol) in 20 mL dichloromethane at rt was added N,N-diisopropylethylamine (3.3 mL, 18.95 mmol, 3 eq.) followed by 1M solution of TiCl₄ in dichloromethane (6.3 mL, 6.3 mmol, I eq.). The mixture was stirred at rt for 3 days, added 10 mL of methanol followed by NaCNBH₃ (3.2 g) and trifluoroacetic acid (4.7 mL). The mixture was stirred overnight at at rt, poured into aq. NaHCO₃ and extracted 3x with dichloromethane. The combined organic layer was washed with brine, dried over MgSO₄, filtered, concentrated and purified by chromatography eluting with 0% to 20% ethyl acetate in hexane to provide 1.16 g of **MCH14-1**.

### [Step 2] Synthesis of N-(3-Amino-1,2-benzisoxazol-6-yl)-4-[4-fluoro-3-[2,2,2-trifluoro-1-(4-morpholinyl)eth yl]phenyl]-alpha(R)-hydroxy-3-oxo-2(R)-morpholineacetamide (example aa149)

Compound **MCH14-1** was converted to example **aa149** using experimental procedure similar to the one described for the preparation of **aa95**

The following examples were prepared using analogous procedure:

| EXAMPLE | Structure | Name |
|---|---|---|
| **aa150** | 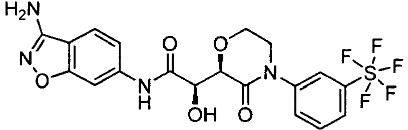 | [3-[2(R)-[2-[(3-Ami no-1,2-benzisoxazol-6-yl)amino]-1(R)-hy droxy-2-oxoethyl]-3 -oxo-4-morpholinyl] phenyl]pentafluoros ulfur |
| **aa151** | 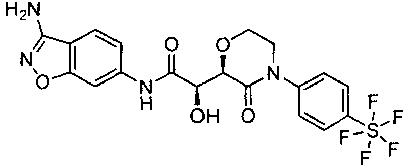 | [4-[2(R)-[2-[(3-Ami no-1.2-benzisoxazol-6-yl)amino]-1(R)-hy droxy-2-oxoethyl]-3 -oxo-4-morpholinyl] phenyl]pentafluoros ulfur |

### EXAMPLE aa152

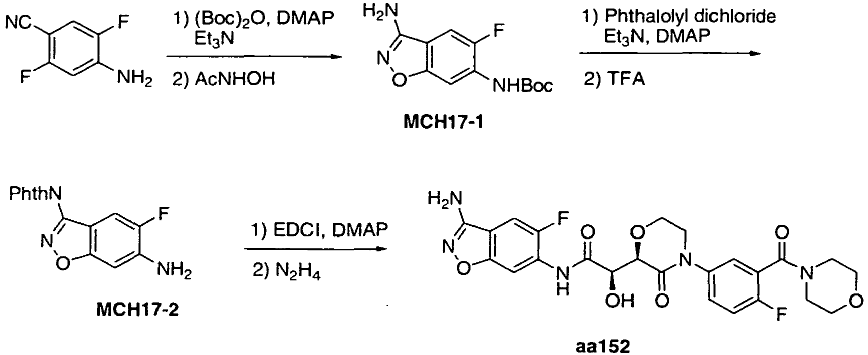

### [Step 1] Synthesis of tert-Butyl 3-amino-5-fluorobenzo[d]isoxazol-6-ylcarbamate (compound MCH17-1)

To a solution of 4-amino-2,5-difluorobenzonitrile (10 g, 64.88 mmol,) di-tert-butyldicarbonate (43 g, 197 mmol, 3 eq.) in dichloromethane at rt was added DMAP (1.6 g, 13.10 mmol, 0. 2eq.) and triethyl amine (28 mL, 200 mmol, 3 eq.). The mixture was stirred overnight at rt and poured into 1N HCl. The organic layer separated and the aqueous phase extracted twice with dichloromethane. The combined organic layer was washed with brined, dried over MgSO₄, filtered and evaporated to dryness to obtain 22.6 g of the protected aniline.

To a solution of the above aniline (12.6 g, 36 mmol) acetohydroxamic acid (16 g, 213 mmol, 6 eq.) and K₂CO₃ (58 g, 0.426 mmol, 12 eq.) in 150 mL of 9:1 DMF-water was heated overnight in an oil bath kept -60°C. It was poured into water, extracted 3x with ethyl acetate, the combined organic layer was washed with brine, dried over MgSO₄, filtered, concentrated and purified by chromatography eluting with 0% to 50% ethyl acetate in hexane to provide 5.76 g of **MCH17-1** contaminated with an unknown by product.

### [Step 2] Synthesis of 2-(6-Amino-5-fluorobenzo[d]isoxazol-3-yl)isoindoline-1,3-dione (compound MCH17-2)

To a solution of **MCH17-1** (520 mg), triethyl amine (0.82 mL) and DMAP (24 mg) in 10 mL dichloromethane at 0°C was added phthaloyl chloride (0.36 mL). The mixture was stirred overnight at rt, diluted with ethyl acetate and washed with 1N HCl, aq. NₐHCO₃ and brine. It was dried over anhydrous MgSO₄, filtered, concentrated and purified by chromatography eluting with 0% to 50% ethyl acetate in hexane to provide 338 mg of phthalimide protected intermediate which was stirred with 5 mL of trifluoroacetic acid at rt for 40 min. The reaction mixture was evaporated to dryness, the residue was suspended in aq. NaHCO₃ and extracted 3x with ethyl acetate. The combined organic layer was washed with brine, dried over MgSO₄, filtered and evaporated to dryness to obtain 220 mg **MCH 17-2**.

### [Step 3] Preparation of N-(3-Amino-5-fluoro-1,2-benzisoxazol-6-yl)-4-[4-fluoro-3-(4-morpholinylcarbonyl)ph enyl]-alpha(R)-hydroxy-3-oxo-2(R)-morpholineacetamide (example aa152)

Compound **MCH17-2** was converted to **aa152** using a procedure similar to the preparation of **aa149**

### EXAMPLE aa153

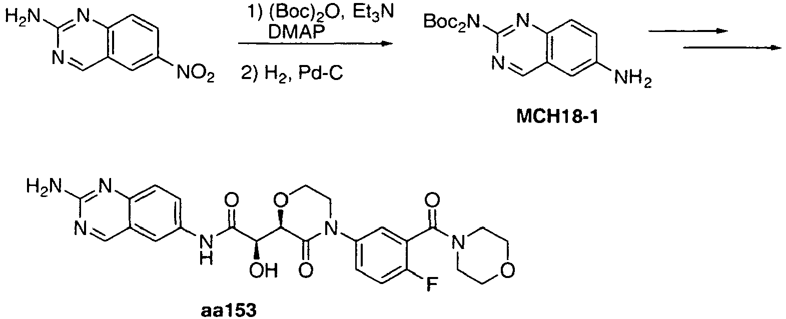

### [Step1] Preparation of (compound MCH18-1)

To 1.7g of 6-nitroquinazolin-2-amine (prepared according the procedure described in WO 2006/039718) in 45mL of dry dichloromethane at room temperature was added 6.2 mL of triethylamine, 5.85g of di-tert-butyldicarbonate, and 110 mg of DMAP and the mixture was stirred under nitrogen overnight. The reaction mixture was washed with water, aq. sodium bicarbonate and brine then dried with magnesium sulfate, filtered and evaporated to dryness. Purification by flash chromatography yielded 3.04g of the protected aniline.

To 0.5g of the above product in 20mL, of methanol was added 50mg of 10% palladium on carbon and the mixture was shaken under 40 psi of hydrogen for 45 minutes. The mixture was filtered and evaporated to dryness. Recrystallization from ethyl acetate/hexanes yielded 220mg of **MCH18-1**.

### [Step 2] Synthesis of N-(2-Amino-6-quinazolinyl)-4-[4-fluoro-3-(4-morpholinylcarbonyl)phenyl]-alpha(R)-h ydroxy-3-oxo-2(R)-morpholineacetamide (example aa153).

Compound MCH18-1 was converted to **aa153** using a procedure similar to the preparation of Example 98.
**Analytical Data:**
Mass Spectra:

| EXAMPLE | **LCMS m/e** |
|---|---|
| **MCH1-3** | 351.2 (MH⁺) |
| **aa136** | 498.3 (MH⁺) |
| **MCH2-2** | 282.2 (MH-Boc⁺) |
| **aa137** | 514.0 (MH⁺) |
| **aa138** | 425.2 (MH⁺) |
| **aa39** | 438.8 (MH⁺) |
| **MCH1-5** | 522.2 (MNa⁺) |
| **MCH1-6** | 388.2 (MNa⁺) |
| **aa140** | 452.2 (MH⁺) |
| **aa141** | 478.3 (MH⁺) |
| **aa142** | 406.2 (MH⁺) |
| **aa143** | 439.2 (MH⁺) |
| **aa144** | 512.3 (MH⁺) |
| **aa145** | 526.3 (MH⁺) |
| **aa146** | 526.3 (MH⁺) |
| **aa147** | 394.2 (MH⁺) |
| **aa148** | 525.2 (MH⁺) |
| **MCH14-1** | MS: 390 (MH⁺) |
| **aa149** | 568.0 (MH⁺) |
| **aa150** | 509.0 (MH⁺) |
| **aa151** | 509.0 (MH⁺) |
| **MCH17-1** | 268.2 (MH⁺) |
| **MCH17-2** | 298.2 (MH⁺) |
| **aa152** | 532.2 (MH⁺) |
| **aa153** | 525.3 (MH⁺) |

| EXAMPLE | **NMR (400 MHz, ppm)** |
|---|---|
| **aa136** | HCl salt in CD₃OD: 7.6 (s, 1H), 7.87 (d, J = 12.1 Hz, 1H), 7.54-7.47 (m, 2H), 7.38-7.34 (m, 2H), 4.84-4.80, m, 4H), 4.28 (s, 2H), 4.24-4.20 (m, 1H), 4.06-4.01 (m, 4H), 3.82-3.80 (m, 2H), 3.73-3.67 (m, 1H) |
| **aa137** | HCl salt in CD₃OD : 8.77 (dd, J = 8.6, 3.3 Hz, 1H), 8.07 (d, J = 8.6 Hz, |
| | 1H), 7.45 - 7.55 (m, 2H), 7.33 - 7.41 (m, 2H), 4.87 (br. s., 4H), 4.29 (s, 2H), 4.14 - 4.25 (m, 1H), 4.01 - 4.08 (m, 4H), 3.79 - 3.84 (m, 1H), 3.65 (s, 4H) |
| **aa138** | HCl salt in DMSO-d₆: 10.25 (s, 1H), 10.19 (s, 1H), 9.6, (s, 1H), 9.13 (s, 1H), 8.31 (s, 1H), 8.16 (d, J = 8.5 Hz, 1H), 7.97 (s, 1H), 7.94 (d, J = 10.3 Hz, 1H), 7.84 (d, J = 7.7 Hz, 1H), 7.64 (d, J = 6.6 Hz, 1H), 7.55 (t, J = 7.5 Hz, 2H), 6.53 (br, 1H), 4.77 (s, 2H), 4.70-4.67 (m, 2H), 4.14-4.11 (m, 1H), 3.98-3.84 (m, 2H), 3.70-3.67 (m, 2H). |
| **aa139** | HCl salt in CD₃OD: 8.26 (d, J = 1.1 Hz, 1H), 8.06-8.04 (m, 1H), 7.99-7.96 (m, 1H), 7.88 (dd, J = 8.5, 1.8 Hz, 1H), 7.68-7.65 (m, 1H), 7.57 (t, J = 7.7 Hz, 2H), 4.85-4.83 (m, 2H), 4.81 (s, 2H), 4.26-4.20 (m, 1H), 4.08-4.00 (m, 2H), 3.92 (s, 3H), 3.73-3.68 (m, 1H) |
| **aa140** | HCl salt in CD₃OD: 8.25 (s. 1H), 8.05 (d, J = 8.4 Hz, 1H), 7.88 (d, J = 8.6 Hz, 1H), 7.55 (dd, J = 7.6, 1.4 Hz, 1H), 7.37 - 7.50 (m, 3H), 4.71 - 4.84 (m. 4H), 4.17 (d, J = 11.2 Hz, 1H), 3.91 - 4.08 (m. 2H). 3.58 (d, J = 11.7 Hz, 1H), 3.09 (s, 3H). 2.95 (s, 3H) |
| **aa141** | HCl salt in CD₃OD: 8.25 (s, 1H), 8.05 (d, J = 8.6 Hz, 1H), 7.87 (d, J = 8.6 Hz. 1H), 7.53 - 7.60 (m, 1H), 7.39 - 7.50 (m, 3H), 4.70 - 4.83 (m. 4H), 4.17 (d, J = 11.2 Hz, 1H), 3.91 - 4.11 (m, 2H), 3.37 - 3.66 (m, 5H), 1.80 - 2.03 (m, 4H) |
| **aa142** | HCl salt in CD₃OD: 8.98 - 9.48 (m, 1H), 8.41 (dd, J = 7.9, 1.1 Hz, 1H), 8.26 (s, 1H), 7.95 - 8.15 (m, 3H), 7.90 (dd, J = 8.6, 1.5 Hz. 1H), 7.83 (t, J = 7.6 Hz, 1H), 5.51 (d, J = 2.0 Hz, 1H), 5.05 (d. J = 2.4 Hz, 1H), 4.79 - 4.84 (m, 2H), 4.53 - 4.62 (m, 1H), 4.41 - 4.51 (m, 2H), 4.13 - 4.24 (m, 1H) |
| **aa143** | HCl salt in CD₃OD: 8.26 (s. I H), 7.99 - 8.08 (m, 2H), 7.88 (dd, J = 8.6, 1.7 Hz, 1H), 7.66 - 7.73 (m, 1H), 7.48 - 7.54 (m, 1H), 7.40 (d, J = 7.9 Hz, 1H), 4.74 - 4.83 (m, 4H), 4.20 - 4.28 (m, 1H), 4.02 - 4.02 (m, 0H), 3.99 - 4.15 (m, 2H), 3.90 (s, 3H), 3.52 - 3.59 (m, 1H) |
| **aa144** | HCl salt in CD₃OD: 8.25 (s, 1H), 8.05 (d, J = 8.6 Hz, 1H), 7.88 (d, J = 8.6 Hz, 1H), 7.46 - 7.58 (m, 2H), 7.30 (t, J = 8.9 Hz, 1H), 4.79 - 4.84 (m. 4H), 4.16 - 4.26 (m, 1H), 3.95 - 4.08 (m, 2H), 3.76 (d, J = 5.9 Hz, 4H), 3.60 - 3.70 (m, 3H), 3.41 (br. s., 2H) |
| **aa145** | HCl salt in DMSO-d₆: 9.77 (s, 1H), 9.22 (s, 1H), 8.31 (s, 1H), 8.23 (d, J = 8.6 Hz, 1H), 7.91 (dd, J = 8.7, 1.2 Hz, 1H), 7.30 - 7.58 (m, 3H), 4.76 (s, 2H), 4.68 (br. s., 2H), 4.05 - 4.21 (m, 1H), 3.80 - 3.99 (m, 2H), 3.61 - 3.78 (m, 3H), 3.27 - 3.55 (m, 4H), 3.02 - 3.19 (m, 1H), 1.14 - 1.30 (m, 3H) |
| **aa146** | HCl salt in DMSO-d₆: 9.78 (br. s., 1H), 9.23 (s, 1H), 8.31 (s, 1H), 8.23 (d, J = 8.4 Hz, 1H), 7.85 - 7.96 (m, 1H), 7.13 - 7.58 (m, 3H), 4.76 (s, 2H), 4.67 (d, J = 5.7 Hz, 2H), 4.06 - 4.20 (m, 1H), 3.79 - 3.98 (m, 2H), 3.60 - 3.78 (m, 3H), 3.23 - 3.59 (m, 4H), 3.09 (t, J = 12.6 Hz, 1H), 1.05 - 1.29 (m, 3H) |
| **aa147** | HCl salt in CD₃OD: 9.19 (br. s, 2H), 8.68 (br. s., 2H), 7.92 - 8.00 (m, 2H), 7.75 - 7.90 (m, 4H), 7.53 - 7.62 (m, 2H), 4.75 - 4.87 (m, J =11.6, 1.9 Hz, 1H), 4.25 - 4.33 (m, 1H), 3.97 - 4.14 (m, 3H), 3.56 - 3.68 (m, 1H) |
| **aa148** | HCl salt in DMSO-d₆: 10.56 (s, 1H), 9.66 (s, 1H), 9.61 (s, 1H), 8.76 (s, 1H), 8.56 (d, J = 1.8 Hz, 1H), 8.35 (dd, J = 9.2, 1.8 Hz, 1H), 7.56-7.49 (m, 2H), 7.56-7.49 (m, 2H), 7.38 (t, J = 8.9 Hz, 2H), 4.75 (s, 2H), 4.15-4.11 (m, 1H), 4.00-3.83 (m. 2H), 3.78-3.43 (m), 3.25 (br, 2H). |
| **aa149** | CDCl₃: 8.96 (s, 1H), 8.0 (s, 1H), 7.47 (d, J = 6.3 Hz, 1H), 7.40 (d. J = 8.4 Hz, 1H), 7.36-7.32 (m. 1H), 7.26-7.23 (m, 1H), 7.16 (m, J = 8.9 Hz, 2H), 4.95-4.90 (m, 1H), 4.72 (d, J = 7.4 Hz, 1H), 4.53 (s, 2H), 4.50-4.42 (m, 1H), 4.25 (d, J = 9.5 Hz, 1H), 4.14-4.02 (m, 2H), 3.7-3.62 (m, 4H), 3.55 (d, J = 10.3 Hz. I H), 2.66-2.51 (m, 4H) |
| **aa150** | DMSOd₆: 9.98 (s, 1H), 8.04-8.02 (m, 2H), 7.82-7.80 (m, 1H), 7.72-7.66 (m, 3H), 7.57 (dd, J = 8.8, 1.5 Hz, 1H), 4.73 (d, J = 1.8 Hz, 1H), 4.65 (d, J = 1.8 Hz, 1H), 4.16-4.10 (m, 1H), 3.99-3.90 (m, 2H), 3.78-3.70 (m, 1H). |
| **aa151** | DMSOd₆: 9.98 (s, 1H), 8.01 (s, 1H), 7.96 (d, J = 9.2 Hz, 2H), 7.71-7.18 (m, 3H), 7.57 (d, J = 7.7 Hz, 1H), 4.73 (d, J = 1.8 Hz, 1H), 4.65 (d, J = 1.8 Hz, 1H), 4.17-4.11 (m, I H), 3.98-3.89 (m, 2H), 3.74 (m, 1H), |
| **aa152** | CD₃OD: 8.39 (d, J = 8.4 Hz, 1H), 7.57 (d, J = 7.7 Hz, 1H), 7.56-7.52 (m, 1H), 7.50 (dd, J = 5.9, 2.6 Hz, I H), 7.30 (t, J = 8.9 Hz, 1H), 4.86 (d, J = 1.8 Hz, 1H), 4.83 (d, J = 1.81 Hz, 1H), 4.24-4.18 (m, 1H), 4.07-3.99 (m, 2H), 3.78-3.75 (m, 4H), 3.65-3.63 (m, 2H), 3.41-3.40 (m, 2H). |
| **aa153** | DMSOd₆: 10.26 (s, 1H), 9.54 (s, 1H), 8.67 (br. s., 1H), 8.23 (d, J = 8.4 Hz, 1H), 7.68 (d, J = 8.8 Hz, 1H), 7.51 (d, J = 15.4 Hz, 2H), 7.37 (t, J = 8.8 Hz, 1H), 4.68 (d, J = 8.1 Hz, 2H), 4.13 (d, J = 10.6 Hz, 1H), 3.80 - 3.97 (m, 2H), 3.35 - 3.75 (m, 7H), 3.25 (br. s., 2H) |

### Examples aa 154 - aa159

### EXAMPLE aa154

### (R)-2-((R)-4-(5-fluoro-2-isopropoxyphenyl)-3-oxomorpholin-2-yl)-2-hydroxy-N-(1-imi noisoindolin-5-yl)acetamide

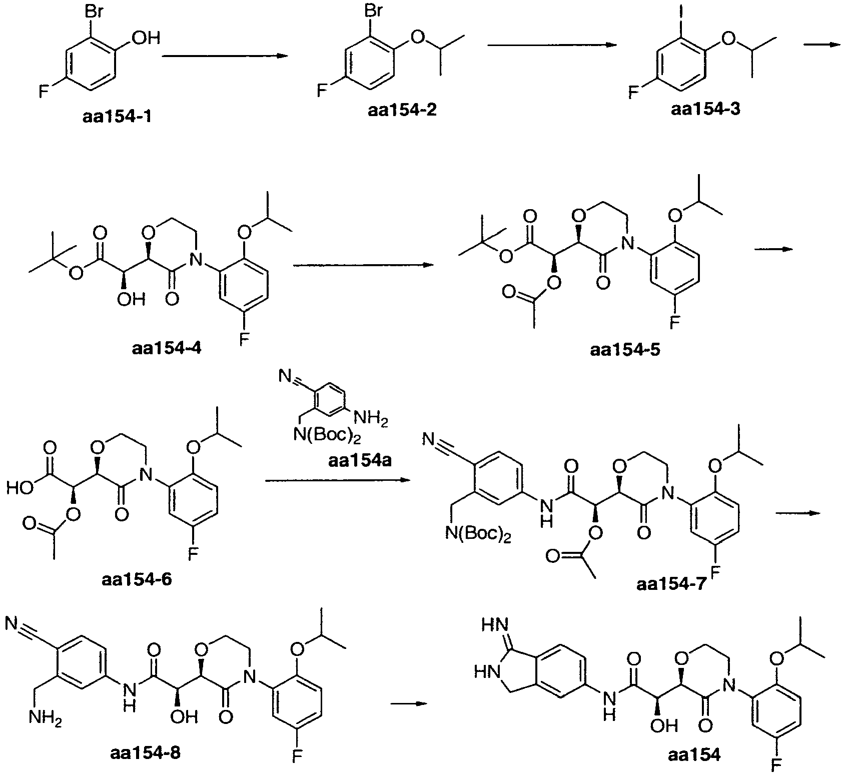

### [Step 1]

To a mixture of 2-bromo-4-fluorophenol (1.3 g, 7.0 mmol) and potassium carbonate (2.0 g, 14 mmol) in DMF (15 mL), 2-iodopropane (1.8 g, 10.5 mmol) was added. The mixture was heated to 55°C and stirred for 16 h. The reaction mixture was cooled to room temperature and diluted with diethyl ether. The organics were washed with saturated aqueous ammonium chloride, water, and dried with Na₂SO₄. The organics were evaporated to dryness to give Compound **aa154-2** (1.4 g, 86%) which was used without further purification in the next step.

### [Step 2]

To a nitrogen purged vessel, a solution of compound **aa154-2** (1.4 g, 6.0 mmol) in 1,4-dioxane (6 mL), sodium iodide (1.8 g, 12.0 mmol), copper iodide (0.057 g, 0.3 mmol), and *trans-N,N*'-dimethylcyclohexane-1,2-diamine (0.085 g, 0.6 mmol) were added. The vessel was sealed and heated to 110 °C for 16 h. The reaction mixture was cooled to room temperature, washed with saturated aqueous ammonium chloride, and extracted with ethyl acetate. The organics were dried with Na₂SO₄ and evaporated to dryness to give Compound **aa154-3** (1.4 g, 83%) which was used without further purification in the next step.

### [Step 3]

To a nitrogen purged vessel, a solution of compound **aa154-3** (1.4 g, 5.0 mmol) in DMSO (40 mL), (*R*)-tert-butyl 2-hydroxy-2-((*R*)-3-oxomorpholin-2-yl)acetate (0.96 g, 4.1 mmol), potassium phosphate (1.75 g, 8.2 mmol), copper iodide (0.157 g, 0.82 mmol), and *trans*-*N,N'*-dimethylcyclohexane-1,2-diamine (0.116 g, 0.82 mmol) were added. The vessel was sealed and heated to 85 °C for 3 h. The reaction mixture was cooled to room temperature, washed with saturated aqueous ammonium chloride, and extracted with ethyl acetate. The organics were dried with Na₂SO₄ and evaporated to dryness. The crude material was purified by silica gel chromatography (ethyl acetate/Hexanes 0 to 45%) to give Compound **aa154-4** (1.2 g, 76%).

### [Step 4]

To a solution of compound **aa154-4** (1.2 g, 3.1 mmol) in DCM (30 mL) that was chilled to 0 °C was added DMAP (0.038 g, 0.31 mmol), pyridine (0.49 g, 6.2 mmol), and acetic anhydride (0.64 g, 6.2 mmol). The reaction was stirred at 0 °C for 3 h. The reaction was diluted with ethyl acetate, washed with aqueous Cu₂SO₄ and H₂O (X3). The organics were dried with Na₂SO₄ and evaporated to dryness. The crude material was purified by silica gel chromatography (ethyl acetate/Hexanes 0 to 45%) to give Compound **aa154-5** (0.9 g, 68%).

### [Step 5]

Compound **aa154-5** was dissolved in DCM (20 mL), TFA (10 mL) and H₂O (0.1mL). The reaction was stirred at room temperature for 2 h. The reaction was evaporated to dryness. The residue was dissolved in 50 mL of toluene and evaporated to dryness (X3). The residue was dissolved in 50 mL of hexanes and evaporated to dryness (X2) to give Compound **aa154-6** (0.8 g, 100%) which was used without further purification in the next step.

### [Step 6]

According to the step 70-5 in the synthetic method for EXAMPLE 70, compound **aa154-6** (0.6 g, 1.6 mmol) was used instead of compound **70-4** to couple to compound **aa154a** (0.55 g, 1.6 mmol) instead of compound **68-12** to obtain compound **aa154-7** (0.16 g, 14%) after chromatography purification on silica gel eluting with DCM/Ethyl Acetate (90/10).

### [Step 7]

According to the step 70-6 in the synthetic method for EXAMPLE 70, compound **aa154-7** (0.16 g, 0.23 mmol) was used instead of compound **70-5.** The crude reaction mixture was evaporated to dryness and dissolved in 4N HCl in dioxane (3 mL). The reaction was stirred at ambient temperature for two h. The reaction mixture was evaporated to dryness to give compound **aa154-8** (0.1g. 95%) which was used without further purification in the next step.

### [Step 8]

Compound **aa154-8** was dissolved in ethanol (5 mL) and heated to 85 °C for 16 h. The reaction was cooled to ambient temperature, evaporated to dryness, and purified by HPLC. The resulting fraction was purified by silica gel chromatography and eluted with DCM/(7N NH₃ in MeOH) (90/10) to obtain compound **aa154** ((R)-2-((R)-4-(5-fluoro-2-isopropoxyphenyl)-3-oxomorpholin-2-yl)-2-hydroxy-N-(1-i minoisoindolin-5-yl)acetamide) (0.002 g, 2%).

### EXAMPLE aa155

### (R)-2-hydroxy-N-(1-iminoisoindolin-5-yl)-2-((R)-3-oxo-4-(2-(trifluoromethyl)phenyl) morpholin-2-yl)acetamide

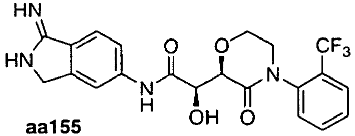

Compound was obtained using the synthetic method for EXAMPLE **aa154** beginning from Step 3 using 1-iodo-2-(trifluoromethyl)benzene instead of compound **aa154-3**.

### EXAMPLE aa156

### (R)-2-((R)-4-(2-(difluoromethoxy)phenyl)-3-oxomorpholin-2-yl)-2-hydroxy-N-(1-imin oisoindolin-5-yl)acetamide

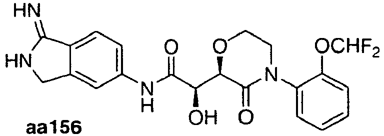

Compound was obtained using the synthetic method for EXAMPLE **aa154** beginning from Step 3 using 1-(difluoromethoxy)-2-iodobenzene instead of compound **aa154-3**.

### EXAMPLE aa157

### (R)-2-hydroxy-N-(1-iminoisoindolin-5-yl)-2-((R)-4-(2-isopropoxyphenyl)-3-oxomorph olin-2-yl)acetamide

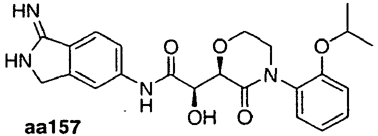

Compound was obtained using the synthetic method for EXAMPLE **aa154** using with 2-iodophenol instead of 2-bromo-4-fluorophenol in Step 1. Step 2 was skipped.

### EXAMPLE aa158

### (R)-2-((R)-4-(5-chloro-2-isopropoxyphenyl)-3-oxomorpholin-2-yl)-2-hydroxy-N-(1-im inoisoindolin-5-yl)acetamide

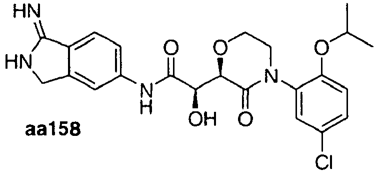

Compound was obtained using the synthetic method for EXAMPLE **aa154** using 2-bromo-4-chlorophenol instead of 2-bromo-4-fluorophenol in Step 1.

### EXAMPLE aa159

### (R)-2-hydroxy-N-(1-iminoisoindolin-5-yl)-2-((R)-3-oxo-4-(2-(trifluoromethoxy)phenyl )morpholin-2-yl)acetamide

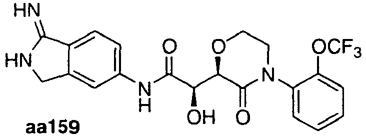

Compound was obtained using the synthetic method for EXAMPLE **aa154** beginning from Step 3 using 1-iodo-2-(trifluoromethoxy)benzene instead of compound **aa154-3**.

| EXAMPLE | LCMS (MH⁺) |
|---|---|
| **aa155** | 449.2 |
| **aa156** | 447.2 |
| **aa154** | 457.2 |
| **aa157** | 439.2 |
| **aa158** | 473.2 |
| **aa159** | 465.2 |

| EXAMPLE | ¹H NMR (400 MHz) |
|---|---|
| **aa155** | (CD₃OD) δ 10.04 (s, 1H), 8.24 (s, 1H), 8.04 (d, J = 8.6 Hz, 1H), 7.70 - 7.94 (m, 3H), 7.62 (t, J = 6.8 Hz, 1H), 7.43 - 7.55 (m, 1H), 7.00 - 7.27 (m, 1H), 4.77 - 4.84 (m, 4H), 4.32 (dt, J = 11.7, 3.8 Hz, 1H), 4.15 - 4.26 (m, 1H), 3.87 - 4.11 (m, 2H), 3.59 (d, J = 12.1 Hz, 1H),3.42 - 3.52 (m, 1H). |
| **aa156** | (CD₃OD) δ 8.25 (s, 1H), 8.05 (d, J = 8.6 Hz, 1H), 7.88 (dd, J = 8.6, 1.7 Hz, 1H), 7.39 - 7.52 (m, 2H), 7.34 (t, J = 8.3 Hz, 2H), 6.79 (t, J = 73.5 Hz, 1H), 4.73 - 4.85 (m, 3H), 4.18 - 4.29 (m, 1H), 3.98 (quin, J = 10.8 Hz, 2H), 3.45 - 3.60 (m, 1H). |
| **aa154** | (CD₃OD) δ 9.42 (br. s., 1H), 8.99 (br. s., 1H), 8.26 (s, 1H), 8.05 (d, J = 8.4 Hz, 1H), 7.88 (d, J = 8.6 Hz, 1H), 6.95 - 7.23 (m, 3H), 4.73 - 4.85 (m, 3H), 4.49 - 4.67 (m, 1H), 4.21 (d, J = 11.6 Hz, 1H), 4.10 - 3.80 (m, 2H), 3.40 - 3.62 (m, 2H), 1.33 (t, J = 5.0 Hz, 4H),1.17 (t, J = 7.0 Hz, 2H). |
| **aa157** | (CD₃OD) δ 9.88 - 10.08 (m, 1H), 9.34 - 9.52 (m, 1H), 8.99 (br. s, 1H), 8.26 (s, 2H), 8.05 (d, J = 8.4 Hz, 2H), 7.88 (dd, J = 8.6, 1.3 Hz, 2H), 7.33 (t, J = 8.1 Hz, 1H), 7.24 (dd, J = 7.7, 1.3 Hz, 2H), 7.12 (d, J = 8.1 Hz, 3H), 6.98 (t, J = 7.5 Hz, 1H), 4.76 - 4.85 (m, 3H), 4.67(quin, J = 6.1 Hz, 1H), 4.22 (d, J = 11.4 Hz, 1H), 3.92 - 4.08 (m, 2H), 1.34 (dd, J = 5.9, 2.2 Hz, 6H). |
| **aa158** | (CD₃OD) δ 10.02 (s, 1H), 9.42 (br. s., 1H), 8.99 (br. s., 1H), 8.25 (s, 1H), 8.04 (d, J = 8.6 Hz, I H), 7.88 (d, J = 8.4 Hz, 1H), 7.21 - 7.46 (m, 2H), 7.12 (d, J = 8.8 Hz, 1H), 4.76 - 4.84 (m, 3H), 4.57 - 4.72 (m, 1H), 4.15 - 4.27 (m, 1H), 3.93 - 4.06 (m, 1H), 3.43 - 3.56 (m, 1H), 1.34 (dd, J = 6.0, 2.5 Hz, 6H). |
| **aa159** | (CD₃OD) δ 8.25 (s, 1H), 8.05 (d, J = 8.6 Hz, 1H), 7.89 (dd, J = 8.6, 1.7 Hz, 1H), 7.38 - 7.57 (m, 4H), 4.77 - 4.84 (m, 3H), 4.18 - 4.31 (m, 1H), 3.90 - 4.10 (m, 2H), 3.57 (d, J = 8.6 Hz, 1H). |

### EXAMPLES aa160 - aa163

### EXAMPLE aa160

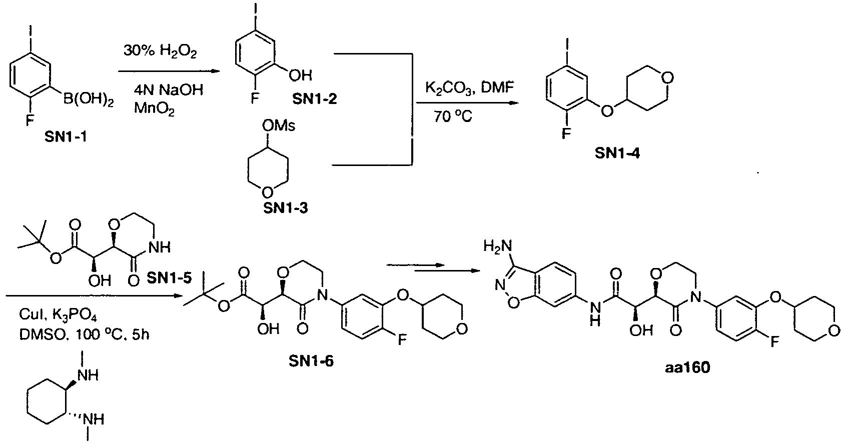

### [Step 1-2]

### Synthesis of SN1-2:

To a cooled solution of 2-fluoro-5-iodophenylboronic acid (5.0g, 18.81 mmol) in THF (200 mL) at 0 °C was added an aqueous solution of 30% H₂O₂ (1.73 mL, 18.0 mmol) dropwise and stirred for 10 min. This was followed by the addition of an aqueous solution of 4N NaOH (0.3 mL, 1.2 mmol). The reaction was then warmed to rt and stirred overnight. MnO₂ (40.0 mg) was then added to the reaction mixture and stirred for 90 min. The reaction mixture was then filtered and concentrated and partitioned between diethyl ether (150 mL) and water (150 mL). Separated the organics and washed further with brine and dried. The product was purified by eluting with silica-gel from 100:0 to 70:30 hexanes to ethyl acetate to give the product **SN1-2**(3.4g, 76%).

### [Step 1-4]

### Synthesis of SN1-4:

To a sealed tube was added **SN1-2** (218 mg, 0.92 mmol), commercially available tetrahydro-2H-pyran-4-yl methanesulfonate (0.2g, 1.1 mmol), potassium carbonate (0.384g, 2.78 mmol) in DMF (4.0 mL) and heated at 70 °C overnight. The reaction mixture was then cooled and extracted with ethyl acetate. The organics were washed multiple times with water and brine and this was followed by washing with 1M NaOH. The organic fractions were concentrated and purified over column chromatography using 25% acetone/hexanes to give product **SN1-4**(120 mg, 41 %). The yield of this reaction was greatly improved by heating at 100 °C for a similar substrate.

### [Step 1-6]

### Synthesis of SN1-6:

A mixture of aryl iodide **SN1-4**(0.120g, 0.37 mmol), previously described **SN1-5** (0.064g, 0.28 mmol), CuI (11mg, 0.056 mmol), K₃PO₄ (119 mg, 0.56 mmol) and trans-*N*,*N'*-dimethylcyclohexane-1,2-diamine (9 µL, 0.056 mmol) were stirred in DMSO (4.0 mL). The degassed reaction mixture was then heated at 100 °C for 5.0h. The reaction was then cooled, quenched with water, extracted with EtOAc and washed with brine. The organic fractions were concentrated and purified with 50-60% EtOAc/hexanes to give the product **SN1-6** (0.084 mg, 71%).

### Synthesis of aa160: N-(3-Amino-1,2-benzisoxazol-6-yl)-4-[4-fluoro-3-[(tetrahydro-2H-pyran-4-yl)oxy]phe nyl]-alpha(R)-hydroxy-3-oxo-2(R)-morphol ineacetamide

The iodide **SN1-6** was converted to **aa160** using experimental procedure described for the preparation of **aa95.**

### EXAMPLE aa161

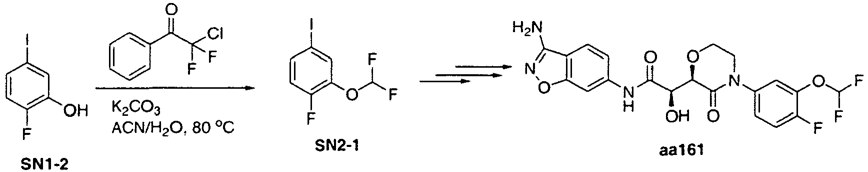

### [Step 2-1]

### Synthesis of 2-1:

A mixture of aryl iodide **SN1-2** (1.0g, 4.2 mmol), potassium carbonate (21.0g, 151.9 mmol), 2-chloro-2,2-difluoro-1-phenylethanone (3.9g, 20.5 mmol) were stirred in acetonitrile/water (1:1, 50 mL) and heated in a sealed tube at 85 °C for 5.0h. The reaction mixture was cooled and the organics were extracted thrice with ether. The ether fractions were washed with 1M NaOH and concentrated. The product was purified by column chromatography with 10-20% EtOAc/hexanes to give the product **SN2-1**(0.5g, 42%).

### Synthesis of aa161: N-(3-Amino-1.2-benzisoxazol-6-yl)-4-[3-(difluoromethoxy)-4-fluorophenyl]-alpha(R)-hydroxy-3-oxo-2(R)-morpholineacetamide

The iodide **SN2-1** was converted to **aa161** using experimental procedure similar to the preparation of **aa95**.

### EXAMPLE aa 162

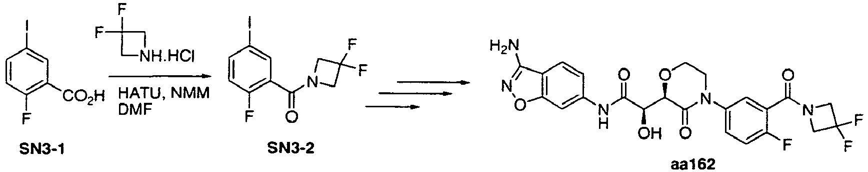

### [Step 3-2]

### Synthesis of SN3-2:

To commercially available **SN3-1** (0.5g, 1.88 mmol) was added 3,3-difluoroazetidine hydrochloride (0.244g, 1.88 mmol), HATU (1.17g, 3.1 mmol), NMM (1.2 mL, 11 mmol) in DMF (4.0 mL) and stirred at 0 °C. The reaction was quenched with NH₄Cl and extracted with ethyl acetate. The organics were washed with brine, concentrated and purified by column chromatography using 40% EtOAc/Hexanes to give the product SN3-2 (0.45g, 70%).

### Synthesis of aa162: N-(3-Amino-1,2-benzisoxazol-6-yl)-4-[3-[(3,3-difluoro-1-azetidinyl)carbonyl]-4-fluoro phenyl]-alpha(R)-hydroxy-3-oxo-2(R)-morpholineacetamide

The iodide **SN3-2** was converted to **aa162** using experimental procedure described for the preparation of **aa95.**

### EXAMPLE aa163:

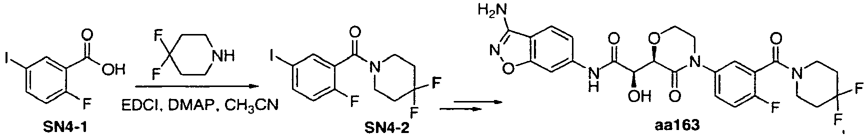

### [Step 4-2]

### Synthesis of SN4-2:

2-fluoro-5-iodobenzoic acid (1.1g, 4.14 mmol) was dissolved in CH₃CN (20 mL) and cooled to 0°C. 4,4-Difluoropiperidine (0.6g, 4.96 mmol) was added to the mixture followed by EDCI (0.95g, 4.96 mmol) and DMAP (0.05g, 0.41 mmol) and the resulting mixture was stirred overnight at room temperature. Reaction mixture was diluted with ethyl acetate and washed with saturated NH₄Cl, water and brine respectively. Organic layer was dried over anhydrous MgSO₄, filtered, concentrated, purified by silica gel column chromatography using (0-70)% ethyl acetate - hexanes as mobile phase and the product **SN1-1** (1.15g, 75.65%) was obtained.

### Synthesis of aa163: N-(3-Amino-1,2-benzisoxazol-6-yl)-4-[3-[(4,4-difluoro-1-piperidinyl)carbonyl]-4-fluor ophenyl]-alpha(R)-hydroxy-3-oxo-2(R)-morpholineacetamide

The iodide **SN4-2** was converted to **aa163** using experimental procedure similar to the preparation of **aa95.**
Analytical Data:
Mass Spectra:

| EXAMPLE | LCMS m/e |
|---|---|
| **aa160** | 501 (M+H)⁺ |
| **aa161** | 467 (M+H)⁺ |
| **aa162** | 520 (M+H)⁺ |
| **aa163** | 548 (M+H)⁺ |

| EXAMPLE | NMR (400 MHz, ppm) |
|---|---|
| **aa160** | CDCl₃: 9.06 (s, 1H), 7.97 (s, 1H), 7.38 (d, 1H, *J* = 8.4 Hz), 7.22 (d, 1H, J = 8.4 Hz), 7.10-7.05 (m, 1H), 7.00-6.98 (m, 1H), 6.86-6.82 (m, 1H), 5.09 (br s, 1H), 4.94 (br d. 1H, J = 5.5 Hz), 4.89 (br s, 1H), 4.62 (s, 2H), 4.40-4.36 (m, 1H), 4.22-4.19 (m, 1H), 3.52-3.42 (m, 5H), 1.98-1.87 (m, 2H), 1.80-1.70 (m, 4H). |
| **aa161** | DMSO-d₆: 9.97 (s, 1H), 8.01 (d, I H, *J* = 1.1Hz), 7.68(d, 1H, *J* = 8.4 Hz), 7.56 (dd, 1H, *J* = 8.4, 1.5 Hz), 7.51-7.43 (m, 2H), 7.36-7.32(m, 1H), 7.25 (s, 1H), 6.43(d. 1H, *J* = 6.97 Hz), 6.32(s, 2H), 4.68(d, 1H, *J* = 1.8 Hz), 4.63 (dd, 1H, *J* = 6.6, 1.8 Hz), 4.14-4.10(m, 1H, J=1.0 Hz), 3.95-3.82(m, 2H), 3.66-3.62(m, 1H). |
| **aa162** | CDCl₃: 8.86 (s, 1H), 8.01 (d, 1H, *J* = 1.5 Hz), 7.65-7.63 (m, 1H); 7.51-7.47 (m, 1H), 7.45 (d, 1H, *J* = 8.4 Hz), 7.30 (dd, 1H, *J* = 8.8, 1.5 Hz), 7.18(t, 1H, *J* = 9.2Hz), 4.94-4.90 (m. 2H), 4.53-4.44(m, 4H), 4.38 (s, 2H), 4.26-4.22 (s, 1H), 4.14-4.02 (m, 2H), 3.97-3.95 (m, 1H), 3.59-3.56(m, 1H). |
| **aa163** | DMSO-d₆: 9.99 ( s, 1H); 8.02 (s, 1H), 7.69 (d, 1H, J=8.8 Hz); 7.57-7.54 (m, 3H); 7.40 (t, 1H, J=9.4 Hz); 6.43 (d, 1H, *J*=6.9 Hz); 6.31 (s, 2H); 4.69 (d, 1H, *J*=1.8 Hz); 4.66 (dd, 1H, *J*=1.8, 6.6 Hz); 4.14 (dd, 1H, *J*=2.2, 8.8 Hz); 3.94 ppm (m, 4H); 3.66 (dd, 1H, *J*=2.2, 9.2 Hz); 3.37 (t, 2H, *J*= 5.5 Hz); 2.12-1.90 (m, 4H). |

### EXAMPLE aa164 - aa165

### EXAMPLE aa 164

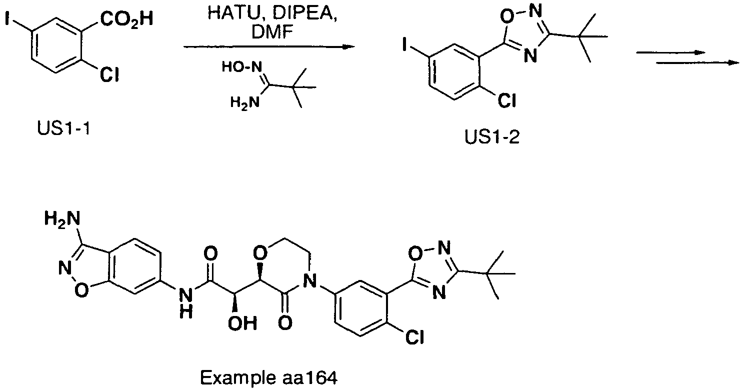

### Synthesis of intermediate US1-2

### (3-tert-butyl-5-(2-chloro-5-iodophenyl)-1,2,4-oxadiazole)

To a solution of 2-chloro-5-iodobenzoic acid (1.0 g, 3.54 mmol) in 40 mL DMF was added diisopropylethyl amine (1.23 mL, 7.08 mmol) followed by HATU (2.02 g, 5.31 mmol), After stirring the reaction at room temperature for 10 min, N-hydroxy-2,2-dimethylpropanimidamide (0.82 g, 7.08 mmol) was added. The resulting reaction mixture was stirred at room temperature for 1h and then at 110 °C for 16h. After cooling to room temperature the reaction was quenched with water and extracted with ethyl acetate. The combined organic fractions were washed with brine, dried (Na₂SO₄), concentrated, and purified by flash chromatography (2-5% EtOAc in hexanes) to yield 660 mg of intermediate US1-2.

### Synthesis of Example aa164

N-(3-amino-1,2-benzisoxazol-6-yl)-4-[4-chloro-3-[3-(1,1-dimethylethyl)-1,2,4-oxadiaz ol-5-yl]phenyl]-alpha(R)-hydroxy-3-oxo-2(R)-morpholineacetamide Intermediate US1-2 was converted to Example aa164 using previously described procedures.

### EXAMPLE aa 165

### N-(3-amino-1,2-benzisoxazol-6-yl)-4-[2-fluoro-5-(trifluoromethyl)phenyl]-alpha(R)-hy droxy-3-oxo-2(R)-morpholineacetamide

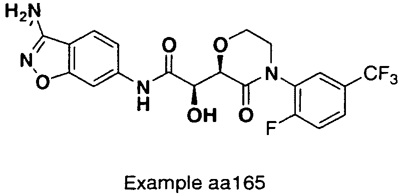

Example aa165 was prepared starting from 1-fluoro-2-iodo-4-(trifluoromethyl)benzene using previously described procedures. Analytical Data:
Mass Spectra:

| EXAMPLE | LCMS (M+H)⁺ |
|---|---|
| **aa164** | 541.3.543.3 |
| **aa165** | 469.2 |

¹H-NMR Data:

| EXAMPLE | NMR (400 MHz, ppm) |
|---|---|
| **aa164** | *CDCl₃*: 8.91 (1H, brs), 8.03 (2H, d, J = 12.9 Hz), 7.59-7.42 (3H, m), 7.28-7.26 (m, 1H), 4.97-4.94 (2H, m), 4.42 (2H, brs), 4.37-4.25 (2H, m), 4.19-4.05 (2H, m), 3.33 (1H, d. J = 11 Hz), 1.43 (9H, s) |
| **aa165** | *DMSO:* 10.0 (1H, s), 8.02 (1H, s), 7.93 (1H, dd, J = 2.2, 7.0 Hz), 7.84-7.80 (1H, m), 7.68 (1H, d, J = 8.8 Hz), 7.62-7.56 (2H, m), 6.45 (1H, d, J = 7.0 Hz), 6.32 (2H, brs), 4.72 (1H, d, J = 1.8 Hz), 4.62 ( 1H, dd, J = 1.8, 7.0 Hz), 4.17-4.13 (1H, m), 4.0-3.94 (1H, m), 3.91-3.84 (1H, m), 3.61 (1H, d, J = 11.7 Hz) |

### Utility

The invention also relates to medicaments which contain an efficacious amount of at least one compound of the Formula (I) and/or of a pharmaceutically acceptable salt of the compound of the Formula (I) and/or an optionally stereoisomeric form of the compound of the Formula (I), together with a pharmaceutically suitable and pharmaceutically acceptable vehicle, additive and/or other active substances and auxiliaries.

On account of their pharmacological properties, the compounds according to the invention are suitable, for example, for the prophylaxis, secondary prevention and therapy of all those diseases which are treatable by inhibition of blood clotting factor IXa. Thus, the compounds according to the invention are suitable as inhibitors both for prophylactic and for therapeutic administration to humans. They are suitable both for acute treatment and for long-term therapy. The compounds of the Formula (I) can be employed in patients who are suffering from disorders of well-being or diseases which accompany thromboses, embolisms, hypercoagulability or fibrotic changes.

These include myocardial infarct, angina pectoris and all other forms of acute coronary syndrome, stroke, peripheral vascular diseases, deep vein thrombosis, pulmonary embolism, embolic or thrombotic events caused by cardiac arrhythmias, cardiovascular events such as restenosis after revascularization, angioplasty and similar interventions such as stent implantations and bypass operations. Furthermore, the compounds of the Formula (I) can be employed in all interventions which lead to contact of the blood with foreign surfaces, as in dialysis patients and patients with indwelling catheters. Compounds of the Formula (I) can also be employed in order to reduce the risk of thrombosis after surgical interventions such as in knee and hip joint operations.

Compounds of the Formula (I) are suitable for the treatment of patients with disseminated intravascular coagulation, sepsis and other intravascular events which accompany inflammation. Furthermore, compounds of the Formula (I) are suitable for the prophylaxis and treatment of patients with atherosclerosis, diabetes and the metabolic syndrome and their sequelae. Disorders of the hemostatic system (for example fibrin deposits) have been implicated in mechanisms which lead to tumor growth and tumor metastasis, and in the inflammatory and degenerative joint diseases such as rheumatoid arthritis and arthrosis. Compounds of the Formula (I) are suitable for the retardation or prevention of such processes.

Further indications for the use of the compounds of the Formula (I) are fibrotic changes of the lungs such as chronic obstructive pulmonary disease, adult respiratory distress syndrome (ARDS) and of the eye, such as fibrin deposits after eye operations. Compounds of the Formula (I) are also suitable for the prevention and/or treatment of scar formation.

The medicaments according to the invention can be administered by oral, inhalative, rectal or transdermal administration or by subcutaneous, intraarticular, intraperitoneal or intravenous injection. Oral administration is preferred. Coating of stents with compounds of the Formula (I) and other surfaces which come into contact with blood in the body is possible.

The invention also relates to a process for the production of a medicament, which comprises bringing at least one compound of the Formula (I) into a suitable administration form using a pharmaceutically suitable and pharmaceutically acceptable carrier and optionally further suitable active substances, additives or auxiliaries.

Suitable solid or galenical preparation forms are, for example, granules, powders, coated tablets, tablets, (micro)capsules, suppositories, syrups, juices, suspensions, emulsions, drops or injectable solutions and preparations having prolonged release of active substance, in whose preparation customary excipients such as vehicles, disintegrants, binders, coating agents, swelling agents, glidants or lubricants, flavorings, sweeteners and solubilizers are used. Frequently used auxiliaries which may be mentioned are magnesium carbonate, titanium dioxide, lactose, mannitol and other sugars, talc, lactose, gelatin, starch, cellulose and its derivatives, animal and plant oils such as cod liver oil, sunflower, peanut or sesame oil, polyethylene glycol and solvents such as, for example, sterile water and mono- or polyhydric alcohols such as glycerol.

Preferably, the pharmaceutical preparations are prepared and administered in dose units, where each unit contains as active constituent a certain dose of the compound of the Formula (I) according to the invention. In the case of solid dose units such as tablets, capsules, coated tablets or suppositories, this dose can be approximately 1000 mg, but preferably approximately 50 to 300 mg and in the case of injection solutions in ampoule form approximately 300 mg, but preferably approximately 10 to 100 mg.

For the treatment of an adult patient weighing approximately 70 kg, depending on the efficacy of the compound according to Formula (I), daily doses of approximately 2 mg to 1000 mg of active substance, preferably approximately 50 mg to 500 mg, are indicated. Under certain circumstances, however, higher or lower daily doses may also be appropriate. The daily dose can be administered both by single administration in the form of an individual dose unit or else of a number of smaller dose units and by multiple administration of subdivided doses at certain intervals.

Compounds of the Formula (I) can be administered both as a monotherapy and in combination or together with all antithrombotics (anticoagulants and platelet aggregation inhibitors), thrombolytics (plasminogen activators of any type), other profibrinolytically active substances, hypotensives, blood sugar regulators, lipid-lowering agents and antiarrhythmics.

The inhibitatory effectiveness of compounds of the present invention to the coagulation factors XIa, VIIa, IXa, Xa, plasma kallikrein or thrombin, can be determined using a relevant purified serine protease, respectively, and an appropriate synthetic substrate.

### Pharmacological examples

### Determination of inhibitory activity against factor IXa

Inhibitory activity against factor IXa was tested using the substrate SPECTROFLUOR FIXa (american diagnostica inc.; 500 West Avenue. Stamford, CT 06902 USA: Pr. No. 299F) and human factor IXa (american diagnostica inc.; Pr. No. 449b). Test substances dissolved in buffer A (50 mM α,α,α-tris (hydroxymethyl) methylamine (Tris), 100 mM NaCl, 5 mM CaCl₂, 15 %(v/v) ethylene glycol, pH 8.0) were mixed with factor IXa (2.0 µg/ml final concentration). The enzyme reaction was started by addition of SPECTROFLUOR FIXa (100 µM final concentration). After incubation for 60 minutes at room temperature, the reaction was stopped by the addition of 20 %(v/v) acetic acid solution, and then measured the fluorescence value (Excitation Wavelength:355nm, Emission Wavelength;460nm) in a microtiter plate reader (ARVO 1420 Multilabel Counter; PerkinElmer).
The IC₅₀ was calculated from a dilution series of the test substance with the aid of the software, Symix Assay Explorer (Symyx Technologies, Inc.). All examples have IC₅₀ of 30 µM or less. Table 5 shows the results specific results.

**Table 5:**

| Example | Factor IXa enzyme assay IC₅₀ [nM] | Example | Factor IXa enzyme assay IC₅₀ [nM] |
|---|---|---|---|
| a1 | 32 | aa142 | 66 |
| a3 | 12 | aa144 | 13 |
| a6 | 9.3 | aa150 | 83 |
| a11 | 9.6 | aa151 | 380 |
| a21 | 9 | aa152 | 1200 |

In one embodiment, the compounds of the present invention were selective factor IXa inhibitors, i.e., selective for factor IXa over other coagulation factors, such as factor Xa.

### Determination of inhibitory activity against factor Xa

This measuring was performed as well as Factor IXa method excluding the following conditions. As substrate and enzyme, SPECTROFLUOR FXa (american diagnostica inc.; Pr. No. 222F, 100 µM final concentration) and human factor Xa (american diagnostica inc.; Pr. No. 526, 44 ng/ml final concentration) were used respectively. Test substances dissolved in buffer B (20 mM Tris, 200 mM NaCl, 2.5 mM CaCl₂, pH 8.0).

### Selectivity calculation

Selectivity for Factor IXa activity over Factor Xa activity can be determined by the following calculation.: (IC50 Factor Xa) / (IC50 Factor IXa). Similar calculations can be made for selectivity of compounds for Factor IXa compared to other coagulation factors.

These as well as other ways of minimizing contact between the components of combination products of the present invention, whether administered in a single dosage form or administered in separate forms but at the same time by the same manner, will be readily apparent to those skilled in the art, once armed with the present disclosure.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the relevant art and are intended to fall within the scope of the appended claim.

A number of references have been cited, the entire disclosures of which have been incorporated herein in their entirety.

The compounds of the present invention may also be useful as inhibitors of additional serine protease, notably human thrombin, human plasma kallikrein and human plasmin. Because of their inhibitory action, these compounds are indicated for use in the prevention or treatment of physiological reactions, as it were "Conditions" including thromboembolic disorder (arterial cardiovascular thromboembolic disorders, venous cardiovascular thromboembolic disorders, thromboembolic disorders in the chambers of the heart, unstable angina, an acute coronary syndrome, atrial fibrillation, first myocardial infarction, recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from (a) prosthetic valves or other implants, (b) indwelling catheters, (c) stents, (d) cardiopulmonary bypass, (e) hemodialysis, or (f) other procedures in which blood is exposed to an artificial surface that promotes thrombosis), blood coagulation, fibrinolysis, blood pressure regulation and inflammation, and wound healing catalyzed by the aforesaid class of enzymes. Specifically, the compounds have utility as drugs for the treatment of diseases arising from elevated thrombin activity of the aforementioned serine proteases, such as myocardial infarction, and as reagents used as anticoagulants in the processing of blood to plasma for diagnostic and other commercial purposes.

The compounds of the present invention can be administered alone or in combination with one or more additional therapeutic agents. These include other anti-coagulant or coagulation inhibitory agents anti-platelet or platelet inhibitory agents, anti-inflammatory agents, thrombin inhibitors, thrombolytic or fibrinolytic agents, thrombin receptor (PAR-1) antagonist, a factor VIIa inhibitor, factor VIIIa inhibitor, a factor IXa inhibitor different from the compound of claim 1, a factor Xa inhibitor, a factor XIa inhibitor, TAFI, and fibrinogen inhibitors.

The compounds are administered to a mammal in a therapeutically effective amount. By "therapeutically effective amount" it is meant an amount of a compound of the present invention that, when administered alone or in combination with an additional therapeutic agent to a mammal, is effective to treat (i.e. prevent, inhibit or ameliorate) the thromboembolic and/or inflammatory disease condition or treat the progression of the disease in a host.

The compounds of the invention are preferably administered alone to a mammal in a therapeutically effective amount. However, the compounds of the invention can also be administered in combination with an additional therapeutic agent, as define below, to a mammal in a therapeutically effective amount. When administered in a combination, the combination of compounds in preferably, but not necessarily, a synergistic combination. Synergy, as described for example by Chou and Talalay, Adv. Enzyme Regul. 1984, 22, 27-55, occurs when the effect (in this case, inhibition of the desired target) of the compounds when administered in combination is greater than the additive effect of the compounds when administered alone as a single agent. In general, a synergistic effect is most clearly demonstrated at suboptimal concentrations of the compounds. Synergy can be in terms of lower cytotoxicity, increased anticoagulant effect, or some other beneficial effect of the combination compared with the individual components.

By "administered in combination" or "combination therapy" it is meant that the compound of the present invention and one or more additional therapeutic agents are administered concurrently to the mammal being treated. When administered in combination each component may be administered at the same time or sequentially in any order at different points in time. Thus, each component may be administered separately but sufficiently closely in time so as to provide the desired therapeutic effect.

Compounds which can be administered in combination with the compounds of the present invention include, but are not limited to, anticoagulants, anti-thrombin agents, anti-platelet agents, fibrinolytics, hypolipidemic agents, antihypertensive agents, and anti-ischemic agents.

Other anticoagulant agents (or coagulation inhibitory agents) that may be used in combination with the compounds of this invention include warfarin, heparin (either unfractionated heparin or any commercially available low molecular weight heparin, for example LOVANO), aprotinin, synthetic pentasaccharide, direct acting thrombin inhibitors including hirudin and argatroban, as well as other factor VIIa inhibior, VIIIa inhibior, IXa inhibior, Xa inhibior, XIa inhibior, thrombin inhibior, fibrinogen inhibitors, TAFI, and known in the art. Factor IXa inhibitors different from the compounds of Formula (I) include synthetic active-site blocked competitive inhibitors, oral inhibitors and RNA aptamers. These are described in the previously cited Howard et al. reference (Howard, EL, Becker KC, Rusconi, CP, Becker RC. Factor IXa Inhibitors as Novel Anticoagulents. Arterioscler Thromb Vasc Biol. 2007; 27: 722-727.).

The term anti-platelet agents (or platelet inhibitory agents), as used herein, denotes agents that inhibit platelet function, for example, by inhibiting the aggregation, adhesion or granular secretion of platelets. Such agents include, but are not limited to, the various known non-steroidal anti-inflammatory drugs (NSAIDS) such as aspirin, ibuprofen, naproxen, sulindac, indomethacin, mefenamate, droxicam, diclofenac, sulfinpyrazone, and piroxicam, including pharmaceutically acceptable salts or prodrugs thereof. Of the NSAIDS, aspirin (acetylsalicylic acid or ASA), and piroxicam are preferred. Other suitable platelet inhibitory agents include IIb/IIIa antagonists (e.g., tirofiban, eptifibatide, and abciximab), thromboxane-A2-receptor antagonists (e.g., ifetroban), thromboxane-A2-synthetase inhibitors, phosphodiesterase-III (PDE-III) inhibitors (e.g., dipyridamole, cilostazol), and PDE V inhibitors (such as sildenafil), and pharmaceutically acceptable solts or prodrugs thereof.

The term anti-platelet agents (or platelet inhibitory agents), as used herein, is also intended to include ADP (adenosine diphosphate) receptor antagonists, preferable antagonists of the purinergic receptors P2Y1 and P2Y12 with P2Y12 being even more preferred. Preferred P2Y12 receptor antagonists include ticlopidine and clopidogrel, including pharmaceutically acceptable salts or prodrugs thereof. Clopidogrel is an even more preferred agent. Ticlopidine and clopidogrel are also preferred compounds since they are known to be gentle on the gastro-intestinal tract in use. The compounds of the present invention may also be dosed in combination with aprotinin.

The term thrombin inhibitors (or anti-thrombin agents), as used herein, denotes inhibitors of the serine protease thrombin. By inhibiting thrombin, various thrombin-mediated processes, such as thrombin-mediated platelet activation (that is, for example, the aggregation of platelets, and/or the granular secretion of plasminogen activator inhibitor-I and/or serotonin), endothelial cell activation, inflammatory reactions, and/or fibrin formation are disrupted. A number of thrombin inhibitors are known to one of skill in the art and these inhibitors are contemplated to be used in combination with the present compounds. Such inhibitors include, but are not limited to, boroarginine derivatives, boropeptides, heparins, hirudin and argatroban, including pharmaceutically acceptable salts and prodrugs thereof. Boroarginine derivatives and boropeptiders include N-acetyl and peptide derivatives of boronic acid, such as C-terminal alpha-aminoboronic acid derivatives of lysine, ornithine, arginine, homoarginine and corresponding isothiouronium analogs thereof. The term hirudin, as used herein, includes suitable derivatives or analogs of hirudin, referred to herein as hirulogs, such as disulfatohirudin.

The term "thrombin receptor antagonists", also known as protease activated receptor (PAR) antagonists or PAR-1 antagonists, are useful in the treatment of thrombotic, inflammatory, atherosclerotic and fibroproliferative disorders, as well as other disorders in which thrombin and its receptor play a pathological role.

Thrombin receptor antagonist peptides have been identified based on structure-activity studies involving substitutions of amino acids on thrombin receptors. In Bernatowicz et al. J. Med. Chem., vol. 39, pp. 4879-4887 (1996), tetra-and pentapeptides are disclosed as being potent thrombin receptor antagonists, for example N-trans-cinnamoyl-p-fluoroPhe-p-guanidinoPhe-Leu-Arg-NH2 and N-trans-cinnamoyl-p-fluoroPhe-p-guanidinoPhe-Leu-Arg-Arg-NH2. Peptide thrombin receptor antagonists are also disclosed in WO 94/03479, published Feb. 17, 1994.

Substituted tricyclic thrombin receptor antagonists are disclosed in U.S. Pat. Nos. 6,063,847, 6,326,380 and WO 01/96330 and 10/271,715.

Other thrombin receptor antagonists include those disclosed in U.S. Pat. Nos. 71304.078; 7,235,567; 7.037,920; 6,645.987; and EP Patent Nos. EP1495018 and EP1294714.

The term thrombolytic (or fibrinolytic) agents (or thrombolytics or fibrinolytics), as used herein, denotes agents that lyse blood clots (thrombi). Such agents include tissue plasminogen activator (TPA, natural or recombinant) and modified forms thereof, anistreplase, urokinase, streptokinase, tenecteplase (TNK), lanoteplase (nPA), factor VIIa inhibitors, PAI-I inhibitors (i.e., inactivators of tissue plasminogen activator inhibitors), alpha-2-antiplasmin inhibitors, and anisoylated plasminogen streptokinase activator complexs, including pharmaceutically acceptable salts or prodrugs thereof. The term anistreplase, as used herein, refers to anisoylated plasminogen streptokinase activator comples, as described, for example, in European Patent Application No. 028,489, the disclosure of which is hereby incorporated herein by reference herein. The term urokinase, as used herein, is intended to denote both dual and single chain urokinase, the latter also being referred to herein as prourokinase.

Examples of suitable anti-arrythmic agents for use in combination with the present compounds include: Class I agents (such as propafenone); Class II agents (such as carvedilol and propranolol); Class III agents (such as sotalol, dofetilide, aminodarone, azimilide and ibutilide); Class IV agents (such as ditiazem and verapamil); K+ cannel openers such as IAch inhibitors, and IKur inhibitors (e.g., compounds such as those disclosed in WO01/40231).

The term antihypertensive agents, as used herein, include: alpha adrenergic blockers; beta adrenergic blockers; calcium channel blockers (e.g., diltiazem, verapamil nifedipine, amlodipine and mybefradil); diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamterene, amiloride, spironolactone); rennin inhibitors; angiotensin-converting enzyme (ACE) inhibitors (e.g., captopril, Lisinopril, fosinopril, enalapril, ceranopril, cilazopril, delapril, pentopril, quinapril, ramipril, Lisinopril); angiotensin-II-receptor antagnonists (e.g., irbestatin, Losartan, valsartan); ET receptor antagonists (e.g., sitaxsentan, atrsentan and compounds disclosed in U.S. Pat. Nos. 5,612,359 and 6,043,265); Dual ET/All antagonist (e.g., compounds disclosed in WO 00/01389); neutral endopeptidase (NEP) inhibitors; vasopepsidase inhibitors (dual CCE/NEP inhibitors, e.g., omapatrilat, gemopatrilat, nitrates); and β-blockers (e.g., propranolol, nadolol, or carvedilol).

Examples of suitable cardiac glycosides for use in combination with compounds of the present invention include digitalis and ouabain.

Examples of suitable mineralocorticoid receptor antagonists for use in combination with the compounds of the present invention include spironolactone and eplirinone.

Examples of suitable cholesterol/lipid lowering agents and lipid profile therapies for use in combination with the compounds of the present invention include: HMG-CoA reductase inhibitors (e.g., pravastatin, lovastatin, atrbastatin, simvastatin, fluvastatin, NK-104 (a.k.a. itavastatin, or nisvastatin or nisbastatin) and ZD-4522 (a.k.a. rosuvastatin, or atavastatin or visastatin)); squalene synthetase inhibitors; fibrates; bile acid sequestrants (such as questran); ACAT inhibitors; MTP inhibitors; lipooxygenase inhibitors; cholesterol absorption inhibitors; and cholesterol ester transfer protein inhibitors (e.g., CP-529414).

Examples of suitable anti-diabetic agents for use in combination with the compounds of the present invention include: biguanides (e.g., metformin); glucosidase inhibitors (e.g., acarbose); insulins (including insulin secretagogues or insulin sensitizers); meglitinides (e.g., repaglinide); sulfonylureas (e.g., glimepiride, glyburide and glipizide); biguanide/glyburide combinations (e.g., glucovance), thiozolidinediones (e.g., troglitazone, rosiglitazone and pioglitazone), PPAR-alpha agonists, PPAR-gamma agonists, PPAR alpha/gamma dual agonists, SGLT2 inhibitors, inhibitors of fatty acid binding protein (aP2) such as those disclosed in WO00/59506, glucagons-like peptide-1 (GLP-1), and dipeptidyl peptidase IV (DP4) inhibitors.

Examples of suitable anti-depressant agents for use in combination with the compounds of the present invention include nefazodone and sertraline.

Examples of suitable anti-inflammatory agents for use in combination with the compounds of the present invention include: prednisone; dexamethasone; enbrel; protein tyrosine kinase (PTK) inhibitors; cyclooxygenase inhibitors (including NSAIDs, and COX-1 and/or COX-2 inhibitors); aspirin; indomethacin; ibuprofen; piroxicam; naproxen; celecoxib; and/or rofecoxib.

Examples of suitable anti-osteoporosis agents for use in combination with the compounds of the present invention include alendronate and raloxifene.

Examples of suitable hormone replacement therapies for use in combination with the compounds of the present invention include estrogen (e.g., conjugated estrogens) and estradiol.

Examples of suitable anti-obesity agents for use in combination with the compounds of the present invention include orlistat and aP2 inhibirotr (such as those disclosed in WO00/59506).

Examples of suitable anti-anxiety agents for use in combination with the compounds of the present invention include diazepam, lorazepam, buspirone, and hydroxyzine pamoate.

Examples of suitable anti-proliferative agents for use in combination with the compounds of the present invention include cyclosporine A, paclitaxel, adriamycin; epithilones, cisplatin, and carboplatin.

Examples of suitable anti-ulcer and gastroesophageal reflux disease agents for use in combination with the compounds of the present invention include famotidine, ranitidine, and omeprazole.

## Claims

1. A compound of the Formula (I) 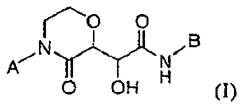 or a pharmaceutically acceptable salt thereof, wherein
A is 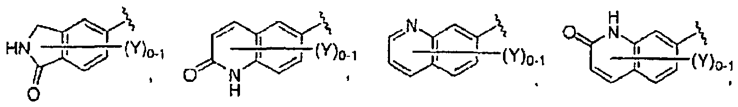 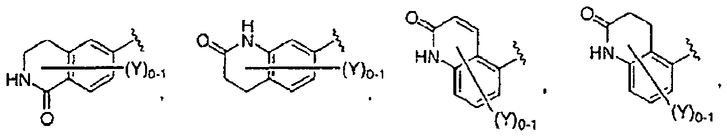 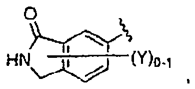 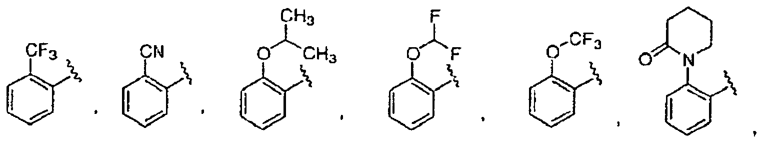 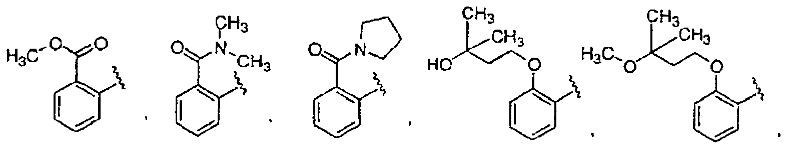 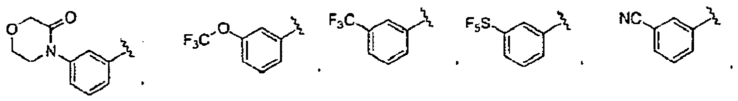 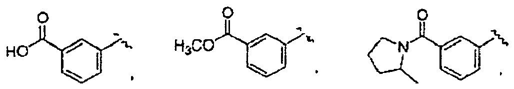 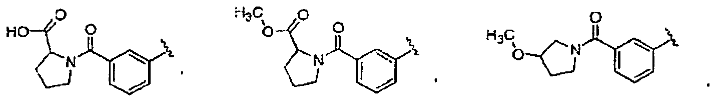 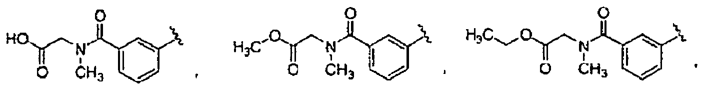 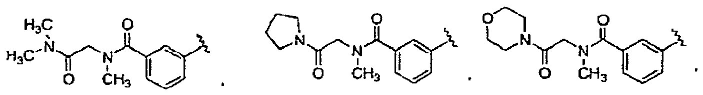 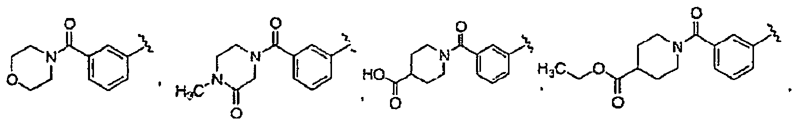 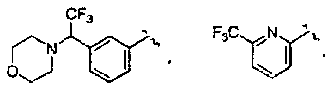 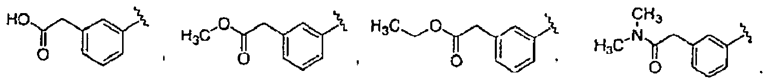 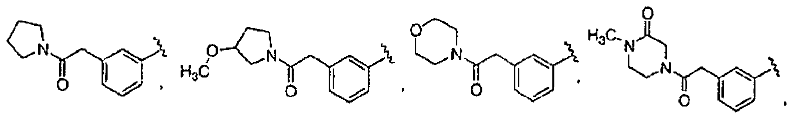 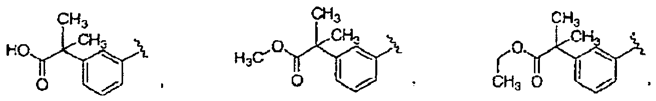 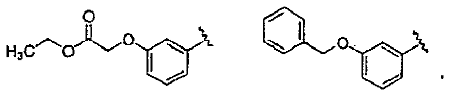 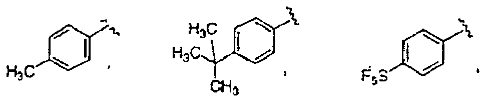 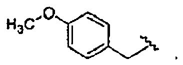 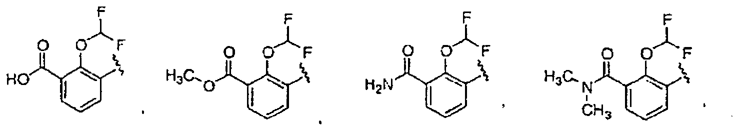 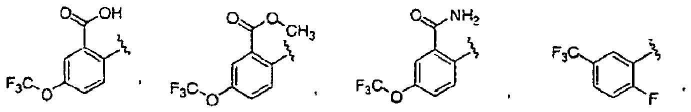 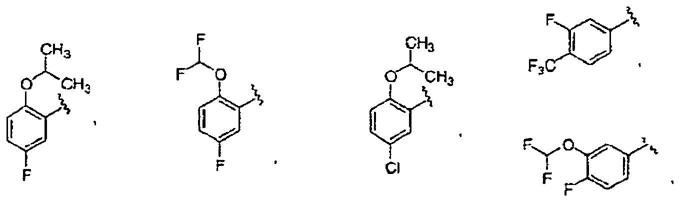 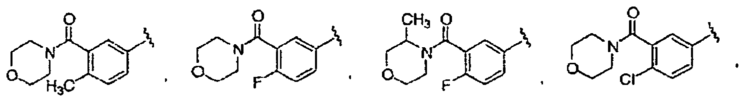 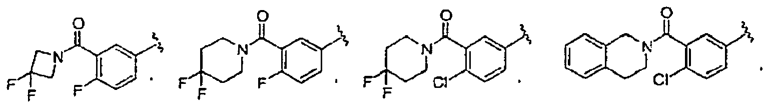 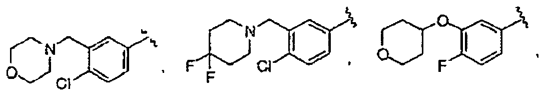 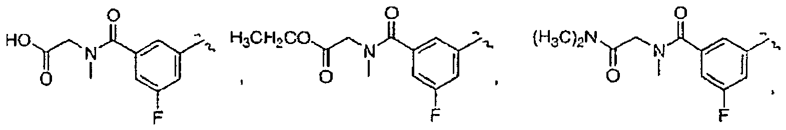 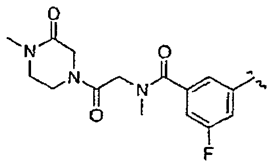 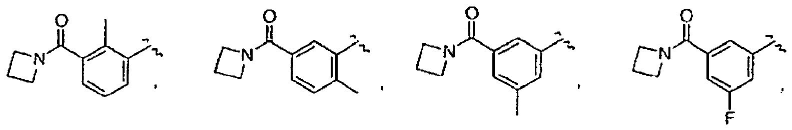 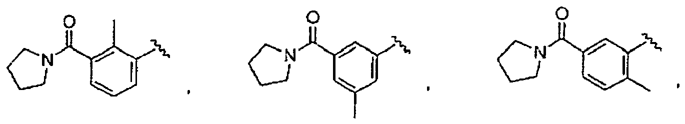 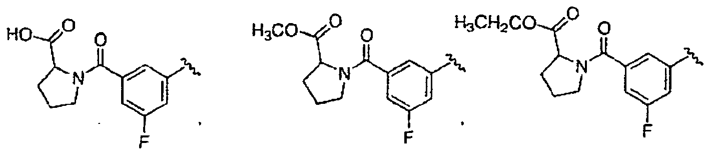 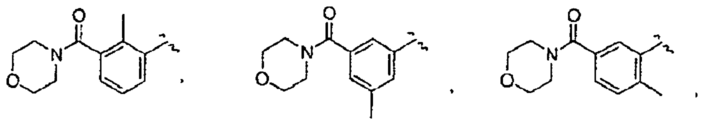 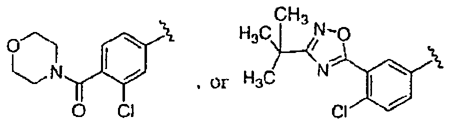 Y, attached to a carbon or nitrogen ring atom, is
1) halogen,
2) -(C₁-C₆)-alkyl,
3) -(C₁-C₃)-haloalkyl,
4) -(C₃-C₈)-cycloalkyl,
5) -OH,
6) -O-(C₁-C₆)-alkyl,
7) -O-(C₁-C₃)-haloalkyl,
8) =O (oxo),
wherein said -(C₁-C₆)-alkyl part of 2) and 6) of said Y is unsubstituted or substituted independently with the substituents selected from the group consisting of -(C₃-C₈)-cycloalkyl, -C(O)OH, and -C(O)-(C₁-C₆)-alkyl.
B is 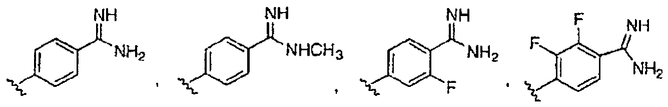 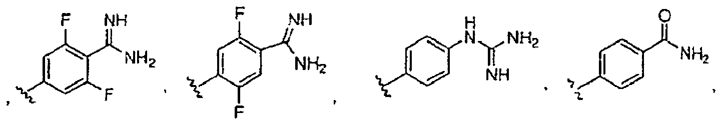 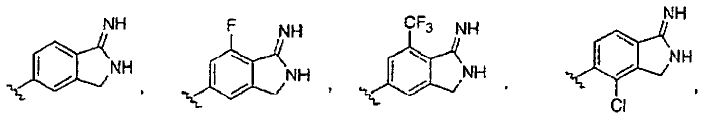 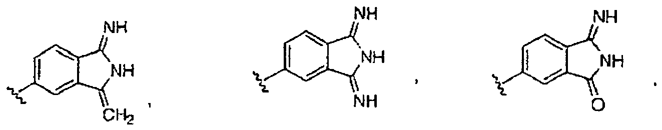 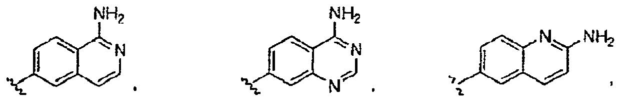 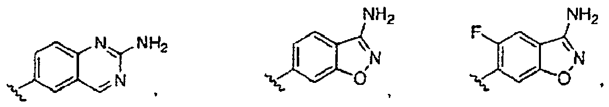 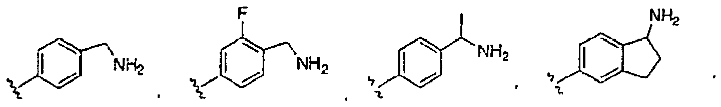 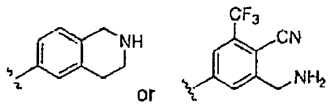 provided that
when A is 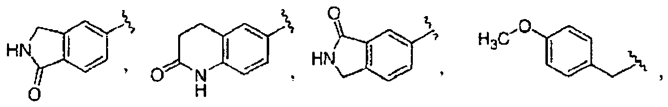 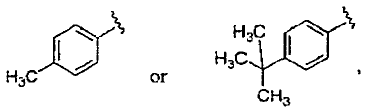 B is not 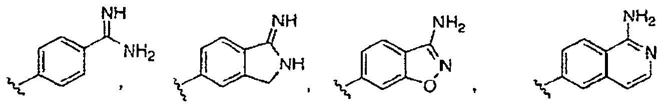 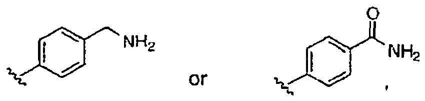 and
when A is 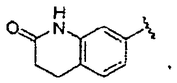 B is not

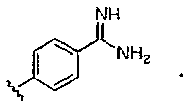

2. A compound of the Formula (I) according to claim 1 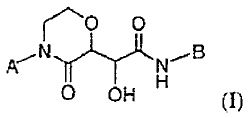 or a pharmaceutically acceptable salt thereof, wherein
A is 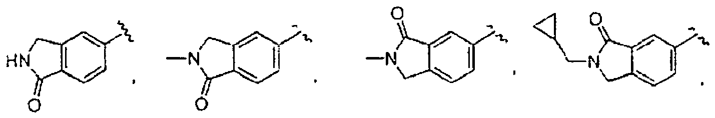 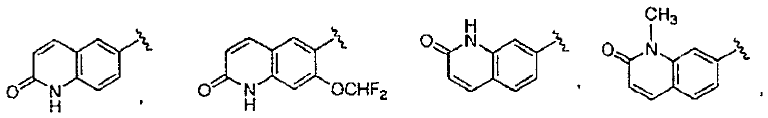 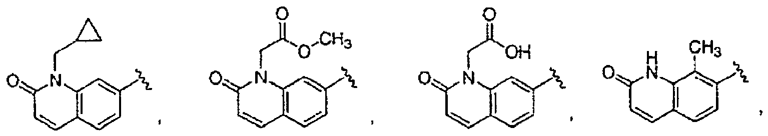 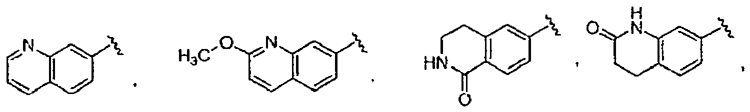 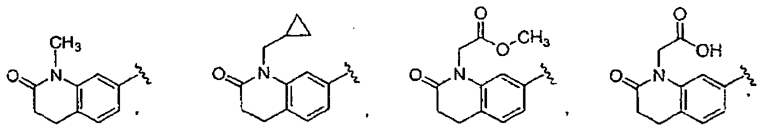 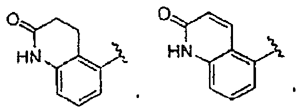 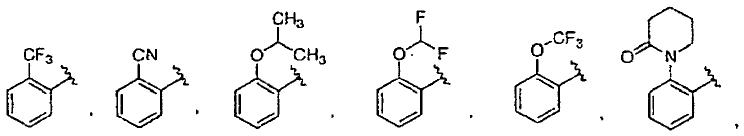 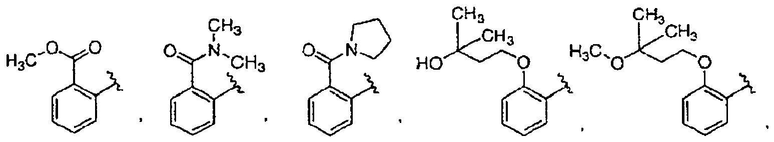 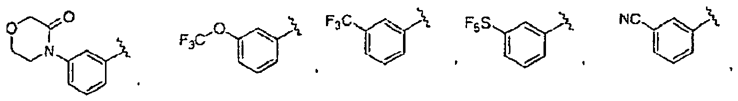 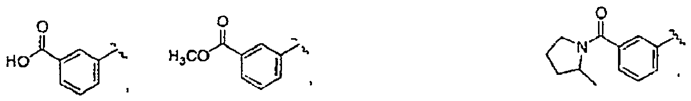 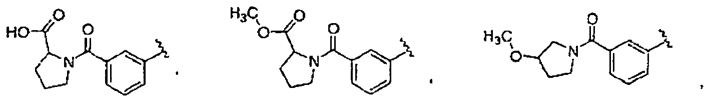 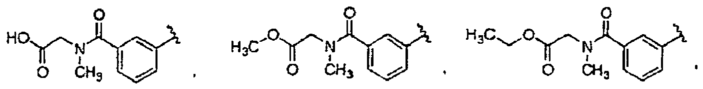 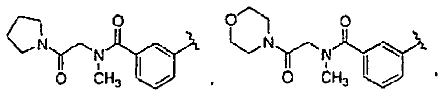 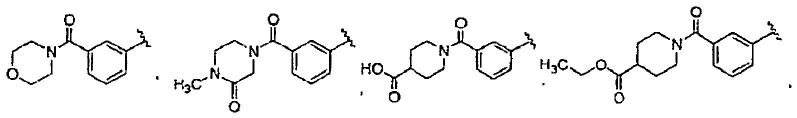 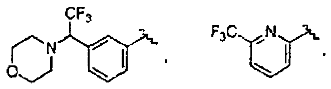 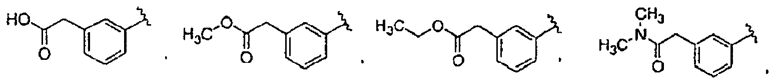 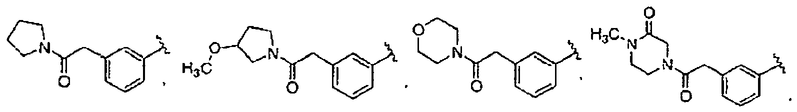 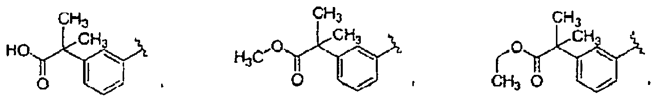 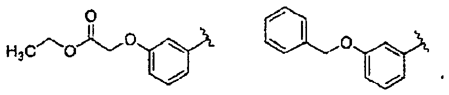 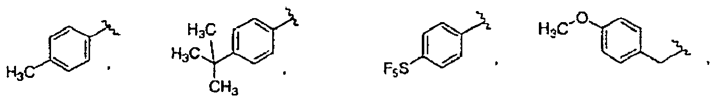 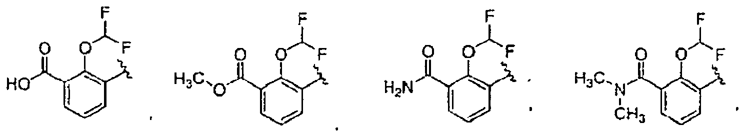 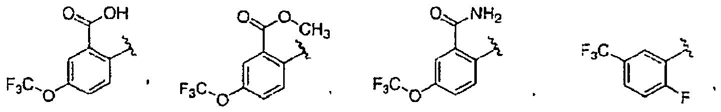 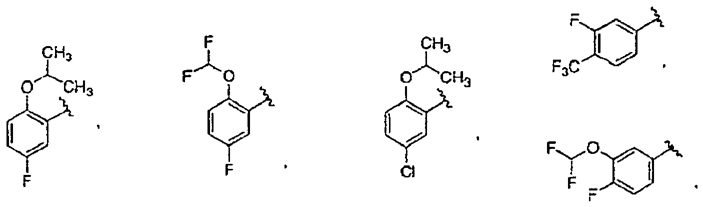 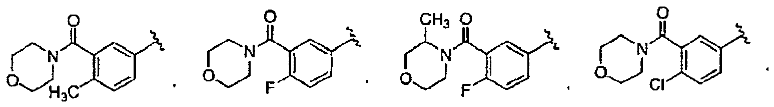 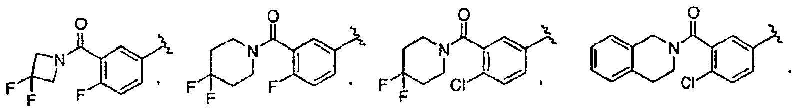 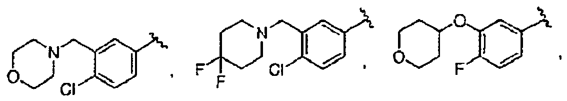 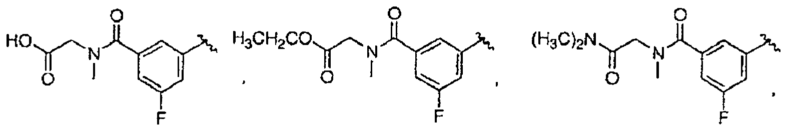 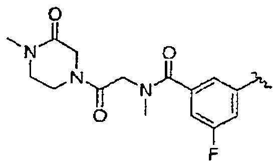 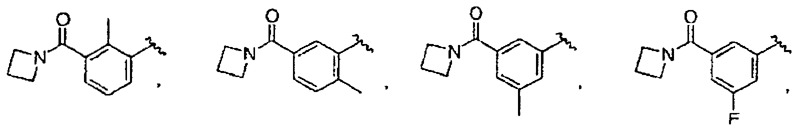 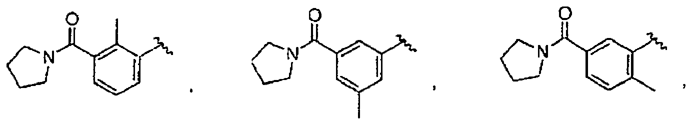 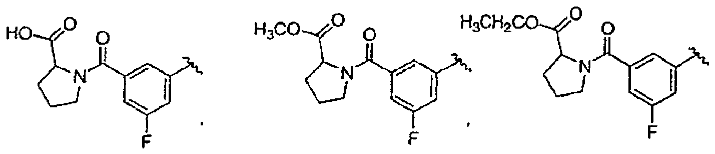 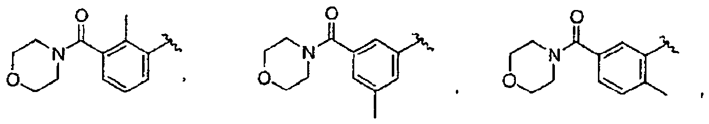 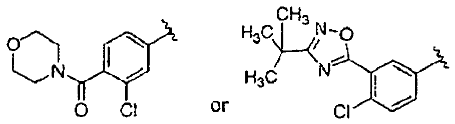 B is 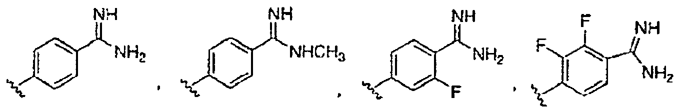 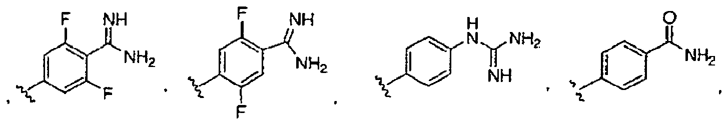 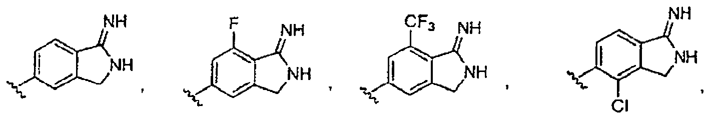 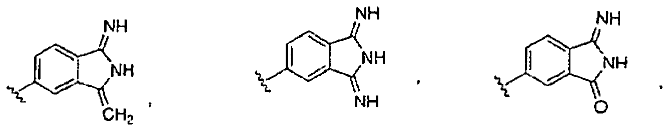 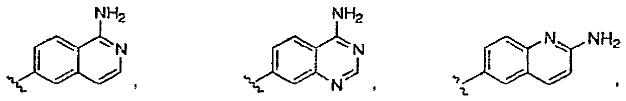 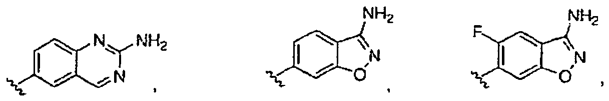 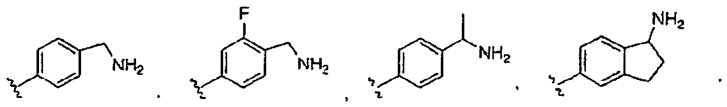 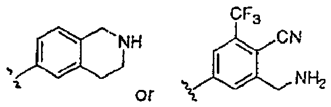 provided that
when A is 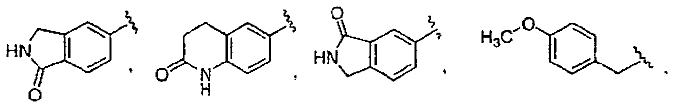 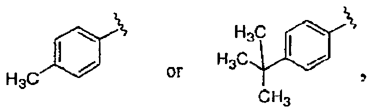 B is not 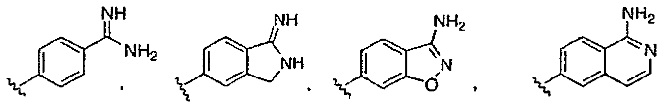 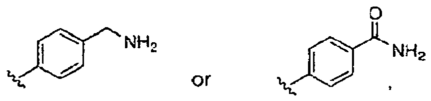 and
when A is 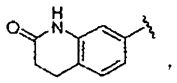 B is not

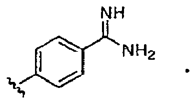

3. A compound of claim 1 or 2, or a pharmaceutically acceptable salt thereof wherein the absolute configuration of Formula (I) is

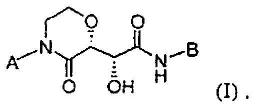

4. A compound of claim 1, which is
(2R)-2-[(2R)-4-(4-tert-butylphenyl)-3-oxomorpholin-2-yl]-2-hydroxy-N-[4-(N-methylcarbamimidoyl)phenyl]acetamide,
(2R)-2-hydroxy-N-(1-imino-2,3-dihydroisoindol-5-yl)-2-[(2R)-3-oxo-4-(1-oxo-2,3-dihydroisoindol-5-yl)morpholin-2-yl]acetamide,
(2R)-2-hydroxy-N-(1-imino-2,3-dihydroisoindol-5-yl)-2-[(2R)-3-oxo-4-(2-oxo-1H-quinolin-6-yl)morpholin-2-yl]acetamide,
(2R)-2-[(2R)-4-[7-(difluoromethoxy)-2-oxo-1H-quinolin-6-yl]-3-oxomorpholin-2-yl]-2 -hydroxy-N-(1-imino-2,3-dihydroisoindol-5-yl)acetamide,
(2R)-N-(4-carbamimidoylphenyl)-2-hydroxy-2-[(2R)-3-oxo-4-(2-oxo-1H-quinolin-6-yl )morpholin-2-yl]acetamide,
(2R)-N-(4-carbamimidoyl-3-fluorophenyl)-2-hydroxy-2-[(2R)-3-oxo-4-(2-oxo-1H-quinolin-6-yl)morpholin-2-yl]acetamide,
(2R)-2-hydroxy-N-(1-imino-2,3-dihydroisoindol-5-yl)-2-[(2R)-3-oxo-4-(2-oxo-1H-quinolin-7-yl)morpholin-2-yl]acetamide,
(2R)-2-hydroxy-N-(1-imino-2,3-dihydroisoindol-5-yl)-2-[(2R)-4-(1-methyl-2-oxoquinolin-7-yl)-3-oxomorpholin-2-yl]acetamide,
(2R)-2-[(2R)-4-[1-(cyclopropylmethyl)-2-oxoquinolin-7-yl]-3-oxomorpholin-2-yl]-2-hydroxy-N-(1-imino-2,3-dihydroisoindol-5-yl)acetamide,
2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetate,
2-[7-[(2R)-2-[(1R)-1-hydroxy-2-[(1-imino-2,3-dihydroisoindol-5-yl)amino]-2-oxoethyl]-3-oxomorpholin-4-yl]-2-oxoquinolin-1-yl]acetic acid,
(2R)-2-hydroxy-N-(1-imino-2,3-dihydroisoindol-5-yl)-2-[(2R)-4-(8-methyl-2-oxo-1H-quinolin-7-yl)-3-oxomorpholin-2-yl]acetamide,
(2R)-2-hydroxy-N-(1-imino-2,3-dihydroisoindol-5-yl)-2-[(2R)-4-(2-methoxyquinolin-7 -yl)-3-oxomorpholin-2-yl]acetamide,
(2R)-2-hydroxy-N-(1-imino-2,3-dihydroisoindol-5-yl)-2-[(2R)-3-oxo-4-quinolin-7-ylmorpholin-2-yl]acetamide,
(2R)-N-(3-amino-1,2-benzisoxazol-6-yl)-2-hydroxy-2-[(2R)-3-oxo-4-quinolin-7-ylmorpholin-2-yl]acetamide,
(2R)-2-hydroxy-N-(1-imino-2,3-dihydroisoindol-5-yl)-2-[(2R)-3-oxo-4-(2-oxo-1H-quinolin-5-yl)morpholin-2-yl]acetamide,
(2R)-2-hydroxy-N-(1-imino-2,3-dihydroisoindol-5-yl)-2-[(2R)-3-oxo-4-(1-oxo-3,4-dihydro-2H-isoquinolin-6-yl)morpholin-2-yl]acetamide,
(2R)-2-hydroxy-N-(1-imino-2,3-dihydroisoindol-5-yl)-2-[(2R)-3-oxo-4-(2-oxo-3,4-dihydro-1H-quinolin-7-yl)morpholin-2-yl]acetamide,
(2R)-2-hydroxy-N-(1iimino-2,3-dihydroisoindol-5-yl)-2-[(2R)-4-(1-methyl-2-oxo-3,4-dihydroquinolin-7-yl)-3-oxomorpholin-2-yl]acetamide,
(2R)-2-[(2R)-4-[1-(cyclopropylmethyl)-2-oxo-3,4-dihydroquinolin-7-yl]-3-oxamorpholin-2-yl]-2-hydroxy-N-(1-imino-2,3-dihydroisoindol-5-yl)acetamide,
methyl 2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydroquinolin-1-yl]acetate,
2-[7-[(2R)-2-[(1R)-1-hydroxy-2-[(1-imino-2,3-dihydroisoindol-5-yl)amino]-2-oxoethyl ]-3-oxomorpholin-4-yl]-2-oxo-3,4-dihydroquinolin-1-yl]acetic acid.
(2R)-N-(4-carbamimidoyl-3-fluorophenyl)-2-hydroxy-2-[(2R)-3-oxo-4-(2-oxo-3,4-dihydro-1H-quinolin-7-yl)morpholin-2-yl]acetamide,
(2R)-N-(3-amino-1,2-benzoxazol-6-yl)-2-hydroxy-2-[(2R)-3-oxo-4-(2-oxo-3,4-dihydro -1H-quinolin-7-yl)morpholin-2-yl]acetamide,
(2R)-N-(1-aminoisoquinolin-6-yl)-2-hydroxy-2-[(2R)-3-oxo-4-(2-oxo-3,4-dihydro-1H-quinolin-7-yl)morpholin-2-yl]acetamide,
(2R)-N-(4-aminoquinazolin-7-yl)-2-hydroxy-2-[(2R)-3-oxo-4-(2-oxo-3,4-dihydro-1H-quinolin-7-yl)morpholin-2-yl]acetamide,
(2R)-2-hydroxy-N-(1-imino-2,3-dihydroisoindol-5-yl)-2-[(2R)-3-oxo-4-(2-oxo-3,4-dihydro-1H-quinolin-5-yl)morpholin-2-yl]acetamide,
(2R)-2-hydroxy-2-[(2R)-4-[2-(3-hydroxy-3-methylbutoxy)phenyl]-3-oxomorpholin-2-yl]-N-(1-imino-2,3-dihydroisoindol-5-yl)acetamide,
(2R)-2-hydroxy-N-(1-imino-2,3-dihydroisoindol-5-yl)-2-[(2R)-4-[2-(3-methoxy-3-methylbutoxy)phenyl]-3-oxomorpholin-2-yl]acetamide.
(2R)-2-hydroxy-N-(1-imino-2,3-dihydroisoindol-5-yl)-2-[(2R)-3-oxo-4-[2-(2-oxopiperidin-1-yl)phenyl]morpholin-2-yl]acetamide,
(2R)-2-[(2R)-4-[2-(difluoromethoxy)-5-fluorophenyl]-3-oxomorpholin-2-yl]-2-hydroxy -N-(1-imino-2,3-dihydroisoindol-5-yl)acetamide,
methyl 2-(difluoromethoxy)-3-[(2R)-2-[(1R)-1-hydroxy-2-[(1-imino-2,3-dihydroisoindol-5-yl)amino]-2-oxoethyl]-3-oxomorpholin-4-yl]benzoate,
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]benzoic acid,
2-(difluoromethoxy)-3-[(2R)-2-[(1R)-1-hydroxy-2-[(1-imino-2,3-dihydroisoindol-5-yl) amino]-2-oxoethyl]-3-oxomorpholin-4-yl]-N,N-dimethylbenzamide,
2-(difluoromethoxy)-3-[2-[(1-hydroxy-2-[(1-imino-2,3-dihydroisoindol-5-yl)amino]-2-oxoethyl]-3-oxomorpholin-4-yl]benzamide,
methyl 2-[(2R)-2-[(1R)-1-hydroxy-2-[(1-imino-2.3-dihydroisoindol-5-yl)amino]-2-oxoethyl]-3-oxomorpholin-4-yl]-5-(trifluoromethoxy)benzoate,
2-[(2R)-2-[(1R)-1-hydroxy-2-[(1-imino-2,3-dihydroisoindol-5-yl)amino]-2-oxoethyl]-3 -oxomorpholin-4-yl]-5-(trifluoromethoxy)benzoic acid,
2-[(2R)-2-[(1R)-1-hydroxy-2-[(1-imino-2,3-dihydroisoindol-5-yl)amino]-2-oxoethyl]-3 -oxomorpholin-4-yl]-5-(trifluoromethoxy)benzamide,
(2R)-N-(3-amino-1,2-benzisoxazol-6-yl)-2-hydroxy-2-[(2R)-3-oxo-4-(2-oxo-1H-quinol in-6-yl)morpholin-2-yl]acetamide,
(2R)-N-(3-amino-1,2-benzisoxazol-6-yl)-2-hydroxy-2-[(2R)-3-oxo-4-(2-oxo-1H-quinol in-7-yl)morpholin-2-yl]acetamide,
(2R)-N-(3-amino-1,2-benzisoxazol-6-yl)-2-hydroxy-2-[(2R)-3-oxo-4-(2-oxo-1H-quinol in-5-yl)morpholin-2-yl]acetamide,
2-(3-((R)-2-((R)-2-(4-carbamimidoylphenylamino)-1-hydroxy-2-oxoethyl)-3-oxomorp holino)phenyl)acetate,
(R)-N-(4-carbamimidoylphenyl)-2-hydroxy-2-((R)-4-(3-methyl-5-(pyrrolidine-1-carbo nyl)phenyl)-3-oxomorpholin-2-yl)acetamide,
(R)-2-hydroxy-N-(1-iminoisoindolin-5-yl)-2-((R)-4-(3-methyl-5-(pyrrolidine-1-carbon yl)phenyl)-3-oxomorpholin-2-yl)acetamide,
2-(3-((R)-2-((R)-1-hydroxy-2-(1-iminoisoindolin-5-ylamino)-2-oxoethyl)-3-oxomorph olino)-N-methylbenzamido)acetic acid,
2-(3-((R)-2-((R)-1-hydroxy-2-(1-iminoisoindolin-5-ylamino)-2-oxoethyl)-3-oxomorph olino)-N-methylbenzamido)acetate,
(S)-1-(3-((R)-2-((R)-2-(4-carbamimidoylphenylamino)-1-hydroxy-2-oxoethyl)-3-oxom orpholino)benzoyl)pyrrolidine-2-carboxylic acid,
1-(3-((R)-2-((R)-2-(4-carbamimidoylphenylamino)-1-hydroxy-2-oxoethyl)-3-oxomorp holino)benzoyl)pyrrolidine-2-carboxylate,
3-((R)-2-((R)-2-(4-carbamimidoylphenylamino)-1-hydroxy-2-oxoethyl)-3-oxomorpholi no)-N-methyl-N-(2-oxo-2-(pyrrolidin-1-yl)ethyl)benzamide,
3-(R)-2-((R)-1-hydroxy-2-(1-iminoisoindolin-5-ylamino)-2-oxoethyl)-3-oxomorpholin o)-N-methyl-N-(2-oxo-2-(pyrrolidin-1-yl)ethyl)benzamide,
(R)-N-(4-carbamimidoylphenyl)-2-hydroxy-2-((R)-4-(3-methyl-5-(morpholine-4-carbo nyl)phenyl)-3-oxomorpholin-2-yl)acetamide,
(R)-2-hydroxy-N-(1-iminoisoindolin-5-yl)-2-((R)-4-(3-methyl-5-(morpholine-4-carbon yl)phenyl)-3-oxomorpholin-2-yl)acetamide,
(R)-2-hydroxy-N-(1-iminoisoindolin-5-yl)-2-((R)-4-(3-((R)-3-methoxypyrrolidine-1-ca rbonyl)phenyl)-3-oxomorpholin-2-yl)acetamide,
(R)-2-hydroxy-N-(1-iminoisoindolin-5-yl)-2-((R)-4-(3-((S)-3-methoxypyrrolidine-1-ca rbonyl)phenyl)-3-oxomorpholin-2-yl)acetamide,
(R)-2-((R)-4-(3-chloro-4-(morpholine-4-carbonyl)phenyl)-3-oxomorpholin-2-yl)-2-hyd roxy-N-(1-iminoisoindolin-5-yl)acetamide,
(R)-2-hydroxy-N-(1-iminoisoindolin-5-yl)-2-((R)-4-(3-(2-morpholino-2-oxoethyl)phen yl)-3-oxomorpholin-2-yl)acetamide,
(S)-1-(3-((R)-2-((R)-1-hydroxy-2-(1-iminoisoindolin-5-ylamino)-2-oxoethyl)-3-oxomo rpholino)benzoyl)pyrrolidine-2-carboxylic acid,
1-(3-((R)-2-((R)-1-hydroxy-2-(1-iminoisoindolin-5-ylamino)-2-oxoethyl)-3-oxomorph olino)benzoyl)pyrrolidine-2-carboxylate,
2-(3-((R)-2-((R)-1-hydroxy-2-(1-iminoisoindolin-5-ylamino)-2-oxoethyl)-3-oxomorph olino)phenyl)acetate,
2-(3-((R)-2-((R)-1-hydroxy-2-(1-iminoisoindolin-5-ylamino)-2-oxoethyl)-3-oxomorph olino)phenyl)acetic acid,
2-(3-((R)-2-((R)-1-hydroxy-2-(1-iminoisoindolin-5-ylamino)-2-oxoethyl)-3-oxomorph olino)-N-methylbenzamido)acetate,
2-(3-((R)-2-((R)-1-hydroxy-2-(1-iminoisoindolin-5-ylamino)-2-oxoethyl)-3-oxomorph olino)phenyl)-2-methylpropanoate,
(R)-2-hydroxy-N-(1-iminoisoindolin-5-yl)-2-((R)-4-(3-(morpholine-4-carbonyl)phenyl) -3-oxomorpholin-2-yl)acetamide,
3-((R)-2-((R)-1-hydroxy-2-(1-iminoisoindolin-5-ylamino)-2-oxoethyl)-3-oxomorpholin o)-N-methyl-N-(2-morpholino-2-oxoethyl)benzamide,
(R)-2-hydroxy-N-(1-iminoisoindolin-5-yl)-2-((R)-4-(3-(2-((R)-3-methoxypyrrolidin-1-yl)-2-oxoethyl)phenyl)-3-oxomorpholin-2-yl)acetamide,
(R)-2-hydroxy-N-(1-iminoisoindolin-5-yl)-2-((R)-4-(3-(2-((S)-3-methoxypyrrolidin-1-yl)-2-oxoethyl)phenyl)-3-oxomorpholin-2-yl)acetamide.
2-(3-((R)-2-((R)-1-hydroxy-2-(1-iminoisoindolin-5-ylamino)-2-oxoethyl)-3-oxomorph olino)phenyl)-2-methylpropanoate,
2-(3-((R)-2-((R)-hydroxy-2-(1-iminoisoindolin-5-ylamino)-2-oxoethyl)-3-oxomorph olino)phenyl)acetate,
(R)-2-hydroxy-N-(1-iminoisoindolin-5-yl)-2-((R)-4-(3-(4-methyl-3-oxopiperazine-1-ca rbonyl)phenyl)-3-oxomorpholin-2-yl)acetamide,
2-(3-((R)-2-((R)-1-hydroxy-2-(1-iminoisoindolin-5-ylamino)-2-oxoethyl)-3-oxomorph olino)phenyl)-2-methylpropanoate,
(R)-2-hydroxy-N-(1-iminoisoindolin-5-yl)-2-((R)-4-(3-(2-(4-methyl-3-oxopiperazin-1-yl)-2-oxoethyl)phenyl)-3-oxomorpholin-2-yl)acetamide,
(R)-2-((R)-4-(3-(2-(dimethylamino)-2-oxoethyl)phenyl)-3-oxomorpholin-2-yl)-2-hydro xy-N-(1-iminoisoindolin-5-yl)acetamide,
1-(3-((R)-2-((R)-1-hydroxy-2-(1-iminoisoindolin-5-ylamino)-2-oxoethyl)-3-oxomorph olino)benzoyl)piperidine-4-carboxylic acid,
1-(3-((R)-2-((R)-1-hydroxy-2-(1-iminoisoindolin-5-ylamino)-2-oxoethyl)-3-oxomorph olino)benzoyl)piperidine-4-carboxylate,
2-(3-((R)-2-((R)-1-acetoxy-2-(1-imino-3-oxoisoindolin-5-ylamino)-2-oxoethyl)-3-oxo morpholino)-N-methylbenzamido)acetic acid,
(R)-N-(1,3-diiminoisoindolin-5-yl)-2-((R)-4-(3-(2-(dimethylamino)-2-oxoethyl)phenyl) -3-oxomorpholin-2-yl)-2-hydroxyacetamide,
(R)-2-(1-imino-3-oxoisoindolin-5-ylamino)-1-((R)-4-(3-(methyl(2-oxo-2-(pyrrolidin-1-yl)ethyl)carbamoyl)phenyl)-3-oxomorpholin-2-yl)-2-oxoethylacetate,
(R)-N-(3-aminobenzo[d]isoxazol-6-yl)-2-((R)-4-(4-fluoro-3-(morpholine-4-carbonyl)p henyl)-3-oxomorpholin-2-yl)-2-hydroxyacetamide,
(R)-N-(3-aminobenzo[d]isoxazol-6-yl)-2-((R)-4-(4-chloro-3-((4,4-difluoropiperidin-1-y l)methyl)phenyl)-3-oxomorpholin-2-yl)-2-hydroxyacetamide),
(R)-N-(3-aminobenzo[d]isoxazol-6-yl)-2-hydroxy-2-((R)-3-oxo-4-(3-(3-oxomorpholin o)phenyl)morpholin-2-yl)acetamide,
(R)-N-(3-aminobenzo[d]isoxazol-6-yl)-2-((R)-4-(2-(difluoromethoxy)phenyl)-3-oxomo rpholin-2-yl)-2-hydroxyacetamide,
(R)-N-(3-aminobenzo[d]isoxazol-6-yl)-2-hydroxy-2-((R)-4-(2-isopropoxyphenyl)-3-ox omorpholin-2-yl)acetamide,
(R)-N-(3-aminobenzo[d]isoxazol-6-yl)-2-((R)-4-(3-cyanophenyl)-3-oxomorpholin-2-yl )-2-hydroxyacetamide,
(R)-N-(3-aminobenzo[d]isoxazol-6-yl)-2-((R)-4-(4-chloro-3-(morpholine-4-carbonyl)p henyl)-3-oxomorpholin-2-yl)-2-hydroxyacetamide.
(R)-N-(3-aminobenzo[d]isoxazol-6-yl)-2-hydroxy-2-((R)-4-(4-methyl-3-(morpholine-4 -carbonyl)phenyl)-3-oxomorpholin-2-yl)acetamide,
(R)-N-(3-aminobenzo[d]isoxazol-6-yl)-2-hydroxy-2-((R)-3-oxo-4-(3-(trifluoromethoxy )phenyl)morpholin-2-yl)acetamide,
(R)-N-(3-aminobenzo[d]isoxazol-6-yl)-2-hydroxy-2-((R)-3-oxo-4-(3-(trifluoromethyl)p henyl)morpholin-2-yl)acetamide,
(R)-N-(3-aminobenzo[d]isoxazol-6-yl)-2-((R)-4-(4-chloro-3-(1,2,3,4-tetrahydroisoquin oline-2-carbonyl)phenyl)-3-oxomorpholin-2-yl)-2-hydroxyacetamide.
(R)-N-(3-aminobenzo[d]isoxazol-6-yl)-2-((R)-4-(4-chloro-3-(4,4-difluoropiperidine-1-carbonyl)phenyl)-3-oxomorpholin-2-yl)-2-hydroxyacetamide,
(R)-N-(3-aminobenzo[d]isoxazol-6-yl)-2-((R)-4-(3-fluoro-4-(trifluoromethyl)phenyl)-3 -oxomorpholin-2-yl)-2-hydroxyacetamide,
(R)-N-(4-carbamimidoyl-3-fluorophenyl)-2-hydroxy-2-((R)-3-oxo-4-(3-(3-oxomorphol ino)phenyl)morpholin-2-yl)acetamide,
(R)-2-hydroxy-N-(1-imino-7-(trifluoromethyl)isoindolin-5-yl)-2-((R)-3-oxo-4-(3-(3-ox omorpholino)phenyl)morpholin-2-yl)acetamide,
(*R*)-2-Hydroxy-N-(1-iminoisoindolin-5-yl)-2-((*R*)-3-oxo-4-(3-(trifluoromethoxy)phenyl )morpholin-2-yl)acetamide,
Ethyl 2-(3-((R)-2-((*R*)-1-hydroxy-2-(1-iminoisoindolin-5-ylamino)-2-oxoethyl)-3-oxomorpho lino)phenoxy)acetate,
(*R*)-2-Hydroxy-*N*-(1-iminoisoindolin-5-yl)-2-((*R*)-4-(4-methoxybenzyl)-3-oxomorpholi n-2-yl)acetamide,
(*R*)-2-((*R*)-4-(3-(Benzyloxy)phenyl)-3-oxomorpholin-2-yl)-2-hydroxy-N-(1-iminoisoin dolin-5-yl)acetamide,
(*R*)-*N*-(1-Aminoisoquinolin-6-yl)-2-((*R*)-4-(4-fluoro-3-(morpholine-4-carbonyl)phenyl) -3-oxomorpholin-2-yl)-2-hydroxyacetamide,
N-(2,3-Dihydro-1-imino-1h-isoindol-5-yl)-4-[2-[(dimethylamino)carbonyl]phenyl]-alp ha(R)-hydroxy-3-oxo-2(R)-morpholineacetamide,
N-(2,3-Dihydro-1-imino-1H-isoindol-5-yl)-alpha(R)-hydroxy-3-oxo-4-[2-(1-pyrrolidin ylcarbonyl)phenyl]-2(R)-morpholineacetamide,
4-(2-Cyanophenyl)-N-(2,3-dihydro-1-imino-1h-isoindol-5-yl)-alpha(R)-hydroxy-3-oxo -2(R)-morpholineacetamide,
N-(2,3-Dihydro-1-imino-1h-isoindol-5-yl)-4-[4-fluoro-3-(4-morpholinylcarbonyl)phen yl]-alpha(R)-hydroxy-3-oxo-2(R)-morpholineacetamide,
N-(2,3-Dihydro-1-imino-1h-isoindol-5-yl)-4-[4-fluoro-3-[(3(R)-methyl-4-morpholinyl) carbonyl]phenyl]-alpha(R)-hydroxy-3-oxo-2(R)-morpholineacetamide,
N-(2,3-Dihydro-1-imino-1h-isoindol-5-yl)-4-[4-fluoro-3-[(3(S)-methyl-4-morpholinyl) carbonyl]phenyl]-alpha(R)-hydroxy-3-oxo-2(R)-morpholineacetamide,
N-[4-(Aminoiminomethyl)phenyl]-4-(2-cyanophenyl)-alpha(R)-hydroxy-3-oxo-2(R)-m orpholineacetamide,
N-(4-Amino-7-quinazolinyl)-4-[4-fluoro-3-(4-morpholinylcarbonyl)phenyl]-alpha(R)-h ydroxy-3-oxo-2(R)morpholineacetamide,
[3-[2(R)-[2-[(3-Amino-1,2-benzisoxazol-6-yl)amino]-1(R)-hydroxy-2-oxoethyl]-3-oxo-4-morpholinyl]phenyl]pentafluorosulfur,
[4-[2(R)-[2-[(3-Amino-1,2-benzisoxazol-6-yl)amino]-1(R)-hydroxy-2-oxoethyl]-3-oxo-4-morpholinyl]phenyl]pentafluorosulfur,
N-(3-Amino-5-fluoro-1,2-benzisoxazol-6-yl)-4-[4-fluoro-3-(4-morpholinylcarbonyl)ph enyl]-alpha(R)-hydroxy-3-oxo-2(R)-morpholineacetamide.
N-(2-Amino-6-quinazolinyl)-4-[4-fluoro-3-(4-morpholinylcarbonyl)phenyl]-alpha(R)-h ydroxy-3-oxo-2(R)-morpholineacetamide,
(R)-2-((R)-4-(5-fluoro-2-isopropoxyphenyl)-3-oxomorpholin-2-yl)-2-hydroxy-N-(1-imi noisoindolin-5-yl)acetamide,
(R)-2-hydroxy-N-(1-iminoisoindolin-5-yl)-2-((R)-3-oxo-4-(2-(trifluoromethyl)phenyl) morpholin-2-yl)acetamide,
(R)-2-((R)-4-(2-(difluoromethoxy)phenyl)-3-oxomorpholin-2-yl)-2-hydroxy-N-(1-imin oisoindolin-5-yl)acetamide,
(R)-2-hydroxy-N-(1-iminoisoindolin-5-yl)-2-((R)-4-(2-isopropoxyphenyl)-3-oxomorph olin-2-yl)acetamide,
(R)-2-hydroxy-N-(1-iminoisoindolin-5-yl)-2-((R)-3-oxo-4-(2-(trifluoromethoxy)phenyl )merpholin-2-yl)acetamide,
*N*-(3-Amino-1,2-benzisoxazol-6-yl)4-[4-fluoro-3-[(tetrahydro-2*H*-pyran-4-yl)oxy]phe nyl]-alpha(R)-hydroxy-3-oxo-2(R)-morpholineacetamide,
N-(3-Amino-1,2-benzisoxazol-6-yl)-4-[3-[(3,3-difluoro-1-azetidinyl)carbonyl]-4-fluoro phenyl]-alpha(R)-hydroxy-3-oxo-2(R)-morpholineacetamide,
*N*-(3-Amino-1,2-benzisoxazol-6-yl)-4-[3-[(4,4-difluoro-1-piperidinyl)carbonyl]-4-fluor ophenyl]-alpha(R)-hydroxy-3-oxo-2(R)-morpholineacetamide,
N-(3-amino-1,2-benzisoxazol-6-yl)4-[4-chloro-3-[3-(1,1-dimethylethyl)-1,2,4-oxadiaz ol-5-yl]phenyl]-alpha(R)-hydroxy-3-oxo-2(R)-morpholineacetamide,
N-(3-amino-1,2-benzisoxazol-6-yl)-4-[2-fluoro-5-(trifluoromethyl)phenyl]-alpha(R)-hy droxy-3-oxo-2(R)-morpholineacetamide,
(R)-N-(4-carbamimidoylphenyl)-2-hydroxy-2-((R)-4-(3-((S)-2-methylpyrrolidine-1-car bonyl)phenyl)-3-oxomorpholin-2-yl)acetamide,
(R)-N-(4-carbamimidoylphenyl)-2-hydroxy-2-((R)-4-(2-methyl-3-(morpholine-4-carbo nyl)phenyl)-3-oxomorpholin-2-yl)acetamide,
(R)-N-(4-carbamimidoylphenyl)-2-hydroxy-2-((R)-4-(2-methyl-3-(pyrrolidine-1-carbo nyl)phenyl)-3-oxomorpholin-2-yl)acetamide,
(R)-2-((R)-4-(3-(azetidine-1-carbonyl)-5-fluorophenyl)-3-oxomorpholin-2-yl)-N-(4-car bamimidoylphenyl)-2-hydroxyacetamide,
(R)-2-((R)-4-(3-(azetidine-1-carbonyl)-2-methylphenyl)-3-oxomorpholin-2-yl)-N-(4-ca rbamimidoylphenyl)-2-hydroxyacetamide,
(R)-2-((R)-4-(5-(azetidine-1-carbonyl)-2-methylphenyl)-3-oxomorpholin-2-yl)-N-(4-ca rbamimidoylphenyl)-2-hydroxyacetamide,
(R)-N-(4-carbamimidoylphenyl)-2-hydroxy-2-((R)-3-oxo-4-(6-(trifluoromethyl)pyridin -2-yl)morpholin-2-yl)acetamide,
(R)-N-(4-carbamimidoylphenyl)-2-hydroxy-2-((R)-4-(2-methyl-5-(pyrrolidine-1-carbo nyl)phenyl)-3-oxomorpholin-2-yl)acetamide,
(R)-2-((R)-4-(3-(azetidine-1-carbonyl)-5-methylphenyl)-3-oxomorpholin-2-yl)-N-(4-ca rbamimidoylphenyl)-2-hydroxyacetamide,
(R)-N-(4-carbamimidoylphenyl)-2-hydroxy-2-((R)-4-(2-methyl-5-(morpholine-4-carbo nyl)phenyl)-3-oxomorpholin-2-yl)acetamide,
(S)-1-(3-((R)-2-((R)-2-(4-carbamimidoylphenylamino)-1-hydroxy-2-oxoethyl)-3-oxom orpholino)-5-fluorobenzoyl)pyrrolidine-2-carboxylic acid,
1-(3-((R)-2-((R)-2-(4-carbamimidoylphenylamino)-1-hydroxy-2-oxoethyl)-3-oxomorp holino)-5-fluorobenzoyl)pyrrolidine-2-carboxylate,
1-(3-((R)-2-((R)-2-(4-carbamimidoylphenylamino)-1-hydroxy-2-oxoethyl)-3-oxomorp holino)-5-fluorobenzoyl)pyrrolidine-2-carboxylate,
2-(3-((R)-2-((R)-2-(4-carbamimidoylphenylamino)-1-hydroxy-2-oxoethyl)-3-oxomorp holino)-5-fluoro-N-methylbenzamido)acetic acid,
2-(3-((R)-2-((R)-2-(4-carbamimidoylphenylamino)-1-hydroxy-2-oxoethyl)-3-oxomorp holino)-5-fluoro-N-methylbenzamido)acetate,
3-((R)-2-((R)-2-(4-carbamimidoylphenylamino)-1-hydroxy-2-oxoethyl)-3-oxomorpholi no)-N-(2-(dimethylamino)-2-oxoethyl)-5-fluoro-N-methylbenzamide,
(R)-N-(1,3-diiminoisoindolin-5-yl)-2-hydroxy-2-((R)-4-(3-(2-(4-methyl-3-oxopiperazin -1-yl)-2-oxoethyl)phenyl)-3-oxomorpholin-2-yl)acetamide,
N-(2-(dimethylamino)-2-oxoethyl)-3-((R)-2-((R)-1-hydroxy-2-(1-iminoisoindolin-5-yla mino)-2-oxoethyl)-3-oxomorpholino)-N-methylbenzamide,
2-((S)-hydroxy((R)-3-oxo-4-p-tolylmorpholin-2-yl)methyl)-4-oxo-1,4-dihydroquinazoli ne-6-carboximidamide,
1,1-dioxo-3-((S)-hydroxy((R)-3-oxo-4-p-tol(Example ylmorpholin-2-yl)methyl)-4H-benzo[e][1,2,4]thiadiazine-7-carboximidamide,
(R)-N-(3-aminobenzo[d]isoxazol-6-yl)-2-((R)-4-(4-chloro-3-(morpholinomethyl)pheny l)-3-oxomorpholin-2-yl)-2-hydroxyacetamide,
(R)-N-(3-aminobenzo[d]isoxazol-6-yl)-2-hydroxy-2-((R)-4-(3-imorpholine-4-carbonyl) phenyl)-3-oxomorpholin-2-yl)acetamide,
3-((R)-2-((R)-2-(4-carbamimidoyl-3-fluorophenylamino)-1-hydroxy-2-oxoethyl)-3-oxo morpholino)benzoic acid,
(R)-N-(4-carbamimidoyl-3,5-difluorophenyl)-2-hydroxy-2-((R)-3-oxo-4-(3-(3-oxomor pholino)phenyl)morpholin-2-yl)acetamide,
(R)-N-(4-carbamimidoyl-2,3-difluorophenyl)-2-hydroxy-2-((R)-3-oxo-4-(3-(3-oxomor pholino)phenyl)morpholin-2-yl)acetamide,
(R)-N-(4-carbamimidoyl-2,5-difluorophenyl)-2-hydroxy-2-((R)-3-oxo-4-(3-(3-oxomor pholino)phenyl)morpholin-2-yl)acetamide,
(R)-N-(3-(aminomethyl)-4-cyano-5-(trifluoromethyl)phenyl)-2-hydroxy-2-((R)-3-oxo-4-(3-(3-oxomorpholino)phenyl)morpholin-2-yl)acetamide,
(*R*)-2-Hydroxy-N-(1-iminoisoindolin-5-yl)-2-((*R*)-4-(2-methyl-1-oxoisoindolin-5-yl)-3-oxomorpholin-2-yl)acetamide,
(*R*)-2-Hydroxy-*N*-(1-iminoisoindolin-5-yl)-2-((*R*)-4-(2-methyl-3-oxoisoindolin-5-yl)-3-oxomorpholin-2-yl)acetamide hydrochloride,
(*R*)-2-((*R*)-4-(2-(Cyclopropylmethyl)-3-oxoisoindolin-5-yl)-3-oxomorpholin-2-yl)-2-hy droxy-N-(1-iminoisoindolin-5-yl)acetamide,
(*R*)-N-(4-(Aminomethyl)-3-fluorophenyl)-2-hydroxy-2-((*R*)-3-oxo-4-(3-(3-oxomorphol ino)phenyl)morpholin-2-yl)acetamide,
(*R*)-*N*-(4-Guanidinophenyl)-2-hydroxy-2-((*R*)-3-oxo-4-(3-(3-oxomorpholino)phenyl)m orpholin-2-yl)acetamide,
(*R*)-N-(4-(Aminomethyl)phenyl)-2-((*R*)-4-(4-fluoro-3-(morpholine-4-carbonyl)phenyl)-3-oxomorpholin-2-yl)-2-hydroxyacetamide,
(*R*)-2-(4-Carbamoylphenylamino)-1-((*R*)-4-(4-fluoro-3-(morpholine-4-carbonyl)phenyl )-3-oxomorpholin-2-yl)-2-oxoethyl acetate,
(*R*)-2-((*R*)-4-(4-Fluoro-3-(morpholine-4-carbonyl)phenyl)-3-oxomorpholin-2-yl)-2-hyd roxy-N-(1,2,3,4-tetrahydroisoquinolin-6-yl)acetamide,
(*R*)-*N*-(4-(Aminomethyl)-3-fluorophenyl)-2-((*R*)-4-(4-fluoro-3-(morpholine-4-carbonyl )phenyl)-3-oxomorpholin-2-yl)-2-hydroxyacetamide,
(*R*)-*N*-(4-((*R*)-1-Aminoethyl)phenyl)-2-((*R*)-4-(4-fluoro-3-(morpholine-4-carbonyl)phe nyl)-3-oxomorpholin-2-yl)-2-hydroxyacetamide,
(*R*)-*N*-((*R*)-1-amino-2,3-dihydro-1H-inden-5-yl)-2-((*R*)-4-(4-fluoro-3-(morpholine-4-ca rbonyl)phenyl)-3-oxomorpholin-2-yl)-2-hydroxyacetamide,
(*R*)-*N*-((*S*)-1-Amino-2,3-dihydro-1*H*-inden-5-yl)-2-((*R*)-4-(4-fluoro-3-(morpholine-4-ca rbonyl)phenyl)-3-oxomorpholin-2-yl)-2-hydroxyacetamide,
(*R*)-*N*-(2-Aminoquinolin-6-yl)-2-((*R*)4-(4-fluoro-3-(morpholine-4-carbonyl)phenyl)-3-oxomorpholin-2-yl)-2-hydroxyacetamide,
N-(7-fluoro-2,3-dihydro-1-imino-1H-isoindol-5-yl)-alpha(R)-hydroxy-3-oxo-4-[3-(3-o xo-4-morpholinyl)phenyl]-2(R)-morpholineacetamide,
N-(4-chloro-2,3-dihydro-1-imino-1H-isoindol-5-yl)-alpha(R)-hydroxy-3-oxo-4-[3-(3-o xo-4-morpholinyl)phenyl]-2(R)-morpholineacetamide,
3-[2(R)-[2-[(2,3-Dihydro-1-imino-1h-isoindol-5-yl)amino]-1(R)-hydroxy-2-oxoethyl]-3-oxo-4-morpholinyl]benzoic acid,
Methyl 3-[2(R)-[2-[(2,3-dihydro-1-imino-1H-isoindol-5-yl)amino]-1(R)-hydroxy-2-oxoethyl]-3-oxo-4-morpholinyl]benzoate,
Methyl 2-[2(R)-[2-[(2,3-dihydro-1-imino-1h-isoindol-5-yl)amino]-1(R)-hydroxy-2-oxoethyl]-3 -oxo-4-morpholinyl]benzoate,
N-(3-Amino-1,2-benzisoxazol-6-yl)-4-[4-fluoro-3-[2,2,2-trifluoro-1-(4-morpholinyl)eth yl]phenyl]-alpha(R)-hydroxy-3-oxo-2(R)-morpholineacetamide,
(R)-2-((R)-4-(5-chloro-2-isopropoxyphenyl)-3-oxomorpholin-2-yl)-2-hydroxy-N(1-im inoisoindolin-5-yl)acetamide, or
*N*-(3-Amino-1,2-benzisoxazol-6-yl)-4-[3-(difluoromethoxy)-4-fluorophenyl]-alpha(R)-hydroxy-3-oxo-2(R)-morpholineacetamide,
or a pharmaceutically acceptable salt thereof.

5. A compound which is 2-((S)-hydroxy((R)-3-oxo-4-p-tolylmorpholin-2-yl)methyl)-4-oxo-1,4-dihydroquinazoline-6-carboximidamide, or 1,1-dioxo-3-((S)-hydroxy((R)-3-oxo-4-p-tolylmorpholin-2-yl)methyl)-4H-benzo[e][1,2,4]thiadiazine-7-c arboximidamide, or a pharmaceutically acceptabte salt thereof.

6. A pharmaceutical composition comprising at least one compound of any one of claims 1 to 5, or a pharmaceutically acceptable salt thereof and at least one pharmaceutically acceptable carrier.

7. A compound of any one of claims 1 to 5, or a pharmaceutically acceptable salt thereof for use in treating a disorder or disease mediated by factor IXa.

8. A compound or a pharmaceutically acceptable salt thereof for use of claim 7, wherein said disorder or disease is a thromboembolic disorder.

9. A compound or a pharmaceutically acceptable salt thereof for use of claim 8, wherein the thromboembolic disorder is selected from the group consisting of arterial cardiovascular thromboembolic disorders, venous cardiovascular thromboembolic disorders, and thromboembolic disorders in the chambers of the heart.

10. A compound or a pharmaceutically acceptable salt thereof for use of claim 8, wherein the thromboembolic disorder is selected from unstable angina, an acute coronary syndrome, atrial fibrillation, first myocardial infarction, recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from (a) prosthetic valves or other implants, (b) indwelling catheters, (c) stents, (d) cardiopulmonary bypass, (e) hemodialysis, or (f) other procedures in which blood is exposed to an artificial surface that promotes thrombosis.

11. A combination of a compound of any one of claims 1-5, or a pharmaceutically acceptable salt thereof and at least one anticoagulant agent selected from the group consisting of a thrombin inhibitor, a thrombin receptor (PAR-1) antagonist, a factor VIIa inhibitor, factor VIIIa inhibitor, a factor IXa inhibitor different from the compound of claims 1-5, a factor Xa inhibitor, a factor XIa inhibitor, TAFI and fibrinogen inhibitors.

12. The combination of claim 11, wherein said factor LXa inhibitor is selected from the group consisting of monoclonal antibodies, synthetic active siteblocked competitive inhibitors, oral inhibitors, and RNA aptamers.

13. A pharmaceutical composition comprising: a therapeutically effective amount of at least one compound of any one of claims 1-5 or a pharmaceutically acceptable salt thereof, and at least one pharmaceutically acceptable carrier, and an effective amount of at least one agent selected from the group sonsisiting of : (a) anticoagulants, (b) anti-thrombin agents, (c) anti-platelet agents, (d) fibrinolytics, (e) hypolipidemic agents, (f) antihypertensive agents, and (g) anti-ischemic agents.

14. A pharmaceutical composition comprising:
a therapeutically effective amount of at least one compound of any one of claims 1-5 or a pharmaceutically acceptable salt thereof, and at least one pharmaceutically acceptable carrier, and an effective amount of at least one agent selected from the group sonsisiting of(a-1) warfarin, (a-2) heparin, (a-3) aprotinin, (a-4) synthetic pentasaccharide, (a-5) direct acting thrombin inhibitors including hirudin and argatroban, (a-6) a factor VIIa inhibitor, (a-7) a factor VIIIa inhibitor, (a-8) a factor IXa inhbitor different from the compounds of Formula (I), (a-9) a factor Xa inhibitor, (a-10) a factor XIa inhibitor, (a-11) a thrombin inhibitor, (a-12) a TAFI, (a-13) a fibrinogen inhibitor, (b-1) a boroarginine derivative, (b-2) a boropeptide, (b-3) heparin, (b-4) hirudin, (b-5) argatroban, (c-1) a NSAID, (c-2) a IIb/IIIa antagonist, (c-3) a thromboxane-A2-receptor antagonist, (c-4) a thromboxane-A2-synthetase inhibitor, (c-5) a PDE-III inhibitor, (c-6) a PDE V inhibitor, (c-7) a ADP receptor antagonist, (c-8) an antagonist of the purinergic receptor P2Y1, (c-9) an antagonist of the purinergic receptor P2Y 12, (d-1) tissue plasminogen activator (TPA, natural or recombinant) and modified forms thereof, (d-2) anistreplase, (d-3) urokinase, (d-4) streptokinase, (d-5) tenecteplase (TNK), (d-6) lanoteplase (nPA), (d-7) a factor VIIa inhibitor, (d-8) a PAI-I inhibitor, (d-9) an alpha-2-antiplasmin inhibitor, (d-10) an anisoylated plasminogen streptokinase activator complex, (c-1) a HMG-CoA reductase inhibitor, (e-2) a squalene synthetase inhibitor, (e-3) a fibrate, (e-4) a bile acid sequestrant, (e-5) an ACAT inhibitor, (e-6) a MTP inhibitor, (e-7) a lipooxygenase inhibitor, (e-8) a cholesterol absorption inhibitor, (e-9) a cholesterol ester transfer protein inhibitor, (f-1) an alpha adrenergic blocker, (f-2) a beta adrenergic blocker, (f-3) a calcium channel blocker, (f-4)a diuretics, (f-5) a rennin inhibitor, (f-6) an angiotensin-converting enzyme inhibitor, (f-7) an angiotensin-II-receptor antagnonist, (f-8) an ET receptor antagonist, (f-9) a Dual ET/All antagonist, (f-10) a neutral endopeptidase inhibitors, (f-11) a vasopepsidase inhibitor, (g-1) a Class I agent, (g-2) a Class II agent, (g-3) a Class III agent, a (g-4) Class IV agent, (g-5) a K+ cannel opener, (g-6) an IKur inhibitor and (g-7) a cardiac glycoside.

15. A combination of a compound of any one of claims 1-5, or a pharmaceutically acceptable salt thereof and one or more additional therapeutic agents.

16. A compound of any one of claims 1-5, or a pharmaceutically acceptable salt thereof for use in therapy.

## Patentansprüche

1. Eine Verbindung der Formel (I) 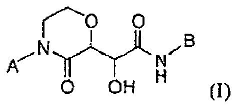 oder ein pharmazeutisch annehmbares Salz davon, wobei
A 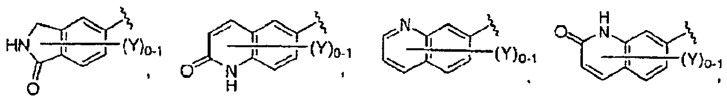 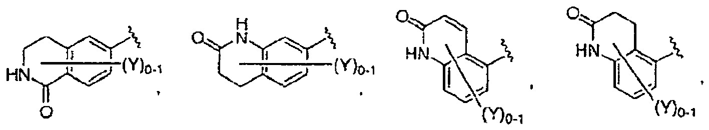 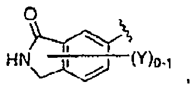 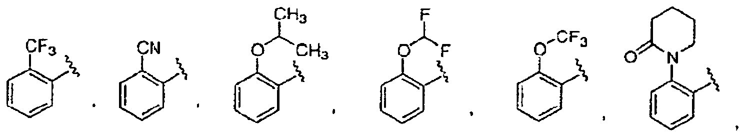 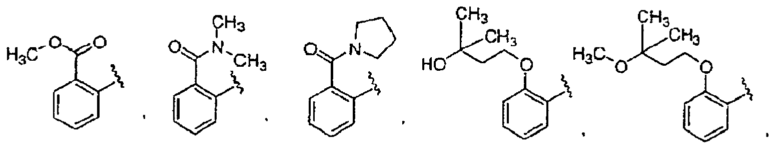 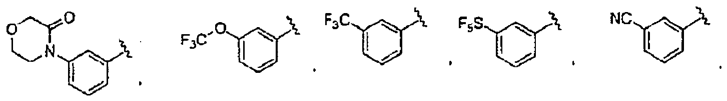 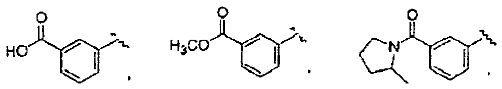 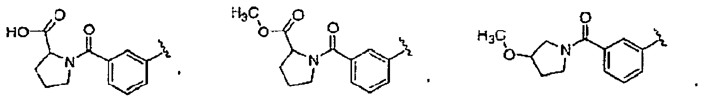 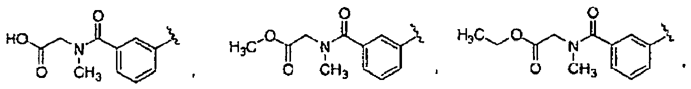 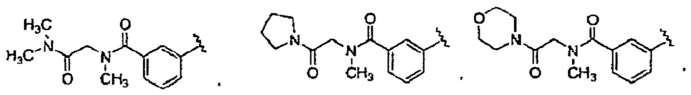 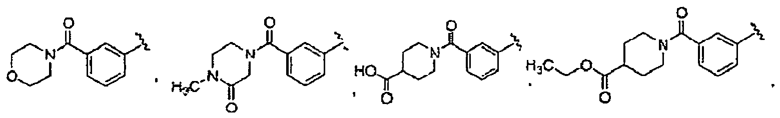 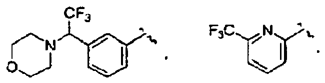 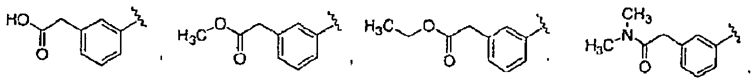 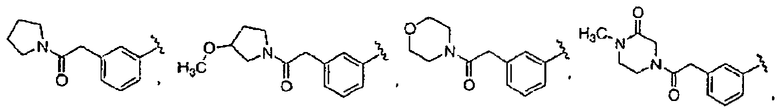 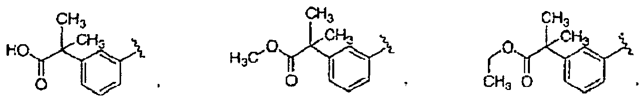 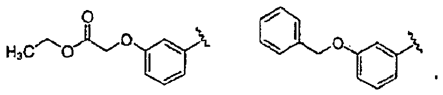 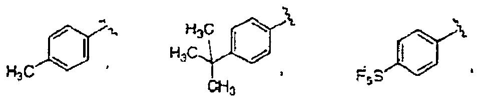 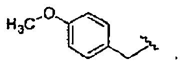 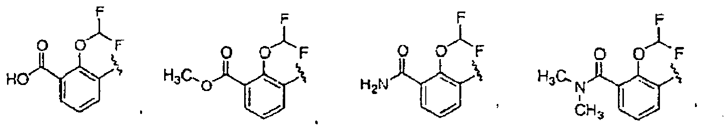 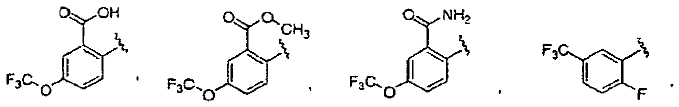 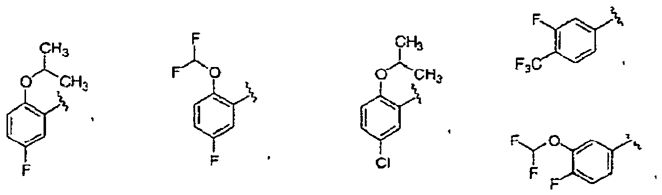 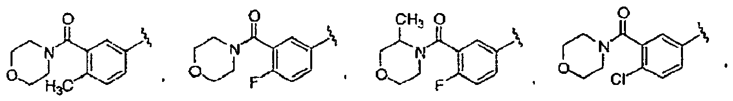 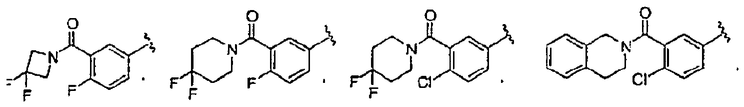 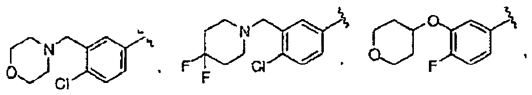 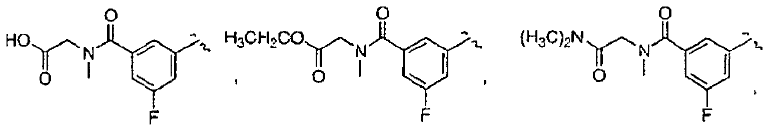 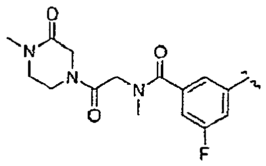 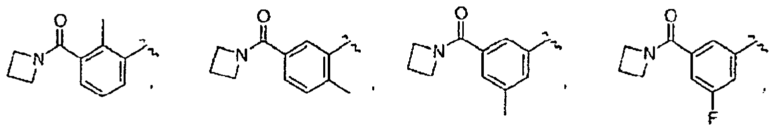 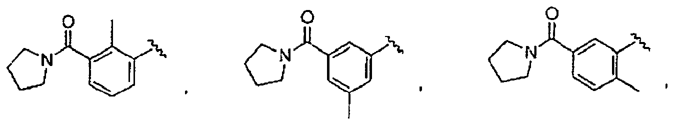 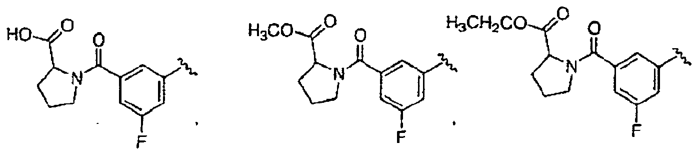 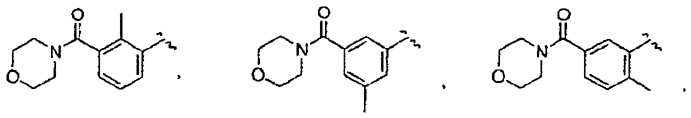 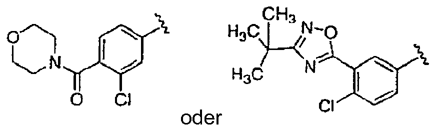 ist,
Y, das an ein Kohlenstoff- oder Stickstoff-Ringatom gebunden ist,
1) Halogen,
2) -(C₁-C₆)-Alkyl,
3) -(C₁-C₃)-Halogenalkyl,
4) -(C₃-C₈)-Cycloalkyl,
5) -OH,
6) -O-(C₁-C₆)-Alkyl,
7) -O-(C₁-C₃)-Halogenalkyl,
8) =O (Oxo) ist,
wobei der -(C₁-C₆)-Alkylteil von 2) und 6) des Restes Y unsubstituiert oder unabhängig substituiert ist mit den Substituenten, ausgewählt aus der Gruppe, bestehend aus -(C₃-C₈)-Cycloalkyl, -C(O)OH und -C(O)-(C₁-C₆)-Alkyl,
B 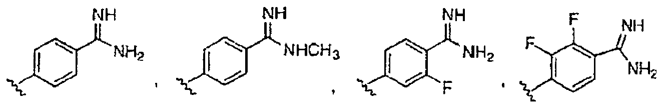 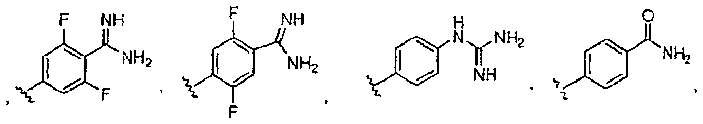 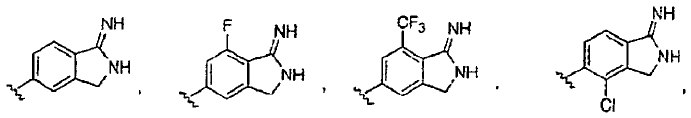 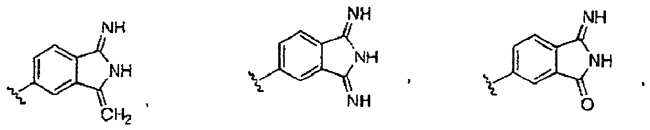 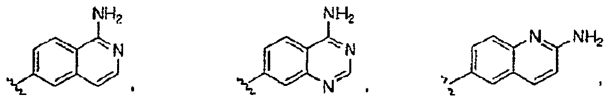 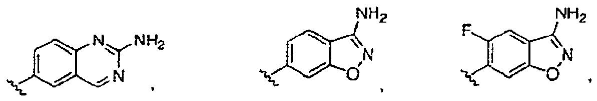 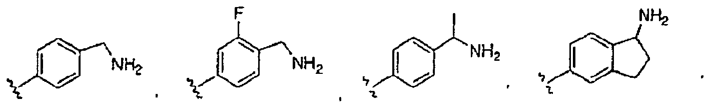 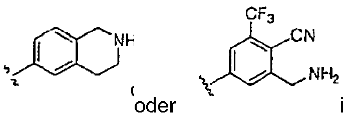 ist,
mit der Maßgabe, dass, wenn A 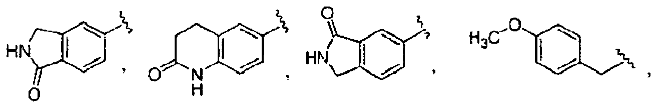 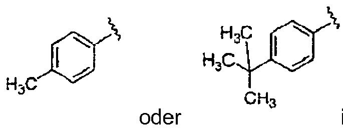 ist,
B nicht 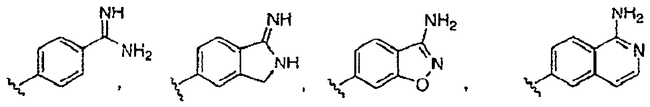 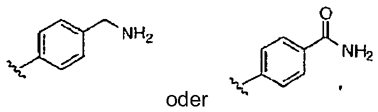 ist, und,
wenn A 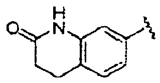 ist, B nicht 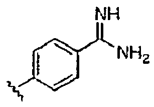 ist.

2. Eine Verbindung der Formel (I) gemäß Anspruch 1 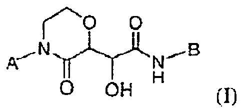 oder ein pharmazeutisch annehmbares Salz davon, wobei
A 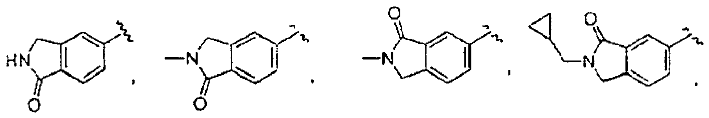 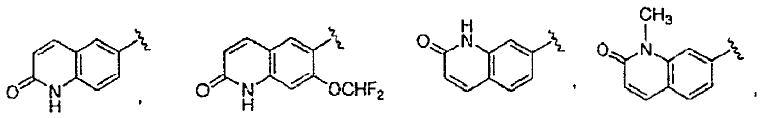 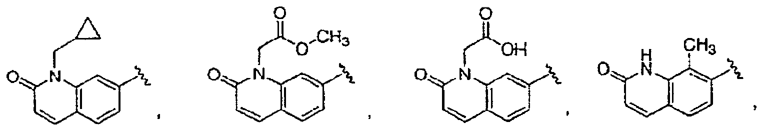 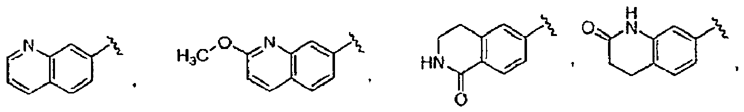 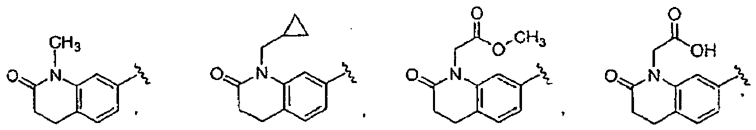 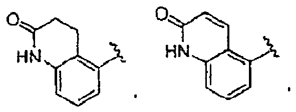 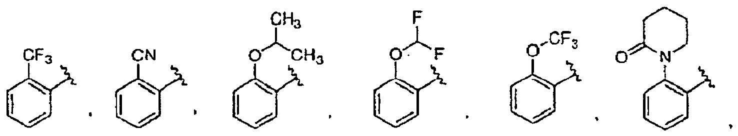 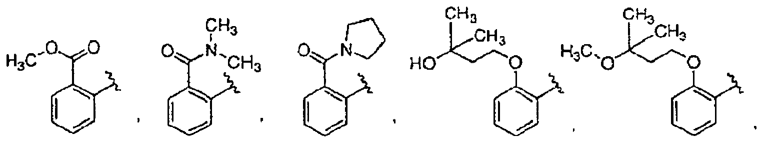 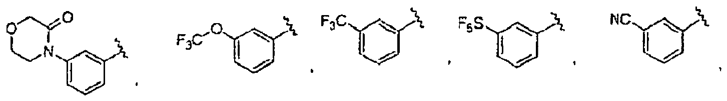 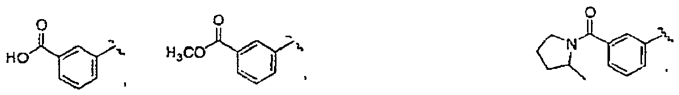 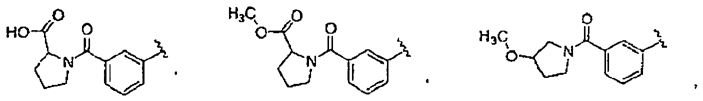 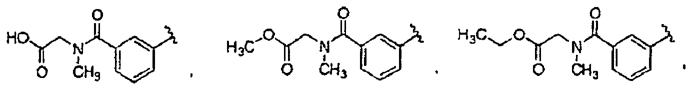 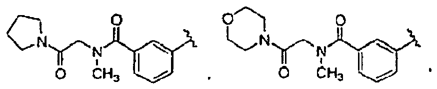 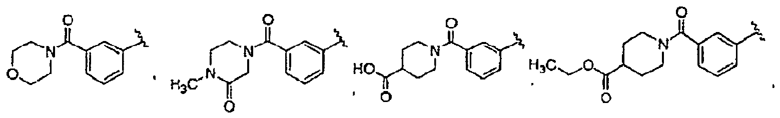 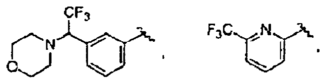 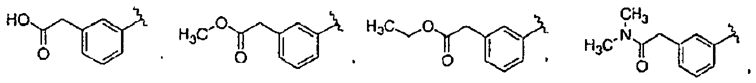 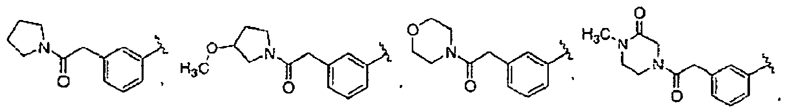 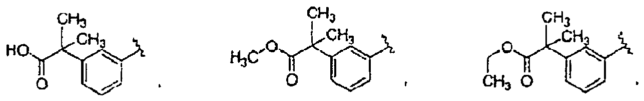 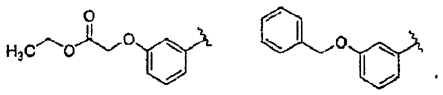 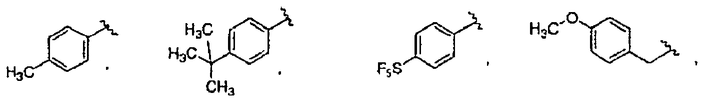 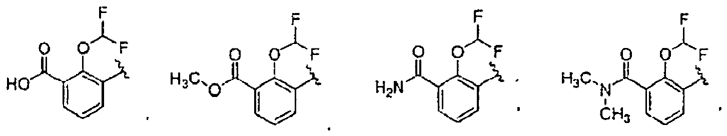 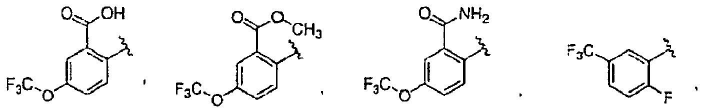 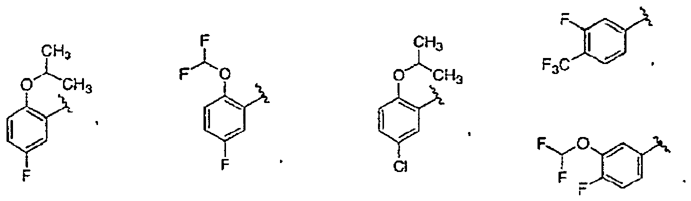 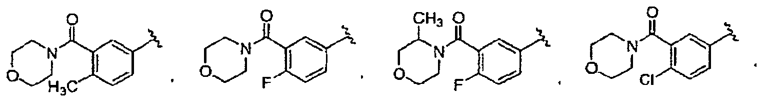 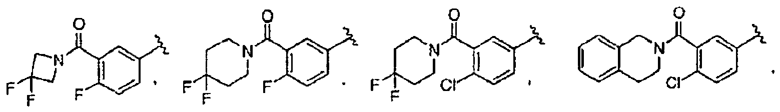 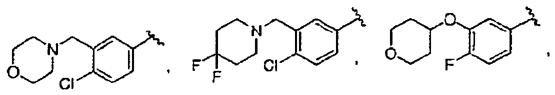 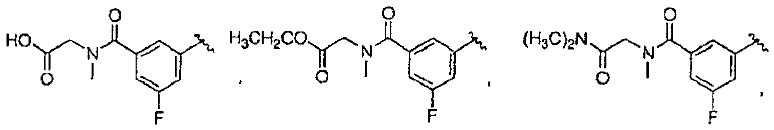 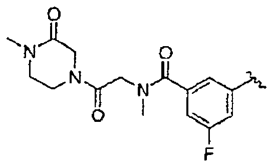 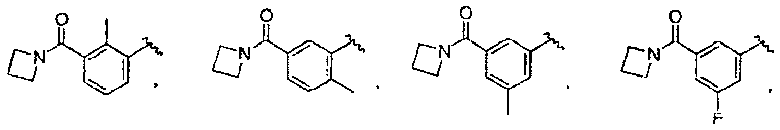 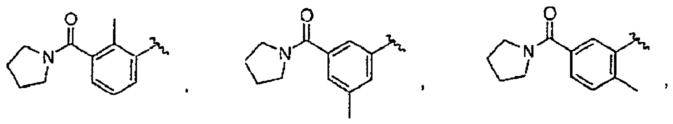 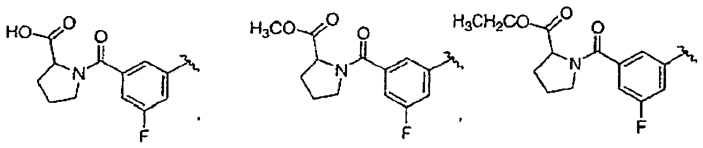 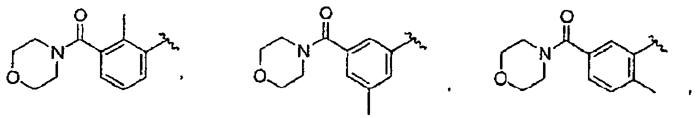 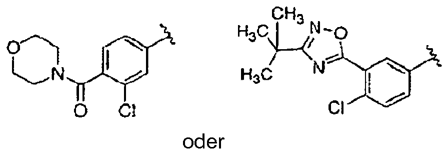 ist, B 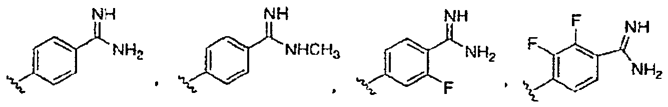 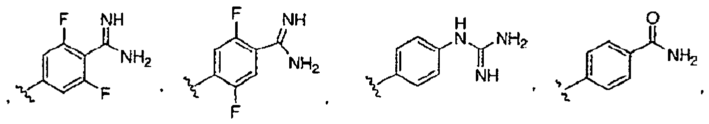 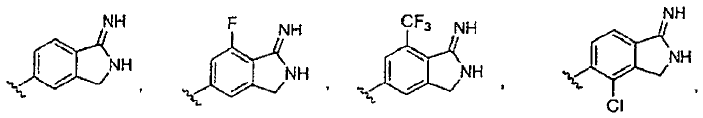 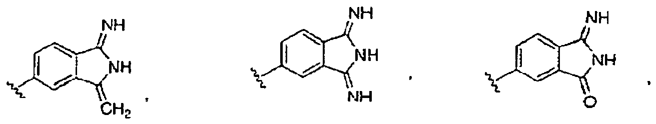 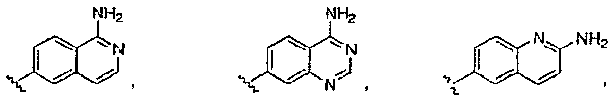 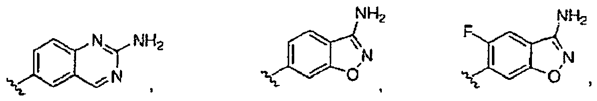 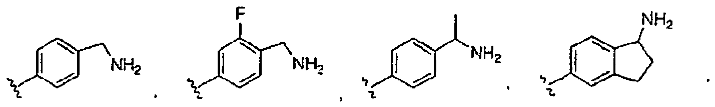 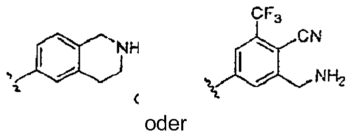 ist,
mit der Maßgabe, dass,
wenn A 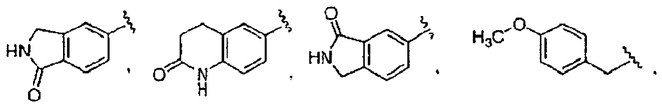 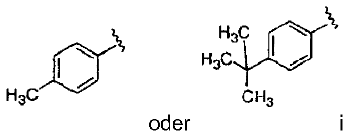 ist,
B nicht 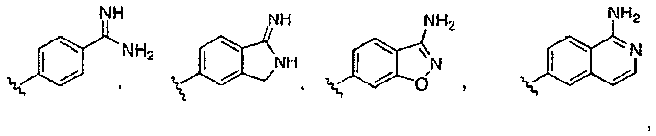 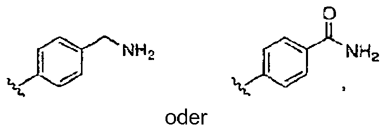 ist, und
wenn A 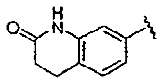 ist, B nicht 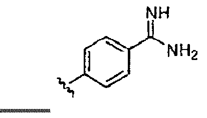 ist.

3. Eine Verbindung nach Anspruch 1 oder 2 oder ein pharmazeutisch annehmbares Salz davon, wobei die absolute Konfiguration von Formel (I) 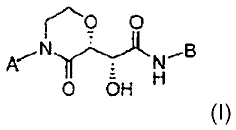 ist.

4. Eine Verbindung nach Anspruch 1, die ist
(2R)-2-[(2R)-4-(4-tert-Butylphenyl)-3-oxomorpholin-2-yl]-2-hydroxy-N-[4-(N-methylcarbamimidoyl)phenyl]acetamid,
(2R)-2-Hydroxy-N-(1-imino-2,3-dihydroisoindol-5-yl)-2-[(2R)-3-oxo-4-(1-oxo-2,3-dihydroisoindol-5-yl)morpholin-2-yl]acetamid,
(2R)-2-Hydroxy-N-(1-imino-2,3-dihydroisoindol-5-yl)-2-[(2R)-3-oxo-4-(2-oxo-1H-chinolin-6-yl)morpholin-2-yl]acetamid,
(2R)-2-[(2R)-4-[7-(Difluormethoxy)-2-oxo-1H-chinolin-6-yl]-3-oxomorpholin-2-yl]-2-hydroxy-N-(1-imino-2,3-dihydroisoindol-5-yl)acetamid,
(2R)-N-(4-Carbamimidoylphenyl)-2-hydroxy-2-[(2R)-3-oxo-4-(2-oxo-1H-chinolin-6-yl)morpholin-2-yl]acetamid,
(2R)-N-(4-Carbamimidoyl-3-fluorphenyl)-2-hydroxy-2-[(2R)-3-oxo-4-(2-oxo-1H-chinolin-6-yl)morpholin-2-yl]acetamid,
(2R)-2-Hydroxy-N-(1-imino-2,3-dihydroisoindol-5-yl)-2-[(2R)-3-oxo-4-(2-oxo-1H-chinolin-7-yl)morpholin-2-yl]acetamid,
(2R)-2-Hydroxy-N-(1-imino-2,3-dihydroisoindol-5-yl)-2-[(2R)-4-(1-methyl-2-oxochinolin-7-yl)-3-oxomorpholin-2-yl]acetamid,
(2R)-2-[(2R)-4-[1-(Cyclopropylmethyl)-2-oxochinolin-7-yl]-3-oxomorpholin-2-yl]-2-hydroxy-N-(1-imino-2,3-dihydroisoindol-5-yl)acetamid,
2-[7-[(2R)-2-[(1R)-1-Hydroxy-2-[(1-imino-2,3-dihydroisoindol-5-yl)amino]-2-oxoethyl]-3-oxomorpholin-4-yl]-2-oxochinolin-1-yl]acetat,
2-[7-[(2R)-2-[(1R)-1-Hydroxy-2-[(1-imino-2,3-dihydroisoindol-5-yl)amino]-2-oxoethyl]-3-oxomorpholin-4-yl]-2-oxochinolin-1-yl]essigsäure,
(2R)-2-Hydroxy-N-(1-imino-2,3-dihydroisoindol-5-yl)-2-[(2R)-4-(8-methyl-2-oxo-1H-chinolin-7-yl)-3-oxomorpholin-2-yl]acetamid,
(2R)-2-Hydroxy-N-(1-imino-2,3-dihydroisoindol-5-yl)-2-[(2R)-4-(2-methoxychinolin-7-yl)-3-oxomorpholin-2-yl]acetamid,
(2R)-2-Hydroxy-N-(1-imino-2,3-dihydroisoindol-5-yl)-2-[(2R)-3-oxo-4-chinolin-7-ylmorpholin-2-yl]acetamid,
(2R)-N-(3-Amino-1,2-benzisoxazol-6-yl)-2-hydroxy-2-[(2R)-3-oxo-4-chinolin-7-ylmorpholin-2-yl]acetamid,
(2R)-2-hydroxy-N-(1-imino-2,3-dihydroisoindol-5-yl)-2-[(2R)-3-oxo-4-(2-oxo-1H-chinolin-5-yl)morpholin-2-yl]acetamid,
(2R)-2-Hyd roxy-N-(1-imino-2,3-dihydroisoindol-5-yl)-2-[(2R)-3-oxo-4-(1-oxo-3,4-dihydro-2H-isochinolin-6-yl)morpholin-2-yl]acetamid,
(2R)-2-Hydroxy-N-(1-imino-2,3-dihydroisoindol-5-yl)-2-[(2R)-3-oxo-4-(2-oxo-3,4-dihydro-1H-chinolin-7-yl)morpholin-2-yl]acetamid,
(2R)-2-Hyd roxy-N-(1-imino-2,3-dihydroisoindol-5-yl)-2-[(2R)-4-(1-methyl-2-oxo-3,4-dihydrochinolin-7-yl)-3-oxomorpholin-2-yl]acetamid,
(2R)-2-[(2R)-4-[1-(Cyclopropylmethyl)-2-oxo-3,4-dihydrochinolin-7-yl]-3-oxomorpholin-2-yl]-2-hyd roxy-N-(1-imino-2,3-dihydroisoindol-5-yl)acetamid,
Methyl-2-[7-[(2R)-2-[(1R)-1-hydroxy-2-[(1-imino-2,3-dihydroisoindol-5-yl)amino]-2-oxoethyl]-3-oxomorpholin-4-yl]-2-oxo-3,4-dihydrochinolin-1-yl]acetat,
2-[7-[(2R)-2-[(1R)-1-Hydroxy-2-[(1-imino-2,3-dihydroisoindol-5-yl)amino]-2-oxoethyl]-3-oxomorpholin-4-yl]-2-oxo-3,4-dihydrochinolin-1-yl]essigsäure,
(2R)-N-(4-Carbamimidoyl-3-fluorphenyl)-2-hydroxy-2-[(2R)-3-oxo-4-(2-oxo-3,4-dihydro-1H-chinolin-7-yl)morpholin-2-yl]acetamid,
(2R)-N-(3-Amino-1,2-benzoxazol-6-yl)-2-hydroxy-2-[(2R)-3-oxo-4-(2-oxo-3,4-dihydro-1H-chinolin-7-yl)morpholin-2-yl]acetamid,
(2R)-N-(1-Aminoisochinolin-6-yl)-2-hydroxy-2-[(2R)-3-oxo-4-(2-oxo-3,4-dihydro-1H-chinolin-7-yl)morpholin-2-yl]acetamid,
(2R)-N-(4-Aminochinazolin-7-yl)-2-hydroxy-2-[(2R)-3-oxo-4-(2-oxo-3,4-dihydro-1H-chinolin-7-yl)morpholin-2-yl]acetamid,
(2R)-2-Hydroxy-N-(1-imino-2,3-dihydroisoindol-5-yl)-2-[(2R)-3-oxo-4-(2-oxo-3,4-dihydro-1H-chinolin-5-yl)morpholin-2-yl]acetamid,
(2R)-2-Hydroxy-2-[(2R)-4-[2-(3-hydroxy-3-methylbutoxy)phenyl]-3-oxomorpholin-2-yl]-N-(1-imino-2,3-dihydroisoindol-5-yl)acetamid,
(2R)-2-Hyd roxy-N-(1-imino-2,3-dihydroisoindol-5-yl)-2-[(2R)-4-[2-(3-methoxy-3-methylbutoxy)phenyl]-3-oxomorpholin-2-yl]acetamid,
(2R)-2-Hyd roxy-N-(1-imino-2,3-dihydroisoindol-5-yl)-2-[(2R)-3-oxo-4-[2-(2-oxopiperidin-1-yl)phenyl]morpholin-2-yl]acetamid,
(2R)-2-[(2R)-4-[2-(Difluormethoxy)-5-fluorphenyl]-3-oxomorpholin-2-yl]-2-hydroxy-N-(1-imino-2,3-dihydroisoindol-5-yl)acetamid,
Methyl-2-(difluormethoxy)-3-[(2R)-2-[(1R)-1-hydroxy-2-[(1-imino-2,3-dihydroisoindol-5-yl)amino]-2-oxoethyl]-3-oxomorpholin-4-yl]benzoat,
2-(Difluormethoxy)-3-[(2R)-2-[(1R)-1-hydroxy-2-[(1-imino-2,3-dihydroisoindol-5-yl)amino]-2-oxoethyl]-3-oxomorpholin-4-yl]benzoesäure,
2-(Difluormethoxy)-3-[(2R)-2-[(1R)-1-hydroxy-2-[(1-imino-2,3-dihydroisoindol-5-yl)amino]-2-oxoethyl]-3-oxomorpholin-4-yl]-N,N-dimethylbenzamid,
2-(Difluormethoxy)-3-[2-[(1-hydroxy-2-[(1-imino-2,3-dihydroisoindol-5-yl)amino]-2-oxoethyl]-3-oxomorpholin-4-yl]benzamid,
Methyl-2-[(2R)-2-[(1R)-1-hydroxy-2-[(1-imino-2,3-dihydroisoindol-5-yl)amino]-2-oxoethyl]-3-oxomorpholin-4-yl]-5-(trifluormethoxy)benzoat,
2-[(2R)-2-[(1R)-1-Hydroxy-2-[(1-imino-2,3-dihydroisoindol-5-yl)amino]-2-oxoethyl]-3-oxomorpholin-4-yl]-5-(trifluormethoxy)benzoesäure,
2-[(2R)-2-[(1R)-1-Hydroxy-2-[(1-imino-2,3-dihydroisoindol-5-yl)amino]-2-oxoethyl]-3-oxomorpholin-4-yl]-5-(trifluormethoxy)benzamid,
(2R)-N-(3-Amino-1,2-benzisoxazol-6-yl)-2-hydroxy-2-[(2R)-3-oxo-4-(2-oxo-1H-chinolin-6-yl)morpholin-2-yl]acetamid,
(2R)-N-(3-Amino-1,2-benzisoxazol-6-yl)-2-hydroxy-2-[(2R)-3-oxo-4-(2-oxo-1H-chinolin-7-yl)morpholin-2-yl]acetamid,
(2R)-N-(3-Amino-1,2-benzisoxazol-6-yl)-2-hydroxy-2-[(2R)-3-oxo-4-(2-oxo-1H-chinolin-5-yl)morpholin-2-yl]acetamid,
2-(3-((R)-2-((R)-2-(4-Carbamimidoylphenylamino)-1-hydroxy-2-oxoethyl)-3-oxomorpholino)phenyl)acetat,
(R)-N-(4-Carbamimidoylphenyl)-2-hydroxy-2-((R)-4-(3-methyl-5-(pyrrolidin-1-carbonyl)phenyl)-3-oxomorpholin-2-yl)acetamid,
(R)-2-Hydroxy-N-(1-iminoisoindolin-5-yl)-2-((R)-4-(3-methyl-5-(pyrrolidin-1-carbonyl)phenyl)-3-oxomorpholin-2-yl)acetamid,
2-(3-((R)-2-((R)-1-Hydroxy-2-(1-iminoisoindolin-5-ylamino)-2-oxoethyl)-3-oxomorpholino)-N-methylbenzamido)essigsäure,
2-(3-((R)-2-((R)-1-Hydroxy-2-(1-iminoisoindolin-5-ylamino)-2-oxoethyl)-3-oxomorpholino)-N-methylbenzamido)acetat,
(S)-1-(3-((R)-2-((R)-2-(4-Carbamimidoylphenylamino)-1-hydroxy-2-oxoethyl)-3-oxomorpholino)benzoyl)pyrrolidin-2-carbonsäure,
1-(3-((R)-2-((R)-2-(4-Carbamimidoylphenylamino)-1-hydroxy-2-oxoethyl)-3-oxomorpholino)benzoyl)pyrrolidin-2-carboxylat,
3-((R)-2-((R)-2-(4-Carbamimidoylphenylamino)-1-hydroxy-2-oxoethyl)-3-oxomorpholino)-N-methyl-N-(2-oxo-2-(pyrrolidin-1-yl)ethyl)benzamid,
3-((R)-2-((R)-1-Hydroxy-2-(1-iminoisoindolin-5-ylamino)-2-oxoethyl)-3-oxomorpholino)-N-methyl-N-(2-oxo-2-(pyrrolidin-1-yl)ethyl)benzamid,
(R)-N-(4-Carbamimidoylphenyl)-2-hydroxy-2-((R)-4-(3-methyl-5-(morpholin-4-carbonyl)phenyl)-3-oxomorpholin-2-yl)acetamid,
(R)-2-Hydroxy-N-(1-iminoisoindolin-5-yl)-2-((R)-4-(3-methyl-5-(morpholin-4-carbonyl)phenyl)-3-oxomorpholin-2-yl)acetamid,
(R)-2-Hydroxy-N-(1-iminoisoindolin-5-yl)-2-((R)-4-(3-((R)-3-methoxypyrrolidin-1-carbonyl)phenyl)-3-oxomorpholin-2-yl)acetamid,
(R)-2-Hydroxy-N-(1-iminoisoindolin-5-yl)-2-((R)-4-(3-((S)-3-methoxypyrrolidin-1-carbonyl)phenyl)-3-oxomorpholin-2-yl)acetamid,
(R)-2-((R)-4-(3-Chlor-4-(morpholin-4-carbonyl)phenyl)-3-oxomorpholin-2-yl)-2-hydroxy-N-(1-iminoisoindolin-5-yl)acetamid,
(R)-2-Hydroxy-N-(1-iminoisoindolin-5-yl)-2-((R)-4-(3-(2-morpholino-2-oxoethyl)phenyl)-3-oxomorpholin-2-yl)acetamid,
(S)-1-(3-((R)-2-((R)-1-Hydroxy-2-(1-iminoisoindolin-5-ylamino)-2-oxoethyl)-3-oxomorpholino)benzoyl)pyrrolidin-2-carbonsäure,
1-(3-((R)-2-((R)-1-Hydroxy-2-(1-iminoisoindolin-5-ylamino)-2-oxoethyl)-3-oxomorpholino)benzoyl)pyrrolidin-2-carboxylat,
2-(3-((R)-2-((R)-1-Hydroxy-2-(1-iminoisoindolin-5-ylamino)-2-oxoethyl)-3-oxomorpholino)phenyl)acetat,
2-(3-((R)-2-((R)-1-Hydroxy-2-(1-iminoisoindolin-5-ylamino)-2-oxoethyl)-3-oxomorpholino)phenyl)essigsäure,
2-(3-((R)-2-((R)-1-Hydroxy-2-(1-iminoisoindolin-5-ylamino)-2-oxoethyl)-3-oxomorpholino)-N-methylbenzamido)acetat,
2-(3-((R)-2-((R)-1-Hydroxy-2-(1-iminoisoindolin-5-ylamino)-2-oxoethyl)-3-oxomorpholino)phenyl)-2-methylpropanoat,
(R)-2-Hydroxy-N-(1-iminoisoindolin-5-yl)-2-((R)-4-(3-(morpholin-4-carbonyl)phenyl)-3-oxomorpholin-2-yl)acetamid,
3-((R)-2-((R)-1-Hydroxy-2-(1-iminoisoindolin-5-ylamino)-2-oxoethyl)-3-oxomorpholino)-N-methyl-N-(2-morpholino-2-oxoethyl)benzamid,
(R)-2-Hydroxy-N-(1-iminoisoindolin-5-yl)-2-((R)-4-(3-(2-((R)-3-methoxypyrrolidin-1-yl)-2-oxoethyl)phenyl)-3-oxomorpholin-2-yl)acetamid,
(R)-2-Hydroxy-N-(1-iminoisoindolin-5-yl)-2-((R)-4-(3-(2-((S)-3-methoxypyrrolidin-1-yl)-2-oxoethyl)phenyl)-3-oxomorpholin-2-yl)acetamid,
2-(3-((R)-2-((R)-1-Hydroxy-2-(1-iminoisoindolin-5-ylamino)-2-oxoethyl)-3-oxomorpholino)phenyl)-2-methylpropanoat,
2-(3-((R)-2-((R)-1-Hydroxy-2-(1-iminoisoindolin-5-ylamino)-2-oxoethyl)-3-oxomorpholino)phenyl)acetat,
(R)-2-Hydroxy-N-(1-iminoisoindolin-5-yl)-2-((R)-4-(3-(4-methyl-3-oxopiperazin-1-carbonyl)phenyl)-3-oxomorpholin-2-yl)acetamid,
2-(3-((R)-2-((R)-1-Hydroxy-2-(1-iminoisoindolin-5-ylamino)-2-oxoethyl)-3-oxomorpholino)phenyl)-2-methylpropanoat,
(R)-2-Hyd roxy-N-(1-iminoisoindolin-5-yl)-2-((R)-4-(3-(2-(4-methyl-3-oxopiperazin-1-yl)-2-oxoethyl)phenyl)-3-oxomorpholin-2-yl)acetamid,
(R)-2-((R)-4-(3-(2-(Dimethylamino)-2-oxoethyl)phenyl)-3-oxomorpholin-2-yl)-2-hydroxy-N-(1-iminoisoindolin-5-yl)acetamid,
1-(3-((R)-2-((R)-1-Hydroxy-2-(1-iminoisoindolin-5-ylamino)-2-oxoethyl)-3-oxomorpholino)benzoyl)piperidin-4-carbonsäure,
1-(3-((R)-2-((R)-1-Hydroxy-2-(1-iminoisoindolin-5-ylamino)-2-oxoethyl)-3-oxomorpholino)benzoyl)piperidin-4-carboxylat,
2-(3-((R)-2-((R)-1-Acetoxy-2-(1-imino-3-oxoisoindolin-5-ylamino)-2-oxoethyl)-3-oxomorpholino)-N-methylbenzamido)essigsäure,
(R)-N-(1,3-Diiminoisoindolin-5-yl)-2-((R)-4-(3-(2-(dimethylamino)-2-oxoethyl)phenyl)-3-oxomorpholin-2-yl)-2-hydroxyacetamid,
(R)-2-(1-Imino-3-oxoisoindolin-5-ylamino)-1-((R)-4-(3-(methyl(2-oxo-2-(pyrrolidin-1-yl)ethyl)carbamoyl)phenyl)-3-oxomorpholin-2-yl)-2-oxoethylacetat,
(R)-N-(3-Aminobenzo[d]isoxazol-6-yl)-2-((R)-4-(4-fluor-3-(morpholin-4-carbonyl)phenyl)-3-oxomorpholin-2-yl)-2-hydroxyacetamid,
(R)-N-(3-Aminobenzo[d]isoxazol-6-yl)-2-((R)-4-(4-chlor-3-((4,4-difluorpiperidin-1-yl)methyl)phenyl)-3-oxomorpholin-2-yl)-2-hydroxyacetamid),
(R)-N-(3-Aminobenzo[d]isoxazol-6-yl)-2-hydroxy-2-((R)-3-oxo-4-(3-(3-oxomorpholino)phenyl)morpholin-2-yl)acetamid,
(R)-N-(3-Aminobenzo[d]isoxazol-6-yl)-2-((R)-4-(2-(difluormethoxy)phenyl)-3-oxomorpholin-2-yl)-2-hydroxyacetamid,
(R)-N-(3-Aminobenzo[d]isoxazol-6-yl)-2-hydroxy-2-((R)-4-(2-isopropoxyphenyl)-3-oxomorpholin-2-yl)acetamid,
(R)-N-(3-Aminobenzo[d]isoxazol-6-yl)-2-((R)-4-(3-cyanophenyl)-3-oxomorpholin-2-yl)-2-hydroxyacetamid,
(R)-N-(3-Aminobenzo[d]isoxazol-6-yl)-2-((R)-4-(4-chlor-3-(morpholin-4-carbonyl)phenyl)-3-oxomorpholin-2-yl)-2-hydroxyacetamid,
(R)-N-(3-Aminobenzo[d]isoxazol-6-yl)-2-hydroxy-2-((R)-4-(4-methyl-3-(morpholin-4-carbonyl)phenyl)-3-oxomorpholin-2-yl)acetamid,
(R)-N-(3-Aminobenzo[d]isoxazol-6-yl)-2-hydroxy-2-((R)-3-oxo-4-(3-(trifluormethoxy)phenyl)morpholin-2-yl)acetamid,
(R)-N-(3-Aminobenzo[d]isoxazol-6-yl)-2-hydroxy-2-((R)-3-oxo-4-(3-(trifluormethyl)phenyl)morpholin-2-yl)acetamid,
(R)-N-(3-Aminobenzo[d]isoxazol-6-yl)-2-((R)-4-(4-chlor-3-(1,2,3,4-tetrahydroisochinolin-2-carbonyl)phenyl)-3-oxomorpholin-2-yl)-2-hydroxyacetamid,
(R)-N-(3-Aminobenzo[d]isoxazol-6-yl)-2-((R)-4-(4-chlor-3-(4,4-difluorpiperidin-1-carbonyl)phenyl)-3-oxomorpholin-2-yl)-2-hydroxyacetamid,
(R)-N-(3-Aminobenzo[d]isoxazol-6-yl)-2-((R)-4-(3-fluor-4-(trifluormethyl)phenyl)-3-oxomorpholin-2-yl)-2-hydroxyacetamid,
(R)-N-(4-Carbamimidoyl-3-fluorphenyl)-2-hydroxy-2-((R)-3-oxo-4-(3-(3-oxomorpholino)phenyl)morpholin-2-yl)acetamid,
(R)-2-Hyd roxy-N-(1-imino-7-(trifluormethyl)isoindolin-5-yl)-2-((R)-3-oxo-4-(3-(3-oxomorpholino)phenyl)morpholin-2-yl)acetamid,
(R)-2-Hydroxy-N-(1-iminoisoindolin-5-yl)-2-((R)-3-oxo-4-(3-(trifluormethoxy)phenyl)morpholin-2-yl)acetamid,
Ethyl-2-(3-((R)-2-((R)-1-hydroxy-2-(1-iminoisoindolin-5-ylamino)-2-oxoethyl)-3-oxomorpholino)phenoxy)acetat,
(R)-2-Hydroxy-N-(1-iminoisoindolin-5-yl)-2-((R)-4-(4-methoxybenzyl)-3-oxomorpholin-2-yl)acetamid, (*R*)-2-((*R*)-4-(3-(Benzyloxy)phenyl)-3-oxomorpholin-2-yl)-2-hydroxy-N-(1-iminoisoindolin-5-yl)acetamid,
(R)-N-(1-Aminoisochinolin-6-yl)-2-((R)-4-(4-fluor-3-(morpholin-4-carbonyl)phenyl)-3-oxomorpholin-2-yl)-2-hydroxyacetamid,
N-(2,3-Dihydro-1-imino-1h-isoindol-5-yl)-4-[2-[(dimethylamino)carbonyl]phenyl]-alpha(R)-hydroxy-3-oxo-2(R)-morpholinacetamid,
N-(2,3-Dihydro-1-imino-1H-isoindol-5-yl)-alpha(R)-hydroxy-3-oxo-4-[2-(1-pyrrolidinylcarbonyl)phenyl]-2(R)-morpholinacetamid,
4-(2-Cyanophenyl)-N-(2,3-dihydro-1-imino-1h-isoindol-5-yl)-alpha(R)-hydroxy-3-oxo-2(R)-morpholinacetamid,
N-(2,3-Dihydro-1-imino-1h-isoindol-5-yl)-4-[4-fluor-3-(4-morpholinylcarbonyl)phenyl]alpha(R)-hydroxy-3-oxo-2(R)-morpholinacetamid,
N-(2,3-Dihydro-1-imino-1h-isoindol-5-yl)-4-[4-fluor-3-[(3(R)-methyl-4-morpholinyl)carbonyl]phenyl]-alpha(R)-hyd roxy-3-oxo-2(R)-morpholinacetamid,
N-(2,3-Dihydro-1-imino-1h-isoindol-5-yl)-4-[4-fluor-3-[(3(S)-methyl-4-morpholinyl)carbonyl]phenyl]-alpha(R)-hydroxy-3-oxo-2(R)-morpholinacetamid,
N-[4-(Aminoiminomethyl)phenyl]-4-(2-cyanophenyl)-alpha(R)-hydroxy-3-oxo-2(R)-morpholinacetamid,
N-(4-Amino-7-chinazolinyl)-4-[4-fluor-3-(4-morpholinylcarbonyl)phenyl]-alpha(R)-hydroxy-3-oxo-2(R)-morpholinacetamid,
[3-[2(R)-[2-[(3-Amino-1,2-benzisoxazol-6-yl)amino]-1(R)-hydroxy-2-oxoethyl]-3-oxo-4-morpholinyl]phenyl]pentafluorschwefel,
[4-[2(R)-[2-[(3-Amino-1,2-benzisoxazol-6-yl)amino]-1(R)-hydroxy-2-oxoethyl]-3-oxo-4-morpholinyl]phenyl]pentafluorschwefel,
N-(3-Amino-5-fluor-1,2-benzisoxazol-6-yl)-4-[4-fluor-3-(4-morpholinylcarbonyl)phenyl]-alpha(R)-hydroxy-3-oxo-2(R)-morpholinacetamid,
N-(2-Amino-6-chinazolinyl)-4-[4-fluor-3-(4-morpholinylcarbonyl)phenyl]-alpha(R)-hydroxy-3-oxo-2(R)-morpholinacetamid,
(R)-2-((R)-4-(5-Fluor-2-isopropoxyphenyl)-3-oxomorpholin-2-yl)-2-hydroxy-N-(1-iminoisoindolin-5-yl)acetamid,
(R)-2-Hydroxy-N-(1-iminoisoindolin-5-yl)-2-((R)-3-oxo-4-(2-(trifluormethyl)phenyl)morpholin-2-yl)acetamid,
(R)-2-((R)-4-(2-(Difluormethoxy)phenyl)-3-oxomorpholin-2-yl)-2-hydroxy-N-(1-iminoisoindolin-5-yl)acetamid,
(R)-2-Hydroxy-N-(1-iminoisoindolin-5-yl)-2-((R)-4-(2-isopropoxyphenyl)-3-oxomorpholin-2-yl)acetamid,
(R)-2-Hydroxy-N-(1-iminoisoindolin-5-yl)-2-((R)-3-oxo-4-(2-(trifluormethoxy)phenyl)morpholin-2-yl)acetamid,
*N*-(3-Amino-1,2-benzisoxazol-6-yl)-4-[4-fluor-3-[(tetrahydro-2*H*-pyran-4-yl)oxy]phenyl]-alpha(R)-hydroxy-3-oxo-2(R)-morpholinacetamid,
N-(3-Amino-1,2-benzisoxazol-6-yl)-4-[3-[(3,3-difluor-1-azetidinyl)carbonyl]-4-fluorphenyl]-alpha(R)-hydroxy-3-oxo-2(R)-morpholinacetamid,
*N*-(3-Amino-1,2-benzisoxazol-6-yl)-4-[3-[(4,4-difluor-1-piperidinyl)carbonyl]-4-fluorphenyl]-alpha(R)-hyd roxy-3-oxo-2(R)-morpholinacetamid,
N-(3-Amino-1,2-benzisoxazol-6-yl)-4-[4-chlor-3-[3-(1,1-dimethylethyl)-1,2,4-oxadiazol-5-yl]phenyl]-alpha(R)-hyd roxy-3-oxo-2(R)-morpholinacetamid,
N-(3-Amino-1,2-benzisoxazol-6-yl)-4-[2-fluor-5-(trifluormethyl)phenyl]-alpha(R)-hydroxy-3-oxo-2(R)-morpholinacetamid,
(R)-N-(4-Carbamimidoylphenyl)-2-hydroxy-2-((R)-4-(3-((S)-2-methylpyrrolidin-1-carbonyl)phenyl)-3-oxomorpholin-2-yl)acetamid,
(R)-N-(4-Carbamimidoylphenyl)-2-hydroxy-2-((R)-4-(2-methyl-3-(morpholin-4-carbonyl)phenyl)-3-oxomorpholin-2-yl)acetamid,
(R)-N-(4-Carbamimidoylphenyl)-2-hydroxy-2-((R)-4-(2-methyl-3-(pyrrolidin-1-carbonyl)phenyl)-3-oxomorpholin-2-yl)acetamid,
(R)-2-((R)-4-(3-(Azetidin-1-carbonyl)-5-fluorphenyl)-3-oxomorpholin-2-yl)-N-(4-carbamimidoylphenyl)-2-hydroxyacetamid,
(R)-2-((R)-4-(3-(Azetidin-1-carbonyl)-2-methylphenyl)-3-oxomorpholin-2-yl)-N-(4-carbamimidoylphenyl)-2-hydroxyacetamid,
(R)-2-((R)-4-(5-(Azetidin-1-carbonyl)-2-methylphenyl)-3-oxomorpholin-2-yl)-N-(4-carbamimidoylphenyl)-2-hydroxyacetamid,
(R)-N-(4-Carbamimidoylphenyl)-2-hydroxy-2-((R)-3-oxo-4-(6-(trifluormethyl)pyridin-2-yl)morpholin-2-yl)acetamid,
(R)-N-(4-Carbamimidoylphenyl)-2-hydroxy-2-((R)-4-(2-methyl-5-(pyrrolidin-1-carbonyl)phenyl)-3-oxomorpholin-2-yl)acetamid,
(R)-2-((R)-4-(3-(Azetidin-1-carbonyl)-5-methylphenyl)-3-oxomorpholin-2-yl)-N-(4-carbamimidoylphenyl)-2-hydroxyacetamid,
(R)-N-(4-Carbamimidoylphenyl)-2-hydroxy-2-((R)-4-(2-methyl-5-(morpholin-4-carbonyl)phenyl)-3-oxomorpholin-2-yl)acetamid,
(S)-1-(3-((R)-2-((R)-2-(4-Carbamimidoylphenylamino)-1-hydroxy-2-oxoethyl)-3-oxomorpholino)-5-fluorbenzoyl)pyrrolidin-2-carbonsäure,
1-(3-((R)-2-((R)-2-(4-Carbamimidoylphenylamino)-1-hydroxy-2-oxoethyl)-3-oxomorpholino)-5-fluorbenzoyl)pyrrolidin-2-carboxylat,
1-(3-((R)-2-((R)-2-(4-Carbamimidoylphenylamino)-1-hydroxy-2-oxoethyl)-3-oxomorpholino)-5-fluorbenzoyl)pyrrolidin-2-carboxylat,
2-(3-((R)-2-((R)-2-(4-Carbamimidoylphenylamino)-1-hydroxy-2-oxoethyl)-3-oxomorpholino)-5-fluor-N-methylbenzamido)essigsäure,
2-(3-((R)-2-((R)-2-(4-Carbamimidoylphenylamino)-1-hydroxy-2-oxoethyl)-3-oxomorpholino)-5-fluor-N-methylbenzamido)acetat,
3-((R)-2-((R)-2-(4-Carbamimidoylphenylamino)-1-hydroxy-2-oxoethyl)-3-oxomorpholino)-N-(2-(dimethylamino)-2-oxoethyl)-5-fluor-N-methylbenzamid,
(R)-N-(1,3-Diiminoisoindolin-5-yl)-2-hydroxy-2-((R)-4-(3-(2-(4-methyl-3-oxopiperazin-1-yl)-2-oxoethyl)phenyl)-3-oxomorpholin-2-yl)acetamid,
N-(2-(Dimethylamino)-2-oxoethyl)-3-((R)-2-((R)-1-hydroxy-2-(1-iminoisoindolin-5-ylamino)-2-oxoethyl)-3-oxomorpholino)-N-methylbenzamid,
2-((S)-Hydroxy-((R)-3-oxo-4-p-tolylmorpholin-2-yl)methyl)-4-oxo-1,4-dihydrochinazolin-6-carboximidamid,
1,1-Dioxo-3-((S)-hydroxy-((R)-3-oxo-4-p-tolylmorpholin-2-yl)methyl)-4H-benzo[e][1,2,4]thiadiazin-7-carboximidamid,
(R)-N-(3-Aminobenzo[d]isoxazol-6-yl)-2-((R)-4-(4-chlor-3-(morpholinomethyl)phenyl)-3-oxomorpholin-2-yl)-2-hydroxyacetamid,
(R)-N-(3-Aminobenzo[d]isoxazol-6-yl)-2-hydroxy-2-((R)-4-(3-(morpholin-4-carbonylphenyl)-3-oxomorpholin-2-yl)acetamid,
3-((R)-2-((R)-2-(4-Carbamimidoyl-3-fluorphenylamino)-1-hydroxy-2-oxoethyl)-3-oxomorpholino)benzoesäure,
(R)-N-(4-Carbamimidoyl-3,5-difluorphenyl)-2-hydroxy-2-((R)-3-oxo-4-(3-(3-oxomorpholino)phenyl)morpholin-2-yl)acetamid,
(R)-N-(4-Carbamimidoyl-2,3-difluorphenyl)-2-hydroxy-2-((R)-3-oxo-4-(3-(3-oxomorpholino)phenyl)morpholin-2-yl)acetamid,
(R)-N-(4-Carbamimidoyl-2,5-difluorphenyl)-2-hydroxy-2-((R)-3-oxo-4-(3-(3-oxomorpholino)phenyl)morpholin-2-yl)acetamid,
(R)-N-(3-Aminomethyl)-4-cyano-5-(trifluormethyl)phenyl)-2-hydroxy-2-((R)-3-oxo-4-(3-(3-oxomorpholino)phenyl)morpholin-2-yl)acetamid,
(R)-2-Hydroxy-N-(1-iminoisoindolin-5-yl)-2-((R)-4-(2-methyl-1-oxoisoindolin-5-yl)-3-oxomorpholin-2-yl)acetamid,
(R)-2-Hydroxy-N-(1-iminoisoindolin-5-yl)-2-((R)-4-(2-methyl-3-oxoisoindolin-5-yl)-3-oxomorpholin-2-yl)acetamid-Hydrochlorid,
(*R*)-2-((*R*)-4-(2-(Cyclopropylmethyl)-3-oxoisoindolin-5-yl)-3-oxomorpholin-2-yl)-2-hydroxy-N-(1-iminoisoindolin-5-yl)acetamid,
(*R*)-N-(4-(Aminomethyl)-3-fluorphenyl)-2-hydroxy-2-((R)-3-oxo-4-(3-(3-oxomorpholino)phenyl)morpholin-2-yl)acetamid, (*R*)-*N*-(4-Guanidinophenyl)-2-hydroxy-2-((*R*)-3-oxo-4-(3-(3-oxomorpholino)phenyl)morpholin-2-yl)acetamid,
(*R*)-*N*-(4-(Aminomethyl)phenyl)-2-((*R*)-4-(4-fluor-3-(morpholin-4-carbonyl)phenyl)-3-oxomorpholin-2-yl)-2-hydroxyacetamid,
(R)-2-(4-Carbamoylphenylamino)-1-((*R*)-4-(4-fluor-3-(morpholin-4-carbonyl)phenyl)-3-oxomorpholin-2-yl)-2-oxoethylacetat,
(*R*)-2-((*R*)-4-(4-Fluor-3-(morpholin-4-carbonyl)phenyl)-3-oxomorpholin-2-yl)-2-hydroxy-N-(1,2,3,4-tetrahydroisochinolin-6-yl)acetamid,
(*R*)-*N*-(4-(Aminomethyl)-3-fluorphenyl)-2-((*R*)-4-(4-fluor-3-(morpholin-4-carbonyl)phenyl)-3-oxomorpholin-2-yl)-2-hydroxyacetamid,
(R)-N-(4-((*R*)-1-Aminoethyl)phenyl)-2-((*R*)-4-(4-fluor-3-(morpholin-4-carbonyl)phenyl)-3-oxomorpholin-2-yl)-2-hydroxyacetamid,
(*R*)-*N*-((*R*)-1-Amino-2,3-dihydro-1H-inden-5-yl)-2-((*R*)-4-(4-fluor-3-(morpholin-4-carbonyl)phenyl)-3-oxomorpholin-2-yl)-2-hydroxyacetamid,
(*R*)-*N*-((*S*)-1-Amino-2,3-dihydro-1*H*-inden-5-yl)-2-((*R*)-4-(4-fluor-3-(morpholin-4-carbonyl)phenyl)-3-oxomorpholin-2-yl)-2-hydroxyacetamid,
(*R*)-*N*-(2-Aminochinolin-6-yl)-2-((*R*)-4-(4-fluor-3-(morpholin-4-carbonyl)phenyl)-3-oxomorpholin-2-yl)-2-hydroxyacetamid,
N-(7-(Fluor-2,3-dihydro-1-imino-1H-isoindol-5-yl)-alpha(R)-hydroxy-3-oxo-4-[3-(3-oxo-4-morpholinyl)phenyl]-2(R)-morpholinacetamid,
N-(4-Chlor-2,3-dihydro-1-imino-1H-isoindol-5-yl)-alpha(R)-hydroxy-3-oxo-4-[3-(3-oxo-4-morpholinyl)phenyl]-2(R)-morpholinacetamid,
3-[2(R)-[2-[(2,3-Dihydro-1-imino-1h-isoindol-5-yl)amino]-1 (R)-hydroxy-2-oxoethyl]-3-oxo-4-morpholinyl]benzoesäure,
Methyl-3-[2(R)-[2-[(2,3-dihydro-1-imino-1H-isoindol-5-yl)amino]-1 (R)-hydroxy-2-oxoethyl]-3-oxo-4-morpholinyl]benzoat,
Methyl-2-[2(R)-[2-[(2,3-dihydro-1-imino-1h-isoindol-5-yl)amino]-1 (R)-hydroxy-2-oxoethyl]-3-oxo-4-morpholinyl]benzoat,
N-(3-Amino-1,2-benzisoxazol-6-yl)-4-[4-fluor-3-[2,2,2-trifluor-1-(4-morpholinyl)ethyl]phenyl]-alpha(R)-hydroxy-3-oxo-2(R)-morpholinacetamid,
(R)-2-((R)-4-(5-Chlor-2-isopropoxyphenyl)-3-oxomorpholin-2-yl)-2-hydroxy-N-(1-iminoisoindolin-5-yl)acetamid oder
*N*-(3-Amino-1,2-benzisoxazol-6-yl)-4-[3-(difluormethoxy)-4-fluorphenyl]-alpha(R)-hydroxy-3-oxo-2(R)-morpholinacetamid,
oder ein pharmazeutisch annehmbares Salz davon.

5. Eine Verbindung, die 2-((S)-Hydroxy((R)-3-oxo-4-p-tolylmorpholin-2-yl)methyl)-4-oxo-1,4-dihydrochinazolin-6-carboximidamid oder 1,1-Dioxo-3-((S)-hydroxy-((R)-3-oxo-4-p-tolylmorpholin-2-yl)methyl-4H-benzo[e][1,2,4]thiadiazin-7-caboximidamid oder ein pharmazeutisch annehmbares Salz davon.

6. Eine pharmazeutische Zusammensetzung, umfassend wenigstens eine Verbindung nach einem der Ansprüche 1 bis 5 oder ein pharmazeutisch annehmbares Salz davon und wenigstens einen pharmazeutisch annehmbaren Träger.

7. Eine Verbindung nach einem der der Ansprüche 1 bis 5 oder ein pharmazeutisch annehmbares Salz davon zur Verwendung bei der Behandlung einer Störung oder Erkrankung, die durch Faktor IXa vermittelt wird.

8. Eine Verbindung oder ein pharmazeutisch annehmbares Salz davon zur Verwendung nach Anspruch 7, wobei die Störung oder Erkrankung eine thromboembolische Störung ist.

9. Eine Verbindung oder ein pharmazeutisch annehmbares Salz davon zur Verwendung nach Anspruch 8, wobei die thromboembolische Störung ausgewählt ist aus der Gruppe, bestehend aus arteriellen kardiovaskulären thromboembolischen Störungen, venösen kardiovaskulären thromboembolischen Störungen und thromboembolischen Störungen in den Herzkammern.

10. Eine Verbindung oder ein pharmazeutisch annehmbares Salz davon zur Verwendung nach Anspruch 8, wobei die thromboembolische Störung ausgewählt ist aus instabiler Angina, einem akuten Koronarsyndrom, Vorhofflimmern, erstmaligem Myokardinfarkt, rezidivierendem Myokardinfarkt, ischämischem plötzlichem Tod, transitorischer ischämischer Attacke, Schlaganfall, Atherosklerose, peripherer arterieller Verschlusskrankheit, Venenthrombose, tiefer Venenthrombose, Thrombophlebitis, arterieller Embolie, Koronararterienthrombose, Zerebralarterienthrombose, zerebraler Embolie, Nierenembolie, Lungenembolie und Thrombosen, die herrühren von (a) Herzklappenprothesen oder anderen Implantaten, (b) Verweilkathetern, (c) Stents, (d) kardiopulmonalem Bypass, (e) Hämodialyse oder (f) anderen Verfahren, bei denen Blut einer künstlichen Oberfläche ausgesetzt ist, die Thrombose fördert.

11. Eine Kombination aus einer Verbindung nach einem der Ansprüche 1-5 oder einem pharmazeutisch annehmbaren Salz davon und wenigstens einem Antikoagulationsmittel, ausgewählt aus der Gruppe, bestehend aus einem Thrombin-Inhibitor, einem Thrombin-Rezeptor(PAR-1)-Antagonisten, einem Faktor-VIIa-Inhibitor, einem Faktor-VIIIA-Inhibitor, einem Faktor-IXa-Inhibitor, der sich von der Verbindung nach den Ansprüchen 1-5 unterscheidet, einem Faktor-Xa-Inhibitor, einem Faktor-XIa-Inhibitor, TAFI und Fibrinogen-Inhibitoren.

12. Die Kombination nach Anspruch 11, wobei der Faktor-IXa-Inhibitor ausgewählt ist aus der Gruppe, bestehend aus monoklonalen Antikörpern, synthetischen kompetitiven Inhibitoren mit blockiertem aktivem Zentrum, oralen Inhibitoren und RNA-Aptameren.

13. Eine pharmazeutische Zusammensetzung, umfassend:
eine therapeutisch wirksame Menge wenigstens einer Verbindung nach einem der Ansprüche 1-5 oder ein pharmazeutisch annehmbares Salz davon und wenigstens einen pharmazeutisch annehmbaren Träger und eine wirksame Menge wenigstens eines Mittels, ausgewählt aus der Gruppe, bestehend aus: (a) Antikoagulantien, (b) Anti-Thrombin-Mitteln, (c) Thrombozytenaggregationshemmern, (d) Fibrinolytika, (e) hypolipidämischen Mitteln, (f) Antihypertensiva und (g) antiischämischen Mitteln.

14. Eine pharmazeutische Zusammensetzung, umfassend:
eine therapeutisch wirksame Menge wenigstens einer Verbindung nach einem der Ansprüche 1-5 oder ein pharmazeutisch annehmbares Salz davon und wenigstens einen pharmazeutisch annehmbaren Träger und eine wirksame Menge wenigstens eines Mittels, ausgewählt aus der Gruppe, bestehend aus (a-1) Warfarin, (a-2) Heparin, (a-3) Aprotinin, (a-4) synthetischem Pentasaccharid, (a-5) direkt wirkenden Thrombin-Inhibitoren, einschließlich Hirudin und Argatroban, (a-6) einem Faktor-VIIa-Inhibitor, (a-7) einem Faktor-VIIIa-Inhibitor, (a-8) einem Faktor-IXa-Inhibitor, der sich von den Verbindungen der Formel (I) unterscheidet, (a-9) einem Faktor-Xa-Inhibitor, (a-10) einem Faktor-XIa-Inhibitor, (a-11) einem Thrombin-Inhibitor, (a-12) einem TAFI, (a-13) einem Fibrinogen-Inhibitor, (b-1) einem Boroarginin-Derivat, (b-2) einem Boropeptid, (b-3) Heparin, (b-4) Hirudin, (b-5) Argatroban, (C-1) einem NSAID, (c-2) einem IIb/IIIa-Antagonisten, (c-3) einem Thromboxan-A2-Rezeptor-Antagonisten, (c-4) einem Thromboxan-A2-Synthetase-Inhibitor, (c-5) einem PDE-III-Inhibitor, (c-6) einem PDE-V-Inhibitor, (c-7) einem ADP-Rezeptor-Antagonisten, (c-8) einem Antagonisten des purinergen Rezeptors P2Y1, (c-9) einem Antagonisten des purinergen Rezeptors P2Y12, (d-1) Gewebeplasminogenaktivator (TPA, natürlich oder rekombinant) und modifizierten Formen davon, (d-2) Anistreplase, (d-3) Urokinase, (d-4) Streptokinase, (d-5) Tenecteplase (TNK), (d-6) Lanoteplase (nPA), (d-7) einem Faktor-VIIa-Inhibitor, (d-8) einem PAI-1-Inhibitor, (d-9) einem alpha-2-Antiplasmin-Inhibitor, (d-10) einem anisoyliertem Plasminogen-Streptokinase-Aktivator-Komplex, (c-1) einem HMG-CoA-Reduktase-Inhibitor, (e-2) einem Squalen-Synthetase-inhibitor, (e-3) einem Fibrat, (e-4) einem Gallensäuresequestriermittel, (e-5) einem ACAT-Inhibitor, (e-6) einem MTP-Inhibitor, (e-7) einem Lipooxygenase-Inhibitor, (e-8) einem Cholesterinabsorptionsinhibitor, (e-9) einem Cholesterinestertransferprotein-Inhibitor, (f-1) einem alpha-adrenergen Blocker, (f-2) einem beta-adrenergen Blocker, (f-3) einem Kalziumkanalblocker (f-4) einem Diuretikum, (f-5) einem Renin-Inhibitor, (f-6) einem Angiotensinkonversionsenzym-Inhibitor, (f-7) einem Angiotensin-II-Rezeptor-Antagonisten, (f-8) einem ET-Rezeptor-Antagonisten, (f-9) einem dualen ET/All-Antagonisten, (f-10) einem neutralen Endopeptidase-Inhibitor, (f-11) einem Vasopeptidase-Inhibitor, (g-1) einem Klasse-I-Mittel, (g-2) einem Klasse-II-Mittel, (g-3) einem Klasse-III-Mittel, (g-4) einem Klasse-IV-Mittel, (g-5) einem K⁺-Kanalöffner, (g-6) einem IKur-Inhibitor und (g-7) einem Herzglycosid.

15. Eine Kombination aus einer Verbindung nach einem der Ansprüche 1-5 oder einem pharmazeutisch annehmbaren Salz davon und einem oder mehreren zusätzlichen therapeutischen Mitteln.

16. Eine Verbindung nach einem der Ansprüche 1-5 oder ein pharmazeutisch annehmbares Salz davon zur Verwendung in der Therapie.

## Revendications

1. Composé de la Formule (I) 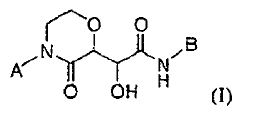 ou un sel pharmaceutiquement acceptable de celui-ci, dans lequel
A est 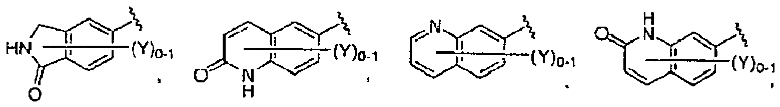 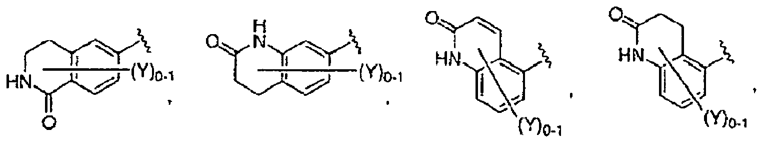 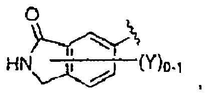 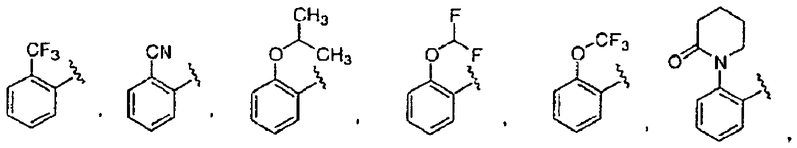 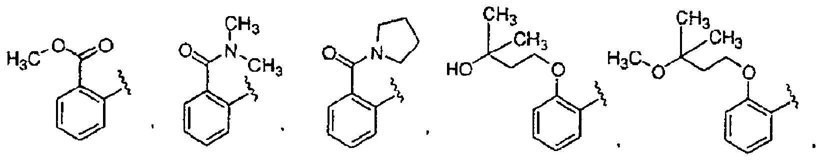 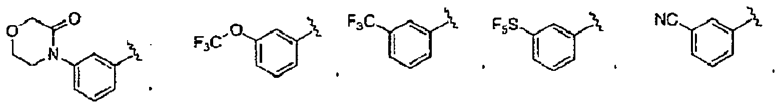 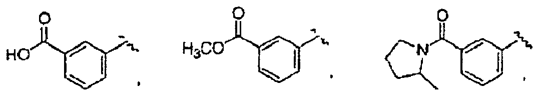 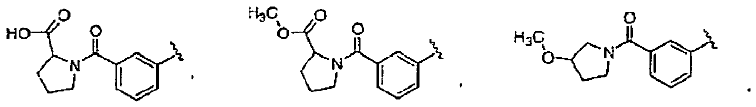 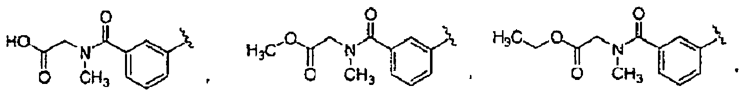 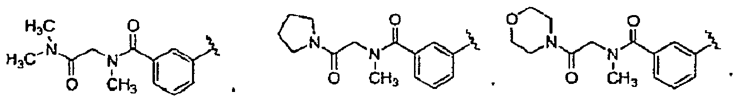 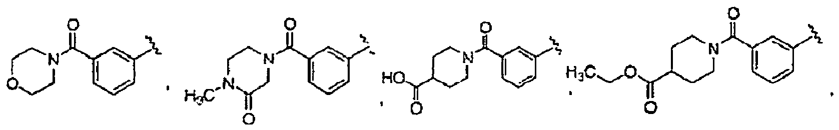 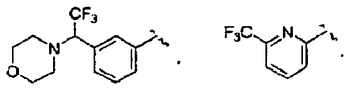 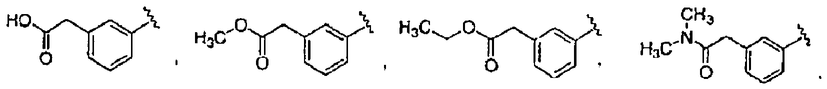 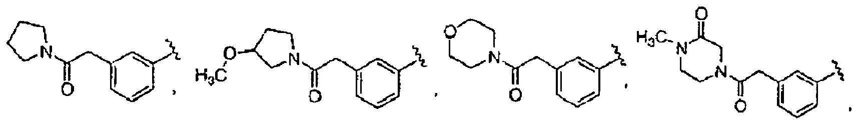 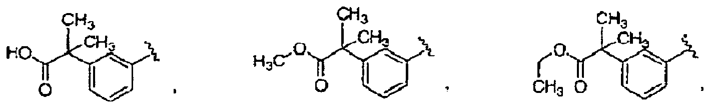 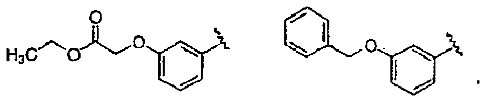 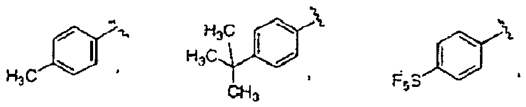 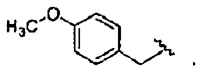 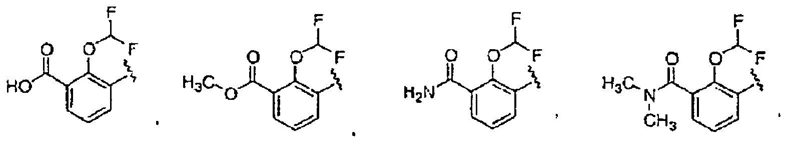 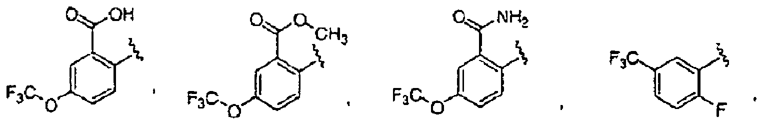 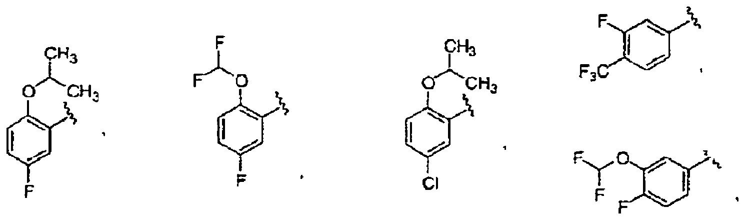 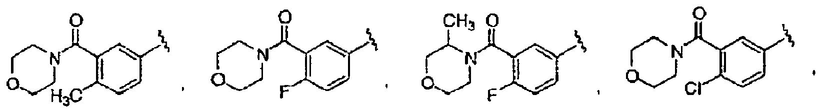 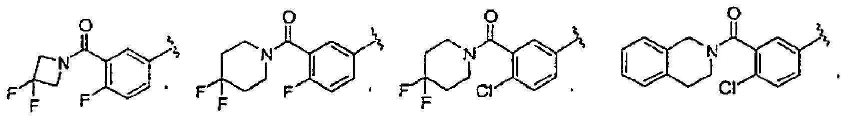 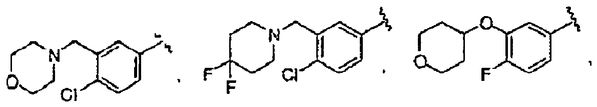 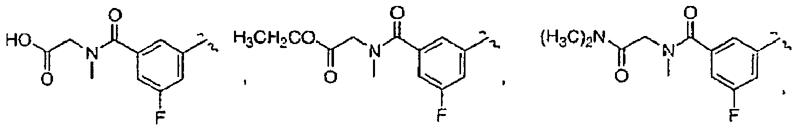 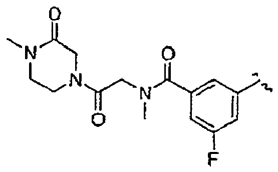 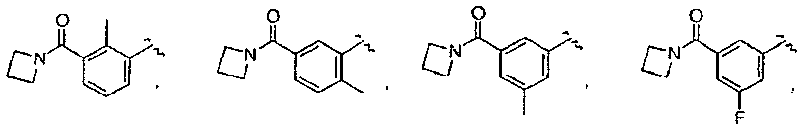 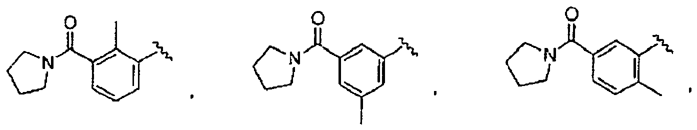 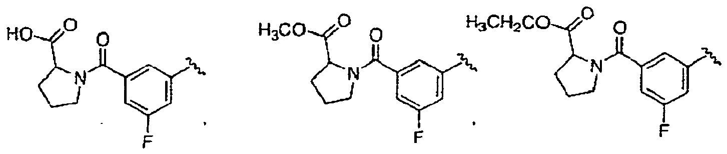 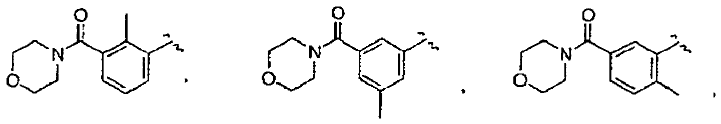 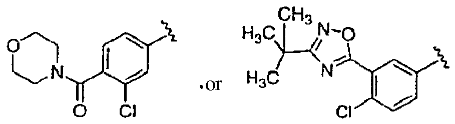 Y, attaché à un atome de carbone ou d'azote cyclique, est
1) halogène,
2) -alkyle-(C₁-C₆),
3) -haloalkyle-(C₁-C₃),
4) -cycloalkyle-(C₃-C₈),
5) -OH,
6) -O-alkyle-(C₁-C₆),
7) -O-haloalkyle-(C₁-C₃),
8) =O (oxo).
où ladite partie -alkyle-(C₁-C₆) de 2) et de 6) dudit Y est non substituée ou indépendamment substituée par les substituants sélectionnés parmi le groupe consistant en -cycloalkyle-(C₃-C₈), -C(O)OH et -C(O)-alkyle-C₁-C₆).
B est 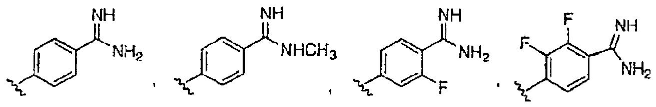 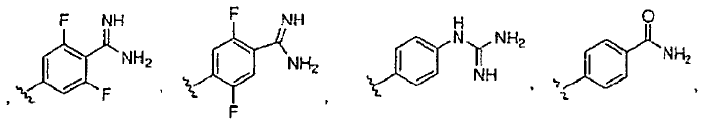 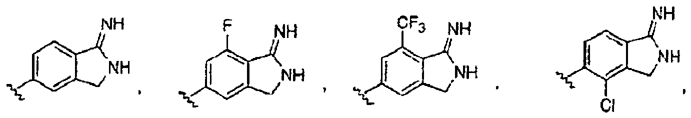 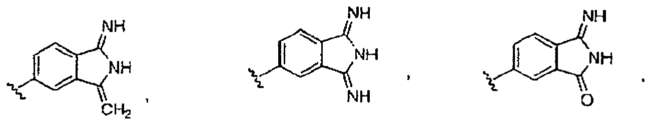 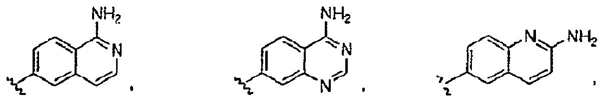 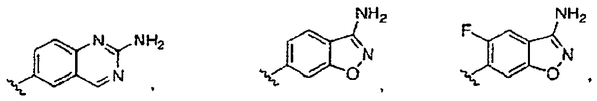 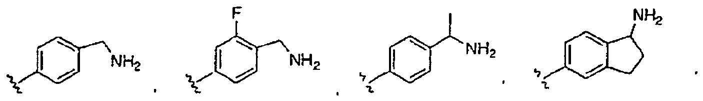 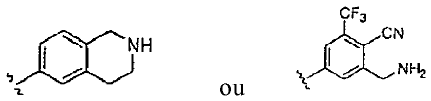 à condition que
lorsque A est 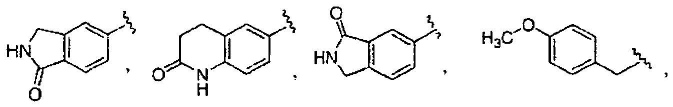 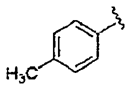 ou 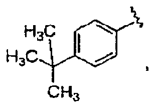 B ne soit pas 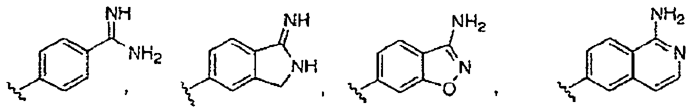 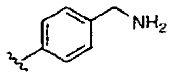 ou 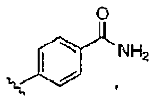 et
lorsque A est 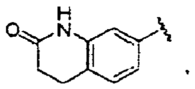 B ne soit pas

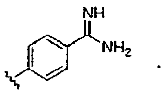

2. Composé de la Formule (I) selon la revendication 1 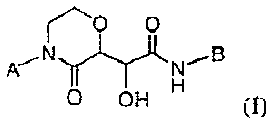 ou un sel pharmaceutiquement acceptable de celui-ci, dans lequel A est 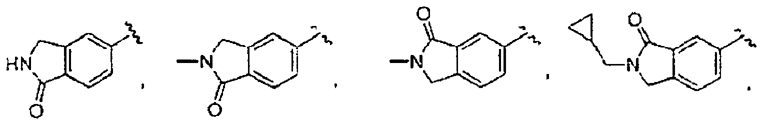 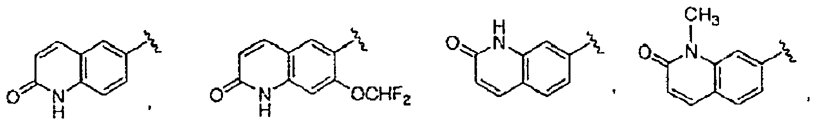 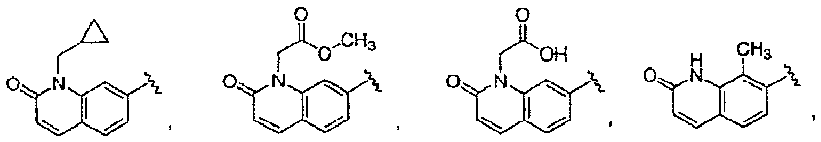 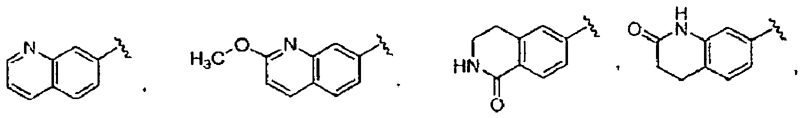 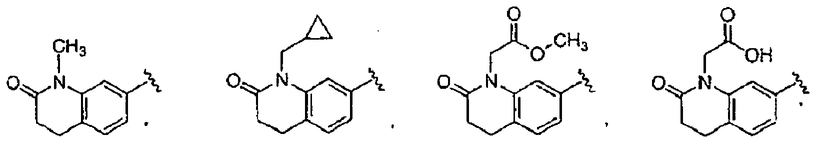 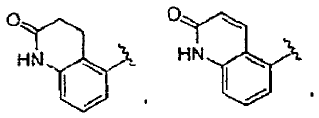 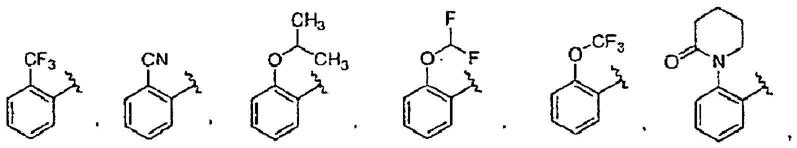 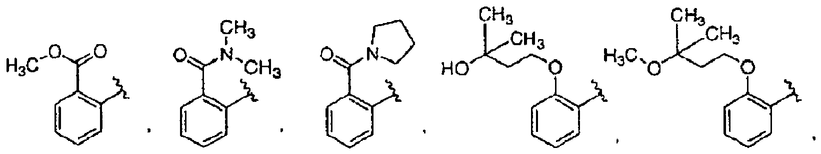 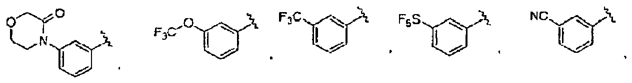 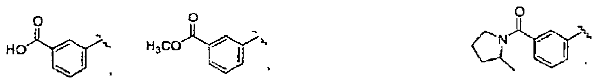 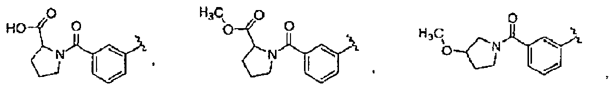 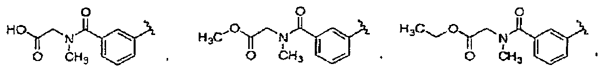 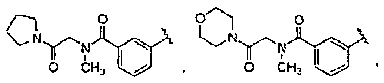 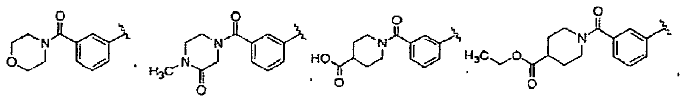 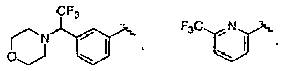 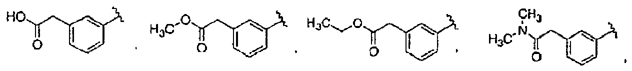 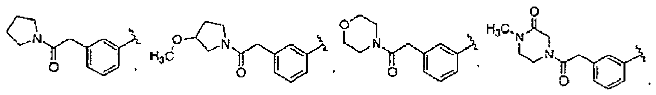 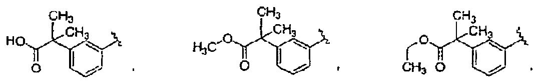 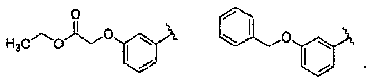 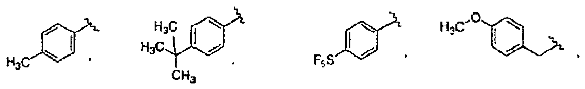 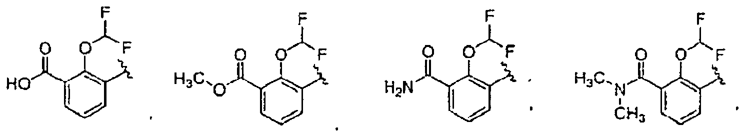 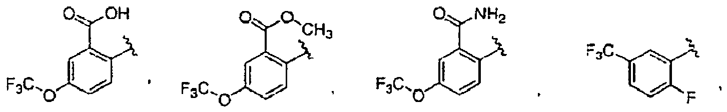 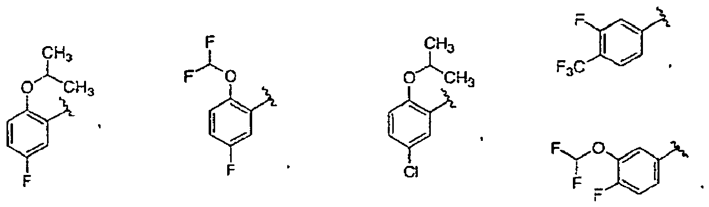 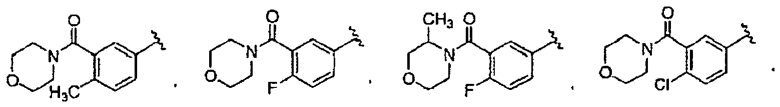 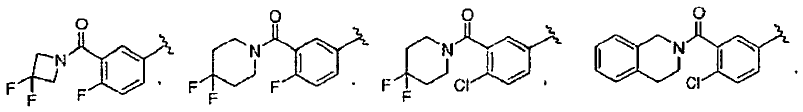 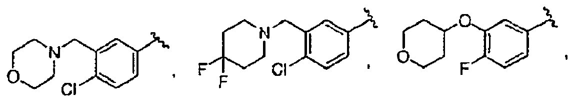 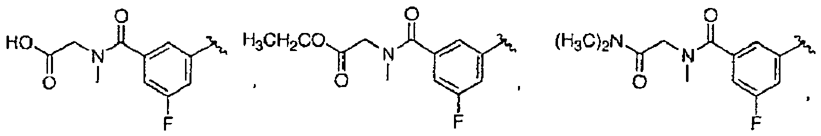 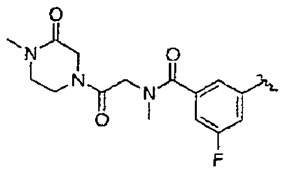 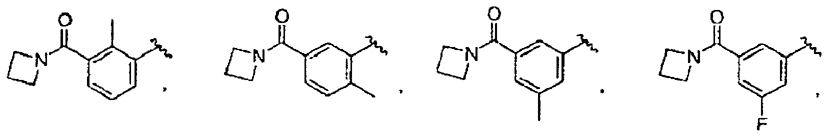 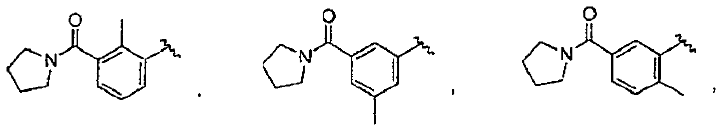 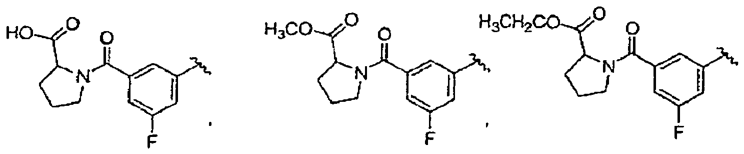 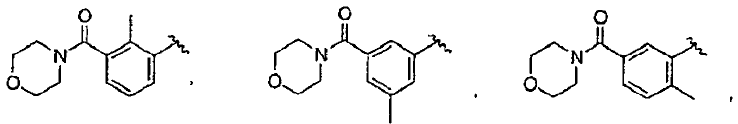 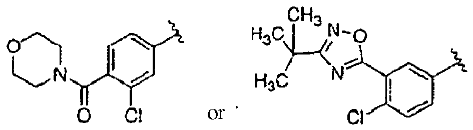 B est 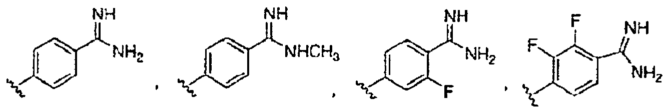 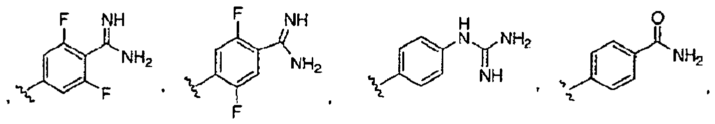 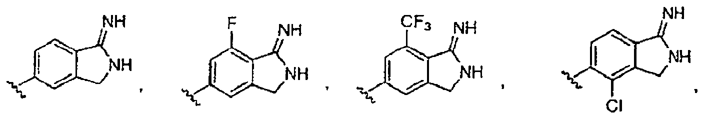 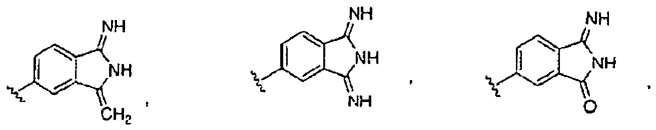 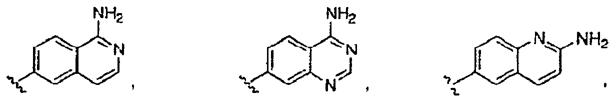 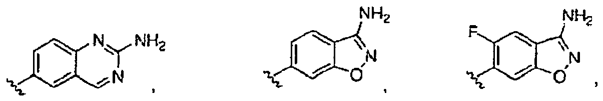 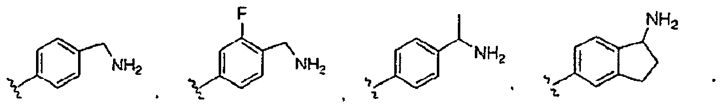 à condition que 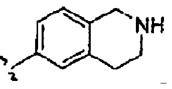 ou 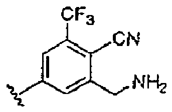 lorsque A est 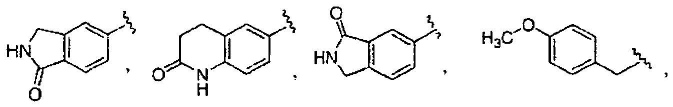 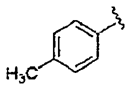 ou 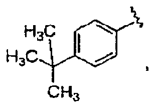 B ne soit pas 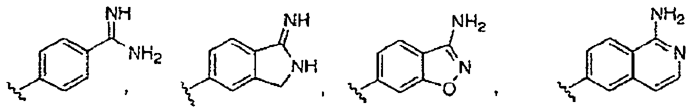 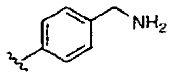 ou 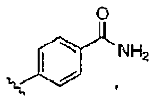 et
lorsque A est 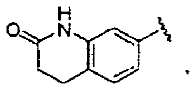 B ne soit pas

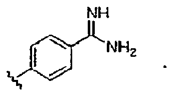

3. Composé selon la revendication 1 ou 2 ou un sel pharmaceutiquement acceptable de celui-ci, dans lequel la configuration absolue de la Formule (I) est

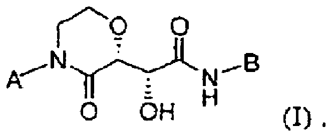

4. Composé selon la revendication 1, qui est du
(2R)-2-[(2R)-4-(4-tert-butylphényl)-3-oxomorpholin-2-yl]-2-hydroxy-N-[4-(N-méthylcarbamimidoyl)phényl]acétamide,
(2R)-2-hydroxy-N-(1-imino-2,3-dihydroisoindol-5-yl)-2-[(2R)-3-oxo-4-(1-oxo-2,3-dihydroisoindol-5-yl)morpholin-2-yl]acétamide,
(2R)-2-hydroxy-N-(1-imino-2,3-dihydroisoindol-5-yl)-2-[(2R)-3-oxo-4-(2-oxo-1H-quinolin-6-yl)morpholin-2-yl]acétamide,
(2R)-2-[(2R)-4-[7-difluorométhoxy)-2-oxo-1H-quinolin-6-yl]-3-oxomorpholin-2-yl]-2-hydroxy-N-(1-imino-2,3-dihydroisoindol-5-yl)acétamide,
(2R)-N-(4-carbamimidoylphényl)-2-hydroxy-2-[(2R)-3-oxo-4-(2-oxo-1H-quinolin-6-yl)morpholin-2-yl]acétamide,
(2R)-N-(4-carbamimidoyl-3-fluorophényl)-2-hydroxy-2-[(2R)-3-oxo-4-(2-oxo-1H-quinolin-6-yl)morpholin-2-yl]acétamide,
(2R)-2-hydroxy-N-(1-imino-2,3-dihydroisoindol-5-yl)-2-[(2R)-3-oxo-4-(2-oxo-1H-quinolin-7-yl)morpholin-2-yl]acétamide,
(2R)-2-hydroxy-N-(1-imino-2,3-dihydroisoindol-5-yl)-2-[(2R)-4-(1-méthyl-2-oxoquinolin-7-yl)-3-oxomorpholin-2-yl]acétamide,
(2R)-2-[(2R)-4-[1-(cyclopropylméthyl)-2-oxoquinolin-7-yl]-3-oxomorpholin-2-yl]-2-hydroxy-N-(1-imino-2,3-dihydroisoindol-5-yl)acétamide,
2-[7-[(2R)-2-[(1R)-1-hydroxy-2-(1-imino-2,3-dihydroisoindol-5-yl)amino]-2-oxoéthyl]-3-oxomorpholin-4-yl]-2-oxoquinolin-1-yl]acétate,
acide 2-[7-[(2R)-2-[(1R)-1-hydroxy-2-(1-imino-2,3-dihydroisoindol-5-yl)amino]-2-oxoéthyl]-3-oxomorpholin-4-yl]-2-oxoquinolin-1-yl]acétique,
(2R)-2-hydroxy-N-(1-imino-2,3-dihydroisoindol-5-yl)-2-[(2R)-4-(8-méthyl-2-oxo-1H-quinolin-7-yl)-3-oxomorpholin-2-yl]acétamide,
(2R)-2-hydroxy-N-(1-imino-2,3-dihydroisoindol-5-yl)-2-[(2R)-4-(2-méthoxyquinolin-7-yl)-3-oxomorpholin-2-yl]acétamide,
(2R)-2-hydroxy-N-(1-imino-2,3-dihydroisoindol-5-yl)-2-[(2R)-3-oxo-4-quinolin-7-ylmorpholin-2-yl]acétamide,
(2R)-N-(3-amino-1,2-benzisoxazol-6-yl)-2-hydroxy-2-[(2R)-3-oxo-4-quinolin-7-ylmorpholin-2-yl]acétamide,
(2R)-2-hydroxy-N-(1-imino-2,3-dihydroisoindol-5-yl)-2-[(2R)-3-oxo-4-(2-oxo-1H-quinolin-5-yl)morpholin-2-yl]acétamide,
(2R)-2-hydroxy-N-(1-imino-2,3-dihydroisoindol-5-yl)-2-[(2R)-3-oxo-4-(1-oxo-3,4-dihydro-2H-isoquinolin-6-yl)morpholin-2-yl]acétamide,
(2R)-2-hydroxy-N-(1-imino-2,3-dihydroisoindol-5-yl)-2-[(2R)-3-oxo-4-(2-oxo-3,4-dihydro-1H-quinolin-7-yl)morpholin-2-yl]acétamide,
(2R)-2-hydroxy-N-(1-imino-2,3-dihydroisoindol-5-yl)-2-[(2R)-4-(1-méthyl-2-oxo-3,4-dihydroquinolin-7-yl)-3-oxomorpholin-2-yl]acétamide,
(2R)-2-[(2R)-4-[1-(cyclopropylméthyl)-2-oxo-3,4-dihydroquinolin-7-yl]-3-oxomorpholin-2-yl]-2-hydroxy-N-(1-imino-2,3-dihydroisoindol-5-yl)acétamide,
2-[7-[(2R)-2-[(1R)-1-hydroxy-2-[(1-imino-2,3-dihydroisoindol-5-yl)amino]-2-oxoéthyl]-3-oxomorpholin-4-yl]-2-oxo-3,4-dihydroquinolin-1-yl]acétate de méthyle,
acide 2-[7-[(2R)-2-[(1R)-1-hydroxy-2-[(1-imino-2,3-dihydroisoindol-5-yl)amino]-2-oxoéthyl]-3-oxomorpholin-4-yl]-2-oxo-3,4-dihydroquinolin-1-yl]acétique,
(2R)-N-(4-carbamimidoyl-3-fluorophényl)-2-hydroxy-2-[(2R)-3-oxo-4-(2-oxo-3,4-dihydro-1H-quinolin-7-yl)morpholin-2-yl]acétamide,
(2R)-N-(3-amino-1,2-benzoxazol-6-yl)-2-hydroxy-2-[(2R)-3-oxo-4-(2-oxo-3,4-dihydro-1H-quinolin-7-yl)morpholin-2-yl]acétamide,
(2R)-N-(1-aminoisoquinolin-6-yl)-2-hydroxy-2-[(2R)-3-oxo-4-(2-oxo-3,4-dihydro-1H-quinolin-7-yl)morpholin-2-yl]acétamide,
(2R)-N-(4-aminoquinazolin-7-yl)-2-hydroxy-2-[(2R)-3-oxo-4-(2-oxo-3,4-dihydro-1H-quinolin-7-yl)morpholin-2-yl]acétamide,
(2R)-2-hydroxy-N-(1-imino-2,3-dihydroisoindol-5-yl)-2-[(2R)-3-oxo-4-(2-oxo-3,4-dihydro-1H-quinolin-5-yl)morpholin-2-yl]acétamide,
(2R)-2-hydroxy-2-[(2R)-4-[2-(3-hydroxy-3-méthylbutoxy)phényl]-3-oxomorpholin-2-yl]-N-(1-imino-2,3-dihydroisoindol-5-yl)-acétamide,
(2R)-2-hydroxy-N-(1-imino-2,3-dihydroisoindol-5-yl)-2-[(2R)-4-[2-(3-méthoxy-3-méthylbutoxy)phényl]-3-oxomorpholin-2-yl]acétamide,
(2R)-2-hydroxy-N-(1-imino-2,3-dihydroisoindol-5-yl)-2-[(2R)-3-oxo-4-[2-(2-oxopipéridin-1yl)phényl]morpholin-2-yl] acétamide,
(2R)-2-[(2R)-4-[2-(difluorométhoxy)-5-fluorophényl]-3-oxomorpholin-2-yl]-2-hydroxy-N-(1-imino-2,3-dihydroisoindol-5-yl)-acétamide,
2-(difluorométhoxy)-3-[(2R)-2-[(1R)-1-hydroxy-2-[(1-imino-2,3-dihydroisoindol-5-yl)amino]-2-oxoéthyl]-3-oxomorpholin-4-yl]benzoate de méthyle,
acide 2-(difluorométhoxy)-3-[(2R)-2-[(1R)-1-hydroxy-2-[(1-imino-2,3-dihydroisoindol-5-yl)amino]-2-oxoéthyl]-3-oxomorpholin-4-yl]benzoïque,
2-(difluorométhoxy)-3-[(2R)-2-[(1R)-1-hydroxy-2-[(1-imino-2,3-dihydroisoindol-5-yl)amino]-2-oxoéthyl]-3-oxomorpholin-4-yl]-N,N-diméthylbenzamide,
2-(difluorométhoxy)-3-[2-[(1-hydroxy-2-[(1-imino-2,3-dihydroisoindol-5-yl)amino]-2-oxoéthyl]-3-oxomorpholin-4-yl]benzamide,
2-[(2R)-2-[(1R)-1-hydroxy-2-[(1-imino-2,3-dihydroisoindol-5-yl)amino]-2-oxoéthyl]-3-oxomorpholin-4-yl]-5-(trifluorométhoxy)benzoate de méthyle,
acide 2-[(2R)-2-[(1R)-1-hydroxy-2-[(1-imino-2,3-dihydroisoindol-5-yl)amino]-2-oxoéthyl]-3-oxomorpholin-4-yl]-5-(trifluorométhoxy)benzoïque,
2-[(2R)-2-[(1R)-1-hydroxy-2-[(1-imino-2,3-dihydroisoindol-5-yl)amino]-2-oxoéthyl]-3-oxomorpholin-4-yl]-5-(trifluorométhoxy)benzamide,
(2R)-N-(3-amino-1,2-benzisoxazol-6-yl)-2-hydroxy-2-[(2R)-3-oxo-4-(2-oxo-1H-quinolin-6-yl)morpholin-2-yl]acétamide,
(2R)-N-(3-amino-1,2-benzisoxazol-6-yl)-2-hydroxy-2-[(2R)-3-oxo-4-(2-oxo-1H-quinolin-7-yl)morpholin-2-yl]acétamide,
(2R)-N-(3-amino-1,2-benzisoxazol-6-yl)-2-hydroxy-2-[(2R)-3-oxo-4-(2-oxo-1H-quinolin-5-yl)morpholin-2-yl]acétamide,
2-(3-((R)-2-((R)-2-(4-carbamimidoylphénylamino)-1-hydroxy-2-oxoéthyl)-3-oxomorpholino)phényl)acétate,
(R)-N-(4-carbamimidoylphényl)-2-hydroxy-2-((R)-4-(3-méthyl-5-(pyrrolidine-1-carbonyl)phényl)-3-oxomorpholin-2-yl)acétamide,
(R)-2-hydroxy-N-(1-iminoisoindolin-5-yl)-2-((R)-4-(3-méthyl-5-(pyrrolidine-1-carbonyl)phényl)-3-oxomorpholin-2-yl)acétamide,
acide 2-(3-((R)-2-((R)-1-hydroxy-2-(1-iminoisoindolin-5-yl)-2-oxoéthyl)-3-oxomorpholino)-N-méthylbenzamido)acétique,
2-(3-((R)-2-((R)-1-hydroxy-2-(1-iminoisoindolin-5-yl)-2-oxoéthyl)-3-oxomorpholino)-N-méthylbenzamido)acétate,
acide (S)-1-(3-((R)-2-((R)-2-(4-carbamimidoylphénylamino)-1-hydroxy-2-oxoéthyl)-3-oxomorpholino)benzoyl)pyrrolidine-2-carboxylique,
1-(3-((R)-2-((R)-2-(4-carbamimidoylphénylamino)-1-hydroxy-2-oxoéthyl)-3-oxomorpholino)benzoyl)pyrrolidine-2-carboxylate,
3-((R)-2-((R)-2-(4-carbamimidoylphénylamino)-1-hydroxy-2-oxoéthyl)-3-oxomorpholino)-N-méthyl-N-(2-oxo-2-(pyrrolidin-1-yl)éthyl)benzamide,
3-((R)-2-((R)-1-hydroxy-2-(1-iminoisoindolin-5-ylamino)-2-oxoéthyl)-3-oxomorpholino)-N-méthyl-N-(2-oxo-2-(pyrrolidin-1-yl)éthyl)benzamide,
(R)-N-(4-carbamimidoylphényl)-2-hydroxy-2-((R)-4-(3-méthyl-5-(morpholine-4-carbonyl)phényl)-3-oxomorpholin-2-yl)acétamide,
(R)-2-hydroxy-N-(1-iminoisoindolin-5-yl)-2-((R)-4-(3-méthyl-5-(morpholine-4-carbonyl)phényl)-3-oxomorpholin-2-yl)acétamide,
(R)-2-hydroxy-N-(1-iminoisoindolin-5-yl)-2-((R)-4-(3-((R)-3-méthoxypyrrolidine-1-carbonyl)phényl)-3-oxomorpholin-2-yl)acétamide,
(R)-2-hydroxy-N-(1-iminoisoindolin-5-yl)-2-((R)-4-(3-((S)-3-méthoxypyrrolidine-1-carbonyl)phényl)-3-oxomorpholin-2-yl)acétamide,
(R)-2-((R)-4-(3-chloro-4-(morpholine-4-carbonyl)phényl)-3-oxomorpholin-2-yl)-2-hydroxy-N-(1-iminoisoindolin-5-yl)acétamide,
(R)-2-hydroxy-N-(1-iminoisoindolin-5-yl)-2-((R)-4-(3-(2-morpholino-2-oxoéthyl))phényl)-3-oxomorpholin-2-yl)acétamide,
acide (S)-1-(3-((R)-2-((R)-1-hydroxy-2-(1-iminoisoindolin-5-ylamino)-2-oxoéthyl)-3-oxomorpholino)benzoyl)pyrrolidine-2-carboxylique,
1-(3-((R)-2-((R)-1-hydroxy-2-(1-iminoisoindolin-5-ylamino)-2-oxoéthyl)-3-oxomorpholino)benzoyl)pyrrolidine-2-carboxylate,
2-(3-((R)-2-((R)-1-hydroxy-2-(1-iminoisoindolin-5-ylamino)-2-oxoéthyl)-3-oxomorpholino)phényl)acétate,
acide 2-(3-((R)-2-((R)-1-hydroxy-2-(1-iminoisoindolin-5-ylamino)-2-oxoéthyl)-3-oxomorpholino)phényl)acétique,
2-(3-((R)-2-((R)-1-hydroxy-2-(1-iminoisoindolin-5-ylamino)-2-oxoéthyl)-3-oxomorpholino)-N-méthylbenzamido)acétate,
propanoate de 2-(3-((R)-2-((R)-1-hydroxy-2-(1-iminoisoindolin-5-ylamino)-2-oxoéthyl)-3-oxomorpholino)phényl)-2-méthyle,
(R)-2-hydroxy-N-(1-iminoisoindolin-5-yl)-2-((R)-4-(3-(morpholine-4-carbonyl)phényl)-3-oxomorpholin-2-yl)acétamide,
3-((R)-2-((R)-1-hydroxy-2-(1-iminoisoindolin-5-ylamino)-2-oxoéthyl)-3-oxomorpholino)-N-méthyl-N-(2-morpholino-2-oxoéthyl)benzamide,
(R)-2-hydroxy-N-(1-iminoisoindolin-5-yl)-2-((R)-4-(3-(2-((R)-3-méthoxypyrrolidin-1-yl)-2-oxoéthyl)phényl)-3-oxomorpholin-2-yl)acétamide,
(R)-2-hydroxy-N-(1-iminoisoindolin-5-yl)-2-((R)-4-(3-(2-((S)-3-méthoxypyrrolidin-1-yl)-2-oxoéthyl)phényl)-3-oxomorpholin-2-yl)acétamide,
propanoate de 2-(3-((R)-2-((R)-1-hydroxy-2-(1-iminoisoindolin-5-ylamino)-2-oxoéthyl)-3-oxomorpholino)phényl)-2-méthyle,
2-(3-((R)-2-((R)-1-hydroxy-2-(1-iminoisoindolin-5-ylamino)-2-oxoéthyl)-3-oxomorpholino)phényl)acétate,
(R)-2-hydroxy-N-(1-iminoisoindolin-5-yl)-2-((R)-4-(3-(4-méthyl-3-oxopipérazine-1-carbonyl)phényl)-3-oxomorpholin-2-yl)acétamide,
propanoate de 2-(3-((R)-2-((R)-1-hydroxy-2-(1-iminoisoindolin-5-ylamino)-2-oxoéthyl)-3-oxomorpholino)phényl)-2-méthyle,
(R)-2-hydroxy-N-(1-iminoisoindolin-5-yl)-2-((R)-4-(3-(2-(4-méthyl-3-oxopipérazin-1-yl)-2-oxoéthyl)phényl)-3-oxomorpholin-2-yl)acétamide,
(R)-2-((R)-4-(3-(2-diméthylamino)-2-oxoéthyl)phényl)-3-oxomorpholin-2-yl)-2-hydroxy-N-(1-iminoisoindolin-5-yl)acétamide,
acide 1-(3-((R)-2-((R)-1-hydroxy-2-(1-iminoisoindolin-5-ylamino)-2-oxoéthyl)-3-oxomorpholino)benzoyl)pipéridine-4-carboxylique,
1-(3-((R)-2-((R)-1-hydroxy-2-(1-iminoisoindolin-5-ylamino)-2-oxoéthyl)-3-oxomorpholino)benzoyl)pipéridine-4-carboxylate,
acide 2-(3-((R)-2-((R)-1-acétoxy-2-(1-imino-3-oxoisoindolin-5-ylamino)-2-oxoéthyl)-3-oxomorpholino)-N-méthylbenzamido)acétique,
(R)-N-(1,3-diiminoisoindolin-5-yl)-2-((R)-4-(3-(2-diméthylamino)-2-oxoéthyl)phényl)-3-oxomorpholin-2-yl)-hydroxyacétamide,
acétate de (R)-2-(1-imino-3-oxoisoindolin-5-ylamino)-1-((R)-4-(3-(méthyl(2-oxo-2-(pyrrolidin-1-yl)éthyl)carbamoyl)phényl)-3-oxomorpholin-2-yl)-2-oxoéthyle,
(R)-N-(3-aminobenzo[d]isoxazol-6-yl)-2-((R)-4-(4-fluoro-3-(morpholine-4-carbonyl)phényl)-3-oxomorpholin-2-yl)-2-hydroxyacétamide,
(R)-N-(3-aminobenzo[d]isoxazol-6-yl)-2-((R)-4-(4-chloro-3-(4,4-difluoropipéridin-1-yl)méthyl)phényl)-3-oxomorpholin-2-yl)-2-hydroxyacétamide,
(R)-N-(3-aminobenzo[d]isoxazol-6-yl)-2-hydroxy-2-((R)-3-oxo-4-(3-oxomorpholino)phényl)morpholin-2-yl)acétamide,
(R)-N-(3-aminobenzo[d]isoxazol-6-yl)-2-((R)-4-(2-(difluorométhoxy)phényl)-3-oxomorpholin-2-yl)-2-hydroxyacétamide,
(R)-N-(3-aminobenzo[d]isoxazol-6-yl)-2-hydroxy-2-((R)-4-(2-isopropoxyphényl)-3-oxomorpholin-2-yl)acétamide),
(R)-N-(3-aminobenzo[d]isoxazol-6-yl)-2-((R)-4-(3-cyanophényl)-3-oxomorpholin-2-yl)-2-hydroxyacétamide,
(R)-N-(3-aminobenzo[d]isoxazol-6-yl)-2-((R)-4-(4-chloro-3-(morpholine-4-carbonyl)phényl)-3-oxomorpholin-2-yl)-2-hydroxyacétamide,
(R)-N-(3-aminobenzo[d]isoxazol-6-yl)-2-hydroxy-2-((R)-4-(4-méthyl-3-(morpholine-4-carbonyl)phényl)-3-oxomorpholin-2-yl)acétamide,
(R)-N-(3-aminobenzo[d]isoxazol-6-yl)-2-hydroxy-2-((R)-3-oxo-4-(3-(trifluorométhoxy)phényl)morpholin-2-yl)acétamide,
(R)-N-(3-aminobenzo[d]isoxazol-6-yl)-2-hydroxy-2-((R)-3-oxo-4-(3-(trifluorométhyl)phényl)morpholin-2-yl)acétamide,
(R)-N-(3-aminobenzo[d]isoxazol-6-yl)-2-((R)-4-(4-chloro-3-(1,2,3,4-tétrahydroisoquinoline-2-carbonyl)phényl)-3-oxomorpholin-2-yl)-2-hydroxyacétamide,
(R)-N-(3-aminobenzo[d]isoxazol-6-yl)-2-((R)-4-(4-chloro-3-(4,4-difluoropipéridine-1-carbonyl)phényl)-3-oxomorpholin-2-yl)-2-hydroxyacétamide,
(R)-N-(3-aminobenzo[d]isoxazol-6-yl)-2-((R)-4-(3-fluoro-4-(trifluorométhyl)phényl)-3-oxomorpholin-2-yl)-2-hydroxyacétamide,
(R)-N-(4-carbamimidoyl-3-fluorophényl)-2-hydroxy-2-((R)-3-oxo-4-(3-oxomorpholino)phényl)morpholin-2-yl)acétamide,
(R)-2-hydroxy-N-(1-imino-7-(trifluorométhyl)isoindolin-5-yl)-2-((R)-3-oxo-4-(3-(3-oxomorpholino)phényl)morpholin-2-yl)acétamide,
(R)-2-hydroxy-N-(1-iminoisoindolin-5-yl)-2-((R)-3-oxo-4-(3-(trifluorométhoxy)phényl)morpholin-2-yl)acétamide,
2-(3-((R)-2-((R)-1-hydroxy-2-(1-iminoisoindolin-5-ylamino)-2-oxoéthyl)-3-oxomorpholino)phénoxy)acétate d'éthyle,
(R)-2-hydroxy-N-(1-iminoisoindolin-5-yl)-2-((R)-4-(4-méthoxybenzyl)-3-oxomorpholin-2-yl)acétamide,
(R)-2((R)-4-(3-(benzyloxy)phényl)-3-oxomorpholin-2-yl)-2-hydroxy-N-(1-iminoisoindolin-5-yl)acétamide,
(R)-N-(1-aminoisoquilolin-6-yl)-2-((R)-4-(4-fluoro-3-(morpholine-4-carbonyl)phényl)-3-oxomorpholin-2-yl)-2-hydroxyacétamide,
N-(2,3-dihydro-1-imino-1H-isoindolin-5-yl)-4-[2-[(diméthylamino)carbonyl]phényl]-alpha(R)-hydroxy-3-oxo-2(R)-morpholineacétamide,
N-(2,3-dihydro-1-imino-1H-isoindolin-5-yl)-alpha(R)-hydroxy-3-oxo-4-[2-(1-pyrrolidinylcarbonyl)phényl]-2(R)-morpholineacétamide,
4-(2-cyanophényl)-N-(2,3-dihydro-1-imino-1H-isoindolin-5-yl)-alpha(R)-hydroxy-3-oxo-2(R)-morpholineacétamide,
N-(2,3-dihydro-1-imino-1H-isoindolin-5-yl)-4-[4-fluoro-3-(4-morpholinylcarbonyl)phényl]-alpha(R)-hydroxy-3-oxo-2(R)-morpholineacétamide,
N-(2,3-dihydro-1-imino-1H-isoindolin-5-yl)-4-[4-fluoro-3-[(3(R)-méthyl-4-morpholinyl)carbonyl]phényl]-alpha(R)-hydroxy-3-oxo-2(R)-morpholineacétamide,
N-(2,3-dihydro-1-imino-1H-isoindolin-5-yl)-4-[4-fluoro-3-[(3(S)-méthyl-4-morpholinyl)carbonyl]phényl]-alpha(R)-hydroxy-3-oxo-2(R)-morpholineacétamide,
N-[4-(aminoiminométhyl)phényl]-4-(2-cyanophényl)-alpha(R)-hydroxy-3-oxo-2(R)-morpholineacétamide,
N-(4-amino-7-quinazolinyl)-4-[4-fluoro-3-(4-morpholinylcarbonyl)phényl]-alpha(R)-hydroxy-3-oxo-2(R)-morpholineacétamide,
[3-[2(R)-[2-[(3-amino-1,2-benzisoxazol-6-yl)amino]-1(R)-hydroxy-2-oxoéthyl]-3-oxo-4-morpholinyl]phényl]pentafluorosulfur,
[4-[2(R)-[2-[(3-amino-1,2-benzisoxazol-6-yl)amino]-1(R)-hydroxy-2-oxoéthyl]-3-oxo-4-morpholinyl]phényl]pentafluorosulfur,
N-(3-amino-5-fluoro-1,2-benzisoxazol-6-yl)-4-[4-fluoro-3-(morpholinylcarbonyl)phényl]-alpha(R)-hydroxy-3-oxo-2(R)-morpholineacétamide,
N-(2-amino-6-quinazolinyl)-4-[4-fluoro-3-(morpholinylcarbonyl)phényl]-alpha(R)-hydroxy-3-oxo-2(R)-morpholineacétamide,
(R)-2-((R)-4-(5-fluoro-2-isopropoxyphényl)-3-oxomorpholin-2-yl)-2-hydroxy-N-(1-iminoisoindolin-5-yl)acétamide,
(R)-2-hydroxy-N-(1-iminoisoindolin-5-yl)-2-((R)-3-oxo-4-(2-trifluorométhyl)phényl)morpholin-2-yl)acétamide,
(R)-2-((R)-4-(2-(difluorométhoxy)phényl)-3-oxomorpholin-2-yl)-2-hydroxy-N-(1-iminoisoindolin-5-yl)acétamide,
(R)-2-hydroxy-N-(1-iminoisoindolin-5-yl)-2-((R)-4-(2-isopropoxyphényl)-3-oxomorpholin-2-yl)acétamide,
(R)-2-hydroxy-N-(1-iminoisoindolin-5-yl)-2-((R)-3-oxo-4-(2-trifluorométhoxy)phényl)morpholin-2-yl)acétamide,
N-(3-amino-1,2-benzisoxazol-6-yl)-4-[4-fluoro-3-[(tétrahydro-2H-pyran-4-yl)oxy]phényl]-alpha(R)-hydroxy-3-oxo-2(R)-morpholineacétamide,
N-(3-amino-1,2-benzisoxazol-6-yl)-4-[3-[(3,3-difluoro-1-azétidinyl)carbonyl]-4-fluorophényl]-alpha(R)-hydroxy-3-oxo-2(R)-morpholineacétamide,
N-(3-amino-1,2-benzisoxazol-6-yl)-4-[3-[(4,4-difluoro-1-pipéridinyl)carbonyl]-4-fluorophényl]-alpha(R)-hydroxy-3-oxo-2(R)-morpholineacétamide,
N-(3-amino-1,2-benzisoxazol-6-yl)-4-[4-chloro-3-[3-(1,1-diméthyléthyl)-1,2,4-oxadiazol-5-yl]phényl]-alpha(R)-hydroxy-3-oxo-2(R)-morpholineacétamide,
N-(3-amino-1,2-benzisoxazol-6-yl)-4-[2-fluoro-5-(trifluorométhyl)phényl]-alpha(R)-hydroxy-3-oxo-2(R)-morpholineacétamide,
(R)-N-(4-carbamimidoylphényl)-2-hydroxy-2-((R)-4-(3-((S)-2-méthylpyrrolidine-1-carbonyl)phényl)-3-oxomorpholin-2-yl)acétamide,
(R)-N-(4-carbamimidoylphényl)-2-hydroxy-2-((R)-4-(2-méthyl-3-(morpholine-4-carbonyl)phényl)-3-oxomorpholin-2-yl)acétamide,
(R)-N-(4-carbamimidoylphényl)-2-hydroxy-2-((R)-4-(2-méthyl-3-(pyrrolidine-4-carbonyl)phényl)-3-oxomorpholin-2-yl)acétamide,
(R)-2-((R)-4-(3-(azétidine-1-carbonyl)-5-fluorophényl)-3-oxomorpholin-2-yl)-N-(4-carbamimidoylphényl)-2-hydroxyacétamide,
(R)-2-((R)-4-(3-(azétidine-1-carbonyl)-2-méthylphényl)-3-oxomorpholin-2-yl)-N-(4-carbamimidoylphényl)-2-hydroxyacétamide,
(R)-2-((R)-4-(5-(azétidine-1-carbonyl)-2-méthylphényl)-3-oxomorpholin-2-yl)-N-(4-carbamimidoylphényl)-2-hydroxyacétamide,
(R)-N-(4-carbamimidoylphényl)-2-hydroxy-2-((R)-3-oxo-4-(6-trifluorométhyl)pyridin-2-yl)morpholin-2-yl)acétamide,
(R)-N-(4-carbamimidoylphényl)-2-hydroxy-2-((R)-4-(2-méthyl-5-(pyrrolidine-1-carbonyl)phényl)-3-oxomorpholin-2-yl)acétamide,
(R)-2-((R)-4-(3-(azétidine-1-carbonyl)-5-méthylphényl)-3-oxomorpholin-2-yl)-N-(4-carbamimidoylphényl)-2-hydroxyacétamide,
(R)-N-(4-carbamimidoylphényl)-2-hydroxy-2-((R)-4-(2-méthyl-5-(morpholine-4-carbonyl)phényl)-3-oxomorpholin-2-yl)acétamide,
acide (S)-1-(3-((R)-2-((R)-2-(4-carbamimidoylphénylamino)-1-hydroxy-2-oxoéthyl)-3-oxomorpholino)-5-fluorobenzoyl)pyrrolidine-2-carboxylique,
1-(3-((R)-2-((R)-2-(4-carbamimidoylphénylamino)-1-hydroxy-2-oxoéthyl)-3-oxomorpholino)-5-fluorobenzoyl)pyrrolidine-2-carboxylate,
1-(3-((R)-2-((R)-2-(4-carbamimidoylphénylamino)-1-hydroxy-2-oxoéthyl)-3-oxomorpholino)-5-fluorobenzoyl)pyrrolidine-2-carboxylate,
acide 2-(3-((R)-2-((R)-2-(4-carbamimidoylphénylamino)-1-hydroxy-2-oxoéthyl)-3-oxomorpholino)-5-fluoro-N-méthylbenzamido)acétique,
2-(3-((R)-2-((R)-2-(4-carbamimidoylphénylamino)-1-hydroxy-2-oxoéthyl)-3-oxomorpholino)-5-fluoro-N-méthylbenzamido)acétate,
3-((R)-2-((R)-2-(4-carbamimidoylphénylamino)-1-hydroxy-2-oxoéthyl)-3-oxomorpholino)-N-(2-(diméthylamino)-2-oxoéthyl)-5-fluoro-N-méthylbenzamide,
(R)-N-(1,3-diiminoisoindolin-5-yl)-2-hydroxy-2-((R)-4-(3-(2-(4-méthyl-3-oxopipérazin-1-yl)-2-oxoéthyl)phényl)-3-oxomorpholin-2-yl)acétamide,
N-(2-(diméthylamino)-2-oxoéthyl)-3-((R)-2-((R)-1-hydroxy-2-(1-iminoisoindolin-5-ylamino)-2-oxoéthyl)-3-oxomorpholino)-N-méthylbenzamide,
2-((S)-hydroxy((R)-3-oxo-4-p-tolylmorpholin-2-yl)méthyl)-4-oxo-1,4-dihydroquinazoline-6-carboximidamide,
1,1-dioxo-3-((S)-hydroxy((R)-3-oxo-4-p-tolylmorpholin-2-yl)méthyl)-4H-benzo[e][1,2,4]thiadiazine-7-carboximidamide,
(R)-N-(3-aminobenzo[d]isoxazol-6-yl)-2-((R)-4-(4-chloro-3-(morpholinométhyl)phényl)-3-oxomorpholin-2-yl)-2-hydroxyacétamide,
(R)-N-(3-aminobenzo[d]isoxazol-6-yl)-2-hydroxy-2-((R)-4-(3-(morpholine-4-carbonyl)phényl)-3-oxomorpholin-2-yl)acétamide,
acide 3-((R)-2-((R)-2-(4-carbamimidoyl-3-fluorophénylamino)-1-hydroxy-2-oxoéthyl)-3-oxomorpholino)benzoïque,
(R)-N-(4-carbamimidoyl-3,5-difluorophényl)-2-hydroxy-2-((R)-3-oxo-4-(3-(3-oxomorpholino)phényl)morpholin-2-yl)acétamide,
(R)-N-(4-carbamimidoyl-2,3-difluorophényl)-2-hydroxy-2-((R)-3-oxo-4-(3-(3-oxomorpholino)phényl)morpholin-2-yl)acétamide,
(R)-N-(4-carbamimidoyl-2,5-difluorophényl)-2-hydroxy-2-((R)-3-oxo-4-(3-(3-oxomorpholino)phényl)morpholin-2-yl)acétamide,
(R)-N-(3-(aminométhyl)-4-cyano-5-(trifluorométhyl)phényl)-2-hydroxy-2-((R)-3-oxo-4-(3-(3-oxomorpholino)phényl)morpholin-2-yl)acétamide,
(R)-2-hydroxy-N-(1-iminoisoindolin-5-yl)-2-((R)-4-(2-méthyl-1-oxoisoindolin-5-yl)-3-oxomorpholin-2-yl)acétamide,
chlorhydrate de (R)-2-hydroxy-N-(1-iminoisoindolin-5-yl)-2-((R)-4-(2-méthyl-3-oxoisoindolin-5-yl)-3-oxomorpholin-2-yl)acétamide,
(R)-2-((R)-4-(2-(cyclopropylméthyl)-3-oxoisoindolin-5-yl)-3-oxomorpholin-2-yl)-2-hydroxy-N-(1-iminoisoindolin-5-yl)acétamide,
(R)-N-(4-(aminométhyl)-3-fluorophényl-2-hydroxy-2-((R)-3-oxo-4-(3-(3-oxomorpholino)phényl)morpholin-2-yl)acétamide,
(R)-N-(4-guanidinophényl)-2-hydroxy-2-((R)-3-oxo-4-(3-(3-oxomorpholino)phényl)morpholin-2-yl)acétamide,
(R)-N-(4-(aminométhyl)phényl)-2-((R)-4-(4-fluoro-3-(morpholine-4-carbonyl)phényl)-3-oxomorpholin-2-yl)-2-hydroxyacétamide,
acétate de (R)-2-(4-carbamoylphénylamino)-1-((R)-4-(4-fluoro-3-(morpholine-4-carbonyl)phényl)-3-oxomorpholin-2-yl)-2-oxyéthyle,
(R)-2-((R)-4-(4-fluoro-3-(morpholine-4-carbonyl)phényl)-3-oxomorpholin-2-yl)-2-hydroxy-N-(1,2,3,4-tétrahydroisoquinolin-6-yl)acétamide,
(R)-N-(4-(aminométhyl)-3-fluorophényl)-2-((R)-4-(4-fluoro-3-(morpholine-4-carbonyl)phényl)-3-oxomorpholin-2-yl)-2-hydroxyacétamide,
(R)-N-(4-((R)-1-aminoéthyl)phényl)-2-((R)-4-(4-fluoro-3-(morpholine-4-carbonyl)phényl)-3-oxomorpholin-2-yl)-2-hydroxyacétamide,
(R)-N-((R)-1-amino-2,3-dihydro-1H-inden-5-yl)-2-((R)-4-(4-fluoro-3-(morpholine-4-carbonyl)phényl)-3-oxomorpholin-2-yl)-2-hydroxyacétamide,
(R)-N-((S)-1-amino-2,3-dihydro-1H-inden-5-yl)-2-((R)-4-(4-fluoro-3-(morpholine-4-carbonyl)phényl)-3-oxomorpholin-2-yl)-2-hydroxyacétamide,
(R)-N-(2-aminoquinolin-6-yl)-2-((R)-4-(4-fluoro-3-(morpholine-4-carbonyl)phényl)-3-oxomorpholin-2-yl)-2-hydroxyacétamide,
N-(7-fluoro-2,3-dihydro-1-imino-1H-isoindol-5-yl)-alpha(R)-hydroxy-3-oxo-4-[3-(3-oxo-4-morpholinyl)phényl]-2(R)-morpholineacétamide,
N-(4-chloro-2,3-dihydro-1-imino-1H-isoindol-5-yl)-alpha(R)-hydroxy-3-oxo-4-[3-(3-oxo-4-morpholinyl)phényl]-2(R)-morpholineacétamide,
acide 3-[2(R)-[2-[(2,3-dihydro-1-imino-1H-isoindol-5-yl)amino]-1(R)-hydroxy-2-oxoéthyl]-3-oxo-4-morpholinyl]benzoïque,
3-[2(R)-[2-[(2,3-dihydro-1-imino-1H-isoindol-5-yl)amino]-1(R)-hydroxy-2-oxoéthyl]-3-oxo-4-morpholinyl]benzoate de méthyle,
2-[2(R)-[2-[(2,3-dihydro-1-imino-1H-isoindol-5-yl)amino]-1(R)-hydroxy-2-oxoéthyl]-3-oxo-4-morpholinyl]benzoate de méthyle,
N-(3-amino-1,2-benzisoxazol-6-yl)-4-[4-fluoro-3-[2,2,2-trifluoro-1-(4-morpholinyl)éthyl]phényl]-alpha(R)-hydroxy-3-oxo-2(R)-morpholineacétamide,
(R)-2-((R)-4-(5-chloro-2-isopropoxyphényl)-3-oxomorpholin-2-yl)-2-hydroxy-N-(1-iminoisoindolin-5-yl)acétamide, ou
N-(3-amino-1,2-benzisoxazol-6-yl)-4-[3-(difluorométhoxy)-4-fluorophényl]-alpha(R)-hydroxy-3-oxo-2(R)-morpholineacétamide,
ou un sel pharmaceutiquement acceptable de ceux-ci.

5. Composé qui est du 2-((S)-hydroxy((R)-3-oxo-4-p-tolylmorpholin-2-yl)méthyl)-4-oxo-1,4-dihydroquinazoline-5-carboximidamide ou du 1,1-dioxo-3-((S)-hydroxy((R)-3-oxo-4-p-tolymorpholin-2-yl-méthyl-4H-benzo[e][1,2,4]thiadiazine-7-carboximidamide ou un sel pharmaceutiquement acceptable de ceux-ci.

6. Composition pharmaceutique comprenant au moins un composé selon l'une quelconque des revendications 1 à 5 ou un sel pharmaceutiquement acceptable de ceux-ci et au moins un véhicule pharmaceutiquement acceptable.

7. Composé selon l'une quelconque des revendications 1 à 5 ou un sel pharmaceutiquement acceptable de celui-ci, à utiliser dans le traitement d'une maladie ou d'un trouble causé par l'intermédiaire du facteur IXa.

8. Composé ou un sel pharmaceutiquement acceptable de celui-ci à utiliser selon la revendication 7, où la maladie ou le trouble est un trouble thromboembolique.

9. Composé ou un sel pharmaceutiquement acceptable de celui-ci à utiliser selon la revendication 8, où le trouble thromboembolique est sélectionné parmi le groupe consistant en troubles thromboemboliques cardiovasculaires artériels, troubles thromboemboliques cardiovasculaires veineux et troubles thromboemboliques dans les chambres du coeur.

10. Composé ou un sel pharmaceutiquement acceptable de celui-ci à utiliser selon la revendication 8, où le trouble thromboembolique est sélectionné parmi l'angine de poitrine instable, un syndrome coronarien aigu, fibrillation auriculaire, premier infarctus du myocarde, infarctus du myocarde récurrent, mort ischémique subite, attaque ischémique transitoire, attaque, athérosclérose, maladie artérielle périphérique occlusive, thrombose veineuse, thrombose veineuse profonde, thrombophlébite, embolie artérielle, thrombose artérielle coronarienne, thrombose artérielle cérébrale, embolie cérébrale, embolie rénale, embolie pulmonaire et thrombose résultant de (a) valves prothétiques ou autres implants, (b) sondes à demeure, (c) stents, (d) pontage cardiopulmonaire, (e) hémodialyse ou (f) d'autres procédures dans lesquelles le sang est exposé à une surface artificielle qui favorise la thrombose.

11. Combinaison d'un composé selon l'une quelconque des revendications 1-5 ou d'un sel pharmaceutiquement acceptable de ceux-ci et d'au moins un agent anticoagulant sélectionné parmi le groupe consistant en un inhibiteur de la thrombine, un antagoniste des récepteurs de la thrombine (PAR-1), un inhibiteur du facteur VIIa, un inhibiteur du facteur VIIIa, un inhibiteur du facteur IXa différent du composé selon les revendications 1-5, un inhibiteur du facteur Xa, un inhibiteur du facteur XIa, un TAFI et des inhibiteurs du fibrinogène.

12. Combinaison selon la revendication 11, dans laquelle ledit inhibiteur du facteur IXa est sélectionné parmi le groupe consistant en anticorps monoclonaux, inhibiteurs compétitifs synthétiques bloqués au niveau du site actif, inhibiteurs oraux et aptamères d'ARN.

13. Composition pharmaceutique comprenant:
une quantité thérapeutiquement efficace d'au moins un composé selon l'une quelconque des revendications 1-5 ou un d'un sel pharmaceutiquement acceptable de celui-ci et au moins un véhicule pharmaceutiquement acceptable et une quantité efficace d'au moins un agent sélectionné parmi le groupe consistant en: (a) anticoagulants, (b) agents antithrombine, (c) agents antiplaquettaires, (d) fibinolytiques, (e) agents hypolipidémiants, (f) agents antihypertenseurs et (g) agents anti-ischémiques.

14. Composition pharmaceutique comprenant:
une quantité thérapeutiquement efficace d'au moins un composé selon l'une quelconque des revendications 1-5 ou un d'un sel pharmaceutiquement acceptable de celui-ci et au moins un véhicule pharmaceutiquement acceptable et une quantité efficace d'au moins un agent sélectionné parmi le groupe consistant en: (a-1) warfarine, (a-2) héparine, (a-3) aprotinine, (a-4) pentasaccharide synthétique, (a-5) inhibiteurs directs de la thrombine comprenant l'hirudine et l'argatroban, (a-6) un inhibiteur du facteur VIIa, (a-7) un inhibiteur du facteur VIIIa, (a-8) un inhibiteur du facteur IXa différent des composés de la Formule (I), (a-9) un inhibiteur du facteur Xa, (a-10) un inhibiteur du facteur XIa, (a-11) un inhibiteur de la thrombine, (a-12) un TAFI, (a-13) un inhibiteur du fibrinogène, (b-1) un dérivé de boroarginine, (b-2) un boropeptide, (b-3) l'héparine, (b-4) l'hirudine, (b-5) l'argatroban, (c-1) un AINS, (c-2) un antagoniste de IIb/IIIa, (c-3) un antagoniste des récepteurs de thromboxane A2, (c-4) un inhibiteur de la synthétase de thromboxane A2, (c-5) un inhibiteur de la PDE-III, (c-6) un inhibiteur de la PDE V, (c-7) un antagoniste des récepteurs de l'ADP, (c-8) un antagoniste du récepteur purinergique de type P2Y1, (c-9) un antagoniste du récepteur purinergique de type P2Y12, (d-1) l'activateur tissulaire du plasminogène (TPA, naturel ou recombinant) et des formes modifiées de celui-ci, (d-2) l'anistréplase, (d-3) l'urokinase, (d-4) la streptokinase, (d-5) la ténectéplase (TNK), (d-6) la lanotéplase (nPA), (d-7) un inhibiteur du facteur VIIa, (d-8) un inhibiteur PAI-1, (d-9) un inhibiteur alpha-2-antiplasmine, (d-10) un complexe activateur anisoylé de plasminogène streptokinase, (e-1) un inhibiteur de la HMG-CoA réductase, (e-2) un inhibiteur de la squalène synthétase, (e-3) un fibrate, (e-4) un séquestrant des acides biliaires, (e-5) un inhibiteur de l'ACAT, (e-6) un inhibiteur de la MTP, (e-7) un inhibiteur de la lipooxygénase, (e-8) un inhibiteur de l'absorption du cholestérol, (e-9) un inhibiteur de la protéine de transfert des esters de cholestérol, (f-1) un bloqueur alpha-adrénergique, (f-2) un bloqueur bêta-adrénergique, (f-3) un bloqueur des canaux calciques, (f-4) des diurétiques, (f-5) un inhibiteur de la rénine, (f-6) un inhibiteur de l'enzyme de conversion de l'angiotensine, (f-7) un antagoniste des récepteurs de l'angiotensine II, (f-8) un antagoniste des récepteurs ET, (f-9) un co-antagoniste ET/AII, (f-10) un inhibiteur de l'endopeptidase neutre, (f-11) un inhibiteur de la vasopeptidase, (g-1) un agent de la Classe I, (g-2) un agent de la Classe II, (g-3) un agent de la Classe III, (g-4) un agent de la Classe IV, (g-5), un ouvreur de canaux K⁺, (g-6) un inhibiteur d'IKur et (g-7) un glycoside cardiaque.

15. Combinaison d'un composé selon l'une quelconque des revendications 1-5 ou d'un sel pharmaceutiquement acceptable de celui-ci et d'un ou de plusieurs agents thérapeutiques supplémentaires.

16. Composé selon l'une quelconque des revendications 1-5 ou un sel pharmaceutiquement acceptable de celui-ci, à utiliser en thérapie.
